(19) Europäisches Patentamt
European Patent Office
Office européen des brevets

(11) EP 1 899 335 B1

(12) # FASCICULE DE BREVET EUROPEEN

(45) Date de publication et mention de la délivrance du brevet:
**19.05.2010 Bulletin 2010/20**

(21) Numéro de dépôt: **06777531.2**

(22) Date de dépôt: **30.06.2006**

(51) Int Cl.:
***C07D 471/04*** (2006.01) ***A61K 31/4745*** (2006.01)
***A61P 35/00*** (2006.01)

(86) Numéro de dépôt international:
**PCT/EP2006/063768**

(87) Numéro de publication internationale:
**WO 2007/003611 (11.01.2007 Gazette 2007/02)**

(54) **DERIVES DE PYRROLOQUINOLINES ET LEUR UTILISATION COMME INHIBITEURS DE PROTEINES KINASES**

PYRROLOCHINOLINDERIVATE UND IHRE VERWENDUNG ALS PROTEINKINASEHEMMER

PYRROLOQUINOLINE DERIVATIVES AND THEIR USE AS PROTEIN KINASE INHIBITORS

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK RS**

(30) Priorité: **01.07.2005 FR 0507009**

(43) Date de publication de la demande:
**19.03.2008 Bulletin 2008/12**

(73) Titulaire: **PIERRE FABRE MEDICAMENT**
**92100 Boulogne-Billancourt (FR)**

(72) Inventeurs:
- **DUMOULIN, Antoine**
  **F-81100 CASTRES (FR)**
- **KALOUN, El Bachir**
  **F-31120 ROQUETTES (FR)**
- **POIGNY, Stéphane**
  **F-31600 SAUBENS (FR)**
- **BIENHAYME, Hugues**
  **F-69360 St Symphorien d'Ozon (FR)**
- **GRISONI, Serge**
  **F-31120 PORTET-SUR-GARONNE (FR)**
- **RABOT, Rémi**
  **F-31400 TOULOUSE (FR)**
- **RAHALI, Rachid**
  **F-30126 Saint Laurent des Arbres (FR)**
- **TAM, Eric**
  **SINGAPOUR - 680549 (ID)**
- **KLEIN, Pascaline**
  **F-31400 TOULOUSE (FR)**
- **BEDJEGUELAL, Karim**
  **F-31400 TOULOUSE (FR)**
- **RAHALI, Houcine**
  **F-30126 Saint Laurent des Arbres (FR)**

(74) Mandataire: **Tetaz, Franck Claude Edouard et al**
**Cabinet Régimbeau**
**20, rue de Chazelles**
**75847 Paris Cedex 17 (FR)**

(56) Documents cités:
**WO-A-01/98299 WO-A-03/082868**

- **BERGMAN J ET AL: "Synthesis of 4-oxo-4,5-dihydro-3H-pyrrolo[2,3-c]quinoline-1-carboxylic acid ethyl ester and its isomer 1-oxo-2,9-dihydro-1H-beta-carboline-4 carboxylic acid ethyl ester" 4 novembre 2002 (2002-11-04), TETRAHEDRON, ELSEVIER SCIENCE PUBLISHERS, AMSTERDAM, NL, PAGE(S) 9179-9185 , XP004390240 ISSN: 0040-4020 page 9182; exemples 30,31**
- **ARIGHI E ET AL: "RET tyrosine kinase signaling in development and cancer" CYTOKINE AND GROWTH FACTOR REVIEWS, OXFORD, GB, vol. 16, no. 4-5, 27 juin 2005 (2005-06-27), pages 441-467, XP004991793 ISSN: 1359-6101**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne de nouveaux composés qui inhibent ou modulent l'activité enzymatique de protéines kinases, et qui peuvent être employés dans le traitement de diverses maladies, notamment cancer, inflammation ou affections du système nerveux central. La présente invention concerne également les compositions pharmaceutiques comprenant les composés selon l'invention et leur utilisation en thérapie.

Les protéines kinases sont des enzymes connues pour catalyser le transfert du groupement phosphate terminal de l'adénosine triphosphate (ATP) sur une tyrosine, une sérine ou une thréonine de leurs protéines substrats. Il existe deux grandes classes de protéines kinases : les tyrosine kinases (récepteur et non récepteur), qui transfèrent un groupement phosphate sur un résidu tyrosine et qui sont des enzymes membranaires ou cytoplasmiques, et les sérine/thréonine kinases, qui transfèrent le phosphate sur une sérine ou une thréonine et qui sont essentiellement des enzymes cytoplasmiques.

Parmi les tyrosine kinases, on distingue deux catégories : les « récepteur » tyrosine kinases, qui sont transmembranaires, et possèdent une partie extracellulaire susceptible de reconnaître un ligand, et les « non-recepteurs » tyrosine kinases, qui sont cytoplasmiques. Ces enzymes jouent un rôle clé dans la transduction du signal cellulaire, et par voie de conséquence sont considérées comme importantes dans des processus physiologiques comme la prolifération cellulaire, la mitose, la différentiation, l'invasion et la mobilité cellulaire, l'apoptose, etc... Ces enzymes sont considérées comme jouant un rôle important lors des différents stades du développement tumoral, et par voie de conséquence comme des cibles pharmaceutiques importantes pour le traitement des cancers en particulier.

Parmi les récepteurs tyrosine kinases (RTK), on peut citer : ALK, EGFR, VEGFR, IGFR, FGFR, Trk, MET et RET. Dans cette famille, RET a été décrit comme un proto-oncogène impliqué dans la survie et la prolifération de certaines cellules. En particulier, il a été montré que la phosphorylation de RET induit l'activation d'autres kinases comme JAK-STAT, PKC, ERK5, p38MAPK, PI3K-AKT, RAS-ERK ou JNK. Toutes ces voies métaboliques contribuent à la transformation cellulaire lorsqu'elles sont suractivées, alors qu'elles participent à la survie des cellules en conditions normales. Il a été montré que RET peut être activé par des mutations ou des réarrangements du gène, entraînant des néoplasies multiples endocrines de type II A (MEN 2A), de type II B (MEN 2B), la maladie de Hirschsprung (HSCR) ou des carcinomes médulaires de la thyroïde (MTC). Certaines des mutations oncogènes peuvent causer une autoactivation de la fonction kinase de RET (Salvatore et al. 2000, J. Clin. Endocr. Metab. 85: 3898-3907). Cette activation résulte en une autophosphorylation de tyrosines entraînant l'induction du signal dépendant de RET.

Plusieurs travaux décrivent les conséquences qu'entraînent l'inhibition de RET sur le métabolisme cellulaire (Pour une revue: Pützer and Drosten, 2004, Trends in Mol. Med. Vol.10, No7: 351-357). En particulier, l'utilisation d'un mutant dominant négatif de RET dans des cellules TT a pour effet d'induire une forte inhibition de la viabilité de ces cellules, effet qui résulte à la fois d'une inhibition de la croissance cellulaire, ainsi que d'une possible induction de l'apoptose (Drosten et al. 2002, Surgery 132:991-7). D'autre part, il a été montré que certains inhibiteurs de kinase avaient une action sur RET; par exemple, STI571 (Gleevec), genistein et allyl-geldanamycin inhibent l'activité de RET d'une manière dose-dépendante (Cohen et al. 2002, Surgery 132:960-7). L'inhibiteur de kinases de la famille Src, PP1, a également la capacité d'inhiber RET (Carlomagno et al. 2002, Cancer Research 62: 1077-1082). Ces auteurs ont montré que PP1 inhibe fortement la tumorigénicité des fibroblastes NIH3T3 exprimant l'oncogène RET/PTC3 dans des souris nudes.

La fonction de RET a été particulièrement bien décrite dans la thyroïde, tissu où ce récepteur est fortement exprimé, notamment en présence de tumeurs. Plus généralement, ce sont les tumeurs qui ont pour origine les crêtes neurales qui expriment RET le plus fortement (neuroblastomes, pheochromocytomes ou carcinomes médullaires de la thyroïde) (Ikeda et al. 1990, Oncogene 5: 1291-1296 ; Santoro et al. 1990, Oncogene 5: 1595-1598). Dans les tissus non tumoraux, RET est exprimé dans les neurones des systèmes entériques périphériques, sympathiques et sensoriels ainsi que dans le système nerveux central (neurones à dopamine et à noradrénaline). De plus, l'expression de RET a été décrite dans le rein durant le développement embryonnaire (Avantaggiato et al. 1994, Cell Growth Differ. 5: 305-311; Attie-Bitach et al. 1998, Am. J. Med. Genet. 80:481-486).

Ces études permettent de montrer l'utilisation d'un inhibiteur de la protéine RET dans les cancers (Takahashi et al. 1991 Oncogene 6:297-301), en particulier mais non limités aux tumeurs solides (Jhiang SM, 2000 Oncogene 19 (49):5590-5597), et dans différents types de tissus surexprimant RET (thyroïde, poumon, prostate, sein, rein, colon, pancréas). Un inhibiteur de RET peut aussi être indiqué pour des maladies inflammatoires (Russell et al. 2004 J. Immunol. 172(7):4059-4067) et pour le traitement du diabète (Harada et al. 2002 Diabetes Care 25(6):1060-1065).

La présente invention concerne des composés de formule I:

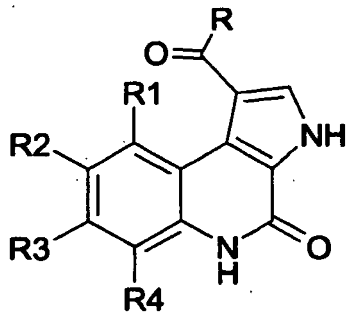

(I)

dans laquelle :

R représente un atome d'hydrogène ou un groupe choisi parmi les radicaux alkyle, alkényle, alkynyle, cycloalkyle, aralkyle, aryle, hétéroaryle -O-R5, ou -NR5-R6, éventuellement substitués par un ou plusieurs substituants,
R1 et R4 représentent indépendamment un atome d'hydrogène, un halogène ou un groupe choisi parmi les radicaux nitro, alkyle, alkényle, alkynyle, alcoxy ou cycloalkyle, éventuellement substitués par un ou plusieurs substituants,
R2 et R3 représentent un atome d'hydrogène, un halogène ou un groupe choisi parmi les radicaux alkyle, alkényle, alkynyle, alcoxy, aryle, hétéroaryle, aryloxy, aralkyloxy, éventuellement substitués par un ou plusieurs substituants, et les radicaux hydroxy, cyano, -Y-(NR7)n-R8, -SOm-R8, -NR7-(Y)n-R8, -NO2, COOR9,
n=0 ou 1
m = 0 ou 1
Y = -CO- ou -SO2-
R2 et R3 n'étant pas simultanément sur le même composé les groupes suivants : aryle, hétéroaryle, aryloxy, aralkyloxy, -Y-(NR7)n-R8, -SOm-R8, -NR7-(Y)n-R8, -NO2, COOR9,
R5 représente un atome d'hydrogène ou un radical alkyle, alkényle d'au moins 3 atomes de carbones non directement branché sur l'insaturation, alkynyle d'au moins 3 atomes de carbone non directement branché sur l'insaturation, cycloalkyle, cycloalkylalkyle, aralkyle, hétéroaralkyle, hétérocycle saturé non azoté alkyle éventuellement substitué par un ou plusieurs substituants,
R6 représente un atome d'hydrogène ou un radical alkyle, alkényle d'au moins 3 atomes de carbones non directement branché sur l'insaturation, alkynyle d'au moins 3 atomes de carbone non directement branché sur l'insaturation, cycloalkyle, cycloalkylalkyle, aryle, hétéroaryle, aralkyle, hétéroaralkyle, hétérocycle saturé non azoté alkyle éventuellement substitué par un ou plusieurs substituants,
R7 représente un atome d'hydrogène ou un radical alkyle, alkényle d'au moins 3 atomes de carbone non directement branché sur l'insaturation, alkynyle d'au moins 3 atomes de carbone non directement branché sur l'insaturation, cycloalkyle, cycloalkylalkyle, éventuellement substitué par un ou plusieurs substituants,
R8 représente un atome d'hydrogène ou un groupe choisi parmi les radicaux alkyle, alkényle, alkynyle, alcoxy, cycloalkyle, cycloalkylalkyle, aralkyle, aryle, hétéroaryle, hétéroaralkyle, hétérocycle saturé ou hétérocycle saturé alkyle, éventuellement substitués par un ou plusieurs substituants,
R9 représente un atome d'hydrogène ou un groupe choisi parmi les radicaux alkyle, alkényle, alkynyle, cycloalkyle, cycloalkylalkyle, aralkyle, aryle, hétéroaralkyle, hétérocycle saturé ou hétérocycle saturé alkyle, éventuellement substitués par un ou plusieurs substituants,
R7 et R8 pouvant former ensemble un cycle ou un bicycle contenant 1 ou plusieurs hétéroatomes avec l'azote auquel ils sont rattachés ledit hétérocycle étant éventuellement substitué par un ou plusieurs groupes alkyle, alkényle, alkynyle, amino, NR10R11, hydroxy, alcoxy, alcoyle, carbamoyle, sulfamoyle, sulfonyle, sulfényle, sulfanyle, cyano ou un radical oxo,
R10 représente un atome d'hydrogène ou un radical alkyle, alkényle d'au moins 3 atomes de carbone non directement branché sur l'insaturation, alkynyle d'au moins 3 atomes de carbone non directement branché sur l'insaturation, cycloalkyle, cycloalkylalkyle, éventuellement substitué par un ou plusieurs substituants,
R11 représente indépendamment un atome d'hydrogène ou un groupe choisi parmi les radicaux alkyle, alkényle, alkynyle, cycloalkyle, cycloalkylalkyle, aralkyle, aryle, hétéroaryle, hétéroaralkyle, hétérocycle saturé ou hétérocycle saturé alkyle, alcoyle, alcoxycarbonyle, carbamoyle, sulfamoyle, éventuellement substitués par un ou plusieurs substituants,
R10 et R11 pouvant former ensemble un cycle contenant 1 ou plusieurs hétéroatomes avec l'azote auquel ils sont rattachés ledit hétérocycle étant éventuellement substitué

et leurs sels physiologiquement acceptables.

L'invention concerne plus particulièrement des composés de formule générale I' :

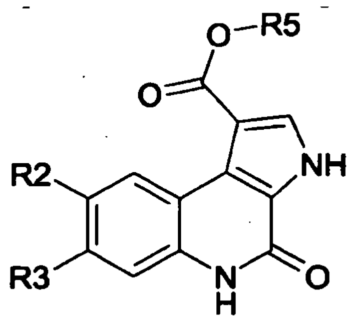

(I')

dans laquelle R2, R3 et R5 sont définis précédemment et ci-après.

Selon un mode particulier de réalisation de l'invention, R2 est choisi parmi les radicaux aryle, alkyle, alkényle, alkynyle, hétéroaryle -SOm-R8, -SO2-(NR7)n-R8 ou -NR7-(Y)n-R8 éventuellement substitué par un ou plusieurs substituants, R7 et R8 étant définis précédemment et ci-après.

De manière avantageuse,

- R3 est un atome d'hydrogène, un halogène, ou un groupe hydroxy ou alkoxy.
- R5 est un radical alkyle, alkényle, alkynyle, cycloalkyle, cycloalkylalkyle, aralkyle, hétérocycle saturé non azoté alkyle, et/ou
- R7 est un hydrogène ou un alkyle, alkényle, alkynyle, cycloalkyle, cycloalkylalkyle, éventuellement substitués par un ou plusieurs substituants, et/ou
- R8 est choisi parmi les radicaux alkyle, cycloalkyle, aryle, hétéroaryle, aralkyle, hétéroaralkyle, hétérocycle saturé ou hétérocycle saturé alkyle, éventuellement substitués par un ou plusieurs substituants, Pour n = 1, R8 peut également être un atome d'hydrogène.

R7 et R8 pouvant former ensemble un cycle ou un bicycle contenant 1 ou plusieurs hétéroatomes avec l'azote auquel ils sont rattachés ledit hétérocycle étant éventuellement substitué par un ou plusieurs groupes alkyle, alkényle, alkynyle, amino, NR10R11, hydroxy, alcoxy, alcoyle, carbamoyle, sulfamoyle, sulfonyle, sulfényle, sulfanyle, cyano, alcoxycarbonyle ou un radical oxo,
R10 et R11 étant définis précédemment.

De manière plus avantageuse,

- R5 est choisi parmi les groupes méthyle, éthyle, propyle, iso-propyle, butyle, iso-butyle, iso-pentyle, allyle, isoprènyle, propargyle, cyclopentyle, cyclohexyle, cyclopropylméthyle, cyclopentylméthyle, cyclohexylméthyle, tétrahydropyran-4-yl-méthyle ou tétrahydrofuran-3-yl-méthyle, et/ou
- R7 est un hydrogène ou choisi parmi les groupes méthyle, éthyle, propyle, iso-propyle, butyle, iso-butyle, allyle, isoprènyle, propargyle cyclopropyle, cyclopentyle, cyclohexyle, cyclopropylméthyle, cyclopentylméthyle, cyclohexylméthyle, tétrahydropyran-4-yl-méthyle, tétrahydrofuran-3-yl-méthyle, hydroxy-2-éthyle, et/ou
- R8 est choisi parmi les groupes méthyle, hydroxy-2-éthyle, amino-2-éthyle, méthylamino-2-éthyle, diméthylamino-2-éthyle, cyanométhyle, carbamoylméthyle, 2-cyanoéthyle, cyclohexyle, cycloheptyle, cyclopropylméthyle, cyclopentylméthyle, cyclohexylméthyle, pipéridin-4-yle, pipéridin-3-yle, pipéridin-2-yle, pipéridin-4-méthyle, pipéridin-3-méthyle, pipéridin-2-méthyle, pyrrolidin-3-yl-méthyle, pyrrolidin-2-yl-méthyle, pyrrolidin-3-yle, azétidin-3-yl-méthyle, tétrahydro-thiopyran-4-yle, tétrahydropyran-4-yle, imidazol-4-yl-méthyle, phényl, o-tolyle, m-tolyle, p-tolyle, 2-fluoro-phényle, 3-fluoro-phényle, 4-fluoro-phényle, 2-chloro-phényle, 3-chloro-phényle, 4-chloro-phényle, 3-hydroxy-phényle, 2-méthoxy-phényle, 3-méthoxy-phényle, 4-méthoxy-phényle, 4-amino-phényle, 4-nitro-phényle, 4-fluoro-2-méthyl-phényle, 3-fluoro-2-méthyl-phényle, 3,4-difluorophényle, 3,5-difluorophényle, 2,6-dichloro-phényle, pyridyle, thiophène, 5-chloro-thiophène ou benzyle,
- R7 et R8 pouvant former avec l'azote auquel ils sont rattachés un cycle 2,3-dihydro-indolyle, pipéridinyle, pipérazinyle, pyrolidinyle ou morpholinyle.

Selon l'invention, les substituants sont avantageusement choisis parmi les halogènes, les groupes hydroxyle, amino (amine primaire, secondaire ou tertiaire), nitro, les radicaux alkyle en C1-C4, les radicaux perfluoroalkyle en C1-C4, alcoxy, perfluoroalcoxy, alcoxycarbonyle, cycloalkyle, carbamoyle, sulfamoyle, sulfonyle, sulfényle, sulfanyle, carbonylamino, aryle, hétéroaryle, cyano ou un radical oxo.

Par halogène, on entend en particulier selon la présente invention les halogènes suivants: F, Cl, Br et I.

Par alkyle, on entend en particulier selon la présente invention les radicaux alkyles linéraires ou ramifiés, en particulier les radicaux alkyles en C1, C2, C3, C4, C5 ou C6, en particulier les radicaux méthyle, éthyle, *n*-propyle, *i*-propyle, *n*-butyle, *i*-butyle ou *t*-butyle. Cette définition s'applique également aux parties alkyles des radicaux alcoxy, alcoxy-carbonyle, carbonylamino, carbonyle, aralkyle ou alkoxycarbonyl-alkyle.

Par alkényle, on entend de préférence selon l'invention une chaîne hydrocarbonée, monovalente, insaturée et comprenant au moins une double liaison, linéaire ou ramifiée, comportant de 2 à 6 atomes de carbone, dont des éléments représentatifs sont par exemple les groupes vinyle, 1-propényle, 2-propényle, isoprenyle, butényle, pentenyle, hexenyle.

Par alkynyle, on entend de préférence selon l'invention une chaîne hydrocarbonée, monovalente, insaturée et comprenant au moins une triple liaison, linéaire ou ramifiée, comportant de 2 à 6 atomes de carbone, dont des éléments représentatifs sont en particulier les radicaux éthynyle, propargyle.

Par cycloalkyle, on entend avantageusement selon l'invention les cycloalkyles en C3-C7, plus particulièrement les radicaux cyclopropyle, cyclobutyle, cyclopentyle, cyclohexyle et cycloheptyle.

Par aryle, on entend de préférence selon l'invention un ou plusieurs cycles aromatiques ayant 6 à 10 atomes de carbone, pouvant être accolés ou fusionnés, en particulier les radicaux phényle, naphtyle. Cette définition s'applique également à la partie aryle des radicaux aralkyles et aryloxy.

Par hétéroaryle, on entend selon l'invention un ou plusieurs cycles aromatiques ayant 5 à 10 atomes, pouvant être accolés ou fusionnés, comprenant dans son cycle au moins un hétéroatome choisi parmi l'azote, l'oxygène ou le soufre, en particulier les radicaux benzimidazolyle, benzofuranyle, isobenzofuranyle, benzoxazolyle, benzothiazolyle, benzothiènyle, chromènyle, isochromènyle, chromanyle, isochromanyle, cinnolinyle, furanyle, imidazolyle, indazolyle, indolyle, isoquinolinyle, isoxazolyle, benzisoxazolyle, isothiazolyle, benzisothiazolyle, oxazolyle, pyrazolyle, pyrazinyle, pyridazinyle, pyridinyle, pyrimidinyle, pyrrolyle, quinazolinyle, quinolinyle, quinoxalinyle, thiazolyle, thiadiazolyle, thiènyle, triazolyle. Cette définition s'applique également à la partie aryle des radicaux aralkyles.

Les radicaux aryles ou hétéroaryles correspondent de préférence aux groupes de formules suivantes,

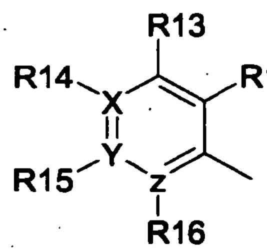

ou

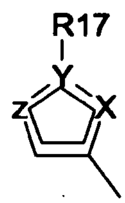

dans lesquelles

X, Y ou Z représentent indépendemment l'un de l'autre un atome de carbone, un atome d'azote, un atome de soufre ou un atome d'oxygène, étant entendu que lorsque X, Y ou Z sont un atome d'azote, de soufre ou d'oxygène, la présence d'un radical R14, R15, R16 ou R17 dépend du respect de la valence dudit atome d'azote ou de soufre préservant l'aromaticité du cycle,

R12 représente un atome d'hydrogène, un halogène ou un groupe alkyle

R13 à R16, représentent, indépendemment l'un de l'autre, un atome d'hydrogène, un atome d'halogène, un groupe hydroxy, un groupe cyano, un radical choisi parmi les groupes alkyles linéaires ou ramifiés, alcoxy, alkylsulfonyle, alkoxycarbonyle, carbamoyle, cycloalkyle, aryle, éventuellement substitués par un ou plusieurs substituants, et le radical -NR7-(Y)n-R8, R7 et R8 étant définis précédemment,

R13 et R14, R14 et R15 ou R15 et R16 pouvant former un cycle aliphatique ou aromatique, avec un ou plusieurs hétéroatomes choisi parmi l'oxygène ou l'azote, éventuellement substitués par un ou plusieurs substituants,

R17 représente un atome d'hydrogène, un radical alkyle, cycloalkyle ou aryle, éventuellement substitués par un ou plusieurs substituants, radical -NR7-(Y)n-R8 ou -NR7-NH-R8, R7 et R8 étant définis précédemment.

Par hétérocycle saturé, on entend avantageusement selon l'invention un cycle en C3-C7 contenant au moins un hétéroatome choisi parmi l'azote, l'oxygène ou le soufre, en particulier les radicaux pyrrolidinyle, pyrrolinyle, pipéridinyle, tétrahydropyridinyle, azépinyle, azétidinyle, indolinyle, isoindolinyle, pipérazinyle, morpholinyle, thiomorpholinyle, tétrahydropyranyle, tétrahydrothiopyranyle, tétrahydrofuranyle, tétrahydrothiényle, perhydropyrrolopyrolyle, quinuclidinyle.

Par sel pharmaceutiquement acceptable on entend de préférence selon l'invention un sel d'acide pharmaceutiquement acceptable, c'est-à-dire avec tout acide non toxique, y compris les acides organiques et inorganiques. De

tels acides incluent l'acide acétique, benzènesulfonique, benzoïque, citrique, éthanesulfonique, fumarique, gluconique, glutamique, bromhydrique, chlorhydrique, lactique, maléique, malique, mandélique, méthanesulfonique, mucique, nitrique, pamoïque, pantothénique, phosphorique, succinique, sulfurique, tartrique et paratoluènesulfonique.

De manière avantageuse, les composés selon l'invention sont choisis parmi les composés des formules II à IX ci-dessous.

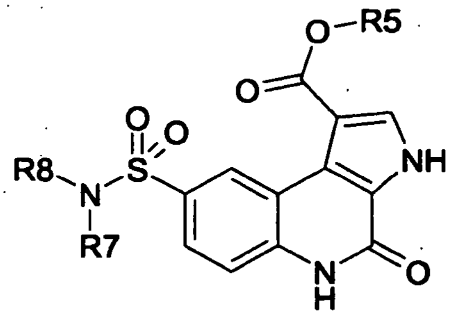
(II),

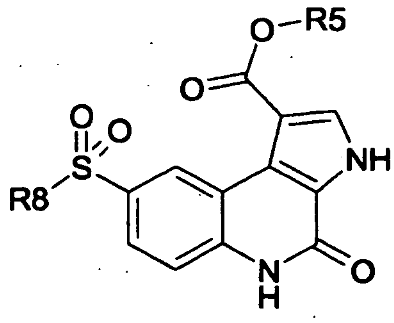
(III),

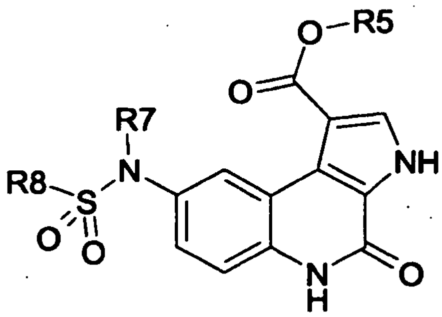
IV),

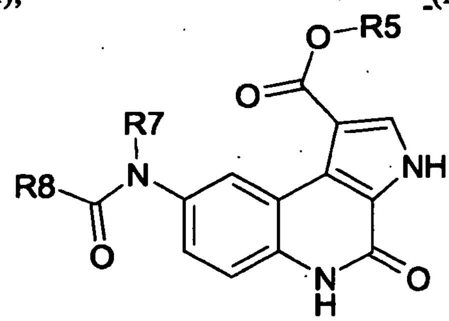
(V),

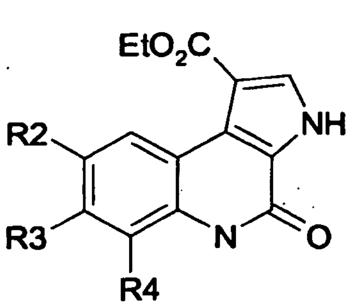
(VI),

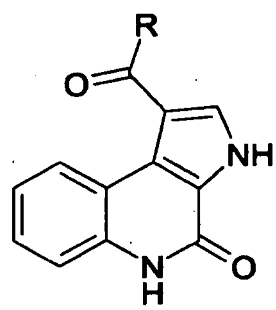
(VII),

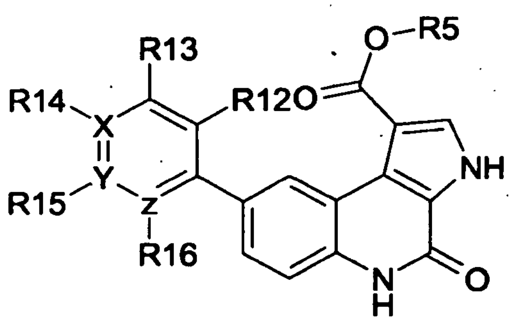
(VIII) ou

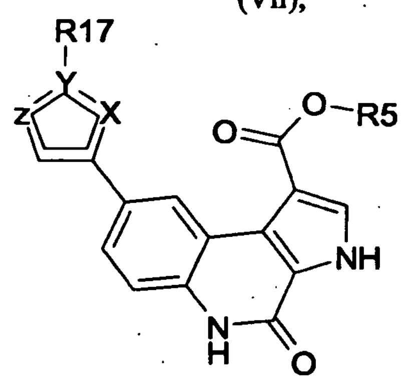
(IX)

R, R2, R3, R4, R5, R7, R8, R12, R13, R14, R15, R16, R17, X, Y et Z étant définis précédemment.

Les composés ci-dessous sont décrits dans la littérature comme intermédiaires de synthèse (Jan Bergman* and Stanley Rehn, Tetrahedron 58 (2002) 9179-9185) sans qu'une quelconque propriété inhibitrice de RET ne soit décrite ni même suggérée.

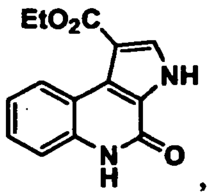 , 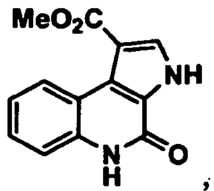 , 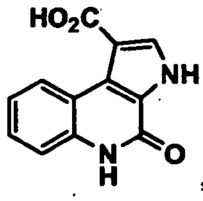 , 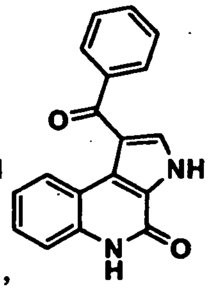

Il est entendu que ces composés ne sont pas partie des composés selon l'invention. En revanche, les compositions pharmaceutiques les comprenant comme leur utilisation en thérapie sont partie de la présente invention.

La présente invention concerne également les composés définis ci-dessus et ci-après pour leur utilisation en thérapie, en particulier pour le traitement des cancers, des maladies inflammatoires ou des affections du système nerveux central.

Elle concerne aussi l'utilisation d'un tel composé pour la préparation d'un médicament destiné au traitement des cancers, des maladies inflammatoires ou du système nerveux central.

L'invention concerne une composition pharmaceutique comprenant un composé tel que défini ci-dessus et ci-après et un véhicule pharmaceutique approprié.

Ces compositions peuvent être formulées pour l'administration aux mammifères, y compris l'homme. La posologie varie selon le traitement et selon l'affection en cause. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Les composés selon l'invention peuvent être employés en thérapie seuls, ou en combinaison avec au moins un autre agent actif. Ces autres agents actifs sont en particulier choisis parmi les actifs appropriés pour le traitement des cancers, des maladies inflammatoires ou des affections du système nerveux central. Il peut s'agir d'adjuvants permettant d'améliorer l'activité des composés selon l'invention, ou encore d'autres actifs connus pour leur emploi dans le traitement des dites affections. De tels agents actifs sont bien connus de l'homme du métier, disponibles dans le commerce ou encore décrits dans des ouvrages de référence comme Le Dictionnaire Vidal, édité avec mises à jours chaque année, en particulier les agents actifs regroupés sous les familles pharmacothérapeutiques « Anti-inflammatoires », « Cancérologie Hématologie » et « Neurologie ».

La présente invention concerne donc également un produit comprenant un composé selon l'invention et un autre agent actif comme produit de combinaison pour une utilisation simultanée, séparée ou étalée dans le temps en thérapie, et en particulier dans le traitement des cancers, des maladies inflammatoires ou des affections du système nerveux central.

L'invention concerne aussi l'utilisation d'un composé défini ci-dessus et ci-après comme inhibiteur de kinase. Cette utilisation peut être faite in vitro, in cellulo ou in vivo. Cette utilisation peut notamment être faite en particulier dans des méthodes de screening afin d'évaluer les propriétés des composés selon l'invention dans différents contextes biologiques.

Les composés selon l'invention peuvent être préparés selon les différents modes de préparation exposés ci-dessous et dans les exemples. Les schémas réactionnels sont donnés en annexe.

La figure 1 donne le schéma de synthèse du coeur tricyclique et accès aux composés de type VII.

La figure 2 donne le schéma d'accès aux composés de type IV, V et VI.

La figure 3 donne le schéma d'accès aux composés de type III ainsi qu'à certains composés de type II.

La figure 4 donne le schéma d'accès à certains composés de type II et VI et VIII.

La figure 5 donne le schéma d'accès aux composés de type IX ainsi qu'à certains composés de type VI et VIII.

L'étape 1 s'effectue en présence d'une base de type di ou tri-alkylamine (typiquement pipéridine) ou de type anionique comme le diisopropylamidure de lithium ou le butyllithium ou de type minéral comme l'hydroxyde de sodium ou le carbonate de potassium selon la nature du groupement R1. La réaction s'effectue dans un solvant aprotique dans le cas des bases anioniques et tout type de solvant dans les autres cas, à une température comprise entre -78°C, pour les bases anioniques, et la température d'ébullition du solvant principalement pour les autres bases. Le milieu réactionnel est souvent acidifié pour permettre l'élimination d'eau et conduire à l'intermédiaire désiré.

L'étape 2 s'effectue avec un isonitrile dérivé de glycine en présence d'une base de typé trialkylamine (plus généralement DBU) ou d'une base anionique de type tert-butylate de potassium ou butyllithium dans un solvant aprotique comme le tétrahydrofurane, le dioxane ou un éther, à une température comprise entre -78°C et la température d'ébullition du solvant.

L'étape 3 s'effectue à l'aide d'un réducteur de la fonction nitro comme le zinc, le fer, ou l'étain par exemple, en présence d'un acide ou l'hydrogène (ou donneur d'hydrogène de type formiate d'ammonium, cyclohexène, cyclohexadiène) en présence d'un catalyseur d'hydrogénation (palladium, platine), dans tout type de solvants et à une température comprise entre -30°C et la température d'ébullition du solvant.

L'étape 5 s'effectue avec un glyoxal, en présence d'une base de type di ou tri-alkylamine, ou de type minéral comme le carbonate de potassium. La réaction s'effectue dans tout type de solvant, à une température comprise entre 0°C et la température d'ébullition du solvant. Le milieu réactionnel est souvent acidifié pour permettre l'élimination d'eau et conduire à l'intermédiaire désiré.

Les produits de départ peuvent être en particulier préparés selon les méthodes décrites dans les brevets suivants (Peng Cho Tang and all US2003/0069451, McNUTT Robert Walton Jr and all WO99/10325, Lanzi chinzia and all WO2004/009083).

L'étape 6 s'effectue avec un dérivé sulfonylméthylisonitrile (typiquement tosylmethylisonitrile) éventuellement substitué par un groupement $R_2$ correspondant à la définition de la formule générale en présence d'une base anionique de type tert-butylate de potassium, une base minérale de type amidure de sodium, ou une base de type trialkylamine (typiquement DBU) au sein d'un solvant inerte tel que le tétrahydrofurane, le dioxane ou un éther à une température comprise entre -20°C et la température d'ébullition du solvant.

L'étape 7 s'effectue en présence de base minérale tel que l'hydroxyde de sodium ou de lithium dans un mélange de solvants composé d'eau et d'un solvant polaire plus généralement un alcool ou un solvant de type cétone (acétone), à une température comprise entre 0°C et le point d'ébullition du solvant.

L'étape 8 s'effectue avec une amine HNR5R6 dans toutes les conditions de couplages de type peptidique connues de l'homme de l'art. Plus généralement, la réaction peut s'effectuer en présence d'agents de couplage de type 1-hydroxybenzotriazole/1-(3-diméthylaminopropyl)-3-ethylcarbodiimide hydrochloride, ou benzotriazole-1-yl-oxy-trispyrrolidinophosphonium hexafluorophosphate dans un solvant inerte tel que le diméthylformamide ou un solvant chloré (chlorure de méthylène, chloroforme par exemple) à une température comprise entre -20°C et la température d'ébullition du solvant.

L'étape 9 s'effectue avec un agent alkylant de type N-X-Succinimide dans lequel X est l'halogène que l'on veut introduire au sein d'un solvant inerte tel que le diméthylformamide ou un solvant chloré (chloroforme ou tétrachlorure de carbone par exemple) à une température comprise entre 20°C et la température d'ébullition du solvant.

L'étape 9a peut aussi s'effectuer avec un dihalogène $Hal_2$ en présence d'un acide (généralement acide acétique) utilisé comme solvant ou dilué dans un solvant inerte à une température comprise entre -40°C et la température d'ébullition du solvant.

L'étape 10 peut être constitué par tout couplage organométallique connu par l'homme de l'art (typiquement les couplages de type Heck, Sonogashira, Stille et Suzuki) et s'effectue dans les conditions usuelles pratiquées par l'homme de l'art.

Typiquement pour l'étape 10a, il s'effectue avec un composé éthylénique $R_1$-$CR_2$=$CHR_3$ dans lequel $R_1$ est soit un groupe électroattracteur (plus généralement un ester, ou un amide) soit un groupe aromatique et $R_2$ et $R_3$ peuvent être n'importe quel groupement à commencer par H en présence d'une base de type trialkylamine (généralement triéthylamine) ou minérale (généralement potassium de carbonate) et d'un composé du palladium d'ordre 0 (palladium tétrakis) ou du couple composé du palladium d'ordre II (généralement acétate de palladium)/ triarylphosphine au sein d'un solvant inerte de type toluène ou diméthylformamide à une température comprise entre -20° et la température d'ébullition du solvant. Il peut également s'éffectuer avec un composé alcynique vrai en présence d'un composé du

palladium d'ordre 0 (palladium tétrakis) ou du couple composé du palladium d'ordre II (généralement acétate de palladium)/ triarylphosphine, d'une base organique (typiquement triéthylamine) ou minérale (typiquement carbonate de potassium) et de iodure de cuivre (I) au sein d'un solvant inerte de type toluène, solvants éthérés (dioxane, diméthoxyéthane, tétrahydrofurane) ou diméthylformamide à une température comprise entre -20° et la température d'ébullition du solvant.

L'étape lOb s'effectue avec un acide boronique (ou ester boronique), un borane ou un stannane en présence d'un composé du palladium d'ordre 0 (palladium tétrakis) ou du couple composé du palladium d'ordre II (généralement acétate de palladium)/ triarylphosphine en présence d'une base minérale (carbonate de potassium par exemple) ou organique (typiquement triéthylamine) au sein d'un solvant inerte de type toluène, solvants éthérés (dioxane, diméthoxyéthane, tétrahydrofurane) ou diméthylformamide à une température comprise entre -20° et la température d'ébullition du solvant.

L'étape 10c s'effectue avec un thiol aromatique ou aliphatique ou l'anion thiolate correspondant en présence d'un catalyseur au palladium ou au cuivre (généralement iodure de cuivre (I)), d'une base minérale (carbonate de potassium par exemple), ou organique (typiquement triéthylamine) et généralement de co-ligands variés au sein d'un solvant inerte de type toluène, solvants éthérés (dioxane, diméthoxyéthane, tétrahydrofurane) ou diméthylformamide à une température comprise entre -20° et la température d'ébullition du solvant.

L'étape 11 s'effectue avec un agent de nitration (typiquement acide nitrique ou nitrate de sodium) au sein d'un acide modéré (typiquement acide acétique) à une température comprise entre -40°C et la température d'ébullition du solvant.

L'étape 12 s'effectue à l'aide d'un réducteur de la fonction nitro comme le zinc, le fer, ou l'étain ar exemple, en présence d'un acide ou l'hydrogène (ou donneur d'hydrogène de type formiate d'ammonium) en présence d'un catalyseur (palladium, platine), dans tout type de solvants et à une température comprise entre -30°C et la température d'ébullition du solvant.

L'étape 13 s'effectue en présence soit d'un chlorure de sulfonyle $R8SO_2Cl$ soit d'un chlorure d'acide R8COCl en présence d'une base de type trialkylamine (typiquement triéthylamine), pyridinique ou une base minérale de type carbonate de potassium au sein d'un solvant inerte tel que le diméthylformamide, acétone ou solvant chloré (chlorure de méthylène, chloroforme par exemple) à une température comprise entre -20°C et la température d'ébullition du solvant.

L'étape 14 s'effectue avec un aldehyde ou une cétone en présence d'un agent réducteur de type borohydrure de sodium ou cyanoborohydrure de sodium au sein d'un alcool ou d'un éther (typiquement le tétrahydrofurane) à une température comprise entre 20°C et la température d'ébullition du solvant.

Alternativement, l'étape 14 peut s'effectuer en deux sous étapes, la première consiste à une condensation d'un agent acylant (typiquement chlorure d'acide, formiate d'éthyle) dans un solvant tel que le diméthylformamide, le toluène ou un solvant halogéné à une température comprise entre -30°C et la température d'ébullition du solvant; la seconde sous étape consiste à une réduction de cet amide intermédiaire par un agent réducteur de type borane dans un solvant inerte de type tétrahydrofurane ou éther à une température comprise entre -30°C et la température d'ébullition du solvant.

L'étape 15 s'effectue avec un agent de chlorosulfonylation (typiquement acide chlorosulfonique mais qui peut-être aussi de l'oléum suivi d'un agent de chlorination de type chlorure de thionyle ou oxychlorure de phosphore) sans solvant ou au sein d'un solvant chloré (chlorure de méthylène ou chloroforme par exemple) à une température comprise entre -20°C et la température d'ébullition des réactifs.

L'étape 16 s'effectue avec une amine HNR7R8 en présence d'une base de type trialkylamine (typiquement triéthylamine), pyridinique ou une base minérale de type carbonate de potassium au sein d'un solvant inerte tel que le diméthylformamide, acétone ou solvant chloré (chlorure de méthylène, chloroforme par exemple) à une température comprise entre -20°C et la température d'ébullition du solvant.

L'étape 17 s'effectue en présence soit d'un système réducteur de type inorganique (typiquement le couple sulfite de sodium, hydrogenosulfate de potassium) dans de l'eau soit d'un métal réducteur de type zinc dans un solvant inerte de type tétrahydrofurane, éther à une température comprise entre 0°C et la température d'ébullition du solvant. Le sulfinate obtenu est alors condensé avec un halogénure d'alkyle dans un mélange composé d'eau et d'un solvant inerte de type éther, cétone ou diméthylformamide à une température comprise entre 0°C et la température d'ébullition du solvant.

Au cours de certaines étapes, les conditions utilisées hydrolysent l'ester. L'estérification (étape 18) s'effectue en présence de chlorure de sulfonyle ou d'un acide anhydre (typiquement HCl) dans un alcool R5OH où R5 est un alkyl à une température comprise entre 0°C et la température d'ébullition du solvant. L'étape 18 (R5 = Me) peut aussi s'effectuer en présence d'un précurseur de diazométhane (typiquement trimethylsilyldiazométhane) dans un mélange de solvants dans lequel les composés sont solubles de type alcohol, diméthylformamide, éther à une température comprise entre 0°C et 50°C. L'étape 18 peut aussi s'effectuer par condensation sur un halogénure d'alkyle ou un agent alkylant (le sulfate de méthyle par exemple) en présence d'une base organique de type amine tertiaire (typiquement triéthylamine) ou minérale (typiquement potassium de carbonate ou fluorure de césium) dans un solvant polaire de type cétone, ether ou diméthylformamide à une température comprise entre 0°C et 100°C.

L'étape 19 s'effectue en présence d'hydrogène (ou donneur d'hydrogène de type formiate d'ammonium, cyclohexène, cyclohexadiène) et d'un catalyseur d'hydrogénation (palladium, platiné), dans tout type de solvants (généralement alcools) en présence ou non d'un acide minéral (acide chlorhydique) ou organique et à une température comprise entre 0°C et la température d'ébullition du solvant et à une pression variant de la pression atmosphérique à quelques bars.

L'étape 20 s'effectue à l'aide d'un oxydant de type oxone, eau oxygénée dans des solvants de type eau, alcools, acétonitrile, ou à l'aide d'oxydant de type acide méta-chloroperbenzoïque dans des solvants chlorés (de type dichlorométhane) ou des solvants inertes de type tétrahydrofurane, dioxane à une température comprise entre 0°C et la température d'ébullition du solvant.

L'étape 21 s'effectue à l'aide de réactifs du type Tert-butoxybis(diméthylamino)méthane ou le diméthyle acétale du *N,N*-diméthylformamide dans des solvants de type dioxane, tétrahydrofurane, diméthylformamide ou sans solvant à une température comprise entre 0°C et la température d'ébullition du solvant.

L'étape 22 s'effectue en présence d'une guanidine dans un solvant du type dioxane ou éthanol, diméthylformamide ou diméthyacétamide à une température comprise entre 20°C et la température d'ébullition du solvant.

L'étape 23 s'effectue en présence d'une hydroxylamine ou d'une hydrazine dans un solvant du type dioxane, éthanol, diméthylformamide ou diméthyacétamide à une température comprise entre 20°C et la température d'ébullition du solvant.

L'étape 24 s'effectue en présence d'un agent d'halogénation du type dibrome, tétrabutylammonium tribromide ou benzyltriméthylammonium dichloroiodate dans un solvant du type dioxane, tétrahydrofurane ou acide acétique à une température comprise entre 0°C et la température d'ébullition du solvant.

L'étape 25 s'effectue en présence d'un thioacétamide ou d'une thiourée dans un solvant du type dioxane, éthanol, diméthylformamide ou diméthyacétamide à une température comprise entre 20°C et la température d'ébullition du solvant.

L'étape 26 s'effectue en présence de tout nucléophile de type amine primaire ou secondaire, alcool, thiol en présence d'une base typiquement une amine tertiaire en utilisant comme solvant soit le nucléophile lui même soit un solvant inerte tel que le diméthylformamide, acétone, acétonitrile ou solvant chloré (chlorure de méthylène, chloroforme par exemple) à une température comprise entre -20°C et la température d'ébullition du solvant.

## Méthodes d'estérification

Au cours de la synthèse des différents composés, une étape d'estérification est parfois nécessaire. Celle-ci a été effectuée de plusieurs manières au cours du temps et selon la nature des composés et de l'ester souhaité. Les différentes conditions expérimentales sont rassemblées ci-après :

### Méthode A :

Dans un tube scellé, à l'acide carboxylique (1 équivalent) en suspension dans l'alcool anhydre correspondant (0.3 moles par litre ou plus dilué) est additionné à 0°C une solution de chlorure de thionyle (10 équivalents) dans l'alcool anhydre correspondant. La solution est agitée à reflux pendant 1 à 5 jours puis refroidie à température ambiante. Le solide, composé principalement de l'acide n'ayant pas réagi, est filtré et le filtrat est évaporé puis purifié par chromatographie sur colonne pour donner l'ester désiré.

### Méthode B :

A l'acide carboxylique (1 équivalent) dissous dans un mélange de solvant approprié (généralement un mélange de diméthylformamide et de méthanol) est additionné une solution 2M dans l'éther diéthylique de triméthylsilyldiazométhane (1 équivalent). La réaction est agitée à température ambiante et suivie très régulièrement par LC/MS. La solution 2M dans l'éther diéthylique de triméthylsilyldiazométhane (généralement 0.5 à 2 equivalent supplémentaires) est additionné jusqu'à disparition de l'acide de départ. De l'acide acétique (5 équivalents) est alors additionnée et les solvants sont évaporés. Le résidu est ensuite purifié par trituration dans l'eau ou par chromatographie sur colonne de silice pour donner l'ester méthylique désiré.

### Méthode C

A l'acide carboxylique (1 équivalent) dissous dans du diméthylformamide sont additionnés de la triéthylamine (5 à 10 équivalents) et le dialkylsulfate approprié (2 équivalents). La solution est agitée à température ambiante pendant 12 heures. Le dialkylsulfate (généralement 3 à 5 équivalents au global) est additionné avec un suivi LC/MS de la réaction. De l'ammoniaque est additionnée et l'agitation est maintenue pour 15 minutes supplémentaires. De l'eau est additionnée

et le produit est extrait avec de l'acétate d'éthyle. Les phases organiques sont séchées sur sulfate de magnésium, filtrées et évaporées et le résidu est purifié par chromatographie sur colonne pour donner l'ester désiré.

Les dialkylsulfates sont soit commerciaux soit préparés suivant la méthode utilisée par SoonY Ko et al., J Org. Chem., 1994, 59, 2570-6.

**Exemple 1 : synthèse des composés**

**4-Oxo-4,5-dihydro-3*H*-pyrrolo[2,3-c]quinoline-1-carborylate d'éthyle (1)**

La synthèse de ce composé est décrite par J.Bergman (Tetrahedron, 2002, 58, pp 9179-9185).

A une solution de 20.3 g (93.6 mmol) de (2-oxo-1,2-dihydro-indol-3-ylidène)-éthanoate d'éthyle dissous dans 340 mL de tétrahydrofurane anhydre sont additionnés 18.3 g (93.6 mmol) d'isonitrile de *p*-toluenesulfonylméthyle suivie d'une solution de 54 mL (93.6 mmol) d'une solution à 20% de *tert*-butoxyde de potassium dans le tétrahydrofurane dans 700 mL de tétrahydrofurane anhydre (temps d'addition 90 minutes). Le mélange réactionnel est porté 1 heure au reflux puis le solide est filtré. Le filtrat est versé dans un mélange de glace et d'eau (environ IL) et la solution est acidifiée à l'aide d'acide acétique jusqu'à un pH de 4. Les solvants sont concentrés par évaporation sous vide puis le solide est filtré puis recristallisé dans l'acide acétique pour conduire à 11.05 g (37%) de 4-oxo-4,5-dihydro-3*H*-pyrrolo[2,3-c]quinolino-1-carboxylate d'éthyle cocristallisé avec un équivalent d'acide acétique sous forme d'un solide blanc.

***LCMS(IE, m/z)** : (M+1) 256.89*

***RMN$^1$H** : $\delta_H$ ppm 250 MHz, DMSO*

*12.97 (1H, sl, NH), 11.66 (1H, s, NH), 9.22 (1H, d, $CH_{arom}$), 7.98 (1H, s, $CH_{arom}$), 7.43-7.35 (2H, m, $2xCH_{arom}$), 7.26-7.17 (1H, m, $CH_{arom}$), 4.30 (2H, q, $CH_2$, 1.34 (3H, t, $CH_3$).*

A une solution de 24 g (180 mmol) de 1,3-dihydro-indol-2-one dissous dans 350 mL d'éthanol sont additionnés 73.5 mL (360 mmol) d'une solution à 50% de glyoxalate d'éthyle dans le toluène puis 3.6 mL (36 mmol) de pipéridine et 4.2 mL (72 mmol) d'acide acétique. Le mélange réactionnel est agité à 70°C pendant 20 heures puis concentré au quart par évaporation des solvants sous vide. Le solide est filtré pour conduire à 18.3 g (47%) de (2-oxo-1,2-dihydro-indol-3-ylidène)-éthanoate d'éthyle sous forme d'un solide orangé. Le filtrat est évaporé puis recristallisé dans l'éthanol pour conduire à 2.7 g (7%) supplémentaires de (2-oxo-1,2-dihydro-indol-3-ylidène)-éthanoate d'éthyle sous forme d'un solide orangé.

4-Oxo-8-phénylsulfamoyl-4,5-dibydro-3*H*-pyrrolo[2,3-*c*]quinoline-1-carboxylate d'éthyle (2)

A 27 mg (0.07 mmol) d'acide 4-oxo-8-phénylsulfamoyl-4,5-dihydro-3*H*-pyrrolo[2,3-*c*]quinoline-1-carboxylique en suspension dans 0.5 mL d'éthanol anhydre, est additionnée goutte à goutte une solution de 20 μL (0.28 mmol) de chlorure de thionyle diluée dans 0.5 mL d'éthanol anhydre. La solution est agitée à reflux pendant 72 heures puis refroidie à température ambiante. Le solide est filtré pour conduire à 3.5 mg (12%) de 4-oxo-8-phénylsulfamoyl-4,5-dihydro-3*H*-pyrrolo[2,3-*c*]quinoline-1-carboxylate d'éthyle sous la forme d'un solide blanc.

***LCMS(IE, m/z)** : (M+1) 411.91*

***RMN$^1$H :** $\delta_H$ ppm 250 MHz, DMSO*

*13.18 (1H, sl, NH), 12.05 (1H, s, NH), 10.26 (1H, s, NH), 9.85 (1H, d, $CH_{arom}$), 8.04 (1H, d, $CH_{arom}$), 7.73 (1H, dd, $CH_{arom}$), 7.47 (1H, d, $CH_{arom}$), 7.22-7.10 (4H, m, $4xCH_{arom}$), 7.00-6.92 (1H, m, $CH_{arom}$), 4.35 (2H, q, $CH_2$), 1.34 (3H, t, $CH_3$).*

A 60 mg (0.18 mmol) d'acide 8-chlorosulfonyl-4-oxo-4,5-dihydro-3*H*-pyrrolo[2,3-*c*]quinoline-1-carboxylique en suspension dans 4 mL de dichlorométhane anhydre et 1 mL de diméthylformamide anhydre, sont ajoutés 30 μL (0.37 mmol) de pyridine et 20 μL (0.22 mmol) d'aniline. Le milieu réactionnel est agité 5 heures à température ambiante puis dilué avec une solution aqueuse à 10% de carbonate de potassium et du dichlorométhane. Les phases sont séparées et la phase aqueuse est acidifiée avec de l'acide acétique jusqu'à un pH de 4. Le produit est extrait avec du dichlorométhane. La phase organique est séchée sur sulfate de magnésium, filtrée puis évaporée. Le résidu est dilué avec de l'eau (5 mL) puis le solide est filtré et séché sous vide pour conduire à 30 mg (42%) d'acide 4-oxo-8-phénylsulfamoyl-4,5-dihydro-3*H*-pyrrolo[2,3-*c*]quinoline-1-carboxylique sous forme d'un solide beige.

A 106 μL (1.65 mmol) d'acide chlorosulfonique refroidi à 0°C par un bain de glace, sont additionnés sur 90 minutes 85 mg (0.33 mmol) de 4-oxo-4,5-dihydro-3*H*-pyrrolo[2,3-*c*]quinoline-1-carboxylate d'éthyle tel que préparé par la synthèse 1 précédente. Après la fin de l'addition, la solution est agitée pendant 1 heure à température ambiante puis 3 heures à 70°C. Le mélange réactionnel est refroidi à température ambiante puis versé dans de l'eau glacé (20 mL). Le mélange est agité 15 minutes puis le solide est filtré, repris dans un mélange méthanol/toluène. Les solvants sont évaporés pour conduire à 64 mg (60%) d'acide 8-chlorosulfonyl-4-oxo-4,5-dihydro-3*H*-pyrrolo[2,3-*c*]quinoline-1-carboxylique sous forme d'un solide beige.

**8-(Méthyl-phénylsulfamoyl)-4-oxo-4,5-dihydro-3*H*-pyrrolo[2,3-*c*]quinolinc-1-carboxylate d'éthyle (3)**

Ce composé a été synthétisé selon la synthèse 1, à partir de 265 mg (0.88 mmol) de 5-(méthylphénylsulfamoyl)-1,3-dihydro-indol-2-one pour donner après recristallisation dans l'acide acétique, 15 mg (2% sur deux étapes) de 8-(méthyl-phénylsulfamoyl)-4-oxo-4,5-dihydro-3*H*-pyrrolo[2,3-*c*]quinoline-1-carboxylate d'éthyle sous la forme d'un solide gris.
***LCMS(IE, m/z) :*** *(M+1) 426.28*
***RMN$^1$H :*** *$\delta_H$ ppm 250 MHz, DMSO*
*13.21 (1H, sl, NH), 12.10 (1H, s, NH), 9.64 (1H, s, $CH_{arom}$), 8.05 (1H, s, $CH_{arom}$), 7.51-7.19 (5H, m, 5x$CH_{arom}$), 7.18-7.06 (2H, m, 2x$CH_{arom}$), 4.26 (2H, q, $CH_2$), 3.17 (3H, s, $CH_3$), 1.32 (3H, t, $CH_3$).*

A 500 mg (2.16 mmol) de chlorure de 2-oxo-2,3-dihydro-1*H*-indole sulfonyle en suspension dans 7 mL de dichlorométhane anhydre, sont ajoutés 350 μL (4.32 mmol) de pyridine et 270 μL (2.48 mmol) de *N*-méthylaniline. Le milieu réactionnel est agité 4 heures à température ambiante puis dilué dans l'eau (20 mL). Le produit est extrait dans du dichlorométhane (3x20 mL). Les phases organiques sont réunies, séchées sur sulfate de magnésium, filtrées puis évaporées. Le résidu est purifié par chromatographie sur silice (éluant dichlorométhane/acétate d'éthyle 8/2) pour conduire à 273 mg (42%) de 5-(méthyl-phénylsulfamoyl)-1,3-dihydro-indol-2-one sous la forme d'un solide légèrement rosé.

Le chlorure de 2-oxo-2,3-dihydro-1*H*-indole sulfonyle a été synthétisé par Peng Cho Tang (US 2003/0069421). Le composé a été préparé selon ce protocole à partir de 2.5 g (18.8 mmol) de 1,3-dihydro-indol-2-one pour conduire à 3.1 g (71%) de chlorure de 2-oxo-2,3-dihydro-1*H*-indole sulfonyle sous la forme d'un solide beige.

**4-Oxo-8-*o*-tolylsulfamoyl-4,5-dihydro-3*H*-pyrrolo[2,3-c]quinoline-1-carboxylate d'éthyle (4)**

Ce composé est préparé selon la synthèse 25, à partir de 150 mg (0.46 mmol) d'acide 8-chlorosulfonyl-4-oxo-4,5-dihydro-3*H*-pyrrolo[2, 3-*c*]quinoline-1-carboxylique (synthèse 2) et de 59 μL (0.55 mmol) de 2-méthylaniline. Après purification par chromatographie sur silice (éluant dichlorométhane/méthanol 96/4) puis trituration dans le méthanol, 17 mg (9%) de 4-oxo-8-*o*-tolylsulfamoyl-4,5-dihydro-3*H*-pyrrolo[2,3-*c*]quinoline-1-carboxylate d'éthyle sont obtenus sous forme d'un solide blanc.
***LCMS (IE, m/z):*** *(M+1) 425.82*
***RMN$^1$H :*** *$\delta_H$ ppm 400 MHz, DMSO*
*13.17 (1H, sl, NH), 12.04 (1H, s, NH), 9.71 (1H, d, $CH_{arom}$), 9.47 (1H, s, NH), 8.03 (1H, s, $CH_{arom}$), 7.61 (1H, dd, $CH_{arom}$), 7.47 (1H, d, $CH_{arom}$), 7.12-7.00 (4H, m, 4x$CH_{arom}$), 4.30 (2H, q, $CH_2$), 2.00 (3H, s, $CH_3$), 1.33 (3H, t, $CH_3$).*

**4-Oxo-8-*m*-tolylsulfamoyl-4,5-dihydro-3*H*-pyrrolo[2,3-*c*]quinoliao-1-carboaylatc d'éthyle (5)**

Ce composé est préparé selon la synthèse 25, à partir de 150 mg (0.46 mmol) d'acide 8-chlorosuifonyl-4-oxo-4,5-dihydro-3*H*-pyrrolo[2,3-*c*]quinoline-1-carboxylique (synthèse 2) et de 59 μL (0.55 mmol) de 3-méthylaniline. Après purification par chromatographie sur silice (étant dichlorométhane/méthanol 96/4) puis trituration dans l'éther diéthylique, 45 mg (23%) de 4-oxo-8-*m*-tolylsulfamoyl-4,5-dihydro-3*H*-pyrrolo[2,3-*c*]quinoline-1-carboxylate d'éthyle sont obtenus sous forme d'un solide blanc.
***LCMS (IE, m/z)****: (M+1) 425.83*
***RMN$^1$H*** *: $\delta_H$ ppm 400 MHz, DMSO*
*13.17 (1H, sl, NH), 12.03 (1H, s, NH), 10.17 (1H, sl, NH), 9.87 (1H, d, $CH_{arom}$), 8.05 (1H, s, $CH_{arom}$), 7.75 (1H, dd, $CH_{arom}$), 7.49 (1H, d, $CH_{arom}$), 7.07-7.00. (2H, m, 2x$CH_{arom}$), 6.92 (1H, dl, $CH_{arom}$), 6.77 (1H, dl, $CH_{arom}$), 4.36 (2H, q, $CH_2$), 2.17 (3H, s, $CH_3$), 1.37 (3H, t, $CH_3$).*

**4-Oxo-8-*p*-tolylsulfamoyl-4,5-dihydro-3*H*-pyrrolo[2,3-*c*]quinoline-1-carboxylate d'éthyle (6)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 61 μL (0.55 mmol) de 4-méthylaniline. Après purification par chromatographie sur silice (éluant dichlorométhane/méthanol 95/5) puis trituration dans l'éther diéthylique, 40 mg (20%) de 4-oxo-8-*p*-tolylsulfamoyl-4,5-dihydro-3*H*-pyrrolo[2,3-*c*]quinoline-1-carboxylate d'éthyle sont obtenus sous forme d'un solide blanc.
***LCMS (IE, m/z)****: (M+1) 425.78*
***RMN$^1$H*** *: $\delta_H$ ppm 400 MHz, DMSO*
*13.13 (1H, sl, NH), 12.01 (1H. s, NH), 10.06 (1H, sl, NH), 9.79 (1H, d, $CH_{arom}$), 8.03 (1H, s, $CH_{arom}$), 7.70 (1H, dd, $CH_{arom}$), 7.46 (1H, d, $CH_{arom}$), 7.02 (2H, d, 2x$CH_{arom}$), 6.98 (2H, d, 2x$CH_{arom}$), 4.35 (2H, q, $CH_2$), 2.14 (3H, s, $CH_3$), 1.36 (3H, t, $CH_3$).*

**8-(2-Méthoxy-phénylsulfamoyl)-4-oxo-4,5-dihydro-3*H*-pyrrolo[2,3-*c*]quinoline-1-carboxylate d'éthyle (7)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 62 µL (0.55 mmol) de 2-méthoxy-aniline. Après purification par chromatographie sur silice (éluant dichlorométhane/méthanol 95/5) puis trituration dans l'éther diéthylique, 22 mg (11%) de 8-(2-méthoxy-phénylsulfamoyl)-4-oxo-4,5-dihydro-3*H*-pyrrolo[2,3-*c*]quinotine-1-carboxylate d'éthyle sont obtenus sous forme d'un solide blanc.
***LCMS (IE, m/z):*** *(M+1) 441.75*
***RMN¹H :*** *$\delta_H$ ppm 400 MHz, DMSO*
*13.16 (1H, sl, NH), 12.01 (1H, s, NH), 9.72 (1H, d, $CH_{arom}$), 9.24 (1H, sl, NH), 8.03 (1H, s, $CH_{arom}$), 7.70 (1H, dd, $CH_{arom}$), 7.46 (1H. d, $CH_{arom}$), 7.29 (1H, dd, $CH_{arom}$), 7.06 (1H, dt, $CH_{arom}$), 6.86-6.81 (2H, m, $2xCH_{arom}$), 4.32 (2H, q, $CH_2$), 3.44 (3H, s, $CH_4$), 1.35 (3H, t, $CH_3$).*

**8-(3-Méthoxy-phénylsulfamoyl)-4-oxo-4,5-dihydro-3*H*-pyrrolo[2,3-*c*]quinoline-1-carboxylate d'éthyle (8)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 62 µL (0.55 mmol) de 3-méthoxy-aniline. Après purification par chromatographie sur silice (éluant dichlorométhane/méthanol 95/5) puis trituration dans l'éther diéthylique, 29 mg (14%) de 8-(3-méthoxy-phénylsulfamoyl)-4-oxo-4,5-dihydro-3*H*-pyrrolo[2,3-*c*]quinoline-1-carboxylate d'éthyle sont obtenus sous forme d'un solide blanc.
***LCMS (IE, mlz):*** *(M+1) 441.77*
***RMN¹H** : $\delta_H$ ppm 400 MHz, DMSO*
*13.17 (1H, sl, NH), 12.04 (1H, s, NH), 10.26 (1H, sl, NH), 9.87 (1H, d, $Ch_{arom}$), 8.05 (1H, d, $CH_{arom}$), 7.76 (1H, dd, $CH_{arom}$), 7.50 (1H, d, $CH_{arom}$), 7. 07 (1H, t, $CH_{arom}$), 6.75 (1H, t, $CH_{arom}$), 6.72-6.66 (1H, m, $CH_{arom}$), 6.53 (1H, dd, $CH_{arom}$), 4.35 (2H, q, $CH_2$), 3.64 (3H, s, $CH_3$), 1.37 (3H, t, $CH_3$).*

**8-(4-Méthoxy-phénylsulfamoyl)-4-oxo-4,5-dihydro-3*H*-pyrrolo[2,3-c]quinoline-1-carboxylate d'éthyle (9)**

Ce composé est préparé selon la synthèse 25, à partir de 150 mg (0.46 mmol) d'acide 8-chlorosulfonyl-4-oxo-4,5-dihydro-3*H*-pyrrolo[2,3-c]quinoline-1-carboxylique (synthèse 2) et de 64 µL (0.55 mmol) de 4-méthoxy-aniline. Après purification par chromatographie sur silice (éluant dichlorométhane/méthanol/acétonitrile 95/3/2) puis trituration dans l'éther diéthylique, 21 mg (10%) de 8-(4-méthoxy-phénylsulfamoyl)-4-oxo-4,5-dihydro-3*H*-pyrrolo[2,3-*c*]quinoline-1-carboxylate d'éthyle sont obtenus sous forme d'un solide blanc.
***LCMS (IE, m/z):*** *(M+1) 441.83*
***RMN¹H**: $\delta_H$ ppm 400 MHz, DMSO*
*13.16 (1H, sl, NH), 12.02 (1H, s, NH), 9.85 (1H, sl, NH), 9.73 (1H, d, $CH_{arom}$), 8.03 (1H, s, $CH_{arom}$), 7.66 (1H, dd, $CH_{arom}$), 7.47 (1H, d, $CH_{arom}$) 7.02 (2H, d, $2xCH_{arom}$), 6.76 (2H, d, $2xCHQ_{arom}$), 4.33 (2H. q, $CH_2$), 3.63 (3H, s, $CH_3$), 1.36 (3H, t, $CH_3$).*

**8-(3-Chloro-phénylsulfamoyl)-4-oxo-4,5-dihydro-3*H*-pyrrolo[2,3-*c*]quinoline-1-carboxylate d'éthyle (10)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 59 µL (0.55 mmol) de 3-chloro-aniline. Après purification par chromatographie sur silice (éluant dichlorométhane/méthanol 95/5) puis trituration dans l'éther diéthylique, 15 mg (7%) de 8-(3-chloro-phénylsulfamoyl)-4-oxo-4,5-dihydro-3*H*-pyrrolo[2, 3-*c*]quinoline-1-carboxylate d'éthyle sont obtenus sous forme d'un solide blanc.
***LCMS (IE, m/z):*** *(M+1) 445.73-447.74*
***RMN¹H**: $\delta_H$ ppm 400 MHz, DMSO*
*13.18 (1H, sl, NH), 12.05 (1H, s. NH), 10.52 (1H, sl, NH), 9.88 (1H, d, $CH_{arom}$), 8.04 (1H, s, $CH_{arom}$), 7.76 (1H, dd, $CH_{arom}$), 7.51 (1H, d, $CH_{arom}$), 7.23-7.18 (2H, m, $2xCH_{arom}$), 7.08 (1H, dl, $CH_{arom}$), 7.01 (1H, dl, $CH_{arom}$), 4.36 (2H, q, $CH_2$), 1.37 (3H, t, $CH_3$).*

**8-(4-Chloro-phénylsulfamoyl)4-oxo-4,5-dihydro-3*H*-pyrrolo[2,3-*c*]quinoline-1-carboxylate d'éthyle (11)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 70 mg (0.55 mmol) de 4-chloro-aniline. Après purification par chromatographie sur silice (éluant dichlorométhane/méthanol 96/4) puis trituration dans l'éther diéthylique, 15 mg (7%) de 8-(4-chloro-phénylsulfamoyl)-4-oxo-4,5-dihydro-3*H*-pyrrolo[2,3-*c*]quinoline-1-carboxylate d'éthyle

sont obtenus sous forme d'un solide blanc.
***LCMS (IE, m/z):*** *(M+1) 445.72-447.61*
***RMN$^1$H*** *: $\delta_H$ ppm 400 MHz, DMSO*
*13.18 (1H, sl, NH), 12.04 (1H, s, NH), 10.39 (1H, sl, NH), 9.82 (1H, d, $CH_{arom}$), 8.04 (1H, s, $CH_{arom}$), 7.73 (1H, dd, $CH_{arom}$), 7.49 (1H, d, $Ch_{arom}$), 7.25 (2H, d, 2x$CH_{arom}$), 7.14 (2H, d 2x$CH_{arom}$), 4.35 (2H, q, $CH_2$), 1.36 (3H, t, $CH_3$).*

**8-(4-Fluoro-phénylsufamoyl)-4-oxo-4,5-dihydro-3*H*-pyrrolo[2,3-*c*]quinoline-1-carboxylate d'éthyle (12)**

Ce composé est préparé selon la synthèse 25, à partir de 300 mg (0.92 mmol) d'acide 8-chlorosulfonyl-4-oxo-4,5-dihydro-3*H*-pyrrolo[2,3-*c*]quinoline-1-carboxylique (synthèse 2) et de 112 µL (1.1 mmol) de 4-fluoro-aniline. Après purification par chromatographie sur silice (éluant dichlorométhane/méthanol 95/5) puis trituration dans l'eau puis l'éther diéthylique, 45 mg (12%) de 8-(4-fluoro-phénylsulfamoyl)-4-oxo-4,5-dihydro-3*H*-pyrrolo[2,3-*c*]quinoline-1-carboxylate d'éthyle sont obtenus sous forme d'un solide blanc.
***LCMS (IE, m/z)****: (M+1) 429.66*
***RMN$^1$H*** *: $\delta_H$ ppm 400 MHz, DMSO*
*13.17 (1H, sl, NH), 12.04 (1H, s, NH), 10.16 (1H, sl, NH), 9.77 (1H, d, $CH_{arom}$), 8.04 (1H, s, $CH_{arom}$), 7.70 (1H, dd, $CH_{arom}$), 7.48 (1H, d, $CH_{arom}$), 7.16-7.08 (2H, m, 2x$CH_{arom}$), 7.08-7.00 (2H, m, 2x$CH_{arom}$), 4.33 (2H, q, $CH_2$), 1.36 (3H, t, $CH_3$).*

**8-[(4-Fluoro-phényl)-*N*-méthyl-sulfamoyl)-4-oxo-4,5-dihydro-3*H*-pyrrolo[2,3-*c*]quinoline-1-carboxylate d'éthyle (13)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 62 µL (0.55 mmol) de (4-fluoro)-*N*-méthyl-aniline. Après purification par chromatographie sur silice (éluant dichlorométhane/méthanol 95/5) puis trituration dans le méthanol, 25 mg (12%) de 8-[(4-fluoro-phényl)-méthyl-sulfamoyl)-4-oxo-4,5-dihydro-3*H*-pyrrolo[2,3-c]quinoline-1-carboxylate d'éthyle sont obtenus sous forme d'un solide blanc.
***LCMS (IE, m/z):*** *(M+1) 443.8*
***RMN$^1$H*** *: $\delta_H$ ppm 400 MHz, DMSO*
*13.20 (1H, sl, NH), 12.10 (1H, s, NH), 9.59 (1H, d, $Ch_{arom}$), 8.04 (1H, s, $CH_{arom}$) 7.51 (1H, d, $CH_{arom}$), 7.46 (1H, dd, $CH_{arom}$), 7.14 (4H, d, 4x$CH_{arom}$), 4.28 (2H, q, $CH_2$), 3.14 (3H, s, $CH_3$), 1.32 (3H, t, $CH_3$).*

**8-(4-Ethoxycarbonyl-phénylsulfamoyl)-4-oxo-4,5-dihydro-3*H*-pyrrolo[2,3-*c*]quinoline-1-carboxylate d'éthyle (14)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 91 mg (0.55 mmol) de 4-amino-benzoate d'éthyle. Après purification par chromatographie sur silice (éluant dichlorométhane/méthanol 96/4) puis trituration dans le méthanol, 4 mg (2%) de 8-(4-éthoxycarbonyl-phénylsulfamoyl)-4-oxo-4,5-dihydro-3*H*-pyrrolo[2, 3-*c*]quinoline-1-carboxylate d'éthyle sont obtenus sous forme d'un solide blanc.
***LCMS (IE, m/z):*** *(M+1) 483.91*
***RMN$^1$H*** *: $\delta_H$ ppm 400 MHz, DMSO*
*13.19 (1H, sl, NH), 12.06 (1H, s, NH), 10.82 (1H, sl, NH), 9.93 (1H, d, $CH_{arom}$), 8.05 (1H, d, $CH_{arom}$), 7.84-7.76 (3H, m, 3x$CH_{arom}$), 7.51 (1H, d, $CH_{arom}$), 7.28 (2H, d, 2x$CH_{arom}$), 4.37 (2H, q, $CH_2$), 4.22 (2H, q, $CH_2$), 1.38 (3H, t, $CH_3$), 1.25 (3H, t, $CH_3$).*

**8-(3-Hydroxy-phénylsulfamoyl)-4-oxo-4,5-dihydro-3*H*-pyrrolo[2,3-*c*]quinoline-1-carboxylate d'éthyle (15)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 60 mg (0.55 mmol) de 3-hydoxy-aniline. Après purification par chromatographie sur silice (éluant dichlorométhane/méthanol 95/5) puis trituration dans l'éther diéthylique, 22 mg (12%) de 8-(3-hydroxy-phénylsulfamoyl)-4-oxo-4,5-dihydro-3*H*-pyrrolo[2,3-*c*]quinoline-1-arboxylate d'éthyle sont obtenus sous forme d'un solide jaune.
***LCMS (IE, m/z):*** *(M+1) 427.77*
***RMN$^1$H*** *: $\delta_H$ ppm 400 MHz, DMSO*
*13.17 (1H, sl, NH), 12.03 (1H, s, NH), 10.12 (1H, sl, NH), 9.84 (1H, d, $CH_{arom}$), 9.36 (1H, s,OH), 8.04 (1H, d, $CH_{arom}$), 7.73 (1H, dd, $CH_{arom}$), 7.48 (1H, d, $CH_{arom}$), 6.94 (1H, t, $CH_{arom}$), 6.61-6.55 (2H, m, 2x$CH_{arom}$), 6.36 (1H, dd, $CH_{arom}$), 4.36 (2H, q, $CH_2$), 1.37 (3H, t, $CH_3$).*

**8-(3-Fluoro-2-méthyl-phénylsulfamoyl)-4-oxo-4,5-dihydro-3*H*-pyrrolo[2,3-*c*]quinoline-1-carboxylate d'éthyle (16)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 63 μL (0.55 mmol) de 3-fluoro-2-méthylaniline. Après purification par chromatographie sur silice (éluant dichlorométhane/méthanol 96/4) puis trituration dans l'éther diéthylique, 3.5 mg (2%) de 8-(3-fluoro-2-méthyl-phénylsulfainoyl)-4-oxo-4,5-dihydro-3*H*-pyrrolo[2,3-*c*]quinoline-1-carboxylate d'éthyle sont obtenus sous forme d'un solide beige.
***LCMS (IE, m/z):*** *(M+1) 443.74*
***RMN¹H*** *: $\delta_H$ ppm 400 MHz, DMSO*
*13.18 (1H, sl, NH), 12.06 (1H, s, NH), 9.70 (2H, m, $CH_{arom}$ et NH), 8.03 (1H, s, $CH_{arom}$), 7.64 (1H, dd, $Ch_{arom}$), 7.49 (1H, d, $CH_{arom}$), 7.13-7.06 (1H, m, $CH_{arom}$), 6.98 (1H, t, $CH_{arom}$), 6.88 (1H, d, $CH_{arom}$), 4.29 (2H, q, $CH_2$), 1.93 (311, d, $CH_3$), 1.33 (3h, t, $CH_3$).*

**8-(4-Fluoro-2-méthyl-phénylsulfamoyl)-4-oxo-4,5-dihydro-3*H*-pyrrolo[2,3-*c*]quinoline-1-carboxylate d'éthyle (17)**

Ce composé est préparé selon la synthèse 25, à partir de 150 mg (0.46 mmol) d'acide 8-chlorosulfonyl-4-oxo-4,s-dihydro-3*H*-pyrrolo[2,3-*c*]quinoline-1-carboxylique (synthèse 2) et de 61 μL (0.55 mmol) de 4-fluoro-2-méthyl-aniline. Après purification par chromatographie sur gel de silice (dichlorométhane/méthanol 95/5) puis trituration dans l'éther diéthylique, 19 mg (9%) de 8-(4-fluoro-2-méthyl-phénylsulfamoyl)-4-oxo-4,5-dihydro-3*H*-pyrrolo[2,3-*c*]quinoline-1-carboxylate d'éthyle sont obtenus sous la forme d'un solide blanc.
***LCMS(IE, m/z) :*** *(M+1) 443.72*
***RMN¹N*** *: $\delta_H$ ppm 400 MHz, DMSO*
*13.17 (1H, sl, NH), 12.06 (1H, s NH), 9.64 (1H, d, $CH_{arom}$), 9.47 (1H, sl, NH), 8.03 (1H, s, $CH_{arom}$), 7.61 (1H, dd, $CH_{arom}$), 7.49 (1H, d, $CH_{arom}$), 7.04-6.87 (3H, m, $3xCH_{arom}$), 4.28 (2H, q, $CH_2$), 1.96 (3H, s, $CH_3$), 1.32 (3H, t, $CH_3$).*

**8-(3,4-Difluoro-phénylsulfamoyl)-4-oxo-4,5-dihydro-3*H*-pyrrolo[2,3-*c*]quinoline-1-carboxylate d'éthyle (18)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 55 μL (0.55 mmol) de 3,4-difluoro-aniline Après purification par chromatographie sur gel de silice (dichlorométhane/méthanol 95/5) puis trituration dans l'éther diéthylique, 24 mg (12%) de 8-(3,4-difluoro-phénylsulfamoyl)-4-oxo-4,5-dihydro-3*H*-pyrrolo[2,3-*c*]quinoline-1-carboxylate d'éthyle sont obtenus sous la forme d'un solide blanc.
***LCMS(IE, m/z)*** *: (M+1) 447.72*
***RMN¹H*** *: $\delta_H$ ppm 400 MHz, DMSO*
*13.19 (1H, sl, NH), 12.06 (1H, s, NH), 10.42 (1H, sl, NH), 9.80 (1H, d, $CH_{arom}$), 8.04 (1H, s, $CH_{arom}$), 7.76 (1H, dd, $CH_{arom}$), 7.51 (1H, d, $CH_{arom}$), 7.32-7.17 (2H, m, $2xCH_{arom}$), 6.91-6.85 (777: m, $CH_{arom}$), 4.34 (2H, q, $CH_2$), 1.36 (3H, t, $CH_3$).*

**8-(3,5-Difluoro-phénylsulfamoyl)-4-oxo-4,5-dihydro-3*H*-pyrrolo[2,3-*c*]quinoline-1-carboxylate d'éthyle (19)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 55 μL (0.55 mmol) de 3,5-difluoro-aniline. Après purification par chromatographie sur gel de silice (dichorométhane/méthanol 95/5) puis trituration dans l'éther diéthylique, 3 mg (2%) de 8-(3,5-difluoro-phénylsulfamoyl)-4-oxo-4,5-dihydro-3*H*-pyrrolo[2,3-*c*]quinoline-1-carboxylate d'éthyle sont obtenus sous la forme d'un solide blanc.
***LCMS(IE, m/z) :*** *(M+1) 447.72*
***RMN¹H*** *: $\delta_H$ ppm 400 MHz, DMSO*
*13.19 (1H, s aplati, NH), 12.08 (1H, sl, NH), 10.80 (1H, s aplati, NH), 9.94 (1H, d, $CH_{arom}$), 8.05 (1H, s, $CH_{arom}$), 7.82 (1H, dd, $CH_{arom}$), 7.54 (1H, d, $CH_{arom}$), 6.86-6.77 (3H, m, $3xCH_{arom}$), 4.36 (2H, q, $CH_2$), 1.37 (3H, t, $CH_3$).*

**8-(4-Amino-phénylsulfamoyl)-4-oxo-4,5-dihydro-3*H*-pyrrolo[2,3-*c*]quinoline-1-carboxylatc d'éthyle (20)**

Ce composé est préparé selon la synthèse 25, à partir de 150 mg (0.46 mmol) d'acide 8-chlorosulfonyl-4-oxo-4,5-dihydro-3*H*=pyrrolo[2,3-*c*]quinoline-1-carboxylique (synthèse 2) et de 115 mg (0.55 mmol) de *N-tert*-butoxycarbonyl-*para*-phénylènediamine. Après purification par chromatographie sur gel de silice (chloroforme/méthanol/ammoniaque 90/25/4) puis trituration dans l'éther diéthylique, 5 mg (3%) de 8-(4-amino-phénylsulfamoyl)-4-oxo-4,5-dihydro-3*H*-pyrrolo[2,3-*c*]quinoline-1-carboxylate d'éthyle sont obtenus sous la forme d'un solide blanc.
***LCMS(IE, mlz)*** *: (M+1) 426.77*
***RMN¹H*** *: $\delta_H$ ppm 400 MHz, DMSO*
*13.14 (1H, sl, NH), 12.00 (1H, s, NH), 9.71 (1H, d, $CH_{arom}$), 9.43 (1H, s, NH), 8.03 (1H, s, $CH_{arom}$), 7.60 (1H, dd, $CH_{arom}$), 7.45 (1H, d, $CH_{arom}$), 6.72 (2H, d, 2x$CH_{arom}$), 6.36 (2H, d, 2x$CH_{arom}$), 4.90 (2H, sl, $NH_2$), 4.33 (2H, q, $CH_2$), 1.36 (3H, t, $CH_3$).*

**8-(2,3-Dihydro-indole-1-sulfonyl)-4-oxo-4,5-dihydro-3*H*-pyrrolo[2,3-*c*]quinoline-1-carboxylate d'éthyle (21)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 62 µL (0.55 mmol) de 2,3-dihydro-1*H*-indole. Après purification par chromatographie sur silice (éluant dichlorométhane/méthanol 96/4) puis trituration dans le méthanol, 40 mg (20%) de 8-(2,3-dihydro-indole-1-sulfonyl)-4-oxo-4,5-dihydro-3*H*-pyrrolo[2,3-*c*]quinoline-1-carboxylate d'éthyle sont obtenus sous forme d'un solide blanc.
***LCMS (IE, mlz):*** *(M+1) 437.77*
***RMN¹H:*** *$\delta_H$ ppm 400 MHz, DMSO*
*13.18 (1H, sl, NH), 12.06 (1H, s, NH), 9.94 (1H, d, $CH_{arom}$), 8.05 (1H, s, $CH_{arom}$), 7.80 (1H, dd, $CH_{arom}$), 7.57 (1H, d, $CH_{arom}$), 7.49 (1H, d, $CH_{arom}$), 7.15-7.08 (2H, m, 2x$CH_{arom}$), 6.91 (1H, td, $CH_{arom}$), 4.39 (2H, q, $CH_2$), 3.96 (2H, t, $CH_2$), 2.93 (2H, t, $CH_2$), 1.39 (3H, t, $CH_3$).*

**8-(4-Morpholin4-yl-phénylsulfamoyl)-4-oxo-4,5-dihydro-3*H*-pyrrolo[2,3-*c*]quinoline-1-carboxylate d'éthyle (22)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 98 mg (0.55 mmol) de 4-morpholin-4-yl-aniline. Après purification par chromatographie sur silice (éluant dichlorométhane/méthanol 95/5) puis trituration dans le méthanol, 57 mg (25%) de 8-(4-morpholin-4-yl-phénylsulfamoyl)-4-oxo-4,5-dihydro-3*H*-pyrrolo[2,3-*c*]quinoline-1-carboxylate d'éthyle sont obtenus sous forme d'un solide gris.
***LCMS (IE, m/z)****: (M+1) 496,93*
***RMN¹H*** *: $\delta_H$ ppm 400 MHz, DMSO*
*13.16 (1H, sl, NH), 12.02 (1H, s, NH), 9.78 (1H, sl, NH), 9.73 (1H, d, $CH_{arom}$), 8.03 (1H, s, $CH_{arom}$), 7.67 (1H, dd, $CH_{arom}$), 7.47 (1H, d, $CH_{arom}$), 6.96 (2H. d, 2x$CH_{arom}$), 6.76 (2H, d, 2x$CH_{arom}$), 4.34 (2H, q, $CH_2$), 3.68-3.64 (4H, m, 4x$CH_{morp}$), 2.98-2.94 (4H, m, 4x$CH_{morp}$), 1.36 (3H, t, $CH_3$).*

**4-Oxo-8-(pyridin-3-ylsulfamoyl)-4,5-dihydro-3*H*-pyrrolo[2,3-*c*]quinoline-1-carboxylate d'éthyle (23)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-i-carboxylique (synthèse 2) et de 52 mg (0.55 mmol) de 3-aminopyridine. Après purification par chromatographie sur silice (éluant dichlorométhane/méthanol 9/1 puis chloroforme/méthanol/ammoniaque 90/25/4) puis trituration dans l'éther diéthylique, 5 mg (3%) de 4-oxo-8-(pyridin-3-ylsulfamoyl)-4,5-dihydro-3*H*-pyrrolo [2, 3-*c*]quinolino-1-carboxylate d'éthyle sont obtenus sous forme d'un solide jaune.
***LCMS (IE, m/z):*** *(M+1) 412.80*
***RMN¹H*** *: $\delta_H$ ppm 400 MHz, DMSO*
*13.18 (1H, sl, NH), 12.06 (1H, s, NH), 10.58 (1H, sl, NH), 9.83 (1H, d, $CH_{arom}$), 8.32 (1H, d, $CH_{arom}$), 8.22 (1H, d, $CH_{arom}$), 8.03 (1H, d, $CH_{arom}$), 7.76 (1H, dd, $CH_{arom}$), 7.64-7.58 (1H, m, $CH_{arom}$), 7.51 (1H, d, $CH_{arom}$), 7.32-7.26 (1H, m, $CH_{arom}$), 4.34 (2H, q, $CH_2$), 1.36 (3H, t, $CH_3$).*

**8-(Benzyl-méthyl-sulfamoyl)-4-oxo-4,5-dihydro-3*H*-pyrrolo[2,3-*c*]quinoline-1-carboxylate d'éthyle (24)**

Ce composé est préparé selon la synthèse 25, à partir de 150 mg (0.46 mmol) d'acide 8-chlorosulfonyl-4-oxo-4,5-dihydro-3*H*-pyrrolo[2, 3-*c*]quinoline-1-carboxylique (synthèse 2) et de 71 µL (0.55 mmol) de benzyl-méthylamine. Après purification par chromatographie sur silice (éluant dichlorométhane/méthanol 95/5) puis trituration dans le méthanol, 35 mg (17%) de 8-(benxyl-méthyl-sulfamoyl)-4-oxo-4,5-dihydro-3*H*-pyrrolo[2,3-*c*]quinoline-1-carboxylate d'éthyle sont obtenus sous forme d'un solide blanc.
***LCMS (IE, m/z):*** *(M+1) 439.71*
***RMN¹H*** *: $\delta_H$ ppm 400 MHz, DMSO*
*13.22 (1H, sl, NH), 12.10 (1H, s, NH), 9.86 (1H, d, $CH_{arom}$), 8.07 (1H, s, $CH_{arom}$), 7.85 (1H, dd, $CH_{arom}$), 7.61 (1H, d, $CH_{arom}$), 7.39-7.27 (5H, m, $5x_{CHarm}$), 4.32 (2H, q, $CH_2$), 4.15 (2H, s, $CH_2$), 2.58 (3H, s, $CH_3$), 1.33 (3H, t, $CH_3$).*

**8-(Méthyl-phénétyl-sulfamoyl)-4-oxo-4,5-dihydro-3*H*-pyrrolo[2,3-*c*]quinoline-1-carboxylate d'éthyle (25)**

A 115 mg (0.35 mmol) d'acide 8-chlorosulfonyl-4-oxo-4,5-dihydro-3*H*-pyrrolo[2,3-*c*]quinoline-1-carboxylique (synthèse 2) en suspension dans 2 mL de dichlorométhane anhydre et 0.5 mL de diméthylformamide anhydre, sont ajoutés 97 μL (0.70 mmol) de triéthylamine et 61 μL (0.42 mmol) de *N*-méthylphénétylamine. Le milieu réactionnel est agité 5 heures à température ambiante. Les solvants sont évaporés puis le résidu est repris dans 2 mL d'éthanol anhydre. Une solution de 255 μL (3.5 mmol) de chlorure de thionyle dans 1 mL d'éthanol anhydre est alors additionnée lentement. Le milieu réactionnel est agité à 70°C pendant 5 jours puis refroidi à température ambiante. Le solide est filtré puis le filtrat est évaporé. Le résidu est purifié par chromatographie sur silice (éluant dichlorométhane/méthanol 96/4) pour conduire à 63 mg (40%) de 8-(méthyl-phénétyl-sulfamoyl)-4-oxo-4,5-dihydro-3*H*-pyrrolo[2,3-*c*]quinoline-1-carboxylate d'éthyle sous la forme d'un solide beige.
***LCMS(IE, mlz)*** *: (M+1) 453.94*
***RMN¹H*** *: $\delta_H$ ppm 400 MHz, DMSO*
*13.20 (1H, sl, NH), 12.07 (1H, s, NH), 9.80 (1H, d, $CH_{arom}$), 8.05 (1H, d, $CH_{arom}$), 7.76 (1H, dd, $CH_{arom}$), 7.56 (1H, d, $CH_{arom}$), 7.28-7.16 (5H, m, 5x$CH_{arom}$), 4.29 (2H, q, $CH_2$), 3.24 (2H, t, $CH_2$), 2.78 (2H, t, $CH_2$), 2.74 (3H, s, $CH_3$), 1.32 (3H, t, $CH_3$).*

**8-Diméthylsulfamoyl-4-oxo-4,5-dihydro-3*H*-pyrrolo[2,3-*c*]quinoline-1-carboxylate d'éthyle (26)**

Ce composé est préparé selon la synthèse 25, à partir de 115 mg (0.35 mmol) d'acide 8-chlorosulfonyl-4-oxo-4,5-dihydro-3*H*-pyrrolo[2,3-*c*]quinoline-1-carboxylique (synthèse 2), de 147 μL (1.05 mmol) de triéthylamine et de 35 mg (0.42 mmol) de chlorhydrate de diméthylamine. Après purification par chromatographie sur silice (éluant dichlorométhane/méthanol 95/5) puis trituration dans le méthanol, 30 mg (24%) de 8-diméthylsulfamoyl-4-oxo-4,5-dihydro-3*H*-pyrrolo[2,3-*c*]quinoline-1-carboxylate d'éthyle sont obtenus sous forme d'un solide blanc.
***LCMS(IE, m/z)*** *: (M+1) 363.95*
***RMN¹H*** *: $\delta_H$ ppm 400 MHz, DMSO*
*13.25 (1H, sl, NH), 12.10 (1H, s, NH), 9.76 (1H, d, $CH_{arom}$), 8.07 (1H, s, $CH_{arom}$), 7.75 (1H, dd, $CH_{arom}$), 7.60 (1H, d, $CH_{arom}$), 4.33 (2H, q, $CH_2$), 2.64 (6H, s, 2x$CH_3$), 1.35 (3H, t, $CH_3$).*

**8-Méthylsulfamoyl-4-oxo-4,5-dihydro-3*H*-pyrrolo[2,3-*c*]quinoline-1-carboxylate d'éthyle (27)**

Ce composé est préparé selon la synthèse 25, à partir de 115 mg (0.35 mmol) d'acide 8-chlorosulfonyl-4-oxo-4,5-dihydro-3*H*-pyrrolo[2,3-*c*]quinoline-1-carboxylique (synthèse 2), de 147 μL (1.05 mmol) de triéthylamine et de 28 mg (0.42 mmol) de chlorhydrate de méthylamine. Après purification par chromatographie sur silice (éluant dichlorométhane/méthanol 9/1), 13 mg (11%) de 8-méthylsulfamoyl-4-oxo-4,5-dihydro-3*H*-pyrrolo[2,3-*c*]quinoline-1-carboxylate d'éthyle sont obtenus sous forme d'un solide blanc.
***LCMS(IE, m/z)*** *: (M+1) 349.93*
***RMN¹H*** *: $\delta_H$ ppm 400 MHz, DMSO*
*13.18 (1H, sl, NH), 12.05 (1H, s, NH), 9.79 (1H, d, $CH_{arom}$), 8.05 (1H, d, $CH_{arom}$), 7.77 (1H, dd, $CH_{arom}$), 7.56 (1H, d, $CH_{arom}$), 7.32 (1H, q, NH), 4.33 (2H, q, $CH_2$), 2.45 (3H, d, $CH_3$), 1.35 (3H, t, $CH_3$).*

**8-(Morpholine-4-sulfonyl)-4-oxo-4,5-dihydro-3*H*-pyrrolo[2,3-*c*]quinoline-1-carboxylate d'éthyle (28)**

Ce composé est préparé selon la synthèse 25, à partir de 115 mg (0.30 mmol) d'acide 8-chlorosulfonyl-4-oxo-4,5-dihydro-3*H*-pyrrolo[2,3-*c*]quinoline-1-carboxylique (synthèse 2) et de 32 μL (0.36 mmol) de morpholine. Après trituration dans le méthanol chaud, 5 mg (4%) de 8-(morpholine-4-sulfonyl)-4-oxo-4,5-dihydro-3*H*-pyrrolo[2,3-*c*]quinoline-1-carboxylate d'éthyle sont obtenus sous forme d'uri solide gris.
***LCMS(IE, m/z)*** *: (M+1) 405.99*
***RMN¹H*** *: $\delta_H$ ppm 400 MHz, DMSO*
*13.22 (1H, sl, NH), 12.11 (1H, s, NH), 9.77 (1H, d, $CH_{arom}$), 8.07 (1H, d, $CH_{arom}$), 7.74 (1H, dd, $CH_{arom}$), 7.60 (1H, d, $CH_{arom}$), 4.33 (2H, q, $CH_2$), 3.65-3.62 (4H, m, 4x$CH_{morp}$), 2.91-2.88 (4H, m, 4x$CH_{morp}$), 1.35 (3H, t, $CH_3$).*

**8-(2-Ethoxycarbonyl-éthylsulfamoyl)-4-oxo-4,5-dihydro-3*H*-pyrrolo[2,3-*c*]quinoline-1-carboxylate d'éthyle (29)**

Ce composé est préparé selon la synthèse 25, à partir de 115 mg (0.35 mmol) d'acide 8-chlorosulfonyl-4-oxo-4,5-dihydro-3*H*-pyrrolo[2,3-*c*]quinoline-1-carboxylique (synthèse 2), de 147 μL (1.05 mmol) de triéthylamine et de 59 mg (0.42 mmol) de chlorhydrate de 3-aminopropanoate de méthyle. Après purification par chromatographie sur silice (éluant dichlorométhane/méthanol 96/4), 25 mg (18%) de 8-(2-éthoxycarbonyl-éthylsulfamoyl)-4-oxo-4,5-dihydro-

3*H*-pyrrolo[2,3-*c*]quinoline-1-carboxylate d'éthyle sont obtenus sous forme d'un solide gris.
***LCMS(IE, mlz)*** *: (M+1) 435.91*
***RMN¹H*** *: $\delta_H$ ppm 400 MHz, DMSO*
*13.19 (1H, sl, NH), 12.05 (1H, s, NH), 9.79 (1H, d, $CH_{arom}$), 8.06 (1H, s, $CH_{arom}$), 7.78 (1H, dd, $CH_{arom}$), 7.60 (1H, t, NH), 7.55 (1H, d, $CH_{arom}$), 4.33 (2H, q, $CH_2$), 3.99 (2H, q, $CH_2$), 3.06-3.00 (2H, m, $CH_2$), 2.44 (2H, t, $CH_2$), 1.35 (3H, t, $CH_3$), 1.12 (3H, t, $CH_3$).*

**8-Cyclohexylsulfamoyl-4-oxo-4,5-dihydro-3*H*-pyrrolo[2,3-*c*]quinoline-1-carboxylate d'éthyle (30)**

Ce composé est préparé selon la synthèse 25, à partir de 115 mg (0.35 mmol) d'acide 8-chlorosulfonyl-4-oxo-4,5-dihydro-3*H*-pyrrolo[2,3-*c*]quinoline-1-carboxylique (synthèse 2) et de 48 µL (0.42 mmol) de cyclohexylamine. Après purification par chromatographie sur silice (éluant dichlorométhane/méthanol 95/5), 20 mg (14%) de 8-cyclohexylsulfamoyl-4-oxo-4,5-dihydro-3*H*-pyrrolo[2,3-*c*]quinoline-1-carboxylate d'éthyle sont obtenus sous forme d'un solide blanc.
***LCMS(IE, m/z) :*** *(M+1) 418.00*
***RMN¹H*** *: $\delta_H$ ppm 400 MHz, DMSO*
*13.17 (1H, sl, NH), 12.03 (1H, s, NH), 9.80 (1H, s, $CH_{arom}$), 8.05 (1H, s, $CH_{arom}$), 7.80 (1H, d, $CH_{arom}$), 7.55-7.49 (2H, m, $CH_{arom}$ et NH), 4.33 (2H, q, $CH_2$), 3.08-2.98 (1H, m, $CH_{cycla}$) 1.64-1.54 (4H, m, $4xCH_{cyclo}$), 1.45-0.96 (9H, m, $6xCH_{cyclo}$et $CH_3$).*

**8-Cycloheptylsulfamoyl-4-oxo-4,5-dihydro-3*H*-pyrrolo[2,3-*c*]quinoline-1-carboxylate d'éthyle (31)**

Ce composé est préparé selon la synthèse 25, à partir de 150 mg (0.46 mmol) d'acide 8-chlorosulfonyl-4-oxo-4,5-dihydro-3*H*-pyrrolo[2,3-*c*]quinoline-1-carboxylique (synthèse 2) et de 70 µL (0.55 mmol) de cycloheptylamine. Après purification par chromatographie sur gel de silice (dichlorométhane/méthanol 96/4) puis trituration dans l'éther diéthylique, 15 mg (8%) de 8-cycloheptylsulfamoyl-4-oxo-4,5-dihydro-3*H*-pyrrolo[2,3-*c*]quinoline-1-carboxylate d'éthyle sont obtenus sous la forme d'un solide blanc.
***LCMS(IE, m/z) :*** *(M+1) 431.80*
***RMN¹H*** *: $\delta_H$ ppm 400 MHz, DMSO*
*13.16 (1H, sl, NH), 12.02 (1H, s, NH), 9.81 (1H, d, $CH_{arom}$), 8.05 (1H, s, $CH_{arom}$), 7.80 (1H, dd, $CH_{arom}$), 7.53 (1H, d, $CH_{arom}$), 7.50 (1H, d, NH), 4.34 (2H, q, $CH_2$), 3.30-3.18 (1H, m, CH), 1.68-1.59 (2H, m, $2xCH_{cyclohep}$), 1.52-1.32 (11H, m, $CH_3$ et $8xCH_{cyclohep}$), 1.29-1.18 (2H, m, $2xCH_{cyclohep}$).*

**8-(3-Diméthylamino-propylsulfamoyl)-4-oxo-4,5-dihydro-3*H*-pyrrolo[2,3-*c*]quinoline-1-carboxylate d'éthyle (32)**

Ce composé est préparé selon la synthèse 25, à partir de 115 mg (0.35 mmol) d'acide 8-chlorosulfonyl-4-oxo-4,5-dihydro-3*H*-pyrrolo[2,3-*c*]quinoline-1-carboxylique (synthèse 2) et de 53 µL (0.42 mmol) de 3-diméthylaminopropylamine. Après purification par chromatographie sur silice (éluant chloroforme/méthanol/ammoniaque 160/25/4), 15 mg (10%) de 8-(3-diméthylamino-propylsulfamoyl)-4-oxo-4,5-dihydro-3*H*-pyrrolo[2,3-*c*]quinoline-1-carboxylate d'éthyle sont obtenus sous forme d'un solide blanc.
***LCMS(IE, m/z) :*** *(M+1) 420.92*
***RMN¹H*** *: $\delta_H$ ppm 250 MHz, DMSO*
*13.17 (1H, sl, NH), 12.04 (1H, s, NH), 9.79 (1H, d, $CH_{arom}$), 8.05 (1H, s, $CH_{arom}$), 7.79 (1H, dd, $CH_{arom}$), 7.55 (1H, d, $CH_{arom}$), 7.50 (1H, sl, NH), 4.33 (2H, q, $CH_2$), 2.81 (2H, t, $CH_2$), 2.24-2.15 (2H, m, $CH_2$), 2.06 (6H, s, $2xCH_3$), 1.50 (2H, quint, $CH_2$), 1.36 (3H, t, $CH_3$).*

**8-(4-Amino-butylsulfamoyl)-4-oxo-4,5-dihydro-3*H*-pyrrolo[2,3-*c*]quinoline-1-carboxylate d'éthyle (33)**

Ce composé est préparé selon la synthèse 25, à partir de 115 mg (0.35 mmol) d'acide 8-chlorosulfonyl-4-oxo-4,5-dihydro-3*H*-pyrrolo[2,3-*c*]quinoline-1-carboxylique (synthèse 2) et de 71 mg (0.42 mmol) de *N*-(*tert*-butoxycarbonyl)-diaminobutane. Après purification par chromatographie sur silice (éluant chloroforme/méthanol/ammoniaque 90/25/4 puis 65/25/4), 26 mg (18%) de 8-(4-amino-butylsulfamoyl)-4-oxo-4,5-dihydro-3*H*-pyrrolo[2,3-*c*]quinoline-1-carboxylate d'éthyle sont obtenus sous forme d'un solide blanc.
***LCMS (IE, m/z):*** *(M+1) 406.99*
***RMN¹H*** *: $\delta_H$ ppm 400 MHz, DMSO*
*13.22 (1H, sl, NH), 12.05 (1H, sl, NH), 9.80 (1H, d, $CH_{arom}$), 8.07 (1H, s, $CH_{arodm}$), 7.79 (1H, dd, $CH_{arom}$), 7.56 (1H, d, $CH_{arom}$), 7.50 (3H, sl, NH et $NH_2$), 4.34 (2H, q, $CH_2$), 2.81 (2H, t, $CH_2$), 2.75 (2H, t, $CH_2$), 1.59-1.33 (4H, m, $2xCH_2$), 1.36 (3H, t, $CH_3$).*

**8-[(3-Chloro-phényl)-méthyl-sulfamoyl)-4-oxo-4,5-dihydro-3*H*-pyrrolo[2,3-*c*]quinolinc-1-carboxylate d'éthyle (34)**

Ce composé est préparé selon la synthèse 3, à partir de 88 mg (0.21 mmol) de 5-(3-chloro-phényl-méthyl-sulfamoyl)-1,3-dihydro-indol-2-one pour donner après purification par LC/MS préparative, 13 mg (6% sur deux étapes) de 8-[(3-chloro-phényl)-méthyl-sulfamoyl]-4-oxo-4,5-dihydro-3*H*-pyrrolo[2,3-*c*]quinoline-1-carboxylate d'éthyle sous la forme d'un solide blanc.
***LCMS (IE, mlz):*** *(M+1) 459.07*
***RMN¹H*** : $\delta_H$ *ppm 200 MHz, DMSO/*$CDCl_3$
*13.0 (1H, sl, NH), 11.96 (1H, s, NH), 9.78 (1H, s,* $CH_{arom}$*), 7.97 (1H, s,* $CH_{arom}$*), 7.54 (1H, d,* $CH_{arom}$*), 7.41 (1H, dd,* $CH_{arom}$*), 7.27 (3H, m,* $3xCH_{arom}$*), 7. 03 (1H, m,* $CH_{arom}$*), 4.34 (2H, q,* $CH_2$*),* 3.22 *(3H, s,* $CH_3$*), 1.40 (3H, t,* $CH_3$*).*

**4-Oxo-8-phénylméthanesulfonyl-4,5-dihydro-3*H*-pyrrolo[2,3-*c*]quinoline-1-carboxylate d'éthyle (35)**

Ce composé est préparé selon la synthèse 1, à partir de 1.08 g (3.76 mmol) du 5-phénylméthanesulfonyl-1,3-dihydro-indol-2-one pour conduire après recristallisation dans l'acide acétique à 0.41g (27% sur deux étapes) de 4-oxo-8-phénylméthanesulfonyl-4,5-dihydro-3*H*-pyrrolo[2,3-*c*]quinoline-1-carboxylate d'éthyle sous la forme d'un solide blanc.
***LCMS(IE, m/z):*** *(M+1) 410.75*
***RMN¹H*** : $\delta_H$ *ppm 400 MHz, DMSO*
*13.21 (1H, sl, NH), 12.10 (1H, s, NH), 9.69 (1H, d,* $CH_{arom}$*), 8.05 (1H, s,* $CH_{arom}$*), 7.64 (1H, dd,* $CH_{arom}$*), 7.50 (1H, d,* $CH_{arom}$*), 7.31-7.24 (3H, m,* $3xCH_{arom}$*), 7.19-7.15 (2H, m,* $2xCH_{arom}$*), 4.61 (2H, s,* $CH_2$*), 4.33 (2H, q,* $CH_2$*), 1.36 (3H, t,* $CH_3$*).*

Le 5-phénylméthanesulfonyl-1,3-dihydro-indol-2-one a été préparé selon la méthode décrite par Jiong Jack Chen and all (Organic Process Research and Development, 2003, 7, 313-317) à partir de 1 g (4.32 mmol) de chlorure de 2-oxo-2,3-dihydro-1*H*-indole-5-sulfonyle et 0.51 mL (4.32 mmol) de bromométhyl-benzène. Après filtration et lavage à l'acétone, 1.08 g (87%) de 5-phénylméthanesulfonyl-1,3-dihydro-indol-2-one sont obtenus sous la forme d'un solide blanc.

**8-(2,6-Dichloro-phénylméthanesulfonyl)-4-oxo-4,5-dihydro-3*H*-pyrrolo[2,3-*c*]quinoline-1-carboxylate d'éthyle (36)**

Ce composé est préparé selon la synthèse 35, à partir de 0.75 g (3.24 mmol) de chlorure de 2-oxo-2,3-dihydro-1*H*-indole-5-sulfonyle (synthetisé comme à la synthèse 35) et de 0.78 g (3.24 mmol) de 2-bromométhyl-1,3-dichloro-benzène pour conduire après recristallisation dans l'acide acétique à 82 mg (6% après trois étapes) de 8-(2,6-dichloro-phénylméthanesulfonyl)-4-oxo-4,5-dihydro-3*H*-pyrrolo[2,3-*c*]quinoline-1-carboxylate d'éthyle sous la forme d'un solide rose.
***LCMS(IE, m/z) :*** *(M+1) 478.72 - 482.73*
***RMN¹H*** : $\delta_H$ *ppm 400 MHz, DMSO*
*13.13 (1H, s aplati, NH), 12.16 (1H, s, NH), 9.74 (1H, d,* $CH_{arom}$*), 8.06 (1H, s,* $CH_{arom}$*), 7.73 (1H, dd,* $CH_{arom}$*), 7.56 (1H, d,* $CH_{arom}$*), 7.49-7.43 (2H, m,* $2xCH_{arom}$*), 7.37 (1H, dd,* $CH_{arom}$*), 4.88 (2H, s,* $CH_2$*), 4.30 (2H, q,* $CH_2$*), 1.34 (3H, t,* $CH_3$*).*

**B-Benzènesulfonylamino-4-oxo-4,5-dihydro-3*H*-pyrrolo[2,3-*c*]quinoline-1-carboxylate d'éthyle (37)**

A 60 mg (0.22 mmol) de 8-amino-4-oxo-4,5-dihydro-3*H*-pyrrolo[2,3-*c*]quinoline-1-carboxylate d'éthyle (synthèse 64) en suspension dans le diméthylformamide anhydre (1 mL) sont ajoutés 62 µL (0.44 mmol) de triéthylamine puis 31 µL (0.24 mmol) de chlorure de benzènesulfonyle. La solution est agitée pendant 1h à 40°C. Le solvant est évaporé et le brut obtenu est purifié par recristallisation dans le méthanol pour conduire à 51 mg (57%) de 8-benzènesulfonylamino-4-oxo-4,5-dihydro-3*H*-pyrrolo[2,3-*c*]quinoline-1-carboxylate d'éthyle sous la forme d'un solide blanc.
***LCMS(IE, mlz)*** *: (M+1) 412.26*
***RMN¹H*** : $\delta_H$ *ppm 250 MHz, DMSO*
*12.99 (1H, sl, NH), 11.62 (1H, s, NH), 10.15 (1H, s, NH), 8.96 (1H, d,* $CH_{arom}$*), 7.94 (1H, d,* $CH_{arom}$*), 7.76 (2H, d,* $2xCH_{arom}$*), 7.60-7.46 (3H, m,* $3xCH_{arom}$*), 7.24 (1H, d,* $CH_{arom}$*), 7.06 (1H, dd,* $CH_{arom}$*), 4.32 (2H, q,* $CH_2$*), 1.34 (3H, t,* $CH_3$*).*

**8-Méthanesulfonylamino-4-oxo-4,5-dihydro-3*H*-pyrrolo[2,3-*c*]quinoline-1-carboxylate d'éthyle (38)**

Ce composé est préparé selon la synthèse 37, à partir de 50 mg (0.18 mmol) de 8-amino-4-oxo-4,5-dihydro-3*H*-pyrrolo[2,3-*c*]quinoline-1-carboxylate d'éthyle (synthèse 64) et de 17 µL (0.20 mmol) de chlorure de méthanesulfonyle. Après recristallisation dans le méthanol, 29 mg (45%) de 8-méthanesulfonylamino-4-oxo-4,5-dihydro-3*H*-pyrrolo[2,3-*c*]quinoline-1-carboxylate d'éthyle sont obtenus sous la forme d'un solide blanc.
***LCMS(IE, m/z)*** *: (M+1) 350.22*
***RMN<sup>1</sup>H*** *: $\delta_H$ ppm 250 MHz, DMSO*
*13.02 (1H, sl, NH), 11.70 (1H, s, NH), 9.64 (1H, s, NH), 9.08 (1H, s, $CH_{arom}$), 7.98 (1H, d, $CH_{arom}$), 7.38 (1H, d, $CH_{arom}$), 7.26 (1H, dd, $CH_{arom}$), 4.32 (2H, q, $CH_2$), 3.01 (3H, s, $CH_3$), 1.34 (3H, t, $CH_3$).*

**4-Oxo-8-phénylméthanesulfonylamino-4,5-dihydro-3*H*-pyrrolo[2,3-*c*]quinoline-1-carboxylate d'éthyle (39)**

Ce composé est préparé selon la synthèse 37, à partir de 50 mg (0.18 mmol) de 8-amino-4-oxo-4,5-dihydro-3*H*-pyrrolo[2,3-*c*]quinoline-1-carboxylate d'éthyle (synthèse 64) et de 39 mg (0.20 mmol) de chlorure de phénylméthanesulfonyle. Après recristallisation dans le méthanol, 9 mg (11%) de 8-phénylméthanesulfonylamino-4-oxo-4,5-dihydro-3*H*-pyrrolo[2,3-*c*]quinoline-1-carboxylate d'éthyle sont obtenus sous la forme d'un solide beige.
***LCMS(IE, m/z)*** *: (M+1) 426.46*
***RMN<sup>1</sup>H*** *: $\delta_H$ ppm 250 MHz, DMSO*
*13.03 (1H, sl, NH), 11.68 (1H, s, NH),* 9.79 *(1H, s, NH), 9.17 (1H, d, $CH_{arom}$), 8.00 (1H, d, $CH_{arom}$), 7.45-7.32 (6H, m, $6xCH_{arom}$), 7.27 (1H, dd, $CH_{arom}$), 4.50 (2H, s, $CH_2$), 4.34 (2H, q, $CH_2$), 1.35 (3H, t, $CH_3$).*

**8-(4-Chloro-benzènesulfonylamino)-4-oxo-4,5-dihydro-3*H*-pyrrolo[2,3-*c*]quinoline-1-carboxylate d'éthyle (40)**

Ce composé est préparé selon la synthèse 37, à partir de 60 mg (0.22 mmol) de 8-amino-4-oxo-4,5-dihydro-3*H*-pyrrolo[2,3-*c*]quinoline-1-carboxylate d'éthyle (synthèse 64) et de 51 mg (0.24 mmol) de chlorure de 4-chloro-benzènesulfonyle. Après recristallisation dans le méthanol, 25 mg (25%) de 8-(4-chloro-benzènesulfonylamino)-4-oxo-4,5-dihydro-3*H*-pyrrolo[2,3-*c*]quinoline-1-carboxylate d'éthyle sont obtenus sous la forme d'un solide blanc.
***LCMS(IE, m/z)*** *: (M+1) 446.24-448.23*
***RMN<sup>1</sup>H*** *: $\delta_H$ ppm 250 MHz, DMSO*
*13.00 (1H, sl, NH), 11.66 (1H, s, NH), 10.20 (1H, s, NH), 8.92 (1H, d, $CH_{arom}$), 7.95 (1H, s, $CH_{arom}$), 7.73 (2H, d, $2xCH_{arom}$), 7.59 (2H, d, $2xCH_{arom}$), 7.28 (1H, d, $CH_{arom}$), 7.08 (1H, dd, $CH_{arom}$), 4.31 (2H, q, $CH_2$), 1.34 (3H, t, $CH_3$).*

**8-(3-Chloro-benzènesulfonylamino)-4-oxo-4,5-dihydro-3*H*-pyrrolo[2,3-*c*]quinoline-1-carboxylate d'éthyle (41)**

Ce composé est préparé selon la synthèse 37, à partir de 60 mg (0.22 mmol) de 8-amino-4-oxo-4,5-dihydro-3*H*-pyrrolo[2,3-*c*]quinoline-1-carboxylate d'éthyle (synthèse 64) et de 34 µL (0.24 mmol) de chlorure de 3-chloro-benzènesulfonyle. Après recristallisation dans le méthanol puis trituration dans le méthanol chaud, 27 mg (27%) de 8-(3-chloro-benzènesulfonylamino)-4-oxo-4,5-dihydro-3*H*-pyrrolo[2,3-*c*]quinoline-1-carboxylate d'éthyle sont obtenus sous la forme d'un solide blanc.
***LCMS(IE, m/z)*** *: (M+1) 445.86-447.88*
***RMN<sup>1</sup>H*** *: $\delta_H$ ppm 250 MHz, DMSO*
*12.99 (1H, sl, NH), 11.64 (1H, s, NH), 10.26 (1H, s, NH), 8.95 (1H, s, $CH_{arom}$), 7.95 (1H, d, $CH_{arom}$), 7.84 (1H, s, $CH_{arom}$), 7.78-7.62 (2H, m, $2xCH_{arom}$), 7.57-7.48 (1H, m, $CH_{arom}$), 7.29 (1H, d, $CH_{arom}$), 7.09 (1H, dd, $CH_{arom}$), 4.32 (2H, q, $CH_2$), 1.34 (3H, t, $CH_3$).*

**8-(2-Chloro-benzènesulfonylamino)-4-oxo-4,5-dihydro-3*H*-pyrrolo[2,3-*c*]quinoline-1-carboxylate d'éthyle (42)**

Ce composé est préparé selon la synthèse 37, à partir de 60 mg (0.22 mmol) de 8-amino-4-oxo-4,5-dihydro-3*H*-pyrrolo[2,3-*c*]quinoline-1-carboxylate d'éthyle (synthèse 64) et de 33 µL (0.24 mmol) de chlorure de 2-chloro-benzènesulfonyle. Après trituration dans le méthanol, 46 mg (46%) de 8-(2-chloro-benzènesulfonylamino)-4-oxo-4,5-dihydro-3*H*-pyrrolo[2,3-*c*]quinoline-1-carboxylate d'éthyle sont obtenus sous la forme d'un solide blanc.
*L**CMS(IE, m/z)** : (M+1) 446.24-448.23*
***RMN<sup>1</sup>H*** *: $\delta_H$ ppm 250 MHz, DMSO*
*12.98 (1H, sl, NH), 11.61 (1H, s, NH), 10.43 (1H, s, NH), 8.98 (1H, d, $CH_{arom}$), 8.02 (1H, d, $CH_{arom}$), 7.93 (1H, d, $CH_{arom}$), 7.65-7.52 (2H, m, $2xCH_{arom}$), 7.47-7.38 (1H, m, $CH_{arom}$), 7.25 (1H, d, $CH_{arom}$), 7.12 (1H, dd, $CH_{arom}$), 4.33 (2H, q, $CH_2$), 1.36 (3H, t, $CH_3$).*

**8-(4-Fluoro-benzènesulfonylamino)-4-oxo-4,5-dihydro-3*H*-pyrrolo[2,3-*c*]quinoline-1-carboxylate d'éthyle (43)**

A 60 mg (0.20 mmol) de chlorhydrate de 8-amino-4-oxo-4,5-dihydro-3*H*-pyrrolo[2,3-*c*]quinoline-1-carboxylate d'éthyle (synthèse 64) en suspension dans le diméthylformamide anhydre (1 mL) sont ajoutés 82 µL (0.59 mmol) de

triéthylamine. Le mélange est refroidi à 0°C, puis 42 mg (0.21 mmol) de chlorure de 4-fluoro-benzènesulfonyle sont ajoutés. La solution est agitée pendant 1h à 0°C. Le solvant est évaporé et le brut obtenu est purifié par recristallisation dans le méthanol pour conduire à 32 mg (38%) de 8-(4-fluoro-bènzenesulfonylamino)-4-oxo-4,5-dihydro-3*H*-pyrrolo [2,3-*c*]quinoline-1-carboxylate d'éthyle sous la forme d'un solide marron clair.
***LCMS(IE, m/z)*** *: (M+1) 429.88*
***RMN¹H*** *: $\delta_H$ ppm 250 MHz, DMSO*
*12.98 (1H, sl, NH), 11.63 (1H, s, NH), 10.12 (1H, s, NH), 8.92 (1H, d, $CH_{arom}$), 7.94 (1H, d, $CH_{arom}$), 7.84-7.74 (2H, m, 2x$CH_{arom}$), 7.40-7.30 (2H, m, 2x$CH_{arom}$), 7.27 (1H, d, $CH_{arom}$), 7.07 (1H, dd, $CH_{arom}$), 4.31 (2H, q, $CH_2$), 1.34 (3H, t, $CH_3$).*

**8-(3-Fluoro-benzènesulfonylamino)-4-oxo-4,5-dihydro-3*H*-pyrrolo[2,3-*c*]quinoline-1-carboxylate d'éthyle (44)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinoline-1-carboxylate d'éthyle (synthèse 64) et de 29 µL (0.21 mmol) de chlorure de 3-fluoro-benzènesulfonyle. Après recristallisation dans le méthanol puis trituration dans le méthanol chaud, 20 mg (24%) de 8-(3-fluoro-benzènesulfonylamino)-4-oxo-4,5-dihydro-3*H*-pyrrolo[2,3-*c*]quinoline-1-carboxylate d'éthyle sont obtenus sous la forme d'un solide beige.
***LCMS(IE, m/z) :*** *(M+1) 429.92*
***RMN¹H*** *: $\delta_H$ ppm 400 MHz, DMSO*
*12.98 (1H, sl, NH), 11.64 (1H, s, NH), 10.25 (1H, s, NH), 8.94 (1H, d, $CH_{arom}$), 7.94 (1H, d, $CH_{arom}$), 7.64-7.52 (3H, m, 3x$CH_{arom}$), 7.45-7.42 (1H, m, $CH_{arom}$), 7.28 (1H, d, $CH_{arom}$), 7.09 (1H, dd, $CH_{arom}$), 4.31 (2H, q, $CH_2$), 1.34 (3H, t, $CH_3$).*

**8-(2-Fluoro-benzènesulfonylamino)-4-oxo-4,5-dihydro-3*H*-pyrrolo[2,3-*c*]quinoline-1-carboxylate d'éthyle (45)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinoline-1-carboxylate d'éthyle (synthèse 64) et de 28 µL (0.21 mmol) de chlorure de 2-fluoro-benzenesulfonyle. Après recristallisation dans le méthanol, 38 mg (45%) de 8-(2-fluoro-benzènesulfonylamino)4-oxo-4,5-dihydro-3*H*-pyrrolo[2,3-*c*]quinoline-1-carboxylate d'éthyle sont obtenus sous la forme d'un solide marron clair.
***LCMS(IE, m/z) :*** *(M+1) 429.88*
***RMN¹H :*** *$\delta_H$ ppm 400 MHz, DMSO*
*12.97 (1H, sl, NH), 11.62 (1H, s, NH), 10.41 (1H, s, NH), 8.97 (1H, d, $CH_{arom}$), 7.93 (1H, d, $CH_{arom}$), 7.79 (1H, ddd, $CH_{arom}$), 7.67-7.60 (1H, m, $CH_{arom}$), 7.41-7.36 (1H, m, $CH_{arom}$), 7.30-7.24 (2H, m, 2x$CH_{arom}$), 7.12 (1H, dd, $CH_{arom}$), 4.32 (2H, q, $CH_2$), 1.35 (3H, t, $CH_3$).*

**8-(4-Bromo-benzènesulfonylamlno)-4-oxo-4,5-dihydro-3*H*-pyrrolo[2,3-*c*]quinoline-1-carboxylate d'éthyle (46)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinolino-1-carboxylate d'éthyle (synthèse 64) et de 52 mg (0.21 mmol) de chlorure de 4-bromo-benzènesulfonyle. Après recristallisation dans le méthanol, 40 mg (42%) de 8-(4-bromo-benzènesulfonylamino)-4-oxo-4,5-dihydro-3*H*-pyrrolo[2,3-*c*]quinoline-1-carboxylate d'éthyle sont obtenus sous la forme d'un solide marron clair.
***LCMS(IE, m/z):*** *(M+1) 489.85-491.84*
***RMN¹H :*** *$\delta_H$ ppm 250 MHz, DMSO*
*12.98 (1H, sl, NH), 11.64 (1H, s, NH), 10.19 (1H, s, NH), 8.92 (1H, d, $CH_{arom}$), 7.94 (1H, d, $CH_{arom}$), 7.73 (2H, d, 2x$CH_{arom}$), 7.65 (2H, d, 2x$CH_{arom}$), 7.28 (1H, d, $CH_{arom}$), 7.08 (1H, dd, $CH_{arom}$), 4.31 (2H, q, $CH_2$), 1.34 (3H, t, $CH_3$).*

**4-Oxo-8-(4-toluènesulfonylamino)-4,5-dihydro-3*H*-pyrrolo[2,3-*c*]quinoline-1-carboxylate d'éthyle (47)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinoline-1-carboxylate d'éthyle (synthèse 64) et de 41 mg (0.21 mmol) de chlorure de 4-toluènesulfonyle. Après recristallisation dans le méthanol, 23 mg (28%) de 4-oxo-8-(4-toluènesulfonylamino)-4,5-dihydro-3*H*-pyrroto[2,3-*c*]quinoline-1-carboxylate d'éthyle sont obtenus sous la forme d'un solide marron.
***LCMS(IE, m/z) :*** *(M+1) 425.88*
***RMN¹H :*** *$\delta_H$ ppm 400 MHz, DMSO*
*12.97 (1H, sl, NH), 11.60 (1H, s, NH), 10.06 (1H, s, NH), 8.95 (1H, d, $CH_{arom}$), 7.94 (1H, d, $CH_{arom}$), 7.64 (2H, d, 2x$CH_{arom}$), 7.29 (2H, d, 2x$CH_{arom}$), 7.24 (1H, d, $CH_{arom}$), 7.06 (1H, dd, $CH_{arom}$), 4.32 (2H, q, $CH_2$), 2.30 (3H, s, $CH_3$),*

*1.35 (3H, t, $CH_3$).*

**4-Oxo-8-(3-toluènesulfonylamino)-4,5-dihydro-3*H*-pyrrolo[2,3-*c*]quinoline-1-carboxylate d'éthyle (48)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinoline-1-carboxylate d'éthyle (synthèse 64) et de 31 mg (0.21 mmol) de chlorure de 3-toluènesulfonyle. Après recristallisation dans le méthanol, 33 mg (40%) de 4-oxo-8-(3-toluènesulfonylamino)-4,5-dihydro-3*H*-pyrrolo[2,3-*c*]quinoline-1-carboxylate d'éthyle sont obtenus sous la forme d'un solide beige.
***LCMS(IE, m/z)** : (M+1) 425.88*
***RMN$^1$H :** $\delta_H$ ppm 400 MHz, DMSO*
*12.97 (1H, sl, NH), 11.60 (1H, s, NH), 10.10 (1H, s, NH), 8.95 (1H, d, $CH_{arom}$), 7.94 (1H, d, $CH_{arom}$), 7.65 (1H, s, $CH_{arom}$), 7.55-7.50 (1H, m, $CH_{arom}$), 7.39-7.34 (2H, m, $2xCH_{arom}$), 7.25 (1H, d, $CH_{arom}$), 7.08 (1H, dd, $CH_{arom}$), 4.32 (2H, q, $CH_2$), 2.32 (3H, s, $CH_3$), 1.35 (3H, t, $CH_3$).*

**8-(4-*t*ert-Butyl-benzènesulfonylamino)-4-oxo-4,5-dihydro-3*H*-pyrrolo[2,3-*c*]quinoline-1-carboxylate d'éthyle (49)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinoline-1-carboxylate d'éthyle (synthèse 64) et de 55 mg (0.23 mmol) de chlorure de 4-*tert*-butyl-benzènesulfonyle. Après recristallisation dans un mélange acétonitrile/dichlorométhane, 36 mg (40%) de 8-(4-*tert*-butyl-benzènesulfonylamino)-4-oxo-4,5-dihydro-3*H*-pyrrolo[2,3-*c*]quinoline-1-carboxylate d'éthyle sont obtenus sous la forme d'un solide marron clair.
***LCMS(IE, m/z) :** (M+1) 468.03*
***RMN$^1$H** : $\delta_H$ ppm 400 MHz, DMSO*
*12.96 (1H, sl, NH), 11.60 (1H, s, NH), 10.01 (1H, s, NH), 8.94 (1H, d, $CH_{arom}$), 7.94 (1H, d, $CH_{arom}$), 7.69 (2H, d, $2xCH_{arom}$), 7.52 (2H, d, $2xCH_{arom}$), 7.25 (1H, d, $CH_{arom}$), 7.11 (1H, dd, $CH_{arom}$), 4.32 (2H, q, $CH_2$), 1.34 (3H, t, $CH_3$), 1.23 (9H, s, $3xCH_3$).*

**8-(4-Méthoxy-benzènesulfonylamino)-4-oxo-4,5-dihydro-3*H*-pyrrolo[2,3-*c*]quinoline-1-carboxylate d'éthyle (50)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinoline-1-carboxylate d'éthyle (synthèse 64) et de 44 mg (0.21 mmol) de chlorure de 4-méthoxy-benzènesulfonyle. Après recristallisation dans le méthanol, 45 mg (53%) de 8-(4-méthoxy-benzènesulfonylamino)-4-oxo-4,5-dffiydro-3*H*-pyrrolo[2,3-*c*]quinoline-1-carboxylate d'éthyle sont obtenus sous la forme d'un solide marron clair.
***LCMS(IE, m/z) :** (M+1) 441.91*
***RMN$^1$H** : $\delta_H$ ppm 400 MHz, DMSO*
*12.98 (1H, sl, NH), 11.60 (1H, s, NH), 9.98 (1H, s, NH), 8.94 (1H, d, $CH_{arom}$), 7.94 (1H, d, $CH_{arom}$), 7.68 (2H, d, $2xCH_{arom}$), 7.24 (1H, d, $CH_{arom}$),7.06 (1H, dd, $CH_{arom}$), 7.01 (2H, d, $2xCH_{arom}$), 4.32 (2H, q, $CH_2$), 3.76 (3H, s, $CH_3$), 1.34 (3H, t, $CH_3$).*

**8-(3-Méthoxy-benzènesulfonylamino)-4-oxo-4,5-dihydro-3*H*-pyrrolo[2,3-*c*]quinoline-1-carboxylate d'éthyle (51)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinoline-1-carboxylate d'éthyle (synthèse 64) et de 30 µL (0.21 mmol) de chlorure de 3-méthoxy-benzènesulfonyle. Après recristallisation dans le méthanol puis trituration dans le méthanol chaud, 21 mg (25%) de 8-(3-méthoxy-benzènesulfonylamino)-4-oxo-4,5-dihydro-3*H*-pyrrolo[2,3-*c*]quinoline-1-carboxylate d'éthyle sont obtenus sous la forme d'un solide beige.
***LCMS(IE, m/z)** : (M+1) 441.91*
***RMN$^1$H** : $\delta_H$ ppm 400 MHz, DMSO*
*12.98 (1H, sl, NH), 11.61 (1H, s, NH), 10.14 (1H, s, NH), 8.97 (1H, s, $CH_{arom}$), 7.94 (1H, s, $CH_{arom}$), 7.42-7.21 (4H, m, $4xCH_{arom}$), 7.16-7.06 (2H, m, $2xCH_{arom}$), 4.31 (2H, q, $CH_2$), 3.75 (3H, s, $CH_3$), 1.34 (3H, t, $CH_3$).*

**8-(4-Acétylamino-benzènesulfonylamino)-4-oxo-4,5-dihydro-3*H*-pyrrolo[2,3-*c*]quinoline-1-carboxylate d'éthyle ( 52)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinoline-1-carboxylate d'éthyle (synthèse 64) et de 50 mg (0.21 mmol) de chlorure de 4-acétylamino-benzènesulfonyle. Après trituration dans le méthanol chaud, 35 mg (38%) de 8-(4-acétylamino-benzènesulfonylamino)-4-oxo-4,5-dihydro-3*H*-pyrrolo[2,3-*c*]quinoline-1-carboxylate d'éthyle sont obtenus sous la forme d'un solide orange-marron.
***LCMS(IE, m/z)*** *: (M+1) 468.93*
***RMN¹H*** *: $\delta_H$ ppm 400 MHz, DMSO*
*12.97 (1H, sl, NH), 11.60 (1H, s, NH), 10.24 (1H, s, NH), 10.00 (1H, s, NH), 8.93 (1H, d, $CH_{arom}$), 7.94 (1H, d, $CH_{arom}$), 7.70-7.63 (4H, m, 4x$CH_{arom}$), 7.24 (1H, d, $CH_{arom}$), 7.05 (1H, dd, $CH_{arom}$), 4.31 (2H, q, $CH_2$), 2.03 (3H, s, $CH_3$), 1.34 (3H, t, $CH_3$).*

**8-(4-Nitro-benzènesulfonylamino)-4-oxo-4,5-dihydro-3*H*-pyrrolo[2,3-*c*]quinoline-1-carboxylate d'éthyle (53)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinoline-1-carboxylate d'éthyle (synthèse 64) et de 45 mg (0.21 mmol) de chlorure de 4-nitro-benzènesulfonyle. Après purification par préparative LCMS, 5 mg (6%) de 8-(4-nitro-benzènesulfonylamino)-4-oxo-4,5-dihydro-3*H*-pyrrolo[2,3-*c*]quinoline-1-carboxylate d'éthyle sont obtenus sous la forme d'un solide marron clair.
***LCMS(IE, m/z)*** *: (M+1) 456.85*
***RMN¹H:*** *$\delta_H$ ppm 400 MHz, DMSO*
*12.99 (1H, sl, NH), 11.66 (1H, s, NH), 10.43 (1H, sl, NH), 8.88 (1H, d, $CH_{arom}$), 8.35 (2H, d, 2x$CH_{arom}$), 7.98 (2H, d, 2x$CH_{arom}$), 7.94 (1H, s, $CH_{arom}$), 7.30 (1H, d, $CH_{arom}$), 7.11 (1H, dd, $CH_{arom}$), 4.26 (2H, q, $CH_2$), 1.32 (3H, t, $CH_3$).*

**8-(Naphtalène-2-sulfonylamino)-4-oxo-4,5-dihydro-3*H*-pyrrolo[2,3-*c*]quinoline-1-carboxylate d'éthyle (54)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinoline-1-carboxylate d'éthyle (synthèse 64) et de 46 mg (0.21 mmol) de chlorure de naphtalène-2-sulfonyle. Après recristallisation dans le méthanol, 36 mg (40%) de 8-(naphtalène-2-sulfonylamino)-4-oxo-4,5-dihydro-3*H*-pyrrolo[2,3-*c*]quinoline-1-carboxylate d'éthyle sont obtenus sous la forme d'un solide marron.
***LCMS(IE, m/z) :*** *(M+1) 461.92*
***RMN¹H*** *: $\delta_H$ ppm 250 MHz, DMSO*
*12.94 (1H, sl, NH), 11.58 (1H, s, NH), 10.26 (1H, s, NH), 8.98 (1H, d, $CH_{arom}$), 8.45 (1H, s, $CH_{arom}$), 8.05 (2H, d, 2x$CH_{arom}$), 7.97 (1H, d, $CH_{arom}$), 7.91 (1H, d, $CH_{arom}$), 7.80 (1H, dd, $CH_{arom}$), 7.69-7.56 (2H, m, 2x$CH_{arom}$), 7.23 (1H, d, $CH_{arom}$), 7.10 (1H, dd, $CH_{arom}$), 4.26 (2H, q, $CH_2$), 1.31 (3H, t, $CH_3$).*

**8-(4-Acétyl-benzènesulfonylamino)-4-oxo-4,5-dihydro-3*H*-pyrrolo[2,3-*c*]quinoline-1-carboxylate d'éthyle (55)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinoline-1-carboxylate d'éthyle (synthèse 64) et de 45 mg (0.21 mmol) de chlorure de 4-acétyl-benzènesulfonyle. Après recristallisation dans le méthanol et trituration dans le méthanol chaud, 44 mg (49%) de 8-(4-acétyl-benzènesulfonylamino)-4-oxo-4,5-dihydro-3*H*-pyrrolo[2,3-*c*]quinoline-1-carboxylate d'éthyle sont obtenus sous la forme d'un solide marron.
***LCMS(IE, m/z)*** *: (M+1) 453.89*
***RMN¹H :*** *$\delta_H$ ppm 250 MHz, DMSO*
*12.97 (1H, sl, NH), 11.63 (1H, s, NH), 10.30 (1H, s, NH), 8.95 (1H, d, $CH_{arom}$), 8.05 (2H, d, 2x$CH_{arom}$), 7.94 (1H, d, $CH_{arom}$), 7.88 (2H, d, 2x$CH_{arom}$), 7.26 (1H, d, $CH_{arom}$), 7.08 (1H, dd, $CH_{arom}$), 4.29 (2H, q, $CH_2$), 2.56 (3H, s, $CH_3$), 1.33 (3H, t, $CH_3$).*

**4-Oxo-8-(thiophène-2-sulfonylamino)-4,5-dihydro-3*H*-pyrrolo[2,3-*c*]quinoline-1-arboxylate d'éthyle (56)**

Ce composé est préparé selon la synthèse 43, à partir de 60 mg (0.20 mmol) de chlorhydrate de 8-amino-4-oxo-4,5-dihydro-3*H*-pyrrolo[2,3-*c*]quinolino-1-carboxylate d'éthyle (synthèse 64) et de 39 mg (0.21 mmol) de chlorure de thiophène-2-sulfonyle. Après recristallisation dans le méthanol, 17 mg (21%) de 4-oxo-8-(thiophène-2-sulfonylamino)-4,5-dihydro-3*H*-pyrrolo[2,3-*c*]quinoline-1-carboxylate d'éthyle sont obtenus sous la forme d'un solide marron clair.
***LCMS(IE, m/z) :*** *(M+1) 417.84*
***RMN¹H:*** *$\delta_H$ ppm 250 MHz, DMSO*

*12.98 (1H, sl, NH), 11.64 (1H, s, NH), 10.27 (1H, s, NH), 9.01 (1H, sl, $CH_{arom}$), 8.00-7.91 (1H, m, $CH_{arom}$), 7.90-7.80 (1H, m, $CH_{arom}$), 7.60-7.45 (1H, m, $CH_{arom}$), 7.34-7.23 (1H, m, $CH_{arom}$), 7.16-7.02 (2H, m, $2xCH_{arom}$), 4.40-4.23 (2H, m, $CH_2$), 1.41-1.26 (3H, m, $CH_3$).*

**8-(5-Chloro-thiophène-2-sulfonylamino)-4-oxo-4,5-dihydro-3*H*-pyrrolo[2,3-*c*]quinoline-1-carboxylate d'éthyle (57)**

Ce composé est préparé selon la synthèse 45, à partir de 60 mg (0.20 mmol) de chlorhydrate de 8-amino-4-oxo-4,5-dihydro-3*H*-pyrrolo[2,3-*c*]quinoline-1l-carboxylate d'éthyle (synthèse 64) et de 29 μL (0.21 mmol) de chlorure de 5-chloro-thiophène-2-sulfonyle. Après recristallisation dans le méthanol et trituration dans du méthanol chaud, 4 mg (3%) de 8-(5-chloro-thiophène-2-sulfonylamino)-4-oxo-4,5-dihydro-3*H*-pyrrolo[2,3-*c*]quinoline-1-carboxylate d'éthyle sont obtenus sous la forme d'un solide blanc.
***LCMS(IE, m/z)*** *: (M+1) 451.85-453.85*
***RMN¹H :*** *$\delta_H$ ppm 400 MHz, DMSO*
*12.99 (1H, sl, NH), 11.66 (1H, s, NH), 10.40 (1H, sl, NH), 8.98 (1H, s, $CH_{arom}$), 7.96 (1H, s, $CH_{arom}$), 7.38-7.28 (2H, m, $2xCH_{arom}$), 7.17-7.12 (2H, m, $2xCH_{arom}$), 4.31 (2H, q, $CH_2$), 1.34 (3H, t, $CH_3$).*

**8-(Benzènesulfonyl-méthyl-amino)-4-oxo-4,5-dihydro-3*H*-pyrrolo[2,3-*c*]quinoline-1-carboxylate d'éthyle (58)**

A 135 mg de chlorhydrate de 8-méthylamino-4-oxo-4,5-dihydro-3*H*-pyrrolo[2,3-*c*]quinoline-1-carboxylate d'éthyle (synthèse 63) contaminés par 30% de 8-amino-4-oxo-4,5-dihydro-3*H*-pyrrolo[2,3-*c*]quinoline-1-carboxylate d'éthyle en suspension dans le diméthylformamide anhydre (2 mL) sont ajoutés 177 μL (1.27 mmol) de triéthylamine et le mélange est refroidi à 0°C, puis 56 μL (0.44 mmol) de chlorure de benzènesulfonyle sont ajoutés. La solution est agitée pendant 1h à 0°C. Le solvant est évaporé et le brut obtenu est purifié par chromatographie sur silice (éluant dichlorométhane/méthanol/acétonitrile 96/2/2) pour conduire après trituration dans l'éther diéthylique à 47 mg (37%) de 8-(benzènesulfonyl-méthyl-amino)-4-oxo-4,5-dihydro-3*H*-pyrrolo[2,3-*c*]quinoline-1-carboxylate d'éthyle sous la forme d'un solide blanc.
***LCMS(IE, m/z)*** *: (M+1) 425.88*
***RMN¹H*** *: $\delta_H$ ppm 400 MHz, DMSO*
*13.04 (1H, sl, NH), 11.77 (1H, s, NH), 8.95 (1H, d, $CH_{arom}$), 7.96 (1H, s, $CH_{arom}$), 7.77-7.65 (1H, m, $CH_{arom}$), 7.61-7.51 (4H, m, $4xCH_{arom}$), 7.35 (1H, d, $CH_{arom}$), 7.13 (1H, dd, $CH_{arom}$), 4.23 (2H, q, $CH_2$), 3.19 (3H, s, $CH_3$), 1.31 (3H, t, $CH_3$).*

**8-(Méthanesulfonyl-méthyl-amino)-4-oxo-4,5-dihydro-3*H*-pyrrolo[2,3-*c*]quinoline-1-carboxylate d'éthyle (59)**

Ce composé est préparé selon la synthèse 58, à partir de 130 mg de chlorhydrate de 8-méthylamino-4-oxo-4,5-dihydro-3*H*-pyrrolo[2,3-*c*]quinoline-1-carboxylate d'éthyle (synthèse 63) contaminés par 30% de 8-amino4-oxo-4,5-dihydro-3*H*-pyrrolo[2,3-*c*]quinoline-1-carboxylate d'éthyle auquel est ajouté 33 μL (0.42 mmol) de chlorure de méthanesulfonyle. Après purification par chromatographie sur silice (éluant dichlorométhane/méthanol/acétonitrile 94/3/3), 32 mg (31%) de 8-(méthanesulfonyl-méthyl-amino)-4-oxo-4,5-dihydro-3*H*-pyrrolo[2,3-*c*]quinoline-1-carboxylate d'éthyle sont obtenus sous la forme d'un solide blanc.
***LCMS(IE, m/z) :*** *(M+1) 363.79*
***RMN¹H*** *: $\delta_H$ ppm 400 MHz, DMSO*
*13.07 (1H, sl, NH), 11.77 (1H, sl, NH), 9.31 (1H, d, $CH_{arom}$), 8.02 (1H, s, $CH_{arom}$), 7.47 (1H, dd, $CH_{arom}$), 7.42 (1H, d, $CH_{arom}$), 4.32 (2H, q, $CH_2$), 3.28 (3H, s, $CH_3$), 3.02 (3H, s, $CH_3$), 1.35 (3H, t, $CH_3$).*

**8-Benzoylamino-4-oxo-4,5-dihydro-3*H*-pyrrolo[2,3-*c*]quinoline-1-carboxylate d'éthyle (60)**

A 50 mg (0.18 mmol) de 8-amino-4-oxo-4,5-dihydro-3*H*-pyrrolo[2,3-*c*]quinoline-1-carboxylate d'éthyle (synthèse 64) en suspension dans le diméthylformamide anhydre (1 mL) sont ajoutés 52 μL (0.37 mmol) de triéthylamine puis 24 μL (0.20 mmol) de chlorure de benzoyle. La solution est agitée pendant 2h30 à 40°C. Le solvant est évaporé et le résidu obtenu est purifié par recristallisation dans le méthanol pour conduire à 36 mg (52%) de 8-benzoylamino-4-oxo-4,5-dihydro-3*H*-pyrrolo[2,3-*c*]quinoline-1-carboxylate d'éthyle sous la forme d'un solide blanc.
***LCMS(IE, m/z) :*** *(M+1) 376.29*
***RMN¹H:*** *$\delta_H$ ppm 250 MHz, DMSO*
*13.02 (1H, sl, NH), 11.70 (1H, s, NH), 10.37 (1H, s, NH), 9.39 (1H, d, $CH_{arom}$), 8.06-7.95 (3H, m, $3xCH_{arom}$), 7.73-7.49 (4H, m, $4xCH_{arom}$), 7.40 (1H, d, $CH_{arom}$), 4.32 (2H, q, $CH_2$), 1.34 (3H, t, $CH_3$).*

**8-Acétylamine-4-oxo-4,5-dihydro-3*H*-pyrrolo[2,3-*c*]quinolinc-1-carboxylate d'éthyle (61)**

A 72 mg (0.24 mmol) de 8-nitro-4-oxo-4,5-dihydro-3*H*-pyrrolo[2,3-*c*]quinoline-1-carboxylate d'éthyle (synthèse 66) dissous dans 4 mL d'acide acétique sont additionnés 134 mg (2.4 mmol) de fer. Le mélange est porté à reflux pendant 1h. La solution est filtrée à chaud et le solide est rincé avec un minimum d'acide acétique chaud. Le filtrat est évaporé puis le résidu (mélange de 8-acétylamino-4-oxo-4,5-dihydro-3*H*-pyrrolo[2,3-*c*]quinoline-1-carboxylate d'éthyle et de 8-amino-4-oxo-4,5-dihydro-3*H*-pyrrolo[2,3-*c*]quinoline-1-carboxylate d'éthyle 85/15) est purifié par préparative LCMS pour donner 4 mg (5 %) de 8-acétylamino-4-oxo-4,5-dihydro-3*H*-pyrrolo[2,3-*c*]quinoline-1-carboxylate d'éthyle sous la forme d'un solide gris.
***LCMS(IE, m/z)** : (M+1) 314.29*
***RMN$^1$H :** $\delta_H$ ppm 250 MHz, DMSO*
*12.96 (1H, sl, NH), 11.62 (1H, s, NH), 9.98 (1H, s, NH), 9.15 (1H, d, $CH_{arom}$), 7.96 (1H, s, $CH_{arom}$), 7.71 (1H, dd, $CH_{arom}$), 7.32 (1H, d, $CH_{arom}$), 4.32 (2H, q, $CH_2$), 2.05 (3H, s, $CH_3$), 1.34 (3H, t, $CH_3$).*

**8-Diméthylamino-4-oxo-4,5-dibydro-3*H*-pyrrolo[2,3-*c*]quinolinc-1-carboxylate d'éthyle (62)**

A 100 mg (0.37 mmol) de 8-amino-4-oxo-4,5-dihydro-3*H*-pyrrolo[2,3-*c*]quinoline-1-carboxylate d'éthyle (synthèse 64) en suspension dans 1.2 ml d'éthanol sont ajoutés 33 μL (0.41 mmol) d'une solution aqueuse à 37% de formaldéhyde, 20 μL (0.35 mmol) d'acide acétique et 26 mg (0.41 mmol) de cyanoborohydrure de sodium. Le mélange est agité 6 heures à température ambiante puis 33 μL (0.41 mmol) d'une solution aqueuse à 37% de formaldéhyde, 20 μL (0.35 mmol) d'acide acétique et 7 mg (0.11 mmol) de cyanoborohydrure de sodium sont additionnés de nouveau. La réaction est agitée une nuit à température ambiante. Les solvants sont évaporés et le résidu obtenu est purifié par préparative LCMS pour donner 5 mg (5%) de 8-diméthylamine-4-oxo-4,5-dihydro-3*H*-pyrrolo[2,3-*c*]quinoline-1-carboxylate d'éthyle sous la forme d'un solide blanc.
***LCMS(IE, m/z) :** (M+1) 299.95*
***RMN$^1$H** : $\delta_H$ ppm 400 MHz, DMSO*
*12.85 (1H, sl, NH), 11.37 (1H, s, NH), 8.72 (1H, d, $CH_{arom}$), 7.93 (1H, s, $CH_{arom}$), 7.26 (1H, d, $CH_{arom}$), 6.96 (1H, dd, $CH_{arom}$), 4.29 (2H, q, $CH_2$), 2.92 (6H, s, $2xCH_3$), 1.33 (3H, t, $CH_3$).*

**Chlorhydrate de 8-méthylamino-4-oxo-4,5-dihydro-3*H*-pyrrolo[2,3-*c*]quinoline-1-carboxylate d'éthyle (63)**

A une solution de 45 mg (0.15 mmol) de 8-formylamino-4-oxo-4,5-dihydro-3*H*-pyrrolo[2,3-*c*]quinoline-1-carboxylate d'éthyle dans 0.5 mL de tétrahydrofurane anhydre sont additionnés 0.41 mL (0.41 mmol) d'une solution 1M d'un complexe de borane-tétmthydrofurane dans du tétrahydrofurane. Le mélange réactionnel est agité une nuit à température ambiante puis 8 heures à reflux avant l'addition supplémentaire de 0.3 mL d'une solution 1M d'un complexe de borane-tétrathydrofurane dans du tétrahydrofurane. La réaction est ensuite agitée 3 heures à reflux. Le solvant est évaporé sous pression réduite, et le résidu est repris dans 1 mL d'éthanol anhydre et 0.76 mL (1.52 mmol) d'acide chlorhydrique 2M dans l'éther diéthylique. La solution est agitée à 70°C pendant 3 heures. Le précipité est filtré, lavé à l'éthanol puis à l'éther diéthylique pour donner 24 mg (50%) de chlorhydrate de 8-méthylamino-4-oxo-4,5-dihydro-3*H*-pyrrolo[2,3-*c*]quinoline-1-carboxylate d'éthyle sous la forme d'un solide blanc.
***LCMS(IE, m/z) :** (M+1) 285.92*
***RMN$^1$H :** $\delta_H$ ppm 400 MHz, DMSO*
*13.12 (1H, sl, NH), 11.82 (1H, s, NH), 10.53 (1H, sl, NH), 9.22 (1H, s, $CH_{arom}$), 8.03 (1H, s, $CH_{arom}$), 7.56-7.36 (2H, m, $2xCH_{arom}$), 4.42-4.24 (2H, m, $CH_2$), 2.93 (3H, s, $CH_3$), 1.48-1.22 (3H, m, $CH_3$).*

A 153 μL (1.63 mmol) d'anhydride acétique refroidis dans un bain de glace sont lentement additionnés 74 μL (1.95 mmol) d'acide formique puis la réaction est agitée 2 heures à 60°C. Le mélange est refroidi à température ambiante puis ajouté à une solution de 500 mg (1.63 mmol) de chlorhydrate de 8-amino-4-oxo-4,5-dihydro-3*H*-pyrrolo[2,3-*c*]quinoline-1-carboxylate d'éthyle (synthèse 64) dissout dans 5 mL de diméthylformamide anhydre et 460 μL (3.25 mmol) de triéthylamine. Le mélange réactionnel est agité 2 heures à 60°C. Le solvant est évaporé et le résidu est trituré dans du méthanol chaud pour donner 359 mg (74%) de 8-formylamino-4-oxo-4,5-dihydro-3*H*-pyrrolo[2,3-*c*]quinoline-1-carboxylate d'éthyle sous la forme d'un solide rose pâle.

**8-Amino-4-pxo-4,5-dihydro-3*H*-pyrrolo[2,3-*c*]quinoline-1-carboxylate d'éthyle (64)**

A 500 mg (1.66 mmol) de 8-nitro-4-oxo-4,5-dihydro-3*H*-pyrrolo[2,3-*c*]quinoline-1-carboxylate d'éthyle (synthèse 66) en suspension dans 10 mL de diméthylformamide anhydre sont ajoutés 30 mg de palladium sur charbon (10% wt.) et 530 mg (8.30 mmol) de formiate d'ammonium. Le mélange réactionnel est agité 2 heures à 60°C. Le mélange est ensuite dilué dans de l'acétate d'éthyle et filtré sur célite. Le filtrat est évaporé et le résidu est trituré dans de l'éther

diéthylique pour donner après filtration 360 mg (80%) de 8-amino-4-oxo-4,5-dihydro-3*H*-pyrrolo[2,3-c]quinoline-1-carboxylate d'éthyle sous la forme d'un solide beige.
***LCMS(IE, m/z) :*** *(M+1) 272.29*
***RMN¹H :*** *$\delta_H$ ppm 250 MHz, DMSO*
*12.81 (1H, sl, NH), 11.30 (1H, s, NH), 8.37 (1H, d, $CH_{arom}$), 7.90 (1H, s, $CH_{arom}$), 7.12 (1H, d, $CH_{arom}$), 6.72 (1H, dd, $CH_{arom}$), 5.04 (2H, sl, $NH_2$), 4.29 (2H, q, $CH_2$), 1.33 (3H, t, $CH_3$).*

**Chlorhydrate de 8-amino-4-oxo-4,5-dihydro-3*H*-pyrrolo[2,3-*c*]quinoline-1-carboxylate d'éthyle (64 HCl)**

A 3.57 g (11.65 mmol) de 8-nitro-4-oxo-4,5-dihydro-3*H*-pyrrolo[2,3-*c*]quinoline-1-carboxylate d'éthyle (synthèse 66) en suspension dans 80 mL de diméthylformamide anhydre sont ajoutés 0.22 g de palladium sur charbon (10% wt.) et 3.74 g (59.25 mmol) de formiate d'ammonium. Le mélange réactionnel est agité 1 nuit à 60°C puis 0.50 g de palladium sur charbon (10% wt.) et 0.75 g (11.89 mmol) de formiate d'ammonium sont ajoutés. L'agitation est poursuivie pendant 4h. Le mélange est ensuite dilué dans de l'acétate d'éthyle et filtré sur célite. Le filtrat est évaporé et trituré dans de l'éther pour donner après filtration 3.16 g d'un mélange de 8-amino-4-oxo-4,5-dihydro-3*H*-pyrrolo[2,3-*c*]quinoline-1-carboxylate d'éthyle et de 8-formylamino-4-oxo-4,5-dihydro-3*H*-pyrrolo[2,3-*c*]quinoline-1-carboxylate d'éthyle. Le résidu beige est mis en suspension dans 40 mL d'éthanol anhydre avant addition de 11.7 mL (23.29 mmol) d'acide chlorhydrique 2M dans l'éther diéthylique. La solution est agitée pendant 90 minutes puis filtrée pour conduire à 3.34 g (92%) de chlorhydrate de 8-amino-4-oxo-4,5-dihydro-3*H*-pyrrolo[2,3-*c*]quinoline-1-carboxylate d'éthyle sont obtenus sous la forme d'un solide violet clair.
***LCMS(IE, m/z) :*** *(M+1) 272.29*
***RMN¹H :*** *$\delta_H$ ppm 250 MHz, DMSO*
*13.17 (1H, sl, NH), 11.91 (1H, s, NH), 10.38 (3H, sl, 3xNH), 9.29 (1H, d, $CH_{arom}$), 8.04 (1H, d, $CH_{arom}$), 7.53 (1H, d, $CH_{arom}$), 7.46 (1H, dd, $CH_{arom}$), 4.33 (2H q, $CH_2$) 1.35 (3H, t, $CH_3$).*

**8-(2-Ethoxycarbonyl-vinyl)-4-oxo-4,5-dihydro-3*H*-pyrrolo[2,3-*c*]quinoline-1-carboxylate d'éthyle (65)**

Ce composé est préparé selon la synthèse 68, à partir de 114 mg (0.3 mmol) de 8-iodo-4-oxo-4,5-dihydro-3*H*-pyrrolo[2,3-*c*]quinoline-1-carboxylate d'éthyle et 36 µL (0.33 mmol) d'acrylate d'éthyle. Après purification par chromatographie sur silice (éluant dichlomméthane/méthanol 98/2) puis trituration dans l'éthanol, 23 mg (22%) de 8-(2-éthoxycarbonyl-vinyl)-4-oxo-4,5-dihydro-3*H*-pyrrolo[2,3-*c*]quinoline-1-carboxylate d'éthyle sont obtenus sous la forme d'un solide blanc.
***LC/MS (IE, m/z) :*** *(M+1) 355.25*
***RMN¹H :*** *$\delta_H$ ppm 400 MHz, DMSO*
*13.09 (1H, sl, NM), 11.88 (1H, sl, NH), 9.56 (1H, s, $CH_{arom}$), 8.03 (1H, s, $CH_{arom}$), 7.80 (1H, d, $CH_{arom}$), 7.68 (1H, d, CH), 7.43 (1H, d, $CH_{arom}$), 6.54 (1H, d, CH), 4.35 (2H, q, $CH_2$), 4.22 (2H, q, $CH_2$), 1.36 (3H, t $CH_3$), 1.29 (3H, t, $CH_3$).*

**8-Nitro-4-oxo-4,5-dihydro-3*H*-pyrrolo[2,3-*c*]quinoline-1-carboxylate d'éthyle (66)**

Dans un bain de glace est mis sous agitation 1 g (3.90 mmol) de 4-oxo-4,5-dihydro-3*H*-pyrrolo[2,3-*c*]quinoline-1-carboxylate d'éthyle (synthèse 1) en suspension dans 15 mL d'anhydride acétique. Une solution de 0.27 mL (5.85 mmol) d'acide nitrique dans 2 mL d'acide acétique est ajoutée goutte à goutte et la suspension est agitée 2 heures à 0°C. A ce mélange est alors additionné 270 µL d'acide nitrique et l'agitation est poursuivie pendant 1h à 0°C. Le solide blanc-jaune est filtré et lavé avec de l'acide acétique pour donner un mélange de 8-nitro-4-oxo-4,5-dihydro-3*H*-pyrrolo [2,3-*c*]quinoline-1-carboxylate d'éthyle et de 6-nitro-4-oxo-4,5-dihydro-3*H*-pyrrolo[2,3-*c*]quinoline-1-carboxylate d'éthyle. Le solide est agité dans de l'acide acétique (20 mL) à 110°C pendant 1h puis filtré à chaud pour donner 0.55g (47%) de 8-nitro-4-oxo-4,5-dihydro-3*H*-pyrrolo[2,3-*c*]quinoline-1-carboxylate d'éthyle sous la forme d'un solide blanc.
***LCMS(IE, m/z) :*** *(M+1) 302.24*
***RMN¹H*** *: $\delta_H$ ppm 250 MHz, DMSO*
*13.29 (1H, sl, NH), 12.28 (1H, s, NH), 10.30 (1H, d, $CH_{arom}$), 8.26 (1H, dd, $CH_{arom}$), 8.08 (1H, d, $CH_{arom}$), 7.54 (1H, d, $CH_{arom}$), 4.35 (2H, q, $CH_2$), 1.36 (3H, t, $CH_3$).*

**6-Nitro-4-oxo-4,5-dihydro-3*H*-pyrrolo[2,3-*c*]quinolinc-1-carboxylate d'éthyle (67)**

Ce composé est formé lors de la synthèse du 8-nitro-4-oxo-4,5-dihydro-3*H*-pyrrolo[2,3-*c*]quinéline-1-carboxylate d'éthyle (synthèse 66). Le filtrat récupéré lors de la filtration à chaud est évaporé. Le solide jaune ainsi obtenu est recristallisé dans le méthanol pour donner 8 mg (1%) de 6-nitro-4-oxo-4,5-dihydro-3*H*-pyrrolo[2,3-*c*]quinoline-1-carboxylate d'éthyle sous la forme d'un solide jaune.

***LCMS(IE, m/z):*** *(M+1) 302.26*
***RMN¹H :*** *$\delta_H$ ppm 250 MHz, DMSO*
*13.41 (1H, sl, MH), 11.24 (1H, s, NH), 9.72 (1H, dd, $CH_{arom}$), 8.34 (1H, dd, $CH_{arom}$), 8.12 (1H, s, $CH_{arom}$), 7.45 (1H, t, $CH_{arom}$), 4.32 (2H, q, $CH_2$), 1.35 (3H, t, $CH_3$).*

**8-(2-Benzylcarbamoyl-vinyl)-4-oxo-4,5-dihydro-3*H*-pyrrolo[2,3-*c*]quinolince-1-carboxylate d'éthyle (68)**

Sous atmosphère d'argon, à 100 mg (0.30 mmol) de 8-bromo-4-oxo-4,5-dihydro-3*H*-pyrrolo[2,3-c]quinoline-1-carboxylate d'éthyle (synthèse 71) dissous dans 0.6 ml de diméthylformamide anhydre sont ajoutés 53 mg (0.33 mmol) de *N*-benzyl-acrylamide, 29 mg (0.10 mmol) de tri-*o*-tolyl-phosphine, 83 µL (0.60 mmol) de triéthylamine et 6 mg (0.02 mmol) d'acétate de palladium. Le mélange réactionnel est agité une nuit à 80˚C. Le mélange est dilué avec de l'acétate d'éthyle (20 mL) et une solution saturée de chlorure de sodium (20 mL). Les deux phases sont séparées et la phase aqueuse est extraite avec de l'acétate d'éthyle). Les phases organiques sont réunies, lavées avec une solution saturée de chlorure de sodium, séchées avec du sulfate de magnésium puis évaporées. Le résidu obtenu est trituré dans un mélange dichlorométhane/méthanol (1.8 mL / 0.2 mL) pour donner 87 mg d'un solide. Après purification par préparative LCMS, 1 mg (1%) de 8-(2-benzylcarbamoyl-vinyl)-4-oxo-4,5-dihydro-3*H*-pyrrolo[2,3-*c*]quinoline-1-carboxylate d'éthyle sont obtenus sous la forme d'un solide blanc.
***LCMS(IE, m/z) :*** *(M+1) 416.41*
***RMN¹H :*** *$\delta_H$ ppm 250 MHz, $CD_3OD$*
*9.73 (1H, s, $CH_{arom}$), 8.06 (1H, s, $CH_{arom}$), 7.70 (1H, d, $CH_{éthyl}$), 7.68-7.60 (1H, m, $CH_{arom}$), 7.41 (1H, d, $CH_{arom}$), 7.38-7.18 (5H, m, $5xCH_{arom}$), 6.73 (1H, d, $CH_{éthyl}$), 4.51 (2H, s, $CH_2$), 4.40 (2H, q, $CH_2$), 1.42 (3H, t, $CH_3$).*

**8-[2-(2-Méthoxycarbonyl-éthylcarbamoyl)-vinyl]-4-oxo-4,5-dihydro-3*H*-pyrrolo[2,3-*c*]quinoline-1-carboxylate d'éthyle (69)**

Ce composé est préparé selon la synthèse 68, à partir de 70 mg (0.21 mmol) de 8-bromo-4-oxo-4,5-dihydro-3*H*-pyrrolo[2,3-*c*]quinoline-1-carboxylate d'éthyle (synthèse 71) et 36 mg (0.23 mmol) de 3-acryloylamino-propionate de méthyle. Après trituration dans le dichlorométhane, 10 mg (12%) de 8-[2-(2-méthoxycarbonyl-éthylcarbamoyl)-vinyl]-4-oxo-4,5-dihydro-3*H*-pyrrolo[2,3-*c*]quinoline-1-arboxylate d'éthyle sont obtenus sous la forme d'un solide marron clair.
***LCMS(IE, m/z) :*** *(M+1) 412.39*
***RMN ¹H :*** *$\delta_H$ ppm 250 MHz, DMSO*
*13.08 (1H, sl, NH), 11.85 (1H, s, NH), 9.45 (1H, s, $CH_{arom}$), 8.37-8.23 (1H, m, NH), 8.02 (1H, s, $CH_{arom}$), 7.62 (1H, d, $CH_{arom}$), 7.53-7.36 (2H, m, $CH_{arom}$ et $CH_{éthyl}$), 6.56 (1H, d, $CH_{éthyl}$), 4.34 (2H, q, $CH_2$), 3.62 (3H, s, $CH_3$), 3.47-3.34 (2H, m, $CH_2$), 2.55 (2H, t, $CH_2$), 1.36 (3H, t, $CH_3$).*

**4-Oxo-8-styryl-4,5-dihydro-3*H*-pyrrolo[2,3-*c*]quinoline-1-carboxylate d'éthyle (70)**

Ce composé est préparé selon la synthèse 68, à partir de 70 mg (0.21 mmol) de 8-bromo-4-oxo-4,5-dihydro-3*H*-pyrrolo[2,3-*c*]quinoline-1-carboxylate d'éthyle (synthèse 71) et 26 µL (0.23 mmol) de styrène. Après filtration et trituration dans l'éther diéthylique, 10 mg (14%) de 4-oxo-8-styryl-4,5-dihydro-3*H*-pyrrolo[2,3-*c*]quinoline-1-carboxylate d'éthyle sont obtenus sous la forme d'un solide marron clair.
***LCMS(IE, m/z) :*** *(M+1) 359.35*
***RMN¹H :*** *$\delta_H$ ppm 250 MHz, DMSO*
*13.04 (1H, sl, MH), 11.77 (1H, s, NH), 9.43 (1H, d, $CH_{arom}$), 8.01 (1H, s, $CH_{arom}$), 7.72 (1H, dd, $CH_{arom}$), 7.60 (2H, d, $2xCH_{arom}$), 7.45-7.14 (6H, m, $4xCH_{arom}$ et $2xCH_{éthyl}$), 4.36 (2H, q, $CH_2$), 1.36 (3H, t, $CH_3$).*

**8-Bromo-4-oxo-4,5-dihydro-3*H*-pyrrolo[2,3-*c*]quinoline-1-carboxylate d'éthyle (71)**

A 500 mg (1.95 mmol) de 4-oxo-4,5-dihydro-3*H*-pyrrolo[2,3-*c*]quinoline-1-carboxylate d'éthyle (synthèse 1) dissous dans 10 ml de diméthylformamide anhydre sont ajoutés 434 mg (2.44 mmol) de *N*-bromosuccinimide et le mélange réactionnel est agité 1h à température ambiante. Le mélange est ensuite dilué dans 10 mL d'acétate d'éthyle, le solide est filtré, rincé à l'éther diéthylique puis recristallisé dans l'acide acétique pour donner 360 mg (55%) de 8-bromo-4-oxo-4,5-dihydro-*3H*-pyrrolo[2,3-*c*]quinoline-1-carboxylate d'éthyle cocristallisé avec un équivalent d'acide acétique sous la forme d'un solide blanc.
***LCMS(IE, m/z) :*** *(M+1) 334.77-336.77*
***RMN¹H :*** *$\delta_H$ ppm 250 MHz, DMSO*
*13.10 (1H, sl, NH), 11.80 (1H, s, NH), 9.49 (1H, d, $CH_{arom}$), 8.01 (1H, s, $CH_{arom}$), 7.56 (1H, dd, $CH_{arom}$), 7.36 (1H, d, $CH_{arom}$), 4.32 (2H, q, $CH_2$), 1.35 (3H, t, $CH_3$).*

**7,8-Diméthoxy-4-oxo-4,5-dihydro-3*H*-pyrrolo[2,3-*c*]quinoline-1-carboxylate d'éthyle (72)**

Ce composé a été synthétisé selon la synthèse 1, à partir de 0.8 g (4.14 mmol) de 5,6-diméthoxy-1,3-dihydro-indol-2-one pour donner après recristallisation dans l'acide acétique, 0.19 g (14% sur deux étapes) de 7,8-diméthoxy-4-oxo-4,5-dihydro-3*H*-pyrrolo[2,3-*c*]quinoline-1-carboxylate d'éthyle cocristallisé avec un équivalent d'acide acétique sous la forme d'un solide gris.
***LCMS(IE, m/z) :*** *(M+1) 316.90*
***RMN¹H :*** *$\delta_H$ ppm 250 MHz, DMSO*
*12.84 (1H, sl, NH), 11.43 (1H, s, NH), 8.94 (1H, s, $CH_{arom}$), 7.94 (1H, s, $CH_{arom}$), 7.00 (1H, s, $CH_{arom}$), 4.30 (2H, q, $CH_2$), 3.83 (3H, s, $CH_3$), 3.80 (3H, s, $CH_3$), 1.33 (3H, t, $CH_3$).*
La synthèse du 5,6-diméthoxy-1,3-dihydro-indol-2-one est décrite par C. Lanzi (WO2004/009083 A1). Ce composé a été préparé selon ce protocole à partir de 4.34 g (22.13 mmol) d'acide (3,4-diméthoxy-phényl)-acétique pour donner après trituration dans l'éther diéthylique, 2.87 g (67 % sur deux étapes) de 5,6-diméthoxy-1,3-dihydro-indol-2-one sous la forme d'un solide beige.

**4-Oxo-4,5-dihydro-3*H*-pyrrolo[2,3-*c*]quinoline-1-carboxylate de méthyle (73)**

A 68 mg (0.3 mmol) d'acide 4-oxo-4,5-dihydro-3*H*-pyrrolo[2,3-*c*]quinoline-1-carboxylique (synthèse 75) en suspension dans 2 mL de méthanol anhydre, est additionnée goutte à goutte une solution de 55 µL (0.75 mmol) de chlorure de thionyle dans 1.5 mL de méthanol anhydre. La solution est agitée à reflux pendant 48 heures puis refroidie à température ambiante. Le solide est filtré pour conduire à 15 mg (20%) de 4-oxo-4,5-dihydro-3*H*-pyrrolo[2,3-*c*]quinoline-1-carboxylate de méthyle sous la forme d'un solide beige.
***LCMS(IE, m/z) :*** *(M+1) 243.30*
***RMN¹H :*** *$\delta_H$ ppm 250 MHz, DMSO*
*12.98 (1H, sl, NH), 11.65 (1H, s, NH), 9.22 (1H, d, $CH_{arom}$), 7.99 (1H, s, $CH_{arom}$), 7.44-7.38 (2H, m, $2xCH_{arom}$), 7.27-7.17 (1H, m, $CH_{arom}$), 3.83 (3H, s, $CH_3$).*

**4-Oxo-4,5-dihydro-3*H*-pyrrolo[2,3-*c*]quinoline-1-carboxylate de benzyle (74)**

A 50 mg (0.22 mmol) d'acide 4-oxo-4,5-dihydro-3*H*-pyrrolo[2,3-*c*]quinoline-1-carboxylique (synthèse 75) dans 0.5 mL de diméthylformamide anhydre, 124 µL (0.88 mmol) de triéthylamine et 79 µL (0.66 mmol) de bromure de benzyle sont additionnés par portion (sur 3 jours) jusqu'à complète disparition du produit de départ. Le résidu est purifié par chromatographie sur gel de silice (éluant dichlorométhane/méthanol 94/6) pour donner 56 mg d'un produit impur. Après recristallisation dans le méthanol, 4 mg (6%) de 4-oxo-4,5-dihydro-3*H*-pyrrolo[2,3-*c*]quinoline-1-carboxylate de benzyle sont obtenus sous la forme d'un solide blanc.
***LCMS(IE, m/z) :*** *(M+1) 319.25*
***RMN¹H :*** *$\delta_H$ ppm 250 MHz, DMSO*
*13.03 (1H, sl, NH), 11.66 (1H, s, NH), 9.21 (1H, d, $CH_{arom}$), 8.05 (1H, s, $CH_{arom}$), 7.55-7.47 (2H, m, $2xCH_{arom}$), 7.46-7.27 (5H, m, $5xCH_{arom}$), 7.24-7.14 (1H, m, $CH_{arom}$), 5.36(2H, s, $CH_2$).*

**Acide 4-oxo-4,5-dihydro-3*H*-pyrrolo[2,3-*c*]quinoline-1-carboxylique (75)**

A 1 g (3.9 mmol) de 4-oxo-4,5-dihydro-3*H*-pyrrolo[2,3-*c*]quinoline-1-carboxylate d'éthyle (synthèse 1) en suspension dans 45 mL d'éthanol et 45 mL d'eau, 1.64 g (39 mmol) d'hydroxyde de lithium monohydraté sont ajoutés et la solution est agitée 17h à 70°C puis 5h à 100°C. L'éthanol est évaporé sous vide et de l'eau distillé est ajoutée. La solution aqueuse est lavée à l'acétate d'éthyle puis acidifiée avec de l'acide acétique jusqu'à un pH 4-5. Le précipité formé est filtré puis séché sous vide à 55°C pour donner 0.9 g (100%) d'acide 4-oxo-4,5-dihydro-3*H*-pyrrolo[2,3-*c*]quinoline-1-carboxylique sous la forme d'un solide beige.
***LCMS(IE, m/z) :*** *(M+1) 229.25*
***RMN¹H :*** *$\delta_H$ ppm 250 MHz, DMSO*
*13.31-11.76 (2H, signal aplati, NH et COOH), 11.55 (1H, s, NH), 9.66 (1H, dd, $CH_{arom}$), 7.78 (1H, s, $CH_{arom}$), 7.38 (1H, dd, $CH_{arom}$), 7.35-7.27 (1H, m, $CH_{arom}$), 7.18-7.10 (1H, m, $CH_{arom}$).*

**1-Benzoyl-3,5-dihydro-pyrrolo[2,3-*c*]quinolin-4-one (76)**

La synthèse de ce composé est décrite par J. Bergman (Tetrahedron, 2002, 58, pp 9179-9185). Le composé a été préparé selon ce protocole à partir de 0.8 g (6 mmol) de 1,3-dihydro-indol-2-one pour conduire après trituration dans le dichlorométhane à 190 mg (11% sur deux étapes) de 1-benzoyl-3,5-dihydro-pyrrolo[2,3-*c*]quinolin-4-one sous

forme d'un solide beige.
***LCMS(IE, m/z) :*** *(M+1) 289.32*
***RMN¹H*** *: $\delta_H$ ppm 250 MHz, DMSO*
*13.04 (1H, sl, NH), 11.72 (1H, s, NH), 8.84 (1H, d, $CH_{arom}$), 7.87-7.80 (2H, m, $2xCH_{arom}$), 7.70-7.51 (4H, m, $4xCH_{arom}$), 7.47-7.35 (2H, m, $2xCH_{arom}$), 7.23-7.14 (1H, m, $CH_{arom}$).*

**1-Acétyl-3,5-dihydro-pyrrolo[2,3-*c*]quinolin-4-one(77)**

Ce composé est préparé selon la synthèse 76, à partir de 930 mg (7 mmol) de 1,3-dihydro-indol-2-one et 1.9 mL (environ 10.5 mmol) d'une solution aqueuse à 40% d'aldéhyde pyruvique. Après trituration dans l'acétate d'éthyle, 63 mg (4% sur deux étapes) de 1-acétyl-3,5-dihydro-pyrrolo[2,3-c]quinolin-4-one sont obtenus sous la forme d'un solide beige.
***LCMS(IE, m/z) :*** *(M+1) 227.27*
***RMN¹H :*** *$\delta_H$ ppm 250 MHz, DMSO*
*13.02 (1H, sl, NH), 11.70 (1H, s, NH), 9.31 (1H, d, $CH_{arom}$), 8.35 (1H, d, $CH_{arom}$), 7.40-7.37 (2H, m, $2xCH_{arom}$), 7.24-7.16 (1H, m, $CH_{arom}$), 2.58 (3H, s, $CH_3$).*

**4-Oxo-4,5-dihydro-3*H*-pyrrolo[2,3-*c*]quinoline-1-carboxylate d'isopropyle (78)**

A une solution de 120 mg (0.47 mmol) d'acide 4-oxo-4,5-dihydro-3*H*-pyrrolo[2,3-*c*]quinoline-1-carboxylique dissous dans 2 mL de diméthylformamide sont additionnés 58 mg (0.42 mmol) de bromure d'isopropyle et 66 μL (0.7 mmol) de carbonate de potassium. Le mélange réactionnel est agité 48 heures à 70°C. La réaction est suivie par LC/MS sur 7 jours avec ajout régulier de bromure d'isopropyle (300 mg, 2.17 mmol) et de carbonate de potassium (132 μL, 1.4 mmol) supplémentaires de façon à avoir complète disparition du produit de départ. Les solvants sont évaporés et le résidu est purifié par chromatographie sur silice (éluant dichlorométhane/méthanol 95/5) pour conduire à 20 mg (15 %) de 4-oxo-4,5-dihydro-3*H*-pyrrolo[2,3-*c*]quinoline-1-carboxylate d'isopropyle sous la forme d'un solide blanc.
***LCMS (IE, m/z):*** *(M+1) 270.76*
***RMN¹H :*** *$\delta_H$ ppm 400 MHz, DMSO*
*12.94 (1H, sl, NH), 11.65 (1H, s, NH), 9.22 (1H, d, $CH_{arom}$), 7.94 (1H, s, $CH_{arom}$), 7.43-7.37 (2H, m, $2xCH_{arom}$), 7.21 (1H, ddd, $CH_{arom}$), 5.14 (1H, sept, CH), 1.34 (6H, d, $2xCH_3$).*

***N*-(2-Diméthylamino-éthyl)-4-oxo-4,5-dihydro-3*H*-pyrrolo[2,3-*c*]quinoline-1-carboxamide (79)**

A 75 mg (0.33 mmol) d'acide 4-oxo-4,5-dihydro-3*H*-pyrrolo[2,3-*c*]quinoline-1-carboxylique (synthèse 75) en suspension dans 0.7 ml de diméthylformamide anhydre sont ajoutés 49 mg (0.36 mmol) de 1-hydroxybenzotriazole, 69 mg (0.36 mmol) de chlorhydrate de 1-éthyl-3-(3-diméthylaminopropyl)carbodiimide, 73 μL (0.66 mmol) de *N*-méthylmorpholine et 40 μL (0.36 mmol) de *N,N*-diméthyléthylènediamine. Le mélange réactionnel est agité une nuit à température ambiante. Le mélange est dilué avec de l'acétate d'éthyle (20 mL) et une solution saturée de chlorure de sodium (20 mL). Les deux phases sont séparées et la phase aqueuse est extraite avec de l'acétate d'éthyle (3x20 mL). Les phases organiques sont réunies, lavées avec une solution aqueuse de carbonate de potassium (10%) (3x40 mL), de l'eau (40 mL) et une solution saturée de chlorure de sodium (50 mL), séchées avec du sulfate de magnésium et évaporées. Le résidu obtenu est trituré dans de l'éther diéthylique pour donner 13 mg (13%) de *N*-(2-diméthylamino-éthyl)-4-oxo-4,5-dihydro-3*H*-pyrrolo[2,3-*c*]quincline-1-carboxainide sous la forme d'un solide marron clair.
***LCMS(IE, m/z) :*** *(M+1) 299.36*
***RMN¹H :*** *$\delta_H$ ppm 250 MHz, $CD_3OD$*
*8.79 (1H, d, $CH_{arom}$), 7.82 (1H, s, $CH_{arom}$), 7.43-7.34 (2H, m, $2xCH_{arom}$), 7.30-7.18 (1H, m, $CH_{arom}$), 3.58 (2H, t, $CH_2$), 2.65 (2H, t, $CH_2$), 2.36 (6H, s, $2xCH_3$).*

***N*-(3-Diméthylamino-propyl)-4-oxo-4,5-dihydro-3*H*-pyrrolo[2,3-*c*]quinoline-1-carboxamide** (80)

Ce composé est préparé selon la synthèse 79 à partir de 75 mg (0.33 mmol) d'acide 4-oxo-4,5-dihydro-3*H*-pyrrolo[2,3-*c*]quinoline-1-carboxylique (synthèse 75) et 46 μL (0.36 mmol) de 3-diméthylaminopropylamine. Après trituration dans l'éther diéthylique, 37 mg (36%) de N-(3-diméthylamino-propyl)-4-oxo-4,5-dihydro-3*H*-pyrrolo[2,3-*c*]quinoline-1-carboxamide sont obtenus sous la forme d'un solide blanc.
***LCMS(IE, m/z)*** *: (M+1) 313.37*
***RMN¹H :*** *$\delta_H$ ppm 250 MHz, $CD_3OD$*
*8.76 (1H, d, $CH_{arom}$), 7.76 (1H, s, $CH_{arom}$), 7.43-7.35 (2H, m, $2xCH_{arom}$), 7.28-7.19 (1H, m, $CH_{arom}$), 3.46 (2H, t, $CH_2$), 2.61-2.51 (2H, m, $CH_2$), 2.35 (6H, s, $2xCH_3$), 1.97-1.81 (2H, m, $CH_2$).*

**3-[(4-Oxo-4,5-dihydro-3*H*-pyrrolo[2,3-*c*]quinoline-1-carbonyl)-amino]-propanoate de méthyle (81)**

Ce composé est préparé selon la synthèse 79 à partir de 75 mg (0.33 mmol) d'acide 4-oxo-4,5-dihydro-3*H*-pyrrolo[2,3-*c*]quinoline-1-carboxylique (synthèse 75), 108 μL (0.99 mmol) de *N*-méthylmorpholine et 51 mg (0.36 mmol) de chlorhydrate de 3-aminopropanoate de méthyle. Après trituration dans le méthanol, 9 mg (9%) de 3-[(4-Oxo-4,5-dihydro-3*H*-pyrrolo[2,3-*c*]quinoline-1-carbonyl)-amino]-propanoate de méthyle sont obtenus sous la forme d'un solide blanc.
***LCMS(IE, m/z) :*** *(M+1) 314.34*
***RMN¹H :*** *$\delta_H$ ppm 250 MHz, DMSO*
*12.60 (1H, sl, NH), 11.54 (1h, s, NH), 8.99 (1H, d, $CH_{arom}$), 8.37-8.27 (1H, m, NH), 7.79 (1H, s, $CH_{arom}$), 7.42-7.28 (2H, m, 2x$CH_{arom}$), 7.22-7.09 (1H, m, $CH_{arom}$), 3.62 (3H, s, $CH_3$), 3.58-3.44 (2H, m, $CH_2$), 2.62 (2H, t, $CH_2$).*

***N*-(Cyclohexylméthyl)-4-oxo-4,5-dihydro-3*H*-pyrrolo[2,3-*c*]quinoline-1-carboxamide (82)**

Ce composé est préparé selon la synthèse 79 à partir de 75 mg (0.33 mmol) d'acide 4-oxo-4,5-dihydro-3*H*-pyrrolo[2,3-*c*]quinoline-1-carboxylique (synthèse 75) et 47 μL (0.36 mmol) de C-cyclohexyl-méthylamine. Après recristallisation dans le méthanol, 8 mg (8%) de *N*-(cyclohexylméthyl)4-oxo-4,s-dihydro-3*H*-pyrrolo[2,3-*c*]quinoline-1-carboxamide sont obtenus sous la forme d'un solide blanc.
***LCMS(IE, m/z)*** *: (M+1) 324.41*
***RMN¹H :*** *$\delta_H$ ppm 250 MHz, $CD_3OD$*
*8.71 (1H, d, $CH_{arom}$), 7.73 (1H, s, $CH_{arom}$), 7.42-7.37 (2H, m, 2x$CH_{axom}$), 7.28-7.18 (1H, m, $CH_{arom}$), 3.26 (2H, d, $CH_2$), 1.94-1.57 (6H, m, 6x$CH_{cyclo}$), 1.43-1.17 (3H, m, 3x$CH_{cyclo}$), 1.15-0.96 (2H, m, 2x$CH_{cyclo}$).*

***N*-(Benzyl)-4-oxo-4,S-dihydro-3*H*-pyrrolo[2,3-*c*]quinoline-1-carboxamide (83)**

Ce composé est préparé selon la synthèse 79 à partir de 75 mg (0.33 mmol) d'acide 4-oxo-4,5-dihydro-3*H*-pyrrolo[2,3-*c*]quinoline-1-carboxylique (synthèse 75) et 40 μL (0.36 mmol) de benzylamine. Après recristallisation dans le méthanol, 8 mg (8%) de *N*-(benzyl)-4-oxo-4,5-dihydro-3*H*-pyrrolo[2,3-*c*]quinoline-1-carboxamide sont obtenus sous la forme d'un solide blanc.
***LCMS(IE, m/z) :*** *(M+1) 318.37*
***RMN¹H :*** *$\delta_H$ ppm 250 MHz, $CD_3OD$*
*8.72 (1H, d, $CH_{arom}$), 7.79 (1H, s, $CH_{arom}$), 7.46-7.16 (8H, m, 8x$CH_{arom}$), 4.62 (2H, s, $CH_2$).*

***N*-[2-(1*H*-Indol-3-yl)-éthyl]-4-oxo-4,5-dihydro-3*H*-pyrrolo[2,3-*c*]quinoline-1-carboxamide (84)**

Ce composé est préparé selon la synthèse 79 à partir de 75 mg (0.33 mmol) d'acide 4-oxo-4,5-Mydro-3*H*-pyrrolo [2,3-*c*]quinoline-1-carboxylique (synthèse 75) et 58 mg (0.36 mmol) de tryptamine. Après trituration dans l'éther diéthylique, 48 mg (40%) de *N*-[2-(1*H*-indol-3-yl)-éthyl]-4-oxo-4,5-dihydro-3*H*-pyrrolo[2,3-*c*]quinoline-1-carboxamide sont obtenus sous la forme d'un solide marron clair.
***LCMS(IE, m/z) :*** *(M+1) 371.37*
***RMN¹H :*** *$\delta_H$ ppm 250 MHz, $CD_3OD$*
*8.68 (1H, d, $CH_{arom}$), 7.64 (1H, d, $CH_{arom}$), 7.59 (1H, s, $CH_{arom}$), 7.42-7.30 (3H, m, 3x$CH_{arom}$), 7.22-6.95 (4H, m, 4x$CH_{arom}$), 3.73 (2H, t, $CH_2$), 3.13 (2H, t, $CH_2$).*

***N*-(3-Hydroxy-propyl)-4-oxo-4,5-dihydro-3*H*-pyrrolo[2,3-*c*]quinoline-1-carboxamide (85)**

Ce composé est préparé selon la synthèse 79 à partir de 75 mg (0.33 mmol) d'acide 4-oxo-4,5-dihydro-3*H*-pyrrolo[2,3-*c*]quinoline-1-carboxylique (synthèse 75) et 28 μL (0.36 mmol) de 3-hydroxy-propylamine. La phase aqueuse est évaporée, le résidu repris dans du méthanol puis filtré. Le filtrat est évaporé et le résidu est purifié par préparative LCMS pour donner 8 mg (9%) de *N*-(3-hydroxy-propyl)-4-oxo-4,5-dihydro-3*H*-pyrrolo[2,3-*c*]quinoline-1-carboxamide sous la forme d'un solide blanc.
***LCMS(IE, m/z)*** *: (M+1) 286.34*
***RMN¹H*** *: $\delta_H$ ppm 250 MHz, DMSO*
*12.57 (1H, s, NH), 11.53 (1H, s, NH), 9.00 (1H, d, $CH_{arom}$), 8.19 (1H, t, NH), 7.79 (1H, s, $CH_{arom}$), 7.40-7.28 (2H, m, 2x$CH_{arom}$), 7.18-7.10 (1H, m, $CH_{arom}$), 4.49 (1H, t, OH), 3.54-3.45 (2H, m, $CH_2$), 3.37-3.27 (2H, m, $CH_2$), 1.70 (2H, quint., $CH_2$).*

**N-(Pyridin-4-ylméthyl)-4-oxo-4,5-dihydro-3H-pyrrolo[2,3-c]quinoline-1-carboxamide (86)**

Ce composé est préparé selon la synthèse 79 à partir de 75 mg (0.33 mmol) d'acide 4-oxo-4,5-dihydro-3*H*-pyrrolo[2,3-*c*]quinoline-1-carboxylique (synthèse 75) et 37 μL (0.36 mmol) de pyridin-4-ylméthylamine. Le précipité formé au cours de la réaction est filtré puis lavé avec du diméthylformamide et de l'éther diéthylique pour conduire à 46 mg (44%) de *N*-(pyridin-4-ylméthyl)-4-oxo-4,5-dihydro-3*H*-pyrrolo[2,3-*c*]quinoline-1-carboxamide sous la forme d'un solide blanc.
***LCMS(IE, m/z)*** *: (M+1) 319.37*
***RMN¹H*** *: $\delta_H$ ppm 250 MHz, DMSO*
*12.68 (1H, sl, NH), 11.57 (1H, sl, NH), 9.07-8.81 (2H, m, NH et $CH_{arom}$), 8.67-8.35 (2H, m, $2xCH_{arom}$), 7.99 (1H, s, $CH_{arom}$), 7.54-7.23 (4H, m, $4xCH_{arom}$), 7.17-7.02 (1H, m, $CH_{arom}$), 4.50 (2H, s, $CH_2$).*

**N-(Phényl)-4-oxo-4,5-dihydro-3H-pyrrolo[2,3-c]quinoline-1-carboxamide (87)**

Ce composé est préparé selon la synthèse 79 à partir de 75 mg (0.33 mmol) d'acide 4-oxo-4,5-dihydro-3*H*-pyrrolo[2,3-*c*]quinoline-1-carboxylique (synthèse 75) et 33 μL (0.36 mmol) d'aniline. Après trituration dans l'éther diéthylique, 45 mg (45%) de *N*-(phényl)-4-oxo-4,5-dihydro-3*H*-pyrrolo[2,3-*c*]quinoline-1-carboxamide sont obtenus sous la forme d'un solide marron clair.
***LCMS(IE, m/z)*** *: (M+1) 304.33*
***RMN¹H*** *: $\delta_H$ ppm 250 MHz, DMSO*
*12.78 (1H, sl, NH), 11.61 (1H, s, NH), 10.15 (1H, s, NH), 8.87 (1H, d, $CH_{arom}$), 8.03 (1H, s, $CH_{arom}$), 7.79 (2H, d, $2xCH_{arom}$), 7.44-7.30 (4H, m, $4xCH_{arom}$), 7.21-7.03 (2H, m, $2xCH_{arom}$).*

**N-(Méthyl)-4-oxo-4,5-dihydro-3H-pyrrolo[2,3-c]quinoline-1-carboxamide (88)**

Ce composé est préparé selon la synthèse 79 à partir de 37 mg (0.17 mmol) d'acide 4-oxo-4,5-dihydro-3*H*-pyrrolo[2,3-*c*]quinoline-1-carboxylique (synthèse 75), 108 μL (0.99 mmol) de *N*-méthylmorpholine et 17 mg (0.25 mmol) de chlorhydrate de méthylamine. Après trituration dans l'éther diéthylique, 3 mg (7%) de *N*-(méthyl)-4-oxo-4,5-dihydro-3*H*-pyrrolo[2,3-*c*]quinoline-1-carboxamide sont obtenus sous la forme d'un solide marron clair.
***LCMS(IE, m/z)*** *: (M+1) 242.29*
***RMN¹H*** *: $\delta_H$ ppm 250 MHz, $CD_3OD$*
*8.77 (1H, d, $CH_{arom}$), 7.74 (1H, s, $CH_{arom}$), 7.43-7.36 (2H, m, $2xCH_{arom}$), 7.29-7.19 (1H, m, $CH_{arom}$), 2.96 (3H, s, $CH_3$).*

**N,N-(Diméthyl)-4-oxo-4,5-dihydro-3H-pyrrolo[2,3-c]quinoline-1-carboxamide (89)**

Ce composé est préparé selon la synthèse 79 à partir de 41 mg (0.18 mmol) d'acide 4-oxo-4,5-dihydro-3*H*-pyrrolo[2,3-*c*]quinoline-1-carboxylique (synthèse 75), 108 μL (0.99 mmol) de *N*-méthylmorpholine et 22 mg (0.25 mmol) de chlorhydrate de diméthylamine. Après filtration du milieu réactionnel, 10 mg (22%) de *N,N*-(diméthyl)-4-oxo-4,5-dihydro-3*H*-pyrrolo[2,3-*c*]quinoline-1-carboxamide sont obtenus sous la forme d'un solide blanc.
***LCMS(IE, m/z) :*** *(M+1) 256.26*
***RMN¹H :*** *$\delta_H$ ppm 250 MHz, DMSO*
*12.54 (1H, sl, NH), 11.48 (1H, s, NH), 7.84 (1H, d, $CH_{arom}$), 7.51 (1H, s, $CH_{arom}$), 7.42-7.29 (2H, m, $2xCH_{arom}$), 7.19-7.11 (1H, m, $CH_{arom}$), 3.02 (6H, sl, $2xCH_3$).*

**4-Oxo-4,5-dihydro-3H-pyrrolo[2,3-c]quinoline-1-carboxamide (90)**

Une suspension de 84 mg (0.4 mmol) de 4-oxo-4,5-dihydro-3*H*-pyrrolo[2,3-*c*]quinoline-1-carbonitrile dans 1 mL d'acide polyphosphorique est portée à 130°C pendant 2h. Le mélange réactionnel est refroidi à température ambiante puis versé dans de la glace. Le mélange est agité 30min et le solide ainsi formé est filtré puis repris dans un mélange méthanol/toluène. Après évaporation des solvants, le résidu (mélange de 4-oxo-4,5-dihydro-3*H*-pyrrolo[2,3-*c*]quinoline-1-carboxamide et d'acide 4-oxo-4,5-dihydro-3*H*-pyrrolo[2,3-*c*]quinoline-1-carboxylique 2/1) est purifié par préparative LCMS pour donner 4 mg (4%) de 4-oxo-4,5-dihydro-3*H*-pyrrolo[2,3-*c*]quinoline-1-carboxamide sous la forme d'un solide blanc.
***LCMS(IE, m/z) :*** *(M+1) 228.24*
***RMN¹H*** *: $\delta_H$ ppm 250 MHz, DMSO*
*12.60 (1H, sl, NH), 11.53 (1H, s, NH), 9.57 (1H, d, $CH_{arom}$), 7.90 (1H, s, $CH_{arom}$), 7.69 (1H, sl, NH), 7.40-7.29 (2H, m, $2xCH_{arom}$), 7.19-7.02 (2H, m, NH et $CH_{arom}$).*
La synthèse du 4-oxo-4,5-dihydro-3*H*-pyrrolo[2,3-*c*]quinoline-1-carbonitrile a été décrite par J. Bergman (Tetrahedron,

2002, 58, pp 9179-9185). Le composé a été préparé selon ce protocole à partir de 1.2 g (7.96 mmol) de 2-nitrobenzaldéhyde pour conduire après recristallisation dans l'acide acétique à 283 mg (17% sur trois étapes) de 4-oxo-4,5-dihydro-3*H*-pyrrolo[2,3-*c*]quinoline-1-carbonitrile sous forme d'un solide blanc.

**4-Oxo-8-(pipéridin-4-ylsulfamoyl)-4,5-dihydro-3*H*-pyrrolo[2,3-*c*]quinoline-1-carboxylate d'éthyle (91)**

Ce composé est préparé selon la synthèse 25, à partir de 326 mg (1 mmol) d'acide 8-chlorosulfonyl-4-oxo-4,5-dihydro-3*H*-pyrrolo[2,3-*c*]quinoline-1-carboxylique (synthèse 2) et de 236 mg (1 mmol) de chlorhydrate de 4-amino-1-*tert*-butoxycarbonyl pipéridine et de 700 µL (5 mmol) de triéthylamine. Après purification par chromatographie sur silice (éluant chloroforme/méthanol/ammoniaque 65/25/4) puis trituration dans le méthanol, 16 mg (4%) de 4-oxo-8-(pipéridin-4-ylsulfamoyl)-4,5-ihydro-3*H*-pyrrolo[2,3-*c*]quinoline-1-carboxylate d'éthyle sont obtenus sous forme d'un solide blanc

***LCMS(IE, m/z) :*** *(M+1) 419.10*

***RMN$^1$H*** *: $\delta_H$ ppm 400 MHz, DMSO*

*12.08 (1H, s, NH), 9.84 (1H, d, $CH_{arom}$), 8.07 (1H, s, $CH_{arom}$), 7.87 (1H, dd, $CH_{arom}$), 7.56 (1H, d, $CH_{arom}$), 4.34 (2H, q, $CH_2$), 3.40-3.30 (1H, m, $CH_{pip}$) 3.20-3.11 (2H, m, $2xCH_{pip}$), 3.00-2.92 (1H, m, NH), 2.91-2.81 (2H, m, $2xCH_{pip}$), 1.84-1.75 (2H, m, $2xCH_{pip}$), 1.61-1.52 (2H, m, $2xCH_{pip}$), 1.36 (3H, t, $CH_3$).*

**8-Cyclopentylsulfamoyl-4-oxo-4,5-dihydro-3*H*-pyrrolo[2,3-*c*]quinoline-1-carboxylate de méthyle (92)**

A 170 mg (0.5 mmol) d'acide 8-chlorosulfonyl-4-oxo-4,5-dihydro-3*H*-pyrrolo[2,3-*c*]quinoline-1-carboxylique (synthèse 2) en suspension dans 2 mL de dichlorométhane anhydre et 0.5 mL de diméthylformamide anhydre, sont ajoutés 146 µL (1.04 mmol) de triéthylamine et 57 µL (0.57 mmol) de cyclopentylamine. Le milieu réactionnel est agité 5 heures à température ambiante. Les solvants sont évaporés puis le résidu est repris dans 2 mL de méthanol anhydre. Une solution de 380 µL (5.2 mmol) de chlorure de thionyle dans 2.5 mL de méthanol anhydre est alors additionnée lentement. Le milieu réactionnel est agité à 70°C pendant 24 heures puis refroidi à température ambiante. Le solide est filtré puis le filtrat est évaporé. Le résidu est purifié par chromatographie sur silice (éluant dichlorométhane/méthanol/acétonitrile 94/3/3) pour conduire à 10 mg (5%) de 8-cyclopentylsulfamoyl-4-oxo-4,5-dihydro-3*H*-pyrrolo[2,3-*c*]quinoline-1-carboxylate de méthyle sous la forme d'un solide beige contaminé par 20% de son analogue carboxylate d'éthyle.

***LCMS(IE, m/z) :*** *(M+1) 389.92*

***RMN$^1$H :*** *$\delta_H$ ppm 400 MHz, DMSO*

*13.19 (1H, s, NH), 12.05 (1H, s, NH), 9.83 (1H, d, $CH_{arom}$), 8.07 (1H, s, $CH_{arom}$), 7.56-7.51 (2H, m, $CH_{arom}$ et NH), 7.54 (1H, d, $CH_{arom}$), 3.86 (3H, s, $CH_3$), 3.53-3.45 (1H, m, $CH_{pen}$) 1.55-1.48 (4H, m, $4xCH_{pen}$), 1.40-1.22 (6H, m, $6xCH_{pen}$).*

**8-(Octahydro-pyrrolo[3,4-*c*]pyrrole-2-sulfonyl)-4-oxo-4,5-dihydro-3*H*-pyrrolo[2,3-*c*]quinoline-1-carboxylate d'éthyle (93)**

A 200 mg (0.61 mmol) d'acide 8-chlorosulfonyl-4-oxo-4,5-dihydro-3*H*-pyrrolo[2,3-*c*]quinoline-1-carboxylique (synthèse 2) en suspension dans 3.5 mL de dichlorométhane anhydre et 1 mL de diméthylformamide anhydre, sont ajoutés 250 µL (1.83 mmol) de triéthylamine et 175 µL (0.57 mmol) de chlorhydrate de 2-benzyl-octahydro-pyrrolo[3,4-*c*]pyrrole (Composé obtenu selon le protocole décrit par A. Madin, US 006107321). Le milieu réactionnel est agité 5 heures à température ambiante. Les solvants sont évaporés puis le résidu est repris dans 2 mL d'éthanol anhydre. Une solution de 450 µL (6.1 mmol) de chlorure de thionyle dans 2.5 mL d'éthanol anhydre est alors additionnée lentement. Le milieu réactionnel est agité à 70°C pendant 24 heures puis refroidi à température ambiante. Le solide est filtré puis le filtrat est évaporé. Le résidu est purifié par chromatographie sur silice (éluant chloroforme/méthanol/ammoniaque 80/5/2) pour conduire à 70 mg d'un solide jaune. Le solide est dissous dans 5 mL d'éthanol. La réaction est agitée sous atmosphère d'hydrogène avec addition régulière de palladium 10% sur charbon et d'une solution aqueuse 1M d'acide chlorhydrique jusqu'à complète disparition du produit de départ. Le milieu réactionnel est filtré sur célite et le filtrat évaporé. Le résidu est purifié par chromatographie sur silice (éluant chloroforme/méthanol/ammoniaque 65/25/4) pour conduire à 18 mg (7% sur trois étapes) de 8-(octahydro-pyrrolo[3,4-*c*]pyrrole-2-sulfonyl)-4-oxo-4,5-dihydro-3*H*-pyrrolo[2,3-*c*]quinoline-1-carboxylate d'éthyle sous la forme d'un solide blanc.

***LCMS(IE, m/z)*** *: (M+1) 431.13*

*RMN **$^1$H** : $\delta_H$ ppm 400 MHz, DMSO*

*12.15 (1H, s, NH), 9.80 (1H, d, $CH_{arom}$), 8.08 (1H, s, $CH_{arom}$), 7.78 (1H, dd, $CH_{arom}$), 7.62 (1H, d, $CH_{arom}$), 4.34 (2H, q, $CH_2$), 3.22-3.17 (2H, m, $2xCH_{pyr}$) 3.08-3.01 (2H, m, $2xCH_{pyr}$), 2.95-2.80 (6H, m, $6xCH_{pyr}$), 1.36 (3H, t, $CH_3$).*

**8-(1-Azabicyclo[2.2.2]oct-3-ylsulfamoyl)-4-oxo-4,5-dihydro-3*H*-pyrrolo[2,3-*c*]quinoline-1-carboxylate d'éthyle (94)**

Ce composé est préparé selon la synthèse 25, à partir de 200 mg (0.6 mmol) d'acide 8-chlorosulfonyl-4-oxo-4,5-dihydro-3*H*-pyrrolo[2,3-*c*]quinoline-1-carboxylique (synthèse 2), de 100 mg (0.8 mmol) de 3-aminoquinuclidine (sous forme libre) et de 170 µL (1.22 mmol) de triéthylamine. Après purification par chromatographie sur silice (éluant chloroforme/méthanol/ammoniaque 85/13/2), 11 mg (4%) de 8-(1-azabicyclo[2.2.2]oct-3-ylsulfamoyl)-4-oxo-4,5-dihydro-3*H*-pyrrolo[2,3-*c*]quinoline-1-carboxylate d'éthyle sont obtenus sous forme d'un solide marron
***LCMS(IE, m/z)** : (M+1) 444.83*
***RMN¹H** : $\delta_H$ ppm 400 MHz, DMSO*
*13.10 (1H, s, NH), 12.07 (1H, s, NH), 9.81 (1H, d, $CH_{arom}$), 8.06 (1H, s, $CH_{arom}$), 7.81 (1H, dd, $CH_{arom}$), 7.75 (1H, d, NH), 7.53 (1H, d, $CH_{arom}$), 4.33 (2H, q, $CH_2$), 3.27-3.22 (1H, m, $CH_{qui}$), 2.91-2.82 (1H, m, $CH_{qui}$), 2.80-2.65 (1H, m, $CH_{qui}$), 2.60-2.36 (3H, m, $3xCH_{qui}$), 1.79-1.69 (1H, m, $CH_{qui}$), 1.68-1.61 (1H, m, $CH_{qui}$), 1.49-1.32 (5H, m, $2xCH_{qui}$ et $CH_3$), 1.24-1.15 (1H, m, $CH_{qui}$).*

**8-(4-Méthyl-pipérazine-1-sulfonyl)-4-oxo-4,5-dihydro-3*H*-pyrrolo[2,3-*c*]quinoline-1-carboxylate de méthyle (95)**

A une suspension de 38 mg (0.1 mmol) d'acide 8-(4-méthyl-pipérazine-1-sulfonyl)-4-oxo-4,5-dihydro-3*H*-pyrrolo[2,3-*c*]quinoline-1-carboxylique dissous dans 1 mL de diméthylformamide et 1 mL de méthanol sont additionnés 60 µL (0.12 mmol) d'une solution 2M dans l'éther diéthylique de triméthylsilyldiazométhane. Après suivi par analyse LC/MS, 60 µL (0.12 mmol) de la solution 2M dans l'éther diéthylique de triméthylsilyldiazométhane sont de nouveau ajoutés puis la réaction est agitée une heure. Le précipité est alors collecté par filtration puis lavé à l'eau, à l'éther diisopropylique puis séché à l'étuve à vide pour obtenir 10 mg (17%) de 8-(4-méthyl-pipérazine-1-sulfonyl)-4-oxo-4,5-dihydro-3*H*-pyrrolo[2,3-*c*]quinoline-1-carboxylate de méthyle sous forme d'un solide blanc.
***LCMS (IE, m/z):** (M+1) 404.87*
***RMN¹H** : $\delta_H$ ppm 400 MHz, DMSO*
*13.25 (1H, sl, NH), 12.12 (1H, s, NH), 9.80 (1H, s, $CH_{arom}$), 8.11 (1H, s, $CH_{arom}$), 7.76 (1H, dd, $CH_{arom}$), 7.61 (1H, d, $CH_{arom}$), 3.86 (3H, s, $CH_3$), 2.95 (4H, m, $2xCH_2$), 2.39 (4H, m, $2xCH_2$).*

L'acide 8-(4-méthyl-pipérazine-1-sulfonyl)-4-oxo-4,5-dihydro-3*H*-pyrrolo[2,3-*c*]quinoline-1-carboxylique est préparé selon la synthèse 25, à partir de 440 mg (0.75 mmol) d'acide 8-chlorosulfonyl-4-oxo-4,5-dihydro-3*H*-pyrrolo[2,3-*c*]quinoline-1-carboxylique (synthèse 2) et de 164 µl (1.48 mmol) de 1-méthyl-pipérazine pour donner après lavage à l'eau 340 mg (65%) d'acide 8-(4-méthyl-pipérazine-1-sulfonyl)-4-oxo-4,5-dihydro-3*H*-pyrrolo[2,3-*c*]quinoline-1-carboxylique sous forme d'un solide blanc .

**4-Oxo-8-sulfamoyl-4,5-dihydro-3*H*-pyrrolo[2,3-*c*]quinoline-1-carboxylate** d'éthyle (96)

Dans un tube scellé, 490 mg (1.5 mmol) d'acide 8-chlorosulfonyl-4-oxo-4,5-dihydro-3*H*-pyrrolo[2,3-*c*]quinoline-1-carboxylique (synthèse 2) sont dissous dans 1.5 mL d'une solution aqueuse d'ammoniaque à 28%. Le mélange est agité à 100°C pendant 4 heures. La solution est refrodie puis les solvants sont évaporés. Le résidu est repris dans 10 mL d'éthanol anhydre. Une solution de 1.1 mL (15 mmol) de chlorure de thionyle dans 4.5 mL d'éthanol anhydre est alors additionnée lentement. Le milieu réactionnel est agité à 70°C pendant 48 heures puis refroidi à température ambiante. Les solvants sont évaporés et le résidu est purifié par chromatographie sur silice (éluant chloroforme/méthanol/ammoniaque 85/13/2) pour conduire à 18 mg (3%) de 4-oxo-8-sulfamoyl-4,5-dihydro-3*H*-pyrrolo[2,3-*c*]quinoline-1-carboxylate d'éthyle sous la forme d'un solide beige ;
***LCMS(IE, m/z)** : (M+1) 335.98*
***RMN¹H** : $\delta_H$ ppm 400 MHz, DMSO*
*13.08 (1H, s, NH), 12.01 (1H, s, NH), 9.82 (1H, d, $CH_{arom}$), 8.05 (1H, s, $CH_{arom}$), 7.84 (1H, dd, $CH_{arom}$), 7.53 (1H, d, $CH_{arom}$), 7.26 (2H, s, $NH_2$), 4.34 (2H, q, $CH_2$), 1.36 (3H, t, $CH_3$).*

**(S) 4-Oxo-8-[(pyrrolidin-3-méthyl)-sulfamoyl]-4,5-dihydro-3*H*-pyrrolo[2,3-*c*]quinoline-1-carboxylate d'éthyle (97)**

Ce composé est préparé selon la synthèse 25, à partir de 392 mg (1.2 mmol) d'acide 8-chlorosulfonyl-4-oxo-4,5-dihydro-3*H*-pyrrolo[2,3-*c*]quinoline-1-carboxylique (synthèse 2), de 252 mg (1.26 mmol) de (R)-2-(aminométhyl)-1-*tert*-butoxycarbony]-pyrrolidine et de 200 µL (1.44 mmol) de triethylamine. Après purification par chromatographie sur silice (éluant chloroforme/méthanol/ammoniaque 80/16/4), 25 mg (4%) de (S)-4-oxo-8-[(pyrrolidin-3-méthyl)-sulfamoyl]-4,5-dihydro-3*H*-pyrrolo[2,3-*c*]quinoline-1-carboxylate d'éthyle sont obtenus sous forme d'un solide blanc
***LCMS(IE, m/z)** : (M+1) 418.93*

***RMN$^1$H :** $\delta_H$ ppm 400 MHz, DMSO*
*13.19 (1H, s, NH), 12.10 (1H, s, NH), 9.84 (1H, d, $CH_{arom}$), 8.87 (1H, sl, NH), 8.07 (1H, s, $CH_{arom}$), 7.95 (1H, s, NH), 7.84 (1H, dd, $CH_{arom}$), 7.59 (1H, d, $CH_{arom}$), 4.34 (2H, q, $CH_2$), 3.60-3.50 (1H, m, CH), 3.21-2.98 (4H, m, 4xCH), 2.21-1.95 (1H, m, CH), 1.94-1.78 (2H, m, 2xCH), 1.66-1.55 (1H, m, CH), 1.36 (3H, t, $CH_3$).*

**8-(4-Hydroxy-phénylsulfamoyl)-4-oxo-4,5-dihydro-3*H*-pyrrolo[2,3-*c*]quinoline-1-carboxylate d'ethyle (98)**

Ce composé est préparé selon la synthèse 25, à partir de 440 mg (0.75 mmol) d'acide 8-chlorosulfonyl-4-oxo-4,5-dihydro-3*H*-pyrrolo[2, 3-*c*]quinoline-1-carboxylique (synthèse 2) et de 161 mg (1.48 mmol) de 4-hydroxyaniline. Après trituration dans un mélange éthanol/éther diisopropylique puis lavage à l'eau et séchage, 33 mg (11%) de 8-(4-hydroxy-phénylsulfamoyl)-4-oxo-4,5-dihydro-3*H*-pyrrolo[2,3-*c*]quinoline-1-carboxylate d'éthyle sont obtenus sous forme d'un solide blanc.
***LCMS (IE, m/z):** (M+1) 427.77*
***RMN$^1$H :** $\delta_H$ ppm 400 MHz, DMSO*
*13.15 (1H, sl, NH), 12.01 (1H, s, NH), 9.71 (1H, s, $CH_{arom}$), 9.67 (1H, s, OH), 9.20 (1H, s, NH), 8.03 (1H, s, $CH_{arom}$), 7.63 (1H, dd, $CH_{arom}$), 7.47 (1H, d, $CH_{arom}$), 6.88 (2H, d, 2x$CH_{arom}$), 6.56 (2H, d, 2x$CH_{arom}$), 4.33 (2H, q, $CH_2$), 1.35 (3H, t, $CH_3$).*

**8-Fluoro-4-oxo-4,5-dihydro-3*H*-pyrrolo[2,3-*c*]quinoline-1-carboxylate d'éthyle (99)**

Ce composé a été synthétisé selon la synthèse 1, à partir de 5.8 g (38 mmol) de 5-fluoro-1,3dihydro-indol-2-one pour donner après recristallisation dans l'acide acétique, 805 mg (7% sur deux étapes) de 8-fluoro-4-oxo-4,5-dihydro-3*H*-pyrrolo[2,3-*c*]quinoline-1-carboxylate d'éthyle cocristallisé avec un équivalent d'acide acétique sous la forme d'un solide gris
***LCMS(IE, m/z) :** (M+1) 275.05*
***RMN$^1$H :** $\delta_H$ ppm 400 MHz, DMSO*
*13.12 (1H, s, NH), 11.78 (1H, s, NH), 9.12-9.07 (1H, m, $CH_{arom}$), 8.02 (1H, s, $CH_{arom}$), 7.45-7.40 (1H, m, $CH_{arom}$), 7.34-7.25 (1H, m, $CH_{arom}$), 4.32 (2H, q, $CH_2$), 1.34 (3H, t, $CH_3$).*

**8-Fluoro-4-oxo-4,5-dihydro-3*H*-pyrrolo[2,3-*c*]quinoline-1-carboxylate** de 2-**diéthylaminoéthyle (100)**

A une solution de 123 mg (0.5 mmol) d'acide 8-fluoro-4-oxo-4,5-dihydro-3*H*-pyrrolo[2,3-*c*]quinoline-1-carboxylique dissous dans 0.5 mL de diméthylformamide sont additionnés 104 mg (0.4 mmol) de bromhydrate de 2-bromoéthyldiéthylamine et 163 mg (0.5 mmol) de carbonate de césium. Le mélange réactionnel est agité 2 heures à température ambiante puis de l'eau est additionné et le solide est filtré. Ce dernier est purifié par chromatographie sur silice (éluant dichlorométhane/méthanol/triéthylamine 95/5/0.1) puis trituré dans le méthanol pour conduire à 13 mg (7 %) de 8-fluoro-4-oxo-4,5-dihydro-3*H*-pyrrolo[2,3-*c*]quinoline-1-carboxylate de 2-diéthylaminoéthyle sous la forme d'un solide blanc.
***LCMS (IE, m/z):** (M+1) 345.97*
***RMN$^1$H :** $\delta_H$ ppm 400 MHz, DMSO*
*13.12 (1H, sl, NH), 11.77 (1H, s, NH), 9.08 (1H, d, $CH_{arom}$), 8.05-7.97 (1H, m, $CH_{arom}$), 7.47-7.41 (1H, m, $CH_{arom}$), 7.33-7.27 (1H, m, $CH_{arom}$), 4.42-4.28 (2H, m, $CH_2$), 2.87-2.70 (2H, m, $CH_2$), 2.66-2.45 (4H, m, 2x$CH_2$), 1.15-0.91 (6H, m, 2x$CH_3$).*
L'acide 8-fluoro-4-oxo-4,5-dihydro-3*H*-pyrrolo[2,3-*c*]quinoline-1-carboxylique est préparé selon la synthèse 75 à partir de 403 mg (1.47 mmol) de 8-fluoro-4-oxo-4,5-dihydro-3*H*-pyrrolo[2,3-*c*]quinoline-1-carboxylate d'éthyle et de 616 mg (14.7 mmol) d'hydroxyde de lithium monohydraté pour conduire après traitement acido-basique à 365 mg (quantitatif) d'acide 8-fluoro-4-oxo-4,5-dihydro-3*H*-pyrrolo[2,3-*c*]quinolino-1-carboxylique sous la forme d'un solide rose pâle.

**8-Acétyl-4-oxo-4,5-dihydro-3*H*-pyrrolo[2,3-*c*]quinoline-1-carboxylate d'éthyle (101)**

Ce composé a été synthétisé selon la synthèse 1, à partir du 5-acétyl-1,3-dihydro-indol-2-one pour donner après précipitation dans 1N HCl et lavage à l'eau et à l'éther diisopropylique, 1.8 g (22% sur deux étapes) de 8-acétyl-4-oxo-4,5-dihydro-3*H*-pyrrolo[2,3-*c*]quinoline-1-carboxylate d'ethyle sous la forme d'un solide marron.
***LCMS(IE, m/z) :** (M+1) 298.94*
***RMN$^1$H :** $\delta_H$ ppm 400 MHz, DMSO*
*13.13 (1H, sl, NH), 11.99 (1H, s, NH), 10.01 (1H, s, $CH_{arom}$), 8.04 (1H, s, $CH_{arom}$), 7.96 (1H, d, $CH_{arom}$), 7.47 (1H, d, $CH_{arom}$), 4.34 (2H, q, $CH_2$), 2.62 (3H, s, $CH_3$), 1.35 (3H, t, $CH_3$).*
La synthèse du 5-acétyl-1,3-dihydro-indol-2-one est décrite par Peng Cho Tang (US2003/0069421 A1). Ce

composé a été préparé selon ce protocole à partir de 6.65 g (50 mmol) de 1,3-dihydro-indol-2-one, de trichlorure d'aluminium (150 mmol) et de chlorure d'acétyle (100 mmol) pour donner après trituration dans l'eau et séchage, 7.32 g (83 %) de 5-acétyl-1,3-dihydro-indol-2-one sous la forme d'un solide beige.

**8-Benzoyl-4-oxo-4,5-dihydro-3*H*-pyrrolo[2,3-*c*]quinoline-1-carboxylate d'éthyle (102)**

Ce composé a été synthétisé selon la synthèse 1, à partir du 5-benzoyl-1,3-dihydro-indol-2-one pour donner après précipitation dans 1N HCl et lavage à l'eau et à l'éther diisopropylique, 7 mg (3% sur deux étapes) de 8-benzoyl-4-oxo-4,5-dihydro-3*H*-pyrrolo[2,3-*c*]quinoline-1-carboxylate d'ethyle sous la forme d'un solide marron.
***LCMS(IE, m/z) :*** *(M+1) 361.06*
***RMN¹H :*** *$\delta_H$ ppm 400 MHz, DMSO*
*13.14 (1H, sl, NH), 12.04 (1H, s, NH), 9.76 (1H, s, $CH_{arom}$), 8.02 (1H, s, $CH_{arom}$), 7.83-7.38 (7H, m, $7xCH_{arom}$), 4.18 (2H, q, $CH_2$), 1.24 (3H, t, $CH_3$).*

La synthèse du 5-benzoyl-1,3-dihydro-indol-2-one est décrite par McNutt (WO99/10325). Ce composé a été préparé selon ce protocole à partir de 665 g (5 mmol) de 1,3-dihydro-indol-2-one, de trichlorure d'aluminium (15 mmol) et de chlorure de benzoyle (10 mmol) pour donner après trituration dans l'eau et séchage, 880 mg (74 %) de 5-benzoyl-1,3-dihydro-indol-2-one sous la forme d'un solide beige.

**Acide 8-[(4-fluoro-phényl)-méthyl-sulfamoyl]-4-oxo-4,5-dihydro-3*H*-pyrrolo [2,3-*c*] quinolinc-1-carboxylique (103)**

Ce composé est préparé selon la synthèse 75 à partir de 412 mg (0.93 mmol) de 8-[(4-fluoro-phényl)-méthyl-sulfamoyl]-4-oxo-4,5-dihydro-3*H*-pyrrolo[2,3-*c*]quinoline-1-carboxylate d'éthyle et de 360 mg (13.95 mmol) d'hydroxyde de lithium monohydraté pour conduire après traitement acido-basique et précipitation dans une phase aqueuse acide (pH 5) à 200 mg (52%) d'acide 8-[(4-fluoro-phényl)-méthyl-sulfamoyl]-4-oxo-4,5-dihydro-3*H*-pyrrolo[2,3-*c*]quinoline-1-carboxylique sous la forme d'un solide rose pâle.
***LCMS (IE, m/z):*** *(M+1) 415.98*
***RMN¹H :*** *$\delta_H$ ppm 400 MHz, $CD_3OD$*
*10.03 (1H, s, $CH_{arom}$), 8.08 (1H, s, $CH_{arom}$), 7.44 (2H, s, $2xCH_{arom}$), 7.17-7.14 (2H, m, $2xCH_{arom}$), 7.03 (2H, t, $2xCH_{arom}$), 3.28 (3H, t, $NCH_3$).*

**8-[(4-Fluoro-phényl)-méthyl-sulfamoyl]-4-oxo-4,5-dihydro-3*H*-pyrrolo[2,3-*c*]quinoline-1-carboxylate de 2-hydroxyéthyle (104)**

A une solution de 62 mg (0.15 mmol) d'acide 8-[(4-fluoro-phényl)-méthyl-sulfamoyl]-4-oxo-4,5-dihydro-3*H*-pyrrolo[2,3-*c*]quinoline-1-carboxylique dissous dans 2 mL d'éthylène glycole est additionné 5 µL d'une solution 2M d'acide chlorhydrique dans l'éther diéthylique. Le milieu réactionnel est agitée à 80°C pendant 20 heures. De l'eau est additionnée et le composé est, extrait avec un mélange dichlorométhane/méthanol (95/5). Les phases organiques sont séchées sur sulfate de magnésium, filtrées et évaporées et le résidu est purifié par chromatoghraphie sur silice (éluant dichlorométhane/méthanol 95/5) pour conduire à 25 mg (36%) de 8-[(4-fluoro-phényl)-méthyl-sulfamoyl]-4-oxo-4,5-dihydro-3*H*-pyrrolo[2,3-*c*]quinoline-1-carboxylate de 2-hydroxyéthyle sous la forme d'un produit brun.
***LCMS (IE, m/z):*** *(M+1) 460.14*
***RMN¹H :*** *$\delta_H$ ppm 400 MHz, $CD_3OD$*
*9.87 (1H, s, $CH_{arom}$), 8.23 (1H, s, $CH_{arom}$), 7.52-7.48 (2H, m, $2xCH_{arom}$), 7.17-7.14 (2H, m, $2xCH_{arom}$), 7.05-7.01 (2H, m, $2xCH_{arom}$), 4.40 (2H, t, $OCH_2$), 3.91 (2H, t, $CH_2$), 3.26 (3H, t, $NCH_3$).*

**Trifluoroacétate de 8-[(4-fluoro-phényl)-méthyl-sulfamoyl]-4-oxo-4,5-dihydro-3*H*-pyrrolo[2,3-*c*]quinoline-1-carboxylate de 2-aminoéthyle (105)**

A une solution de 5 mg (0.01 mmol) de 8-[(4-fluoro-phényl)-méthyl-sulfamoyl]-4-oxo-4,5-dihydro-3*H*-pyrrolo [2,3-*c*]quinoline-1-carboxylale de 2-*tert*-butoxycarbonylaminoéthyle dissous dans 1 mL de dichloroéthane sont additionnés 10 µL d'acide trifluoroacétique. La solution est agitée 18 heures à température ambiante puis le résidu est recristallisé dans un mélange dichlorométhane/éther diéthylique pour conduire à 4 mg (80%) de trifluoroacétate de 8-[(4-fluoro-phényl)-méthyl-sulfamoyl]-4-oxo-4,5-dihydro-3*H*-pyrrolo[2,3-*c*]quinoline-1-carboxylate de 2-aminoéthyle sous forme d'un solide beige.
***LCMS (IE, m/z):*** *(M+1) 459.26*
***RMN¹H*** *: $\delta_H$ ppm 400 MHz, $CD_3OD$*
*9.77 (1H, s, $CH_{arom}$), 8.29 (1H, s, $CH_{arom}$), 7.52-7.50 (2H, m, $2xCH_{arom}$), 7.15-7.13 (2H, m, $2xCH_{arom}$), 7.06-7.04 (2H,*

*m, 2xCH$_{arom}$), 4.56 (2H, t, OCH$_2$), 3.61-3.59 (2H, m, NCH$_2$), 3.24 (3H, t, NCH$_3$).*

**8-[(4-Fluoro-phényl)-méthyl-sulfamoyl]-4-oxo-4,5-dihydro-3*H*-pyrrolo[2,3-*c*]quinoline-1-carboxylate de 2-*tert*-butoxycarbonylaminoéthyle (106)**

A une solution de 20 mg (0.05 mmol) d'acide 8-[(4-fluoro-phényl)-mélhyl-sulfamoyl]-4-oxo-4,5-dihydro-3*H*-pyrrolo[2,3-*c*]quinoline-1-carboxylique dissous dans 0.5 mL de diméthylformamide sont additionnés 7 mg (0.03 mmol) de 2-bromo-*N*-*tert*-butoxycarbonyl-éthylamine et 19 mg (0.06 mmol) de carbonate de césium. Le mélange réactionnel est agité 18 heures à température ambiante puis les solvants sont évaporés. Le résidu est purifié par chromatographie sur silice (éluant cyclohexane/acétate d'éthyle 2/8) puis trituré dans l'éther diéthylique pour conduire à 5 mg (18 %) de 8-[(4-fluoro-phényl)-méthyl-sulfamoyl]-4-oxo-4,5-dihydro-3*H*-pyrrolo[2,3-*c*]quinoline-1-carboxylatede3-*tert*-butoxycarbonylaminoéthyle sous la forme d'un solide blanc.
***LCMS (IE, m/z):*** *(M+1) 559.19*
***RMN$^1$H :*** *$\delta_H$ ppm 400 MHz, CD$_3$OD*
*9.84 (1H, s, CH$_{arom}$), 8.16 (1H, s, CH$_{arom}$), 7.46-7.44 (2H, m, 2xCH$_{arom}$), 7.14-7.03 (2H, m, 2xCH$_{arom}$), 7.00-6.98 (2H, m, 2xCH$_{arom}$), 4.42 (2H, t, OCH$_2$), 3.60-3.45 (2H, m, NCH$_2$), 3.23 (3H, t, NCH$_3$), 1.41 (9H, s, t-Bu).*

**Trifluoroacétate de 8-[(4-fluoro-phényl)-méthyl-sulfamoyl]-4-oxo-4,5-dihydro-3*H*-pyrrolo[2,3-*c*]quinoline-1-carboxylate de 3-aminopropyle (107)**

A une solution de 45 mg (0.08 mmol) de 8-[(4-fluoro-phényl)-méthyl-sulfamoyl]-4-oxo-4,5-dihydro-3*H*-pyrrolo [2,3-*c*]quinoline-1-carboxylate de 2-*tert*-butoxycarbonylaminopropyle dissous dans 1 mL de dichloroéthane sont additionnés 100 µL d'acide trifluoroacétique. La solution est agitée 18 heures à température ambiante puis le résidu est recristallisé dans un mélange dichlorométhane/éther diéthylique pour conduire à 12 mg (26%) de trifluoroacétate de 8-[(4-fluoro-phényl)-méthyl-sulfamoyl]-4-oxo-4,5-dihydro-3*H*-pyrrolo[2,3-*c*]quinoline-1-carboxylate de 2-aminopropyle sous la forme d'un solide beige.
***LCMS (IE, m/z):*** *(M+1) 473.21*
***RMN$^1$H :*** *$\delta_H$ ppm 400 MHz, CD$_3$OD*
*9.71 (1H, s, CH$_{arom}$), 8.08 (1H, s, CH$_{arom}$), 7.45 (2H, dd, 2xC$_{arom}$), 7.09-7.06 (2H, m, 2xCH$_{arom}$), 6.99-6.97 (2H, m, 2xCH$_{arom}$), 4.37 (2H, t, OCH$_2$), 3.17 (3H, t, NCH$_3$), 3.10-3.07 (2H, m, NCH$_2$), 2.11-2.09 (2H, m, CH$_2$).*

**8-[(4-Fluoro-phényl)-méthyl-sulfamoyl]-4-oxo-4,5-dihydro-3*H*-pyrrolo[2,3-*c*]quinolinc-1-carboxylate de 3-*tert*-butoxycarbonylamino-propyle (108)**

A une solution de 21 mg (0.05 mmol) d'acide 8-[(4-fluoro-phényl)-méthyl-sulfamoyl]-4-oxo-4,5-dihydro-3*H*-pyrrolo[2,3-*c*]quinoline-1-carboxylique dissous dans 0.5 mL de diméthylformamide sont additionnés 9 mg (0.04 mmol) de 3-bromo-*N*-*tert*-butoxycarbonyl-propylamine et 8 mg (0.06 mmol) de carbonate de potassium. Le mélange réactionnel est agité 18 heures à température ambiante puis les solvants sont évaporés. Le résidu est purifié par chromatographie sur silice (éluant cyclohexane/acétate d'éthyle 2/8) puis trituré dans l'éther diéthylique pour conduire à 6 mg (21 %) de 8-[(4-fluoro-phényl)-méthyl-sulfamoyl]-4-oxo-4,5-dihydro-3*H*-pyrrolo[2,3-*c*]quinoline-1-carboxylate de 3-*tert*-butoxycarbonylamino-propyle sous la forme d'un solide blanc.
***LCMS (IE, m/z):*** *(M+1) 573.26*
***RMN$^1$H :*** *$\delta_H$ ppm 400 MHz, CD$_3$OD*
*9.72 (1H, s, CH$_{arom}$), 8.04 (1H, s, CH$_{arom}$), 7.39-7.36 (2H, m, 2xCH$_{arom}$), 7.05-7.02 (2H, m, 2xCH$_{arom}$), 0.93-0.91 (2H, m, 2xCH$_{arom}$), 4.25 (2H, t, OCH$_2$), 3.25 (2H, m, NCH$_2$), 3.14 (3H, t, NCH$_3$), 1.86-1.83 (2H, m, CH$_2$) 1.33 (9H, s, t-Bu).*

***N*-Propyl-8-diméthylsulfamoyl-4-oxo-4,5-dihydro-3*H*-pyrrolo[2,3-*c*]quinoline-1-carboxamide (109).**

Ce composé est préparé selon la synthèse 79 à partir de 100 mg (0.3 mmol) d'acide 8-diméthylsulfamoyl-4-oxo-4,5-dihydro-3*H*-pyrrolo[2,3-*c*]quinoline-1-carboxylique et de 27 µL (0.33 mmol) de propylamine pour donner après purification par chromatographie sur colonne (éluant dichlorométhanelméthanol/acétonitrile 90/5/5), 45 mg (40%) de *N*-propyl-8-diméthylsulfamoyl-4-oxo-4,5-dihydro-3*H*-pyrrolo[2,3-*c*]quinoline-1-carboxamide sous la forme d'un solide blanc.
***LCMS (IE, m/z):*** *(M+1) 377.16*
***RMN$^1$H :*** *$\delta_H$ ppm 400 MHz, DMSO*
*12.79 (1H, sl, NH), 11.94 (1H, sl, NH), 9.62 (1H, d, CH$_{arom}$), 8.25 (1H, t, NH), 7.92 (1H, s, CH$_{arom}$), 7.69 (1H, dd, CH$_{arom}$), 7.54 (1H, d, CH$_{arom}$), 3.24 (2H, q, CH$_2$), 2.63 (6H, s, 2x CH$_3$), 1.56 (2H, sext, CH$_2$), 0.92 (3H, t, CH$_3$).*
L'acide 8-diméthylsulfamoyl-4-oxo-4,5-dihydro-3*H*-pyrrolo[2,3-*c*]quinoline-1-carboxylique est préparé selon la

synthèse 178, à partir de 326 mg (1 mmol) d'acide 8-chlorosulfonyl-4-oxo-4,5-dihydro-3*H*-pyrrolo[2,3-*c*]quinoline-1-carboxylique et de 85 mg (1.05 mmol) de chlorhydrate de diméthylamine pour donner après purification par précipitation et trituration dans l'eau, 210 mg (62%) d'acide 8-diméthylsulfamoyl4-oxo-4,5-dihydro-3*H*-pyrrolo[2,3-*c*]quinoline-1-carboxylique sous la forme d'un solide gris.

**N-Méthyl-N-propyl-8-diméthylsulfamoyl-4-oxo-4,5-dihydro-3H-pyrrolo[2,3-c]quinoline-1-carboxamide (110)**

Ce composé est préparé selon la synthèse 79 à partir de 100 mg (0.3 mmol) d'acide 8-diméthylsulfamoyl-4-oxo-4,5-dihydro-3*H*-pyrrolo[2,3-*c*]quinoline-1-carboxylique et de 34 μL (0.33 mmol) de *N*-méthyl-propylamine pour donner après purification par chromatographie sur colonne (éluant dichlorométhane/méthanol/acétonitrile 90/5/5), 46 mg (40%) de *N*-méthyl-*N*-propyl-8-diméthylsulfamoyl-4-oxo-4,5-dihydro-3*H*-pyrrolo[2,3-*c*]quinoline-1-carboxamide sous la forme d'un solide beige.

***LCMS (IE, m/z):*** *(M+1) 391.35*

***RMN¹H :*** *$\delta_H$ ppm 400 MHz, DMSO*

*12.77 (1H sl, NH), 11.93 (1H, sl, NH), 8.37-8.19 (1H, m, $CH_{arom}$), 7.69 (1H, dd, $CH_{arom}$), 7.65-7.56 (1H, m, $CH_{arom}$), 7.57 (1H, d, $CH_{arom}$), 3.50-3.34 (2H, m, $CH_2$), 3.01 (3H, tl, $CH_3$), 2.60 (6H, s, 2x$CH_3$), 1.75-1.37 (2H, m, $CH_2$), 1.01-0.53 (3H, m, $CH_3$).*

**8-Cyclohexylsulfamoyl-oxo-4,5-dihydro-3H-pyrrolo[2,3-c]quinoline-1-carboxylate de méthyle (111)**

Ce composé a été obtenu par la méthode d'estérification générale B à partir de 258 mg (0.66 mmol) d'acide 8-cyclohexylsulfamoyl-4-oxo-4,5-dihydro-3*H*-pyrrolo[2,3-*c*]quinoline-1-carboxylique et 995 μL (1.99 mmol) d'une solution 2M dans l'éther diéthylique de triméthylsilyldiazométhane pour donner après purification par chromatographie sur silice (éluant dichlorométhane/méthanol 98/2), 57 mg (21%) de 8-cyclohexylsulfamoyl-4-oxo-4,5-dihydro-3*H*-pyrrolo[2,3-*c*]quinoline-1-carboxylate de méthyle sous la forme d'un solide blanc.

***LC/MS (IE, m/z) :*** *(M+1) 404.05*

***RMN¹H :*** *$\delta_H$ ppm 400 MHz, DMSO*

*13.19 (1H, sl, NH), 12.04 (1H, sl, NH), 9.83 (1H, s, $CH_{arom}$), 8.07 (1H, s, $CH_{arom}$), 7.80 (1H, dd, $CH_{arom}$), 7.54-7.51 (2H, m, $CH_{arom}$, NH), 3.86 (3H, s, $CH_3$), 3.02 (1H, m, CH), 1.62-1.56 (4H, m, 2x$CH_2$), 1.42 (2H, m, $CH_2$), 1.14-0.99 (5H, m, $CH_2$ et $CH_3$).*

A 490 mg (1.5 mmol) d'acide 8-chlorosulfonyl-4-oxo-4,5-dihydro-3*H*-pyrrolo[2,3-*c*]quinoline-1-carboxylique en suspension dans 4 mL de dichlorométhane anhydre et 1 mL de diméthylformamide anhydre, sont ajoutés 314 μL (2.25 mmol) de triéthylamine et 189 μL (1.65 mmol) de cyclohexylamine. Le milieu réactionnel est agité 2 jours à température ambiante. Les solvants sont évaporés puis le résidu est repris dans de l'eau. Le solide est filtré, rincé avec de l'eau puis de l'éther diisopropylique et séché sous vide pour donner 258 mg (44%) d'acide 8-cyclohexylsulfamoyl-4-oxo-4,5-dihydro-3*H*-pyrrolo[2,3-*c*]quinoline-1-carboxylique sous la forme d'un solide crème.

Dans un tube scellé, 1.42 g (4.5 mmol) de 4-oxo-4,5-dihydro-3*H*-pyrrolo[2,3-*c*]quinoline-1-carboxylate d'éthyle cocristallisé avec un équivalent d'acide acétique (synthèse 1) est dissous dans 6 mL (90 mmol) d'acide chlorosulfonique. Le mélange réactionnel est placé dans un bain d'huile préalablement porté à 85°C pendant 20 minutes, refroidi par un bain de glace puis versé lentement sur de la glace pilée. Le solide est filtré, rincé avec de l'eau, de l'éther diisopropylique et du pentane puis séché sous vide pour conduire à 1.47 g (quantitatif) d'acide 8-chlorosulfonyl-4-oxo-4,5-dihydro-3*H*-pyrrolo[2,3-*c*]quinoline-1-carboxylique sous la forme d'un solide marron.

**4-Oxo-8-(pipéridin-3-ylsulfamoyl)-4,5-dihydro-3H-pyrrolo[2,3-c]quinoline-1-carboxylate de méthyle (112)**

A une solution de 172 mg (0.32 mmol) de 8-(1-benzyloxycarbonyl-pipéridin-3-ylsulfamoyl)-4-oxo-4,5-dihydro-3*H*-pyrrolo[2,3-*c*]quinoline-1-carboxylate de méthyle dans 10 mL de méthanol sont additionnés une solution d'acide chlorhydrique 1M dans le méthanol (510 μL, 0.64 mmol) et 17 mg de palladium 10% sur charbon. La solution est agitée à température ambiante pendant 5 jours puis filtrée sur célite. Le filtrat est évaporé, le résidu est repris dans du dichlorométhane et de la triéthylamine est additionnée pour libérer l'amine sous la forme de base libre. Les solvants sont évaporés puis le résidu est purifié par chromatographie sur silice (éluant chloroforme/méthanol/ammoniaque 150/25/4) pour conduire à 56 mg (43%) de 4-oxo-8-(pipéridin-3-ylsulfamoyl)-4,5-dihydro-3*H*-pyrrolo[2,3-*c*]quinoline-1-carboxylate de méthyle sous la forme d'un solide blanc.

***LCMS (IE, m/z):*** *(M+1) 405.05*

***RMN¹H :*** *$\delta_H$ ppm 400 MHz, DMSO*

*12.05 (1H, sl, NH), 9.84 (1H, d, $CH_{arom}$), 8.07 (1H, s, $CH_{arom}$), 7.81 (1H, dd, $CH_{arom}$), 7.63 (1H, sl, NH), 7.54 (1H, d, $CH_{arom}$), 3.86 (3H, s, $CH_3$), 3.09-3.00 (1H, m, $CH_{piper}$), 2.81 (1H, dd, $CH_{piper}$), 2.71 (1H, dd, $CH_{piper}$), 2.37-2.31 (1H, m, $CH_{piper}$), 2.28 (1H, dd, $CH_{piper}$), 1.68-1.60 (1H, m, $CH_{piper}$), 1.55-1.49 (1H, m, $CH_{piper}$), 1.28-1.21 (2H, m, 2x$CH_{piper}$).*

Le 8-(1-benzyloxycarbonyl-pipéridin-3-ylsulfamoyl)-4-oxo-4,5-dihydro-3*H*-pyrrolo[2,3-*c*]quinoline-1-carboxylate de méthyle est préparé selon la synthèse 111, à partir de 326 mg (1 mmol) d'acide 8-chlorosulfonyl-4-oxo-4,5-dihydro-3*H*-pyrrolo[2,3-*c*]quinoline-1-carboxylique et de 246 mg (1.05 mmol) de 3-amino-1-benzyloxycarbonyl-pipéridine pour donner après estérification suivant la méthode générale B et purification par trituration dans l'eau, 172 mg (32% sur deux étapes) de 8-(1-benzyloxycarbonyl-pipéridin-3-ylsulfamoyl)-4-oxo-4,5-dihydro-3*H*-pyrrolo[2,3-*c*]quinoline-1-carboxylate de méthyle sous la forme d'un solide blanc.

**4-Oxo-8-(pipéridin-4-ylsulfamoyl)-4,5-dihydro-3*H*-pyrrolo[2,3-*c*]quinoline-1-carboxylate** de **méthyle (113)**

Ce composé est préparé selon la synthèse 111, à partir de 294 mg (0.9 mmol) d'acide 8-chlorosulfonyl-4-oxo-4,5-dihydro-3*H*-pyrrolo[2,3-*c*]quinoline-1-carboxylique, de 276 μL (1.99 mmol) de triéthylamine et de 224 mg (0.95 mmol) de chlorhydrate de 4-amino-1-*tert*-butoxycarbonyl-pipéridine pour donner après estérification suivant la méthode générale A (méthanol comme alcool utilisé) et purification par chromatographie sur silice (éluant chloroforme/méthanol/ammoniaque 90/25/4), 205 mg (52% sur deux étapes) de 4-oxo-8-(pipéridin-4-ylsulfamoyl)-4,5-dihydro-3*H*-pyrrolo[2,3-*c*]quinoline-1-carboxylate de méthyle sous la forme d'un solide jaune clair.
***LCMS (IE, m/z):*** *(M+1) 419.05*
***RMN$^1$H :*** *$\delta_H$ ppm 400 MHz, DMSO*
*13.21 (1H, sl, NH), 12.10 (1H, sl, NH), 9.84 (1H, d, $CH_{arom}$), 8.08 (1H, s, $CH_{arom}$), 7.92 (1H, d, NH), 7.83 (1H, dd, $CH_{arom}$), 7.57 (1H, d, $CH_{arom}$), 3.86 (3H, s, $CH_3$), 3.40-3.31 (1H, m, $CH_{piper}$), 3.19-3.10 (2H, m, $2xCH_{piper}$), 2.91-2.80 (2H, m, $2xCH_{piper}$), 1.84-1.74 (2H, m, $2xCH_{piper}$), 1.65-1.54 (2H, m, $2xCH_{piper}$).*

**4-Oxo-8-(pipéridin-4-ylsulfamoyl)-4,5-dihydro-3*H*-pyrrolo[2,3-*c*]quinoline-1-carboxylate** de **propyle (114).**

A une solution de 150 mg (0.3 mmol) d'acide 8-(1-*tert*-butoxycarbonyl-pipéridin-4-ylsulfamoyl)-4-oxo-4,5-dihydro-3*H*-pyrrolo[2,3-*c*]quinoline-1-carboxylique dissous dans 2 mL de diméthylformamide anhydre sont additionnés 107 μL (0.76 mmol) de triéthylamine et 100 μL (0.61 mmol) de di-*n*-propylsulfate. La solution est agitée à température ambiante pendant 20 heures puis 43 μL (0.3 mmol) de triéthylamine et 51 μL (0.3 mmol) de di-*n*-propylsulfate sont de nouveau additionnés. L'agitation est poursuivie pendant 24 heures supplémentaires et 3 mL d'ammoniaque sont additionnés. Après 15 minutes d'agitation supplémentaires, de l'eau est additionné et le produit est extrait avec du dichlorométhane. Les phases organiques sont séchées sur sulfate de magnésium, filtrées puis évaporées. Le résidu est dissous dans 2 mL de dichloroéthane et 1 mL d'acide trifluoroacétique. La solution est agitée à température ambiante pendant 1 heure puis les solvants sont évaporés. Le solide est repris dans du dichlorométhane puis de la triéthylamine est additionnée pour libérer l'amine. Les solvants sont de nouveau évaporés et le résidu est purifié par chromatographie sur silice (éluant chloroforme/méthanol/ammoniaque 160/25/4) pour conduire à 44 mg (33% sur deux étapes) de 4-oxo-8-(pipéridin-4-ylsulfamoyl)-4,5-dihydro-3*H*-pyrrolo[2,3-*c*]quinoline-1-carboxylate de propyle sous la forme d'un solide blanc.
*LCMS **(IE, m/z):** (M+1) 433.20*
***RMN$^1$H :*** *$\delta_H$ ppm 400 MHz, DMSO*
*9.82 (1H, d, $CH_{arom}$), 8.05 (1H, s, $CH_{arom}$), 7.80 (1H, dd, $CH_{arom}$), 7.58 (1H, dl, NH), 7.52 (1H, d, $CH_{arom}$), 4.25 (2H, t, $CH_2$), 3.13-3.04 (1H, m, $CH_{pipér}$), 2.82-2.75 (2H, m, $2xCH_{pipér}$), 2.35-2.26 (2H, m, $2xCH_{pipér}$), 1.81-1.72 (2H, m, $CH_2$), 1.56-1.48 (2H, m, $2xCH_{pipér}$), 1.27-1.14 (2H, m, $2xCH_{pipér}$), 0.99 (3H, t, $CH_3$).*
L'acide 8-(1-*tert*-butoxycarbonyl-pipéridin-4-ylsulfamoyl)-4-oxo-4,5-dihydro-3*H*-pyrrolo[2,3-*c*]quinoline-1-carboxylique est préparé selon la synthèse 178, à partir de 2.1 g (6.45 mmol) d'acide 8-chlorosulfonyl-4-oxo-4,5-dihydro-3*H*-pyrrolo[2,3-*c*]quinoline-1-carboxylique, 1.8 mL (12.9 mmol) de triéthylamine et de 1.29 g (6.45 mmol) de 4-amino-1-*tert*-butoxycarbonyl-pipéridine pour donner après purification par précipitation et trituration dans l'eau, 1.86 g (59%) d'acide 8-(1-*tert*-butoxycarbonyl-pipéridin-4-ylsulfamoyl)-4-oxo-4,5-dihydro-3*H*-pyrrolo[2,3-*c*]quinoline-1-carboxylique sous la forme d'un solide beige.

**4-Oxo-8-(pipéridin-4-ylsulfamoyl)-4,5-dihydro-3*H*-pyrrolo[2,3-*c*]quinoline-1-carboxylate de butyle (115).**

Ce composé est préparé selon la synthèse 114, à partir de 100 mg (0.2 mmol) d'acide 8-(1-*tert*-butoxycarbonyl-pipéridin-4-ylsulfamoyl)-4-oxo-4,5-dihydro-3*H*-pyrrolo[2,3-*c*]quinoline-1-carboxylique, 85 μL (0.61 mmol) de triéthylamine et de 102 μL (0.51 mmol) de di-*n*-butylsulfate pour donner après purification par chromatographie sur silice (éluant chloroforme/méthanol/ammoniaque 160/25/4), 31 mg (34% sur deux étapes) de 4-oxo-8-(pipéridin-4-ylsulfamoyl)-4,5-dihydro-3*H*-pyrrolo[2,3-*c*]quinoline-1-carboxylate de butyle sous la forme d'un solide blanc.
***LCMS (IE, mlz):*** *(M+1) 447.01*
***RMN$^1$H :*** *$\delta_H$ ppm 400 MHz, DMSO*
*9.82 (1H, d, $CH_{arom}$), 8.04 (1H, s, $CH_{arom}$), 7.80 (1H, dd, $CH_{arom}$), 7.58 (1H, dl, NH), 7.52 (1H, d, $CH_{arom}$), 4.29 (2H,*

*t, $CH_2$), 3.15-3.04 (1H, m, $CH_{pipér}$), 2.83-2.75 (2H, m, 2x$CH_{pipér}$), 2.35-2.26 (2H, m, 2x$CH_{pipér}$), 1.77-1.69 (2H, m, $CH_2$), 1.56-1.49 (2H, m,2x$CH_{pipér}$), 1.48-1.40 (2H, m, $CH_2$), 1.27-1.15 (2H, m, 2x$C_{pipér}$), 0.95 (3H, t, $CH_3$).*

**4-Oxo-8-(pipéridin-4-ylsulfamoyl)-4,5-dihydro-3*H*-pyrrolo[2,3-*c*]quinoline-1-carboxylate** de **cyclopentyle (116).**

Ce composé est préparé selon la synthèse 114, à partir de 150 mg (0.3 mmol) d'acide 8-(1-*tert*-butoxycarbonyl-pipéridin-4-ylsulfamoyl)-4-oxo-4,5-dihydro-3*H*-pyrrolo[2,3-*c*]quinoline-1-carboxylique, 322 μL (2.31 mmol) de triéthylamine et de 500 mg (2.13 mmol) de dicyclopentylsulfate pour donner après purification par chromatographie sur silice (éluant chloroforme/méthanol/ammoniaque 160/25/4), 80 mg (42% sur deux étapes) de 4-oxo-8-(pipéridin-4-ylsulfamoyl)-4,5-dihydro-3*H*-pyrrolo[2,3-*c*]quinoline-1-carboxylate de cyclopentyle sous la forme d'un solide blanc.
***LCMS (IE, m/z):** (M+1) 459.19*
***RMN$^1$H :** $\delta_H$ ppm 400 MHz, DMSO*
*9.83 (1H, d, $CH_{arom}$), 8.02 (1H, s, $CH_{arom}$), 7.80 (1H, dd, $CH_{arom}$), 7.58 (1H, sl, NH), 7.52 (1H, d, $CH_{arom}$), 5.37-5.32 (1H, m, CH), 3.14-3.05 (1H, m, CH), 2.82-2.75 (2H, m, 2xCH), 2.35-2.26 (2H, m, 2xCH), 1.99-1.89 (2H, m, 2xCH), 1.86-1.72 (4H, m, 4xCH), 1.67-1.49 (4H, m, 4xCH), 1.27-1.15 (2H, m, 2xCH).*

**4-Oxo-8-(pipéridin-4-ylsulfamoyl)-4,5-dihydro-3*H*-pyrrolo[2,3-*c*]quinoline-1-carboxylate de méthylcyclohexyle (117).**

Ce composé est préparé selon la synthèse 114, à partir de 150 mg (0.3 mmol) d'acide 8-(1-*tert*-butoxycarbonyl-pipéridin-4-ylsulfamoyl)-oxo-4,5-dihydro-3*H*-pyrrolo[2,3-*c*]quinoline-1-carboxylique, 496 μL (3.56 mmol) de triéthylamine et de 975 mg (3.35 mmol) de dicyclohexylméthylsulfate pour donner après purification par chromatographie sur silice (éluant chloroforme/méthanol/ammoniaque 160/25/4) puis recristallisation dans du méthanol, 11 mg (7% sur deux étapes) de 4-oxo-8-(pipéridin-4-ylsulfamoyl)-4,5-dihydro-3*H*-pyrrolo[2,3-*c*]quinoline-1-carboxylate de méthylcyclohexyle sous la forme d'un solide blanc.
***LCMS (IE, m/z):** (M+1) 487.19*
***RMN$^1$H :** $\delta_H$ ppm 400 MHz, DMSO*
*12.01 (1H, sl, NH), 9.83 (1H, d, $CH_{arom}$), 8.05 (1H, s, $CH_{arom}$), 7.80 (1H, dd, $CH_{arom}$), 7.57 (1H, dl, NH), 7.52 (1H, d, $CH_{arom}$), 4.11 (2H, d, $CH_2$), 3.14-3.04 (1H, m, CH), 2.82-2.75 (2H, m, 2xCH), 2.35-2.26 (2H, m, 2xCH), 1.84-1.60 (6H, m, 6xCH), 1.55-1.47 (2H, m, 2xCH), 1.33-0.98 (7H, m, 7xCH).*

**4-Oxo-8-(pipéridin-4-ylsulfamoyl)-4,5-dihydro-3*H*-pyrrolo[2,3-*c*]quinoline-1-carboxylate** de **méthyltétrahydrofuran-3-yle (118)**

Ce composé est préparé selon la synthèse 114, à partir de 152 mg (0.31 mmol) d'acide 8-(1-*tert*-butoxycarbonyl-pipéridin-4-ylsulfamoyl)-4-oxo-4,5-dihydro-3*H*-pyrrolo[2,3-*c*]quinoline-1-carboxylique, 216 μL (1.55 mmol) de triéthylamine et de 372 mg (1.4 mmol) de di-(tétrahydrofuran-3-yl)-méthylsulfate pour donner après purification par chromatographie sur silice (éluant chloroforme/méthanol/ammoniaque 100/25/4), 19 mg (13% sur deux étapes) de 4-oxo-8-(pipéridin-4-ylsulfamoyl)-4,5-dihydro-3*H*-pyrrolo[2,3-*c*]quinoline-1-carboxylate de méthyltétrahydrofuran-3-yle sous la forme d'un solide blanc.
***LCMS (IE, m/z):** (M+1) 475.13*
***RMN$^1$H :** $\delta_H$ ppm 400 MHz, DMSO*
*12.02 (1H, sl, NH), 9.82 (1H, d, $CH_{arom}$), 8.08 (1H, s, $CH_{arom}$), 7.81 (1H, dd, $CH_{arom}$), 7.65 (1H, d, NH), 7.53 (1H, d, $CH_{arom}$), 4.28 (1H, dd, $CH_{ali}$), 4.20 (1H, dd, $CH_{ali}$), 3.84-3.75 (2H, m, 2x$CH_{ali}$), 3.70-3.63 (1H, m, $CH_{ali}$), 3.61-3.55 (1H, m, $CH_{ali}$), 3.17-3.07 (1H, m, $CH_{ali}$), 2.88-2.80 (2H, m, 2x$CH_{ali}$), 2.73-2.63 (1H, m, $CH_{ali}$), 2.44-2.33 (2H, m, 2x$CH_{ali}$), 2.10-2.00 (1H, m, $CH_{ali}$), 1.74-1.65 (1H, m, $CH_{ali}$), 1.60-1.50 (2H, m, 2x$CH_{ali}$), 1.31-1.18 (2H, m, 2x$CH_{ali}$).*

**8-(1-Méthyl-pipéridin-4-ylsulfamoyl)-4-oxo-4,5-dihydro-3*H*-pyrrolo[2,3-*c*]quinoline-1-carboxylate de cyclopentyle (119)**

A une solution de 55 mg (0.12 mmol) de 4-oxo-8-(pipéridin-4-ylsulfamoyl)-4,5-dihydro-3*H*-pyrrolo[2,3-*c*]quinoline-1-carboxylate de cyclopentyle dissous dans 5 mL de diméthyformamide anhydre sont additionnés 83 μL (0.6 mmol) de triéthylamine et 22 μL (0.24 mmol) de diméthylsulfate. La solution est agitée à température ambiante pendant 3 heures avant addition de 2 mL d'ammoniaque. Après avoir poursuivie l'agitation 10 minutes supplémentaires, de l'eau est additionnée et le produit est extrait avec de l'acétate d'éthyle. Les phases organiques sont séchées sur sulfate de magnésium, filtrées et évaporées. Le résidu est purifié par chromatographie sur silice (éluant chloroforme/méthanol/ammoniaque 300/24/4) pour conduire à 22 mg (39%) de 8-(1-méthyl-pipéridin-4-ylsulfamoyl)-4-oxo-4,5-dihydro-3*H*-pyrrolo[2,3-*c*]quinoline-1-carboxylate de cyclopentyle sous la forme d'un solide blanc.

***LCMS (IE, m/z):*** *(M+1) 473.26*
***RMN¹H :*** *$\delta_H$ ppm 400 MHz, $CD_3OD$*
*10.04 (1H, d, $CH_{arom}$), 8.05 (1H, s, $CH_{arom}$), 7.90 (1H, dd, $CH_{arom}$), 7.55 (1H, d, $CH_{arom}$), 5.47-5.42 (1H, m, $CH_{ali}$), 3.55-3.47 (1H, m, $CH_{ali}$), 3.44-3.35 (2H, m, $2xCH_{ali}$), 3.13-2.94 (2H, m, $2xCH_{ali}$), 2.80 (3H, s, $CH_3$), 2.15-1.97 (4H, m, $4xCH_{ali}$), 1.96-1.65 (8H, m, $8xCH_{ali}$).*

**8-(Méthyl-pipéridin-4-yl-sulfamoyl)-4-oxo-4,5-dihydro-3*H*-pyrrolo[2,3-*c*]quinoline-1-carboxylate de propyle (120)**

Ce composé est préparé selon la synthèse 114, à partir de 294 mg (0.6 mmol) d'acide 8-[(4-*tert*-butoxycarbonyl-cyclohexyl)-méthyl-sulfamoyl]-4-oxo-4,5-dihydro-3*H*-pyrrolo[2,3-*c*]quinoline-1-carboxylique, 418 µL (3 mmol) de triéthylamine et de 345 µL (2.1 mmol) de di-*n*-propylsulfate pour donner après purification par chromatographie sur silice (éluant chloroforme/méthanol/ammoniaque 220/25/4), 80 mg (30% sur deux étapes) de 8-(méthyl-pipéridin-4-yl-sulfamoyl)-4-oxo-4,5-dihydro-3*H*-pyrrolo[2,3-*c*]quinoline-1-caboxylate de propyle sous la forme d'un solide blanc.
***LCMS (IE, m/z):*** *(M+1) 447.08*
***RMN¹H :*** *$\delta_H$ ppm 400 MHz, DMSO*
*9.83 (1H, d, $CH_{arom}$), 8.06 (1H, s, $CH_{arom}$), 7.78 (1H, dd, $CH_{arom}$), 7.53 (1H, d. $CH_{arom}$), 4:25 (2H, t, $CH_2$), 3.86-3.75 (1H, m, $CH_{pipér}$), 2.85 (2H, d, $2xCH_{pipéri}$), 2.69 (3H, s, $CH_3$), 2.41 (2H, t, $2xCH_{pipér}$), 1.75 (2H, sex, $CH_2$), 1.45-1.33 (2H, m, $2xCH_{pipér}$), 1.22 (2H, d, $2xCH_{pipér}$), 0.99 (3H, t, $CH_3$).*
L'acide 8-[(4-*tert*-butoxycarbonyl-cyclohexyl)-méthyl-sulfamoyl]-4-oxo-4,5-dihydro-3*H*-pyrrolo[2,3-*c*]quinoline-1-carboxylique est préparé selon la synthèse 178, à partir de 653 mg (2 mmol) d'acide 8-chlorosulfonyl-4-oxo-4,5-dihydro-3*H*-pyrrolo[2,3-*c*]quinoline-1-carboxylique, 334 mL (2.4 mmol) de triéthylamine et de 557 mg (2.6 mmol) de 4-méthylamino-1-*tert*-butoxycarbonyl-pipéridine pour donner après purification par précipitation et trituration dans l'eau, 770 mg (76%) d'acide 8-[(4-*tert*-butoxycarbonyl-cyclohexyl)-méthyl-sulfamoyl]-4-oxo-4,5-dihydro-3*H*-pyrrolo[2,3-*c*]quinoline-1-carboxylique sous la forme d'un solide beige.
A 3.7 mL (30 mmol) d'une solution de méthylamine à 33% dans l'éthanol sont additionnés 3.8 mL (13 mmol) d'isopropoxyde de titanium et 2 g (10 mmol) de 1-*tert*-butoxycarbonyl-pipéridin-4-one. La solution est agitée 5 heures à température ambiante puis 378 mg (10 mmol) de borohydrure de sodium sont additionnés. L'agitation est maintenue 2 heures supplémentaires puis 2 mL d'eau sont additionnés. Le mélange réactionnel est filtré puis les solvants sont évaporés pour conduire à 1.18 g (55%) de 4-méthylamino-1-*tert*-butoxycarbonyl-pipéridine sous la forme d'une huile.

**4-Oxo-8-[(pipéridin-4-ylméthyl)-sulfamoyl]-4,5-dihydro-3*H*-pyrrolo[2,3-*c*]quinoline-1-carboxylate d'éthyle (121).**

Ce composé est préparé selon la synthèse 25, à partir de 433 mg (1.32 mmol) d'acide 8-chlorosulfonyl-4-oxo-4,5-dihydro-3*H*-pyrrolo[2,3-*c*]quinoline-1-carboxylique, de 276 µL (1.99 mmol) de triéthylamine et de 313 mg (1.46 mmol) de 4-aminométhyl-1-*tert*-butoxycarbonyl-pipéridine pour conduire après purification par chromatographie sur silice (éluant chloroforme/méthanol/ammoniaque 65/25/4) puis par LC préparative à 13 mg (2% sur deux étapes) de 4-oxo-8-[(pipéridin-4-ylméthyl)-sulfamoyl]-4,5-dihydro-3*H*-pyrrolo[2,3-*c*]quinoline-1-carboxylate d'éthyle sous la forme d'un solide beige.
***LCMS (IE, m/z):*** *(M+1) 433.09*
***RMN¹H*** *: $\delta_H$ ppm 400 MHz, DMSO*
*13.27 (1H, sl, NH), 12.06 (1H, sl, NH), 9.79 (1H, d, $CH_{arom}$), 8.45 (1H, m, NH), 8.10 (1H, sl, NH), 8.06 (1H, d, $CH_{arom}$), 7.79 (1H, dd, $CH_{arom}$), 7.65 (1H, t, NH), 7.55 (1H, d, $CH_{arom}$), 4.33 (2H, q, $CH_2$), 3.28-3.20 (2H, m, $2xCH_{pipér}$), 2.87-2.75 (2H, m, $2xCH_{pipér}$), 2.72 (2H, t, $CH_2$), 1.83-1.75 (2H, m, $2xCH_{pipér}$), 1.73-1.63 (1H, m, $CH_{pipér}$), 1.36 (3H, t, $CH_3$), 1.28-1.16 (2H, m, $2xCH_{pipér}$).*

**8-(8-Aza-bicyclo[3.2.1]oct-3-ylsulfamoyl)-4-oxo-4,5-dihydro-3*H*-pyrrolo[2,3-*c*]quinoline-1-carboxylate d'éthyle (122)**

A une solution de 75 mg (0.14 mmol) de 8-(8-benzyl-8-aza-bicyclo[3.2.1]oct-3-ylsulfamoyl)-4-oxo-4,5-dihydro-3*H*-pyrrolo[2,3-*c*]quinoline-1-carboxylate d'éthyle dissous dans 15 mL d'éthanol sont additionnés 4 mg de Pd 10% activé sur charbon. La solution est agitée sous atmosphère d'hydrogène à température ambiante pendant 18 heures puis filtrée sur célite. Le filtrat est évaporé et le résidu purifié par chromatographie sur silice (éluant dichlorométhane/méthanol/ammoniaque 200/25/4) pour donner 20 mg (32%) de 8-(8-aza-bicyclo[3.2.1]oct-3-ylsulfamoyl)-4-oxo-4,5-dihydro-3*H*-pyrrolo[2,3-*c*]quinoline-1-carboxylate d'éthyle sous la forme d'un solide brun.
***LCMS (IE, m/z):*** *(M+1) 444.97*
***RMN¹H*** *: $\delta_H$ ppm 400 MHz, DMSO*

*9.85 (1H, s, $CH_{arom}$), 8.06 (1H, s, $CH_{arom}$), 7.77 (1H, dd, $CH_{arom}$), 7.52 (1H, d, $CH_{arom}$), 7.42 (1H, d, NH), 4.33 (2H, dd, $OCH_2$), 2.41-2.51 (3H, m, 3xCH), 3.14 (3H, t, $CH_3$), 1.52-1.24 (8H, m,8xCH) 0.92 (3H, t, $CH_3$).*

Le 8-(8-benzyl-8-aza-bicyclo[3.2.1]oct-3-ylsulfamoyl)-4-oxo-4,5-dihydro-3*H*-pyrrolo[2,3-*c*]quinoline-1-carboxylate d'éthyle est préparé selon la synthèse 111, à partir de 360 mg (1.1 mmol) d'acide 8-chlorosulfonyl-4-oxo-4,5-dihydro-3*H*-pyrrolo[2,3-*c*]quinoline-1-carboxylique et de 238 mg (1.1 mmol) de 8-benzyl-8-aza-bicyclo[3.2.1]oct-3-ylamine pour donner après estérification suivant la méthode générale C (diéthylsulfate comme dialkylsulfate utilisé) et précipitation dans l'eau, 411 mg (69% sur deux étapes) de 8-(8-benzyl-8-aza-bicyclo[3.2.1]oct-3-ylsulfamoyl)-4-oxo-4,5-dihydro-3*H*-pyrrolo[2,3-*c*]quinoline-1-carboxylate d'éthyle sous la forme d'un solide brun.

**8-Cyclopropylsulfamoyl-4-oxo-4,5-dihydro-3*H*-pyrrolo[2,3-*c*]quinoline-1-carboxylate d'éthyle (123)**

Ce composé est préparé selon la synthèse 111, à partir de 326 mg (1 mmol) d'acide 8-chlorosulfonyl-4-oxo-4,5-ihydro-3*H*-pyrrolo[2,3-*c*]quinoline-1-carboxylique et de 73 μL (1.05 mmol) de cyclopropylamine pour donner après estérification suivant la méthode générale C (diéthylsulfate comme dialkylsulfate utilisé) et purification par chromatographie sur silice (éluant dichlorométhane/méthanol 97/3), 85 mg (23% sur deux étapes) de 8-cyclopropylsulfamoyl-4-oxo-4,5-dihydro-3*H*-pyrrolo[2,3-c]quinoline-1-carboxylate d'éthyle sous la forme d'un solide jaune clair.

***LCMS (IE, m/z):** (M+1) 376.07*

***RMN$^1$H :** $\delta_H$ ppm 400 MHz, DMSO*

*13.16 (1H, sl, NH), 12.06 (1H, sl, NH), 9.83 (1H, d, $CH_{arom}$), 8.05 (1H, s, $CH_{arom}$), 7.83-7.78 (2H, m, NH et $CH_{arom}$), 7.56 (1H, d, $CH_{arom}$), 4.33 (2H, q, $CH_2$), 2.20-2.12 (1H, m, $CH_{cyclopro}$), 1.36 (3H, t, $CH_3$), 0.51-0.44 (2H, m, $2xCH_{cyclopro}$), (2H, m, $2xCH_{cyclopro}$).*

**8-Cyclopropylsulfamoyl-4-oxo-4,5-dihydro-3*H*-pyrrolo[2,3-*c*]quinoline-1-carboxylate de propyle (124)**

Ce composé est préparé selon la synthèse 111, à partir de 1.47 g (4.5 mmol) d'acide 8-chlorosulfonyl-4-oxo-4,5-dihydro-3*H*-pyrrolo[2,3-*c*]quinoline-1-carboxylique et de 624 μL (9 mmol) de cyclopropylamine pour donner après estérification suivant la méthode générale C (di-*n*-propylsulfate comme dialkylsulfate utilisé) et purification par chromatographie sur silice (éluant dichlorométhane/méthanol 98/2 puis 90/10), 99 mg (6% sur deux étapes) de 8-cyclopropylsulfamoyl-4-oxo-4,5-dihydro-3*H*-pyrrolo[2,3-c]quinoline-1-carboxylate de propyle sous la forme d'un solide blanc.

*LC/MS (IE, m/z) : (M+1) 390.12*

***RMN$^1$H:** $\delta_H$ ppm 400 MHz, DMSO*

*13.21 (1H, sl, NH), 12.07 (1H, sl, NH), 9.85 (1H, s, $CH_{arom}$), 8.06 (1H, s, $CH_{arom}$), 7.80 (2H, m, NH et $CH_{arom}$), 7.56 (1H, d, $CH_{arom}$), 4.24 (2H, q, $CH_2$), 2.20-2.05 (1H, m, $CH_{cycloprop}$), 1.87-1.67 (2H, m, $CH_2$), 0.99 (3H, t, $CH_3$), 0.58-0.28 (4H, m, $4xCH_{cycloprop}$).*

**8-(Cyclopropyl-méthyl-sulfamoyl)-4-oxo-4,5-dihydro-3*H*-pyrrolo[2,3-*c*]quinoline-1-carboxylate de propyle (125)**

Ce composé est préparé selon la synthèse 111, à partir de 196 mg (0.6 mmol) d'acide 8-chlorosulfonyl-4-oxo-4,5-dihydro-3*H*-pyrrolo[2,3-*c*]quinoline-1-carboxylique, 485 μL (4.8 mmol) de triéthylamine et de 62 mg (0.9 mmol) de chlorhydrate de *N*-méthyl-cyclopropylamine pour donner après estérification suivant la méthode générale C (di-*n*-propylsulfate comme dialkylsulfate utilisé) et purification par chromatographie sur silice (éluant dichlorométhane/méthanol 97/3) puis trituration dans l'éther diéthylique, 100 mg (41% sur deux étapes) de 8-(cyclopropyl-méthyl-sulfamoyl)-4-oxo-4,5-dihydro-3*H*-pyrrolo[2,3-*c*]quinoline-1-carboxylate de propyle sous la forme d'un solide blanc.

***LCMS (IE,** m/z): (M+1) 404.12*

***RMN$^1$H :** $\delta_H$ ppm 400 MHz, DMSO*

*13.23 (1H, sl, NH), 12.31 (1H, sl, NH), 9.85 (1H, s, $CH_{arom}$), 8.07 (1H, s, $CH_{arom}$), 7.80 (1H, dd, $CH_{arom}$), 7.60 (1H, d, $CH_{arom}$), 4.25 (2H, t, $OCH_2$), 2.70 (3H, s, $NCH_3$), 1.84-1.83 (1H, m, $CH_{cycloprop}$), 1.83-1.78 (2H, m, $CH_2$), 1.78-1.73 (2H, m, $CH_2$), 1.00 (3H, t, $CH_3$), 0.80-0.77(4H, m, $4xCH_{cycloprop}$).*

Dans un tube scellé, une solution de 1.14 g (20 mmol) de cyclopropylamine dans 5 mL d'éthylformate est chauffée à 60°C pendant 5 heures. Les solvants sont évaporés et le résidu est additionné par portions à une solution de 1.07 g (28 mmol) hydrogénure de lithium et d'aluminium dans 100 mL de tétrahydrofurane anhydre. Le mélange réactionnel est agité à température ambiante pendant 18 heures. Du méthanol est additionné et le mélange réactionnel est filtré sur célite. 10 mL (20 mmol) d'une solution 2M d'acide chlorhydrique dans l'éther diéthylique sont additionnés au filtrat. Les solvants sont évaporés pour conduire à 1.5 g (74%) de chlorhydrate de *N*-méthylcyclopropylamine sous la forme d'une huile.

**8-[(2-Cyano-éthyl)-méthyl-sulfamoyl]-4-oxo-4,5-dihydro-3*H*-pyrrolo[2,3-*c*]quinolino-1-carboxylate de propyle (126)**

Ce composé est préparé selon la synthèse 111, à partir de 163 mg (0.5 mmol) d'acide 8-chlorosulfonyl-4-oxo-4,5-dihydro-3*H*-pyrrolo[2,3-*c*]quinoline-1-carboxylique et de 29 mg (0.5 mmol) de 3-méthylamino-propionitrile pour donner après estérification suivant la méthode générale C (di-*n*-propylsulfate comme dialkylsulfate utilisé) et purification par chromatographie sur silice (éluant dichlorométhane/méthanol 97/3) puis trituration dans l'éther diéthylique, 30 mg (14% sur deux étapes) de 8-[(2-cyano-éthyl)-méthyl-sulfamoyl]-4-oxo-4,5-dihydro-3*H*-pyrrolo[2,3-*c*]quinoline-1-carboxylate de propyle sous la forme d'un solide blanc.
***LCMS (IE, m/z):*** *(M+1) 416.95*
***RMN$^1$H*** *: $\delta_H$ ppm 400 MHz, DMSO*
*13.13 (1H, sl, NH), 9.82 (1H, s, $CH_{arom}$), 8.08 (1H, s, $CH_{arom}$), 7.82 (1H, dd, $CH_{arom}$), 7.58 (1H, d, $CH_{arom}$), 4.26 (2H, t, $OCH_2$), 3.31-3.28 (2H, m, $CH_2$), 2.83-2.81 (2H, m, $NCH_2$), 2.75 (3H, s, $NCH_3$), 1.77-1.75 (2H, dd,$CH_2$), 1.00 (3H, t,$CH_3$).*

**8-[(2-Cyano-éthyl)-cyclopropyl-sulfamoyl]-4-oxo-4,5-dihydro-3*H*-pyrrolo[2,3-*c*]quinoline-1-carboxylate d'éthyle (127)**

Ce composé est préparé selon la synthèse 111, à partir de 326 mg (1 mmol) d'acide 8-chlorosulfonyl-4-oxo-4,5-dihydro-3*H*-pyrrolo[2,3-*c*]quinoline-1-carboxylique et de 118 μL (1.05 mmol) de 3-cyclopropylamino-propionitrile pour donner après estérification suivant la méthode générale C (diéthylsulfate comme dialkylsulfate utilisé) et purification par chromatographie sur silice (éluant dichlorométhane/méthanol 97/3), 60 mg (14% sur deux étapes) de 8-[(2-cyano-éthyl)-cyclopropyl-sulfamoyl]-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 429.13*
***RMN$^1$H*** *: $\delta_H$ ppm 400 MHz, DMSO*
*13.23 (1H, sl, NH), 12.13 (1H, sl, NH), 9.88 (1H, d, $CH_{arom}$), 8.07 (1H, s, $CH_{arom}$), 7.85 (1H, dd, $CH_{arom}$), 7.59 (1H, d, $CH_{arom}$), 4.33 (2H, q, $CH_2$), 3.41 (2H, t, $CH_2$), 2.84 (2H, t, $CH_2$), 2.08-2.01 (1H, m, $CH_{ali}$), 1.35 (3H, t, $CH_3$), 0.90-0.82 (2H, m, 2x$CH_{ali}$), 0.77-0.70 (2H, m, 2x$CH_{ali}$).*

**8-(Cyanométhyl-méthyl-sulfamoyl)-4-oxo-4,5-dihydro-3*H*-pyrrolo[2,3-*c*]quinoline-1-carboxylate de propyle (128)**

Ce composé est préparé selon la synthèse 111, à partir de 294 mg (0.9 mmol) d'acide 8-chlorosulfonyl-4-oxo-4,5-dihydro-3*H*-pyrrolo[2,3-*c*]quinoline-1-carboxylique, de 273 μL (2.7 mmol) de triéthylamine et de 143 mg (1.35 mmol) de chlorhydrate de méthylaminoacétonitrile pour donner après estérification suivant la méthode générale C (di-*n*-propylsulfate comme dialkylsulfate utilisé) et purification par chromatographie sur silice (éluant dichlorométhane/méthanol 99/1 à 95/5), 137 mg (38% sur deux étapes) de 8-(cyanométhyl-méthyl-sulfamoyl)-4-oxo-4,5-dihydro-3*H*-pyrrolo[2,3-*c*]quinoline-1-carboxylate de propyle sous la forme d'un solide blanc.
***LCMS (IE, m/z):*** *(M+1) 403.10*
***RMN$^1$H :*** *$\delta_H$ ppm 400 MHz, DMSO*
*13.24 (1H, sl, NH), 12.12 (1H, s, NH), 9.86 (1H, d, $CH_{arom}$), 8.07 (1H, s, $CH_{arom}$), 7.82 (1H, dd, $CH_{arom}$), 7.59 (1H, d, $CH_{arom}$), 4.40 (2H, s, $CH_2$), 4.24 (2H, t, $CH_2$), 2.84 (3H, s, $CH_3$), 1.80-1.71 (2H, m, $CH_2$), 0.99 (3H, t, $CH_3$).*

**8-([1,3]Dioxolan-2-ylméthyl-méthyl-sulfamoyl)4-oxo-4,5-dihydro-3*H*-pyrrolo[2,3-*c*]quinoline-1-carboxylate de propyle (129)**

Ce composé est préparé selon la synthèse 111, à partir de 326 mg (1 mmol) d'acide 8-chlorosulfonyl-4-oxo-4,5-dihydro-3*H*-pyrrolo[2,3-*c*]quinoline-1-mboxylique, de 117 mg (1 mmol) de [1,3]dioxolan-2-ylméthyl-méthyl-amine pour donner après estérification suivant la méthode générale C (di-*n*-propylsulfate comme dialkylsulfate utilisé) et purification par chromatographie sur silice (éluant dichlorométhane/méthanol 97/3), 100 mg (21% sur deux étapes) de 8-([1,3]dioxolan-2-ylméthyl-méthyl-sulfamoyl)-4-oxo-4,5-dihydro-3*H*-pyrrolo[2,3-c]quinoline-1-carboxylate de propyle sous la forme d'un solide blanc.
***LCMS (IE, m/z):*** *(M+1) 449.95*
***RMN$^1$H :*** *$\delta_H$ ppm 400 MHz, DMSO*
*12.09 (1H, sl, NH), 9.81 (1H, s, $CH_{arom}$), 8.07 (1H, s, $CH_{arom}$), 7.80 (1H, dd, $CH_{arom}$), 7.87 (1H, d, $CH_{arom}$), 4.99 (1H, t, CH), 4.45 (2H, t, $OCH_2$), 3.89-3.78 (4H, m, $OCH_2CH_2O$),3.13 (2H, d, $CH_2$), 2.80 (3H, s, $NCH_3$), 1.78-1.73 (2H, m, $CH_2$), 0.98 (3H, t,$CH_3$).*

**8-[(1-Benzhydryl-azétidin-3-yl)-méthyl-sulfamoyl]-4-oxo-4,5-dihydro-3*H*-pyrrolo[2,3-*c*]quinoline-1-carboxylate de propyle (130)**

Ce composé est préparé selon la synthèse 111, à partir de 196 mg (0.6 mmol) d'acide 8-chlorosulfonyl-4-oxo-4,5-dihydro-3*H*-pyrrolo[2,3-*c*]quinoline-1-carboxylique, de 334 μL (2.4 mmol) de triéthylamine et de 50 mg (0.72 mmol) de chlorhydrate de (1-benzyhydryl-azétidin-3-yl)-méthylamine (préparée selon la méthode décrite par Okada Tetsuo et al., Chem. Pharm. Bull., 1993, 41(1), 126-31) pour donner après estérification suivant la méthode générale C (di-*n*-propylsulfate comme dialkylsulfate utilisé) et purification par chromatographie sur silice (éluant dichlorométhane/méthanol 97/3), 60 mg (17% sur deux étapes) de 8-[(1-benzhydryl-azétidin-3-yl)-méthyl-sulfamoyl]-4-oxo-4,5-dihydro-3*H*-pyrrolo[2,3-*c*]quinoline-1-carboxylate de propyle sous la forme d'un solide blanc.
***LCMS (IE, m/z):*** *(M+1) 627.53*
***RMN$^1$H :*** *$\delta_H$ ppm 400 MHz, DMSO*
*13.24 (1H, sl, NH), 12.08 (1H, sl, NH), 9.76 (1H, s, $CH_{arom}$), 8.08 (1H, s, $CH_{arom}$), 7.73 (1H, dd, $CH_{arom}$), 7.56 (1H, d, $CH_{arom}$), 7.38 (4H, dd, $CH_{arom}$), 7.26-7.22 (4H. m, $CH_{arom}$), 7.18-7.16 (2H, m, $2xCH_{arom}$), 4.42 (1H, s, CH), 4.23 (2H, t, $OCH_2$), 3.74 (1H, quin, CH), 3.35 (2H, t, $NCH_2$), 2.90 (2H, t, $NCH_2$), 2.59 (3H, s, $NCH_3$), 1.80-1.75 (2H, m, $CH_2$), 1.05-1.01 (3H, m, $CH_3$).*

**8-[Méthyl-(tétrahydro-thiopyran-4-yl)-sulfamoyl]-4-oxo-4,5-dihydro-3*H*-pyrrolo[2,3-*c*]quinoline-1-carboxylate de propyle (131)**

Ce composé est préparé selon la synthèse 111, à partir de 326 mg (1 mmol) d'acide 8-chlorosulfonyl-4-oxo-4,5-dihydro-3*H*-pyrrolo[2,3-*c*]quinoline-1-carboxylique, de 558 μL (4 mmol) de triéthylamine et de 168 mg (1 mmol) de chlorhydrate de méthyl-(tétrahydro-thiopyran-4-yl)-amine pour donner après estérification suivant la méthode générale C (di-*n*-propylsulfate comme dialkylsulfate utilisé) et purification par chromatographie sur silice (éluant dichlorométhane/méthanol 97/3) puis trituration dans l'éther diéthylique, 140 mg (30% sur deux étapes) de 8-[méthyl-(tétrahydro-thiopyran-4-yl)-sulfamoyl]-4-oxo-4,5-dihydro-3*H*-pyrrolo[2,3-*c*]quinoline-1-carboxylate de propyle sous la forme d'un solide blanc.
***LCMS (IE, m/z):*** *(M+1) 464.13*
***RMN$^1$H :*** *$\delta_H$ ppm 400 MHz, DMSO*
*12.09 (1H, sl, NH), 9.86 (1H, s, $CH_{arom}$), 8.26 (1H, s, $CH_{arom}$), 7.80 (1H, dd, $CH_{arom}$), 7.52 (1H, d, $CH_{arom}$), 4.26 (2H, t, $OCH_2$), 3.80 (1H, quin, CH), 2.71 (3H, s, $NCH_3$), 2.50-2.52 (4H, m, $2xCH_2$), 1.77 (2H, q, $CH_2$), 1.68-1.62 (4H, m, $2xCH_2$), 1.00 (3H, t, $CH_3$).*

A 3.7 mL (30 mmol) d'une solution de méthylamine à 33% dans l'éthanol sont additionnés 4 mL (13.2 mmol) d'isopropoxyde de titanium et 1.16 g (10 mmol) de tétrahydro-thiopyran-4-one. La solution est agitée 5 heures à température ambiante puis 378 mg (10 mmol) de borohydrure de sodium sont additionnés. L'agitation est maintenue pendant 2 heures supplémentaires puis 2 mL d'eau sont additionnés. Le mélange réactionnel est filtré puis les solvants sont évaporés. Le résidu est repris dans de l'éther diéthylique puis une solution 2M d'acide chlorhydrique dans l'éther diéhylique est additionné. Le solide est filtré et lavé avec de l'éther diéthylique pour conduire à 920 mg (55%) de chlorhydrate de *N*-méthyl-(tétrahydro-thiopyran-4-yl)-amine sous la forme d'un solide brun.

**8-[(1,1-Dioxo-hexahydro-116-thiopyran-4-yl)-méthyl-sulfamoyl]-4-oxo-4,5-dihydro-3*H*-pyrrolo[2,3-*c*]quinoline-1-carboxylate de propyle (132)**

A une solution de 46 mg (0.1 mmol) de 8-[méthyl-(tétrahydro-thiopyran-4-yl)-sulfamoyl]-4-oxo-4,5-dihydro-3*H*-pyrrolo[2,3-*c*]quinoline-1-carboxylate de propyle dissous dans du methanol (2 mL) sont additionnés 307 mg (0.5 mmol) d'oxone. La solution est agitée à température ambiante pour 48 heures. Les solvants sont évaporés puis le résidu est repris à l'eau. Le solide est filtré puis purifié par chromatographie sur silice (éluant dichlorométhane/méthanol 97/3) puis trituré dans le méthanol pour conduire à 36 mg (72%) de 8-[(1,1-doxo-hexahydro-116-thiopyran-4-yl)-méthyl-sulfamoyl]-4-oxo-4,5-dihydro-3*H*-pyrrolo[2,3-*c*]quinoline-1-carboxylate de propyle sous la forme d'un solide blanc.
***LCMS (IE, m/z):*** *(M+1) 496.03*
***RMN$^1$H :*** *$\delta_H$ ppm 400 MHz, DMSO*
*13.23 (1H, sl, NH), 12.09 (1H, sl, NH), 9.82 (1H, s, $CH_{arom}$), 8.08 (1H, s, $CH_{arom}$), 7.84 (1H, d, $CH_{arom}$), 7.55 (1H, d, $CH_{arom}$), 4.29-4.18 (3H, m, $CH_2$ et CH), 3.02 (2H, d, $CH_2$), 2.73 (3H, s, $NCH_3$), 2.05 (2H, d, $CH_2$), 1.73-1.67 (4H, m, $2xCH_2$), 1.00 (3H, t, $CH_3$).*

**8-[Méthyl-(tétrahydro-pyran-4-yl)-sulfamoyl]-4-oxo-4,5-dihydro-3*H*-pyrrolo[2,3-*c*]quinoline-1-carboxylate de propyle (133)**

Ce composé est préparé selon la synthèse 111, à partir de 163 mg (0.5 mmol) d'acide 8-chlorosulfonyl-4-oxo-4,5-dihydro-3*H*-pyrrolo[2,3-*c*]quinoline-1-carboxylique, de 278 μL (2 mmol) de triéthylamine et de 76 mg (0.54 mmol) de chlorhydrate de méthyl-(tétrahydro-pyran-4-yl)-amine pour donner après estérification suivant la méthode générale C (di-*n*-propylsulfate comme dialkylsulfate utilisé) et purification par chromatographie sur silice (éluant dichlorométhane/méthanol 97/3) puis trituration dans l'éther diéthylique, 40 mg (18% sur deux étapes) de 8-[méthyl-(tétrahydro-pyran-4-yl)-sulfamoyl]-4-oxo-4,5-dihydro-3*H*-pyrrolo[2,3-*c*]quinoline-1-carboxylate de propyle sous la forme d'un solide blanc.
***LCMS (IE, m/z):** (M+1) 448.39*
***RMN$^1$H :** $\delta_H$ ppm 400 MHz, DMSO*
*13. 22 (1H, sl, NH), 12.07 (1H, sl, NH), 9.85 (1H, s, $CH_{arom}$), 8.07 (1H, s, $CH_{arom}$), 7.81 (1H, dd, $CH_{arom}$), 7.54 (1H, d, $CH_{arom}$), 4.26 (2H, t, $CH_2$), 4.12-4.00 (1H, m, CH), 3.78 (2H, dd, $CH_2$), 3.32-3.30 (2H, m, $CH_2$), 2.71 (3H, s, $NCH_3$), 1.82-1.76 (2H, m, $CH_2$), 1.75-1.55 (2H, m, $CH_2$), 1.59 (2H, dd, $CH_2$), 1.01 (3H, t, $CH_3$).*

A 3.7 mL (30 mmol) d'une solution de méthylamine à 33% dans l'éthanol sont additionnés 4 mL (13.2 mmol) d'isopropoxyde de titanium et 1 g (10 mmol) de tétrahydro-pyran-4-one. La solution est agitée 5 heures à température ambiante puis 378 mg (10 mmol) de borohydrure de sodium sont additionnés. L'agitation est maintenue pendant 2 heures supplémentaires puis 2 mL d'eau sont additionnés. Le mélange réactionnel est filtré puis les solvants sont évaporés. Le résidu est repris dans de l'éther diéthylique puis une solution 2M d'acide chlorhydrique dans l'éther diéthylique est additionné. Le solide est filtré et lavé avec de l'éther diéthylique pour conduire à 400 mg (26%) de chlorhydrate de *N*-méthyl-(tétrahydro-pyran-4-yl)-amine sous la forme d'un solide brun.

**8-(Méthyl-prop-2-ynyl-sulfamoyl)-4-oxo-4,5-dihydro-3*H*-pyrrolo[2,3-*c*]quinoline-1-carboxylate de propyle (134)**

Ce composé est préparé selon la synthèse 111, à partir de 196 mg (0.6 mmol) d'acide 8-chlorosulfonyl-4-oxo-4,5-dihydro-3*H*-pyrrolo[2,3-*c*]quinoline-1-carboxylique, de 242 μL (2.4 mmol) de triéthylamine et de 45 mg (0.66 mmol) de *N*-méthyl-prop-2-ynyl-amine pour donner après estérification suivant la méthode générale C (di-*n*-propylsulfate comme dialkylsulfate utilisé) et purification par chromatographie sur silice (éluant dichlorométhane/méthanol 97/3) puis trituration dans l'éther diéthylique, 80 mg (33% sur deux étapes) de 8-(méthyl-prop-2-ynyl-sulfamoyl)-4-oxo-4,5-dihydro-3*H*-pyrrolo[2,3-*c*]-quinoline-1-carboxylate de propyle sous la forme d'un solide blanc.
***LCMS (IE, m/z):** (M+1) 401.96*
***RMN$^1$H :** $\delta_H$ ppm 400 MHz, DMSO*
*13.22 (1H, sl, NH), 12.08 (1H, sl, NH), 9.82 (1H, s, $CH_{arom}$), 8.07 (1H, s, $CH_{arom}$), 7.79 (1H, dd, $CH_{arom}$), 7.56 (1H, d, $CH_{arom}$), 4.25 (2H, t, $OCH_2$), 4.01 (2H, s, $NCH_2$), 3.07 (1H, s, CH), 2.77 (3H, s, $NCH_3$), 1.80-1.75 (2H, m, $CH_2$), 1.00 (3H, t, $CH_3$).*

**8-[Méthyl-(2-méthylsulfanyl-éthyl)-sulfamoyl]-4-oxo-4,5-dihydro-3*H*-pyrrolo[2,3-*c*]quinoline-1-carboxylate de propyle (135)**

Ce composé est préparé selon la synthèse 111, à partir de 196 mg (0.6 mmol) d'acide 8-chlorosulfonyl-4-oxo-4,5-dihydro-3*H*-pyrrolo[2,3-*c*]quinoline-1-carboxylique, de 242 μL (2.4 mmol) de triéthylamine et de 102 mg (0.72 mmol) de chlorhydrate de *N*-méthyl-(2-méthylsulfanyl-éthyl)-amine pour donner après estérification suivant la méthode générale C (di-*n*-propylsulfate comme dialkylsulfate utilisé) et purification par chromatographie sur silice (éluant dichlorométhane/méthanol 97/3) puis trituration dans l'éther diéthylique, 80 mg (30% sur deux étapes) de 8-[méthyl-(2-méthylsulfanyl-éthyl)-sulfamoyl]-4-oxo-4,5-dihydro-3*H*-pyrrolo[2,3-*c*]quinoline-1-carboxylate de propyle sous la forme d'un solide blanc.
***LCMS (IE, m/z):** (M+1) 438.10*
***RMN$^1$H** : $\delta_H$ ppm 400 MHz, DMSO*
*13.22 (1H, sl, NH), 12.08 (1H, sl, NH), 9.81 (1H, s, $CH_{arom}$), 8.07 (1H, s, $CH_{arom}$), 7.79 (1H, dd, $CH_{arom}$), 7.57 (1H, d, $CH_{arom}$), 4.24 (2H, t, $OCH_2$), 3.20 (2H, t, $CH_2$), 2.74 (3H, s, $NCH_3$), 2.63 (2H, t, $CH_2$), 2.08 (3H, s, $SCH_3$), 1.77-1.72 (2H, m, $CH_2$), 1.00 (3H, t, $CH_3$).*

Dans un tube scellé, une solution de 1 g (11 mmol) de 2-méthylsulfanyl-éthylamine dans 20 mL d'éthylformate est chauffée à 60°C pendant 5 heures. Les solvants sont évaporés et le résidu est additionné par portions à une solution de 478 mg (12.6 mmol) d'hydrogénure de lithium et d'aluminium dans 100 mL de tétrahydrofurane anhydre. Le mélange réactionnel est agité à température ambiante pendant 18 heures. Du méthanol est additionné et le mélange réactionnel est filtré sur célite. 10 mL (20 mmol) d'une solution 2M d'acide chlorhydrique dans l'éther diéthylique est additionné au filtrat. Le solide est évaporé et lavé avec de l'éther diéthylique pour conduire à 600 mg (50%) de chlorhydrate de *N*-méthyl-(2-méthylsulfanyl-éthyl)-amine sous la forme d'un solide brun.

**8-[(2-Diméthylamino-éthyl)-méthyl-sulfamoyl]-4-oxo-4,5-dihydro-3*H*-pyrrolo[2,3-*c*]quinoline-1-carboxylate de propyle (136)**

A 456 mg (1.4 mmol) d'acide 8-chlorosulfonyl-4-oxo-4,5-dihydro-3*H*-pyrrolo[2,3-*c*]quinoline-1-carboxylique en suspension dans 4 mL de dichlorométhane anhydre et 1 mL de diméthylformamide anhydre, sont ajoutés 292 μL (2.09 mmol) de triéthylamine et 213 μL (1.68 mmol) de *N,N,N*'-triméthyl-éthane-1,2-diamine. Le milieu réactionnel est agité 20 heures à température ambiante. Les solvants sont évaporés puis le résidu est repris dans du diméthylformamide anhydre (6 mL) avant addition de 322 μL (2.31 mmol) de triéthylamine et 215 μL (1.31 mmol) de di-*n*-propylsulfate. La réaction est suivie par LC/MS sur trois jours avec addition de 107 μL (0.77 mmol) et de 63 μL (0.39 mmol) de di-n-propylsulfate de façon à consommer la totalité de l'acide carboxylique intermédiaire. De l'ammoniaque (1 mL) est additionné et les solvants sont évaporés. Le résidu est purifié par chromatographie sur silice (éluant dichlorométhane/méthanol/ammoniaque 200/16/3) pour conduire à 37% (6% sur deux étapes) de 8-[(2-diméthylamino-éthyl)-méthyl-sulfamoyl]-4-oxo-4,5-dihydro-3*H*-pyrrolo[2,3-*c*]quinoline-1-carboxylate de propyle sous la forme d'un solide blanc.
***LC/MS (IE, m/z)** : (M+1) 435.29*
***RMN$^1$H :** $\delta_H$ ppm 400 MHz, DMSO*
*13.21 (1H, sl, NH), 12.05 (1H, sl, NH), 9.80 (1H, s, $CH_{arom}$), 8.06 (1H, s, $CH_{arom}$), 7.77 (1H, dd, $CH_{arom}$), 7.56 (1H, d, $CH_{arom}$), 4.24 (2H, t, $CH_2$), 3.08 (2H, t, $CH_2$), 2.72 (3H, s, $CH_3$), 2.38 (2H, t, $CH_2$), 2.13 (6H, s, 2x$CH_3$), 1.79-1.70 (2H, m, $CH_2$), 0.99 (3H, t, $CH_3$).*

**8-[Méthyl-(2-pyrrolidin-1-yl-éthyl)-sulfamoyl]-4-oxo-4,5-dihydro-3*H*-pyrrolo[2,3-*c*]quinoline-1-carboxylate de propyle (137)**

Ce composé est préparé selon la synthèse 136, à partir de 196 mg (0.6 mmol) d'acide 8-chlorosulfonyl4-oxo-4,5-dihydro-3*H*-pyrrolo[2,3-c]quinoline-1-carboxylique, de 485 μL (8 mmol) de triéthylamine et de 128 mg (0.78 mmol) de chlorhydrate de *N*-méthyl-(2-pyrrolidin-1-yl-éthyl)-amine pour donner après estérification suivant la méthode générale C (di-*n*-propylsulfate comme dialkylsulfate utilisé) et purification par chromatographie sur silice (éluant dichlorométhane/méthanol/triéthylamine 97/3/1) puis trituration dans l'éther diéthylique, 50 mg (18% sur deux étapes) de 8-[méthyl-(2-pyrrolidin-1-yl-éthyl)-sulfamoyl]-4-oxo-4,5-dihydro-3*H*-pyrrolo[2,3-*c*]quinoline-l-carboxylate de propyle sous la forme d'un solide jaune.
***LCMS (IE, m/z):** (M+1) 461.30*
***RMN$^1$H** : $\delta_H$ ppm 400 MHz, DMSO*
*13.23 (1H, sl, NH), 12.08 (1H, sl, NH), 9.80 (1H, s, $CH_{arom}$), 8.07 (1H, s, $CH_{arom}$), 7.78 (1H, dd, $CH_{arom}$), 7.58 (1H, d, $CH_{arom}$), 4.24 (2H, t, $OCH_2$), 3.40-3.32 (2H, m, $CH_2$),3.12 (2H, t, $CH_2$), 2.53-2.52 (2H, m, $CH_2$), 2.73 (3H, s, $NCH_3$), 2.44-2.43 (2H, m, $CH_2$), 1.77-1.72 (2H, m, $CH_2$), 1.23-1.20 (3H, m, $CH_3$), 1.19-0.98 (4H, m, 2x$CH_2$).*

Dans un tube scellé, une solution de 1.14 g (10 mmol) de 2-pyrrolidin-1-yl-éthylamine dans 5 mL d'éthylformate est chauffée à 60˚C pendant 5 heures. Les solvants sont évaporés et le résidu est additionné par portions à une solution de 520 mg (13.7 mmol) d'hydrogénure de lithium et d'aluminium dans 100 mL de tétrahydrofurane anhydre. Le mélange réactionnel est agité à température ambiante pendant 18 heures. Du méthanol est additionné et le mélange réactionnel est filtré sur célite puis 10 mL (20 mmol) d'une solution 2M d'acide chlorhydrique dans l'éther diéthylique est additionné. Le solide est évaporé et lavé avec de l'éther diéthylique pour conduire à 980 mg (65%) de chlorhydrate de *N*-méthyl-2-pyrrolidin-1-yl-éthylamine sous la forme d'un solide brun.

**8-[(2-Diéthylamino-éthyl)-méthyl-sulfamoyl]-4-oxo-4,5-dihydro-3*H*-pyrrolo[2,3-*c*]quinoline-1-carboxylate de propyle (138)**

Ce composé est préparé selon la synthèse 111, à partir de 326 mg (1 mmol) d'acide 8-chlorosulfonyl-4-oxo-4,5-dihydro-3*H*-pyrrolo[2,3-*c*]quinoline-1-carboxylique et de 156 mg (1.2 mmol) de *N,N*-diéthyl-*N*'-méthyl-éthane-1,2-diamine pour donner après estérification suivant la méthode générale C (di-*n*-propylsulfate comme dialkylsulfate utilisé) et purification par chromatographie sur silice (éluant dichlorométhane/méthanol/ammoniaque 200/20/0 puis 200/20/3) puis trituration dans l'acétonitrile, 29 mg (6% sur deux étapes) de 8-[(2-diéthylamino-éthyl)-méthyl-sulfamoyl]-4-oxo-4,5-dihydro-3*H*-pyrrolo[2,3-c]quinoline-1-carboxylate de propyle sous la forme d'un solide blanc.
***LCMS (IE, m/z):** (M+1) 463.35*
***RMN$^1$H** : $\delta_H$ ppm 400 MHz, DMSO*
*13.22 (1H, sl, NH), 12.06 (1H, s, NH), 9.80 (1H, s, $CH_{arom}$), 8.06 (1H, s, $CH_{arom}$), 7.77 (1H, dd, $CH_{arom}$), 7.56 (1H, d, $CH_{arom}$), 4.24 (2H, t, $CH_2$), 3.06 (2H, dd, $CH_2$), 2.73 (3H, s, $CH_3$), 2.57-2.51 (2H, m, $CH_2$), 2.44 (4H, q, 2x$CH_2$), 1.80-1.71 (2H, m, $CH_2$), 0.99 (3H, t, $CH_3$), 0.90 (6H, t, 2x$CH_3$).*

**Chlorhydrate de 8-[méthyl-(2-méthylamino-éthyl)-sulfamoyl]-4-oxo-4,5-dihydro-3*H*-pyrrolo[2,3-*c*]quinoline-1-carboxylate de propyle (139)**

A une solution de 50 mg (0.1 mmol) de 8-{[2-(*tert*-butoxycarbonyl-méthyl-amino)-éthyl]-méthyl-sulfamoyl}-4-oxo-4,5-dihydro-3*H*-pyrrolo[2,3-*c*]quinoline-1-carboxylate de propyle dissous dans 5 mL de tétrahydrofurane anhydre sont additionnés 2 mL (4 mmol) d'une solution 2M d'acide chlorhydrique dans l'éther diéthylique. La solution est agitée à température ambiante pendant 3 jours puis le solide formé est filtré, trituré dans l'éther diisopropylique pour conduire à 26 mg (60%) de chlorhydrate de 8-[méthyl-(2-méthylamino-éthyl)-sulfamoyl]-4-oxo-4,5-dihydro-3*H*-pyrrolo[2,3-*c*]quinoline-1-carboxylate de propyle sous la forme d'un solide blanc.
***LCMS (IE, m/z):*** *(M+1) 421.26*
***RMN$^1$H*** *: $\delta_H$ ppm 400 MHz, DMSO*
*13.26 (1H, sl, NH), 12.15 (1H, s, NH), 9.82 (1H, s, $CH_{arom}$), 8.78 (2H, sl, NH.HCl), 8.07 (1H, d, $CH_{arom}$), 7.79 (1H, dd, $CH_{arom}$), 7.62 (1H, d, $CH_{arom}$), 4.24 (2H, t, $CH_2$), 3.28-3.22 (2H, m, $CH_2$), 3.17-3.09 (2H, m, $CH_2$), 2.75 (3H, s, $CH_3$), 2.59 (3H, t, $CH_3$), 1.80-1.70 (2H, m, $CH_2$), 0.99 (3H, t, $CH_3$).*

**8-{[2-(*tert*-Butoxycarbonyl-méthyl-amino)-éthyl]-méthyl-sulfamoyl}-4-oxo-4,5-dihydro-3*H*-pyrrolo[2,3-*c*]quinoline-1-carboxylate de propyle (140)**

Ce composé est préparé selon la synthèse 111, à partir de 326 mg (1 mmol) d'acide 8-chlorosulfonyl-4-oxo-4,5-dihydro-3*H*-pyrrolo[2,3-*c*]quinoline-1-carboxylique et de 226 mg (1.2 mmol) de *N*-*tert*-butoxycarbonyl-*N,N'*-diméthyl-éthane-1,2-diamine (préparé selon la méthode décrite par Kleemann, Heinz-Werner et al., J. Med. Chem., 1992, 35(3), 559-67) pour donner après estérification suivant la méthode générale C (di-*n*-propylsulfate comme dialkylsulfate utilisé) et purification par trituration dans un mélange dichlorométhane/acétate d'éthyle 1/1, 264 mg (50% sur deux étapes) de 8-{[2-(*tert*-butoxycarbonyl-méthyl-amino)-éthyl]-méthyl-sulfamoyl}-4-oxo-4,5-dihydro-3*H*-pyrrolo[2,3-*c*]quinoline-1-carboxylate de propyle sous la forme d'un solide blanc.
***LCMS (IE, m/z):*** *(M+1) 521.18*
***RMN$^1$H*** *: $\delta_H$ ppm 400 MHz, DMSO*
*13.21 (1H, sl, NH), 12.07 (1H, s, NH), 9.79 (1H, s, $CH_{arom}$), 8.06 (1H, s, $CH_{arom}$), 7.75 (1H, dd, $CH_{arom}$), 7.57 (1H, d, $CH_{arom}$), 4.23 (2H, t, $CH_2$), 3.37-3.31 (2H, m, $CH_2$), 3.16-3.08 (2H, m, $CH_2$), 2.80 (3H, s, $CH_3$), 2.72 (3H, s, $CH_3$), 1.79-1.70 (2H, m, $CH_2$), 1.39 (9H, s, $3xCH_3$), 0.99 (3H, t, $CH_3$).*

**Chlorhydrate de 8-[cyclopropyl-(2-méthyl-2-méthylamino-propyl)-sulfamoyl]-4-oxo-4,5-dihydro-3*H*-pyrrolo[2,3-*c*]quinoline-1-carboxylate de propyle (141)**

Ce composé est préparé selon la synthèse 139, à partir de 47 mg (0.1 mmol) 8-{[2-(*tert*-butoxycarbonyl-méthyl-amino)-2-méthyl-propyl]-cyclopropyl-sulfamoyl}-4-oxo-4,5-dihydro-3*H*-pyrrolo[2,3-*c*]quinoline-1-carboxylate de propyle et de 3 mL (6 mmol)) d'une solution 2M d'acide chlorhydrique dans l'éther diéthylique pour donner après filtration, 32 mg (62%) de chlorhydrate de 8-[cyclopropyl-(2-méthyl-2-méthylamino-propyl)-sulfamoyl]-4-oxo-4,5-dihydro-3*H*-pyrrolo[2,3-*c*]quinoline-1-carboxylate de propyle sous la forme d'un solide blanc.
***LC/MS (IE, m/z)*** *: (M+1) 475.00*
***RMN$^1$H*** *: $\delta_H$ ppm 400 MHz, DMSO*
*13.26 (1H, sl, NH), 12.14 (1H, sl, NH), 9.88 (1H, s. $CH_{arom}$), 8.63 (2H, sl, NH.HCl), 8.07 (1H, s, $CH_{arom}$), 7.85 (1H, dd, $CH_{arom}$), 7.60 (1H, d, $CH_{arom}$), 4.23 (2H, t, $CH_2$), 3.46 (2H, s. $CH_2$), 2.58 (3H, s, $CH_3$), 2.37-2.30 (1H, m, CH), 1.80-1.71 (2H, m, $CH_2$), 1.34 (6H, s, $2xCH_3$), 1.0 (3H, t, $CH_3$), 0.79-0.64 (4H, m, $4xCH_{cycloprop}$).*

Le 8-{[2-(*tert*-butoxycarbonyl-méthyl-amino)-2-méthyl-propyl]-cyclopropyl-sulfamoyl}-4-oxo-4,5-dihydro-3*H*-pyrrolo[2,3-*c*]quinoline-1-carboxylate de propyle est préparé selon la synthèse 111, à partir de 360 mg (1.1 mmol) d'acide 8-chlorosulfonyl-4-oxo-4,5-dihydro-3*H*-pyrrolo[2,3-*c*]quinoline-1-carboxylique et de 290 mg (1.2 mmol) de *N*-*tert*-butoxycarbonyl-*N'*-cyclopropyl-*N*-méthyl-2-méthyl-propane-1,2-diamine pour donner après estérification suivant la méthode générale C (di-*n*-propylsulfate comme dialkylsulfate utilisé) et purification par chromatographie sur silice (éluant cyclohexane/acétate d'éthyle 7/3 puis 1/1), 50 mg (8% sur deux étapes) de 8-{[2-(*tert*-butoxycarbonyl-méthyl-amino)-2-méthyl-propyl]-cyclopropyl-sulfamoyl}-4-oxo-4,5-dihydro-3*H*-pyrrolo[2,3-*c*]quinoline-1-carboxylate de propyle sous la forme d'un solide blanc.

A une solution de 1.57 g (27.6 mmol) de cyclopropylamine dans 10 mL de méthanol anhydre sont additionnés 3.6 mL (12 mmol) d'isopropoxyde de titanium et 1.85 g (9.2 mmol) de 2-(*tert*-butoxycarbonyl-méthyl-amino)-2-méthyl-propionaldéhyde (préparée selon une méthode similaire à celle décrite par Kato, shiro et al., J. Chem. Soc. Perkin Trans. 1, 1997, 21, 3219-26). La solution est agitée 5 heures à température ambiante puis 454 mg (12 mmol) de borohydrure de sodium sont additionnés. L'agitation est maintenue pendant 2 heures supplémentaires puis 2 mL d'eau sont additionnés. Le mélange réactionnel est filtré puis les solvants sont évaporés. Le résidu est purifié par chromatographie sur

silice (éluant dichlorométhane/méthanol 98/2) pour conduire à 1.18 g (53%) de *N-tert*-butoxycarbonyl-*N*'-cyclopropyl-*N*-méthyl-2-méthyl-propane-1,2-diamine sous la forme d'une huile incolore.

**Chlorhydrate de 8-[cyclopropyl-(2-isopropylamino-éthyl)-sulfamoyl]-4-oxo-4,5-dihydro-3*H*-pyrrolo[2,3-*c*]quinoline-1-carboxylate de propyle (142)**

Ce composé est préparé selon la synthèse 139, à partir de 195 mg (0.34 mmol) de 8-{[2-(*tert*-butoxycarbonyl-isopropyl-amino)-éthyl]-cyclopropyl-sulfamoyl}-4-oxo-4,5-dihydro-3*H*-pyrrolo[2,3-c]quinoline-1-carboxylate de propyle et de 6.8 mL (13.6 mmol) d'une solution 2M d'acide chlorhydrique dans l'éther diéthylique pour donner après filtration, 160 mg (92%) de chlorhydrate de 8-[cyclopropyl-(2-isopropylamino-éthyl)-sulfamoyl]-4-oxo-4,5-dihydro-3*H*-pyrrolo [2,3-*c*]quinoline-1-carboxylate de propyle sous la forme d'un solide blanc.
***LCMS (IE, m/z):** (M+1) 475*
***RMN$^1$H** : $\delta_H$ ppm 400 MHz, DMSO*
*13.26 (1H, sl, NH), 12.15 (1H, s, NH), 9.89 (1H, s, $CH_{arom}$), 8.67 (2H, sl, NH.HCl), 8.07 (1H, ds, $CH_{arom}$), 7.84 (1H, dd, $CH_{arom}$), 7.62 (1H, d, $CH_{arom}$), 4.24 (2H, t, $CH_2$), 3.43-3.25 (3H, m, CH et $CH_2$), 3.17-3.09 (2H, m, $CH_2$), 2.14-2.09 (1H, m, $CH_{cycloprop}$), (2H, m, $CH_2$), 1.23 (6H, d, $2xCH_3$), 0.99 (3H, t, $CH_3$), 0.90-0.86 (2H, m, $2xCH_{cycloprop}$), 0.78-0.72 (2H, m, $2xCH_{cycloprop}$).*

Le 8-{[2-(*tert*-butoxycarbonyl-isopropyl-amino)-éthyl]-cyclopropyl-sulfamoyl}-4-oxo-4,5-dihydro-3*H*-pyrrolo [2,3-*c*]quinoline-1-carboxylate de propyle est préparé selon la synthèse 111, à partir de 326 mg (1 mmol) d'acide 8-chlorosulfonyl-4-oxo-4,5-dihydro-3*H*-pyrrolo[2,3-*c*]quinoline-1-carboxylique et de 288 mg (1.2 mmol) de *N-tert*-butoxycarbonyl-*N*'-cyclopropyl-*N*-isopropyl-éthane-1,2-diamine pour donner après estérification suivant la méthode générale C (di-*n*-propylsulfate comme dialkylsulfate utilisé) et purification par chromatographie sur silice (éluant cyclohexane/acétate d'éthyle 1/1 puis 1/3), 201 mg (35% sur deux étapes) de 8-{[2-(*tert*-butoxycarbonyl-isopropyl-amino)-éthyl]-cyclopropyl-sulfamoyl}-4-oxo-4,5-dihydro-3*H*-pyrrolo[2,3-c]quinoline-1-carboxylate de propyle sous la forme d'un solide blanc.

A une solution de 1.71 g (30 mmol) de cyclopropylamine dans 15 mL de méthanol anhydre sont additionnés 3.9 mL (13 mmol) d'isopropoxyde de titanium et 2 g (10 mmol) de (*tert*-butoxycarbonyl-isopropyl-amino)-acetaldéhyde (préparée selon la méthode décrite par Kato, shiro et al., J. Chem. Soc. Perkin Trans. 1, 1997, 21, 3219-26). La solution est agitée 5 heures à température ambiante puis 454 mg (12 mmol) de borohydrure de sodium sont additionnés. L'agitation est maintenue pendant 2 heures supplémentaires puis 2 mL d'eau sont additionnés. Le mélange réactionnel est filtré puis les solvants sont évaporés. Le résidu est purifié par chromatographie sur silice (éluant dichlorométhane/méthanol 98/2 puis 95/5) pour conduire à 852 mg (35%) de *N-tert*-butoxycarbonyl-*N*-cyclopropyl-*N*'-isopropyl-éthane-1,2-diamine sous la forme d'une huile incolore.

**Chlorhydrate de 8-[cyclopropyl-(1*H*-imidazol-4-ylméthyl)-sulfamoyl]-4-oxo-4,5-dihydro-3*H*-pyrrolo[2,3-*c*]quinoline-1-carboxylate de propyle (143)**

Ce composé est préparé selon la synthèse 114, à partir de 353 mg (0.67 mmol) d'acide 8-[cyclopropyl-(1*H*-imidazol-4-ylméthyl)-sulfamoyl]-4-oxo-4,5-dihydro-3*H*-pyrrolo[2,3-*c*]quinoline-1-carboxylique, 934 μL (6.7 mmol) de triéthylamine et de 440 μL (2.68 mmol) de di-*n*-propylsulfate. La base libre est purifiée par chromatographie sur silice (éluant chloroforme/méthanol/ammoniaque 220/25/4) puis le chlorhydrate est formé par ajout d'une solution 2M d'acide chlorhydrique dans l'éther sur le composé libre dissous dans du tétrahydrofurane anhydre pour conduire à 16 mg (26% sur deux étapes) de chlorhydrate de 8-[cyclopropyl-(1*H*-imidazol-4-ylméthyl)-sulfamoyl]-4-oxo-4,5-dihydro-3*H*-pyrrolo [2,3-*c*]quinoline-1-carboxylate de propyle sous la forme d'un solide blanc.
***LCMS (IE, m/z):** (M+1) 470.29*
***RMN$^1$H** : $\delta_H$ ppm 400 MHz, DMSO*
*14.35 (2H, sl, NH et HCl), 13.25 (1H, sl, NH), 12.15 (1H, sl, NH), 9.87 (1H, d, $CH_{arom}$), 8.99 (1H, s, $CH_{arom}$), 8.08 (1H, d, $CH_{arom}$), 7.82 (1H, dd, $CH_{arom}$), 7.60 (1H, d, $CH_{arom}$), 7.53 (1H, s, $CH_{arom}$), 4.40 (2H, s, $CH_2$), 4.24 (2H, t, $CH_2$), 2.09-2.02 (1H, m, $CH_{ali}$), 1.75 (2H, sex, $CH_2$), 0.99 (3H, t, $CH_3$), 0.74-0.64 (4H, m, $4xCH_{ali}$).*

L'acide 8-[cyclopropyl-(1*H*-imidazol-4-ylméthyl)-sulfamoyl]-4-oxo-4,5-dihydro-3*H*-pyrrolo[2,3-*c*]quinoline-1-carboxylique est préparé selon la synthèse 178, à partir de 284 mg (0.87 mmol) d'acide 8-chlorosulfonyl-4-oxo-4,5-dihydro-3*H*-pyrrolo[2,3-*c*]quinoline-1-carboxylique, 605 μL (4.35 mmol) de triéthylamine et de 309 mg (1.3 mmol) de cyclopropyl-(1-*tert*-butoxycarbonyl-1*H*-imidazol-4-ylméthyl)-amine pour donner après purification par précipitation et trituration dans l'eau, 350 mg (76%) d'acide 8-[cyclopropyl-(1*H*-imidazol-4-ylméthyl)-sulfamoyl]-4-oxo-4,5-dihydro-3*H*-pyrroto[2,3-*c*]quinoline-1-carboxylique sous la forme d'un solide beige.

A une solution de 824 mg (4.2 mmol) de 1-*tert*-butoxycarbonyl-1*H*-imidazole-4(5)-carbaldéhyde dans 20 mL de méthanol anhydre sont additionnés 438 μL (6.3 mmol) de cyclopropylamine, 527 mg (8.4 mmol) de cyanoborohydrure de sodium et 1.52 mL (42 mmol) d'acide acétique. La solution est agitée à température ambiante pendant 24 heures

puis une solution aqueuse saturée en hydrogénocarbonate de sodium est additionnée pour ajuster le pH à 7. Le composé est extrait avec de l'acétate d'éthyle. Les phases organiques sont séchées sur sulfate de magnésium, filtrées puis évaporées et le résidu est purifié par chromatographie sur silice (éluant dichlorométhane/méthanol 95/5) pour conduire à 398 mg (40%) de cyclopropyl-(1-*tert*-butoxycarbonyl-1*H*-imidazol-4-ylméthyl)-amine sous la forme d'une huile visqueuse.

A une solution de 961 mg (10 mmol) d'1*H*-imidazole-4-carboxaldéhyde dissous dans 40 mL d'acétonitrile sont additionnés 2.6 g (11.9 mmol) de dicarbonate de di-*tert*-butyle et 610 mg (5 mmol) de 4-diméthylaminopyridine. La solution est agitée à température ambiante pendant 7 heures puis une solution aqueuse saturée en chlorure d'ammonium est additionnée pour ajuster le pH à 5. Le produit est extrait avec de l'acétate d'éthyle et les phases organiques sont séchées sur sulfate de magnésium, filtrées et évaporées. Le résidu est purifié par chromatographie sur silice (éluant dichlorométhane/méthanol 88/12) pour conduire à 1.68 g (85%) de 1-*tert*-butoxycarbonyl-1*H*-imidazole-4(5)-carbaldéhyde.

**Chlorhydrate de (S)-8-(cyclopropyl-pyrrolidin-2-ylméthyl-sulfamoyl)-4-oxo-4,5-dihydro-3*H*-pyrrolo[2,3-*c*]quinoline-1-carboxylate de propyle (144)**

Ce composé est préparé selon la synthèse 139, à partir de 97 mg (0.17 mmol) de (S) 8-[(1-*tert*-butoxycarbonyl-pyrrolidin-2-ylméthyl)-cyclopropyl-sulfamoyl]-4-oxo-4,5-dihydro-3*H*-pyrrolo[2,3-*c*]quinoline-1-carboxylate de propyle et de 3.4 mL (6.8 mmol) d'une solution 2M d'acide chlorhydrique dans l'éther diéthylique pour donner après filtration, 46 mg (53%) de chlorhydrate de (S) 8-(cyclopropyl-pyrrolidin-2-ylméthyl-sulfamoyl)-4-oxo-4,5-dihydro-3*H*-pyrrolo[2,3-*c*] quinoline-1-carboxylate de propyle sous la forme d'un solide blanc.

***LCMS (IE, m/z):*** *(M+1) 473.2*

***RMN$^1$H*** *: $\delta_H$ ppm 400 MHz, DMSO*

*13.26 (1H sl, NH), 12.15 (1H, s, NH), 9.88 (1H, d, $CH_{arom}$), 9.27 (1H, sl, NH.HCl), 8.51 (1H, sl, NH.HCl), 8.07 (1H, d, $CH_{arom}$), 7.84 (1H, dd, $CH_{arom}$), 7.62 (1H, d, $CH_{arom}$), 4.24 (2H, t, $CH_2$), 3.89-3.78 (1H, m, CH), 3.38-3.18 (4H, m, $4xCH_{ali}$), 2.15-2.02 (2H, m, 2xCH), 1.99-1.86 (2H, m, 2xCH), 1.80-1.70 (2H, m, $CH_2$), 1.68-1.58 (1H, m, CH), 0.99 (3H, t, $CH_3$), 0.91-0.86 (2H, m, $2xCH_{cycloprop}$), 0.75-0.72 (2H, m, $2xCH_{cycloprop}$).*

Le (S) 8-[(1-*tert*-butoxycarbonyl-pyrrolidin-2-ylméthyl)-cyclopropyl-sulfamoyl]-4-oxo-4,5-dihydro-3*H*-pyrrolo [2,3-*c*]quinoline-1-carboxylate de propyle est préparé selon la synthèse 111, à partir de 326 mg (1 mmol) d'acide 8-chlorosulfonyl-4-oxo-4,5-dihydro-3*H*-pyrrolo[2,3-*c*]quinoline-1-carboxylique et de 288 mg (1.2 mmol) de (S) 1-*tert*-butoxycarbonyl-2-cyclopropylaminométhyl-pyrrolidine pour donner après estérification suivant la méthode générale C (di-*n*-propylsulfate comme dialkylsulfate utilisé) et purification par chromatographie sur silice (éluant dichlorométhane/méthanol 99/1 puis 95/5), 99 mg (17% sur deux étapes) de (S) 8-[(1-*tert*-butoxycarbonyl-pyrrolidin-2-ylmélhyl)-cyclopropyl-sulfamoyl]-4-oxo-4,5-dihydro-3*H*-pyrrolo[2,3-*c*]quinoline-1-carboxylate de propyle sous la forme d'un solide blanc.

A une solution de 1.7 mL (30 mmol) de cyclopropylamine dans 10 mL de méthanol anhydre sont additionnés 3.78 mL (13 mmol) d'isopropoxyde de titanium et 2 g (10 mmol) de (S) 1-*tert*-butoxycarbonyl-pyrrolidine-2-carbaldéhyde. La solution est agitée 5 heures à température ambiante puis 378 mg (10 mmol) de borohydrure de sodium sont additionnés. L'agitation est maintenue pour 2 heures supplémentaires puis 2 mL d'eau sont additionnés. Le mélange réactionnel est filtré puis les solvants sont évaporés. De l'eau est additionné et le composé est extrait avec de l'éther diéthylique. Les phases organiques sont séchées sur sulfate de magnésium, filtrées et évaporées pour conduire à 2.34 g (97%) de (S) *N*-cyclopropyl-1-*tert*-butoxycarbonyl-pyrrolidin-2-ylméthyl-amine sous la forme d'une huile incolore.

**(S) 8-[Cyclopropyl-(1-méthyl-pyrrolidin-2-ylméthyl)-sulfamoyl]4-oxo-4,5-dihydro-3*H*-pyrrolo[2,3-*c*]quinoline-1-carboxylate de propyle (145)**

Ce composé est préparé selon la synthèse 119, à partir de 76 mg (0.15 mmol) de chlorhydrate de (S) 8-(cyclopropyl-pyrrolidin-2-ylméthyl-sulfamoyl)-4-oxo-4,5-dihydro-3*H*-pyrrolo[2,3-*c*]quinoline-1-carboxylate de propyle, de 104 $\mu$L (0.75 mmol) de triéthylamine et de 28 $\mu$L (0.3 mmol)) de diméthylsulfate pour donner après précipitation dans l'eau et trituration dans l'éther diisopropylique, 48 mg (65%) de (S) 8-[cyclopropyl-(1-méthyl-pyrrolidin-2-ylméthyl)-sulfamoyl]-4-oxo-4,5-dihydro-3*H*-pyrrolo[2,3-*c*]quinoline-1-carboxylate de propyle sous la forme d'un solide blanc.

***LCMS (IE, m/z):*** *(M+1) 487.35*

***RMN$^1$H*** *: $\delta_H$ ppm 400 MHz, DMSO*

*12.00 (1H, sl, NH), 9.86 (1H, sl, $CH_{arom}$), 8.07 (1H, s, $CH_{arom}$), 7.79 (1H, sl, $CH_{arom}$), 7.56 (1H, sl, $CH_{arom}$), 4.31-4.15 (2H, m, $CH_2$), 3.30-2.60 (4H, m, $CH_2$ et 2xCH), 2.26 (3H, s, $CH_3$), 2.10-1.60 (8H, m, 6xCH et $CH_2$), 0. 99 (3H, t, $CH_3$), 1.00-0.64 (4H, m, 4xCHcycloprop).*

**(R) 8-[Cyclopropyl-(1-méthyl-pyrrolidin-2-ylméthyl)-sulfamoyl]4-oxo-4,5-dihydro-3*H*-pyrrolo[2,3-*c*]quinoline-1-carboxylate de propyle (146)**

Ce composé est préparé selon la synthèse 119, à partir de 51 mg (0.1 mmol) de chlorhydrate de (R) 8-(cyclopropyl-pyrrolidin-2-ylméthyl-sulfamoyl)-4-oxo-4,5-dihydro-3*H*-pyrrolo[2,3-*c*]quinoline-1-carboxylate de propyle, de 67 µL (0.5 mmol) de triéthylamine et de 14 µL (0.15 mmol)) de diméthylsulfate pour donner après précipitation dans l'eau et trituration dans l'éther diisopropylique, 29 mg (60%) de (R) 8-[cyclopropyl-(1-méthyl-pyrrolidin-2-ylméthyl)-sulfamoyl]-4-oxo-4,5-dihydro-3*H*-pyrrolo[2,3-*c*]quinoline-1-carboxylate de propyle sous la forme d'un solide blanc.
***LC/MS (IE, m/z)*** *: (M+1) 487.36*
***RMN$^1$H :*** *$\delta_H$ ppm 400 MHz, DMSO*
*11.06 (1H, sl, NH), 9.86 (1H, s, $CH_{arom}$), 8.06 (1H, s, $CH_{arom}$), 7.81 (1H, d, $CH_{arom}$), 7.58 (1H, d, $CH_{arom}$), 4.24 (2H, t, $CH_2$), 3.03-2.90 (2H, m, 2xCH), 2.27 (3H, s, $NCH_3$), 2.12-2.02 (2H, m, 2xCH), 1.85-1.60 (7H, m, 7xCH), 0.99 (3H, t, $CH_3$), 0.85 (2H, m, $CH_2$), 0.73 (2H, m, $CH_2$).*

**(R) 8-[(1-*tert*-Butoxycarbonyl-pyrrolidin-2-ylméthyl)-cyclopropyl-sulfamoyl]-4-oxo-4,5-dihydro-3*H*-pyrrolo [2,3-*c*]quinoline-1-carboxylate de propyle (147)**

Ce composé est préparé selon la synthèse 111, à partir de 360 mg (1.1 mmol) d'acide 8-chlorosulfonyl-4-oxo-4,5-dihydro-3*H*-pyrrolo[2,3-*c*]quinoline-1-carboxylique et de 292 mg (1.21 mmol) de (R) 1-*tert*-butoxycarbonyl-2-cyclopmpylaminométhyl-pyrrolidine pour donner après estérification suivant la méthode générale C (di-*n*-propylsulfate comme dialkylsulfate utilisé) et purification par chromatographie sur silice (éluant cyclohexane/acétate d'éthyle 1/1 puis 1/4), 226 mg (36% sur deux étapes) de (R) 8-[(1-*tert*-butoxycarbonyl-pyrrolidin-2-ylméthyl)-cyclopropyl-sulfamoyl]-4-oxo-4,5-dihydro-3*H*-pyrrolo[2,3-*c*]quinoline-1-carboxylate de propyle sous la forme d'un solide blanc.
***LC/MS (IE, m/z) :*** *(M+1) 573.40*
***RMN$^1$H :*** *$\delta_H$ ppm 400 MHz, $CDCl_3$*
*11.43 (1H, sl, NH), 10.73 (1H, sl, NH), 10.05 (1H, d, $CH_{arom}$), 8.00 (1H, d, $CH_{arom}$), 7.90-7.84 (1H, m, $CH_{arom}$), 7.49-7.43 (1H, m, $CH_{arom}$), 4.25 (2H, t, $CH_2$), 3.47-3.34 (4H, m, $2xCH_2$), 3.11 (1H, dd, CH), 2.28-2.24 (1H, m, CH), 2.13-2.05 (2H, m, 2xCH), 1.90-1.87 (2H, m, 2xCH), 1.77 (2H, q, $CH_2$), 1.42 (9H, s, $3xCH_3$), 1.00 (3H, t, $CH_3$), 0.83-0.70 (4H, m, $4xCH_{cycloprop}$)*

A une solution de 1 mL (30 mmol) de cyclopropylamine dans 5 mL de méthanol anhydre sont additionnés 1.9 mL (6 mmol) d'isopropoxyde de titanium et 1 g (10 mmol) de (R) 1-*tert*-butoxycarbonyl-pyrrolidine-2-carbaldéhyde. La solution est agitée 5 heures à température ambiante puis 246 mg (605 mmol) de borohydrure de sodium sont additionnés. L'agitation est maintenue pendant 2 heures supplémentaires puis 2 mL d'eau sont additionnés. Le mélange réactionnel est filtré puis les solvants sont évaporés. De l'eau est additionné et le composé est extrait avec de l'éther diéthylique. Les phases organiques sont séchées sur sulfate de magnésium, filtrées et évaporées pour conduire à 1.17 g (97%) de (R) 1-*tert*-butoxycarbonyl-2-cyclopropylaminométhyl-pyrrolidine sous la forme d'une huile incolore.

**Chlorhydrate de (R) 8-(cyclopropyl-pyrrolidin-2-ylméthyl-sulfamoyl)-4-oxo-4,5-dihydro-3*H*-pyrrolo[2,3-*c*]quinoline-1-carboxylate de propyle (148)**

Ce composé est préparé selon la synthèse 139, à partir de 220 mg (0.38 mmol) de (R) 8-[(1-*tert*-butoxycarbonyl-pyrrolidin-2-ylméthyl)-cyclopropyl-sulfamoyl]-4-oxo-4,5-dihydro-3*H*-pyrrolo[2,3-c]quinoline-1-carboxylate de propyle et de 11.5 mL (23 mmol) d'une solution 2M d'acide chlorhydrique dans l'éther diéthylique pour donner après filtration, 126 mg (64%) de chlorhydrate de (R) 8-(cyclopropyl-pyrrolidin-2-ylméthyl-sulfamoyl)-4-oxo-4,5-dihydro-3*H*-pyrrolo[2,3-c] quinoline-1-carboxylate de propyle sous la forme d'un solide blanc.
***LC/MS (IE, m/z) :*** *(M+1) 473.34*
***RMN$^1$H*** *: $\delta_H$ ppm 400 MHz, DMSO*
*13.26 (1H, sl, NH), 12.16 (1H, sl, NH), 9.90 (1H, s, $CH_{arom}$), 9.34 (1H, sl, NH.HCl), 8.59 (1H, sl, NH.HCl), 8.08 (1H, d, $CH_{arom}$), 7.86 (1H, d, $CH_{arom}$), 7.63 (1H, d, $CH_{arom}$), 4.24 (2H, t, $CH_2$), 3.86-3.80 (1H, m, $CH_{ali}$), 3.50-3.31 (2H, m, $2xCH_{ali}$), 3.31-3.13 (2H, m, $2xCH_{ali}$), 2.13-2.03 (2H, m, $2xCH_{ali}$), 1.99-1.87 (2H, m, $2xCH_{ali}$), 1.82-1.67 (2H, m, $CH_2$), 1.69-1.60 (1H, m, $CH_{ali}$), 0.99 (3H, $CH_3$), 0.93-0.83 (2H, m, $2xCH_{cycloprop}$), 0.77-0.68 (2H, m, $2xCH_{cycloprop}$).*

**Chlorhydrate de (S) 8-(isopropyl-pyrrolidin-2-ylméthyl-sulfamoyl)-4-oxo-4,5-dihydro-3*H*-pyrrolo[2,3-*c*]quinoline-1-carboxylate de propyle (149)**

Ce composé est préparé selon la synthèse 139, à partir de 103 mg (0.18 mmol) de (S) 8-[1-*tert*-butoxycarbonyl-isopropyl-pyrrolidin-2-ylméthyl-sulfamoyl]4-oxo-4,5-dihydro-3*H*-pyrrolo[2,3-*c*]quinoline-1-carboxylate de propyle et de 4.5 mL (9 mmol) d'une solution 2M d'acide chlorhydrique dans l'éther diéthylique pour donner après filtration, 60 mg

(65%) de chlorhydrate de (S) 8-(isopropyl-pyrrolidin-2-ylméthyl-sulfamoyl)-4-oxo-4,5-dihydro-3*H*-pyrrolo[2,3-*c*]quinoline-1-carboxylate de propyle sous la forme d'un solide blanc.

***LCMS (IE, m/z):*** *(M+1) 475.2*

***RMN¹H*** *:* $\delta_H$ *ppm 400 MHz, DMSO*

*13.26 (1H, sl, NH), 12.12 (1H, s, MH), 9.88 (1H, d,* $CH_{arom}$*), 9.18 (1H, sl, NH.HCl), 8.53 (1H, sl, AH.HCl), 8.07 (1H, s,* $CH_{arom}$*), 7.87 (1H, dd,* $CH_{arom}$*), 7.58 (1H, d,* $CH_{arom}$*), 4.25 (2H, t,* $CH_2$*), 4.09-4.00 (1H, m,* $CH_{ali}$*), 3.85-3.75 (1H, m,* $CH_{ali}$*), 3.48-3.17 (4H, m, 4x*$CH_{ali}$*), 2.19-2.09 (1H, m,* $CH_{ali}$*), 2.05-1.86 (2H, m, 2x*$CH_{ali}$*), 1.82-1.65 (3H, m,* $CH_2$ *et* $CH_{ali}$*), 1.05-0.91 (9H, m, 3x*$CH_3$*).*

Le (S) 8-[1-*tert*-butoxycarbonyl-isopropyl-pyrrolidin-2-ylméthyl-sulfamoyl]-4-oxo-4,5-dihydro-3*H*-pyrrolo[2,3-*c*] quincline-1-carboxylate de propyle est préparé selon la synthèse 111, à partir de 326 mg (1 mmol) d'acide 8-chlorosulfonyl-4-oxo-4,5-dihydro-3*H*-pyrrolo[2,3-*c*]quinoline-1-mrboxylique et de 290 mg (1.2 mmol) de (S) 1-*tert*-butoxycarbonyl-2-isopropylaminométhyl-pyrrolidine pour donner après estérification suivant la méthode générale C (di-*n*-propylsulfate comme dialkylsulfate utilisé) et purification par chromatographie sur silice (éluant cyclohexane/acétate d'éthyle 1/1 puis 1/3), 106 mg (18% sur deux étapes) de (S) 8-[1-*tert*-butoxycarbonyl-isopropyl-pyrrolidin-2-ylméthyl-sulfamoyl]-4-oxo-4,5-dihydro-3*H*-pyrrolo[2,3-*c*]quinoline-1-carboxylate de propyle sous la forme d'un solide blanc.

A une solution de 886 mg (15 mmol) d'isopropylamine dans 10 mL de méthanol anhydre sont additionnés 1.9 mL (6 mmol) d'isopropoxyde de titanium et 1 g (5 mmol) de (S) 1-*tert*-butoxycarbonyl-pyrrolidine-2-carbaldéhyde. La solution est agitée 5 heures à température ambiante puis 227 mg (6 mmol) de borohydrure de sodium sont additionnés. L'agitation est maintenue pour 2 heures supplémentaires puis 2 mL d'eau sont additionnés. Le mélange réactionnel est filtré puis les solvants sont évaporés. De l'eau est additionné et le composé est extrait avec de l'éther diéthylique. Les phases organiques sont séchées sur sulfate de magnésium, filtrées et évaporées pour conduire à 1.05 g (86%) de (S) 1-*tert*-butoxycarbonyl-2-isopropylaminométhyl-pyrrolidine sous la forme d'une huile incolore.

**Chlorhydrate de (S) 4-Oxo-8-(prop-2-ynyl-pyrrolidin-2-ylméthyl-sulfamoyl)-4,5-dihydro-3*H*-pyrrolo[2,3-*c*]quinoline-1-carboxylate de propyle (150)**

Ce composé est préparé selon la synthèse 139, à partir de 188 mg (0.33 mmol) de (S) 8-[1-*tert*-butoxycarbonyl-prop-2-ynil-pyrrolidin-2-ylmémyl-sulfamoyl]-4-oxo-4,5-dihydro-3*H*-pyrrolo[2,3-*c*]quinoline-1-carboxylate de propyle et de 8.3 mL (16.6 mmol) d'une solution 2M d'acide chlorhydrique dans l'éther diéthylique pour donner après filtration, 110 mg (65%) de chlorhydrate de (S) 4-oxo-8-(prop-2-ynil-pyrrolidin-2-ylméthyl-sulfamoyl)- 4,5-dihydro-3*H*-pyrrolo[2,3-*c*] quinoline-1-carboxylate de propyle sous la forme d'un solide blanc.

***LCMS (IE, m/z):*** *(M+1) 471.3*

***RMN¹H :*** $\delta_H$ *ppm 400 MHz, DMSO*

*13.26 (1H, sl, NH), 12.13 (1H, s, NH), 9.89 (1H, d,* $CH_{arom}$*), 9.23 (1H, sl, NH.HCl), 8.54 (1H, sl, NH.HCl), 8.08 (1H, s,* $CH_{arom}$*), 7.84 (1H, dd,* $CH_{arom}$*), 7.57 (1H, d,* $CH_{arom}$*), 4.30-4.20 (4H, m, 2x*$CH_2$*), 3.91-3.80 (1H, m,* $CH_{ali}$*), 3.54-3.42 (1H,* m, $CH_{ali}$*), 3.41-3.33 (1H, m,* $CH_{ali}$*), 3.27-3.15 (1H,* m, $CH_{ali}$*), 3.00 (1H, t,* $CH_{alcyne}$*),2.12-1.88 (3H, m, 3x*$CH_{ali}$*), 1.81-1.66 (3H,* m, $CH_{ali}$ *et* $CH_2$*), 1.00 (3H, t,* $CH_3$*).*

Le (S) 8-[1-*tert*-butoxycarbonyl-prop-2-ynil-pyrrolidin-2-ylméthyl-sulfamoyl]-4-oxo-4,5-dihydro-3*H*-pyrrolo [2,3-*c*]quinoline-1-carboxylate de propyle est préparé selon la synthèse 111, à partir de 326 mg (1 mmol) d'acide 8-chlorosulfonyl-4-oxo-4,5-dihydro-3*H*-pyrrolo[2,3-*c*]quinoline-l-carboxylique et de 288 mg (1.2 mmol) de (S) 1-*tert*-butoxycarbonyl-prop-2-ynilaminométhyl-pyrrolidine pour donner après estérification suivant la méthode générale C (di-*n*-propylsulfate comme dialkylsulfate utilisé) et purification par chromatographie sur silice (éluant cyclohexane/acétate d'éthyle 1/1 puis 1/3), 192 mg (33% sur deux étapes) de (S) 8-[1-*tert*-butoxycarbonyl-prop-2-ynil-pyrrolidin-2-ylméthyl-sulfamoyl]-4-oxo-4,5-dihydro-3*H*-pyrrolo[2,3-*c*]quinoline-1-carboxylate de propyle sous la forme d'un solide blanc.

A une solution de 826 mg (15 mmol) de propargylamine dans 10 mL de méthanol anhydre sont additionnés 1.9 mL (6 mmol) d'isopropoxyde de titanium et 1 g (5 mmol) de (S) 1-*tert*-butoxycarbonyl-pyrrolidine-2-carbaldéhyde. La solution est agitée 5 heures à température ambiante puis 227 mg (6 mmol) de borohydrure de sodium sont additionnés. L'agitation est maintenue pendant 2 heures supplémentaires puis 2 mL d'eau sont additionnés. Le mélange réactionnel est filtré puis les solvants sont évaporés. De l'eau est additionné et le composé est extrait avec de l'éther diéthylique. Les phases organiques sont séchées sur sulfate de magnésium, filtrées et évaporées pour conduire à 1.2 g (quantitatif) de (S) 1-*tert*-butoxycarbonyl-prop-2-ynilaminométhyl-pyrrolidine sous la forme d'une huile incolore.

**(S) 8-[(1-Méthyl-pyrrolidin-2-ylméthyl)-prop-2-ynyl-sulfamoyl]-4-oxo-4,5-dihydro-3*H*-pyrrolo[2,3-*c*]quinoline-1-carboxylate de propyle (151)**

Ce composé est préparé selon la synthèse 119, à partir de 51 mg (0.1 mmol) de chlorhydrate de (S) 4-oxo-8-(prop-2-ynyl-pyrrolidin-2-ylméthyl-sulfamoyl)-4,5-dihydro-3*H*-pyrcolo[2,3-*c*]quinoline-1-carboxylate de propyle, de 67 µL (0.5 mmol) de triéthylamine et de 14 µL (0.15 mmol) de diméthylsulfate pour donner après précipitation dans l'eau

et trituration dans l'éther diisopropylique, 34 mg (70%) de (S) 8-[(1-méthyl-pyrrolidin-2-ylméthyl)-prop-2-ynyl-sulfamoyl]-4-oxo-4,5-dihydro-3*H*-pyrrolo[2,3-*c*]quinoline-1-carboxylate de propyle sous la forme d'un solide blanc.
***LCMS (IE, m/z):*** *(M+1) 485.3*
***RMN$^1$H :*** *$\delta_H$ ppm 400 MHz, DMSO*
*12.00 (1H, s, NH), 9.84 (1H, d, $CH_{arom}$), 8.05 (1H, s, $CH_{arom}$), 7.78 (1M, dd, $CH_{arom}$),* 7.53 *(1H, d, $CH_{arom}$), 4.29-4.11 (4H, m, $2xCH_2$), 3.20-288 (4H, m, $4xCH_{ali}$), 2.75 (1H, t, $CH_{alcyne}$), 2.25 (3H, s, $NCH_3$), 2.15-2.05 (1H, m, $CH_{ali}$), 1.92-1-55 (6H, m, $6xCH_{ali}$), 0.99 (3H, t, $CH_3$).*

**(S) 8-[(2-*tert*-Butoxycarbonylamino-propyl)-cyclopropyl-sulfamoy]-4-oxo-4,5-dihydro-3*H*-pyrrolo[2,3-*c*]quinoline-1-carboxylate de propyle (152)**

Ce composé est préparé selon la synthèse 111, à partir de 326 mg (1 mmol) d'acide 8-chlorosulfonyl-4-oxo-4,5-dihydro-3H-pyrrolo[2,3-*c*]quinoline-1-carboxylique, 1.11 mL (8 mmol) de triéthylamine et de 321 mg (1.5 mmol) de chlorhydrate de (S) N'-*tert*-butoxycarbonyl-N-cyclopropyl-propane-1,2-diamine pour donner après estérification suivant la méthode générale C (di-*n*-propylsulfate comme dialkylsulfate utilisé) et purification par chromatographie sur silice (éluant dichlorométhane/méthanol 97/3) puis trituration dans l'éther diéthylique, 115 mg (21% sur deux étapes) de (S) 8-[(2-*tert*-butoxycarbonylamino- propyl)-cyclopropyl- sulfamoyl]- 4- oxo- 4,5- dihydro- 3*H*-pyrrolo [2,3-*c*] quinoline- 1- carboxylate de propyle sous la forme d'un solide blanc.
***LCMS (IE, m/z):*** *(M+1) 547.19*
***RMN$^1$H*** *: $\delta_H$ ppm 400 MHz, $CDCl_3$*
*11.57 (1H, sl, NH), 10.91 (1H, sl, NH), 10.01 (1H, s, $CH_{arom}$), 8.01 (1H, s, $CH_{arom}$), 7.87 (1H, d, $CH_{arom}$), 7.46 (1H, d, $CH_{arom}$), 4.97 (1H, sl, NH), 4.26 (2H, t, $OCH_2$), 4.06 (1H, s; CH), 3.47 (1H, s, $NCH_2$), 3.17 (1H, s, $NCH_2$), 2.18 (1H, s, CH), 1.79 (2H, dd, $CH_2$), 1.42 (9H, s, $3xCH_3$), 1.21 (3H, d, $CH_3$), 1.03 (3H, t, $CH_3$), 0.98 (2H, sl, $2xCH_{cyclaprop}$) 0.74 (2H, sl, $2xCH_{cycloprop}$),*

A une solution de 1.88 g (33 mmol) de cyclopropylamine dans 10 mL de méthanol anhydre sont additionnés 4.3 mL (14.3 mmol) d'isopropoxyde de titanium et 1.9 g (5 mmol) de (S) 2-(*tert*-butoxycarbonylamino)-propionaldéhyde (préparé selon la méthode décrite par Itaya, taisuke et al., Tetrahedron Lett., 1986, 52(27), 6349-52). La solution est agitée 5 heures à température ambiante puis 227 mg (6 mmol) de borohydrure de sodium sont additionnés. L'agitation est maintenue pendant 2 heures supplémentaires puis 2 mL d'eau sont additionnés. Le mélange réactionnel est filtré puis les solvants sont évaporés. Le résidu est purifié par chromatographie sur silice (éluant dichlorométhane/méthanol 98/2) pour conduire à 400 mg (17%) de (S) N'-*tert*-butoxycarbonyl-*N*-cyclopropyl-propane-1,2-diamine sous la forme d'une huile incolore.

**Chlorhydrate de (S) 8-[(2-amino-propyl)-cyclopropyl-sulfamoyl]-4-oxo-4,5-dihydro-3*H*-pyrrolo[2,3-*c*]quinoline-1-carboxylate de propyle (153)**

Ce composé est préparé selon la synthèse 139, à partir de 44 mg (0.08 mmol) (S) 8-[(2-*tert*-butoxycarbonylamino-propyl)-cyclopropyl-sulfamoyl]-4-oxo-4,5-dihydro-3*H*-pyrrolo[2,3-*c*]quinoline-1-carboxylate de propyle et de 2 mL (4 mmol) d'une solution 2M d'acide chlorhydrique dans l'éther diéthylique pour donner après filtration, 35 mg (78%) de chlorhydrate de(S)8-[(2-amino-propyl)-cyclopropyl-sulfamoyl]-4-oxo-4,5-dihydro-3*H*-pyrrolo[2,3-*c*]quinoline-1-carboxylate de propyle sous la forme d'un solide blanc.
***LCMS (IE, m/z):*** *(M+1) 447.28*
***RMN$^1$H:*** *$\delta_H$ ppm 400 MHz, DMSO*
*13.32 (1H, sl, NH), 12.22 (1H, sl, NH), 9.93 (1H, s, $CH_{arom}$), 8.12 (1H, s, $CH_{arom}$), 8.04 (2H, sl, $NH_2$), 7.88 (1H, d, $CH_{arom}$), 7.67 (1H, d, $CH_{arom}$), 4.27 (2H, t, $OCH_2$), 3.32-3.12 (3H, m, $3xCH_{ali}$), 2.01 (1H, sl, CH), 1.85-1.75 (2H, m, $CH_2$), 1.23 (3H, d, $CH_3$), 1.05-1.00 (3H, m, $CH_3$), 0.93-0.74 (4H, m, $4xCH_{cycloprop}$).*

**(R) 8-[(2-*tert*-Butoxycarbonylamino-propyl)-cyclopropyl-sulfamoyl]-4-oxo-4,5-dihydro-3*H*-pyrrolo[2,3-*c*]quinoline-1-carboxylate de propyle (154)**

Ce composé est préparé selon la synthèse 111, à partir de 326 mg (1 mmol) d'acide 8-chlorosulfonyl-4-oxo-4,5-dihydro-3*H*-pyrrolo[2,3-c]quinoline-l-carboxylique, 1.11 mL (8 mmol) de triéthylamine et de 321 mg (1.5 mmol) de chlorhydrate de (R) *N'*-*tert*-butoxycarbonyl-*N*-cyclopropyl-propane-1,2-diamine pour donner après estérification suivant la méthode générale C (di-*n*-propylsulfate comme dialkylsulfate utilisé) et purification par chromatographie sur silice (éluant dichlorométhane/méthanol 97/3) puis trituration dans l'éther diéthylique, 165 mg (30% sur deux étapes) de (R) 8-[(2-*tert*-butoxycarbonylamino- propyl)-cyclopropyl- sulfamoyl]- 4- oxo- 4,5- dihydro- 3*H*-pyrrolo [2,3-*c*] quinoline- 1- carboxylate de propyle sous la forme d'un solide blanc.
***LCMS (IE, m/z):*** *(M+1) 547.19*

***RMN$^1$H** : $\delta_H$ ppm 400 MHz, $CDCl_3$*

*11.57 (1H, sl, NH), 10.91 (1H, sl, NH), 10.01 (1H, s, $CH_{arom}$), 8.01 (1H, s, $CH_{arom}$), (1H, d, $CH_{arom}$), 7.46 (1H, d, $CH_{arom}$), 4.97 (1H, sl, NH), 4.26 (2H, t, $OCH_2$), 4.06 (1H, s, CH), 3.47 (1H, s, $CH_2$), 3.17 (1H, s, $CH_2$), 2.18 (1H, s, CH), 1.79 (2H, dd, $CH_2$), 1.42 (9H, s, $3xCH_3$), 1.21 (3H, d, $CH_3$), 1.03 (3H, t, $CH_3$), 0.98 (2H, sl, $2xCH_{cycloprop}$) 0.74 (2H, sl, $2xCH_{cycloprop}$).*

A une solution de 1.88 g (33 mmol) de cyclopropylamine dans 10 mL de méthanol anhydre sont additionnés 4.3 mL (14.3 mmol) d'isopropoxyde de titanium et 1.9 g (5 mmol) de (R) 2-(*tert*-butoxycarbonylamino)-propionaldéhyde (préparé selon la méthode décrite par Itaya, taisuke et al., Tetrahedron Lett., 1986, 52(27), 6349-52). La solution est agitée 5 heures à température ambiante puis 227 mg (6 mmol) de borohydrure de sodium sont additionnés. L'agitation est maintenue pour 2 heures supplémentaires puis 2 mL d'eau sont additionnés. Le mélange réactionnel est filtré puis les solvants sont évaporés. Le résidu est purifié par chromatographie sur silice (éluant dichlorométhane/méthanol 98/2) pour conduire à 350 mg (14%) de (R) *N*'-*tert*-butoxycarbonyl, *N*-cyclopropyl-propane-1,2-diamine sous la forme d'une huile incolore.

**Chlorhydrate de (R) 8-[(2-amino-propyl)-cyclopropyl-sulfamoyl]-4-oxo-4,5-dihydro-3*H*-pyrrolo[2,3-*c*]quinoline-1-carboxylate de propyle (155).**

Ce composé est préparé selon la synthèse 139, à partir de 44 mg (0.08 mmol) (R) 8-[(2-*tert*-butoxycarbonylamino-propyl)-cyclopropyl-sulfamoyl]-4-oxo-4,5-dihydro-3*H*-pyrrolo[2,3-*c*]quinoline-1-carboxylate de propyle et de 2 mL (4 mmol) d'une solution 2M d'acide chlorhydrique dans l'éther diéthylique pour donner après filtration, 36 mg (81%) de chlorhydrate de (R) 8-[(2-amino-propyl)-cyclopropyl-sulfamoyl]-4-oxo-4,5-dihydro-3*H*-pyrrolo[2,3-*c*]quinoline-1-carboxylate de propyle sous la forme d'un solide blanc.

***LCMS (IE, m/z)** : (M+1) 447.23*

***RMN$^1$H :** $\delta_H$ ppm 400 MHz, DMSO*

*13.32 (1H, sl, NH), 12.22 (1H, sl, NH), 9.93 (1H, s, $CH_{arom}$), 8.12 (1H, s, $CH_{arom}$), 8.04 (2H, sl, $NH_2$), 7.88 (1H, d, $CH_{arom}$), 7.67 (1H, d, $CH_{arom}$), 4.27 (2H, t, $OCH_2$), 3.32-3.12 (3H, m, $3xCH_{ali}$), 2.01 (1H, sl, CH), 1.85-1.75 (2H, m, $CH_2$), 1.23 (3H, d, $CH_3$), 1.05-1.00 (3H, m, $CH_3$), 0.93-0.74 (4H, m, $4xCH_{cycloprop}$).*

**Chlorhydrate de 8-[(2-Méthylamino-éthyl)-prop-2-ynyl-sulfamoyl]-4-oxo-4,5-dihydro-3*H*-pyrrolo[2,3-*c*]quinoline-1-carboxylate de propyle (156).**

Ce composé est préparé selon la synthèse 139, à partir de 54 mg (0.1 mmol) 8-{[2-(*tert*-butoxycarbonyl-méthyl-amino)-éthyl]-prop-2-ynyl-sulfamoyl}-4-oxo-4,5-dihydro-3*H*-pyrrolo[2,3-*c*]quinoline-1-carboxylate de propyle et de 2.5 mL (5 mmol) d'une solution 2M d'acide chlorhydrique dans l'éther diéthylique pour donner après filtration, 42 mg (87%) de chlorhydrate de 8-[(2-méthylamino-éthyl)-prop-2-ynyl-sulfamoyl]-4-oxo-4,5-dihydro-3*H*-pyrrolo[2,3-*c*]quinoline-1-mboxylate de propyle sous la forme d'un solide blanc.

***LCMS (IE, m/z):** (M+1) 445.23*

***RMN$^1$H :** $\delta_H$ ppm 400 MHz, DMSO*

*13.22 (1H, sl, NH), 12.13 (1H, sl, NH), 9.89 (1H, s, $CH_{arom}$), 8.64 (2H, sl, $NH_2$), 8.07 (1H, s, $CH_{arom}$), 7.83 (1H, dd, $CH_{arom}$), 7.57 (1H, d, $CH_{arom}$), 4.26-4.22 (4H, m, $NCH_2$, $OCH_2$), 3.45 (2H, sl, $CH_2$), 3.20-3.18 (2H, m, $CH_2$), 3.03 (1H, s, $CH_{alcyne}$), 2.62 (3H, s, $NCH_3$), 1.79-1.73 (2H m, $CH_2$), 1.00 (3H, t, $CH_3$).*

**8-{[2-(*tert*-Butoxycarbonyl-méthyl-amino)-éthyl]-prop-2-ynyl-sulfamoyl}-4-oxo-4,5-dihydro-3*H*-pyrrolo[2,3-*c*] quinoline-1-carboxylate de propyle (157)**

Ce composé est préparé selon la synthèse 111, à partir de 326 mg (1 mmol) d'acide 8-chlorosulfonyl-4-oxo-4,5-ihydro-3H-pyrrolo[2,3-*c*]quinoline-1-carboxylique, 1.11 mL (8 mmol) de triéthylamine et de 318 mg (1.5 mmol) de *N*-*tert*-butoxycarbonyl-*N*-méthyl-*N'*-prop-2-ynyl-éthane-1,2-diamine pour donner après estérification suivant la méthode générale C (di-*n*-propylsulfate comme dialkylsulfate utilisé) et purification par chromatographie sur silice (éluant cyclohexane/acétate d'éthyle 1/1 puis 1/9), 69 mg (12% sur deux étapes) de 8-{[2-(*tert*-butoxycarbonyl-méthyl-amino)-éthyl]-prop-2-ynyl-sulfamoyl}-4-oxo-4,5-dihydro-3*H*-pyrrolo[2,3-*c*]quinoline-1-mboxylate de propyle sous la forme d'un solide blanc.

***LCMS (IE, m/z):** (M+1-Boc) 445.26*

***RMN$^1$H :** $\delta_H$ ppm 400 MHz, $CDCl_3$*

*11.18 (1H, sl, NH), 10.41 (1H, sl, NH), 10.11 (1H, s, $CH_{arom}$), 8.03 (1H, s, $CH_{arom}$), 7.88 (1H, dd, $CH_{arom}$), 7.45 (1H, sl, $CH_{arom}$), 4.32-4.22 (4H, m, $2xCH_2$), 3.51 (2H, sl, $CH_2$), 2.96 (3H, s, $NCH_3$), 1.97-1.91 (1H, m, $CH_{alcyne}$), 1.81 (2H, dd, $CH_2$), 1.46 (9H, t, $3xCH_3$), 1.04 (3H, t, $CH_3$).*

A une solution de 1.16 g (21 mmol) de propargylamine dans 50 mL de tétrahydrofurane anhydre sont additionnés

2.7 mL (9.1 mmol) d'isopropoxyde de titanium et 1.2 g (7 minol) de (*tert*-butoxycarbonyl-méthyl-amino)-acétaldéhyde (préparé selon la méthode décrite par Kato, shiro et al., J. Chem. Soc. Perkin Trans. 1, 1997, 21, 3219-26). La solution est agitée 5 heures à température ambiante puis 5 mL de méthanol anhydre et 317 mg (8.4 mmol) de borohydrure de sodium sont additionnés. L'agitation est maintenue pendant 2 heures supplémentaires puis 2 mL d'eau sont additionnés. Le mélange réactionnel est filtré puis les solvants sont évaporés. Le résidu est purifié par chromatographie sur silice (éluant dichlorométhane/méthanol 98/2) pour conduire à 550 mg (37%) de *N*-*tert*-butoxycarbonyl-*N*-méthyl-*N*-prop-2-ynyl-éthane-1,2-diamine sous la forme d'une huile incolore.

**Chlorhydrate de 8-[(2-isopropylamlno-éthyl)-prop-2-ynyl-sulfamoyl]-4-oxo-4,5-dihydro-3*H*-pyrrolo[2,3-*c*]quinoline-1-carboxylate de propyle (158)**

Ce composé est préparé selon la synthèse 139, à partir de 57 mg (0.1 mmol) 8-{[2-(*tert*-butoxycarbonyl-isopropyl-amino)-éthyl]-prop-2-ynyl-sulfamoyl}-4-oxo-4,5-dihydro-3*H*-pyrrolo[2,3-*c*]quinoline-1-carboxylate de propyle et de 2.5 mL (5 mmol) d'une solution 2M d'acide chlorhydrique dans l'éther diéthylique pour donner après filtration, 42 mg (82%) de chlorhydrate de 8-[(2-isopropylamino-éthyl)-prop-2-ynyl-sulfamoyl]-4-oxo-4,5-dihydro-3*H*-pyrrolo[2,3-*c*]quinoline-1-carboxylate de propyle sous la forme d'un solide blanc.
***LCMS (IE, m/z):*** *(M+1) 473.25*
***RMN¹H :*** *$\delta_H$ ppm 400 MHz, DMSO*
*13.27 (1H, sl, NH), 12.15 (1H, sl, NH), 9.91 (1H, s, $CH_{arom}$), 8.49 (2H, sl, $NH_2$), 8.12 (1H, s, $CH_{arom}$), 7.86 (1H, d, $CH_{arom}$), 7.57 (1H, d, $CH_{arom}$), 4.28-4.24 (4H, m, $2xCH_2$), 3.59-3.48 (2H, m, $NCH_2$), 3.42-3.36 (1H, m, CH), 3.23-3.14 (2H, sl, $CH_2$), 3.09 (1H, s, $CH_{alcyne}$), 1.82-1.75 (2H, m, $CH_2$), 1.27 (6H, d, $2xCH_3$), 1.00 (3H, t, $CH_3$).*

**8-{[2-(*tert*-Butoxycarbonyl-isopropyl-amino)-éthyl]-prop-2-ynyl-sulfamoyl}-4-oxo-4,5-dihydro-3*H*-pyrrolo[2,3-*c*]quinoline-1-carboxylate de propyle (159)**

Ce composé est préparé selon la synthèse 111, à partir de 326 mg (1 mmol) d'acide 8-chlorosulfonyl-4-oxo-4,5-dihydro-3*H*-pyrrolo[2,3-*c*]quinoline-1-carboxylique, 1.11 mL (8 mmol) de triéthylamine et de 360 mg (1.5 mmol) de *N*-*tert*-butoxycarbonyl-*N*-isopropyl-*N'*-prop-2-ynyl-éthane-1,2-diamine pour donner après estérification suivant la méthode générale C (di-*n*-propylsulfate comme dialkylsulfate utilisé) et purification par chromatographie sur silice (éluant cyclohexane/acétate d'éthyle 1/1 puis 1/9) puis trituration dans l'éther diéthylique, 260 mg (45% sur deux étapes) de 8-{[2-(*tert*-butoxycarbonyl-méthyl-amino)-éthyl]-prop-2-ynyl-sulfamoyl}-4-oxo-4,5-dihydro-3*H*-pyrrolo[2,3-*c*]quinoline-1-carboxylate de propyle sous la forme d'un solide blanc.
***LCMS (IE, m/z):*** *(M+1) 573.38*
***RMN¹H:*** *$\delta_H$ ppm 400 MHz, $CDCl_3$*
*11.36 (1H, sl, NH), 10.65 (1H, sl, NH), 10.10 (1H, s, $CH_{arom}$), 8.03 (1H, s, $CH_{arom}$), 7.88 (1H, d, $CH_{arom}$), 7.45 (1H, sl, $CH_{arom}$), 4.29-4.22 (4H, m, $2xCH_2$), 3.45-3.36 (4H, m, $2xCH_2$), 1.97 (1H, s, $CH_{alcyne}$), 1.82-1.75 (2H, m, $CH_2$), 1.67-1.62 (1H, m, $CH_{ali}$), 1.46 (9H, s, $3xCH_3$) 1.20 (6H, d, $2xCH_3$), 1.00 (3H, t, $CH_3$).*
A une solution de 1.65 g (30 mmol) de propargylamine dans 50 mL de tétrahydrofurane anhydre sont additionnés 3.9 mL (13 mmol) d'isopropoxyde de titanium et 2 g (10 mmol) de (*tert*-butoxycarbonyl-isopropyl-amino)acétaldéhyde (préparé selon la méthode décrite par Kato, shiro et al., J. Chem. Soc. Perkin Trans. 1, 1997, 21, 3219-26). La solution est agitée 5 heures à température ambiante puis 5 mL de méthanol anhydre et 317 mg (8.4 mmol) de borohydrure de sodium sont additionnés. L'agitation est maintenue pendant 2 heures supplémentaires puis 2 µL d'eau sont additionnés. Le mélange réactionnel est filtré puis les solvants sont évaporés. Le résidu est purifié par chromatographie sur silice (éluant dichlorométhane/méthanol 98/2) pour conduire à 1.2 g (50%) de *N*-*tert*-butoxycarbonyl-*N*-isopropyl-*N*'-prop-2-ynyl-éthane-1,2-diamine sous la forme d'une huile incolore.

**Chlorhydrate de (S) 8-(éthyl-pyrrolidin-3-yl-sulfamoyl)-4-oxo-4,5-dihydro-3*H*-pyrrolo[2,3-*c*]quinoline-1-carboxylate de propyle (160)**

Ce composé est préparé selon la synthèse 139, à partir de 82 mg (0.15 mmol) de (S) 8-[(1-*tert*-butoxycarbonyl-éthyl-pyrrolidin-3-yl)-sulfamoyl]-4-oxo-4,5-dihydro-3*H*-pyrrolo[2,3-*c*]quinoline-1-carboxylate de propyle et de 7 mL (14 mmol) d'une solution 2M d'acide chlorhydrique dans l'éther diéthylique pour donner après filtration, 15 mg (20%) de chlorhydrate de (S) 8-(éthyl-pyrrolidin-3-yl-sulfamoyl)-4-oxo-4,5-dihydro-3*H*-pyrrolo[2,3-*c*]quinoline-1-carboxylate de propyle sous la forme d'un solide blanc.
***LCMS (IE, m/z):*** *(M+1) 447.30*
***RMN¹H :*** *$\delta_H$ ppm 400 MHz, DMSO*
*13.29 (1H, sl, NH), 12.16 (1H, sl, NH), 9.86 (1H, d, $CH_{arom}$), 9.00-8.85 (1H, m, NH.HCl), 8.78-8.64 (1H, m, NH.HCl), 8.09 (1H, d, $CH_{arom}$), 7.85 (1H, dd, $CH_{arom}$), 7.57 (1H, d, $CH_{arom}$), 4.61-4.51 (1H, m, $CH_{ali}$), 4.25 (2H, t, $CH_2$), 3.63-3.57*

*(1H, m, $CH_{ali}$), 3.29-3.16 (3H, m, 3x$CH_{ali}$), 3.06-2.95 (1H, m, $CH_{ali}$), 2.85-2.75 (1H, m, $CH_{ali}$), 2.05-1.96 (1H, m, $CH_{ali}$), 1.82-1.69 (3H, m, $CH_{ali}$ et $CH_2$), 1.20 (3H, t, $CH_3$), 1.00 (3H, t, $CH_3$).*

Le (S) 8-[(1-*tert*-butoxycarbonyl-éthyl-pyrrolidin-3-yl)-sulfamoyl]-4-oxo-4,5-dihydro-3*H*-pyrrolo[2,3-*c*]quinoline-1-carboxylate de propyle est préparé selon la synthèse 111, à partir de 500 mg (1.53 mmol) d'acide 8-chlorosulfonyl-4-oxo-4,5-dihydro-3*H*-pyrrolo[2,3-*c*]quinoline-1-carboxylique, 640 μL (4.6 mmol) de triéthylamine et de 655 mg (3 mmol) de (S) 1-*tert*-butoxycarbonyl-3-éthylamino-pyrrolidine pour donner après estérification suivant la méthode générale C (di-*n*-propylsulfate comme dialkylsulfate utilisé) et purification par chromatographie sur silice (éluant cyclohexane/acétate d'éthyle 1/1 puis 1/3), 85 mg (10% sur deux étapes) de (S) 8-[(1-*tert*-butoxycarbonyl-éthyl-pyrrolidin-3-yl)-sulfamoyl]-4-oxo-4,5-dihydro-3*H*-pyrrolo[2,3-*c*]quinoline-1-carboxylate de propyle sous la forme d'un solide blanc.

A une solution de 1.47 mL (8.4 mmol) de (S) 3-amino-1-*tert*-butoxycarbonylpyrrolidine dans 30 mL de méthanol anhydre sont additionnés 367 μL (6.5 mmol) d'acétaldéhyde, 817 mg (13 mmol) de cyanoborohydrure de sodium et 3.75 mL (65 mmol) d'acide acétique. La solution est agitée à température ambiante pour 24 heures puis une solution aqueuse saturée en hydrogénocarbonate de sodium est additionnée pour ajuster le pH à 7. Le composé est extrait avec de l'acétate d'éthyle. Les phases organiques sont séchées sur sulfate de magnésium, filtrées puis évaporées et le résidu est purifié par chromatographie sur silice (éluant dichlorométhane/méthanol 94/6) pour conduire à 970 mg (70%) de (S) 1-*tert*-butoxycarbonyl-3-éthylamino-pyrrolidine sous la forme d'une huile jaune.

**(S) 8-[Ethyl-(1-méthyl-pyrrolidin-3-yl)-sulfamoyl]-4-oxo-4,5-dihydro-3*H*-pyrrolo[2,3-*c*]quinoline-1-carboxylate de propyle (161)**

Ce composé est préparé selon la synthèse 119, à partir de 77 mg (0.16 mmol) de chlorhydrate de (S) 8-(éthyl-pyrrolidin-3-yl-sulfamoyl)-4-oxo-4,5-dihydro-3*H*-1-pyrrolo[2,3-*c*]quinoline-1-carboxylate de propyle, de 134 μL (0.96 mmol) de triéthylamine et de 30 μL (0.32 mmol)) de diméthylsulfate pour donner après purification par chromatographie sur silice (éluant dichlorométhane/méthanol 9/1), 17 mg (23%) de (S) 8-[éthyl-(1-méthyl-pyrrolidin-3-yl)-sulfamoyl]-4-oxo-4,5-dihydro-3*H*-pyrrolo[2,3-*c*]quinoline-1-carboxylate de propyle sous la forme d'un solide blanc.

***LCMS (IE, m/z):*** *(M+1) 461.34*

***RMN$^1$H*** *: $\delta_H$ ppm 400 MHz, DMSO*

*13.22 (1H, sl, NH), 12.08 (1H, sl, NH), 9.82 (1H, d, $CH_{arom}$), 8.07(1H, d, $CH_{arom}$), 7.80 (1H, dd, $CH_{arom}$), 7.54 (1H, d, $CH_{arom}$), 4.55-4.44 (1H, m, $CH_{ali}$), 4.25 (2H, t, $CH_2$), 3.36-3:21 (2H, m, 2x$CH_{ali}$), 2.81-2.61 (1H, m, $CH_{ali}$), 2.52-2.38 (1H, m, $CH_{ali}$), 2.25 (3H, s, $CH_3$), 2.09-1.95 (1H, m, $CH_{ali}$), 1.90-1.76 (2H, m, $CH_2$), 1.63-1.50 (1H, m, $CH_{ali}$), 1.39-1.24 (4H, m, $CH_{ali}$ et $CH_3$), 1.06 (3H, t, $CH_3$), 0.97-0.87 (1H, m, $CH_{ali}$).*

**4-Oxo-8-(pyrrolidine-1-sulfonyl)-4,5-dihydro-3*H*-pyrrolo[2,3-*c*]quinoline-1-carboxylate d'éthyle (162)**

Ce composé est préparé selon la synthèse 111, à partir de 326 mg (1 mmol) d'acide 8-chlorosulfonyl-4-oxo-4,5-dihydro-3*H*-pyrrolo[2,3-*c*]quinoline-1-carboxylique et de 86 μL (1.05 mmol) de pyrrolidine pour donner après estérification suivant la méthode générale C (diéthylsulfate comme dialkylsulfate utilisé) et purification par chromatographie sur silice (éluant dichlorométhane/méthanol 97/3), 150 mg (38% sur deux étapes) de 4-oxo-8-(pyrrolidine-1-sulfonyl)-4,5-dihydro-3*H*-pyrrolo[2,3-*c*]quinoline-1-carboxylate d'éthyle sous la forme d'un solide blanc.

***LCMS (IE, m/z):*** *(M+1) 390.14*

***RMN$^1$H :*** *$\delta_H$ ppm 400 MHz, DMSO*

*13.20 (1H, sl, NH), 12.08 (1H, sl, NH), 9.80 (1H, d, $CH_{arom}$), 8.06 (1H, s, $CH_{arom}$), 7.80 (1H, dd, $CH_{arom}$), 7.57 (1H, d, $CH_{arom}$), 4.33 (2H, q, $CH_2$), 3.23-3.15 (4H, m, 4x$CH_{ali}$), 1.67-1.58 (4H, m, 4x$CH_{ali}$), 1.35 (3H, t, $CH_3$).*

**(S) 4-Oxo-8-(2-pyrrolidin-1-ylméthyl-pyrrolidine-1-sulfonyl)-4,5-dihydro-3*H*-pyrrolo[2,3-*c*]quinoline-1-carboxylate d'éthyle (163)**

Ce composé est préparé selon la synthèse 111, à partir de 326 mg (1 mmol) d'acide 8-chlorosulfonyl-4-oxo-4,5-dihydro-3*H*-pyrrolo[2,3-*c*]quinoline-1-carboxylique et de 162 mg (1.05 mmol) de (S) 2-pyyrolidin-1-ylméthylpyrrolidine pour donner après estérification suivant la méthode générale C (diéthylsulfate comme dialkylsulfate utilisé) et purification par chromatographie sur silice (éluant chloroforme/méthanol/ammoniaque 250/25/4), 56 mg (12% sur deux étapes) de (S) 4-oxo-8-(2-pyrrolidin-1-ylméthyl-pyrrolidine-1-sulfonyl)-4,5-dihydro-3*H*-pyrrolo[2,3-*c*]quinoline-1-carboxylate d'éthyle sous la forme d'un solide blanc.

***LCMS (IE, m/z):*** *(M+1) 473.10*

***RMN $^1$H:*** *$\delta_H$ ppm 400 MHz, DMSO*

*13.21 (1H, sl, NH), 12.08 (1H, sl, NH), 9.87 (1H, d, $CH_{arom}$), (1H, s, $CH_{arom}$), 7.81 (1H, dd, $CH_{arom}$), 7.56 (1H, d, $CH_{arom}$), 4.32 (2H, q, $CH_2$), 3.85-3.78 (1H, m, $CH_{ali}$), 3.11-3.03 (1H, m, $CH_{ali}$), 2.65-2.39 (7H, m, 7x$CH_{ali}$), 1.78-1.58 (6H; m, 6x$CH_{ali}$), 1.45-1.33 (2H; m, 2x$CH_{ali}$), 1.34 (3H, t, $CH_3$).*

**(S) 8-(3-*tert*-Butoxycarbonylamino-pyrrolidine-1-sulfonyl)-4-oxo-4,5-hydro-3*H*-pyrrolo[2,3-*c*]quinoline-1-carboxylate de propyle (164)**

Ce composé a été préparé selon la méthode générale d'estérification C à partir de 5.45 g (11.44 mmol) d'acide (S) 8-(3-*tert*-butoxycarbonylamino-pyrrolidine-1-sulfonyl)-4-oxo-4,5-dihydro-3*H*-pyrrolo[2,3-*c*]quinoline-1-carboxylique, de 7.99 mL (45 mmol) de triéthylamine et de 2.07 mL (12.58 mmol) de di-*n*-propylsulfate. L'agitation de la solution à 60°C pour 4 heures conduit après purification par chromatographie sur silice (éluant acétate d'éthyle) puis recristallisation dans un mélange propanol/éther diisopropylique à 2.94 g (49%) de (S) 8-(3-*tert*- butoxycarbonylamino-pyrrolidine-1-sulfonyl)-4-oxo-4,5-dihydro-3*H*-pyrrolo[2,3-*c*]quinoline-1-carboxylate de propyle sous la forme d'un solide blanc.
***LC/MS (IE, m/z) :*** *(M+1) 519.35*
***RMN¹H :*** *δH ppm 400 MHz, DMSO*
*13.24 (1H, sl, NH), 12.13 (1H, sl, NH), 9.82 (1H, s, $CH_{arom}$), 8.08 (1H, s, $CH_{arom}$), 7.78 (1H, d, $CH_{arom}$), 7.58 (1H, d, $CH_{arom}$), 6.94 (1H, d, BocNH), 4.25 (2H, t, $CH_2$), 3.83-3.75 (1H, m, $CH_{pyrrol}$), 3.38-3.30 (2H, m, 2x $CH_{pyrrol}$), 3.24-3.17 (1H, m, $CH_{pyyrrol}$), 3.00-2.94 (1H, m, $CH_{pyrrol}$), 1.91-1.82 (1H, m, $CH_{pyrrol}$), 1.75 (2H, m, $CH_2$), 1.65-1.57 (1H, m, $CH_{pyrrol}$), 1.28 (9H, s, 3x$CH_3$), 0.99 (3H, t, $CH_3$).*

(S) **Acide-8-(3-*tert*-butoxycarbonylamino-pyrrolidine-1-sulfonyl)-4-oxo-4,5-dihydro-3*H*-pyrrolo[2,3-*c*]quinoline-1-carboxylique (165)**

Ce composé est préparé selon la synthèse 178, à partir de 5.88 g (18 mmol) d'acide 8-chlorosulfonyl-4-oxo-4,5-dihydro-3*H*-pyrrolo[2,3-c]quinoline-1-carboxylique, 12.54 mL (90 mmol) de triéthylamine et de 3.69 g (19.8 mmol) de (S) *N*-*tert*-butoxycarbonyl-*N*'-pyrrolidin-3yl-amine pour donner après purification par précipitation et trituration dans l'eau, 6.18 g (72%) d'acide (S) 8-(3-*tert*-butoxycarbonylamino-pyrrolidine-l-sulfonyl)-4-oxo-4,5-dihydro-3*H*-pyrrolo[2,3-*c*]quinoline-1-carboxylique sous la forme d'un solide beige.
***LC/MS (IE, mlz):*** *(M+1) 477.31*
***RMN¹H*** *: $\delta_H$ ppm 400 MHz, DMSO*
*12.64 (1H, sl, $CO_2H$), 12.06 (1H, sl, NH), 9.97 (1H, s, $CH_{arom}$), 7.99 (1H, s, $CH_{arom}$), 7.76 (1H, d, $CH_{arom}$), 7.55 (1H, d, $CH_{arom}$), 6.94 (1H, sl, BocNH), 3.89-3.75 (1H, m, $CH_{pyrrol}$), 3.30-3.18 (3H, m, 3x$CH_{pyrrol}$), 3.03-2.93 (1H, m, $CH_{pyrrol}$), 1.93-1.82 (1H, m, $CH_{pyrrol}$), 1.68-1.57 (1H, m, $CH_{pyrrol}$), 1.30 (9H, s, 3x$CH_3$).*

**Chlorhydrate du (S) 8-(3-amino-cyclopentanesulfonyl)-4-oxo-4,5-dibydro-3*H*-pyrrolo[2,3-*c*]quinoline-1-carboxylate de propyle (166)**

Ce composé est préparé selon la synthèse 139, à partir de 93 mg (0.18 mmol) de (S) 8-(3-*tert*-butoxycarbonylamino-pyrrolidine-1-sulfonyl)-4-oxo-4,5-dihydro-3*H*-pyrrolo[2,3-*c*]quinoline-1-carboxylate de propyle et de 4.5 mL (9 mmol) d'une solution 2M d'acide chlorhydrique dans l'éther diéthylique pour donner après filtration, 58 mg (71%) de chlorhydrate de (S) 8-(3-amino-cyclopenlanesufonyl)-4-oxo-4,5-dihydro-3*H*-pyrrolo[2,3-*c*]quinoline-1-carboxylate de propyle sous la forme d'un solide blanc:
***LC/MS (IE, m/z) :*** *(M+1) 419.29*
***RMN¹H:*** *$\delta_H$ ppm 400 MHz, DMSO*
*13.25 (1H, sl, NH), 12.15 (1H, sl, NH), 9.84 (1H, s, $CH_{arom}$), 8.15 (2H, sl, NH.HCl), 8.09 (1H, s, $CH_{arom)}$, 7.82 (1H, d, $CH_{arom}$), 7.62 (1H, d, $CH_{arom}$), 4.26 (2H, t, $CH_2$), 3.75-3.63 (1H, m, $CH_{pyyrol}$), 3.49-3.39 (1H, m, $CH_{pyyrol}$), 3.39-3.27 (1H, m, $Ch_{pyyrol}$), 3.27-3.12 (2H, m, 2x$CH_{pyyrol}$), 2.10-2.00 (1H, m, $CH_{pyyrol}$, 1.81-1.74 (3H, m, $CH_{pyrrol}$ et $CH_2$), 1.05 (3H, t, $CH_3$).*

**Chlorhydrate du (R) 8-(3-amino-cyclopentanesulfonyl)-4-oxo-4,5-dihydro-3*H*-pyrroto[2,3-*c*]quinoline-1-carboxylate de propyle (167)**

Ce composé est préparé selon la synthèse 139, à partir de 72 mg (0.14 mmol) de (R) 8-(3-*tert*-butoxycarbonylamino-pyrrolidine-1-sulfonyl)-4-oxo-4,5-dihydro-3*H*-pyrrolo[2,3-*c*]quinoline-1-carboxylate de propyle et de 3.5 mL (7 mmol) d'une solution 2M d'acide chlorhydrique dans l'éther diéthylique pour donner après filtration, 34 mg (53%) de chlorhydrate de (R) 8-(3-amino-cyclopentanesulfonyl)-4-oxo-4,5-dihydro-3*H*-pyrrolo[2,3-*c*]quinoline-1-carboxylate de propyle sous la forme d'un solide blanc.
***LC/MS (IE, m/z) :*** *(M+1) 419.29*
***RMN¹H :*** *$\delta_H$ ppm 400 MHz, DMSO*
*13.25 (1H, sl, NH), 12.15 (1H, sl, NH), 9.84 (1H, s, $CH_{arom}$), 8.15 (2H, sl, NH.HCl), 8.09 (1H, s, $CH_{arom}$), 7.82 (1H, d, $CH_{arom}$), 7.62 (1H, d, $CH_{arom}$), 4.26 (2H, t, $CH_2$), 3.75-3.63 (1H, m, $CH_{pyyrol}$), 3.49-3.39 (1H, m, $CH_{pyyrol}$), 3.39-3.27 (1H, m, $CH_{pyyrol}$), 3.27-3.12 (2H, m, 2x$CH_{pyyrol}$), 2.10-2.00 (1H, m, $CH_{pyyrol}$), 1.81-1.74 (3H, m, $CH_{pyrrol}$ et $CH_2$), 1.05*

*(3H, t, $CH_3$).*

Le (R) 8-(3-*tert*-butoxycarbonylamino-pyrrolidine-1-sulfonyl)-4-oxo-4,5-dihydro-3*H*-pyrrolo[2,3-*c*]quinoline-1-carboxylate de propyle est préparé selon la synthèse 136, à partir de 360 mg (1.1 mmol) d'acide 8-chlorosulfonyl-4-oxo-4,5-dihydro-3*H*-pyrrolo[2,3-*c*]quinoline-1-carboxylique, de 767 μL (5.5 mmol) de triéthylamine et de 138 mg (1.21 mmol) de (R) *N*-*tert*-butoxycarbonyl-*N*'-pyrrolidin-3yl amine pour donner après estérification suivant la méthode générale C (di-*n*-propylsulfate comme dialkylsulfate utilisé) et purification par chromatographie sur silice (éluant cyclohexane/acétate d'éthyle 2/3 puis acétate d'éthyle/méthanol 98/2), 80 mg (14% sur deux étapes) de (R) 8-(3-*tert*-butoxycarbonylamino-pyrrolidine-1-sulfonyl)-4-oxo-4,5-dihydro-3*H*-pyrrolo[2,3-*c*]quinoline-1-carboxylate de propyle sous la forme d'un solide brun.

**Chlorhydrate du 8-(3-méthylamino-pyrrolidine-1-sulfonyl)-4-oxo-4,5-dihydro-3*H*-pyrrolo[2,3-*c*]quinoline-1-carboxylate de propyle (168)**

Ce composé est préparé selon la synthèse 139, à partir de 129 mg (0.25 mmol) de 8-[3-(*tert*-butoxycarbonyl-méthyl-amino)-pyrrolidine-1-sulfonyl]-4-oxo-4,5-dihydro-3*H*-pyrrolo[2,3-*c*]quinoline-1-carboxylate de propyle et de 6.3 mL (12.6 mmol) d'une solution 2M d'acide chlorhydrique dans l'éther diéthylique pour donner après filtration, 92 mg (78%) de chlorhydrate du 8-(3-méthylamino-pyrrolidine-1-sulfonyl)-4-oxo-4,5-dihydro-3*H*-pyrrolo[2,3-*c*]quinoline-1-carboxylate de propyle sous la forme d'un solide crème.

***LC/MS (IE, m/z) :*** *(M+1) 433.27*

***RMN¹H*** *: $\delta_H$ ppm 400 MHz, DMSO*

*13.25 (1H, sl, NH), 12.15 (1H, sl, NH), 9.82 (1H, s, $CH_{arom}$), 9.13 (2H, sl, NH.HCl), 8.07 (1H, s, $CH_{arom}$), 7.82 (1H, d, $CH_{arom}$), 7.62 (1H, d, $CH_{arom}$), 4.29-4.18 (2H, m, $CH_2$), 3.67-3.56 (1H, m, $CH_{pyrrol}$), 3.45-3.25 (3H, m, $3xCH_{pyrrol}$), 3.19-3.10 (1H, m, $CH_{pyrrol}$), 2.50 (3H, s, $NCH_3$), 2.11-2.02 (1H; m, $CH_{pyrrol}$), 1.97-1.87 (1H, m, $CH_{pyrrol}$), 1.81-1.67 (2H, m, $CH_2$), 1.04-0.95 (3H, t, $CH_3$).*

**8-[3-(*tert*-Butoxycarbonyl-methyl-amino)-pyrrolidine-1-suifonyl]-4-oxo-4,5-dihydro-3*H*-pyrrolo[2,3-*c*]quinoline-1-carboxylate de propyle (169)**

Ce composé a été préparé selon la méthode générale d'éstérification C à partir de 294 mg (0.6 mmol) d'acide 8-[3-(*tert*-butoxycarbonyl-méthyl-amino)-pyrrolidine-1-sulfonyl]-4-oxo-4,5-dihydro-3*H*-pyrrolo[2,3-*c*]quinoline-1-carboxylique, de 836 μL (6 mmol) de triéthylamine et de 492 μL (3 mmol) de di-n-propylsulfate pour conduire après purification par chromatographie sur silice (éluant dichlorométhane/méthanol 98/2), à 155 mg (50%) de 8-[3-(*tert*-butoxycarbonyl-méthyl-amino)-pyrrolidine-1-sulfonyl]-4-oxo-4,5-dihydro-3*H*-pyrrolo[2,3-*c*]quinoline-1-carboxylate de propyle sous la forme d'un solide brun.

***LC/MS (IE, m/z) :*** *(M+1) 533.41*

***RMN¹H :*** *$\delta_H$ ppm 400 MHz, $CDCl_3$*

*11.41 (1H, sl, NH), 10.71 (1H, sl, NH), 10.03 (1H, s, $CH_{arom}$), 8.02 (1H, s, $CH_{arom}$), 7.86 (1H, d, $CH_{arom}$), 7.47 (1H, d, $CH_{arom}$), 4.77-4.62 (1H, m, CH), 4.28 (2H, t, $CH_2$), 3.71-3.58 (1H, m, CH), 3.53-3.43 (1H, m, CH), 3.35-3.321 (2H, m, 2xCH), 2.74 (3H, s, $NCH_3$), 2.10-1.89 (2H, m, 2xCH), 1.85-1.74 (2H, m, $CH_2$), 1.40 (9H, s, $3xCH_3$), 1.02 (3H, t, $CH_3$).*

**Acide 8-[3-(*tert*-butoxycarbonyl-méthyl-amino)-pyrrolidine-1-sulfonyl]-4-oxo-4,5-dihydro-3*H*-pyrrolo[2,3-*c*] quinoline-1-carboxylique (170)**

Ce composé est préparé selon la synthèse 178, à partir de 326 mg (1 mmol) d'acide 8-chlorosulfonyl-4-oxo-4,5-dihydro-3*H*-pyrrolo[2,3-*c*]quinoline-1-carboxylique, 697 μL (5 mmol) de triéthylamine et de 220 mg (1.1 mmol) de *N*-*tert*-butoxycarbonyl-*N*-méthyl-*N*'-pyrrolidin-3yl-amine pour donner après purification par précipitation et trituration dans l'eau, 389 mg (79%) d'acide 8-[3-(*tert*-butoxycarbonyl-méthyl-amino)-pyrrolidine-1-sulfonyl]-4-oxo-4,5-dihydro-3*H*-pyrrolo[2,3-*c*]quinoline-1-carboxylique sous la forme d'un solide brun.

***LC/MS (IE, mlz)*** *: (M+1) 491.27*

***RMN¹H :*** *$\delta_H$ ppm 400 MHz, $CD_3OD$*

*10.15 (1H, s, $CH_{arom}$), 8.10 (1H, s, $CH_{arom}$), 7.88 (1H, d, $CH_{arom}$), 7.59 (1H, d, $CH_{arom}$), 4.55-4.35 (1H, m, CH), 3.63-3.39 (2H, m, $CH_2$), 2.70 (3H, s, $NCH_3$), 2.05-1.86 (3H, m, 3xCH), 1.34 (9H, s, $3xCH_3$).*

**(S) 8-(3-Diméthylamino-cyclopentanesuifonyl)-4-oxo-4,5-dihydro-3*H*-pyrrolo[2,3-*c*]quinoline-1-carboxylate de propyle (171)**

Ce composé est préparé selon la synthèse 136, à partir de 360 mg (1.1 mmol) d'acide 8-chlorosulfonyl-4-oxo-4,5-dihydro-3*H*-pyrrolo[2,3-*c*]quinoline-1-carboxylique, de 767 μL (5.5 mmol) de triéthylamine et de 138 mg (1.21 mmol)

de (S) *N*,*N*-diméthyl-*N*'-pyrrolidin-3yl-amine pour donner après estérification suivant la méthode générale C (di-*n*-propylsulfate commme dialkylsulfate utilisé) et purification par chromatographie sur silice (éluant dichlorométhane/méthanol/ammoniaque 200/16/2) puis trituration dans l'éther diéthylique, 68 mg (13% sur deux étapes) de (S) 8-(3-diméthylamino-cyclopentanesulfonyl)-4-oxo-4,5-dihydro-3*H*-pyrrolo[2,3-*c*]quinoline-1-carboxytate de propyle sous la forme d'un solide brun.

***LC/MS (IE, m/z) :*** *(M+1) 447.4*

***RMN$^1$H :*** *$\delta_H$ ppm 400 MHz, DMSO*

*13.26 (1H, sl, NH), 12.12 (1H, sl, NH), 9.84 (1H, s, $CH_{arom}$), 8.08 (1H, s, $CH_{arom}$), 7.83 (1H, d, $CH_{arom}$), 7.58 (1H, d, $CH_{arom}$), 4.24 (2H, t, $CH_2$), 3.54-3.45 (1H, m, $CH_{pyrrol}$), 3.19-3.08 (1H, m, $CH_{pyrrol}$), 2.98-2.86 (1H, m, $CH_{pyrrol}$), 2.01 (6H, s, 2x$CH_3$), 1.95-1.80 (1H, m, $CH_{pyrrol}$), 1.79-1.70 (2H, m, $CH_2$), 1.54-1.42 (1H, m, $CH_{pyrrol}$), 0.99 (3H, t, $CH_3$).*

**(R) 8-(3-Diméthylamino-cyclopentanesulfonyl)-4-oxo-4,5-dihydro-3*H*-pyrrolo[2,3-*c*]quinoline-1-carboxylate de propyle (172)**

Ce composé est préparé selon la synthèse 136, à partir de 360 mg (1.1 mmol) d'acide 8-chlorosulfonyl-4-oxo-4,5-dihydro-3*H*-pyrrolo[2,3-*c*]quinoline-1-carboxylique, de 767 µL (5.5 mmol) de triéthylamine et de 138 mg (1.21 mmol) de (R) *N*,*N*-diméthyl-*N*'-pynolidin-3yl-amine pour donner après estérification suivant la méthode générale C (di-*n*-propylsulfate comme dialkylsulfate utilisé) et purification par chromatographie sur silice (éluant dichlorométhane/méthanol/ammoniaque 200/16/2) puis trituration dans l'éther diéthylique, 119 mg (24% sur deux étapes) de (R) 8-(3-diméthylamino-cyclopentanesulfonyl)-4-oxo-4,5-dihydro-3*H*-pyrrolo[2,3-*c*]quinoline-1-carboxylate de propyle sous la forme d'un solide brun.

***LC/MS (IE, m/z)*** *: (M+1) 447.34*

***RMN$^1$H:*** *$\delta_H$ ppm 400 MHz, DMSO*

*13.26 (1H, sl, NH), 12.12 (1H, sl, NH), 9.84 (1H, s, $CH_{arom}$), 8.08 (1H, s, $CH_{arom}$), 7.83 (1H, d, $CH_{arom}$), 7.58 (1H, d, $CH_{arom}$), 4.24 (2H, t, $CH_2$), 3.54-3.45 (1H, m, $CH_{pyrrol}$), (1H, m, $CHp_{yrrol}$), 2.98-2.86 (1H, m, $CH_{pyrrol}$), 2.01 (6H, s, 2x$CH_3$), 1.95-1.80 (1H, m, $CH_{pyrrol}$), 1.79-1.70 (2H, m, $CH_2$), 1.54-1.42 (1H, m, $CH_{pyrrol}$), 0.99 (3H, t, $CH_3$).*

**8-[(2-Hydroxy-éthyl)-isopropyl-sulfamoyl]-4-oxo-4,5-dihydro-3*H*-pyrrolo[2,3-*c*]quinoline-1-carboxylate de propyle (173)**

Ce composé est préparé selon la synthèse 136, à partir de 457 mg (1.4 mmol) d'acide 8-chlorosulfonyl-4-oxo-4,5-dihydro-3*H*-pyrrolo[2,3-*c*]quinoline-1-carboxylique, de 585 µL (4.2 mmol) de triéthylamine et de 193 µL (1.68 mmol) de 2-isopropylamino-éthanol pour donner après estérification suivant la méthode générale C (di-*n*-propylsulfate comme dialkylsulfate utilisé) et purification par chromatographie sur silice (éluant dichlorométhane/méthanol 96/4), 25 mg (4% sur deux étapes) de 8-[(2-hydroxy-éthyl)-isopmpyl-sulfamoyl]-4-oxo-4,5-dihydro-3*H*-pyrrolo[2,3-*c*]quinoline-1-carboxylate de propyle sous la forme d'un solide blanc.

***LCMS (IE, mlz):*** *(M+1) 436.30*

***RMN$^1$H*** *: $\delta_H$ ppm 400 MHz, DMSO*

*13.17 (1H, sl, NH), 12.06 (1H, sl, NH), 9.82 (1H, d, $CH_{arom}$), 8.06 (1H, s, $CH_{arom}$), 7.80 (1H, dd, $CH_{arom}$), 7.54 (1H, d, $CH_{arom}$), 4.75 (1H, t, OH), 4.25 (2H, t, $CH_2$), 4.00 (1H, sept, CH), 3.55 (2H, q, $CH_2$), 3.13 (2H, t, $CH_2$), 1.76 (2H, sex, $CH_2$), 0.99 (3H, t, $CH_3$), 0.95 (6H, m, 2x$CH_3$).*

**8-[Cyclopropyl-(2-hydroxy-éthyl)-sulfamoyl]-4-oxo-4,5-dihydro-3*H*-pyrrolo[2,3-*c*]quinoline-1-carboxylate** de **propyle (174)**

Ce composé est préparé selon la synthèse 136, à partir de 490 mg (1.5 mmol) d'acide 8-chlorosulfonyl-4-oxo-4,5-dihydro-3*H*-pyrrolo[2,3-*c*]quinoline-1-carboxylique, de 627 µL (3 mmol) de triéthylamine et de 227 mg (2.25 mmol) de 2-cyclopropylamino-éthanol (préparé selon la méthode décrite par Morrow, D. F. et al., J. Med Chem., 1973, 16(6), 736-9) pour donner après estérification suivant la méthode générale C (di-*n*-propylsulfate comme dialkylsulfate utilisé) et purification par chromatographie sur silice (éluant dichlorométhane/méthanol 95/5), 116 mg (17% sur deux étapes) de 8-[cyclopropyl-(2-hydroxy-éthyl)-sulfamoyl]-4-oxo-4,5-dihydro-3*H*-pyrrolo[2,3-*c*]quinoline-1-carboxylate de propyle sous la forme d'un solide blanc.

***LCMS (IE, m/z):*** *(M+1) 434.22*

***RMN$^1$H:*** *$\delta_H$ ppm 400 MHz, DMSO*

*13.19 (1H, sl, NH), 12.08 (1H, sl, NH), 9.86 (1H, d, $CH_{arom}$), 8.06 (1H, s, $CH_{arom}$), 7.82 (1H, dd, $CH_{arom}$), 7.58 (1H, d, $CH_{arom}$), 4.71 (1H, t, OH), 4.25 (2H, t, $CH_2$), 3.53 (2H, q, $CH_2$), 3.18 (2H, t, $CH_2$), 2.10-2.03 (1H, m, $CH_{ali}$), 1.80-1.68 (2H, m, $CH_2$), 0.99 (3H, t, $CH_3$), 0.86-0.77 (2H, m, 2x$CH_{ali}$), 0.73-0.64 (2H, m, 2x$CH_{ali}$).*

**8-[(2-Hydroay-éthyl)-prop-2-ynyl-sulfamoyl]-4-oxo-4,5-dihydro-3*H*-pyrrolo[2,3-*c*]quinoline-1-carboxylate de propyle (175)**

Ce composé est préparé selon la synthèse 136, à partir de 490 mg (1.5 mmol) d'acide 8-chlorosulfonyl-4-oxo-4,5-dihydro-3*H*-pyrrolo[2,3-*c*]quinoline-1-carboxylique et de 223 mg (2.25 mmol) de 2-prop-2-ynylamino-éthanol (préparé selon la méthode décrite par Carretero, Juan Carlos; Adrio Javier, Synthesis, 2001, 12, 1888-96), pour donner après estérification suivant la méthode générale C (di-*n*-propylsulfate comme dialkylsulfate utilisé) et purification par chromatographie sur silice (éluant dichlorométhane/méthanol 95/5), 80 mg (10% sur deux étapes) de 8-[(2-hydroxy-éthyl)-prop-2-ynyl-sulfamoyl]-4-oxo-4,5-dihydro-3*H*-pyrrolo[2,3-*c*]quinoline-1-carboxylate de propyle sous la forme d'un solide blanc.
*LCMS **(IE, m/z):** (M+1) 32.20*
***RMN$^1$H :** $\delta_H$ ppm 400 MHz, DMSO*
*13.20 (1H, sl, NH), 12.06 (1H, sl, NH), 9.84 (1H, d, $CH_{arom}$), 8.06 (1H, s, $CH_{arom}$), 7.80 (1H, dd, $CH_{arom}$), 7.53 (1H, d, $CH_{arom}$), 4.82 (1H, t, OH), 4.25 (2H, t, $CH_2$), 4.19-4.15 (2H, m, $CH_2$), 3.56 (2H, q, $CH_2$), 3.23 (2H, t, $CH_2$), 3.00-2.96 (1H, m, CH), 1.76 (2H, m, $CH_2$), 1.00 (3H, t, $CH_3$).*

**8-[Bis(2-hydroxy-éthyl)-sulfamoyl]-4-oxo-4,5-dihydro-3*H*-pyrrolo[2,3-*c*]quinoline-1-carboxylate de propyle (176)**

Ce composé est préparé selon la synthèse 136, à partir de 490 mg (1.5 mmol) d'acide 8-chlorosulfonyl-4-oxo-4,5-dihydro-3*H*-pyrrolo[2,3-*c*]quinoline-1-carboxylique et de 236 mg (2.25 mmol) de 2-(2-hydroxy-éthylamino)-éthanol pour donner après estérification suivant la méthode générale C (di-*n*-propylsulfate comme dialkylsulfate utilisé) et purification par LC/MS préparative, 5 mg (1% sur deux étapes) de 8-[bis-(2-hydroxy-éthyl)-sulfamoyl]-4-oxo-4,5-dihydro-3*H*-pyrrolo[2,3-*c*]quinoline-1-carboxylate de propyle sous la forme d'un solide blanc.
***LCMS (IE, m/z):** (M+1) 438.25*
***RMN$^1$H:** $\delta_H$ ppm 400 MHz, DMSO*
*13.24 (1H, sl, NH), 12.09 (1H, sl, NH), 9.82 (1H, d, $CH_{arom}$), 8.07 (1H, s, $CH_{arom}$), 7.80 (1H, dd, $CH_{arom}$), 7.55 (1H, d, $CH_{arom}$), 4.83 (2H, t, 2xOH), 4.25 (2H, t, $CH_2$), 3.53 (4H, q, 2x$CH_2$), 3.19 (4H, t, $CH_2$), 1.75 (2H, m, $CH_2$), 0.99 (3H, t, $CH_3$).*

**8-(Carbamoylméthyl-methyl-sulfamoyl)-4-oxo-4,5-dihydro-3*H*-pyrrolo[2,3-*c*]quinoline-1-carboxylate de propyle (177)**

Ce composé a été préparé selon la méthode générale d'estérification C à partir de 265 mg (0.7 mmol) d'acide 8-(carbamoylméthyl-méthyl-sulfamoyl)-4-oxo-4,5-dihydro-3*H*-pyrrolo[2,3-*c*]quinoline-1-carboxylique, de 1.4 mL (1.93 mmol) de triéthylamine et de 690 µL (4.2 mmol) de di-*n*-propylsulfate pour conduire après purification par LC préparative à 35 mg (11%) de 8-(carbainoylméthyl-méthyl-sulfarnoyl)-4-oxo-4,5-dihydro-3*H*-pyrrolo[2,3-*c*]quinoline-1-carboxylate de propyle sous la forme d'un solide blanc
***LCMs (IE, m/z) :** (M+1) 421.29*
***RMN$^1$H :** $\delta_H$ ppm 400 MHz, DMSO*
*13.21 (1H, sl, NH), 12.08 (1H, sl, NH), 9.80 (1H, d, $CH_{arom}$), 8.07 (1H, s, $CH_{arom}$), 7.78 (1H, dd, $CH_{arom}$), 7.57 (1H, d, $CH_{arom}$), 7.38 (1H, s. NH), 7.17 (1H, s, NH), 4.25 (2H, t, $CH_2$), 3.58 (2H, s, $CH_2$), 2.76 (3H, s, $CH_3$), 1.75 (2H, sext, $CH_2$), 0.99 (3H, t, $CH_4$).*

Acide **8-(carbamoylméthyl-méthyl-sulfamoyl)-oxo-4,5-dihydro-3*H*-pyrrolo[2,3-*c*]quinoline-1-carboxylique (178)**

A 456 mg (1.4 mmol) d'acide 8-chlorosulfonyl-4-oxo-4,5-dihydro-3*H*-pyrrolo[2,3-*c*]quinoline-1-carboxylique en suspension dans 4 mL de dichlorométhane anhydre et 1 mL de diméthylformamide anhydre, sont ajoutés 584 µL (3 mmol) de triéthylamine et 208 mg (1.68 mmol) de 2 méthylamino-acétamide. Le milieu réactionnel est agité 3 jours à température ambiante. Les solvants sont évaporés puis le résidu est repris dans du méthanol. Le solide est filtré, rincé avec du méthanol puis de l'éther diisopropylique et séché sous vide pour donner 279 mg (53%) d'acide 8-(carbamoylméthyl-méthyl-sulfamoyl)-4-oxo-4,5-dihydro-3*H*-pyrrolo[2,3-*c*]quinoline-1-carboxylique sous la forme d'un solide blanc.
***LC/MS (IE, m/z):** (M+1) 379.01*
***RMN$^1$H :** $\delta_H$ ppm 400 MHz, DMSO*
*13.10 (1H, sl, NH), 12.56 (1H, sl, $CO_2H$), 12.04 (1H, s, NH), 9.92 (1H, s, $CH_{arom}$), 8.01 (1H, d, $CH_{arom}$), (1H, dd, $CH_{arom}$), 7.55 (1H, d, $CH_{arom}$), 7.38 (1H, sl, NH), 7.18 (1H, sl, NH), 3.58 (2H, s, $CH_2$), 2.77 (3H, s, $CH_3$).*

**8-(3-Carbamoyl-pipéridine-1-sulfonyl)-oxo-4,5-dihydro-3H-pyrrolo[2,3-*c*]quinoline-1-carboxylate de propyle (179)**

Ce composé a été préparé selon la méthode générale d'éstérification C à partir de 251 mg (0.6 mmol) d'acide 8-(3-carbamoyl-pipéridine-1-sulfonyl)-4-oxo-4,5-dihydro-3*H*-pyrrolo[2,3-*c*]quinoline-1-carboxylique, de 1.67 mL (12 mmol) de triéthylamine et de 394 μL (2.4 mmol) de di-n-propylsulfate pour conduire après purification par LC préparative à 13 mg (5%) de 8-(3-carbamoyl-pipéridine-1-sulfonyl)-4-oxo-4,5-dihydro-3*H*-pyrrolo[2,3-*c*]quinoline-1-carboxylate de propyle sous la forme d'un solide blanc

***LC/MS (IE, m/z) :*** *(M+1) 461.23*

***RMN$^1$H :*** *$\delta_H$ ppm 400 MHz, DMSO*

*13.20 (1H, sl, NH), 12.08 (1H, s, NH), 9.77 (1H, s, $CH_{arom}$), 8.06 (1H, s, $CH_{arom}$) 7.73 (1H, d, $CH_{arom}$), 7.59 (1H, d, $CH_{arom}$), 7.38 (1H, sl0, $NH_2$), 6.88 (1H, sl, $NH_2$), 4.24 (2H, t, $CH_2$), 3.69-3.58 (2H, m, $2xCH_{pipér}$), 2.42-2.32 (1H, m, $CH_{pipér}$), 2.23 (2H, dd, $2XCH_{pipér}$), 1.80-1.68 (4H m, $2xCH_{pipér}$ et $CH_2$), 1.53-1.40 (1H, m, $CH_{pipér}$), 1.27-1.16 (1H, m, $CH_{pipér}$), 0.99 (3H, t, $CH_3$).*

**Acide 8-(3-carbamoyl-pipéridine-1-sulfonyl)-4-oxo-4,5-dihydro-3*H*-pyrrolo[2,3-*c*]quinolinc-1-carboxylique (180)**

Ce composé est préparé selon la synthèse 178, à partir de 360 mg (1.1 mmol) d'acide 8-chlorosulfonyl-4-oxo-4,5-dihydro-3*H*-pyrrolo[2,3-*c*]quinoline-1-carboxylique et de 154 mg (1.2 mmol) de pipéridine-3-carboxamide pour donner après purification par précipitation et trituration dans le méthanol, 275 mg (66%) d'acide 8-(3-carbamoyl-pipéridine-1-sulfonyl)-4-oxo-4,5-dihydro-3*H*-pyrrolo[2,3-*c*]quinoline-1-carboxylique sous la forme d'un solide crème.

***LC/MS (IE, m/z) : (M+1)*** *419.4*

***RMN$^1$H :*** *$\delta_H$ ppm 400 MHz, DMSO*

*13.07 (1H, sl, NH), 12.48 (1H, sl, $CO_2H$), 12.05 (1H, s, NH), 9.90 (1H, s, $CH_{arom}$), 8.01 (1H, s, $CH_{arom}$), 7.73 (1H, dd, $CH_{arom}$), 7.57 (1H, d, $CH_{arom}$), 7.39 (1H, sl, NH), 6.87 (1H, sl, NH), 3.69-3.58 (2H, m, $2xCH_{pipér}$), 2.42-2.32 (1H, m, $CH_{pipér}$), 2.30-2.16 (2H, m, $2xCH_{pipér}$), 1.80-1,65 (2H, m, $2xCH_{pipér}$), 1.53-1.40 (1H, m, $CH_{pipér}$), 1.29-1.16 (1H, m, $CH_{pipér}$).*

**8-[(2-Benzyloxy-éthyl)-cyclopropyl-sulfamoyl]-4-oxo-4,5-dihydro-3*H*-pyrrolo[2,3-*c*]quinoline-1-carboxylate de propyle (181)**

Ce composé est préparé selon la synthèse 111, à partir de 398 mg (1.22 mmol) d'acide 8-chlorosulfonyl-4-oxo-4,5-dihydro-3*H*-pyrrolo[2,3-*c*]quinoline-1-carboxylique et de 333 mg (1.46 mmol) de (2-benzyloxy-éthyl)-cyclopropyl-amine pour donner après estérification suivant la méthode générale C (di-*n*-propylsulfate comme dialkylsulfate utilisé) et purification par chromatographie sur silice (éluant dichlorométhane/méchanol 99/1), 193 mg (30% sur deux étapes) de 8-[(2-benzyloxy-éthyl)-cyclopropyl-sulfamoyl]-4-oxo-4,5-dihydro-3*H*-pyrrolo[2,3-*c*]quinoline-1-carboxylate de propyle sous la forme d'un solide blanc.

***LC/MS (IE, m/z) :*** *(M+1) 524.45*

***RMN$^1$H :*** *$\delta_H$ ppm 400 MHz, DMSO*

*13.21 (1H, sl, NH), 12.07 (1H, sl, NH), 9.87 (1H, d, $CH_{arom}$), 8.05 (1H, s, $CH_{arom}$), 7.81 (1H, dd, $CH_{arom}$), 7.55 (1H, d, $CH_{arom}$), 7.32-7.18 (5H, m, $5xCH_{arom}$), 4.40 (2H, s, $CH_2$), 4.22 (2H, q, $CH_2$), 3.59 (2H, t, $CH_2$), 3.36 (2H, t, $CH_2$), 2.15-2.07 (1H, m, $CH_{cycloprop}$), 1.76-1.69 (2H, m, $CH_2$), 1.03 (3H, t, $CH_3$), 0.83-0.77 (2H, m, $2CH_{cycloprop}$), 0.72-0.67 (2H, m, $2xCH_{cycloprop}$).*

A une solution de 2.1 mL (30 mmol) de cyclopropylamine dans 10 mL de méthanol anhydre sont additionnés 3.8 mL (13 mmol) d'isopropoxyde de titanium et 1.5 g (10 mmol) de benzyloxy-acetaldéhyde. La solution est agitée 5 heures à température ambiante puis 378 mg (10 mmol) de borohydrure de sodium sont additionnés. L'agitation est maintenue pour 2 heures supplémentaires puis 2 mL d'eau sont additionnés. Le mélange réactionnel est filtré puis les solvants sont évaporés. Le résidu est repris dans de l'éther diéthylique puis une solution 2M d'acide chlorhydrique dans l'éther diéhylique est additionné. Le solide est filtré et lavé avec de l'éther diéthylique pour conduire à 1.94 g (85%) de chlorhydrate de (2-benzyloxy-éthyl)-cyclopropylamine sous la forme d'un solide brun.

**8-(Carboxyméthyl-méthyl-sulfamoyl)-4-oxo-4,5-dihydro-3*H*-pyrrolo[2,3-*c*]quinoline-1-carboxylate de propyle (182)**

A une solution de 95 mg (0.2 mmol) de 8-(*tert*-butoxycarbonylméthyl-méthyl-sulfamoyl)-4-oxo-4,5-dihydro-3*H*-pyrrolo[2,3-*c*]quinoline-1-carboxylate de propyle dissous dans 9 mL de dichloroéthane est additionné lentement de l'acide trifluoroacétique (1 mL). La solution est agitée à température ambiante pour 5 heures puis les solvants sont

évaporés. Le résidu est repris et trituré dans l'acétonitrile puis dans l'éther diisopropylique pour conduire à 76 mg (90%) de 8-(carboxyméthyl-méthyl-sulfamoyl)-4-oxo-4,5-dihydro-3*H*-pyrrolo[2,3-*c*]quinoline-1-carboxylate de propyle sous la forme d'un solide blanc

***LCMS (IE, m/z):*** *(M+1) 422*

***RMN¹H :*** *$\delta_H$ ppm 400 MHz, DMSO*

*13.21 (1H, s, NH), 12.61 (1H, s, $CO_2H$), 12.06 (1H, s, NH), 9.80 (1H, d, $CH_{arom}$), 8.07 (1H, d, $CH_{arom}$), 7.77 (1H, dd, $CH_{arom}$), 7.55 (1H, d, $CH_{arom}$), 4.25 (2H, t, $CH_2$), 3.87 (2H, s, $NCH_2$), 2.80 (3H, s, $NCH_3$), 1.75 (2H, m, $CH_2$), 0.99 (3H, t, $CH_3$).*

**8-(tert-Butoxycarbonylméthyl-méthyl-sulfamoyl)4-oxo-4,5-dihydro-3H-pyrrolo[2,3-c]quinoline-1-carboxylate de propyle (183)**

Ce composé est préparé selon la synthèse 111, à partir de 326 mg (1 mmol) d'acide 8-chlorosulfonyl-4-oxo-4,5-dihydro-3*H*-pyrrolo[2,3-*c*]quinoline-1-carboxylique et de 272 mg (1.46 mmol) de chlorhydrate de l'ester *tert*-butylique de la sarcosine pour donner après estérification suivant la méthode générale C (di-*n*-propylsulfate comme dialkylsulfate utilisé) et purification par chromatographie sur silice (éluant acétate d'éthyle/méthanol 98/2 puis 95/5), 104 mg (21% sur deux étapes) de 8-(*tert*-butoxycarbonylméthyl-méthyl-sulfamoyl)-4-oxo-4,5-dihydro-3*H*-pyrrolo[2,3-c]quinoline-1-carboxylate de propyle sous la forme d'un solide blanc.

***LCMS (IE, m/z):*** *(M+1) 478*

***RMN¹H :*** *$\delta_H$ ppm 400 MHz, DMSO*

*13.21 (1H, s, NH), 12.06 (1H, s, NH), 9.79 (1H, d, $CH_{arom}$), 8.06 (1H, d, $CH_{arom}$), 7.77 (1H, dd, $CH_{arom}$), 7.54 (1H, d, $CH_{arom}$), 4.25 (2H, t, $CH_2$), 3.90 (2H, s, $NCH_2$), 2.83 (3H, s, $NCH_3$), 1.80-1.71 (2H, m, $CH_2$), 1.27 (9H, s, $3xCH_3$), 0.99 (3H, t, $CH_3$).*

**8-[(2-Diméthylamino-éthyl)-méthyl-sulfamoyl]-7-fluoro-4-oxo-4,5-dihydro-3*H*-pyrrolo[2,3-*c*]quinoline-1-carboxylate de propyle (184)**

Ce composé est préparé selon la synthèse 136, à partir de 345 mg (1 mmol) d'acide 7-fluoro-8-chlorosulfonyl-4-oxo-4,5-dihydro-3*H*-pyrrolo[2,3-*c*]quinoline-1-carboxylique, de 1 mL (10 mmol) de triéthylamine et de 153 mg (1.5 mmol) de *N,N,N'*-triméthyl-éthane-1,2-diamine pour donner après estérification suivant la méthode générale C (di-*n*-propylsulfate comme dialkylsulfate utilisé) et purification par chromatographie sur silice. (éluant dichlorométhane/méthanol/ammoniaque 400/16/3 puis 200/16/13) puis trituration dans l'éther diéthylique, 30 mg (7% sur deux étapes) de 8-[(2-diméthylamino-éthyl)-méthyl-sulfamoyl]-7-fluoro-4-oxo-4,5-dihydro-3*H*-pyrrolo[2,3-*c*]quinoline-1-carboxylate de propyle sous la forme d'un solide blanc.

***LCMS (IE, m/z):*** *(M+1) 453.17*

***RMN¹H :*** *$\delta_H$ ppm 400 MHz, DMSO*

*13.30 (1H, sl, NH), 12.17 (1H, sl, NH), 9.88 (1H, s, $CH_{arom}$), 8.09 (1H, s, $CH_{arom}$), 7.35 (1H, d, $CH_{arom}$), 4.24 (2H, t, $OCH_2$), 3.81-3.62 (2H, m, $NCH_2$), 2.83 (3H, s, $MCH_4$), 1.76 (2H, d, $CH_2$), 1.00-0.86 (3H, m, $CH_3$).*

Dans un tube scellé, 601 mg (1.8 mmol) de 7-fluoro-4-oxo-4,5-dihydro-3*H*-pyrrolo[2,3-*c*]quinoline-1-carboxylate d'éthyle cocristallisé avec un équivalent d'acide acétique est dissous dans 2.4 mL (36 mmol) d'acide chlorosulfonique. Le mélange réactionnel est placé dans un bain d'huile préalablement porté à 85°C pour 2 heures, refroidi par un bain de glace puis versé lentement sur de la glace pilée. Le solide est filtré, rincé avec de l'eau, de l'éther diisopropylique et du pentane puis séché sous vide pour conduire à 580 mg (93%) d'acide 8-chlorosulfonyl-7-fluoro-4-oxo-4,5-dihydro-3*H*-pyrrolo[2,3-*c*]quinoline-1-carboxylique sous la forme d'un solide marron.

**7-Fluoro-8-[(2-hydroxy-éthyl)-isopropyl-sulfamoyl]-4-oxo-4,5-dihydro-3*H*-pyrrolo[2,3-*c*]quinoline-1-carboxylate de propyle (185)**

Ce composé est préparé selon la synthèse 136, à partir de 172 mg (0.5 mmol) d'acide 7-fluoro-8-chlorosulfonyl-4-oxo-4,5-dihydro-3*H*-pyrrolo[2,3-*c*]quinoline-1-carboxylique, de 0.5 mL (10 mmol) de triéthylamine et de 77 mg (0.75 mol) de 2-isopropylamino-éthanol pour donner après estérification suivant la méthode générale C (di-*n*-propylsulfate comme dialkylsulfate utilisé) et purification par chromatographie sur silice (éluant dichlorométhane/méthanol 97/3) puis trituration dans l'éther diéthylique, 30 mg (13% sur deux étapes) de 7-fluoro-8-[(2-hydroxy-éthyl)-isopropyl-sulfamoyl]-4-oxo-4,5-dihydro-3*H*-pyrrolo[2,3-*c*]quinolin-1-carboxylate de propyle sous la forme d'un solide blanc.

***LCMS (IE, m/z):*** *(M+1) 454.26*

***RMN¹H :*** *$\delta_H$ ppm 400 MHz, DMSO*

*13.22 (1H, sl, NH), 12.10 (1H, sl, NH), 9.89 (1H, d, $CH_{arom}$), 8.06 (1H, s, $CH_{arom}$), 7.32 (1H, d, $CH_{arom}$), 4.78 (1H, sl, OH), 4.26 (2H, t, $OCH_2$), 4.01-3.90 (1H, m, CH), 3.52 (2H, dd, $CH_2$), 3.25 (2H, dd, $ACH_2$), 1.82-1.74 (2H, m, $CH_2$),*

*1.05-0.95 (9H, m, 3x$CH_3$).*

**8-Diméthylsulfamoyl-7-fluoro4-oxo-4,5-dibydro-3*H*-pyrrolo[2,3-*c*]quinoline-1-carboxylate de propyle (186)**

Ce composé est préparé selon la synthèse 111, à partir de 172 mg (0.5 mmol) d'acide 7-fluoro-8-chlorosulfonyl-4-oxo-4,5-dihydro-3*H*-pyrrolo[2,3-*c*]quinoline-1-carboxylique, 697 μL (5 mmol) de triéthylamine et de 61 mg (0.75 mmol) de chlorhydrate de diméthylamine pour donner après estérification suivant la méthode générale C (di-*n*-propylsulfate comme dialkylsulfate utilisé) et purification par chromatographie sur silice (éluant dichlorométhane/méthanol 97/3), 59 mg (30% sur deux étapes) de 8-diméthylsulfamoyl-7-fluoro-4-oxo-4,5-dihydro-3*H*-pyrrolo[2,3-*c*]quinoline-1-carboxylate de propyle sous la forme d'un solide blanc.
***LCMS (IE, m/z):*** *(M+1) 396.06*
***RMN$^1$H :*** *$\delta_H$ ppm 400 MHz, DMSO*
*13.23 (1H, sl, NH), 12.11 (1H, sl, NH), 9.83 (1H, d, $CH_{arom}$), 8.06 (1H, s, $CH_{arom}$), 7.33 (1H, d, $CH_{arom}$), 4.24 (2H, t, $OCH_2$), 2.75 (6H, s, 2x$CH_3$), 1.74 (2H, dd, $CH_2$), 0.9 (3H, t, $CH_3$).*

**8-(Cyclopropyl-méthyl-sulfamoyl)-7-fluoro-4-oxo-4,5-dihydro-3*H*-pyrrolo[2,3-*c*]quinolino-1-carboxylate de propyle (187)**

Ce composé est préparé selon la synthèse 111, à partir de 172 mg (0.5 mmol) d'acide 7-fluoro-8-chlorosulfonyl-4-oxo-4,5-dihydro-3*H*-pyrrolo[2,3-*c*]quinoline-1-carboxylique, 697 μL (5 mmol) de triéthylamine et de 80 mg (0.75 mmol) de chlorhydrate de cyclopropyl-méthyl-amine pour donner après estérification suivant la méthode générale C (di-*n*-propylsulfate comme dialkylsulfate utilisé) et purification par chromatographie sur silice (éluant dichlorométhane/méthanol 97/3), 90 mg (43% sur deux étapes) de 8-(cyclopropyl-méthyl-sulfamoyl)-7-fluoro-4-oxo-4,5-dihydro-3*H*-pyrrolo[2,3-*c*]quinoline-1-carboxylate de propyle sous la forme d'un solide blanc.
***LCMS (IE, m/z):*** *(M+1) 422.18*
***RMN$^1$H :*** *$\delta_H$ ppm 400 MHz, DMSO*
*13.22 (1H, sl, NH), 12.12 (1H, sl, NH), 9.88 (1H, d, $CH_{arom}$), 8.06 (1H, s, $CH_{arom}$), 7.32 (1H, d, $CH_{arom}$), 4.25 (2H, t, $OCH_2$), 2.79 (3H, s, $NCH_3$), 2.11-2.05 (1H, m, $CH_{cycloprop}$), 1.76 (2H, dd, $CH_2$), 0.90 (3H, t, $CH_3$), 0.76-0.66 (4H, m, 4x$CH_{cycloprop}$).*

**7-Chloro-8-(cyclopropyl-méthyl-sulfamoyl)-4-oxo-4,5-dihydro-3*H*-pyrrolo[2,3-*c*]quinoline-1-carboxylate de propyle (188)**

Ce composé est préparé selon la synthèse 111, à partir de 86 mg du mélange 2/1 d'acide 7-chloro-8-chlorosulfonyl-4-oxo-4,5-dihydro-3H-pyrrolo[2,3-*c*]quinoline-1-carboxylique et d'acide 7-chloro-6-chlorosulfonyl-4-oxo-4,5-dihydro-3*H*-pyrrolo[2,3-*c*]quinoline-1-carboxylique, 77 μL (0.55 mmol) de triéthylamine et de 30 mg (0.28 mmol) de chlorhydrate de cyclopropyl-méthyl-amine pour donner après estérification suivant la méthode générale C (di-*n*-propylsulfate comme dialkylsulfate utilisé) et purification par LC préparative, 4 mg (2% sur trois étapes) de 7-chloro-8-(cyclopropyl-méthyl-sulfamoyl)-4-oxo-4,5-dihydro-3*H*-pyrrolo[2,3-*c*]quinoline-1-carboxylate de propyle sous la forme d'un solide blanc.
***LCMS (IE, m/z):*** *(M+1) 434.28*
***RMN$^1$H :*** *$\delta_H$ ppm 400 MHz, $CDCl_3$*
*10.30 (1H, sl, NH), 10.04 (1H, s, $CH_{arom}$), 9.35 (1H, sl, NH), 8.01 (1H, d, $CH_{arom}$), 6.78 (1H, s, $CH_{arom}$), 4.36 (2H, t, $CH_2$), 4.01 (3H, s, $CH_3$), 2.99 (3H, s, $CH_3$), 2.35-2.27 (1H, m, $CH_{ali}$), 1.82 (2H, sext, $CH_2$), 1.03 (3H, t, $CH_3$), 0.93-0.81 (2H, m, 2x$CH_{ali}$), 0.71-0.63 (2H, m, 2x$CH_{ali}$).*
Dans un tube scellé, 100 mg (0.35 mmol) de 7-chloro-4-oxo-4,5-dihydro-3*H*-pyrrolo[2,3-*c*]quinoline-1-carboxylate d'éthyle cocristallisé avec un équivalent d'acide acétique est dissous dans 465 μL (7 mmol) d'acide chlorosulfonique. Le mélange réactionnel est placé dans un bain d'huile préalablement porté à 50°C pour 2 heures, refroidi par un bain de glace puis versé lentement sur de la glace pilée. Le solide est filtré, rincé avec de l'eau, de l'éther diisopropylique et du pentane puis séché sous vide pour conduire à 86 mg d'un mélange 2/1 d'acide 7-chloro-8-chlorosulfonyl-4-oxo-4,5-dihydro-3*H*-pyrrolo[2,3-*c*]quinoline-1-carboxylique et d'acide 7-chloro-6-chlorosulfonyl-4-oxo4,5-dihydro-3*H*-pyrrolo [2,3-*c*]quinoline-1-carboxylique sous la forme d'un solide marron.

**7-Chloro-8-diméthylsulfamoyl-4-oxo-4,5-dihydro-3*H*-pyrrolo[2,3-*c*]quinoline-1-carboxylate de propyle (189)**

Ce composé est préparé selon la synthèse 111, à partir de 542 mg (1.5 mmol) d'acide 7-chloro-8-chlorosulfonyl-4-oxo-4,5-dihydro-3*H*-pyrrolo[2,3-*c*]quinoline-1-carboxylique, 627 μL (4.5 mmol) de triéthylamine et de 146 mg (1.8 mmol) de chlorhydrate de diméthylamine pour donner après estérification suivant la méthode générale C (di-*n*-propylsulfate comme dialkylsulfate utilisé) et purification par précipitation dans l'eau puis trituration dans le méthanol et l'éther diisopropylique, 83 mg (13% sur deux étapes) de 7-chloro-8-diméthylsulfamoyl-4-oxo-4,5-dihydro-3*H*-pyrrolo[2,3-*c*]quinoline-1-carboxytate de propyle sous la forme d'un solide brun.

***LC/MS (IE, m/z):*** *(M+1) 412.01*

***RMN$^1$H :*** *$\delta_H$ ppm 400 MHz, DMSO*

*13.27 (1H, sl, MH), 12.10 (1H, sl, NH), 9.93 (1H, s, $CH_{arom}$), 8.09 (1H, s, $CH_{arom}$), 7.61 (1H, s, $CH_{arom}$), 4.24 (2h, q, $CH_2$), 2.87 (6H s, 2x$CH_3$), 1.85-1.64 (2H, m, $CH_2$), 0.99 (3H, t, $CH_3$).*

Dans un tube scellé, 1.16 g (4 mmol) de 7-chloro-4-oxo-4,5-dihydro-3*H*-pyrroto[2,3-*c*]quinoline-1-carboxylate d'éthyle est dissous dans 5.3 mL (80 mmol) d'acide chlorosulfonique. Le mélange réactionnel est placé dans un bain d'huile préalablement porté à 90°C pour 6 heures, refroidi par un bain de glace puis versé lentement sur de la glace pilée. Le solide est filtré, rincé avec de l'eau, de l'éther diisopropylique et du pentane puis séché sous vide pour conduire à 1.3 g (90%) d'acide 7-chloro-8-chlorosulfonyl-4-oxo-4,5-dihydro-3*H*-pyrrolo[2,3-*c*]quinoline-1-carboxylique sous la forme d'un solide marron.

**Chlorhydrate de (S) 8-(3-amino-cyclopentanesulfonyl)-7-chloro-4-oxo-4,5-dihydro-3*H*-pyrrolo[2,3-*c*]quinoline-1-carboxylate de propyle (190)**

Ce composé est préparé selon la synthèse 139, à partir de 136 mg (0.24 mmol) de (S) 8-(3-*tert*-butoxycarbonylamino-cyclopentanesulfonyl)-7-chloro-4-oxo-4,5-dihydro-3*H*-pyrrolo[2,3-*c*]quinoline-1-carboxylate de propyle et de 9.8 mL (19.6 mmol) d'une solution 2M d'acide chlorhydrique dans l'éther diéthylique pour donner après filtration, 80 mg (66%) de chlorhydrate de (S) 8-(3-amino-cyclopentanesulfonyl)-7-chloro-4-oxo-4,5-dihydro-3*H*-pyrroto[2,3-*c*]quinoline-1-carboxylate de propyle sous la forme d'un solide blanc.

***LCMS (IE, m/z):*** *(M+1) 453*

***RMN$^1$H :*** *$\delta_H$ ppm 400 MHz, DMSO*

*13.34 (1H, sl, NH), 12.17 (1H, sl, NH), 9.99 (1H, s, $CH_{arom}$), 8.34-8.20 (3H, m, $NH_2$.HCl), 8.10 (1H, d, $CH_{arom}$), 7.65 (1H, s, $CH_{arom}$), 4.24 (2H, t, $CH_2$), 3.89-3.80 (1H, m, $CH_{ali}$), 3.68-3.53 (2H, m, 2x$CH_{ali}$), 3.44-3.33 (2H, m, 2x$CH_{ali}$), 2.32-2.20 (1H, m, $CH_{ali}$), 2.01-1.90 (1H, m, $CH_{ali}$), 1.75 (2H, sext, $CH_2$), 0.99 (3H, t, $CH_3$).*

Le (S) 8-(3-*tert*-butoxycarbonylamino-cyclopentanesulfonyl)-7-chloro-4-oxo-4,5-dihydro-3*H*-pyrrolo[2,3-*c*]quinoline-1-carboxylate de propyle est préparé selon la synthèse 111, à partir de 505 mg (1.4 mmol) d'acide 8-chlorosulfonyl-4-oxo-4,5-dihydro-3*H*-pyrrolo[2,3-*c*]quinoline-1-carboxylique, 582 µL (4.2 mmol) de triéthylamine et de 391 mg (2.1 mmol) de (S) *N-tert*-butoxycarbonyl-*N*'-pyrrolidin-3yl-amine pour donner après estérification suivant la méthode générale C (di-*n*-propylsulfate comme dialkylsulfate utilisé) et purification par chromatographie sur silice (éluant dichlorométhane/acétate d'éthyle 6/4), 136 mg (10% sur deux étapes) de (S) 8-(3-*tert*-butoxycarbonylamino-cyclopentanesulfonyl)-7-chloro-4-oxo-4,5-dihydro-3*H*-pyrrolo[2,3-*c*]quinoline-1-mboxylate de propyle sous la forme d'un solide blanc.

**4-Oxo-8-(pipéridin-1-ylsulfamoyl)-4,5-dihydro-3*H*-pyrrolo[2,3-*c*]quinoline-1-carboxylate de méthyle (191)**

Ce composé est préparé selon la synthèse 111, à partir de 490 mg (1.5 mmol) d'acide 8-chlorosulfonyl-4-oxo-4,5-dihydro-3*H*-pyrrolo[2,3-*c*]quinoline-1-carboxylique et de 178 µL (1.65 mmol) de 1-aminopipéridine pour donner après estérification suivant la méthode générale B et purification par trituration dans l'eau, 125 mg (20% sur deux étapes) de 4-oxo-8-(pipéridin-1-ylsulfamoyl)-4,5-dihydro-3*H*-pyrrolo[2,3-*c*]quinoline-1-carboxylate de méthyle sous la forme d'un solide marron clair.

***LC/MS (IE, m/z):*** *(M+1) 404.99*

***RMN$^1$H:*** *$\delta_H$ ppm 400 MHz, DMSO*

*13.19 (1H, sl, MH), 12.06 (1H, sl, NH), 9.90 (1H, s, $CH_{arom}$), 8.57 (1H, s, $CH_{arom}$), 8.07 (1H, s, $CH_{arom}$), 7.84 (1H, dd, $CH_{arom}$), 7.53 (1H, d, $CH_{arom}$), 3.87 (3H, s, $CH_3$), 2.46-2.40 (4H, m, 4x$CH_{pipér}$), 1.39-1.31 (4H, m, 2x$CH_{pipér}$), 1.20-1.15 (2H, m, $CH_{pipér}$).*

**8-(Hexahydro-cyclopenta[*c*]pyrrol-2-ylsulfamoyl)-4-oxo-4,5-dihydro-3*H*-pyrrolo[2,3-*c*]quinoline-1-carboxylate de méthyle (192)**

Ce composé est préparé selon la synthèse 111, à partir de 490 mg (1.5 mmol) d'acide 8-chlorosulfonyl-4-oxo-4,5-dihydro-3*H*-pyrrolo[2,3-*c*]quinoline-1-carboxylique, de 627 µL (4.5 mmol) de triéthylamine et de 269 mg (1.65 mmol) de chlorhydrate de 2-amino-hexahydro-cyclopenta[*c*]pyrrole pour donner après estérification suivant la méthode générale B et purification par trituration dans l'eau, 67 mg (10% sur deux étapes) de 8-(hexahydro-cyclopenta[*c*]pyrrol-2-ylsulfamoyl)-4-oxo-4,5-dihydro-3*H*-pyrrolo[2,3-*c*]quinoline-1-carboxylate de méthyle sous la forme d'un solide marron clair.

***LC/MS (IE, m/z):*** *(M+1) 431.04*

***RMN$^1$H :*** *$\delta_H$ ppm 400 MHz, DMSO*
*13.17 (1H, sl, MH), 12.06 (1H, sl, NH), 9.87 (1H, d, $CH_{arom}$), 8.42 (1H, s, $CH_{arom}$), 8.07 (1H, s, $CH_{arom}$), 7.80 (1H, dd, $CH_{arom}$), 7.53 (1H, d, $CH_{arom}$), 3.86 (3H, s, $CH_3$), 2.63-2.53 (2H, m, $2xCH_{ali}$), 2.28-2.17 (4H, m, $4xCH_{ali}$), 1.53-1.42 (3H, m, $3xCH_{ali}$), 1.28-1.19 (1H, m, $CH_{ali}$), 1.16-1.05 (2H, m, $2xCH_{ali}$).*

**8-(4-Méthyl-pipérazin-1-ylsulfamoyl)-4-oxo-4,5-dihydro-3*H*-pyrrolo[2,3-*c*]quinoline-1-carboxylate de méthyle (193)**

Ce composé est préparé selon la synthèse 111, à partir de 490 mg (1.5 mmol) d'acide 8-chlorosulfonyl-4-oxo-4,5-dihydro-3*H*-pyrrolo[2,3-*c*]quinoline-1-carboxylique et de 199 μL (1.65 mmol) de 1-amino-4-méthylpipérazine pour donner après estérification suivant la méthode générale B et purification par chromatographie sur silice (éluant dichlorométhane/méthanol 95/5), 33 mg (5% sur deux étapes) de 8-(4-méthyl-pipérazin-1-ylsulfamoyl)-4-oxo-4,5-dihydro-3*H*-pyrrolo[2,3-*c*]quinoline-1-carboxylate de méthyle sous la forme d'un solide blanc.
***LC/MS (IE, m/z) :*** *(M+1) 419.97*
***RMN$^1$H :*** *$\delta_H$ ppm 400 MHz, DMSO*
*13.16 (1H, sl, NH), 12.06 (1H, sl, NH), 9.90 (1H, s, $CH_{arom}$), 8.66 (1H, s, $CH_{arom}$), 8.07 (1H, s, $CH_{arom}$), 7.83 (1H, dd, $CH_{arom}$), 7.52 (1H, d, $CH_{arom}$), 4.11-4.06 (1H, m, NH), 3.87 (3H, s, $CH_3$), 3.31 (4H, s, $2xCH_2$), 3.16 (3H, sl, $NCH_3$), 2.33-2.17 (2H, m, $2xCH_{pipér}$), 2.12-2.04 (2H, m, $CH_{pipér}$).*

**Acide 8-(2,6-dichloro-phénylméthanesulfonyl)-4-oxo-4,5-dihydro-3*H*-pyrrolo[2,3-*c*]quinoline-1-carboxylate d'éthyle (194)**

A une solution de 139 mg (1.1 mmol) de sulfite de sodium et de 78 mg (0.55 mmol) de phosphate de sodium dibasique dissous dans 2.5 mL d'eau sont additionnés 180 mg (0.55 mmol) d'acide 8-chlorospifonyl-4-oxo-4,5-dihydro-3*H*-pyrrolo[2,3-*c*]quinoline-1-carboxylique. La solution est portée à 60°C pendant 20 heures puis une solution de 132 mg (0.55) de bromure de 2,6-dichlorobenzyle dans 2.5 mL de diméthylformamide est additionnée. L'agitation est maintenue à 60°C pendant 4 heures puis la solution est filtrée. Le solide est trituré dans du méthanol chaud puis de nouveau filtré pour conduire à 88 mg (35%) d'acide 8-(2,6-dichloro-phénylméthanesulfonyl)-4-oxo-4,5-dihydro-3*H*-pyrrolo[2,3-*c*] quinoline-1-carboxylate d'éthyle sous la forme d'un solide blanc.
***LCMS (IE, m/z):*** *(M+1) 450.87*
***RMN$^1$H :*** *$\delta_H$ ppm 400 MHz, DMSO*
*13.20 (1H, sl, NH), 12.34 (1H, sl, $CO_2H$), 12.09 (1H, s, MH), 9.94 (1H, d, $CH_{arom}$), 7.99 (1H, s, $CH_{arom}$), 7.73 (1H, dd, $CH_{arom}$), 7.54 (1H, d, $CH_{arom}$), 7.48-7.44 (2H, m, $2xCH_{arom}$), 7.37 (1H, dd, $CH_{arom}$), 4.86 (2H, s, $CH_2$).*

**4-Oxo-8-(pyridin-4-ylméthanesulfonyl)-4,5-dihydro-3*H*-pyrrolo[2,3-*c*]quinoline-1-carboxylate d'éthyle (195)**

A une solution de 272 mg (2 mmol) de sulfite de sodium et de 142 mg (1 mmol) de phosphate de sodium dibasique dissous dans 3 mL d'eau sont additionnés 326 mg (1 mmol) d'acide 8-chlorosulfonyl-4-oxo-4,s-dihydro-3*H*-pyrrolo[2,3-*c*]quinoline-1-carboxylique. La solution est portée à 60°C pendant 20 heures puis une solution de 150 mg (1.2 mmol) de 4-chlorométhylpyridine dans 3 mL d'acétone est additionnée. L'agitation est maintenue à 60°C pendant 24 heures puis la solution est filtrée. Le résidu est estérifié par la méthode générale C avec 520 μL (4 mmol) de diéthylsulfate en présence de 700 μL (5 mmol) de triéthylamine pour conduire après purification par chromatographie sur silice (éluant dichlorométhane/méthanol 96/4) à 11 mg (2%) de 4-oxo-8-(pyridin-4-ylméthanesulfonyl)-4,5-dihydro-3*H*-pyrrolo[2,3-*c*]quinoline-1-carboxylate d'éthyle sous la forme d'un solide blanc.
***LCMS (IE, m/z):*** *(M+1) 412.25*
***RMN$^1$H :*** *$\delta_H$ ppm 400 MHz, DMSO*
*13.20 (1H, sl, NH), 12.09 (1H, s, NH), 9.68 (1H, d, $CH_{arom}$), 8.46 (2H, d, $2xCH_{arom}$), 8.04 (1H, s, $CH_{arom}$), 7.67 (1H, dd, $CH_{arom)}$, 7.51 (1H, d, $CH_{arom}$), 7.16 (2H, d, $2xCH_{arom}$), 4.70 (2H, s, $CH_2$), 4.31 (2H, q, $CH_2$), 1.35 (3H, t, $CH_3$).*

**4-Oxo-8-(pyridin-2-ylméthanesulfonyl)-4,5-dihydro-3*H*-pyrrolo[2,3-*c*]quinoline-1-carboxylate d'éthyle (196)**

Ce composé est préparé selon la synthèse 195, à partir de 326 mg (1 mmol) d'acide 8-chlorosulfonyl-4-oxo-4,5-dihydro-3*H*-pyrroo[2,3-*c*]quinoline-1-carboxylique et de 130 mg (1.1 mmol) de 2-chlorométhylpyridine pour donner après estérification suivant la méthode générale C (diéthylsulfate utilisé comme dialkylsulfate) et purification par trituration dans un mélange dichlorométhane/méthanol 1/1, 40 mg (18% sur deux étapes) de 4-oxo-8-(pyridin-2-ylméthanesulfonyl)-4,5-dihydro-3*H*-pyrrolo[2,3-*c*]quinoline-1-carboxylate d'éthyle sous la forme d'un solide blanc.
***LCMS (IE, m/z):*** *(M+1) 412.18*
***RMN$^1$H :*** *$\delta_H$ ppm 400 MHz, DMSO*

*13.18 (1H, sl, NH), 12.10 (1H, s, NH), 9.66 (1H, d, $CH_{arom}$), 8.35 (1H, d, $CH_{arom}$), 8.05 (1H, s, $CH_{arom}$), 7.76 (1H, td, $CH_{arom}$), 7.62 (1H, dd, $CH_{arom}$), 7.49 (1H, d, $CH_{arom}$), 7.39 (1H, d, $CH_{arom}$), 7.32-7.27 (1H, m, $CH_{arom}$), 4.74 (2H, s, $CH_2$), 4.31 (2H, q, $CH_2$), 1.35 (3H, t, $CH_3$).*

**8-Cyclohexanesulfonyl-4-oxo-4,5-dihydro-3*H*-pyrrolo[2,3-*c*]quinoline-1-carboxylate d'éthyle (197)**

A une solution de 49 mg (0.13 mmol) de 8-cyclohexylsulfanyl-4-oxo-4,5-dihydro-3*H*-pyrrolo[2,3-*c*]quinoline-1-carboxylate d'éthyle dans du méthanol (5 mL) et de l'acétonitrile (1 mL) est additionnée une solution de 410 mg (0.66 mmol) d'oxone dans 2 mL d'eau. La solution est agitée à température ambiante pendant 24 heures. Les solvants sont évaporés puis le résidu est repris à l'eau. Le produit est extrait avec de l'acétate d'éthyle et les phases organiques sont séchées sur sulfate de magnésium, filtrées et évaporées. Le résidu est trituré dans de l'éther diéthylique pour conduire à 40 mg (74%) de 8-cyclohexanesulfonyl-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 403.31*
***RMN¹H :*** *$\delta_H$ ppm 400 MHz, DMSO*
*13.21 (1H, sl, NH), 12.12 (1H, sl, NH), 9.81 (1H, d, $CH_{arom}$), 8.06 (1H, s, $CH_{arom}$), 7.80 (1H, dd, $CH_{arom}$), 7.39 (1H, d, $CH_{arom}$), 4.33 (2H, q, $CH_2$), 3.12-3.03 (1H, m, CH), 1.97-1.89 (2H, m, 2xCH), 1.77-1.69 (2H, m, 2xCH), 1.60-1.53 (1H, m, CH),1.35 (3H, t, $CH_3$), 1.32-1.15 (4H, m, (4xCH), 1.12-0.99 (1H, m, CH).*

**4-Oxo-8-phénylméthanesulfinyl-4,5-dihydro-3*H*-pyrrolo[2,3-*c*]quiooline-1-carboxylate d'éthyle (198)**

A une solution de 90 mg (0.24 mmol) de 8-benzylsulfanyl-4-oxo-4,5-dihydro-3*H*-pyrolo[2,3-*c*]quinoline-1-carboxylate d'éthyle en suspension dans du dichlorométhane (8 mL) et du dioxane (8 mL) sont ajoutés à 0˚C 41 mg (0.24 mmol) d'acide métachloroperbenzoïque. Le milieu réactionnel est agité 6 heures à 0-5˚C puis une solution aqueuse de carbonate de potassium est additionnée et le produit est extrait avec de l'acétate d'éthyle. La phase organique est lavée avec une solution aqueuse de chlorure de sodium, séchée sur sulfate de magnésium, filtrée et évaporée. Le résidu est purifié par préparative LCMS pour conduire à 40 mg (42%) de 4-oxo-8-phénylméthanesulfinyl-4,5-dihydro-3*H*-pyrrolo [2,3-*c*]quinoline-1-carboxylate d'éthyle sous la forme d'un solide blanc
***LCMS (IE, m/z):*** *(M+1) 395.31*
***RMN¹H :*** *$\delta_H$ ppm 400 MHz, DMSO*
*13.12 (1H, sl, NH), 11.93 (1H, s, NH), 9.38 (1H, s, $CH_{arom}$), 8.02 (1H, s, $CH_{arom}$), 7.56 (1H, dd, $CH_{arom}$), 7.51 (1H, d, $CH_{arom}$), 7.26-7.24 (3H, m,3x$CH_{arom}$), 7.14-7.09 (2H, m, 2x$CH_{arom}$), 4.30 (2H, q, $CH_2$), 4.20 (1H, d, $CH_{ali}$), 4.15 (1H, d, $CH_{ali}$), 1.35 (3H, t, $CH_3$).*

**8-Ethynyl-4-oxo-4,5-dihydro-3*H*-pyrrolo[2,3-*c*]quinoline-1-carboxylate d'éthyle (199)**

A une solution de 398 mg (1.13 mmol) de 4-oxo-8-triméthylsilanyléthynyl-4,5-dihydro-3*H*-pyrrolo[2,3-*c*]quinoline-1-carboxylate d'éthyle dans 10 ml de tétrahydrofurane sont ajoutés 1.2 ml (1.2 mmol) d'une solution 1M de fluorure de tétrabutylammonium dans le tétrahydrofurane puis le mélange est agité 1 heure à température ambiante. Le solvant est évaporé et le produit extrait avec de l'acétate d'éthyle. La phase organique est lavée avec une solution aqueuse 1M d'acide chlorhydrique, une solution saturée de chlorure de sodium, séchée sur sulfate de magnésium, filtrée et évaporée. Le produit est cristallisé dans un mélange méthanol/acétone (4/1) pour donner 160 mg (50%) de 8-éthynyl-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 281.17*
***RMN¹H :*** *$\delta_H$ ppm 400 MHz, DMSO*
*13.12 (1H, s, NH), 11.88 (1H, s, NH), 9.43 (1H, s, $CH_{arom}$), 8.02 (1H, s, $CH_{arom}$), 7.50 (1H, d, $CH_{arom}$), 7.39 (1H, d, $CH_{arom}$), 4.33 (2H, q, $CH_2$), 4.12 (1H, s, CH), 1.37 (3H, t, $CH_3$).*

**4-Oxo-8-triméthylsilanyléthynyl-4,5-dihydro-3*H*-pyrrolo[2,3-*c*]quinoline-1-carboxylate d'éthyle (200)**

A une suspension de 898 mg (235 mmol) de 8-iodo-4-oxo-4,5-dihydro-3*H*-pyrrolo[2,3-*c*]quinoline-1-carboxylate d'éthyle dans 30 ml de dioxane anhydre sont ajoutés 224 mg (1.2 mmol) d'iodure de cuivre, 309 mg (3 mmol) de triéthylamine et 165 mg (0.23 mmol) de dichlorobis(triphénylphosphine)-palladium (II) puis ce mélange est dégazé sous argon. Après 15 minutes, 346 mg (3.52 mmol) d'éthynyl-triméthyl-silane sont ajoutés et le milieu réactionnel est chauffé à 100˚C pendant 3 heures. Le brut réactionnel est filtré sur célite, puis le filtrat est évaporé à sec et le résidu est purifié sur silice (éluant acétate d'éthyle) pour conduire à 400 mg (48%) de 4-oxo-8-triméthylsilanyléthynyl-4,5-dihydro-3*H*-pyrrolo[2,3-*c*]quinoline-1-carboxylate d'éthyle sous la forme d'un solide jaune.
***LCMS (IE, m/z):*** *(M+1) 353.20*

***RMN$^1$H :*** *$\delta_H$ ppm 400 MHz, DMSO*
*13.13 (1H, s, NH), 11.91 (1H, s, NH), 9.37 (1H, s, $CH_{arom}$), 8.03 (1H, s, $CH_{arom}$), 7.47 (1H, d, $CH_{arom}$), 7.40 (1H, d, $CH_{arom}$), 4.32 (2H, q, $CH_2$), 1.36 (3H, t, $CH_3$), 0.25 (9H, s, $3xCH_3$).*

**4-Oxo-8-phényléthynyl-4,5-dihydro-3*H*-pyrrolo[2,3-*c*]quinoline-1-carboxylate d'éthyle (201)**

A une suspension de 80 mg (0.21 mmol) de 8-iodo4-oxo-4,5-dihydro-3*H*-pyrrolo[2,3-*c*]quinoline-1-carboxylate d'éthyle dans 2.4 ml de dioxane anhydre sont ajoutés 20 mg (0.11 mmol) d'iodure de cuivre, 28 mg (0.27 mmol) de triéthylamine et 14 mg (0.02 mmol) de dichlorobis(triphénylphosphine)-palladium (II) puis ce mélange est dégazé sous argon. Après 15 minutes, 32 mg (0.31 mmol) de phénylacétylène sont ajoutés puis le milieu est chauffé à 100˚C pendant 3 heures. Le brut réactionnel est filtré sur célite, le filtrat est évaporé à sec et le résidu est purifié par chromatographie sur silice (éluant cyclohéxane/acétate d'éthyle) pour conduire à 70 mg (93%) de 4-oxo-8-phényléthynyl-4,5-dihydro-3*H*-pyrrolo[2,3-*c*]quinoline-1-carboxylate d'éthyle sous la forme d'un solide jaune.
***LCMS (IE, m/z):*** *(M+1) 357.26*
***RMN$^1$H :*** *$\delta_H$ ppm 400 MHz, DMSO*
*13.14 (1H, s, NH), 11.92 (1H, s, NH), 9.48 (1H, s, $CH_{arom}$), 8.04 (1H, s, $CH_{arom}$), 7.57-7.59 (3H, m, $CH_{arom}$), 7.43-7.45 (4H, m, $CH_{arom}$), 4.33 (2H, q, $CH_2$), 1.37 (3H, t, $CH_3$).*

**4-Oxo-8-pyridin-2-yléthynyl-4,5-dihydro-3*H*-pyrrolo[2,3-*c*]quinoline-1-carboxylate d'éthyle (202)**

A une suspension de 80 mg (0.21 mmol) de 8-iodo-4-oxo-4,5-dihydro-3*H*-pyrrolo[2,3-*c*]quinoline-1-carboxylate d'éthyle dans 2.4 ml de dioxane anhydre sont ajoutés 20 mg (0.11 mmol) d'iodure de cuivre, 28 mg (0.27 mmol) de triéthylamine et 14 mg (0.02 mmol) de dichlorobis(triphénylphosphine)-palladium (II) puis ce mélange est dégazé sous argon. Après 15 minutes, 32 mg (0.31 mmol) de 2-éthynyl-pyridine sont ajoutés puis le milieu est chauffé à 100˚C pendant 3 heures. Le brut réactionnel est alors filtré sur célite, le filtrat est évaporé à sec et le résidu est purifié par chromatographie sur silice (éluant acétate d'éthyle) pour conduire à 34 mg (46%) de 4-oxo-8-pyridin-2-yléthynyl-4,5-dihydro-3*H*-pyrrolo[2,3-*c*]quinoline-1-carboxylate d'éthyle sous la forme d'un solide marron.
***LCMS (IE, m/z):*** *(M+1) 358.21*
***RMN$^1$H :*** *$\delta_H$ ppm 400 MHz, DMSO*
*13.15 (1H, s, NH), 11.94 (1H, s, NH), 9.56 (1H, s, $CH_{arom}$), 8.63 (1H, m, $CH_{arom}$), 8.05 (1H, s, $CH_{arom}$), 7.84-7.88 (1H, m, $CH_{arom}$), 7.62-7.67 (2H, m, $CH_{arom}$), 7.42-7.48 (2H, m, $CH_{arom}$), 4.34 (2H, q, $CH_2$), 1.37 (3H, t, $CH_3$).*

**8-(3-Diméthylamino-prop-1-ynyl)-4-oxo-4,5-dihydro-3*H*-pyrrolo[2,3-*c*]quinoline-1-carboxylate d'éthyle (203)**

A une suspension de 200 mg (0.52 mmol) de 8-iodo-4-oxo-4,5-dihydro-3*H*-pyrrolo[2,3-*c*]quinoline-1-carboxylate d'éthyle dans 5 ml de dioxane anhydre sont ajoutés 50 mg (0.26 mmol) d'iodure de cuivre, 69 mg (0.68 mmol) de triéthylamine et 37 mg (0.05 mmol) de dichlorobis(triphénylphosphine)-palladium (II) puis ce mélange est dégazé sous argon. Après 15 minutes, 65 mg (0.78 mmol) de diméthyl-prop-2-ynyl-amine sont ajoutés puis le milieu est chauffé à 100˚C pendant 6 heures. Le brut réactionnel est alors filtré sur célite, le filtrat est évaporé à sec et le résidu est purifié par chromatographie sur silice (acétate d'éthyle/méthanol/ammoniaque) pour conduire à 64 mg (36%) de 8-(3-diméthylamino-prop-1-ynyl)-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 338.27*
***RMN$^1$H :*** *$\delta_H$ ppm 400 MHz, DMSO*
*12.82 (1H, sl, NH), 11.58 (1H, s, NH), 9.14 (1H, s, $CH_{arom}$), 7.77 (1H, s, $CH_{arom}$), 7.21 (1H, d, $CH_{arom}$), 7.16 (1H, d, $CH_{arom}$), 4.09 (2H, q, $CH_2$), 3.24 (2H, s, $CH_2$), 2.04 (6H, s, $2xCH_3$), 1.13 (3H, t, $CH_3$).*

**8-(3-Hydroxy-3-méthyl-but-1-ynyl)-4-oxo-4,5-dihydro-3*H*-pyrrolo[2,3-*c*]quinoline-1-carboxylate d'éthyle (204)**

A une suspension de 200 mg (0.52 mmol) de 8-iodo-4-oxo-4,5-dihydro-3*H*-pyrrolo[2,3-*c*]quinoline-1-carboxylate d'éthyle dans 5 ml de dioxane anhydre sont ajoutés 50 mg (0.26 mmol) d'iodure de cuivre, 69 mg (0.68 mmol) de triéthylamine et 37 mg (0.05 mmol) de dichlorobis(triphénylphosphine)-palladium (II) puis ce mélange est dégazé sous argon. Après 15 minutes, 66 mg (0.78 mmol) de 2-méthyl-but-3-yn-2-ol sont ajoutés puis le milieu est chauffé à 100˚C pendant 1 heure. Le brut réactionnel est alors filtré sur célite, le filtrat est évaporé à sec et le résidu est purifié par chromatographie sur silice (acétate d'éthyle) puis le produit est trituré dans du méthanol pour conduire à 135 mg (76%) de 8-(3-hydroxy-3-méthyl-but-1-ynyl)-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 339.26*

***RMN¹H :*** *$\delta_H$ ppm 400 MHz, DMSO*
*13.07 (1H, s, NH), 11.82 (1H, s, NH), 9.32 (1H, s, $CH_{arom}$), 8.01 (1H, s, $CH_{arom}$), 7.36-7.48 (3H, m, 2x$CH_{arom}$ et OH), 4.32 (2H, q, $CH_2$), 1.49 (6H, s, 2x$CH_3$), 1.36 (3H, t, $CH_3$).*

**8-(3-Morpholin-4-yl-prop-1-ynyl)-4-oxo-4,5-dihydro-3*H*-pyrrolo[2,3-*c*]quinoline-1-carboxylate d'éthyle (205)**

A une suspension de 47 mg (0.17 mmol) de 8-éthynyl-4-oxo-4,5-dihydro-3*H*-pyrrolo[2,3-*c*]quinoline-1-carboxylate d'éthyle dans 5 ml de dioxane anhydre sont ajoutés 15 mg (0.17 mmol) de para-formaldéhyde, 15 mg (0.17 mmol) de morpholine et 3 mg (0.02 mmol) d'iodure de cuivre puis une goutte d'acide acétique est ajoutée et le mélange est chauffé à 85°C pendant 48 heures. Le brut réactionnel est alors filtré puis le filtrat est évaporé et le résidu purifié par LCMS préparative pour donner 4 mg (6%) de 8-(3-morpholin-4-yl-prop-1-ynyl)-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 380.25*
***RMN¹H :*** *$\delta_H$ ppm 400 MHz, $CD_3OD$*
*9.55 (1H, sl, $CH_{arom}$), 8.06 (1H, s, $CH_{arom}$), 7.49 (1H, dd, $CH_{arom}$), 7.38 (1H, d, $CH_{arom}$), 4.41 (2H, q, $CH_2$), 3.92-3.86 (2H, m, $CH_2$), 3.83-3.76 (2H, m, $CH_2$), 3.63 (2H, s, $CH_2$), 3.27-3.21 (2H, m, $CH_2$), 2.83-2.72 (2H, m, $CH_2$), 1.45 (3H, t, $CH_3$).*

**8-{3-[(2-Cyano-éthyl)-méthyl-amino]-prop-1-ynyl}-4-oxo-4,5-dihydro-3*H*-pyrrolo[2,3-*c*]quinoline-1-carboxylate d'éthyle (206)**

A une suspension de 98 mg (0.35 mmol) de 8-éthynyl-4-oxo-4,5-dihydro-3*H*-pyrrolo[2,3-*c*]quinoline-1-carboxylate d'éthyle dans 3 ml de dioxane anhydre sont ajoutés 31 mg (0.35 mmol) de para-formaldéhyde, 35 mg (0.42 mmol) de 3-(méthyl-prop-2-ynyl-amino)-propionitrile et 7 mg (0.04 mmol) d'iodure de cuivre puis le mélange est chauffé à 110°C pendant 6 heures. Le brut réactionnel est alors filtré puis le filtrat est évaporé. Le résidu est trituré avec du méthanol puis le nouveau filtrat est evaporé. Le produit est recristallisé avec du méthanol pour donner 10 mg (8%) de 8-{3-[(2-cyano-éthyl)-méthyl-amino]-prop-1-ynyl}-4-oxo-4,5-dihydro-3*H*-pyrrolo[2,3-*c*]quinoline-1-carboxylate d'éthyle sous la forme d'un solide blanc.
*LCMS **(IE, m/z):** (M+1) 377.35*
***RMN¹H :*** *$\delta_H$ ppm 400 MHz, DMSO*
*13.06 (1H, sl, NH),11.82 (1H, sl, NH), 9.37 (1H, s, $CH_{arom}$), 8.01 (1H, s, $CH_{arom}$), 7.51-7.35 (2H, m, 2x$CH_{arom}$), 4.33 (2H, q, $CH_2$), 3.64 (2H, s, $CH_2$), 3.30-3.26 (2H, m, $CH_2$), 2.72 (3H, s, $CH_3$), 2.38-2.31 (2H, m, $CH_2$), 1.35 (3H, t, $CH_3$).*

**4-Oxo-8-phénéthyl-4,5-dihydro-3*H*-pyrrolo[2,3-*c*]quinoline-1-carboxylate d'éthyle (207)**

A une suspension de 14 mg (0.04 mmol) de 4-oxo-8-phényléthynyl-4,5-dihydro-3*H*-pyrrolo[2,3-*c*]quinoline-1-carboxylate d'éthyle dans 5 ml d'éthanol sont ajoutés 10 mg de palladium sur charbon et le mélange est placé sous atmosphère d'hydrogène pour 24 heures. Le brut réactionnel est alors filtré sur célite puis le filtrat est évaporé. Le résidu est trituré avec du méthanol pour donner 4 mg (28%) de 4-oxo-8-phénéthyl-4,5-dihydro-3*H*-pyrrolo[2,3-*c*]quinoline-1-carboxylate d'éthyle sous la forme d'un solide blanc.
***LCMS (IE, m/z):*** *(M+1) 361.36*
***RMN¹H :*** *$\delta_H$ ppm 400 MHz, DMSO*
*12.98 (1H, sl, NH), 11.62 (1H, sl, NH), 9.15 (1H, s, $CH_{arom}$), 8.00 (1H, s, $CH_{arom}$), 7.32-7.14 (7H, m, 7x$CH_{arom}$), 4.36 (2H, q, $CH_2$), 3.02-2.97 (4H, m, 2x$CH_2$), 1.42-1.37 (3H, t, $CH_3$).*

**8-(3-Hydroxy-3-méthyl-butyl)4-oxo-4,5-dihydro-3*H*-pyrrolo[2,3-*c*]quinoline-1-carboxylate d'éthyle (208)**

Une suspension de 47 mg (0.14 mmol) de 8-(3-hydroxy-3-méthyl-but-1-ynyl)-4-oxo-4,5-dihydro-3*H*-pyrrolo [2,3-*c*]quinoline-1-carboxylate d'éthyle, de 164 mg (2.6 mmol) de formiate d'ammonium et de 5 mg de palladium sur charbon dans 10 ml d'éthanol est portée au reflux pendant 8 heures. La solution est alors filtrée sur célite puis évaporée. De l'eau est ajoutée et le produit extrait deux fois avec de l'acétate d'éthyle. Les phases organiques sont séchées sur sulfate de magnésium, filtrées et évaporées pour conduire à 5 mg (10%) de 8-(3-hydroxy-3-méthyl-butyl)-4-oxo-4,5-dihydro-3*H*-pyrrolo[2,3-*c*]quinoline-1-carboxylate d'éthyle sont obtenus sous la forme d'une huile incolore.
***LCMS (IE, m/z):*** *(M+1) 343.29*
***RMN¹H :*** *$\delta_H$ ppm 400 MHz, $CD_3OD$*
*9.15 (1H, s, $CH_{arom}$), 7.93 (1H, s, $CH_{arom}$), 7.18-7.25 (2H, m, 2x$CH_{arom}$), 4.30 (2H, q, $CH_2$), 2.70-2.74 (2H, m, $CH_2$), 1.73-1.78 (2H, m, $CH_2$), 1.33 (3H, t, $CH_3$) 1.19 (6H, s, 2x$CH_3$).*

**8-Iodo-4-oxo-4,5-dihydro-3*H*-pyrrolo[2,3-*c*]quinoline-1-carboxylate d'éthyle (209)**

**Méthode D**

A une suspension de 1.61 g (5.17 mmol) de diiode et de 1.31 g (5.17 mmol) de sulfate d'argent dans de l'éthanol anhydre (100 mL) est additionné 1.49 g (4.7 mmol) de 4-oxo-4,5-dihydro-3*H*-pyrrolo[2,3-*c*]quinoline-1-carboxylate d'éthyle cocristallisé avec un équivalent d'acide acétique. Le milieu réactionnel est agité à température ambiante pendant 6 heures puis les solvants sont évaporés sous vide. Le résidu est repris à l'eau et le produit est extrait abondamment avec un mélange d'acétate d'éthyle/méthanol 95/5. Les phases organiques sont séchées sur sulfate de magnésium, filtrées sur célite et évaporées sous vide pour conduire à 1.4 g (78%) de 8-iodo-4-oxo-4,5-dihydro-3*H*-pyrrolo[2,3-*c*]quinoline-1-carboxylate d'éthyle sous la forme d'un solide blanc.

Méthode E

Alternativement à une solution de 316 mg (1 mmol) de 4-oxo-4,5-dihydro-3*H*-pyrrolo[2,3-*c*]quinoline-1-carboxylate d'éthyle cocristallisé avec 1 équivalent d'acide acétique et de 652 mg (1 mmol) de triflate d'indium dans de l'acétonitrile anhydre (20 mL) est additionnée 1.5 mL (1.5 mmol) d'une solution 1M de monochlorure d'iode dans du dichlorométhane. La solution est portée 3 heures à 45°C puis agitée à température ambiante pendant 20 heures. Le solide est filtré, et trituré avec de l'acétonitrile et de l'éther diisopropylique pour conduire à 368 mg (96%) de 8-iodo-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 382.85*
***RMN $^1$H :*** *$\delta_H$ ppm 400 MHz, DMSO*
*13.10 (1H, sl, NH), 11.79 (1H, sl, NH), 9.65 (1H, s, $CH_{arom}$), 8.01 (1H, d, $CH_{arom}$), 7.69 (1H, d, $CH_{arom}$), 7.22 (1H, d, $CH_{arom}$), 4.33 (2H, dd, $OCH_2$), 1.37 (3H, t, $CH_3$).*

**8-Iodo-4-oxo-4,5-dihydro-3*H*-pyrrolo[2,3-*c*]quinoline-1-carboxylate de propyle (210)**

Ce composé est préparé selon la selon la synthèse 209 (méthode D), à partir de 202 mg (0.75 mmol) de 4-oxo-4,5-dihydro-3*H*-pyrrolo[2,3-*c*]quinoline-1-carboxylate de propyle, de 190 mg (0.75 mmol) de diiode et de 257 mg ( 0.82 mmol) de sulfate d'argent pour conduire après recristallisation dans un mélange dichlorométhane/pentane à 190 mg (64%) de 8-iodo-4-oxo-4,5-dihydro-3*H*-pyrrolo[2,3-*c*]quinoline-1-carboxylate de propyle sous la forme d'un solide blanc.
***LCMS (IE, m/z):*** *(M+1) 396.80*
***RMN $^1$H :*** *$\delta_H$ ppm 400 MHz, DMSO*
*13.11 (1H, sl, NH), 11.78 (1H, sl, NH), 9.64 (1H, s, $CH_{arom}$), 8.01 (1H, d, $CH_{arom}$), 7.70 (1H, d, $CH_{arom}$), 7.22 (1H, d, $CH_{arom}$), 4.24 (2H, t, $OCH_2$), 1.79-1.74 (2H, m, $CH_2$), 1.01 (3H, t, $CH_3$).*
Le 4-oxo-4,5-dihydro-3*H*-pyrrolo[2,3-*c*]quinoline-1-carboxylate de propyle a été préparé selon la méthode d'estérification C à partir de 194 mg (0.85 mmol) d'acide 4-oxo-4,5-dihydro-3*H*-pyrrolo[2,3-*c*]quinoline-1-carboxylique, 1.29 mL (12.75 mmol) de triéthylamine et de 698 µL (1.1 mmol) de di-*n*-propylsulfate pour conduire après précipitation dans l'eau à 200 mg (87%) de 4-oxo-4,5-dihydro-3*H*-pyrrolo[2,3-*c*]quinoline-1-carboxylate de propyle sous la forme d'un solide beige.

**8-Iodo-4-oxo-4,5-dihydro-3*H*-pyrrolo[2,3-*c*]quinoline-1-carboxylate de cyclopentyle (211)**

Ce composé est préparé selon la selon la synthèse 209 (méthode D), à partir de 251 mg (0.85 mmol) de 4-oxo-4,5-dihydro-3*H*-pyrrolo[2,3-*c*]quinoline-1-carboxylate de cyclopentyle, de 237 mg (0.93 mmol) de diiode et de 291 mg (0.93 mmol) de sulfate d'argent pour conduire après trituration dans l'éther diéthylique à 275 mg (76%) de 8-iodo-4-oxo-4,5-dihydro-3*H*-pyrrolo[2,3-*c*]quinoline-1-carboxylate de cyclopentyle sous la forme d'un solide jaune pâle.
***LCMS (IE, m/z):*** *(M+1) 422.88*
***RMN $^1$H :*** *$\delta_H$ ppm 400 MHz, DMSO*
*13.14 (1H, sl, NH), 11.82 (1H, sl, NH), 9.68 (1H, s, $CH_{arom}$), 8.03 (1H, d, $CH_{arom}$), 7.74 (1H, d, $CH_{arom}$), 7.27 (1H, d, $CH_{arom}$), 5.40 (1H, sl, OCH), 1.99-1.67 (8H, m, $CH_2$).*
Le 4-oxo-4,5-dihydro-3*H*-pyrrolo[2,3-*c*]quinoline-1-carboxylate de cyclopentyle a été préparé selon la méthode d'estérification C à partir de 194 mg (0.85 mmol) d'acide 4-oxo-4,5-dihydro-3*H*-pyrrolo[2,3-*c*]quinoline-1-carboxylique, 1.29 mL (12.75 mmol) de triéthylamine et de 1.99 g (8.5 mmol) de dicyclopentylsulfate pour conduire après précipitation dans l'eau à 250 mg (98%) de 4-oxo-4,5-dihydro-3*H*-pyrrolo[2,3-*c*]quinoline-1-carboxylate de cyclopentyle sous la forme d'un solide beige.

**8-Iodo-4-oxo-4,5-dihydro-3*H*-pyrrolo[2,3-*c*]quinoline-1-carboxylate d'isobutyle (212)**

Ce composé est préparé selon la selon la synthèse 209 (méthode D), à partir de 165 mg (0.58 mmol) de 4-oxo-4,5-dihydro-3*H*-pyrrolo[2,3-*c*]quinoline-1-carboxylate d'isobutyle, de 465 mg (1.83 mmol) de diiode et de 570 mg (1.83 mmol) de sulfate d'argent pour conduire après trituration dans le méthanol à 160 mg (67%) de 8-iodo-4-oxo-4,5-dihydro-3*H*-pyrrolo[2,3-*c*]quinoline-1-carboxylate d'isobutyle sous la forme d'un solide blanc.
***LCMS (IE, m/z):*** *(M+1) 410.89*
***RMN$^1$H :*** *$\delta_H$ ppm 400 MHz, DMSO*
*13.13 (1H, sl, NH), 11.79 (1H, sl, NH), 9.65 (1H, s, $CH_{arom}$), 8.01 (1H, d, $CH_{arom}$), 7.70 (1H, d, $CH_{arom}$), 7.23 (1H, d, $CH_{arom}$), 4.07 (2H, d, $OCH_2$), 2.03-2.10 (1H, m, CH), 1.01 (6H, t, $2xCH_3$).*

Le 4-oxo-4,5-dihydro-3*H*-pyrrolo[2,3-*c*]quinoline-1-carboxylate d'isobutyle a été préparé selon la méthode d'estérification C à partir de 194 mg (0.85 mmol) d'acide 4-oxo-4,5-dihydro-3*H*-pyrrolo[2,3-*c*]quinoline-1-carboxylique, 1.29 mL (12.75 mmol) de triéthylamine et de 892 mg (4.25 mmol) de diisobutylsulfate pour conduire après précipitation dans l'eau à 164 mg (68%) de 4-oxo-4,5-dihydro-3*H*-pyrrolo[2,3-*c*]quinoline-1-carboxylate d'isobutyle sous la forme d'un solide beige.

**7-méthoxy-4-oxo-4,5-dihydro-3*H*-pyrrolo[2,3-*c*]quinoline-1-carboxylate d'éthyle (213)**

Ce composé a été synthétisé selon la synthèse 1, à partir de 0.5 g (3.06 mmol) de 6-méthoxy-1,3-dihydro-indol-2-one (préparé selon George J. Quallich et P.M. Morissey, Synthesis, 1993, 51-53) pour donner après recristallisation dans l'acide acétique, 140 mg (13% sur deux étapes) de 7-méthoxy-4-oxo-4,5-dihydro-3*H*-pyrrolo[2,3-*c*]quinoline-1-carboxylate d'éthyle cocristallisé avec un équivalent d'acide acétique sous la forme d'un solide beige.
***LCMS (IE, m/z):*** *(M+1) 287*
***RMN $^1$H :*** *$\delta_H$ ppm 400 MHz, DMSO*
*12.86 (1H, sl, NH), 11.53 (1H, s, NH), 9.14 (1H, d, $CH_{arom}$), 7.93 (1H, s, $CH_{arom}$), 6.95 (1H, d, $CH_{arom}$), 6.84 (1H, dd, $CH_{arom}$), 4.29 (2H, q, $CH_2$), 3.50 (3H, s, $CH_3$), 1.33 (3H, t, $CH_3$).*

**8-Iodo-7-méthoxy-4-oxo-4,5-dihydro-3*H*-pyrrolo[2,3-*c*]quinoline-1-carboxylate d'éthyle (214)**

Ce composé est préparé selon la selon la synthèse 209 (méthode E), à partir de 50 mg (0.14 mmol) de 7-méthoxy-4-oxo-4,5-dihydro-3*H*-pyrrolo[2,3-*c*]quinoline-1-carboxylate d'éthyle, 81 mg (0.14 mg) de triflate d'indium et 140 µL (0.14 mmol) d'une solution 1M de monochlorure d'iode dans le dichlorométhyane pour donner après filtration et trituration dans l'acétonitrile, 25 mg (43%) de 8-iodo-7-méthoxy-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 413.19*
***RMN $^1$H :*** *$\delta_H$ ppm 400 MHz, DMSO*
*12.97 (1H, sl, NH), 11.64 (1H, s, NH), 9.69 (1H, s, $CH_{arom}$), 7.95 (1H, s, $CH_{arom}$), 7.00 (1H, s, $CH_{arom}$), 4.31 (2H, q, $CH_2$), 3.86 (3H, s, $CH_3$), 1.35 (3H, t, $CH_3$).*

**7-Fluoro-4-oxo-4,5-dihydro-3*H*-pyrrolo[2,3-*c*]quinoline-1-carboxylate d'éthyle (215)**

Ce composé a été synthétisé selon la synthèse 1, à partir de 5 g (33 mmol) de 6-fluoro-1,3-dihydro-indol-2-one pour donner après trituration dans l'éther diéthylique, 5.6 g ( 50% sur deux étapes) de 7-fluoro-4-oxo-4,5-dihydro-3*H*-pyrrolo[2,3-*c*]quinoline-1-carboxylate d'éthyle cocristallisé avec un équivalent d'acide acétique sous la forme d'un solide brun.
***LCMS (IE, m/z):*** *(M+1) 275.10*
***RMN $^1$H :*** *$\delta_H$ ppm 400 MHz, DMSO*
*13.02 (1H, sl, NH), 11.77 (1H, sl, NH), 9.30, (1H, dd, $CH_{arom}$), 7.99 (1H, s, $CH_{arom}$), 7.17 (1H, d, $CH_{arom}$), 7.09 (1H, t, $CH_{arom}$), 4.30 (2H, dd, $OCH_2$), 1.34 (3H, t, $CH_3$).*

**7-Chloro-4-oxo-4,5-dihydro-3*H*-pyrrolo[2,3-*c*]quinoline-1-carboxylate d'éthyle (216)**

Ce composé a été synthétisé selon la synthèse 1, à partir de 0.5 g (3.06 mmol) de 6-chloro-1,3-dihydro-indol-2-one pour donner après recristallisation dans l'éthanol, 3.68 g ( 21% sur deux étapes) de 7-chloro-4-oxo-4,5-dihydro-3*H*-pyrrolo[2,3-*c*]quinoline-1-carboxylate d'éthyle sous la forme d'un solide crème.
***LC/MS (IE, m/z) :*** *(M+1) 291.01*
***RMN$^1$H :*** *$\delta_H$ ppm 400 MHz, DMSO*
*13.10 (1H, sl, NH), 11.79 (1H, sl, NH), 9.26 (1H, d, $CH_{arom}$), 8.01 (1H, s, $CH_{arom}$), 7.44 (1H, d, $CH_{arom}$), 7.27 (1H, dd,*

*$CH_{arom}$), 4.30 (2H, q, $CH_2$), 1.34 (3H, t, $CH_3$).*

**8-(3-Diméthylamino-acryloyl)-4-oxo-4,5-dihydro-3*H*-pyrrolo[2,3-*c*]quinoline-1-carboxylate d'éthyle (217)**

A une solution de 1.49 g (5 mmol) de 8-acétyl-4-oxo-4,5-dihydro-3*H*-pyrrolo[2,3-*c*]quinoline-1-carboxylate d'éthyle dissous dans 25 mL de dioxane anhydre sont ajoutés 4.36 g (25 mmol) de *C-tert*-butoxy-*N*,*N*,*N'*,*N'*-tétraméthyl-méthanediamine. Le mélange est agité 1 heure à 90°C puis le solvant est évaporé. Le résidu est trituré avec un mélange d'acétate d'éthyle et d'éther diisopropylique pour donner un solide qui est filtré et lavé avec de l'éther diisopropylique puis du pentane. Après séchage, 1.66 g (94%) de 8-(3-diméthylamino-acryloyl)-4-oxo-4,5-dihydro-3*H*-pyrmlo[2,3-*c*] quinoline-1-carboxylate d'éthyle sont obtenus sous la forme d'un solide vert foncé. Le produit peut-être utilisé sans purification complémentaire. Une purification supplémentaire par chromatographie sur silice (éluant chloroforme/méthanol 95/5) du solide vert (50 mg) donne le produit sous la forme d'un solide beige.
***LCMS (IE, m/z):** (M+1) 354*
***RMN$^1$H :** $\delta_H$ ppm 400 MHz, DMSO*
*13.04 (1H, s, NH), 11.83 (1H, s, NH), 9.87 (1H, d, $CH_{arom}$), 8.00 (1H, s, $CH_{arom}$), 7.92 (1H, dd, $CH_{arom}$), 7.72 (1H, dd, $CH_{arom}$), 7.41 (1H, d, $CH_{arom}$), 5.94 (1H, d, $CH_{arom}$), 4.33 (2H, q, $CH_2$), 3.16 (3H, sl, $CH_3$), 2.94 (3H, s, $CH_3$), 1.36 (3H, t, $CH_3$).*

**8-(2-Morpholin-4-yl-acétyl)-4-oxo-4,5-dihydro-3*H*-pyrrolo[2,3-*c*]quinoline-1-carboxylate d'éthyle (218)**

Une solution de 80 mg (0.24 mmol) de 8-(2-chloro-acétyl)-4-oxo-4,5-dihydro-3*H*-pyrrolo[2,3-*c*]quinoline-1-carboxylate d'éthyle dans 420 μL (4.8 mmol) de morpholine est portée à 60°C pour 4 heures. De l'eau est additionné et le solide est filtré puis purifié par chromatographie sur silice (éluant dichlorométhane/méthanol 95/5) pour conduire à 24 mg (26%) de 8-(2-morpholin-4-yl-acétyl)-4-oxo-4,5-dihydro-3*H*-pyrrolo[2,3-*c*]quinoline-1-carboxylate d'éthyle sous la forme d'un solide brun.
***LCMS (IE, m/z):** (M+1) 384.074*
***RMN$^1$H** : $\delta_H$ ppm 400 MHz, DMSO*
*13.04 (1H, s, NH), 11.99 (1H, s, NH), 10.02 (1H, d, $CH_{arom}$), 8.04 (1H, s, $CH_{arom}$), 8.00 (1H, dd, $CH_{arom}$), 7.47 (1H, d, $CH_{arom}$), 4.33 (2H, q, $CH_2$), 3.84 (2H, s, $CH_2$), 3.62-3.55 (4H, m, 2x$CH_2$), 2.60-2.53 (4H, m, 2x$CH_2$), 1.36 (3H, t, $CH_3$).*

A une suspension de 596 mg (2 mmol) de 8-acétyl-4-oxo-4,5-dihydro-3*H*-pyrrolo[2,3-*c*]quinoline-1-carboxylate d'éthyle dans 10 mL d'un mélange 1,2-dichloroéthane/méthanol 3/1 sont ajoutés 1.04 g (3 mmol) de benzyltriméthylammonium de dichloroiodate. Le mélange est agité à 80°C pendant 48 heures puis le mélange est refroidi à température ambiante. L'excès de réactif est détruit par addition d'une solution aqueuse de bisulfite de sodium puis le solvant est évaporé. De l'eau est ajoutée au résidu puis le mélange est agité à l'aide d'un bain à ultra-sons pour obtenir une fine suspension. Le précipité est collecté par filtration puis trituré dans du méthanol, de l'éther diisopropylique puis du pentane pour donner après séchage, 385 mg (58%) de 8-(2-chloro-acétyl)-4-oxo-4,5-dihydro-3*H*-pyrrolo[2,3-*c*]quinoline-1-carboxylate d'éthyle sous la forme d'un solide beige.

**Chlorhydrate de (S) 8-(cyclopropyl-pyrrolidin-2-ylméthyl-carbamoyl)-4-oxo-4,5-dihydro-3*H*-pyrrolo[2,3-*c*]quinoline-1-carboxylate d'éthyle (219)**

Ce composé est préparé selon la synthèse 139, à partir de 78 mg (0.15 mmol) de (*S*) 8-[(1-*tert*-butoxycarbonyl-pyrrolidin-2-ylméthyl)-cyclopropyl-carbamoyl]-4-oxo-4,5-dihydro-3*H*-pyrrolo[2,3-*c*]quinoline-1-carboxylate d'éthyle et de 4 mL (7.5 mmol) d'une solution 2M d'acide chlorhydrique dans l'éther diéthylique pour donner après filtration, 67 mg (97%) de chlorhydrate de (*S*) 8-(cyclopropyl-pyrrolidin-2-ylméthyl-carbamoyl)-4-oxo-4,5-dihydro-3*H*-pyrrolo[2,3-*c*]quinoline-1-carboxylate d'éthyle sous la forme d'un solide beige.
***LCMS (IE, m/z):** (M+1) 423.34*
***RMN$^1$H :** $\delta_H$ ppm 400 MHz, DMSO*
*13.10 (1H, sl, NH), 11.89 (1H, s, NH), 9.57 (1H, sl, NH.HCl), 9.48 (1H, s, $CH_{arom}$), 8.69 (1H, sl, NH.HCl), 8.01 (1H, d, $CH_{arom}$), 7.75 (1H, dd, $CH_{arom}$), 7.42 (1H, d, $CH_{arom}$), 4.24 (2H, q, $CH_2$), 3.92-3.71 (3H, m, 3x$CH_{ali}$), 3.19-3.12 (3H, m, 3x$CH_{ali}$), 2.28-2.15 (1H m, $CH_{ali}$), 2.03-1.87 (2H, m, 2x$CH_{ali}$), 1.79-1.64 (1H, m, $CH_{ali}$), 1.34 (3H, t, $CH_3$), 0.58-0.43 (4H, m, 4x$CH_{cycloprop}$).*

A une solution de 169 mg (0.35 mmol) de (*S*) 2-{[cyclopropyl-(3-éthoxycarbonylméthylene-2-oxo-2,3-dihydro-1*H*-indole-5-carbonyl)-amino]-méthyl}-pyrrolidine-1-carboxylate de *tert*-butyle et de 75 mg (0.38 mmol) de 1-isocyanométhanesulfonyl-4-méthyl-benzène dans 5 ml de dioxane anhydre sont ajoutés 232 μL d'une solution à 20% de *tert*-butoxyde de potassium dans le THF puis la réaction est agitée 1 heure à 90°C. Le solvant est évaporé puis le produit est extrait deux fois à l'acétate d'éthyle. Les phases organiques sont lavées à l'eau, par une solution saturée de chlorure de sodium et séchées sur sulfate de magnésium. Le produit est purifié par chromatographie sur silice (éluant acétate

d'éthyle/méthanol 9/1) pour donner 82 mg (45%) de (*S*) 8-[(1-*tert*-butoxycarbonyl-pyrrolidin-2-ylméthyl)-cyclopropyl-carbamoyl]-4-oxo-4,5-dihydro-3*H*-pyrrolo[2,3-*c*]quinoline-1-carboxylate d'éthyle sous la forme d'un solide beige.

A une solution de 231 mg (0.58 mmol) de (*S*) 2-{[cyclopropyl-(2-oxo-2,3-dihydro-1*H*-indole-5-carbonyl)-amino]-méthyl}-pyrrolidine-1-carboxylate de *tert*-butyle, de 65 mg (0.64 mmol) de glyoxalate d'éthyle dans 10 ml d'éthanol sont ajoutés 5 mg (0.05 mmol) de pipéridine puis le mélange est chauffé 18 heures à 70°C. Le solvant est évaporé puis le produit purifié par chromatographie sur silice (éluant cyclohéxane/acétate d'éthyle 25/75) pour donner 173 mg (62%) de (*S*) 2-{[cyclopropyl-(3-éthoxycarbonylméthylène-2-oxo-2,3-dihydro-1*H*-indole-5-carbonyl)-amino]-méthyl}-pyrrolidine-1-carboxylate de *tert*-butyle sous la forme d'un solide jaune.

A une solution de 177 mg (1 mmol) d'acide 2-oxo-2,3-dithydro-1*H*-indole-5-carboxylique (préparé suivant la procédure suivante : Li Sun, Ngoc Tran, Congxin Liang, Flora Tang, Audie Rice, Randall Schreck, Kara Waltz, Laura K. Shawver, Gerald McMahon, and Cho Tang, Journal of Medicinal Chemistry, 1999, Vol. 42, 25, 5120), de 417 mg (1.1 mmol) de *O*-(benzotriazol-1-yl)-*N*,*N*,*N'*,*N'*-tétraméthyluronium, de 646 mg (5 mmol) de diisopropyléthylamine et de 27 mg (0.1 mmol) de 1-hydroxybenzotriazole dans 2 ml de diméthylformamide sont ajoutés 264 mg (1.1 mmol) de (*S*) 2-cyclopropylaminométhyl-pyrrolidine-1-carboxylate de *tert*-butyle puis la réaction est agitée à température ambiante pendant 30 minutes. Le solvant est évaporé puis le produit extrait à l'acétate d'éthyle. Les phases organiques sont séchées sur sulfate de magnésium, filtrées et évaporées. Le résidu est purifié par chromatographie sur silice (acétate d'éthyle/méthanol 95/5 puis 9/1) pour donner 254 mg (64%) de (*S*) 2-{[cyclopropyl-{2-oxo-2,3-dihydro-1*H*-indole-5-carbonyl}-amino]-méthyl}-pyrrolidine-1-carboxylate de *tert*-butyle sous la forme d'une solide beige.

**4-Oxo-8-phénylsulfanyl-4,5-dihydro-3*H*-pyrrolo[2,3-*c*]quinoline-1-carboxylate d'éthyle (220)**

Dans un tube scellé, une suspension de 152 mg (0.4 mmol) de 8-iodo-4-oxo-4,5-dihydro-3*H*-pyrrolo[2,3-*c*]quinoline-1-carboxylate d'éthyle (synthèse 209) et 110 mg (0.8 mmol) de carbonate de potassium dans du diméthoxyéthane (0.6 mL) est purgé par un courant d'argon avant l'ajout de 15 mg (0.08 mmol) de iodure de cuivre et de 86 mg (0.6 mmol) de thiophénol. Le mélange réactionnel est chauffé à 90°C pour 5 à 6 jours puis filtré sur célite et rincé avec du dichlorométhane puis du méthanol. Le filtrat est évaporé puis le résidu obtenu est purifié par chromatographie sur silice (éluant dichlorométhane/méthanol 96/4 puis 9/1) pour conduire à 42 mg (29%) de 4-oxo-8-phénylsulfanyl-4,5-dihydro-3*H*-pyrrolo[2,3-*c*]quinoline-1-carboxylate d'éthyle sous la forme d'un solide blanc.

***LCMS (IE, m/z):*** *(M+1) 365.20*

***RMN¹H :*** *$\delta_H$ ppm 400 MHz, DMSO*

*13.10 (1H, sl, NH), 11.85 (1H, s, NH), 9.48 (1H, s, $CH_{arom}$), 8.01 (1H, s, $CH_{arom}$), 7.46 (2H, s, 2x$CH_{arom}$), 7.35-7.27 (2H, m, 2x$CH_{arom}$), 7.24-7.15 (3H, m, 3x$CH_{arom}$), 4.26 (2H, q, $CH_2$), 1.30 (3H, t, $CH_3$).*

**8-Cyclohexylsulfanyl-oxo-4,5-dihydro-3*H*-pyrrolo[2,3-*c*]quinoline-1-carboxylate d'éthyle (221)**

Ce composé est préparé selon la selon la synthèse 220, à partir de 152 mg (0.4 mmol) de 8-iodo-4-oxo-4,5-dihydro-3*H*-pyrrolo[2,3-*c*]quinoline-1-carboxylate d'éthyle et de 150 μL (1.2 mmol) de cyclohexylthiol pour donner après purification par chromatographie sur silice (éluant dichlorométhane/méthanol 98/2) 105 mg (71%) de 8-cyclohexylsulfanyl-4-oxo-4,5-dihydro-3*H*-pyrrolo[2,3-*c*]quinoline-1-carboxylate d'éthyle sous la forme d'un solide blanc.

***LCMS (IE, m/z):*** *(M+1) 371.33*

***RMN¹H :*** *$\delta_H$ ppm 400 MHz, DMSO*

*13.02 (1H, sl, NH), 11.72 (1H, sl, NH), 9.33 (1H, d, $CH_{arom}$), 7.99 (1H, d, $CH_{arom}$), 7.41 (1H, dd, $CH_{arom}$), 7.36 (1H, d, $CH_{arom}$), 4.33 (2H, q, $CH_2$), 3.20-3.12 (1H, m, $CH_{cyclo}$), 1.98-1.88 (2H, m, 2x$CH_{cyclo}$), 1.76-1.66 (2H, m, 2x$CH_{cyclo}$), 1.60-1.53 (1H, m, $CH_{cyclo}$), 1.35 (3H, t, $CH_3$), 1.35-1.15 (5H, m, 5x$CH_{cyclo}$).*

**8-Benzylsulfanyl-4-oxo-4,5-dihydro-3*H*-pyrrolo[2,3-*c*]quinoline-1-carboxylate d'éthyle (222)**

Ce composé est préparé selon la selon la synthèse 220, à partir de 152 mg (0.4 mmol) de 8-iodo-4-oxo-4,5-dihydro-3*H*-pyrrolo[2,3-*c*]quinoline-1-carboxylate d'éthyle et de 34 μL (0.29 mmol) de phénylméthanethiol pour donner après purification par chromatographie sur silice (éluant dichlorométhane/méthanol 96/4) 106 mg (70%) de 8-benzylsulfanyl-4-oxo-4,5-dihydro-3*H*-pyrrolo[2,3-*c*]quinoline-1-carboxylale d'éthyle sous la forme d'un solide blanc.

***LCMS (IE, m/z):*** *(M+1) 379.11*

***RMN¹H*** *: $\delta_H$ ppm 400 MHz, DMSO*

*13.04 (1H, sl, NH), 11.71 (1H, s, NH), 9.31 (1H, s, $CH_{arom}$), 7.99 (1H, s, $CH_{arom}$), 7.41-7.30 (4H, m, 4x$CH_{arom}$), 7.26 (2H, t, 2x$CH_{arom}$), 7.20 (1H, d, $CH_{arom}$), 4.31 (2H, q, $CH_2$), 4.23 (2H, s, $CH_2$), 1.34 (3H, t, $CH_3$).*

**8-(4-Acétylamino-phénylsulfanyl)-4-oxo-4,5-dihydro-3*H*-pyrrolo[2,3-*c*]quinoline-1-carboxylate d'éthyle (223)**

Ce composé est préparé selon la selon la synthèse 220, à partir de 152 mg (0.4 mmol) de 8-iodo-4-oxo-4,5-dihydro-3*H*-pyrrolo[2,3-*c*]quinoline-1-carboxylate d'éthyle et de 100 mg (0.6 mmol) de 4-acétylaminobenzènethiol pour donner après purification par chromatographie sur silice (éluant dichlorométhane/méthanol 96/4) puis trituration dans l'éther diéthylique 67 mg (40%) de 8-(4-acétylamino-phénylsulfanyl)-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 422.25*
***RMN¹H*** *: $\delta_H$ ppm 400 MHz, DMSD*
*13.06 (1H, sl, NH), 11.79 (1H, s, NH), 10.00 (1H, s, NH), 9.37 (1H, s, $CH_{arom}$), 7.99 (1H, s, $CH_{arom}$), 7.55 (2H, d, $2xCH_{arom}$), 7.41 (1H, d, $CH_{arom}$), 7.34 (1H, dd, $CH_{arom}$), 7.23 (2H, m, $2xCH_{arom}$), 4.26 (2H, q, $CH_2$), 2.02 (3H, s, $CH_3$), 1.31 (3H, t, $CH_3$).*

**8-(3-Amino-phénylsulfanyl)-4-oxo-4,5-dihydro-3*H*-pyrrolo[2,3-*c*]quinoline-1-carbogylate d'éthyle (224)**

Ce composé est préparé selon la synthèse 220, à partir de 305 mg (0.8 mmol) de 8-iodo-4-oxo-4,5-dihydro-3*H*-pyrrolo[2,3-*c*]quinoline-1-carboxylate d'éthyle et 127 µL (1.2 mmol) de 3-aminobenzènethiol pour donner après purification par chromatographie sur silice (éluant dichlorométhane/méthanol 96/4) puis trituration dans le méthanol 150 mg (49%) de 8-(3-amino-phénylsulfanyl)-4-oxo-4,5-dihydro-3*H*-pyrroto[2,3-*c*]quinoline-1-carboxylate d'éthyle sous la forme d'un solide blanc.
***LCMS (IE, m/z):*** *(M+1) 380.15*
***RMN¹H:*** *$\delta_H$ ppm 400 MHz, DMSO*
*13.07 (1H, sl, NH), 11.82 (1H, s, NH), 9.44 (1H, s, $CH_{arom}$), 8.00 (1H, s, $CH_{arom}$), 7.45-7.39 (2H, m, $2xCH_{arom}$), 6.92 (1H, t, $CH_{arom}$), 6.39-6.31 (3H, m, $3xCH_{arom}$), 5.13 (2H, s, $NH_2$), 4.28 (2H, q, $CH_2$), 1.32 (3H, t, $CH_3$).*

**8-(3-Acétylamino-phénylsulfanyl)-4-oxo-4,5-dihydro-3*H*-pyrrolo[2,3-*c*]quinoline-1-carboxylate d'éthyle (225)**

Ce composé est préparé selon la synthèse 60, à partir de 132 mg (0.32 mmol) de 8-(3-amino-phénylsulfanyl)-4-oxo-4,5-dihydro-3*H*-pyrrolo[2,3-*c*]quinoline-1-carboxylate d'éthyle, de 89 µL (0.64 mmol) de triéthylamine et de 25 µL (0.35 mmol) de chlorure d'acétyle pour donner après précipitation dans l'eau et recristallisation dans le méthanol 70 mg (62%) de 8-(3-acétylamino-phénylsulfanyl)-4-oxo-4,5-dihydro-3*H*-pyrrolo[2,3-*c*]quinoline-1-carboxylate d'éthyle sous la forme d'un solide beige.
***LCMS (IE, m/z):*** *(M+1) 422.21*
***RMN¹H :*** *$\delta_H$ ppm 400 MHz, DMSO*
*13.09 (1H, sl, NH), 11.86 (1H, s, NH), 9.89 (1H, s, NH), 9.47 (1H, s, $CH_{arom}$), 8.01 (1H, d, $CH_{arom}$), 7.48-7.38 (4H, m, $4xCH_{arom}$), 7.21 (1H, t, $CH_{arom}$), 6.86-6.82 (1H, m, $CH_{arom}$), 4.27 (2H, q, $CH_2$), 1.97 (3H, s, $CH_3$), 1.31 (3H, t, $CH_3$).*

**Chlorhydrate de 8-(2-amino-éthylsulfanyl)-4-oxo-4,5-dihydro-3*H*-pyrrolo[2,3-*c*]quinolinc-1-carboxylate d'éthyle (226)**

Ce composé est préparé selon la synthèse 139, à partir de 331 mg (0.8 mmol) de 8-(2-*tert*-butoxycarbonylamino-éthylsulfanyl)-4-oxo-4,5-dihydro-3*H*-pyrrolo[2,3-*c*]quinoline-1-carboxylate d'éthyle et de 20 mL (40 mmol) d'une solution 2M d'acide chlorhydrique dans l'éther diéthylique pour donner après filtration 194 mg (65%) de chlorhydrate de 8-(2-amino-éthylsulfanyl)-4-oxo-4,5-dihydro-3*H*-pyrrolo[2,3-*c*]quinoline-1-carboxylate d'éthyle sous la forme d'un solide crème.
***LCMS (IE, mlz):*** *(M+1) 332.36*
***RMN¹H:*** *$\delta_H$ ppm 400 MHz, DMSO*
*13.08 (1H, sl, NH), 11.79 (1H, s, NH), 9.37 (1H, d, $CH_{arom}$), 8.05 (3H, sl, $NH_2.HCl$), 8.01 (1H, d, $CH_{arom}$), 7.52 (1H, dd, $CH_{arom}$), 7.42 (1H, d, $CH_{arom}$), 4.32 (2H, q, $CH_2$), 3.18 (2H, t, $CH_2$), 3.03-2.94 (2H, m, $CH_2$), 1.35 (3H, t, $CH_3$).*

Le 8-(2-*tert*-butoxycarbonylamino-éthylsulfanyl)-4-oxo-4,5-dihydro-3*H*-pyrrolo[2,3-*c*]quinoline-1-carboxylate d'éthyle est préparé selon la synthèse 220, à partir de 305 mg (0.8 mmol) de 8-iodo-4-oxo-4,5-dihydro-3*H*-pyrrolo[2,3-*c*]quinoline-1-carboxylate d'éthyle et de 202 µL (1.2 mmol) de 2-tert-butoxycarbonylamine-éthanethiol pour donner après purification par chromatographie sur silice (éluant dichlorométhane/méthanol 96/4) puis trituration dans l'éther diéthylique 345 mg (quantitatif) de 8-(2-*tert*-butoxycarbonylamino-éthylsulfanyl)-4-oxo-4,5-dihydro-3*H*-pyrrolo[2,3-*c*]quinoline-1-carboxylate d'éthyle sous la forme d'un solide blanc.

**4-Oxo-8-sulfo-4,5-dihydro-3*H*-pyrrolo[2,3-c]quinoline-1-carboxylate d'éthyle (227)**

Ce composé a été isolé lors de la purification par LC préparative du trifluoroacétate de 4-oxo-8-[(pipéridin-4-ylméthyl)-sulfamoyl]-4,5-dihydro-3*H*-pyrrolo[2,3-*c*]quinoline-1-carboxylate d'éthyle ( ) pour donner 4 mg (1%) de 4-oxo-8-sulfo-4,5-dihydro-3*H*-pyrrolo[2,3-*c*]quinoline-1-carboxylate d'éthyle sous la forme d'un solide beige.
***LCMS (IE, m/z):*** *(M+1) 336.92*
***RMN¹H :*** *$\delta_H$ ppm 400 MHz, DMSO*
*12.96 (1H, sl, NH), 11.70 (1H, sl, NH), 9.49 (1H, d, $CH_{arom}$), 7.97 (1H, d, $CH_{arom}$), 7.62 (1H, dd, $CH_{arom}$), 7.31 (1H, d, $CH_{arom}$), 4.32 (2H, q, $CH_2$), 1.37 (3H, t, $CH_3$).*

**4-Oxo-8-phényl-4,5-dihydro-3*H*-pyrrolo[2,3-*c*]quinoline-1-carboxylate d'éthyle (228)**

A une solution dégazée de 100 mg (0.25 mmol) de 8-bromo-4-oxo-4,5-dihydro-3*H*-pyrrolo[2,3-*c*]quinoline-1-carboxylate d'éthyle dissous dans 5 mL de dioxane anhydre sont additionnés 17 mg (0.025 mmol) de dichlorobis (triphénylphosphine)-palladium (II), 45 mg (0.37 mmol) d'acide phénylboronique et 0.6 mL (1.2 ml) d'une solution aqueuse 2M de carbonate de potassium. La réaction est agitée à 110°C pour 20 heures. De l'eau est additionnée et le produit est extrait avec de l'acétate d'éthyle. Les phases organiques sont lavées avec une solution aqueuse saturée en chlorure de sodium, séchées sur sulfate de magnésium, filtrées et évaporées. Le résidu est purifié par chromatographie sur silice (éluant dichlorométhane/méthanol 98/2) puis trituré dans le méthanol pour conduire à 16 mg (19%) de 4-oxo-8-phényl-4,5-dihydro-3*H*-pyrrolo[2,3-*c*]quinoline-1-carboxylate d'éthyle sous la forme d'un solide blanc.
***LCMS (IE, m/z):*** *(M+1) 332.96*
***RMN¹H :*** *$\delta_H$ ppm 400 MHz, $CDCl_3$*
*12.07 (1H, sl, NH), 10.76 (1H, sl, NH), 9.72 (1H, d, $CH_{arom}$), 8.07 (1H, d, $CH_{arom}$), 7.74 (2H), d, $2xCH_{arom}$), 7.63 (1H, dd, $CH_{arom}$), 7.50-7.42 (3H, m, $3xCH_{arom}$), 7.35 (1H, d, $CH_{arom}$), 4.36 (2H, q, $CH_2$), 1.35 (3H, t, $CH_3$).*

**8-(2-Chloro-phényl)-4-oxo-4,5-dihydro-3*H*-pyrrolo[2,3-*c*]quinoline-1-carboxylate d'éthyle (229)**

A une solution dégazée de 200 mg (0.49 mmol) de 8-bromo-oxo-4,5-dihydro-3*H*-pyrrolo[2,3-*c*]quinoline-1-carboxylate d'éthyle dissous dans 10 mL de dioxane anhydre sont additionnés 68 mg (0.01 mmol) de dichlorobis (triphénylphosphine)-palladium (II), 92 mg (0.59 mmol) d'acide 2-chlorophénylboronique et 1 mL (2 mmol) d'une solution aqueuse 2M de triphosphate de potassium tribasique. La réaction est agitée à 110°C pour 20 heures. De l'eau est additionnée et le produit est extrait avec de l'acétate d'éthyle. Les phases organiques sont lavées avec une solution aqueuse saturée en chlorure de sodium, séchées sur sulfate de magnésium, filtrées et évaporées. Le résidu est purifié par chromatographie sur silice (éluant dichlorométhane/méthanol 98/2) pour conduire à 27 mg (15%) de 8-(2-chloro-phényl)-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 366.86-368.80*
***RMN¹H :*** *$\delta_H$ ppm 400 MHz, DMSO*
*13.04 (1H, sl, NH), 11.80 (1H, sl, NH), 9.35 (1H, d, $CH_{arom}$), 8.00 (1H, d, $CH_{arom}$), 7.61-7.58 (1H, m, $CH_{arom}$), 7.49-7.39 (5H, m, $5xCH_{arom}$),4.25 (2H, q, $CH_2$), 1.28 (3H, t, $CH_3$).*

**8-(2-*tert*-Butoxycarbonylamino-phényl)-4-oxo-4,5-dihydro-3*H*-pyrrolo[2,3-*c*]quinoline-1-carboxylate d'éthyle (230)**

Ce composé est préparé selon la synthèse 229, à partir de 200 mg (0.49 mmol) de 8-bromo-4-oxo-4,5-dihydro-3*H*-pyrrolo[2,3-*c*]quinoline-1-carboxylate d'éthyle et de 234 mg (0.74 mmol) de 4,4,5,5-tétrainéthyl-2-(2-*tert*-butoxyaminophényl)-[1,3,2]dioxaborolane pour conduire après purification par chromatographie sur silice (éluant dichlorométhane/méthanol 96/4) puis trituration dans le méthanol à 111 mg (50%) de 8-(2-*tert*-butoxycarbonylamino-phényl)-4-oxo-4,5-dihydro-3*H*-pyrrolo[2,3-*c*]quinoline-1-carboxylate d'éthyle sous la forme d'un solide légèrement jaune.
***LCMS (IE, m/z):*** *(M+1) 448.06*
***RMN¹H*** *: $\delta_H$ ppm 400 MHz, DMSO*
*13.02 (1H, sl, NH), 11.73 (1H, sl, NH), 9.29 (1H s, $CH_{arom}$), 8.13 (1H, s, NH), 8.00 (1H, d, $CH_{arom}$), 7.52-7.47 (1H, m, $CH_{arom}$), 7.46-7.40 (2H, m, $2xCH_{arom}$), 7.37-7.37 (2H, m, $2xCH_{arom}$),7.29-7.24 (1H, m, $CH_{arom}$), 4.25 (2H, q, $CH_2$), 1.28 (3H, t, $CH_3$), 1.26 (9H, s, $3xCH_3$).*

**8-(2-Amino-phényl)-4-oxo-4,5-dihydro-3*H*-pyrrolo[2,3-c]quinoline-1-carboxylate d'éthyle (231)**

A une solution de 100 mg (0.22 mmol) de 8-(2-*tert*-butoxycarbonylamino-phényl)-4-oxo-4,5-dihydro-3*H*-pyrrolo [2,3-*c*]quinoline-1-carboxylate d'éthyle dissous dans 2 mL de dichloroéthane sont additionnés 0.5 mL d'acide trifluoroacétique. La solution est agitée à température ambiante pour 30 minutes puis les solvants sont évaporés. Le résidu est trituré avec de l'éther diisopropylique puis le solide est filtré et lavé avec du dichlorométhane. Il est repris dans 2mL de dichlorométhane puis de la triéthylamine est additionnée pour libérer l'amine. De l'eau est additionnée et le composé est extrait avec du dichlorométhane. Les phases organiques sont séchées sur sulfate de magnésium, filtrées et évaporées. Le solide est trituré avec de l'eau pour conduire à 35 mg (45%) de 8-(2-amino-phényl)-4-oxo-4,5-dihydro-3*H*-pyrrolo[2,3-c]quinoline-1-carboxylate d'éthyle sous la forme d'un solide blanc.

***LCMS (IE, m/z):*** *(M+1) 348.05*

***RMN$^1$H*** *: $\delta_H$ ppm 400 MHz, DMSO*

*13.01 (1H, sl, NH), 11.76 (1H, sl, NH), 9.27 (1H, d, $CH_{arom}$), 7.99 (1H, s, $CH_{arom}$), 7.49 (1H, d, $CH_{arom}$), 7.43 (1H, dd, $CH_{arom}$), 7.08-7.02 (2H, m, $2xCH_{arom}$),6.77 (1H, dd, $CH_{arom}$), 6.65 (1H, ddd, $CH_{arom}$), 4.78 (2H, sl, $NH_2$), 4.26 (2H, q, $CH_2$), 1.29 (3H, t, $CH_3$).*

**8-(2-Méthanesulfonylamino-phényl)-oxo-4,5-dihydro-3*H*-pyrrolo[2,3-*c*]quinoline-1-carboxylate d'éthyle (232)**

A une solution de 70 mg (0.2 mmol) de 8-(2-amino-phényl)-4-oxo-4,5-dihydro-3*H*-pyrrolo[2,3-*c*]quinoline-1-carboxylate d'éthyle dissous dans 0.3 mL de pyridine anhydre sont additionnés 24 μL (0.3 mmol) de chlorure de méthanesulfonyle. La solution est agitée à température ambiante pour 4 heures puis de l'eau est additionnée. Le composé est extrait avec de l'acétate d'éthyle. Les phases organiques sont lavées avec une solution aqueuse de sulfate de cuivre, séchées sur sulfate de magnésium, filtrées et évaporées pour conduire à 22 mg (25%) de 8-(2-méthanesulfonylamino-phényl)-4-oxo-4,5-dihydro-3*H*-pyrrolo[2,3-*c*]quinoline-1-carboxylate d'éthyle sous la forme d'un solide blanc.

***LCMS (IE, m/z):*** *(M+1) 426.01*

***RMN$^1$H*** *: $\delta_H$ ppm 400 MHz, DMSO*

*13.01 (1H, sl, NH), 11.77 (1H, sl, NH), 9.27 (1H, d, $CH_{arom}$), 8.81 (1H, sl, NH), 7.99 (1H, s, $CH_{arom}$), 7.54 (1H, dd, $CH_{arom}$), 7.49-7.44 (2H, m, $2xCH_{arom}$), 7.43-7.31 (3H, m, $3xCH_{arom}$), 4.25 (2H, q, $CH_2$), 2.79 (3H, s, $CH_3$), 1.28 (3H, t, $CH_3$).*

**8-(3-*tert*-Butoxycarbonylamino-phényl)-4-oxo-4,5-dihydro-3*H*-pyrrolo[2,3-*c*]quinoline-1-carbonate d'éthyle (233)**

Ce composé est préparé selon la synthèse 229, à partir de 200 mg (0.49 mmol) de 8-bromo-4-oxo-4,5-dihydro-3*H*-pyrrolo[2,3-*c*]quinoline-1-carboxylate d'éthyle et de 174 mg (0.74 mmol) d'acide 3-*tert*-butoxyaminophénylboronique pour conduire après purification par chromatographie sur silice (éluant dichlorométhane/méchanol 95/5) à 135 mg (61%) de 8-(3-*tert*-butoxycarbonylamino-phényl)-4-oxo-4,5-dihydro-3*H*-pyrrolo[2,3-*c*]quinoline-1-carboxylate d'éthyle sous la forme d'un solide légèrement jaune.

***LCMS (IE, m/z):*** *(M+1) 447.95*

***RMN$^1$H*** *: $\delta_H$ ppm 400 MHz, DMSO*

*13.04 (1H, sl, NH), 11.76 (1H, sl, NH), 9.55 (1H, d, $C_{arom}$), 9.44 (1H, sl, NH), 8.01 (1H, d, $CH_{arom}$), 7.85 (1H, s, $CH_{arom}$), 7.62 (1H, dd, $CH_{arom}$), 7.49 (1H, d,$CH_{arom}$), 7.45-7.35 (2H, m, $2xCH_{arom}$), 7.32-7.28 (1H, m, $CH_{arom}$), 4.33 (2H, q, $CH_2$), 1.50 (9H, s, $3xCH_3$), 1.33 (3H, t, $CH_3$).*

**Trifluoroacétate de 8-(3-amino-phényl)-4-oxo-4,5-dihydro-3*H*-pyrrolo[2,3-*c*]quinoline-1-carboxylate d'éthyle (234).**

A une solution de 120 mg (0.22 mmol) de 8-(3-*tert*-butoxycarbonylamino-phényl)-4-oxo-4,5-dihydro-3*H*-pyrrolo[2,3-*c*]quinoline-1-carboxylate d'éthyle dissous dans 2 mL de dichloroéthane sont additionnés 0.5 mL d'acide trifluoroacétique. La solution est agitée à température ambiante pour 30 minutes puis les solvants sont évaporés. Le résidu est trituré avec de l'éther diisopropylique puis le solide est filtré et lavé avec du dichlorométhane pour conduire à 86 mg (69%) de trifluoroacétate de 8-(3-amino-phényl)-4-oxo-4,5-dihydro-3*H*-pyrrolo[2,3-*c*]quinoline-1-carboxylate d'éthyle sous la forme d'un solide beige.

***LCMS (IE, m/z):*** *(M+1) 348.00*

***RMN$^1$H :*** *$\delta_H$ ppm 400 MHz, DMSO*

*13.05 (1H, sl, NH), 11.79 (1H, sl, NH), 9.58 (1H, s, $CH_{arom}$), 8.02 (1H, s, $CH_{arom}$), 7.65 (1H, d, $CH_{arom}$), 7.49 (1H, d, $CH_{arom}$), 7.38 (1H, t, $CH_{arom}$), 7.32-7.19 (2H, m, $2xCH_{arom}$), 6.98-6.90 (1H, m, $CH_{arom}$), 4.32 (2H, q, $CH_2$), 1.34 (3H, t, $CH_3$).*

**8-(3-Méthanesulfonylamino-phényl)-4-oxo-0,5-dihydro-3*H*-pyrrolo[2,3-*c*]quinoline-1-carboxylate d'éthyle (235)**

Ce composé est préparé selon la synthèse 232, à partir de 70 mg (0.15 mmol) de trifluoroacétate de 8-(3-amino-phényl)-4-oxo-4,5-dihydro-3*H*-pyrrolo[2,3-*c*]quinolino-1-carboxylate d'éthyle et de 14 µL (0.18 mmol) de chlorure de méthanesulfonyle pour conduire après purification par chromatographie sur silice (éluant acétate d'éthyle/cyclohexane 7/3) à 29 mg (45%) de 8-(3-méthanesulfonylamino-phényl)-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 425.95*
***RMN$^1$H :*** *$\delta_H$ ppm 400 MHz, DMSO*
*13.04 (1H, sl, NH), 11.77 (1H, sl, NH), 9.91 (1H, sl, NH), 9.61 (1H, s, $CH_{arom}$), 8.02 (1H, s, $CH_{arom}$), 7.67 (1H, dd, $CH_{arom}$), 7.55 (1H, d, $CH_{arom}$), 7.50 (1H, d, $CH_{arom}$), 7.49-7.41 (2H, m, 2x$CH_{arom}$), 7.22-7.17 (1H, m, $CH_{arom}$), 4.32 (2H, q, $CH_2$), 3.09 (3H, s, $CH_3$), 1.33 (3H, t, $CH_3$).*

**8-(3-Acétylamino-phényl)-4-oxo-4,5-dihydro-3*H*-pyrrolo[2,3-*c*]quinoline-1-carboxylate d'éthyle (236)**

Ce composé est préparé selon la synthèse 229, à partir de 150 mg (0.36 mmol) de 8-bromo-4-oxo-4,5-dihydro-3*H*-pyrrolo[2,3-*c*]quinoline-1-carboxylate d'éthyle et de 98 mg (0.55 mmol) d'acide 3-acétylaminophénylboronique pour conduire après purification par chromatographie sur silice (éluant dichlorométhane/méthanol 98/2) à 23 mg (16%) de 8-(3-acétylamino-phényl)-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 389.96*
***RMN$^1$H :*** *$\delta_H$ ppm 400 MHz, DMSO*
*13.04 (1H, sl, NH), 11.77 (1H, sl, NH), 10.04 (1H, sl, NH), 9.56 (1H, s, $CH_{arom}$), 8.02 (1H, s, $CH_{arom}$), 7.88 (1H, s, $CH_{arom}$), 7.61 (2H, t,2x$CH_{arom}$), 7.50 (1H, d, $CH_{arom}$), 7.42 (1H, t, $CH_{arom}$), 7.35 (1H, d, $CH_{arom}$), 4.32 (2H, q, $CH_2$), 2.08 (3H, s, $CH_3$), 1.33 (3H, t, $CH_3$).*

**8-(3-Hydroxy-phényl)-4-oxo-4,5-dihydro-3*H*-pyrrolo[2,3-*c*]quinolinc-1-carboxylate d'éthyle (237)**

Ce composé est préparé selon la synthèse 229, à partir de 150 mg (0.36 mmol) de 8-bromo-4-oxo-4,5-dihydro-3*H*-pyrrolo[2,3-*c*]quinoline-1-carboxylate d'éthyle et de 76 mg (0.55 mmol) d'acide 3-hydroxyphénylboronique pour conduire après purification par chromatographie sur silice (éluant dichlorométhane/méthanol 95/5) à 10 mg (8%) de 8-(3-hydroxy-phényl)-4-oxo-4,5-dihydro-3*H*-pyrrolo[2,3-*c*]quinoline-1-carboxylate d'éthyle sous la forme d'un solide jaune.
***LCMS (IE, mlz):*** *(M+1) 348.88*
***RMN$^1$H :*** *$\delta_H$ ppm 400 MHz, DMSO*
*12.95 (1H, sl, NH), 11.75 (1H, sl, NH), 9.58 (1H, d, $CH_{arom}$), 9.53 (1H, s, OH), 8.01 (1H, s, $CH_{arom}$), 7.67 (1H, dd, $CH_{arom}$), 7.47 (1H, d, $CH_{arom}$), 7.28 (1H, t, $CH_{arom}$), 7.16-7.09 (2H, m, 2x$CH_{arom}$), 6.78-6.74 (1H, m, $CH_{arom}$) 4.33 (2H, q, $CH_2$), 1.34 (3H, t, $CH_3$).*

**8-(3-Méthanesulfonyl-phényl)-4-oxo-4,5-dihydro-3*H*-pyrrolo[2,3-*c*]quinoline-1-carboxylate d'éthyle (238)**

Ce composé est préparé selon la synthèse 229, à partir de 140 mg (0.36 mmol) de 8-iodo-4-oxo-4,5-dihydro-3*H*-pyrrolo[2,3-*c*]quinoline-1-carboxylate d'éthyle et de 105 mg (0.55 mmol) d'acide 3-méthanesulfonylphénylboronique pour conduire après purification par chromatographie sur silice (éluant dichlorométhane/méthanol 95/5) puis trituration dans le méthanol à 30 mg (20%) de 8-(3-méthanesulfonyl-phényl)-4-oxo-4,5-dihydro-3*H*-pyrrolo[2,3-*c*]quinoline-1-carboxylate d'éthyle sous la forme d'un solide beige.
***LCMS (IE, m/z):*** *(M+1) 410.97*
***RMN$^1$H :*** *$\delta_H$ ppm 400 MHz, DMSO*
*13.08 (1H, sl, NH), 11.83 (1H, sl, NH), 9.69 (1H, s, $CH_{arom}$), 8.22 (1H, s, $CH_{arom}$), 8.09-8.01 (2H, m, 2x$CH_{arom}$), 7.92 (1H, d, $CH_{arom}$), 7.87-7.77(2H, m, 2x$CH_{arom}$), 7.54 (1H, d, $CH_{arom}$), 4.33 (2H, q, $CH_2$), 3.31 (3H, s, $CH_3$), 1.33 (3H, t, $CH_3$).*

**8-(3-Cyano-phényl)-4-oxo-4,5-dihydro-3*H*-pyrrolo[2,3-*c*]quinoline-1-carboxylate d'éthyle (239)**

Ce composé est préparé selon la synthèse 229, à partir de 200 mg (0.52 mmol) de 8-iodo-4-oxo-4,5-dihydro-3*H*-pyrrolo[2,3-*c*]quinoline-1-carboxylate d'éthyle et de 115 mg (0.78 mmol) d'acide 3-cyanophénylboronique pour conduire après purification par chromatographie sur silice (éluant dichlorométhane/méthanol 98/2) à 19 mg (10%) de 8-(3-cyano-phenyl)-4-oxo-4,5-dihydro-3*H*-pyrrolo[2,3-c]quinoline-1-carboxylate d'éthyle sous la forme d'un solide jaune pâle.
***LCMS (IE, m/z):*** *(M+1) 358.29*

***RMN$^1$H :*** *$\delta_H$ ppm 400 MHz, DMSO*
*13.08 (1H, sl, NH), 11.82 (1H, sl, NH),* 9.67 *(1H, s, $CH_{arom}$), 8.14 (1H, s, $CH_{arom}$), 8.08-8.01 (2H, m, 2x$CH_{arom}$), 7.83 (2H d,2x$CH_{arom}$), 7.73(1H, t, $CH_{arom}$), 7.51 (1H, d, $CH_{arom}$), 4.33 (2H, q, $CH_2$), 1.33 (3H, t, $CH_3$).*

**8-(3-Hydroxyméthyl-phényl)-4-oxo-4,5-dihydro-3*H*-pyrrolo[2,3-*c*]quinoline-1-carboxylate d'éthyle (240)**

Ce composé est préparé selon la synthèse 229, à partir de 200 mg (0.49 mmol) de 8-bromo-4-oxo-4,5-dihydro-3*H*-pyrrolo[2,3-*c*]quinoline-1-carboxylate d'éthyle et de 158 mg (0.73 mmol) d'acide 3-bromométhylphénylboronique pour conduire après purification par chromatographie sur silice (éluant dichlorométhane/méthanol 96/4) à 29 mg (14%) de 8-(3-hydroxyméthyl-phényl)-4-oxo-4,5-dihydro-3*H*-pyrrolo[2,3-*c*]quinoline-1-carboxylate d'éthyle sous la forme d'un solide jaune pâle.
***LCMS (IE, m/z):*** *(M+1) 363.03*
***RMN$^1$H :*** *$\delta_H$ ppm 400 MHz, DMSO*
*13.04 (1H, sl, NH), 11.76 (1H, sl, NH), 9.61 (1H, d, $CH_{arom}$), 8.01 (1H, s, $CH_{arom}$), 7.72 (1H, dd, $CH_{arom}$), 7.67 (1H, s, $CH_{arom}$), 7.59(1H, d, $CH_{arom}$), 7.49 (1H, d, $CH_{arom}$), 7.46 (1H, t, $CH_{arom}$), 7.31 (1H, d, $CH_{arom}$), 5.25 (1H, t, OH), 4.59 (2H, d, $CH_2$), 4.33 (2H, q, $CH_2$), 1.34 (3H, t, $CH_3$).*

**8-(3-Méthylaminométhyl-phényl)-4-oxo-4,5-dihydro-3*H*-pyrrolo[2,3-*c*]quinoline-1-carboxylate d'éthyle (241)**

A une solution dégazée de 200 mg (0.52 mmol) de 8-iodo-4-oxo-4,5-dihydro-3*H*-pyrrolo[2,3-*c*]quinoline-1-carboxylate d'éthyle dissous dans 10 mL de dioxane anhydre sont additionnés 68 mg (0.1 mmol) de dichlorobis(triphénylphosphine)-palladium (II), 117 mg (0.78 mmol) d'acide 3-formylphénylboronique et 1 mL (2 mmol) d'une solution aqueuse 2M de triphosphate de potassium tribasique. La réaction est agitée à 110°C pour 20 heures. De l'eau est additionnée et le produit est extrait avec de l'acétate d'éthyle. Les phases organiques sont lavées avec une solution aqueuse saturée en chlorure de sodium, séchées sur sulfate de magnésium, filtrées et évaporées. Le résidu est purifié par chromatographie sur silice (éluant dichlorométhane/méthanol 95/5) pour conduire à 107 mg d'un mélange 8/2 de 8-(3-formyl-phényl)-4-oxo-4,5-dihydro-3*H*-pyrrolo[2,3-*c*]quinoline-1-carboxylate d'éthyle et de 4-oxo-4,5-dihydro-3*H*-pyrrolo[2,3-*c*]quinoline-1-carboxylate d'éthyle. Le mélange est repris dans 0.3 mL de méthanol anhydre avant addition de 220 µL d'une solution éthanolique à 33% de méthylamine et de 300 µL (1 mmol) d'isopropoxyde de titanium. La solution est agitée 5 heures à température ambiante puis 133 mg (3.5 mmol) de borohydrure de sodium sont additionnés. L'agitation est maintenue 24 heures supplémentaires puis 2 mL d'eau sont additionnés. Le mélange réactionnel est filtré puis les solvants sont évaporés. Le résidu est purifié par chromatographie sur silice (éluant dichlorométhane/méthanol 9/1 puis chloroforme/méthanol/aminoniaque 200/16/3) pour conduire à 10 mg (35%) de 8-(3-méthylaminométhyl-phényl)-4-oxo-4,5-dihydro-3*H*-pyrrolo[2,3-*c*]quinoline-1-carboxylate d'éthyle sous la forme d'un solide blanc.
***LCMS (IE,* m/z):** *(M+1) 376.15*
***RMN$^1$H :*** *$\delta_H$ ppm 400 MHz, DMSO*
*11.75 (1H, sl, NH), 9.61 (1H, d, $CH_{arom}$), 8.01 (1H, s, $CH_{arom}$), 7.73 (1H, dd, $CH_{arom}$), 7.66 (1H, s,$CH_{arom}$), 7.58(1H, d, $CH_{arom}$), 7.49 (1H, d, $CH_{arom}$), 7.44 (1H, t, $CH_{arom}$), 7.30 (1H, d, $CH_{arom}$), 4.33 (2H, q, $CH_2$), 3.72 (2H, s, $CH_2$), 2.31 (3H, s, $CH_3$), 1.34 (3H, t, $CH_3$).*

**8-(3-Diméthylaminomethyl-phényl)-4-oxo-4,5-dihydro-3*H*-pyrrolo[2,3-*c*]quinoline-1-carboxylate d'éthyle (242)**

Dans un tube scéllé, 30 mg (0.14 mmol) d'acide 3-bromométhylphénylboronique sont dissous dans 100 µL d'une solution aqueuse à 40% de diméthylamine et la solution est chauffée à 100°C pour 4 heures. Les solvants sont évaporés et le résidu est repris dans 1.5 mL de dioxane anhydre. La solution est dégazée avant addition de 38 mg (0.14 mmol) de 8-bromo-4-oxo-4,5-dihydro-3*H*-pyrrolo[2,3-*c*]quinoline-1-carboxylate d'éthyle, 13 mg (0.028 mmol) de dichlorobis(triphénylphosphine)-palladium (II) et 200 µL (0.4 mmol) d'une solution aqueuse 2M de triphosphate de potassium tribasique. La réaction est agitée à 110°C pour 20 heures. De l'eau est additionnée et le produit est extrait avec de l'acétate d'éthyle. Les phases organiques sont lavées avec une solution aqueuse saturée en chlorure de sodium, séchées sur sulfate de magnésium, filtrées et évaporées. Le résidu est purifié par chromatographie sur silice (éluant chloroforme/méthanol/ammoniaque 90/25/4) pour conduire à 5 mg (10%) de 8-(3-diméthylaminométhyl-phényl)-4-oxo-4,5-dihydro-3*H*-pyrrolo[2,3-*c*]quinoline-1-carboxylate d'éthyle sous la forme d'un solide beige.
***LCMS (IE, m/z):*** *(M+1) 390.16*
***RMN$^1$H :*** *$\delta_H$ ppm 400 MHz, DMSO*
*13.04 (1H, sl, NH), 11.76 (1H, sl, NH), 9.62 (1H, d, $CH_{arom}$), 8.02 (1H, s, $CH_{arom}$), 7.73 (1H, dd, $CH_{arom}$), 7.67 (1H, s, $CH_{arom}$), 7.64 (1H, d, $CH_{arom}$), 7.49 (2H, d, 2x$CH_{arom}$), 7.32 (1H, d, $CH_{arom}$), 4.33 (2H, q, $CH_2$), 3.65 (2H, s, $CH_2$), 2.31 (6H, s, 2x$CH_3$),1.34 (3H, t, $CH_3$).*

**8-(4-*tert*-Butoxycarbonylamino-phényl)-4,5-dihydro-3*H*-pyrrolo[2,3-c]quinoline-1-carboxylate d'éthyle (243)**

Ce composé est préparé selon la synthèse 229, à partir de 200 mg (0.49 mmol) de 8-bromo-4-oxo-4,5-dihydro-3*H*-pyrrolo[2,3-*c*]quinoline-1-carboxylate d'éthyle et de 174 mg (0.74 mmol) d'acide 4-*tert*-butoxyaminophénylboronique pour conduire après purification par chromatographie sur silice (éluant dichlorométhane/méthanol 95/5) à 131 mg (59%) de 8-(4-*tert*-butoxycarbonylamino-phényl)-4-oxo-4,5-dihydro-3*H*-pyrrolo[2,3-*c*]quinoline-1-carboxylate d'éthyle sous la forme d'un solide légèrement jaune.
***LCMS (IE, m/z):*** *(M+1) 447.92*
***RMN¹H :*** *$\delta_H$ ppm 400 MHz, DMSO*
*13.01 (1H, sl, NH), 11.71 (1H, sl, NH), 9.58 (1H, d, $CH_{arom}$), 9.43 (1H, sl, NH), 8.00 (1H, s, $CH_{arom}$), 7.69 (1H, dd, $CH_{arom}$), 7.62 (2H, d, 2x$CH_{arom}$), 7.57 (2H, d, 2x$CH_{arom}$), 7.46 (1H, d, $CH_{arom}$), 4.33 (2H, q, $CH_2$), 1.50 (9H, s, 3x$CH_3$), 1.34 (3H, t, $CH_3$).*

**8-(4-Amino-phényl)-4-oxo-4,5-dihydro-3*H*-pyrrolo[2,3-*c*]quinoline-1-carboxylate d'éthyle (244)**

Ce composé est préparé selon la synthèse 051816, à partir de 110 mg (0.24 mmol) de 8-(4-*tert*-butoxycarbonylamino-phényl)-4-oxo-4,5-dihydro-3*H*-pyrrolo[2,3-*c*]quinoline-1-carboxate d'éthyle pour conduire après trituration dans l'éther diéthylique à 35 mg (41%) de 8-(4-amino-phényl)-4-oxo-4,5-dihydro-3*H*-pyrrolo[2,3-*c*]quinoline-1-carboxylate d'éthyle sous la forme d'un solide beige.
***LCMS (IE, m/z):*** *(M+1) 348.01*
***RMN¹H*** *: $\delta_H$ ppm 400 MHz, DMSO*
*12.97 (1H, sl, NH), 11.64 (1H, sl, NH), 9.47 (1H s, $CH_{arom}$), 7.99 (1H, s, $CH_{arom}$), 7.61 (1H, d, $CH_{arom}$), 7.46-7.357 (3H, m, 3x$CH_{arom}$),* 6.67 *(2H, d, 2x$CH_{arom}$), 5.19 (2H, s, $NH_2$), 4.33 (2H, q, $CH_2$), 1.34 (3H, t, $CH_3$).*

**8-(4-Méthanesulfonyl-phényl)-4-oxo-4,5-dihydro-3*H*-pyrrolo[2,3-*c*]quinoline-1-carboxylate d'éthyle (245)**

Ce composé est préparé selon la synthèse 229, à partir de 200 mg (0.49 mmol) de 8-bromo-4-oxo-4,5-dihydro-3*H*-pyrrolo[2,3-*c*]quinoline-1-carboxylate d'éthyle et de 110 mg (0.55 mmol) d'acide 4-méthanesulfonylphénylboronique pour conduire après purification par chromatographie sur silice (éluant dichlorométhane/méthanol 95/5) à 49 mg (32%) de 8-(4-méthanesulfonyl-phényl)-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 410.86*
***RMN¹H :*** *$\delta_H$ ppm 400 MHz, DMSO*
*13.09 (1H, sl, NH), 11.84 (1H, sl, NH), 9.73 (1H, d, $CH_{arom}$), 8.05 (2H, d, 2x$CH_{arom}$), 8.04 (1H, s, $CH_{arom}$), 7.97 (2H, d, 2x$CH_{arom}$), 7.83 (1H, dd, $CH_{arom}$), 7.54 (1H, d, $CH_{arom}$), 4.33 (2H, q, $CH_2$), 3.27 (3H, s, $CH_3$), 1.34 (3H, t, $CH_3$).*

**8-(4-Méthoxy-phényl)-oxo-4,5-dihydro-3*H*-pyrrolo[2,3-*c*]quinoline-1-carboxylate d'éthyle (246)**

Ce composé est préparé selon la synthèse 229, à partir de 200 mg (0.49 mmol) de 8-iodo-4-oxo-4,5-dihydro-3*H*-pyrrolo[2,3-*c*]quinoline-1-carboxylate d'éthyle et de 119 mg (0.78 mmol) d'acide 4-méthoxyphénylboronique pour conduire après purification par chromatographie sur silice (éluant dichlorométhane/méthanol 97/3) à 51 mg (27%) de 8-(4-méthoxy-phényl)-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 363.21*
***RMN¹H :*** *$\delta_H$ ppm 400 MHz, DMSO*
*13.01 (1H, sl, NH), 11.71 (1H, sl, NH), 9.58 (1H, d, $CH_{arom}$), 8.01 (1H, s, $CH_{arom}$), 7.69 (1H, dd, $CH_{arom}$), 7.66 (2H, d, 2x$CH_{arom}$), 7.46 (1H, d, $CH_{arom}$), 7.08 (2H, d, 2x$CH_{arom}$), 4.32 (2H, q, $CH_2$), 3.81 (3H, s, $CH_3$), 1.34 (3H, t, $CH_3$).*

**8-(4-Cyano-phényl)-4-oxo-4,5-dihydro-3*H*-pyrrolo[2,3-*c*]quinolinc-1-carboxylatc d'éthyle (247)**

Ce composé est préparé selon la synthèse 229, à partir de 200 mg (0.49 mmol) de 8-iodo-4-oxo-4,5-dihydro-3*H*-pyrrolo[2,3-*c*]quinoline-1-carboxylate d'éthyle et de 115 mg (0.78 mmol) d'acide 4-cyanophénylboronique pour conduire après purification par chromatographie sur silice (éluant dichlorométhane/méthanol 98/2) puis recristallisation dans le méthanol à 11 mg (6%) de 8-(4-cyano-phényl)-oxo-4,5-dihydro-3*H*-pyrrolo[2,3-*c*]quinoline-1-carboxylate d'éthyle sous la forme d'un solide jaune pâle.
***LCMS (IE, m/z):*** *(M+1) 358.17*
***RMN¹H :*** *$\delta_H$ ppm 400 MHz, DMSO*
*13.09 (1H, sl, NH), 11.85 (1H, sl, NH), 9.75 (1H, d, $CH_{arom}$), 8.04 (1H, s, $CH_{arom}$), 7.98 (2H, d, 2x$CH_{arom}$), 7.93 (2H,*

*d, 2x$CH_{arom}$), 7.85 (1H, dd, $CH_{arom}$), 7.53 (1H, d, $CH_{arom}$), 4.33 (2H, q, $CH_2$), 1.34 (3H, t, $CH_3$).*

**8-(2,3-Dihydro-benzo[1,4]dioxio-6-yl)-4-oxo-4,5-dihydro-3*H*-pyrrolo[2,3-*c*]quinoline-1-carboxylate d'éthyle (248)**

Ce composé est préparé selon la synthèse 229, à partir de 200 mg (0.52 mmol) de 8-iodo-4-oxo-4,5-dihydro-3*H*-pyrrolo[2,3-*c*]quinoline-1-carboxylate d'éthyle et de 141 mg (0.78 mmol) d'acide 2,3-dihydro-benzo[1,4]dioxin-6-ylboronique pour conduire après purification par chromatographie sur silice (éluant dichlorométhane/mémano! 98/2) puis recristallisation dans le méthanol à 70 mg (34%) de 8-(2,3-dihydro-benzo[1,4]dioxin-6-yl)-4-oxo-4,5-dihydro-3*H*-pyrrolo[2,3-*c*]quinoline-1-carboxylate d'éthyle sous la forme d'un solide beige.
***LCMS (IE, m/z):*** *(M+1) 391.22*
***RMN¹H :*** *$\delta_H$ ppm 400 MHz, DMSO*
*13.01 (1H, sl, NH), 11.71 (1H, sl, NH), 9.56 (1H, d, $CH_{arom}$), 8.00 (1H, s, $CH_{arom}$), 7.64 (1H, dd, $CH_{arom}$), 7.44 (1H, d, $CH_{arom}$), 7.22-7.18 (2H, m, 2x$CH_{arom}$), 6.98 (1H, d, $CH_{arom}$), 4.33 (2H, q, $CH_2$), 4.29 (4H, s, 2x$CH_2$), 1.34 (3H, t, $CH_3$).*

**8-Naphthalèn-1-yl-4-oxo-4,5-dihydro-3*H*-pyrrolo[2,3-*c*]quinolinc-1-carboxylate d'éthyle (249)**

A une solution dégazée de 200 mg (0.49 mmol) de 8-bromo-4-oxo-4,5-dihydro-3*H*-pyrrolo[2,3-*c*]quinoline-1-carboxylate d'éthyle dissous dans 4 mL de dioxane anhydre sont additionnés 68 mg (0.1 mmol) de dichlorobis(triphénylphosphine)-palladium (II), 149 mg (0.59 mmol) de bis(pinacolo)diborane et 273 µL (1.96 mmol) de triéthylamine. La réaction est agitée à 110°C pour 4 jours. De l'eau est additionnée et le produit est extrait avec de l'acétate d'éthyle. Les phases organiques sont lavées avec une solution aqueuse saturée en chlorure de sodium, séchées sur sulfate de magnésium, filtrées et évaporées. Le résidu est repris dans 5 mL de dioxane anhydre. La solution est dégazée avec un courant d'azote avant d'ajouter 81 µL (0.59 mmol) de 1-bromonaphtalène, 69 mg (0.1 mmol) de dichlorobis(triphénylphosphine)-palladium (II) et 1 mL (2 mmol) d'une solution aqueuse 2M de triphosphate de potassium tribasique. La réaction est agitée à 110°C pour 20 heures. De l'eau est additionnée et le produit est extrait avec de l'acétate d'éthyle. Les phases organiques sont lavées avec une solution aqueuse saturée en chlorure de sodium, séchées sur sulfate de magnésium, filtrées et évaporées. Le résidu est purifié par chromatographie sur silice (éluant dichlorométhane/méthanol 98/2) pour conduire à 27 mg (3% sur deux étapes) de 8-naphthalèn-1-yl-4-oxo-4,5-dihydro-3*H*-pyrrolo[2,3-*c*]quinoline-1-carboxylate d'éthyle sous la forme d'un solide beige.
***LCMS (IE, m/z):*** *(M+1) 383.18*
***RMN¹H :*** *$\delta_H$ ppm 400 MHz, $CD_3OD$*
*9.57 (1H, s, $CH_{arom}$), 8.07 (1H, s, $CH_{arom}$), 7.96 (2H, d, 2x$CH_{arom}$), 7.92 (1H, d, $CH_{arom}$), 7.60-7.43 (6H, m, 6x$CH_{arom}$), 4.26 (2H, q, $CH_2$), 1.28 (3H, t, $CH_3$).*

**4-Oxo-8-quinolin-8-yl-4,5-dihydro-3*H*-pyrrolo[2,3-*c*]quinoline-1-carboxylate d'éthyle (250)**

Ce composé est préparé selon la synthèse 229, à partir de 180 mg (0.47 mmol) de 8-iodo-4-oxo-4,5-dihydro-3*H*-pyrrolo[2,3-*c*]quinoline-1-carboxylate d'éthyle et de 244 mg (1.41 mmol) d'acide 8-quinolinylboronique pour donner, après purification par chromatographie sur silice (éluant dichlorométhane/méthanol 98/2) puis trituration dans un mélange de dichlorométhane, d'acétate d'éthyle et de méthanol 1/1/1, 2 mg (3%) de 4-oxo-8-quinolin-8-yl-4,5-dihydro-3*H*-pyrrolo [2,3-*c*]quinoline-1-carboxylate d'éthyle sous la forme d'un solide blanc
***LCMS (IE, m/z):*** *(M+1) 384.27*
***RMN¹H :*** *$\delta_H$ ppm 400 MHz, DMSO*
*13.00 (1H, sl, NH), 11.76 (1H, s, NH), 9.42 (1H, d, $CH_{arom}$), 8.90-8.88 (1H, m, $CH_{arom}$), 8.45 (1H, dd, $CH_{arom}$), 8.02 (1H, dd, $CH_{arom}$), 7.99 (1H, s, $CH_{arom}$), 7.82 (1H, dd, $CH_{arom}$), 7.75-7.70 (2H, m, 2x$CH_{arom}$), 7.58 (1H, d, $CH_{arom}$), 7.50 (1H, d $CH_{arom}$), 4.20 (2H, q, $CH_2$), 1.22 (3H, t, $CH_2$).*

**8-(2-Amino-5-méthanesulfonyl-phényl)-4-oxo-4,5-dihydro-3*H*-pyrrolo[2,3-*c*]quinoline-1-carboxylate d'éthyle (251)**

Ce composé est préparé selon la synthèse 249, à partir de 200 mg (0.49 mmol) de 8-bromo-4-oxo-4,5-dihydro-3*H*-pyrrolo[2,3-*c*]quinoline-1-carboxylale d'éthyle, de 149 mg (0.59 mmol) de bis(pinacolo)diborane et de 175 mg (0.59 mmol) de 2-iodo-4-méthanesulfonyl-phénylamine (préparée selon la méthode décrite par Lachance nicolas, Chan wing yan, J. Heterocycl. Chem., 2003, 10(2), 289-96) pour donner, après purification par chromatographie sur silice (éluant dichlorométhane/méthanol 98/2 puis 96/4), 26 mg (12%) de 8-(2-amino-5-méthanesulfonyl-phényl)-4-oxo-4,5-dihydro-3*H*-pyrrolo[2,3-*c*]quinoline-1-carboxylate d'éthyle sous la forme d'un solide beige.
***LCMS (IE, m/z):*** *(M+1) 426.05*

***RMN$^1$H :*** *$\delta_H$ ppm 400 MHz, DMSO*
*13.02 (1H, sl, NH), 11.79 (1H, sl, NH), 9.31 (1H, d, $CH_{arom}$), 8.00 (1H, s, $CH_{arom}$), 7.56 (1H, dd, $CH_{arom}$), 7.51 (1H, d, $CH_{arom}$), 7.47 (1H, d, $CH_{arom}$), 7.44 (1H, dd, $CH_{arom}$), 6.88 (1H, d, $CH_{arom}$), 5.81 (2H, sl, $NH_2$), 4.26 (2H, q, $CH_2$), 1.29 (3H, t, $CH_3$).*

**8-(1*H*-Indol-5-yl)-oxo-4,5-dihydro-3*H*-pyrrolo[2,3-*c*]quinoline-1-carboxylate d'éthyle (252)**

A une solution de 70 mg (0.15 mmol) de 8-(1-*tert*-butoxycarbonyl-1*H*-indol-5-yl)-4-oxo-4,5-dihydro-3*H*-pyrrolo [1,3-*c*]quinoline-1-carboxylate d'éthyle dissous dans 4 mL de dichloroéthane est additionné 1 mL d'acide trifluoroacétique. La solution est agitée 2 heures à température ambiante puis le précipité formé est filtré et lavé avec de l'éther diéthylique pour conduire à 26 mg (45%) de 8-(1*H*-indol-5-yl)-4-oxo-4,5-dihydro-3*H*-pyrrolo[2,3-*c*]quinoline-1-carboxylate d'éthyle sous la forme d'un solide brun.
***LCMS (IE, m/z):*** *(M+1) 372.2*
***RMN$^1$H :*** *$\delta_H$ ppm 400 MHz, DMSO*
*13.02 (1H, sl, NH), 11.73 (1H, s, NH), 11.16 (1H, s, NH), 9.61 (1H, s, $CH_{arom}$), 8.01 (1H, d, $CH_{arom}$), 7.86 (1H, s, $CH_{arom}$), 7.74 (1H, dd, $CH_{arom}$), 7.55-7.40 (3H, m, $3xCH_{arom}$), 7.38 (1H, d, $CH_{arom}$), 6.50 (1H, s, $CH_{arom}$), 4.32 (2H, q, $CH_2$), 1.33 (3H, t, $CH_3$).*

**8-(1-*tert*-Butoxycarbonyl-1*H*-indol-5-yl)-4-oxo-4,5-dihydro-3*H*-pyrrolo[2,3-*c*]quinoline-1-carboxylate d'éthyle (253)**

Ce composé est préparé selon la synthèse 229, à partir de 191 mg (0.5 mmol) de 8-iodo-4-oxo-4,5-dihydro-3*H*-pyrrolo[2,3-*c*]quinoline-1-carboxylate d'éthyle et de 343 mg (1 mmol) de 1-*tert*-butoxycarbonyl-5-(4,4,5,5-tétraméthyl-[1,3,2]dioxaborolan-2-yl)-indole pour conduire après purification par chromatographie sur silice (éluant acétate d'éthyle/méthanol 95/5) à 97 mg (41%) de 8-(1-*tert*-butoxycarbonyl-1*H*-indol-5-yl)-4-oxo-4,5-dihydro-3*H*-pyrrolo[2,3-*c*] quinoline-1-carboxylate d'éthyle sous la forme d'un solide légèrement jaune pâle.
***LCMS (IE, m/z):*** *(M+1) 472.3*
***RMN$^1$H :*** *$\delta_H$ ppm 400 MHz, DMSO*
*13.07 (1H, sl, NH), 11.79 (1H, s, NH), 9.65 (1H, s, $CH_{arom}$), 8.14 (1H, d, $CH_{arom}$), 8.03 (1H, s, $CH_{arom}$), 7.93 (1H, s, $CH_{arom}$), 7.76-7.48 (4H, m, $4xCH_{arom}$), 6.80 (1H, d, $CH_{arom}$), 4.32 (2H, q, $CH_2$), 1.66 (9H, s, $3xCH_3$), 1.33 (3H, t, $CH_3$).*

**8-(3-Méthanesulfonyl-phényl)-4-oxo-4,5-dihydro-3*H*-pyrrolo[2,3-*c*]quinoline-1-carboxylate de propyle (254)**

Ce composé est préparé selon la synthèse 229, à partir de 147 mg (0.37 mmol) de 8-iodo-4-oxo-4,5-dihydro-3*H*-pyrrolo[2,3-*c*]quinoline-1-carboxylate de propyle et de 148 mg (0.74 mmol) d'acide 3-méthanesulfonylphénylboronique pour conduire après purification par chromatographie sur silice (éluant dichlorométhane/méthanol 96/4) puis trituration dans le méthanol à 70 mg (45%) de 8-(3-méthanesulfonyl-phényl)-4-oxo-4,5-dihydro-3*H*-pyrrolo[2,3-*c*]quinoline-1-carboxylate de propyle sous la forme d'un solide légèrement jaune.
*LCMS **(IE, m/z):** (M+1) 425.15*
***RMN$^1$H :*** *$\delta_H$ ppm 400 MHz, DMSO*
*13.10 (1H, sl, NH), 11.83 (1H, sl, NH), 9.69 (1H, s, $CH_{arom}$), 8.21 (1H, s, $CH_{arom}$), 8.07 8.03 (2H, m, $2xCH_{arom}$), 7.94-7.91 (1H, m, $CH_{arom}$), 7.86-7.79 (2H, m, $2xCH_{arom}$), 7.54 (1H, d, $CH_{arom}$), 4.26 (2H, t, $OCH_2$), 3.31 (3H, s, $CH_3$), 1.78-1.72 (2H, m, $CH_2$), 0.98 (3H, t, $CH_3$).*

**8-(3-Méthanesulfonyl-phényl)-4-oxo-4,5-dihydro-3*H*-pyrrolo[2,3-*c*]quinoline-1-carboxylate d'isobutyle (255)**

Ce composé est préparé selon la synthèse 229, à partir de 152 mg (0.37 mmol) de 8-iodo-4-oxo-4,5-dihydro-3*H*-pyrrolo[2,3-*c*]quinoline-1-carboxylate d'isobutyle et de 148 mg (0.74 mmol) d'acide 3-méthanesulfonylphénylboronique pour conduire après purification par chromatographie sur silice (éluant dichlorométhane/méthanol 96/4) puis trituration dans le méthanol à 45 mg (28%) de 8-(3-méthanesulfonyl-phényl)-4-oxo-4,5-dihydro-3*H*-pyrrolo[2,3-*c*]quinoline-1-carboxylate d'isobutyle sous la forme d'un solide légèrement jaune.
***LCMS (IE, m/z):*** *(M+1) 439.18*
***RMN$^1$H :*** *$\delta_H$ ppm 400 MHz, DMSO*
*13.12 (1H, sl, NH), 11.83 (1H, sl, NH), 9. 70 (1H, s, $CH_{arom}$), 8.22 (1H, s, $CH_{arom}$), 8.05-8.03 (2H, m, $2xCH_{arom}$), 7.93-7.91 (1H, m, $CH_{arom}$), 7.86-7.80 (2H, m, $2xCH_{arom}$), 7.55 (1H, d, $CH_{arom}$), 4.09 (2H, t, $OCH_2$), 3.31 (3H, s, $CH_3$), 2.12-2.00 (1H, m, CH), 0.98 (6H, d, $2xCH_3$).*

**8-(3-Méthanesulfonyl-phényl)-4-oxo-4,5-dihydro-3*H*-pyrrolo[2,3-*c*]quinoline-1-carboxylate de cyclopentyle (256)**

Ce composé est préparé selon la synthèse 229, à partir de 224 mg (0.53 mmol) de 8-iodo-4-oxo-4,5-dihydro-3*H*-pyrrolo[2,3-*c*]quinoline-1-carboxylate de cyclopentyle et de 212 mg (1.06 mmol) d'acide 3-méthanesulfonylphénylboronique pour conduire après purification par chromatographie sur silice (éluant dichlorométhane/méthanol 96/4) puis trituration dans le méthanol à 95 mg (40%) de 8-(3-méthanesulfonyl-phényl)-4-oxo-4,5-dihydro-3*H*-pyrrolo[2,3-*c*]quinoline-1-carboxylate de cyclopentyle sous la forme d'un solide légèrement jaune.
***LCMS (IE, m/z):*** *(M+1) 451.17*
***RMN$^1$H*** *: $\delta_H$ ppm 400 MHz, DMSO*
*13.06 (1H, sl, NH), 11.80 (1H, sl, NH), 9.68 (1H, s, $CH_{arom}$), 8.20 (1H, s, $CH_{arom}$), 8.05-7.83 (3H, m, 3x$CH_{arom}$), 7.82-7.78 (2H, m, 2x$CH_{arom}$), 7.53 (1H, d, $CH_{arom}$), 5.35 (1H, sl, OCH), 3.31 (3H, s, $CH_3$), 1.92-1.59 (8H, m, 9x$CH_{pyrrol}$ et $CH_3$).*

**7-Chloro-8-(3-méthanesulfonyl-phényl)-4-oxo-4,5-dihydro-3*H*-pyrrolo[2,3-*c*]quineline-1-carboxylate d'éthyle (257)**

Ce composé est préparé selon la synthèse 229, à partir de 196 mg (0.47 mmol) de 7-chloro-8-iodo-4-oxo-4,5-dihydro-3*H*-pyrrolo[2,3-*c*]quinoline-1-carboxylate d'éthyle pour donner après purification par chromatographie sur silice (éluant dichlorométhane/méthanol 96/4) puis trituration dans du méthanol 60 mg (29%) de 7-chloro-8-(3-méthanesulfonyl-phényl)-4-oxo-4,5-dihydro-3*H*-pyrrolo[2,3-*c*]quinoline-1-carboxylate d'éthyle sous la forme d'un solide beige.
***LCMS (IE, m/z):*** *(M+1) 445.07-447.05*
***RMN$^1$H :*** *$\delta_H$ ppm 400 MHz, DMSO*
*13.16 (1H, sl, NH), 11.89 (1H, s, NH), 9.40 (1H, s, $CH_{arom}$), 8.05-7.98 (3H, m, 3x$CH_{arom}$), 7.88-7.78 (2H, m, 2x$CH_{arom}$), 7.61 (1H, s, $CH_{arom}$), 4.24 (2H, q, $CH_2$), 3.29 (3H, s, $CH_3$), 1.26 (3H, t, $CH_3$).*

**4-Oxo-8-pyridin-3-yl-4,5-dihydro-3*H*-pyrrolo[2,3-*c*]quinoline-1-carboxylate d'éthyle (258)**

A une solution dégazée de 300 mg (0.73 mmol) de 8-bromo-4-oxo-4,5-dihydro-3*H*-pyrrolo[2,3-*c*]quinoline-1-carboxylate d'éthyle dissous dans 15 mL de dioxane anhydre sont additionnés 63 mg (0.001 mmol) de dichlorobis (triphénylphosphine)-palladium (II), 197 mg (1.34 mmol) de 3-diéthylboranyl-pyridine et 7 mL d'une solution aqueuse saturée en hydrogénocarbonate de sodium. La réaction est agitée à 110°C pour 20 heures. Le solide formé est filtré pour donner 210 mg d'un mélange de 4-oxo-8-pyridin-3-yl-4,5-dihydro-3*H*-pyrrolo[2,3-*c*]quinoline-1-carboxylate d'éthyle et d'acide 4-oxo-8-pyridin-3-yl-4,5-dihydro-3*H*-pyrrolo[2,3-*c*]quinoline-1-carboxylique. Ce mélange est estérifié selon la méthode générale A d'estérification (éthanol comme alcool utilisé) pour conduire après purification par chromatographie sur silice (éluant dichlorométhane/méthanol 85/15 puis chloroforme/méthanol/ammoniaque 65/25/4) à 37 mg (15%) de 4-oxo-8-pyridin-3-yl-4,5-dihydro-3*H*-pyrrolo[2,3-*c*]quinoline-1-carboxylate d'éthyle sous la forme d'un solide beige.
***LCMS (IE, m/z):*** *(M+1) 334.10*
***RMN $^1$H :*** *$\delta_H$ ppm 400 MHz, DMSO*
*13.10 (1H, sl, NH), 11.84 (1H, sl, NH), 9.70 (1H, s, $CH_{arom}$), 8.97 (1H, s, $CH_{arom}$), 8.58 (1H s, $CH_{arom}$), 8.11 (1H, d, $CH_{arom}$), 8.04 (1H, d, $CH_{arom}$), 7.82 (1H, d, $CH_{arom}$), 7.59-7.50 (2H, m, 2x$CH_{arom}$), 4.33 (2H, q, $CH_2$), 1.33 (3H, t, $CH_3$).*

**8-(6-Chloro-pyridin-3-yl)-4-oxo-4,5-dihydro-3*H*-pyrrolo[2,3-*c*]qulnoline-1-carboxylate d'éthyle (259)**

Ce composé est préparé selon la synthèse 229, à partir de 200 mg (0.49 mmol) de 8-bromo-4-oxo-4,5-dihydro-3*H*-pyrrolo[2,3-*c*]quinoline-1-arboxylate d'éthyle et de 117 mg (0.49 mmol) de 2-chloro-5-(4,4,5,5-tetraméthyl-[1,3,2]dioxaborolan-2-yl)-pyridine pour conduire après purification par chromatographie sur silice (éluant acétate d'éthyle) puis trituration dans de l'acétonitrile à 14 mg (8%) de 8-(6-chloro-pyridin-3-yl)-4-oxo-4,5-dihydro-3*H*-pyrrolo[2,3-*c*]quinoline-1-carboxylate d'éthyle sous la forme d'un solide blanc.
***LCMS (IE, m/z):*** *(M+1) 368.00-369.96*
***RMN$^1$H*** *: $\delta_H$ ppm 400 MHz, DMSO*
*13.09 (1H, sl, NH), 11.83 (1H, sl, NH), 9.68 (1H, d, $CH_{arom}$), 8.77 (1H, d, $CH_{arom}$), 8.17 (1H, dd, $CH_{arom}$), 8.03 (1H, s, $CH_{arom}$), 7.82 (1H, dd, $CH_{arom}$), 7.67 (1H, d, $CH_{arom}$), 7.53 (1H, d, $CH_{arom}$), 4.33 (2H, q, $CH_2$), 1.34 (3H, t, $CH_3$).*

**8-(6-Méthoxy-pyridin-3-yl)-4-oxo-4,5-dihydro-3*H*-pyrrolo[2,3-*c*]quinoline-1-carboxylate d'éthyle (260)**

Ce composé est préparé selon la synthèse 229, à partir de 200 mg (0.49 mmol) de 8-bromo-4-oxo-4,5-dihydro-3*H*-pyrrolo[2,3-*c*]quinoline-1-carboxylate d'éthyle et de 112 mg (0.73 mmol) d'acide 6-méthoxy-pyridin-3-ylboronique

pour conduire après purification par chromatographie sur silice (éluant dichlorométhane/méthanol 95/5 puis 90/10) à 86 mg (48%) de 8-(6-méthoxy-pyridin-3-yl)-4-oxo-4,5-dihydro-3*H*-pyrrolo[2,3-*c*]quinoline-1-carboxylate d'éthyle sous la forme d'un solide blanc.

***LCMS (IE, m/z):*** *(M+1) 363.96*

***RMN¹H :*** *$\delta_H$ ppm 400 MHz, DMSO*

*13.04 (1H, sl, NH), 11.75 (1H, sl, NH), 9.61 (1H, d, $CH_{arom}$), 8.53 (1H, d, $CH_{arom}$), 8.04 (1H, dd, $CH_{arom}$), 8.02 (1H, s, $CH_{arom}$), 7.73 (1H, dd, $CH_{arom}$), 7.49 (1H, d, $CH_{arom}$), 6.97 (1H, d, $CH_{arom}$), 4.33 (2H, q, $CH_2$), 3.91 (3H, s, $CH_3$), 1.34 (3H, t, $CH_3$).*

**8-(2-Amino-pyrimidin-4-yl)-4-oxo-4,5-dihydro-3*H*-pyrrolo[2,3-*c*]quinoline-1-carboxylate d'éthyle (261)**

A une suspension de 204 mg (0.58 mmol) de 8-(3-diméthylamino-acryloyl)-4-oxo-4,5-dihydro-3*H*-pyrrolo[2,3-*c*]quinoline-1-carboxylate d'éthyle dans 2.3 mL de diméthylacétamide anhydre sont ajoutés 83 mg (0.87 mmol) de chlorhydrate de guanidine. Le mélange est agité à 130°C pendant 4 jours puis le solvant est évaporé. Le résidu est trituré avec du méthanol pour donner un solide qui est collecté par filtration puis lavé avec du méthanol puis de l'éther diisopropylique. Après séchage, 114 mg (56%) de 8-(2-amino-pyrimidin-4-yl)-4-oxo-4,5-dihydro-3*H*-pyrrolo[2,3-*c*]quinoline-1-carboxylate d'éthyle sont obtenus sous la forme d'un solide marron.

***LCMS (IE, m/z):*** *(M+1) 350.23*

***RMN¹H*** *: $\delta_H$ ppm 400 MHz, DMSO*

*11.64 (1H, s, NH), 9.96 (1H, s, $CH_{arom}$), 8.32 (1H, d, $CH_{arom}$), 8.11 (1H, dd, $CH_{arom}$), 7.98 (1H, s, $CH_{arom}$), 7.45 (1H, d, $CH_{arom}$), 7.09 (1H, d, $CH_{arom}$), 6.58 (2H, sl, $NH_2$), 4.33 (2H, q, $CH_2$), 1.34 (3H, t, $CH_3$).*

**8-(2-Diméthylamino-pyrimidin-4-yl)-4-oxo-4,5-dihydro-3*H*-pyrrolo[2,3-*c*]quinoline-1-carboxylate d'éthyle (262)**

A une suspension de 102 mg (0.29 mmol) de 8-(3-diméthylamino-acryloyl)-4-oxo-4,5-dihydro-3*H*-pyrrolo[2,3-*c*]quinoline-1-carboxylate d'éthyle dans 2.5 mL de diméthylacétamide anhydre sont ajoutés 80.5 mg (0.44 mmol) de sulfate de *N,N*-diméthylguanidine. Le mélange est agité à 130°C pendant 5 jours puis le solvant est évaporé. Le produit est purifié par LCMS préparative pour donner après séchage 10 mg (9%) de 8-(2-diméthylamino-pyrimidin-4-yl)-4-oxo-4,5-dihydro-3*H*-pyrrolo[2,3-*c*]quinoline-1-carboxylate d'éthyle sous la forme d'un solide marron.

***LCMS (IE, m/z):*** *(M+1) 378.15*

***RMN¹H*** *: $\delta_H$ ppm 400 MHz, DMSO*

*13.06 (1H, s, NH), 11.86 (1H, s, NH), 10.16 (1H, s, $CH_{arom}$), 8.43 (1H, d, $CH_{arom}$), 8.21 (1H, d, $CH_{arom}$), 8.02 (1H, s, $CH_{arom}$), 7.50 (1H, d, $CH_{arom}$), 7.13 (1H, d, $CH_{arom}$), 4.33 (2H, q, $CH_2$), 3.25 (6H, s, $2xCH_3$), 1.34 (3H, t, $CH_3$).*

**Formiate de 8-(2-méthylamino-pyrimidin-4-yl)-4-oxo-4,5-dihydro-3*H*-pyrrolo[2,3-*c*]quinoline-1-carboxylate d'éthyle (263)**

A une suspension de 102 mg (0.29 mmol) de 8-(3-diméthylamino-acryloyl)-4-oxo-4,5-dihydro-3*H*-pyrrolo[2,3-*c*]quinoline-1-carboxylate d'éthyle dans 2.5 mL de diméthylacétamide anhydre sont ajoutés 48 mg (0.44 mmol) de chlorhydrate de *N*-méthylguanidine. Le mélange est agité à 130°C pendant 4 jours puis le solvant est évaporé. Le produit est purifié par LCMS préparative pour donner après séchage, 31 mg (26%) de 8-(2-méthylamino-pyrimidin-4-yl)-4-oxo-4,5-dihydro-3*H*-pyrrolo[2,3-*c*]quinoline-1-carboxylate d'éthyle sous la forme d'un solide marron mono sel d'acide formique.

***LCMS (IE, m/z):*** *(M+1) 364.1*

***RMN¹H*** *: $\delta_H$ ppm 400 MHz, DMSO*

*13.07 (1H, s, NH), 11.88 (1H, s, NH), 10.06 (1H, s, $CH_{arom}$), 8.38 (1H, sl, $CH_{arom}$), 8.20 (1H, dl, $CH_{arom}$), 8.16 (1H, s, $HCO_2H$), 8.03 (1H, s, $CH_{arom}$), 7.50 (1H, d, $CH_{arom}$), 7.11 (1H, d, $CH_{arom}$), 7.04 (1H, q, NH), 4.34 (2H, ql, $CH_2$), 2.94 (3H, sl, $CH_3$), 1.35 (3H, t, $CH_3$).*

**8-(2-Ethylamino-pyrimidin-4-yl)-4-oxo-4,5-dihydro-3*H*-pyrrolo[2,3-*c*]quinoline-1-carboxylate d'éthyle (264)**

A une suspension de 102 mg (0.29 mmol) de 8-(3-diméthylamino-acryloyl)-4-oxo-4,5-dihydro-3*H*-pyrrolo[2,3-*c*]quinoline-1-carboxylate d'éthyle dans 2.5 mL de diméthylacétamide anhydre sont ajoutés 60 mg (0.44 mmol) d'hémisulfate de *N*-éthylguanidine. Le mélange est agité à 130°C pendant 5 jours puis 121 $\mu$L (0.44 mmol) d'une solution à 21% d'éthylate de sodium dans l'éthanol sont ajoutés pour accélérer la réaction. Après une nuit à 130°C, le solvant est évaporé. Le produit est purifié par LCMS préparative pour donner après séchage, 15 mg (13%) de 8-(2-éthylamino-pyrimidin-4-yl)-4-oxo-4,5-dihydro-3*H*-pyrrolo[2,3-*c*]quinoline-1-carboxylate d'éthyle sous la forme d'un solide marron.

***LCMS (IE, m/z):*** *(M+1) 378.38*

***RMN¹H** : $\delta_H$ ppm 400 MHz, DMSO*
*13.05 (1H, s, NH), 11.88 (1H, s, NH), 10.07 (1H, s, $CH_{arom}$), 8.37 (1H, sl, $CH_{arom}$), 8.18 (1H, d, $CH_{arom}$), 8.03 (1H, s, $CH_{arom}$), 7.50 (1H, d, $CH_{arom}$), 7.14-7.09 (2H, m, NH et $CH_{arom}$), 4.35 (2H, ql, $CH_2$), 3.45 (2H, m, $CH_2$), 1.36 (3H, t, $CH_3$), 1.19 (3H, t, $CH_3$).*

**4-Oxo-8-[2-(4-pyridin-2-yl-pipérazin-1-yl)-pyrimidin4-yl]-4,5-dihydro-3*H*-pyrrolo[2,3-*c*]quinoline-1-carboxylate d'éthyle (265)**

A une suspension de 102 mg (0.29 mmol) de 8-(3-diméthylamino-acryloyl)-4-oxo-4,5-dihydro-3*H*-pyrrolo[2,3-*c*] quinoline-1-carboxylate d'éthyle dans 2.5 mL de diméthylacétamide anhydre sont ajoutés 145 mg (0.44 mmol) de iodhydrate de 4-pyridin-2-ylpipérazine-1-carboximidamide. Le mélange est agité à 130°C pendant 4 jours puis le solvant est évaporé. Le produit est purifié par LCMS préparative pour donner après séchage, 25 mg (17%) de 4-oxo-8-[2-(4-pyridin-2-yl-pipérazin-1-yl)-pyrimidin-4-yl]-4,5-dihydro-3*H*-pyrrolo[2,3-*c*]quinoline-1-carboxylate d'éthyle sous la forme d'un solide marron.
***LCMS (IE, m/z):** (M+1) 496.2*
***RMN¹H** : $\delta_H$ ppm 400 MHz, DMSO*
*13.08 (1H, s, NH), 11.88 (1H, s, NH), 10.26 (1H, s, $CH_{arom}$), 8.50 (1H, d, $CH_{arom}$), 8.22 (1H, d, $CH_{arom}$), 8.16 (1H, sl, $CH_{arom}$), 8.04 (1H, s, $CH_{arom}$), 7.61-7.55 (1H, m, $CH_{arom}$), 7.52 (1H, d, $CH_{arom}$), 7.22 (1H, d, $CH_{arom}$), 6.90 (1H, d, $CH_{arom}$), 6.68 (1H, d, $CH_{arom}$), 4.39 (2H, q, $CH_2$), 4.04 (4H, sl, $2xCH_2$), 3.65 (4H, sl, $2xCH_2$), 1.39 (3H, t, $CH_3$).*

**4-Oxo-8-(2-phénylamino-pyrimidin-4-yl)-4,5-dihydro-3*H*-pyrrolo[2,3-*c*]quinoline-1-carboxylate d'éthyle (266)**

A une suspension de 88 mg (0.25 mmol) de 8-(3-diméthylamino-acryloyl)-4-oxo-4,5-dihydro-3*H*-pyrrolo[2,3-*c*] quinoline-1-carboxylate d'éthyle dans 2 mL de diméthylacétamide anhydre sont ajoutés 51 mg (0.38 mmol) de carbonate de phénylguanidine. Le mélange est agité à 130°C pendant 2 jours puis le solvant est évaporé. Le résidu est trituré avec de l'acétonitrile pour donner un solide qui est collecté par filtration puis lavé avec de l'acétonitrile puis de l'éther diisopropylique. Après séchage, 47 mg (44%) de 4-oxo-8-(2-phénylamino-pyrimidin-4-yl)-4,5-dihydro-3*H*-pyrrolo[2,3-*c*]quinoline-1-carboxylate d'éthyle sont obtenus sous la forme d'un solide marron.
***LCMS (IE, m/z):** (M+1) 426.07*
***RMN¹H** : $\delta_H$ ppm 400 MHz, DMSO*
*13.11 (1H, sl, NH), 11.94 (1H, s, NH), 10.05 (1H, s, NH), 9.63 (1H, s, $CH_{arom}$), 8.59 (1H, d, $CH_{arom}$), 8.23 (1H, dd, $CH_{arom}$), 8.05 (1H, s, $CH_{arom}$), 7.90 (2H, d, $2xCH_{arom}$), 7.55 (1H, d, $CH_{arom}$), 7.37-7.29 (3H, m, $3xCH_{arom}$), 6.96 (1H, t, $CH_{arom}$), 4.33 (2H, q, $CH_2$), 1.34 (3H, t, $CH_3$).*

**8-[2-(1*H*-Benzoimidazol-2-ylamino)-pyrimidin-4-yl]-4-oxo-4,5-dihydro-3*H*-pyrrolo[2,3-*c*]quinoline-1-carborylate d'éthyle (267)**

A une suspension de 102 mg (0.29 mmol) de 8-(3-diméthylamino-acryloyl)-4-oxo-4,5-dihydro-3*H*-pyrrolo[2,3-*c*] quinoline-1-carboxylate d'éthyle dans 2.5 mL de diméthylacétamide anhydre sont ajoutés 76 mg (0.44 mmol) de 2-guanidinobenzimidazole. Le mélange est agité à 130°C pendant 4 jours puis le solvant est évaporé. Le solide est trituré dans du méthanol puis collecté par filtration. Le solide est lavé avec du dioxane chaud puis avec du méthanol chaud pour donner après séchage, 21 mg (15%) de 8-[2-(1*H*-benzoimidazol-2-ylamino)-pyrimidin-4-yl]-4-oxo-4,5-dihydro-3*H*-pyrrolo[2,3-*c*]quinoline-1-carboxylate d'éthyle sous la forme d'un solide marron.
***LCMS (IE, m/z):** (M+1) 466.17*
***RMN¹H** : $\delta_H$ ppm 400 MHz, DMSO*
*13.16 (1H, s, NH), 12.15-11.85 (2H, m, 2xNH), 11.21 (1H, s, NH), 10.16 (1H, s, $CH_{arom}$), 8.76 (1H, d, $CH_{arom}$), 8.28 (1H, d, $CH_{arom}$), 8.08 (1H, s, $CH_{arom}$), 7.64-7.52 (2H, m, $2xCH_{arom}$), 7.44 (2H, sl, $2xCH_{arom}$), 7.07 (2H, sl, $2xCH_{arom}$), 4.26 (2H, q, $CH_2$), 1.31 (3H, t, $CH_3$).*

**8-[2-(4-Acétyl-pipérazin-1-yl)-pyrimidin-4-yl]-4-oxo-4,5-dihydro-3*H*-pyrrolo[2,3-*c*]quinoline-1-carboxylate d'éthyle (268)**

A une suspension de 102 mg (0.29 mmol) de 8-(3-diméthylamino-acryloyl)-4-oxo-4,5-dihydro-3*H*-pyrrolo[2,3-*c*] quinoline-1-carboxylate d'éthyle dans 2.5 mL de diméthylacétamide anhydre sont ajoutés 130 mg (0.44 mmol) de iodhydrate de 4-acétyl-pipérazine-1-carboxamidine. Le mélange est agité à 130°C pendant 4 jours puis le solvant est évaporé. Le produit est purifié par LCMS préparative pour donner après séchage, 32 mg (24%) de 8-[2-(4-acétyl-pipérazin-1-yl)-pyrimidin-4-yl]-4-oxo-4,5-dihydro-3*H*-pyrrolo[2,3-*c*]quinoline-1-carboxylate d'éthyle sous la forme d'un solide marron.

***LCMS (IE, m/z):*** *(M+1) 461.2*
***RMN¹H*** *: $\delta_H$ ppm 400 MHz, DMSO*
*13.08 (1H, s, NH), 11.88 (1H, s, NH), 10.26 (1H, s, $CH_{arom}$), 8.48 (1H, d, $CH_{arom}$), 8.20 (1H, d, $CH_{arom}$), 8.04 (1H, s, $CH_{arom}$), 7.51 (1H, d, $CH_{arom}$), 7.23 (1H, d, $CH_{arom}$), 4.35 (2H, q, $CH_2$), 3.93 (4H, dl, $2xCH_2$), 3.59 (4H, sl, $2xCH_2$), 2.08 (3H, s, $CH_3$), 1.37 (3H, t, $CH_3$).*

**Formiate de 8-{2-[(2-diméthylamino-éthyl)-méthyl-amino]-pyrimidin-4-yl}-4-oxo-4,5-dihydro-3*H*-pyrrolo[2,3-*c*] quinoline-1-carboxylate d'éthyle (269)**

A une suspension de 102 mg (0.29 mmol) de 8-(3-diméthylamino-acryloyl)-4-oxo-4,5-dihydro-3*H*-pyrrolo[2,3-*c*] quinoline-1-carboxylate d'éthyle dans 2.5 mL de diméthylacétamide anhydre sont ajoutés 119 mg (0.44 mmol) de iodhydrate de *N*-[2-(diméthylamino)éthyl]-*N*-méthylguanidine. Le mélange est agité à 130°C pendant 5 jours puis le solvant est évaporé. Le produit est purifié par LCMS préparative pour donner après séchage, 22 mg (16%) de 8-(2-méthylamino-pyrimidin-4-yl)-4-oxo-4,5-dihydro-3*H*-pyrrolo[2,3-*c*]quinoline-1-carboxylate d'éthyle sous la forme d'un solide marron mono sel d'acide formique.
***LCMS (IE, m/z):*** *(M+1) 435.2*
***RMN¹H*** *: $\delta_H$ ppm 400 MHz, DMSO*
*13.07 (1H, s, NH), 11.88 (1H, s, NH), 10.16 (1H, s, $CH_{arom}$), 8.44 (1H, d, $CH_{arom}$), 8.22-8.17 (2H, m, $HCO_2H$ et $CH_{arom}$), 8.03 (1H, s, $CH_{arom}$), 7.50 (1H, d, $CH_{arom}$), 7.14 (1H, d, $CH_{arom}$), 4.34 (2H, q, $CH_2$), 3.87 (2H, sl, $CH_2$), 3.24 (2H, sl, $CH_2$), 2.54 (3H, s, $CH_3$), 2.25 (6H, s, $2xCH_3$), 1.36 (3H, t, $CH_3$).*

**8-(2-Amino-thiazol-4-yl)-4-oxo-4,5-dihydro-3*H*-pyrrolo[2,3-*c*]quinoline-1-carboxylate d'éthyle (270)**

A une suspension de 57 mg (0.15 mmol) de 8-(2-bromo-acétyl)-4-oxo-4,5-dihydro-3*H*-pyrrolo[2,3-*c*]quinoline-1-carboxylate d'éthyle dans 3 mL d'éthanol sont ajoutés 17 mg (1.5 mmol) de thiourée. Le mélange est agité à 90°C pendant 3 heures puis le mélange est refroidi à température ambiante. De la triéthylamine (2 mmol) est ajoutée puis le solvant est évaporé et le produit purifié par chromatographie sur silice (chloroforme/méthanol/triéthylamine 8/2/1). Après séchage, 9 mg (17%) de 8-(2-amino-thiazol-4-yl)-4-oxo-4,5-dihydro-3*H*-pyrrolo[2,3-*c*]quinoline-1-carboxylate d'éthyle sont obtenus sous la forme d'un solide beige.
***LCMS (IE, m/z):*** *(M+1)* 355.1
***RMN¹H*** *: $\delta_H$ ppm 400 MHz, DMSO*
*12.99 (1H, sl, NH), 11.70 (1H, sl, NH), 9.62 (1H, d, $CH_{arom}$), 7.98 (1H, d, $CH_{arom}$), 7.80 (1H, dd, $CH_{arom}$), 7.38 (1H, d, $CH_{arom}$), 7.05 (2H, sl, $NH_2$), 6.78 (1H, s, $CH_{arom}$), 4.33 (2H, q, $CH_2$), 1.33 (3H, t, $CH_3$).*

A une solution de 596 mg (2 mmol) de 8-acétyl-4-oxo-4,5-dihydro-3*H*-pyrrolo[2,3-*c*]quinoline-1-carboxylate d'éthyle dans 10 mL de dioxane anhydre sont ajoutés 383 mg (2.4 mmol) de brome. Le mélange est agité à 70°C pendant 2 heures puis le mélange est refroidi à température ambiante. Le précipité est collecté par filtration puis trituré dans de l'éther diisopropylique puis du pentane pour donner après séchage, 610 mg (81%) de 8-(2-bromo-acétyl)-4-oxo-4,5-dihydro-3*H*-pyrrolo[2,3-*c*]quinoline-1-carboxylate d'éthyle sous la forme d'un solide marron.

**4-Oxo-8-(2-phénylamino-thiazol-4-yl)-4,5-dihydro-3*H*-pyrrolo[2,3-*c*]quinoline-1-carboxylate d'éthyle (271)**

A une suspension de 57 mg (0.15 mmol) de 8-(2-bromo-acétyl)-4-oxo-4,5-dihydro-3*H*-pyrrolo[2,3-*c*]quinoline-1-carboxylate d'éthyle dans 3 mL d'éthanol sont ajoutés 34 mg (1.5 mmol) de *N*-phénylthiourée. Le mélange est agité à 90°C pendant 48 heures puis le mélange est refroidi à température ambiante. De la triéthylamine (2 mmol) est ajoutée puis le solvant est évaporé et le produit purifié par chromatographie sur silice (chloroforme/méthanol/triéthylamine 8/2/1). Après séchage, 11 mg (17%) de 4-oxo-8-(2-phénylamino-thiazol-4-yl)-4,5-dihydro-3*H*-pyrrolo[2,3-*c*]quinoline-1-carboxylate d'éthyle sont obtenus sous la forme d'un solide beige.
***LCMS (IE, m/z):*** *(M+1) 431.2*
***RMN¹H*** *: $\delta_H$ ppm 400 MHz, DMSO*
*12.99 (1H, s, NH), 11.67 (1H, s, MH), 9.15 (1H, d, $CH_{arom}$), 7.95 (1H, s, $CH_{arom}$), 7.28 (2H,* m, *$2xCH_{arom}$), 7.23-7.15 (4H,* m, *$4xCH_{arom}$), 7.02 (1H, dd, $CH_{arom}$), 6.27 (1H, s, $CH_{arom}$), 4.33 (2H, q. $CH_2$), 1.36 (3H, t, $CH_3$).*

**8-[2-(2-Chloro-phénylamino)-thiazol-4-yl]-4-oxo-4,5-dihydro-3*H*-pyrrolo[2,3-*c*]quinoline-1-carboxylate d'éthyle (272)**

A une suspension de 124 mg (0.33 mmol) de 8-(2-bromo-acétyl)-4-oxo-4,5-dihydro-3*H*-pyrrolo[2,3-*c*]quinoline-1-carboxylate d'éthyle dans 3.5 mL d'éthanol sont ajoutés 74 mg (1.2 mmol) de (2-chloro-phényl)-thiourée. Le mélange est agité à 90°C pendant 6 heures puis le mélange est refroidi à température ambiante. Le solvant est évaporé puis le

produit purifié par LCMS préparative. Après séchage, 41 mg (27%) de 8-[2-(2-chloro-phénylamino)-thiazol-4-yl]-4-oxo-4,5-dihydro-3*H*-pyrrolo[2,3-*c*]quinoline-1-carboxylate d'éthyle sont obtenus sous la forme d'un solide beige.
***LCMS (IE, m/z):*** *(M+1) 465.20*
***RMN¹H :*** *$\delta_H$ ppm 400 MHz, DMSO*
*13.03 (1H, sl, NH), 11.75 (1H, s, NH), 9.78 (1H, s, $CH_{arom}$), 9.61 (1H, s, $CH_{arom}$), 7.98 (1H, sl, $CH_{arom}$), 7.76 (1H, d, $CH_{arom}$), 7.61 (2H, d, $CH_{arom}$), 7.40 (2H, d, $CH_{arom}$), 7.02 (1H, sl, $CH_{arom}$), 4.26 (2H, q, $CH_2$), 1.32 (3H, t, $CH_3$).*

**8-[2-(3,4-Dichloro-phénylamino)-thiazol-4-yl]-4-oxo-4,5-dihydro-3*H*-pyrrolo[2,3-*c*]quinoline-1-carboxylate d'éthyle (273)**

A une suspension de 124 mg (0.33 mmol) de 8-(2-bromo-acétyl)-4-oxo-4,5-dihydro-3*H*-pyrrolo[2,3-*c*]quinoline-1-carboxylate d'éthyle dans 3.5 mL d'éthanol sont ajoutés 87 mg (1.2 mmol) de (3,4-dichloro-phényl)-thiourée. Le mélange est agité à 90°C pendant 6 heures puis le mélange est refroidi à température ambiante. Le solvant est évaporé puis le produit purifié par LCMS préparative. Après séchage, 23 ring (14%) de 8-[2-(3,4-dichloro-phénylamino)-thiazol-4-yl]-4-oxo-4,5-dihydro-3*H*-pyrrolo[2,3-*c*]quinoline-1-carboxylate d'éthyle sont obtenus sous la forme d'un solide beige.
***LCMS (1E, m/z):*** *(M+1) 499.20*
***RMN¹H :*** *$\delta_H$ ppm 400 MHz, DMSO*
*13.02 (1H, sl, NH), 11.76 (1H, s, NH), 10.78 (1H, s, NH), 9.91-9.87 (1H, m, $CH_{arom}$), 8.18 (1H, dd, $CH_{arom}$), 8.04-8.00 (1H, m, $CH_{arom}$), 7.93-7.89 (2H, m, $CH_{arom}$, 7.59 (1H, d, $CH_{arom}$), 7.44 (1H, d, $C_{arom}$), 7.26 (1H, sl, $CH_{arom}$), 4.38 (2H, q, $CH_2$), 1.36 (3H, t, $CH_3$).*

**8-[2-(2,6-Dichloro-phénylamino)-thiazol-4-yl]-4-oxo-4,5-dihydro-3*H*-pyrrolo[2,3-*c*]quinoline-1-carboxytate d'éthyle (274)**

A une suspension de 124 mg (0.33 mmol) de 8-(2-bromo-acétyl)-4-oxo-4,5-dihydro-3*H*-pyrrolo[2,3-*c*]quinoline-1-carboxylate d'éthyle dans 3.5 mL d'éthanol sont ajoutés 87 mg (1.2 mmol) de (2,6-dichloro-phényl)-thiourée. Le mélange est agité à 90°C pendant 6 heures puis le mélange est refroidi à température ambiante. Le solvant est évaporé puis le produit est purifié par LCMS préparative. Après séchage, 43 mg (26%) de 8-[2-(2,6-dichloro-phénylamino)-thiazol-4-yl]-4-oxo-4,5-dihydro-3*H*-pyrrolo[2,3-*c*]quinoline-1-carboxylate d'éthyle sont obtenus sous la forme d'un solide beige.
***LCMS (IE, m/z):*** *(M+1) 499.22*
***RMN¹H :*** *$\delta_H$ ppm 400 MHz, DMSO*
*13.03 (1H, sl, NH), 11.76 (1H, s, NH), 10.58 (1H, s, $CH_{arom}$), 9.89 (1H, s, $CH_{arom}$), 8.17 (1H, d, $CH_{arom}$), 8.02 (1H, sl, $CH_{arom}$), 7.91 (1H, sl, $CH_{arom}$), 7.61 (2H, d, $CH_{arom}$), 7.44 (1H, d, $CH_{arom}$), 7.26 (1H, sl, $CH_{arom}$), 4.36 (2H, q, $CH_2$), 1.36 (3H, t, $CH_3$).*

**8-[2-(Cyclopropanecarbonyl-amino)-thiazol-4-yl]-4-oxo-4,5-dihydro-3*H*-pyrrolo[2,3-*c*]quinoline-1-carboxylate d'éthyle (275)**

A une suspension de 1.24 mg (0.33 mmol) de 8-(2-bromo-acétyl)-4-oxo-4,5-dihydro-3*H*-pyrrrolo[2,3-*c*]quinoline-1-carboxylate d'éthyle dans 3.5 mL d'éthanol sont ajoutés 57 mg (1.2 mmol) de cyclopropanecarbonyl-thiourée. Le mélange est agité à 90°C pendant 6 heures puis le mélange est refroidi à température ambiante. Le solvant est évaporé puis le produit est purifié par LCMS préparative. Après séchage, 39 mg (28%) de 8-[2-(cyclopropanecarbonyl-amino)-thiazol-4-yl]-4-oxo-4,5-dihydro-3*H*-pyrrolo[2,3-*c*]quinoline-1-carboxylate d'éthyle sont obtenus sous la forme d'un solide beige.
***LCMS (IE, mlz):*** *(M+1) 423.24*
***RMN¹H :*** *$\delta_H$ ppm 400 MHz, DMSO*
*11.82 (1H,s, NH), 9.75 (1H, s, $CH_{arom}$), 8.03 (1H, s, $C_{arom}$), 7 92 (1H, d, $CH_{arom}$), 7.46 (1H, d $CH_{arom}$), 7.34 (1H, s, $CH_{arom}$), 4.34 (2H, q, $CH_2$), 2.02 (1H, m, CH), 1.35 (3H, t, $CH_3$), 0.91 (4H, m, $4xCH_{cyclopr}$).*

**4-Oxo-8-(2-phénylsulfanylméthyl-thiazol-4-yl)-4,5-dihydro-3*H*-pyrrolo[2,3-*c*]quinoline-1-carboxylate d'éthyle (276)**

A une suspension de 57 mg (0.15 mmol) de 8-(2-bromo-acétyl)-4-oxo-4,5-dihydro-3*H*-pyrrolo[2,3-*c*]quinoline-1-carboxylate d'éthyle dans 3 mL d'éthanol sont ajoutés 38 mg (1.5 mmol) de phénylsulfanylméthyl-thiourée. Le mélange est agité à 90°C pendant 3 heures puis le mélange est refroidi à température ambiante. De la triéthylamine (2 mmol) est ajoutée puis le solvant est évaporé et le produit purifié par chromatographie sur silice (chloroforme/méthanol 98/2 puis 95/5). Après séchage, 26 mg (38%) de 4-oxo-8-(2-phénylsulfanylméthyl-thiazol-4-yl)-4,5-dihydro-3*H*-pyrrolo[2,3-*c*]quinoline-1-carboxylate d'éthyle sont obtenus sous la forme d'un solide beige.

***LCMS (IE, m/z):*** *(M+1) 462*
***RMN$^1$H :*** *$\delta_H$ ppm 400 MHz, DMSO*
*13.03 (1H, sl, NH), 11.77 (1H, sl, NH), 9.75 (1H, d, $CH_{arom}$), 8.00 (1H, d, $CH_{arom}$), 7.92 (1H, dd, $CH_{arom}$), 7.76 (1H, s, $CH_{arom}$), 7.54-7.47 (2H, m, $2xCH_{arom}$), 7.42-7.35 (3H, m, $3xCH_{arom}$), 7.22 (1H, t, $CH_{arom}$), 4.69 (2H, s, $SCH_2$), 4.33 (2H, q, $CH_2$), 1.36 (3H, t, $CH_3$).*

**4-Oxo-8-(2-pipéridin-4-yl-thiazol-4-yl)-4,5-dihydro-3*H*-pyrrolo[2,3-*c*]quinoliae-1-carboxylate d'éthyle (277)**

A une suspension de 57 mg (0.15 mmol) de 8-(2-bromo-acétyl)-4-oxo-4,5-dihydro-3*H*-pyrrolo[2,3-*c*]quinoline-1-carboxylate d'éthyle dans 3 mL d'éthanol sont ajoutés 55 mg (1.5 mmol) de 4-thiocarbamoyl-pipéridino-1-carboxylate de *tert*-butyle. Le mélange est agité à 90°C pendant 3 heures puis le mélange est refroidi à température ambiante. De la triéthylamine (2 mmol) est ajoutée puis le solvant est évaporé et le produit purifié par chromatographie sur silice (chloroforme/méthanol/triéthylamine 8/2/1). Après séchage, 11 mg (17%) de 4-oxo-8-(2-pipéridin-4-yl-thiaxol-4-yl)-4,5-dihydro-3*H*-pyrrolo[2,3-*c*]quinoline-1-carboxylate d'éthyle sont obtenus sous la forme d'un solide beige.
***LCMS (IE, m/z):*** *(M+1) 423.24*
***RMN$^1$H :*** *$\delta_H$ ppm 400 MHz, DMSO*
*11.76 (1H, sl, NH), 9.76 (1H, d, $CH_{arom}$), 8.00 (1H, s, $CH_{arom}$), 7. 95 (1H, dd, $CH_{arom}$) 7.74 (1H, s, $CH_{arom}$), 7.44 (1H, d, $CH_{arom}$), 4.35 (2H, q, $CH_2$), 3.35-3.30 (1H, m, $CH_{pipér}$), 3.16-3.10 (2H, m, $2xCH_{pipér}$), 2.80-2.72 (2H, m, $2xCH_{pipér}$), 2.13-2.06 (2H, m, $2xCH_{pipér}$), 1.77-1.68 (2H, m, $2xCH_{pipér}$), 1.36 (3H, t, $CH_3$).*

**8-(2-Méthyl-thiazol4-yl)4-oxo-4,5-dihydro-3*H*-pyrrolo[2,3-*c*]quinoline-1-carboxylate d'éthyle (278)**

A une suspension de 57 mg (0.15 mmol) de 8-(2-bromo-acétyl)-4-oxo-4,5-dihydro-3*H*-pyrmlo[2,3-*c*]quinoline-1-carboxylate d'éthyle dans 3 mL d'éthanol sont ajoutés 17 mg (1.5 mmol) de thioacétamide. Le mélange est agité à 90°C pendant 3 heures puis le mélange est refroidi à température ambiante. De la triéthylamine (2 mmol) est ajoutée puis le solvant est évaporé et le produit purifié par chromatographie sur silice (chloroforme/méthanol 95/5). Après séchage, 18 mg (34%) de 8-(2-méhyl-thiazol-4-yl)-4-oxo-4,5-dihydro-3*H*-pyrrolo[2,3-*c*]quinoline-1-carboxylate d'éthyle sont obtenus sous la forme d'un solide beige.
***LCMS (IE, m/z)****: (M+1) 354.09*
***RMN$^1$H :*** *$\delta_H$ ppm 400 MHz, DMSO*
*13.02 (1H, s, NH), 11.75 (1H, sl, NH), 9.76 (11H, d, $CH_{arom}$), 8.00 (1H, d, $CH_{arom}$), 7.95 (1H dd, $CH_{arom}$), 7.69 (1H, s. $CH_{arom}$), 7.44 (1H, d, $CH_{arom}$), 4.35 (2H, q, $CH_2$), 2.74 (3H, s, $CH_3$), 1.37 (3H, t, $CH_3$).*

**4-Oxo-8-(2-phényl-thlazol-4-yl)-4,5-dihydro-3*H*-pyrrolo[2,3-*c*]quinoline-1-carboxylate d'éthyle (279)**

A une suspension de 124 mg (0.33 mmol) de 8-(2-bromo-acétyl)-4-oxo-4,5-dihydro-3*H*-pyrrolo[2,3-*c*]quinoline-1-carboxylate d'éthyle dans 3.5 mL d'éthanol sont ajoutés 54 mg (1.2 mmol) de thiobenzamide. Le mélange est agité à 90°C pendant 6 heures puis le mélange est refroidi à température ambiante. Le solvant est évaporé puis le produit purifié par LCMS préparative. Après séchage, 29 mg (21%) de 4-oxo-8-(2-phényl-thiazol-4-yl)-4,5-dihydro-3*H*-pyrrolo [2,3-*c*]quinoline-1-carboxylate d'éthyle sont obtenus sous la forme d'un solide beige.
***LCMS (IE, m/z)****: (M+1) 416.20*
***RMN$^1$H :*** *$\delta_H$ ppm 400 MHz, DMSO*
*13.03 (1H, sl, NH), 11.79 (1H, s, NH), 9.92 (1H, s, $CH_{arom}$), 8.92-7-9.5 (4H, m, $4xCH_{arom}$), 7.67-7.48 (5H, m, $5xCH_{arom}$), 4.38 (2H, q, $CH_2$), 1.38 (3H, t, $CH_3$).*

**4-Oxo-8-[2-(*N*'-phényl-hydrazino)-thiazol-4-yl]-4,5-dihydro-3*H*-pyrrolo[2,3-*c*]quinoline-1-carboxylate d'éthyle (280)**

A une suspension de 124 mg (0.33 mmol) de 8-(2-bromo-acétyl)-4-oxo-4,5-dihydro-3*H*-pyrrolo[2,3-*c*]quinoline-1-carboxylate d'éthyle dans 3.5 mL d'éthanol sont ajoutés 66 mg (1.2 mmol) de 1-phénylthiosemicarbazide. Le mélange est agité à 90°C pendant 6 heures puis le mélange est refroidi à température ambiante. Le solvant est évaporé puis le produit purifié par LCMS préparative. Après séchage, 62 mg (42%) de 4-oxo-8-[2-(*N*'-phényl-hydrazino)-thiazol-4-yl]-4,5-dihydro-3*H*-pyrrolo[2,3-*c*]quinoline-1-carboxylate d'éthyle sont obtenus sous la forme d'un solide beige.
***LCMS (IE, m/z):*** *(M+1) 446.24*
***RMN$^1$H :*** *$\delta_H$ ppm 400 MHz, DMSO*
*12.70 (1H, sl, NH), 11.41 (1H s, NH), 9.10 (1H, s, $CH_{arom}$), 7.69 (1H, sl, $C_{arom}$), 7.04-6.95 (2H, m, $2xCH_{arom}$), 7.73-6.45 (4H, m, $4xCH_{arom}$), 7.20 (2H, d, $2xCH_{arom}$), 5.01 (1H, sl, NH), 4.0 (2H, t, $CH_2$), 1.08 (3H, t, $CH_3$).*

**8-Isoxazol-5-yl-4,5-dihydro-3*H*-pyrrolo[2,3-*c*]quinolin-1-carboxylate d'éthyle (281)**

A une solution de 64 mg (0.18 mmol) de 8-(3-diméthylamino-acryloyl)-4-oxo-4,5-dihydro-3*H*-pyrrolo[2,3-*c*] quinoline-1-carboxylate d'éthyle dans 2 mL de diméthylformamide anhydre sont ajoutés 25 mg (0.36 mmol) de chlorhydrate d'hydroxylamine. Le mélange est agité à 130°C pendant 3 heures puis le produit est précipité par addition de méthanol. Le solide est trituré dans du méthanol puis collecté par filtration. Le solide est lavé avec de l'éther diisopropylique puis du pentane pour donner après séchage, 22 mg (38%) de 8-isoxazol-5-yl-4-oxo-4,5-dihydro-3*H*-pyrrolo [2,3-*c*]quinoline-1-carboxylate d'éthyle sous la forme d'un solide beige.
***LCMS (IE, m/z):*** *(M+1) 324.04*
***RMN¹H :*** *$\delta_H$ ppm 400 MHz, DMSO*
*13.13 (1H, s, NH), 11.93 (1H, sl, NH), 9.84 (1H, d, $CH_{arom}$), 8.64 (1H, d, $CH_{arom}$), 8.05 (1H, s, $CH_{arom}$), 7.90 (1H, dd, $CH_{arom}$), 7.53 (1H, d, $CH_{arom}$), 6.87 (1H, d, $CH_{arom}$), 4.36 (2H, q, $CH_2$), 1.36 (3H, t, $CH_3$).*

**4-Oxo-8-(1*H*-pyrazol-3-yl)-4,5-dihydro-3*H*-pyrrolo[2,3-*c*]quinoline-1-carboxylate d'éthyle (282)**

A une suspension de 88 mg (0.25 mmol) de 8-(3-diméthylamino-acryloyl)-4-oxo-4,5-dihydro-3*H*-pyrrolo[2,3-*c*] quinoline-1-carboicylate d'éthyle dans 2 mL d'éthanol sont ajoutés 63 mg (1.25 mmol) d'hydrate d'hydrazine. Le mélange est agité à 80°C pendant 3 heures puis le mélange est refroidi à température ambiante. Le précipité est filtré puis trituré dans de l'éthanol. Le solide est lavé avec de l'éther diisopropylique puis du pentane pour donner après séchage, 46 mg (57%) de 4-oxo-8-(1*H*-pyrazol-3-yl)-4,5-dihydro-3*H*-pyrrolo[2,3-*c*]quinoline-1-carboxylate d'éthyle sous la forme d'un solide beige.
***LCMS (IE, m/z):*** *(M+1) 323.05*
***RMN¹H*** *: $\delta_H$ ppm 400 MHz, DMSO*
*12.82 (1H, s, 1H), 11.70 (1H, sl, NH), 9.69 (1H, sl, $CH_{arom}$), 7.99 (1H, s, $CH_{arom}$), 7.85-7.40 (2H, m, $2xCH_{arom}$), 7.42 (1H, d, $CH_{arom}$), 6.63 (1H, s, $CH_{arom}$), 4.34 (2H, q, $CH_2$), 1.36 (3H, t, $CH_3$).*

**8-(1-Méthyl-1*H*-pyrazol-3-yl)-4-oxo-4,5-dihydro-3*H*-pyrrolo[2,3-*c*]quinoline-1-carboxylate d'éthyle (283)**

A une suspension de 88 mg (0.25 mmol) de 8-(3-diméthylamino-acryloyl)4-oxo-4,5-dihydro-3*H*-pyrrolo[2,3-*c*] quinoline-1-carboxylate d'éthyle dans 2 mL d'éthanol sont ajoutés 58 mg (1.25 mmol) de méthylhydrazine. Le mélange est agité à 80°C pendant 3 heures puis le mélange est refroidi à température ambiante. Le précipité est filtré puis trituré dans de l'éthanol. Le solide est lavé avec de l'éther diisopropylique puis du pentane pour donner après séchage, 27 mg (32%) de 8-(1-méthyl-1*H*-pyrazol-3-yl)4-oxo-4,5-dihydro-3*H*-pyrrolo[2,3-*c*]quinoline-1-carboxylate d'éthyle sous la forme d'un solide beige.
***LCMS (IE, m/z):*** *(M+1) 337.05*
***RMN¹H :*** *$\delta_H$ ppm 400 MHz, DMSO*
*13.07 (1H, s, NH), 11.83 (1H, s, NH), 9.45 (1H, d, $CH_{arom}$), 8.02 (1H, s, $CH_{arom}$), 7.58-7.48 (3H, m, $3xCH_{arom}$), 6.41 (1H, d, $CH_{arom}$), 4.30 (2H, q, $CH_2$), 3.92 (3H, s, $CH_3$), 1.31 (3H, t, $CH_3$).*

**Exemple 2 : Test de mesure de l'inhibition de l'activité kinase de RET par la méthode de RET recombinante**

L'inhibition de l'activité kinase des composés de l'invention est estimée par la mesure de leur IC50 sur RET. Cette IC50 représente la concentration de composé nécessaire pour inhiber 50% de l'activité kinase de RET et est calculée à partir de la courbe de régression non linéaire dont l'équation est la suivante:

$$Y = Bottom + \frac{(Top - Bottom)}{1 + 10^{(logIC50 - X)}}$$

où:

- Y est la valeur de la luminescence
- X est le logarithme de la concentration en composé
- Bottom est la valeur Y minimale
- Top est la valeur Y maximale
- LogIC50 est la valeur de X lorsque *Y est à 50% de Top* - Bottom.

La protéine RET (Upstate #14-570MG lot PP030) est incubée dans un tampon contenant 40 mM MOPS/NaOH pH7.0 (Sigma M-8899), 1mM EDTA (Amresco 0105-100G-APP), en présence de 1,5 mg/ml de substrat poly-EY4 (Sigma #P-0275), de 30mM d'acétate de magnésium (Sigma 229768) et de 20μM d'ATP. La quantité de RET nécessaire est de 2μg/ml.

Le tampon de dilution de RET est le suivant: 20mM MOPS/NaOH pH7.0, 1mM EDTA, 5% Glycérol, 0.01% Brij35 (Sigma B4184), 0.1% 2-Mercaptoélhanol, 1mg/ml BSA (Sigma A3059-10G).

Le composé à tester est ajouté préalablement à l'enzyme de manière à atteindre un volume réactionnel de 50μl. La dilution des composés de l'invention est répartie de façon à obtenir une échelle de concentrations finales comprises entre 10nM à 30μM.

Après une incubation de la réaction de deux heures à 30°C, le taux d'ATP restant est dosé en utilisant le Kinase-Glo Luminescent Kinase Assay (Promega #V6712) selon les conditions préconisées par le vendeur. La luminescence induite est lue dans un lecteur de plaque (Envision-Perkin Elmer).

Les tableaux ci-après indiquent les valeurs d'IC50 pour des composés de formules II à IX, les substituants R, R2, R3, R4, R5, R7, R8, R12, R13, R14, R15, R16 et R17 étant définis ci-dessus. Les valeurs d'IC50 sont données par catégories :

| | |
|---|---|
| IC50 < 100nM | Catégorie A |
| 100nm < IC50 < 1μM | Catégorie B |
| 1μM < IC50 < 10μM | Catégorie C |
| 10μM < IC50 < 10μM | Catégorie D |

***<u>TABLEAU I</u>***

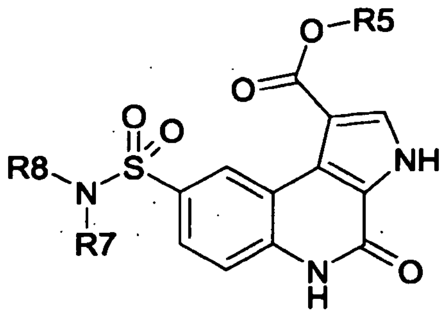

(II)

| Composé | Formule | Nom | $IC_{50}$ |
|---|---|---|---|
| 2 | 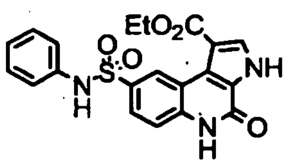 | 4-Ox-phénylsulfamoyl-4,5-dihydro-3*H*-pyrrolo[2,3-*c*]quinoline-1-carboxylate d'éthyle | A |
| 3 | 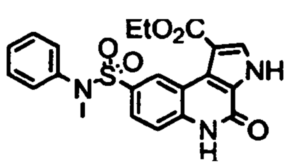 | 8-(Méthyl-phényl-sulfamoyl)-4-oxo-4,5-dihydro-3*H*-pyrrolo[2,3-*c*]quinoline-1-carboxylate d'éthyle | A |
| 4 | 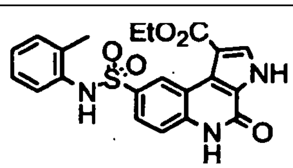 | 4-Oxo-*o*-tolylsulfamoyl-4,5-dihydro-3*H*-pyrrolo[2,3-*c*]quinoline-1-carboxylate d'éthyle | A |

(suite)

| Composé | Formule | Nom | $IC_{50}$ |
|---|---|---|---|
| 5 | 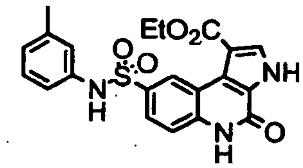 | 4-Oxo-8-*m*-tolylsulfamoyl-4,5-dihydro-3*H*-pyrrolo[2,3-*c*]quinoline-1-carboxylate d'éthyle | B |
| 6 | 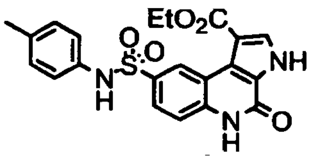 | 4-Oxo-8-*p*-tolylsulfamoyl-4,5-dihydro-3*H*-pyrrolo[2,3-*c*]quinoline-1-carboxylate d'éthyle | B |
| 7 | 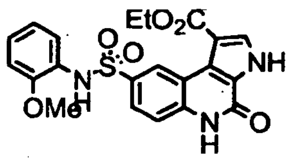 | 8-(2-Méthoxy-phénylsulfainoyl)4-oxo-4,5-dihydro-3*H*-pyrrolo[2,3-*c*]quinoline-1-carboxylate d'éthyle | B |
| 8 | 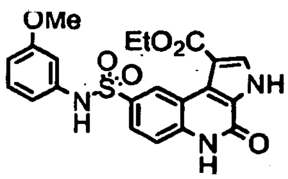 | 8-(3-Méthoxy-phénylsulfamoyl)-4-oxo-4,5-dihydro-3*H*-pyrrolo[2,3-*c*]quinoline-1-carboxylate d'éthyle | B |
| 9 | 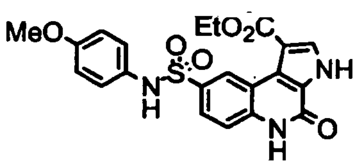 | 8-(4-Méthoxy-phénylsulfamoyl)4-oxo-4,5-dihydro-3*H*-pyrrolo[2,3-*c*]quinoline-1-carboxylate d'éthyle | B |
| 10 | 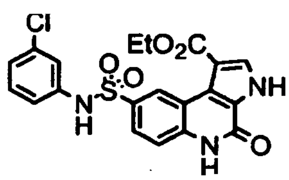 | 8-(3-Chloro-phénylsulfamoyl)-4-oxo-4,5-dhydro-3*H*-pyrrolo[2,3-*c*]quinoline-1-carboxylate d'éthyle | B |
| 11 | 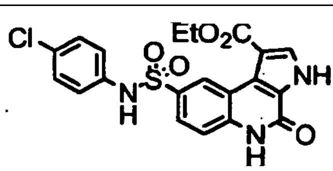 | 8-(4-Chloro-phénylsulfamoyl)-4-oxo-4,5-dihydro-3*H*-pyrrolo[2,3-*c*]quinoline-1-carboxylate d'éthyle | B |
| 12 | 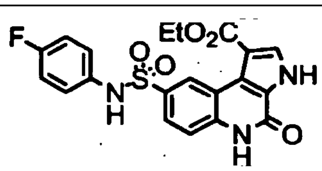 | 8-(4-Fluoro-phénylsulfamoyl)-4-oxo-4,5-dihydro-3*H*-pyrrolo[2,3-*c*]quinoline-1-carboxylate d'éthyle | A |
| 13 | 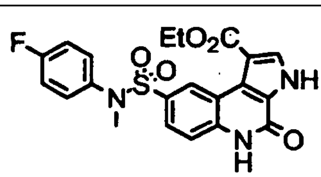 | 8-[(4-Fluoro-phényl)-méthyl-sulfamoyl)-4-oxo-4,5-dihydro-3*H*-pyrrolo[2,3-*c*]quinoline-1-carboxylate d'éthyle | A |

(suite)

| Composé | Formule | Nom | $IC_{50}$ |
|---|---|---|---|
| 103 | 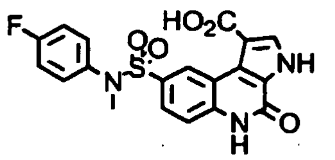 | Acide 8-[(4-fluoro-phényl)-méthyl-sulfamoyl]-4-oxo-4,5-dihydro-3*H*-pyrrolo[2,3-*c*] quinoline-1-carboxylique | D |
| 104 | 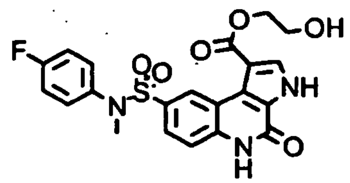 | 8-[(4-Fluoro-phényl)-méthyl-sulfamoyl]-4-oxo-4,5-dihydro-3*H*-pyrrolo[2,3-*c*]quinoline-1-carboxylate de 2-hydroxyethyle | C |
| 105 | 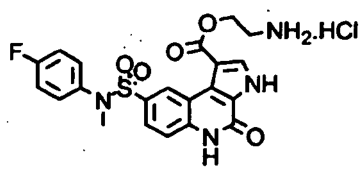 | Chlorhydrate de 8-[(4-fluoro-phényl)-méthyl-sulfamoyl]-4-oxo-4,5-dihydro-3*H*-pyrrolo [2,3-*c*]quinoline-1-carboxylate de 2-aminoéthyle | D |
| 106 | 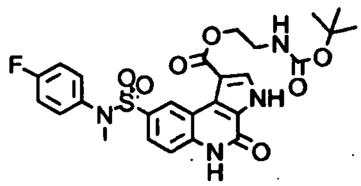 | 8-[(4-Fluoro-phényl)-méthyl-sulfamoyl]-4-oxo-4,5-dihydro-3*H*-pyrrolo[2,3-*c*]quinoline-1-carboxylate de 2-*tert*-butoxycarbonylaminoéthyle | D |
| 107 | 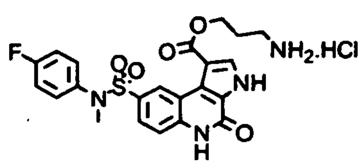 | Chlorhydrate de 8-[(4-fluoro-phényl)-méthyl-sulfamoyl]-4-oxo-4,5-dihydro-3*H*-pyrrolo [2,3-*c*]quinoline-1-carboxylate de 3-aminopropyle | D |
| 108 | 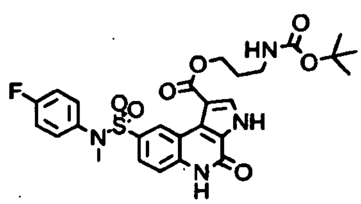 | 8-[(4-Fluoro-phényl)-méthyl-sulfamoyl]-4-oxo-4,5-dihydro-3*H*-pyrrolo[2,3-*c*]quinoline-1-carboxylate de 3-*tert*-butoxycarbonylamino- propyle | D |
| 14 | 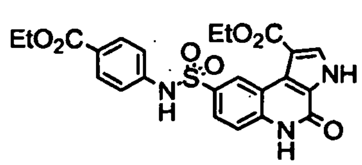 | 8-(4-Ethoxycarbonyl-phénylsulfamoyl)-4-oxo-4,5-dihydro-3*H*-pyrrolo[2,3-*c*]quinoline-1-carboxylate d'éthyle | C |
| 15 | 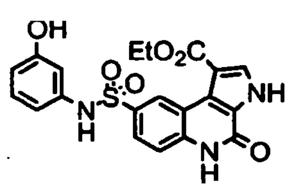 | 8-(3-Hydroxy-phénylsulfamoyl)-4-oxo-4,5-dihydro-3*H*-pyrrolo[2,3-*c*]quinoline-1-carboxylate d'éthyle | B |

(suite)

| Composé | Formule | Nom | $IC_{50}$ |
|---|---|---|---|
| 16 | 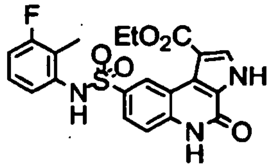 | 8-(3-Fluoro-2-méthyl-phénylsulfamoyl)-4-oxo-4,5-dihydro-3*H*-pyrrolo[2,3-*c*]quinoline-1-carboxylate d'éthyle | A |
| 17 | 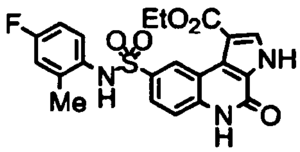 | 8-(4-Fluoro-2-méthyl-phénylsulfamoyl)-4-oxo-4,5-dihydro-3*H*-pyrrolo[2,3-*c*]quinolinie-1-carboxylate d'éthyle | A |
| 18 | 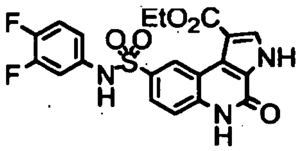 | 8-(3,4-Difluoro-phénylsulfamoyl)-4-oxo-4,5-dihydro-3*H*-pyrrolo [2,3- c] quinoline- 1-carboxylate d'éthyle | A |
| 19 | 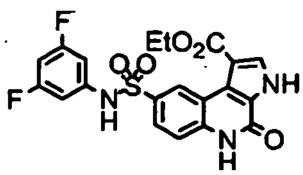 | 8-(3,5-Difluoro-phénylsulfamoyl)-4-oxo-4,5-dihydro-3*H*-pyrrolo[2,3-*c*]quinoline-1-carboxylate d'éthyle | B |
| 20 | 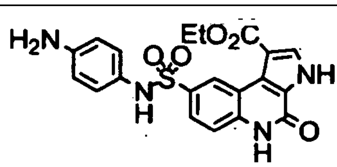 | 8-(4-Amino-phénylsulfamoyl)-4-oxo-4,5-dihydro-3*H*-pyrrolo[2,3-*c*]quinoline-1-carboxylate d'éthyle | A |
| 21 | 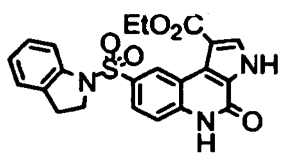 | 8-(2,3-Dihydro-indole-1-sulfonyl)-4-oxo-4,5-dihydro-3*H*-pyrrolo[2,3-*c*]quinoline-1-carboxylate d'éthyle | B |
| 22 | 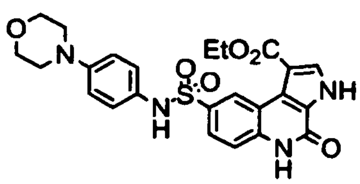 | 8-(4-Morpholin-4-yl-phénylsulfamoyl)-4-oxo-4,5-dihydro-3*H*-pyrrolo[2,3-*c*]quinoline-1-carboxylate d'éthyle | D |
| 23 | 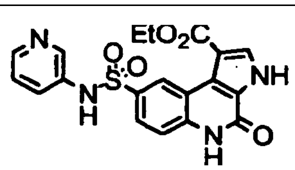 | 4-Oxo-8-(pyndin-3-ylsulfamoyl)- 4,5-dihydro-3*H*-pyrrolo[2,3-*c*]quincline-1-carboxylate d'éthyle | B |
| 24 | 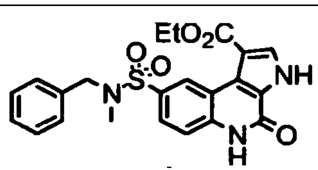 | 8-(Benzyl-méthyl-sulfamoyl)-4-oxo-4,5-dihydro-3*H*-pyrrolo[2,3-*c*]quinoline-1-carboxylate d'éthyle | B |

(suite)

| Composé | Formule | Nom | $IC_{50}$ |
|---|---|---|---|
| 25 | 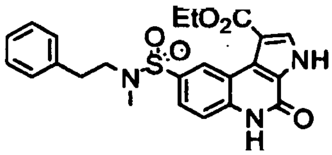 | 8-(Méthyl-phénétyl-sulfamoyl)-4-oxo-4,5-dihydro-3*H*-pyrrolo[2,3-*c*]quinoline-1-carboxylate d'éthyle | C |
| 26 | 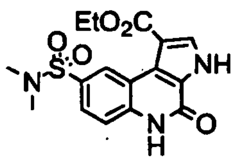 | 8-Diméthylsulfamoyl-4-oxo-4,5-dihydro-3*H*-pyrrolo[2,3-*c*]quinoline-1-carboxylate d'éthyle | B |
| 109 | 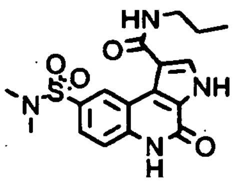 | *N*-propyl-8-diméthylsulfamoyl-4-oxo-4,5-dihydro-3*H*-pyrrolo[2,3-*c*]quinoline-1-carboxamide | D |
| 110 | 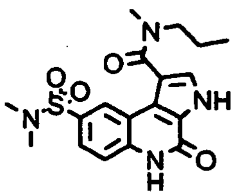 | *N*-Méthyl-*N*-propyl-8-diméthylsulfamoyl-4-oxo-4,5-dihydro-3*H*-pyrrolo[2,3-*c*]quinoline-1-carboxamide | D |
| 27 | 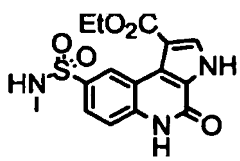 | 8-Méthylsulfamoyl-4-oxo-4,5-dihydro-3*H*-pyrrolo[2,3-*c*]quinoline-1-carboxylate d'éthyle | B |
| 28 | 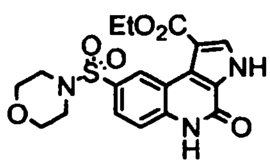 | 8-(Morpholine-4-sulfonyl)-4-oxo-4,5-dihydro-3*H*-pyrrolo[2,3-*c*]quinoline-1-carboxylate d'éthyle | B |
| 29 | 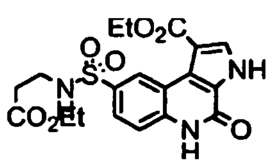 | 8-(2-Ethoxycarbonyl-éthylsulfamoyl)-4-oxo-4,5-dihydro-3*H*-pyrrolo[2,3-*c*]quinoline-1-carboxylate d'éthyle | C |
| 111 | 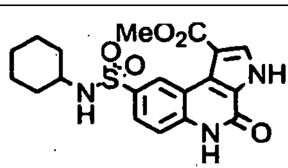 | 8-Cyclohexylsulfamoyl-4-oxo-4,5-dihydro-3*H*-pyrrolo[2,3-*c*]quinoline-1-carboxylate de méthyle | C |
| 30 | 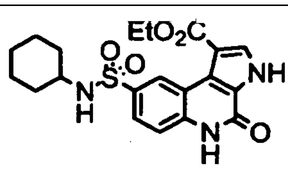 | 8-Cyclohexylsulfamoyl-4-oxo-4,5-dihydro-3*H*-pyrrolo[2,3-*c*]quinoline-1-carboxylate d'éthyle | B |

(suite)

| Composé | Formule | Nom | $IC_{50}$ |
|---|---|---|---|
| 31 | 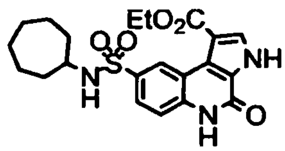 | 8-Cycloheptylsulfamoyl-4-oxo-4,5-dihydro-3*H*-pyrrolo [2,3- c] quinoline- 1- carboxylate d'éthyle | B |
| 32 | 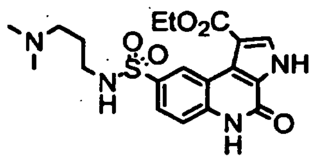 | 8-(3-Diméthylamino-propylsulfamoyl)-4-xo-4,5-dihydro-3*H*-pyrrolo[2,3-*c*]quinoline-1-carboxylate d'éthyle | B |
| 33 | 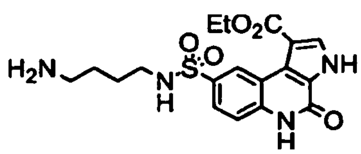 | 8-(4-Amino-butylsulfamoyl)-4-oxo-4,5-dihydro-3*H*-pyrrolo[2,3-*c*]quinoline-1-carboxylate d'éthyle | B |
| 34 | 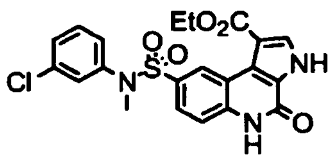 | 8-[(3-Chloro-phényl)-méthyl-sulfamoyl)-4-oxo-4,5-dihydro-3*H*-pyrrolo[2,3-*c*]quinoline-1-carboxylate 4'éthyle | B |
| 91 | 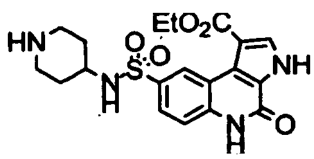 | 4-Oxo-8-(pipéridin-4-ylsulfamoyl)-4,5-dihydro-3*H*-pyrrolo[2,3-*c*]quinoline-1-carboxylate d'éthyle | B |
| 112 | 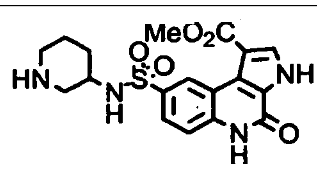 | 4-Oxo-8-(pipéridin-3-ylsulfamoyl)-4,5-dihydro-3*H*-pyrrolo[2,3-*c*]quinoline-1-carboxylate de méthyle | C |
| 113 | 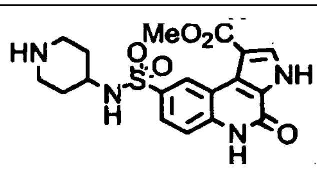 | 4-Oxo-8-(pipéridin-4-ylsulfamoyl)-4,5-dihydro-3*H*-pyrrolo[2,3-*c*]quinoline-1-carboxylate de méthyle | B |
| 114 | 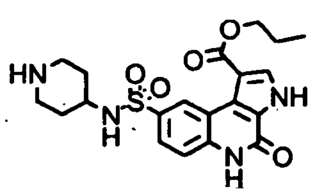 | 4-Oxo-8-(pipéridin-4-ylsulfmoyl)-4,s-dihydro-3*H*-pyrrolo[2,3-*c*]quinoline-1-carboxylate de propyle | A |
| 115 | 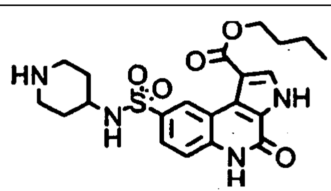 | 4-Oxo-8-(pipéridin-4-ylsulfamoyl)-4,5-dihydro-3*H*-pyrrolo[2,3-*c*]quinoline-1-carboxylate de butyle | A |

(suite)

| Composé | Formule | Nom | $IC_{50}$ |
|---|---|---|---|
| 116 | 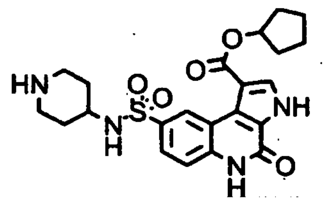 | 4-Oxo-8-(pipéridin-4-ylsulfamoyl)-4,5-dihydro-3*H*-pyrrolo[2,3-*c*]quinoline-1-carboxylate de pentyle | A |
| 117 | 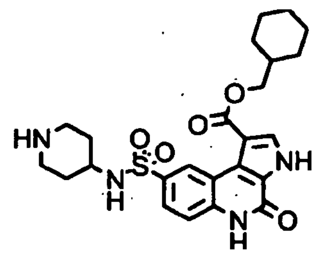 | 4-Oxo-8-(pipéridin-4-ylsulfamoyl)-4,5-dihydro-3*H*-pyrrolo[2,3-*c*]quinoline-1-carboxylate de méthylecyclohexyle | B |
| 118 | 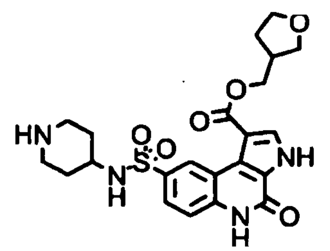 | 4-Oxo-8-(pipéridin-4-ylsulfamoyl)-4,5-dihydro-3*H*-pyrrolo[2,3-*c*]quinoline-1-carboxylate de méthyltétrahydrofuran-3-yle | B |
| 119 | 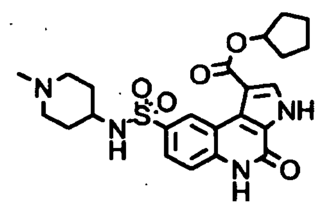 | 8-(1-Méthyl-pipéridin-4-ylsulfamoyl)-4-oxo-4,5-dihydro-3*H*-pyrrolo[2,3-*c*]quinoline-1-carboxylate de cyclopentyle | B |
| 120 | 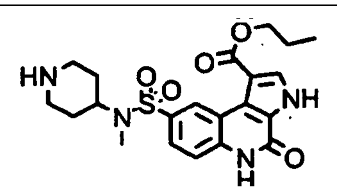 | 8-(Méthyl-pipéridin-4-yl-sulfamoyl)-4-oxo-4,5-dihydro-3*H*-pyrrolo[2,3-*c*]quinoline-1-carboxylate de propyle | A |
| 121 | 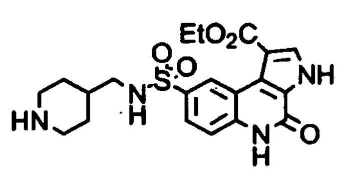 | 4-Oxo-8-[(pipéridin-4-ylméthyl)-sulfamoyl]-4,5-dihydro-3*H*-pyrrolo[2,3-*c*]quinoline-1-carboxylate d'éthyle | C |
| 122 | 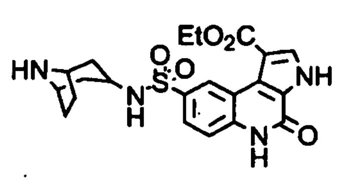 | 8-(8-Aza-bicyclo[3.2.1]oct-3-ylsulfamoyl)-4-oxo-4,5-dihydro-3*H*-pyrrolo[2,3-*c*]quinoline-1-carboxylate d'éthyle | A |

(suite)

| Composé | Formule | Nom | $IC_{50}$ |
|---|---|---|---|
| 92 | 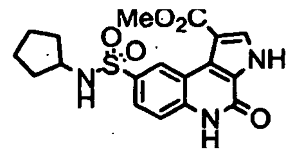 | 8-Cyclopentylsulfamoyl-4-oxo-4,5-dihydro-3*H*-pyrrolo[2,3-*c*]quinoline-1-carboxylate de méthyle | C |
| 93 | 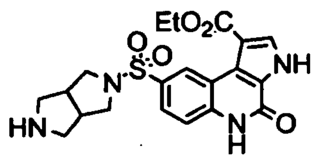 | 8-(Hexahydro-pyrrolo[3,4-*c*]pyrrole-2-sulfonyl)-4-oxo-4,5-dihydro-3*H*-pyrrolo [2,3-*c*]quinoline-1-carboxylate d'éthyle | B |
| 94 | 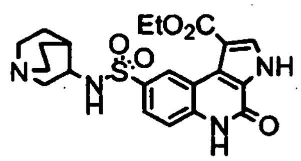 | 8-(1-Azabicyclo[2.2.2]oct-3-ylsulfamoyl)-4-oxo-4,5-dihydro-3*H*-pyrrolo[2,3-*c*]quinoline-1-carboxylate d'éthyle | B |
| 95 | 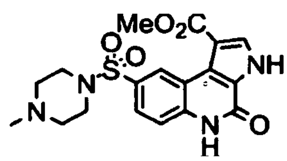 | 8-(4-Méthyl-pipérazine-1-sulfonyl)-4-oxo-4,5-dihydro-3*H*-pyrrolo[2,3-*c*]quinoline-1-carboxylate d'éthyle | C |
| 96 | 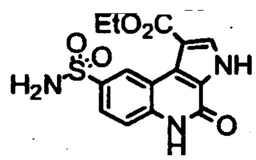 | 4-Oxo-8-sulfamoyl-4,5-dihydro-3*H*-pyrrolo [2,3-*c*]quinoline-1-carboxylate d'éthyle | B |
| 97 | 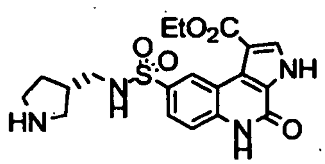 | 4-Oxo-8-[(pyrrolidin-3-méthyl)-sulfamoyl]-4,5-dihydro-3*H*-pyrrolo[2,3-*c*]quinolino-1-carboxylate d'éthyle | B |
| 98 | 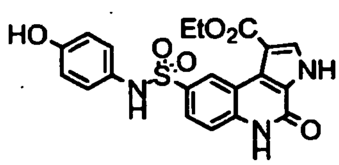 | 4-Oxo-8-(4-hydroxy-phénylsulfamoyl)-4,5-dihydro-3*H*-pyrrolo[2,3-*c*]quinoline-1-carboxylate d'éthyle | A |
| 123 | 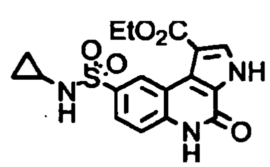 | 8-Cyclopropylsulfamoyl-4-oxo-4,5-dihydro-3*H*-pyrrolo[2,3-*c*]quinoline-1-carboxylate d'éthyle | B |
| 124 | 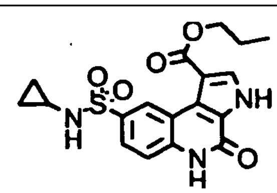 | 8-Cyclopropylsulfamoyl-4-oxo-4,5-dihydro-3*H*-pyrrolo[2,3-*c*]quinoline-1-carboxylate de propyle | B |

(suite)

| Composé | Formule | Nom | $IC_{50}$ |
|---|---|---|---|
| 125 | 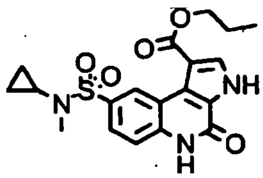 | 8-(Cyclopropyl-méthyl-sulfamoyl)-4-oxo-4,5-dihydro-3*H*-pyrrolo[2,3-*c*]quinoline-1-carboxylate de propyle | A |
| 126 | 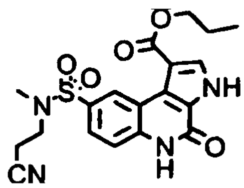 | 8-[(2-Cyano-éthyl)-méthyl-sulfamoyl]-4-oxo-4,5-dihydro-3*H*-pyrrolo[2,3-*c*]quinoline-1-carboxylate de propyle | B |
| 127 | 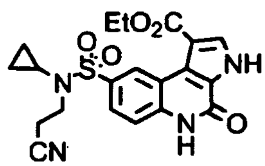 | 8-[(2-Cyano-éthyl)-cyclopropyl-sulfamoyl]-4-oxo-4,5-dihydro-3*H*-pyrrolo[2,3-*c*]quinoline-1-carboxylate d'éthyle | A |
| 128 | 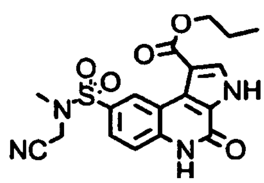 | 8-(Cyanométhyl-méthyl-sulfamoyl)-4-oxo-4,5-dihydro-3*H*-pyrrolo[2,3-*c*]quinoline-1-carboxylate de propyle | B |
| 129 | 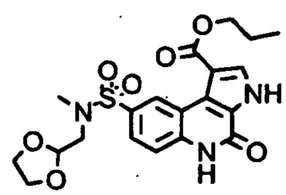 | 8-([1,3]Dioxolan-2-ylméthyl-méthyl-sulfamoyl)-4-oxo-4,5-dihydro-3*H*-pyrrolo [2,3-*c*]quinoline-1-carboxylate de propyle | B |
| 130 | 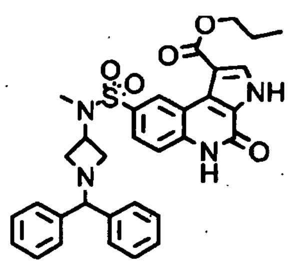 | 8-[(1-Benzhydryl-azétidin-3-yl)-méthyl-sulfamoyl]-4-oxo-4,5-dihydro-3*H*-pyrrolo [2,3-*c*]quinoline-1-carboxylate de propyle | B |
| 131 | 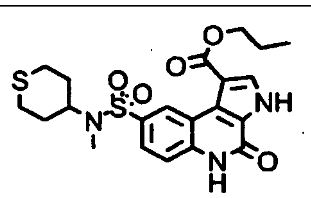 | 8-[Méthyl-(tétrahydro-thiopyran-4-yl)-sulfamoyl]-4-oxo-4,5-dihydro-3*H*-pyrrolo [2,3-*c*]quinoline-1-carboxylate de propyle | B |
| 132 | 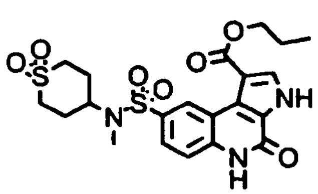 | 8-[(1,1-Dioxo-hexahydro-116-thiopyran-4-yl)-méthyl-sulfamoyl]-4-oxo-4,5-dihydro-3*H*-pyrrolo [2,3- c] quinoline- 1- carboxylate de propyle | C |

(suite)

| Composé | Formule | Nom | $IC_{50}$ |
|---|---|---|---|
| 133 | 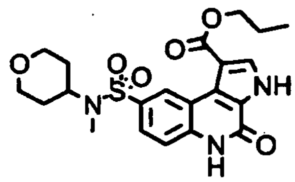 | 8-[Méthyl-(tétrahydro-pyran-4-yl)-sulfamoyl]-4-oxo-4,5-dihydro-3*H*-pyrrolo[2,3-*c*] quinoline-1-carboxylate de propyle | A |
| 134 | 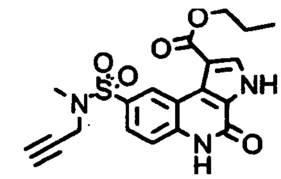 | 8-(Méthyl-prop-2-ynyl-sulfamoyl)-4-oxo-4,5-dihydro-3*H*-pyrrolo[2,3-*c*]quinoline-1-carboxylate de propyle | B |
| 135 | 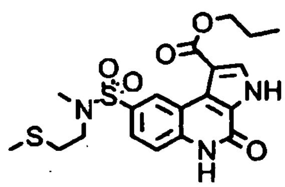 | 8-[Méthyl-(2-méthylsulfanyl-éthyl)-sulfamoyl]-4-oxo-4,5-dihydro-3*H*-pyrrolo[2,3-*c*]quinoline-1-carboxylate de propyle | B |
| 136 | 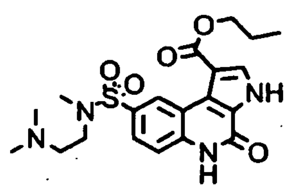 | 8-[(2-Diméthylamino-éthyl)-méthyl-sulfamoyl]4-oxo-4,5-dihydro-3*H*-pyrrolo [2,3-*c*]quinoline-1-carboxylate de propyle | A |
| 137 | 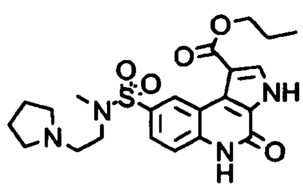 | 8-[Méthyl-(2-pyrrolidin-1-yl-éthyl)-sulfamoyl]-4-oxo-4,5-dihydro-3*H*-pyrrolo[2,3-*c*]quinoline-1-carboxylate de propyle | B |
| 138 | 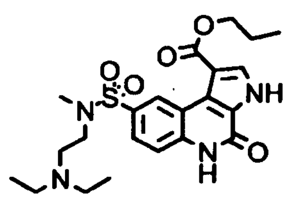 | 8-[(2-Diéthylamino-éthyl)-méthyl-sulfamoyl]-4-oxo-4,5-dihydro-3*H*-pyrrolo[2,3-*c*] quinoline-1-carboxylate de propyle | B |
| 139 | 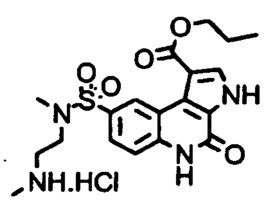 | Chlorhydrate de 8-[méthyl-(2-méthylamino-éthyl)-sulfamoyl]-4-oxo-4,5-dihydro-3*H*-pyrrolo[2,3-*c*]quinoline-1-carboxylate de propyle | A |

(suite)

| Composé | Formule | Nom | $IC_{50}$ |
|---|---|---|---|
| 140 | 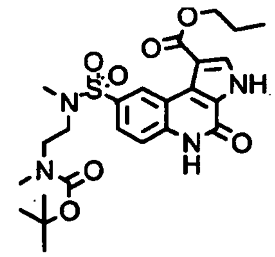 | 8-{[2-(*tert*-Butoxycarbonyl-méthyl-amino)-éthyl]-méthyl-sulfamoyl}-4-oxo-4,5-dihydro-3*H*-pyrrolo[2,3-*c*]quinoline-1-carboxylate de propyle | C |
| 141 | 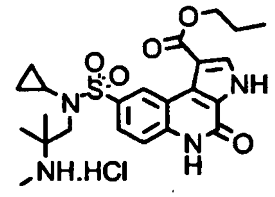 | Chlorhydrate de 8-[cyclopropyl-(2-méthyl-2-méthylamino-propyl)-sulfamoyl]-4-oxo-4,5-dihydro-3*H*-pyrrolo[2,3-*c*]quinoline-1-carboxylate de propyle | B |
| 142 | 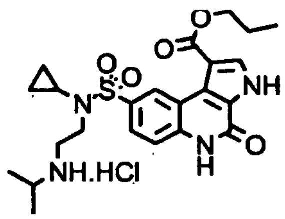 | Chlorhydrate de 8-[cyclopropyl-(2-isopropylamino-éthyl)-sulfamoyl]-4-oxo-4,5-dihydro-3*H*-pyrrolo[2,3-*c*]quinoline-1-carboxylate de propyle | B |
| 143 | 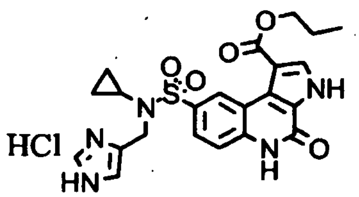 | Chlorhydrate de 8-[cyclopropyl-(1*H*-imidazol-4-ylméthyl)-sulfamoyl]-4-oxo-4,5-dihydro-3*H*-pyrrolo[2,3-*c*]quinoline-1-carboxylale de propyle | B |
| 144 | 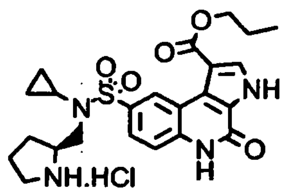 | Chlorhydrate de (S) 8-(cyclopropyl-pyrrolidin-2-ylméthyl-sulfamoyl)-4-oxo-4,5-dihydro-3*H*-pyrrolo[2,3-*c*]quinoline-1-carboxylate de propyle | A |
| 145 | 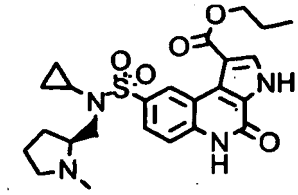 | (S) 8-[Cyclopropyl-(1-méthyl-pyrrolidin-2-ylméthyl)-sulfamoyl]-4-oxo-4,5-dihydro-3*H*-pyrrolo[2,3-*c*]quinoline-1-carboxylale de propyle | B |
| 146 | 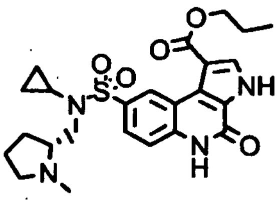 | (R) 8-[Cyclopropyl-(1-méthyl-pyrrolidin-2-ylméthyl)-sulfamoyl]-4-oxo-4,5-dihydro-3*H*-pyrrolo[2,3-*c*]quinoline-1-carboxylate de propyle | B |

(suite)

| Composé | Formule | Nom | IC$_{50}$ |
|---|---|---|---|
| 147 | 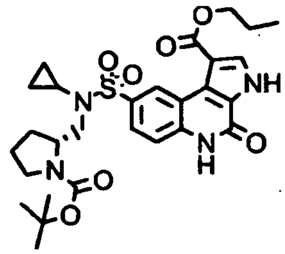 | (R) 8-[(1-*tert*-Butoxycarbonyl-pyrrolidin-2-ylméthyl)-cyclopropyl-sulfamoyl]4-oxo-4,5-dihydro-3*H*-pyrrolo[2,3-*c*]quinoline-1-carboxylate de propyle | C |
| 148 | 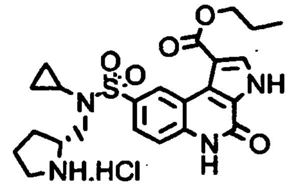 | Chlorhydrate de (R) 8-(cyclopropyl-pyrrolidin-2-ylméthyl-sulfamoyl)-4-oxo-4,5-dihydro-3*H*-pyrrolo[2,3-*c*]quinoline-1-carboxylate de propyle | B |
| 149 | 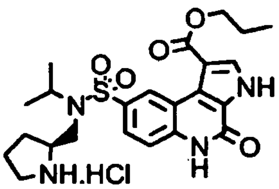 | Chlorhydrate de (S) 8-(isopropyl-pyrrolidin-2-ylméthyl-sulfamoyl)-4-oxo-4,5-dihydro-3*H*-pyrrolo[2,3-*c*]quinoline-1-carboxylate de propyle | A |
| 150 | 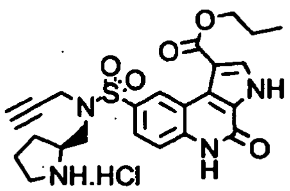 | Chlorhydrate de (S) 4-Oxo-8-(prop-2-ynyl-pyrrolidin-2-ylméthyl-sulfamoyl)4,5-dihydro-3*H*-pyrrolo[2,3-*c*]quinoline-1-carboxylate de propyle | A |
| 151 | 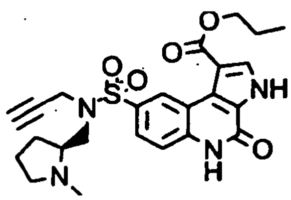 | (S) 8-[(1-Méthyl-pyrrolidin-2-ylméthyl)-prop-2-ynyl-sulfamoyl]-4-oxo-4,5-dihydro-3*H*-pyrrolo[2,3-*c*]quinoline-1-carboxylate de propyle | B |
| 152 | 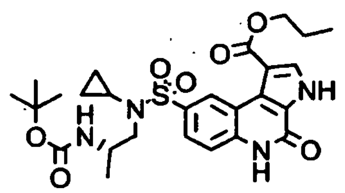 | (S) 8-[(2-*tert*-Butoxycarbonylamino-propyl)-cyclopropyl-sulfamoyl]-4-oxo-4,5-dihydro-3*H*-pyrrolo[2,3-*c*]quinoline-1-carboxylate de propyle | B |
| 153 | 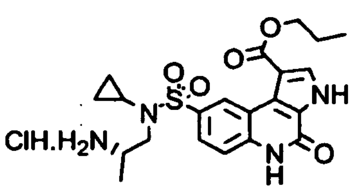 | Chlorhydrate de (S) 8-[(2-amino-propyl)-cyclopropyl-sulfamoyl]-4-oxo-4,5-dihydro-3*H*-pyrrolo[2,3-*c*]quinoline-1-carboxylate de propyle | A |

(suite)

| Composé | Formule | Nom | $IC_{50}$ |
|---|---|---|---|
| 154 | 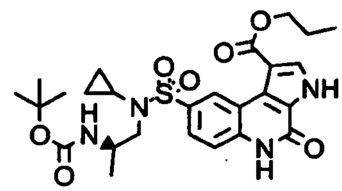 | (R) 8-[(2-*tert*-Butoxycarbonylamino-propyl)-cyclopropyl-sulfamoyl]-4-oxo-4,5-dihydro-3*H*-pyrrolo[2,3-*c*]quinoline-1-carboxylate de propyle | C |
| 155 | 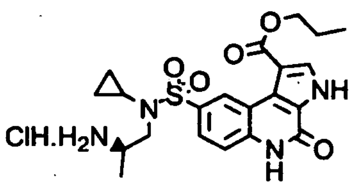 | Chlorhydrate de (R) 8-[(2-amino-propyl)-cyclopropyl-sulfamoyl]-4-oxo-4,5-dihydro-3H-pyrrolo[2,3-*c*]quinoline-1-carboxylate de propyle | A |
| 156 | 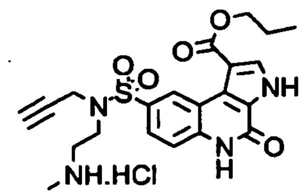 | Chlorhydrate de 8-[(2-méthylamino-éthyl)-prop-2-ynyl-sulfamoyl]-4-oxo-4,5-dihydro-3*H*-pyrrolo[2,3-*c*]quinoline-1-carboxylate de propyle | A |
| 157 | 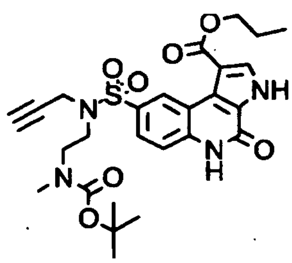 | 8-{[2-(*tert*-Butoxycarbonyl-méthyl-amino)-éthyl]-prop-2-ynyl-sulfamoyl}-4-oxo-4,5-dihydro-3*H*-pyrrolo[2,3-*c*]quinoline-1-carboxylate de propyle | A |
| 158 | 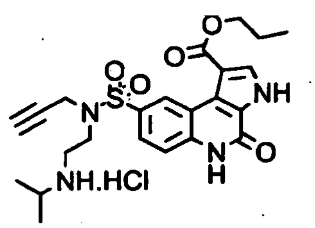 | Chlorhydrate de 8-[(2-isopropylamino-éthyl)-prop-2-ynyl-sulfamoyl]-4-oxo-4,5-dihydro-3*H*-pyrrolo[2,3-*c*]quinoline-1-carboxylate de propyle | B |
| 159 | 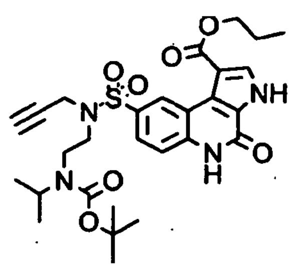 | 8-{[2-(*tert*-Butoxycarbonyl-isopropyl-amino)-éthyl]-prop-2-ynyl-sulfamoyl}-4-oxo-4,5-dihydro-3*H*-pynrolo[2,3-*c*]quinoline-1-carboxylate de propyle | C |
| 160 | 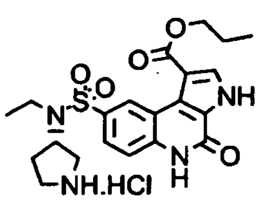 | Chlorhydrate de 8-(éthyl-pyrrolidin-3-yl-sulfamoyl)-4-oxo-4,5-dihydro-3*H*-pyrrolo [2,3-*c*]quinoline-1-carboxylate de propyle | A |

(suite)

| Composé | Formule | Nom | $IC_{50}$ |
|---|---|---|---|
| 161 | 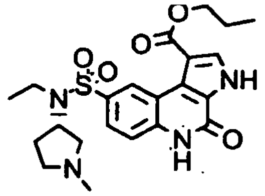 | 8-[Ethyt-(1-méthyl-pyrrolidin-3-yl)-sulfamoyl]-4-oxo-4,5-dihydro-3*H*-pyrrolo[2,3-*c*]quinoline-1-carboxylate de propyle | A |
| 162 | 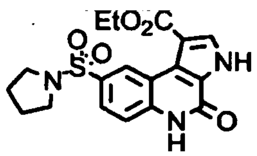 | 4-Oxo-8-(pyrrolidine-1-sulfonyl)-4,5-dihydro-3*H*-pyrrolo[2,3-*c*]quinoline-1-carboxylate d'éthyle | B |
| 163 | 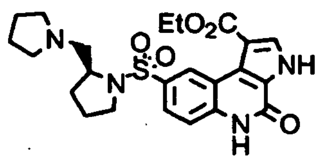 | 4-Oxo-8-(2-pyrrolidin-1-ylméthyl-pyrrolidine-1-sulfonyl)-4,5-dihydro-3*H*-pyrrolo[2,3-*c*] quinoline-1-carboxylate d'éthyle | B |
| 164 | 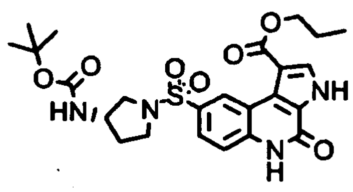 | (S) 8-(3-*tert*-Butoxycarbonylamino-pyrrolidine-1-sulfonyl)-4-oxo-4,5-dihydro-3*H*-pyrrolo[2,3-*c*]quinoline-1-carboxylate de propyle | C |
| 165 | 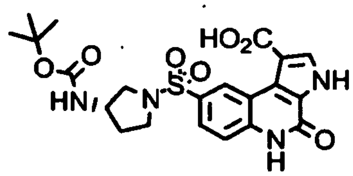 | Acide (S) 8-(3-*tert*-butoxycarbonylamino-pyrrolidine-1-sulfonyl)-4-oxo-4,5-dihydro-3*H*-pyrrolo[2,3-*c*]quinoline-1-carboxylique | D |
| 166 | 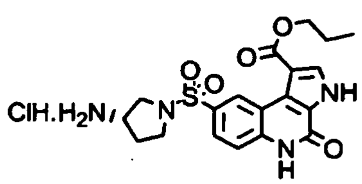 | Chlorhydrate de (S) 8-(3-amino-cyclopentanesulfonyl)-4-oxo-4,5-dihydro-3*H*-pyrrolo[2,3-*c*]quinoline-1-carboxylate de propyle | A |
| 167 | 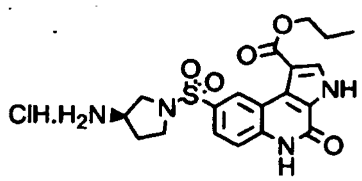 | Chlorhydrate de (R) 8-(3-amino-cyclopentanesulfonyl)-4-oxo-4,5-dihydro-3*H*-pyrrolo[2,3-*c*]quinoline-1-carboxylate de propyle | B |
| 168 | 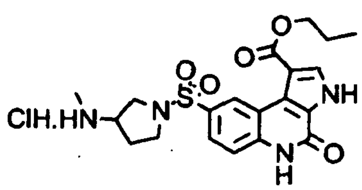 | Chlorhydrate de 8-(3-méthylamino-pyrrolidine-1-sulfonyl)-4-oxo-4,5-dihydro-3*H*-pyrrolo[2,3-*c*]quinoline-1-carboxylate de propyle | B |

(suite)

| Composé | Formule | Nom | $IC_{50}$ |
|---|---|---|---|
| 169 | 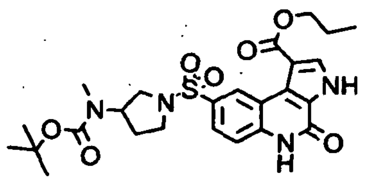 | 8-[3-(*tert*-Butoxycarbonyl-méthyl-amino)-pyrrolidine-1-sulfonyl]-4-oxo-4,5-dihydro-3*H*-pyrrolo[2,3-*c*]quinoline-1-carboxylate de propyle | C |
| 170 | 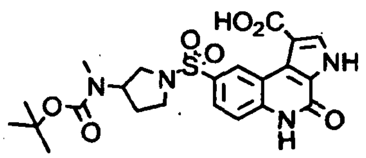 | Acide de 8-[3-(*tert*-butoxycarbonyl-méthyl-amino)-pyrrolidine-1-sulfonyl]-4-oxo-4,5-dihydro-3*H*-pyrrolo[2,3-*c*]quinoline-1-carboxylique | D |
| 171 | 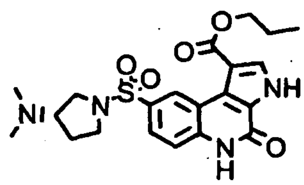 | (S) 8-(3-Diméthylamino-cyclopentanesulfonyl)-4-oxo-4,5-dihydro-3*H*-pyrrolo[2,3-*c*]quinoline-1-carboxylate de propyle | B |
| 172 | 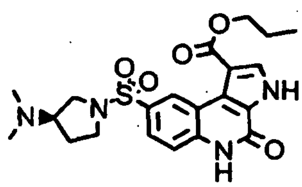 | (R) 8-(3-Diméthylamino-cyclopentanesulfonyl)-4-oxo-4,5-dihydro-3*H*-pyrrolo[2,3-*c*]quinoline-1-carboxylate de propyle | C |
| 173 | 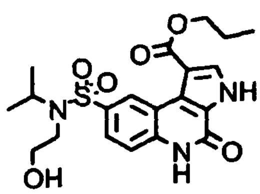 | 8-[(2-Hydroxy-éthyl)-isopropyl-sulfamoyl]-4-oxo-4,5-dihydro-3*H*-pyrrolo[2,3-*c*]quinoline-1-carboxylate de propyle | A |
| 174 | 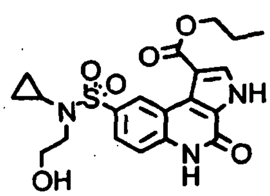 | 8-[Cyclopropyl-(2-hydroxy-éthyl)-sulfamoyl]-4-oxo-4,5-dihydro-3*H*-pyrrolo[2,3-*c*] quinoline-1-carboxylate de propyle | A |
| 175 | 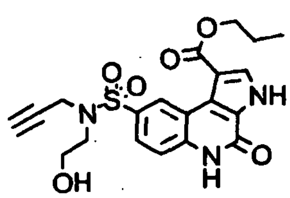 | 8-[(2-Hydroxy-éthyl)-prop-2-ynyl-sulfamoyl]-4-oxo-4,5-dihydro-3*H*-pyrrolo[2,3-*c*] quinoline-1-carboxylate de propyle | B |
| 176 | 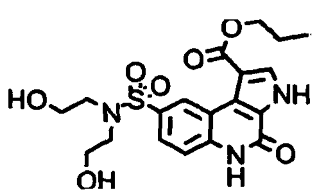 | 8-[Bis-(2-hydroxy-éthyl)-sulfamoyl]-4-oxo-4,5-dihydro-3*H*-pyrrolo[2,3-*c*]quinoline-1-carboxylate de propyle | B |

(suite)

| Composé | Formule | Nom | IC$_{50}$ |
|---|---|---|---|
| 177 | 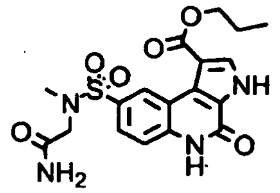 | 8-(Carbamoylméthyl-méthyl-sulfamoyl)-4-oxo-4,5-dihydro-3*H*-pyrrolo[2,3-*c*]quinoline-1-carboxylate de propyle | B |
| 178 | 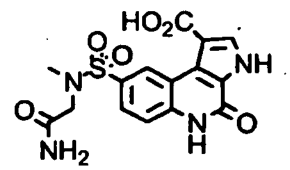 | Acide 8-(carbamoylméthyl-méthyl-sulfamoyl)-4-oxo-4,5-dihydro-3*H*-pyrrolo [2,3-*c*]quinoline-1-carboxylique | C |
| 179 | 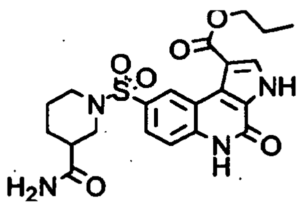 | 8-(3-Carbamoyl-pipéridine-1-sulfonyl)-4-oxo-4,5-dihydro-3*H*-pyrrolo[2,3-*c*]quinoline-1-carboxylate de propyle | B |
| 180 | 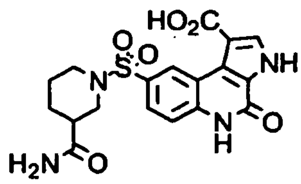 | Acide 8-(3-carbamoyl-pipéridine-1-sulfonyl)-4-oxo-4,5-dihydro-3*H*-pyrrolo[2,3-*c*] quinoline-1-carboxylique | D |
| 181 | 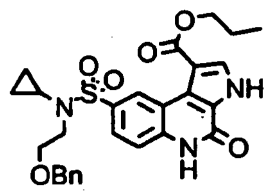 | 8-[(2-Benzyloxy-éthyl)-cyclopropyl-sulfamoyl]-4-oxo-4,5-dihydro-3*H*-pyrrolo [2,3-*c*]quinoline-1-carboxylate de propyle | C |
| 182 | 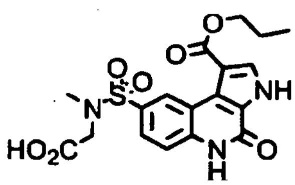 | 8-(Carboxyméthyl-méthyl-sulfamoyl)-4-oxo-4,5-dihydro-3*H*-pyrrolo[2,3-*c*]quinoline-1-carboxylate de propyle | C |
| 183 | 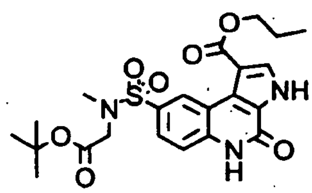 | 8-(*tert*-Butoxycarbonylméthyl-méthyl-sulfamoyl)-4-oxo-4,5-dihydro-3*H*-pyrrolo [2,3-*c*]quinoline-1-carboxylate de propyle | C |
| 184 | 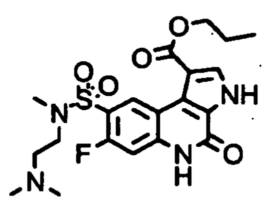 | 8-[(2-Diméthylamino-éthyl)-méthyl-sulfamoyl]-7-fluoro-4-oxo-4,5-dihydro-3*H*-pyrrolo[2,3-*c*]quinoline-1-carboxylate de propyle | A |

(suite)

| Composé | Formule | Nom | $IC_{50}$ |
|---|---|---|---|
| 185 | 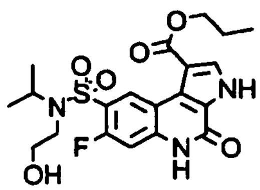 | 7-Fluoro-8-[(2-hydroxy-éthyl)-isopropyl-sulfamoyl]-4-oxo-4,5-dihydro-3*H*-pyrrolo [2,3-*c*]quinoline-1-carboxylate de propyle | A |
| 186 | 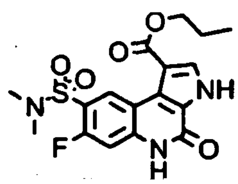 | 8-Diméthylsulfamoyl-7-fluoro-4-oxo-4,5-dihydro-3*H*-pyrrolo[2,3-*c*]quinoline-1-carboxylate de propyle | B |
| 187 | 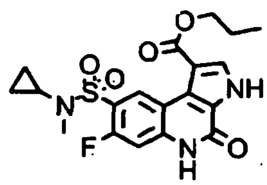 | 8-(Cyclopropyl-mélhyl-sulfamoyl)-7-fluoro-4-oxo-4,5-dihydro-3*H*-pyrrolo[2,3-*c*]quinoline-1-carboxylate de propyle | B |
| 188 | 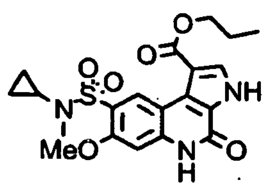 | 8-(Cyclopropyl-méthyl-sulfamoyl)- 7-méthoxy-4-oxo-4,5-dihydro-3*H*-pyrrolo [2,3-*c*]quinoline-1-carboxylate de propyle | C |
| 189 | 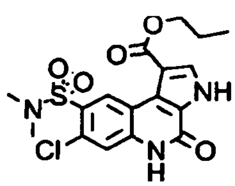 | 7-Chloro-8-diméthylsulfamoyl-4-oxo-4,5-dihydro-3*H*-pyrrolo[2,3-*c*]quinoline-1-carboxylate de propyle | B |
| 190 | 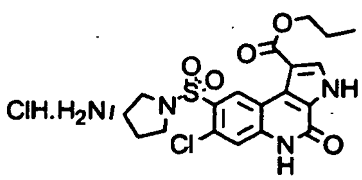 | Chlorhydrate de (S) 8-(3-amino-cyclopentanesulfonyl)-7-chloro-4-oxo-4,5-dihydro-3*H*-pyrrolo[2,3-*c*]quinoline-1-carboxylate de propyle | B |
| 191 | 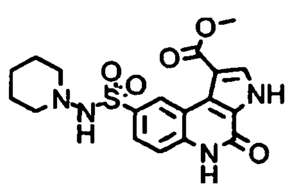 | 4-Oxo-8-(pipéridin-1-ylsulfamoyl)-4,5-dihydro-3*H*-pyrrolo[2,3-*c*]quinoline-1-carboxylate de méthyle | C |
| 192 | 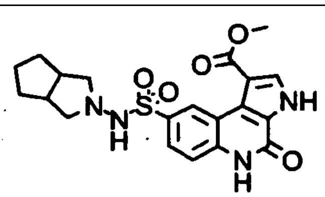 | 8-(Hexahydro-cyclopenta[*c*]pyrrol-2-ylsulfamoyl)-4-oxo-4,5-dihydro-3*H*-pyrrolo [2,3-*c*]quinoline-1-carboxylate de méthyle | C |

(suite)

| Composé | Formule | Nom | $IC_{50}$ |
|---|---|---|---|
| 193 | 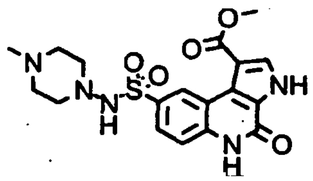 | 8-(4-Méthyl-pipérazin-1-ylsulfamoyl)-4-oxo-4,5-dihydro-3*H*-pyrrolo[2,3-*c*]quinoline-1-carboxylate de méthyle | C |

**TABLEAU II**

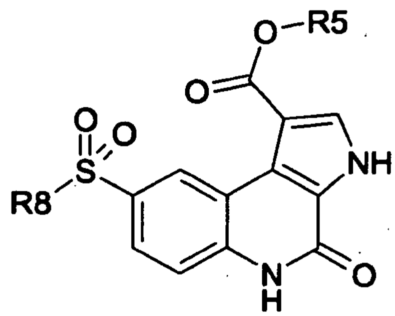

III)

| Composé | Formule | Nom | IC50 |
|---|---|---|---|
| 35 | 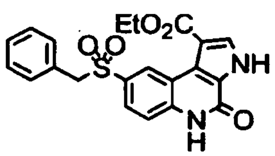 | 4-Oxo-8-phénylméthanesulfonyl-4,5-dihydro-3*H*-pyrrolo[2,3-*c*]quinoline-1-carboxylate d'éthyle | A |
| 36 | 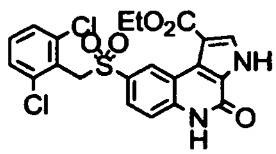 | 8-(2,6-Dichloro-phénylméthanesulfonyl)-4-oxo-4,5-dihydro-3*H*-pyrrolo[2,3-*c*]quinoline-1-carboxylate d'éthyle | B |
| 194 | 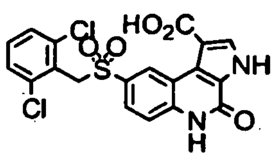 | Acide 8-(2,6-dichloro-phénylméthanesulfonyl)-4-oxo-4,5-dihydro-3*H*-pyrrolo[2,3-*c*]quinoline-1-carboxylate d'éthyle | C |
| 195 | 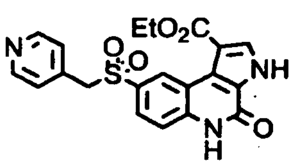 | 4-Oxo-8-(pyridin-4-ylméthanesulfonyl)-4,5-dihydro-3*H*-pyrrolo[2,3-*c*]quinoline-1-carboxylate d'éthyle | B |
| 196 | 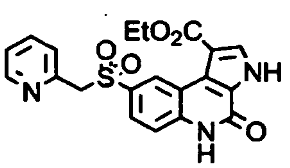 | 4-Oxo-8-(pyridin-2-ylméthanesulfonyl)-4,5-dihydro-3*H*-pyrrolo[2,3-*c*]quinoline-1-carboxylate d'éthyle | B |

(suite)

| Composé | Formule | Nom | IC50 |
|---|---|---|---|
| 197 | 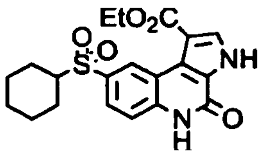 | 8-Cyclohexanesulfonyl-4-oxo-4,5-dihydro-3*H*-pyrrolo [2,3-*c*]quinoline-1-carboxylate d'éthyle | B |
| 198 | 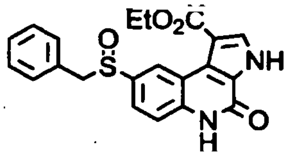 | 4-Oxo-8-phénylméthanesulfinyl-4,5-dihydro-3*H*-pyrrolo[2,3-*c*]quinoline-1-carboxylate d'éthyle | B |

**TABLEA III**

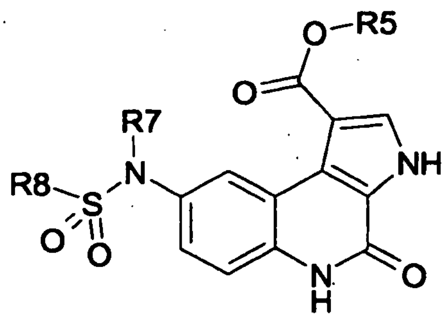

IV)

| Composé | Formule | Nom | IC50 |
|---|---|---|---|
| 37 | 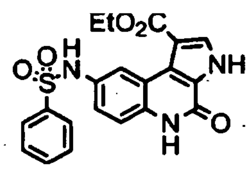 | 8-Benzènesulfonylamino-4-oxo-4,5-dihydro-3*H*-pyrrolo[2,3-*c*]quinoline-1-carboxylate d'éthyle | B |
| 38 | 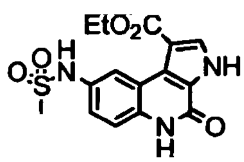 | 8-Méthanesulfonylamino-4-oxo-4,5-dihydro-3*H*-pyrrolo[2,3-*c*]quinoline-1-carboxylate d'éthyle | B |
| 39 | 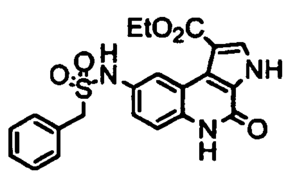 | 4-Oxo-8-phénylméthanesulfonylamino-4,5-dihydro-3*H*-pyrrolo[2,3-*c*]quinolina-1-carboxylate d'éthyle | B |
| 40 | 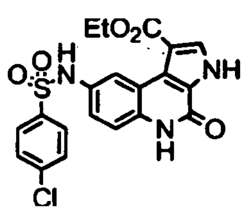 | 8-(4-Chloro-benzènesulfonylamino)-4-oxo-4,5-dihydro-3*H*-pyrrolo[2,3-*c*]quinoline-1-carboxylate d'éthyle | B |

(suite)

| Composé | Formule | Nom | IC50 |
|---|---|---|---|
| 41 | 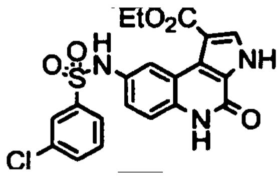 | 8-(3-Chloro-benzènesulfonylamino)-4-oxo-4,5-dihydro-3*H*-pyrrolo[2,3-*c*]quinoline-1-carboxylate d'éthyle | B |
| 42 | 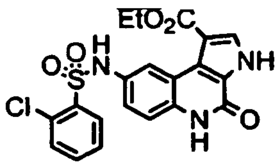 | 8-(2-Chloro-benzènesulfonylamino)-4-oxo-4,5-dihydro-3*H*-pyrrolo[2,3-*c*]quinoline-1-carboxylate d'éthyle | D |
| 43 | 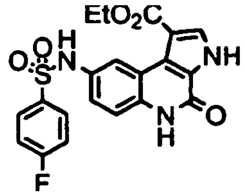 | 8-(4-Fluoro-benzènesulfonylamino)-4-oxo-4,5-dihydro-3*H*-pyrrolo[2,3-*c*]quinoline-1-carboxylate d'éthyle | B |
| 44 | 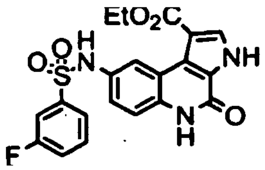 | 8-(3-Fluoro-benzènesulfonylamino)-4-oxo-4,5-dihydro-3*H*-pyrrolo[2,3-*c*]quinoline-1-carboxylate d'éthyle | B |
| 45 | 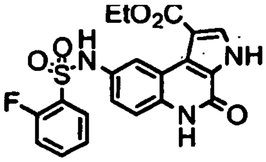 | 8-(2-Fluoro-bènzenesulfonylamino)-4-oxo-4,5-dihydro-3*H*-pyrrolo[2,3-*c*]quinoline-1-carboxylate d'éthyle | C |
| 46 | 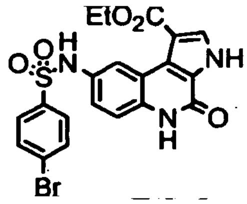 | 8-(4-Bromo-benzènesulfonylamino)4-oxo-4,5-dihydro-3*H*-pyrrolo[2,3-*c*]quinoline-1-carboxylate d'éthyle | B |
| 47 | 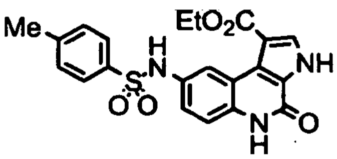 | 4-Oxo-8-(4-toluènesulfonylamino)-4,5-dihydro-3*H*-pyrrolo[2,3-*c*]quinoline-1-carboxylate d'éthyle | B |
| 48 | 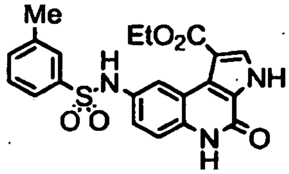 | 4-Oxo-8-(3-toluènesulfonylamino)-4,5-dihydro-3*H*-pyrrolo[2,3-*c*]quinoline-1-carboxylate d'éthyle | D |

(suite)

| Composé | Formule | Nom | IC50 |
|---|---|---|---|
| 49 | 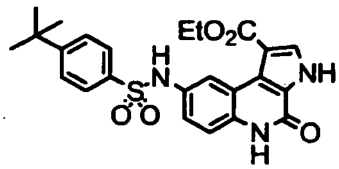 | 8-(4-*tert*-Butyl-benzènesulfonylamino)-4-oxo-4,5-dihydro-3*H*-pyrrolo[2,3-*c*]quinoline-1-carboxylate d'éthyle | D |
| 50 | 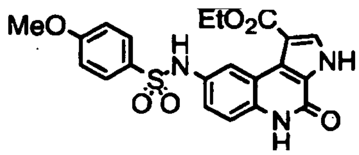 | 8-(4-Méthoxy-benzènesulfonylamino)-4-oxo-4,5-dihydro-3*H*-pyrrolo[2,3-*c*]quinoline-1-carboxylate d'éthyle | C |
| 51 | 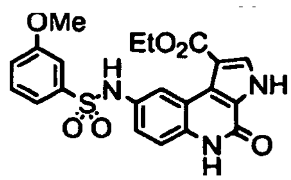 | 8-(3-Méthoxy-benzènesulfonylamino)-4-oxo-4,5-dihydro-3*H*-pyrrolo[2,3-*c*]quinoline-1-carboxylate d'éthyle | C |
| 52 | 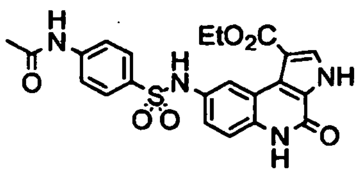 | 8-(4-Acétylamino-benzènesulfonylamino)-4-oxo-4,5-dihydro-3*H*-pyrrolo[2,3-*c*]quinoline-1-carboxylate d'éthyle | C |
| 53 | 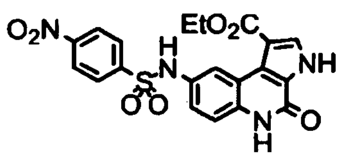 | 8-(4-Nitro-benzènesulfonylamino)-4-oxo-4,5-dihydro-3*H*-pyrrolo[2,3-*c*]quinoline-1-carboxylate d'éthyle | B |
| 54 | 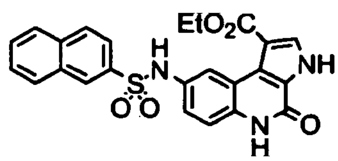 | 8-(Naphtalène-2-sulfonylamino)-4-oxo-4,5-dihydro-3*H*-pyrrolo[2,3-*c*]quinotine-1-carboxytate d'éthyle | D |
| 55 | 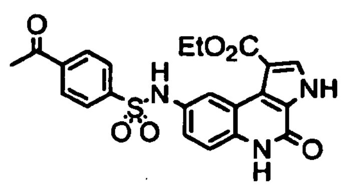 | 8-(4-Acétyl-benzènesulfonylamino)-4-oxo-4,5-dihydro-3*H*-pyrrolo[2,3-*c*]quinoline-1-carboxylate d'éthyle | C |
| 56 | 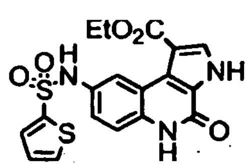 | 4-Oxo-8-(thiophène-2-sulfonylamino)-4,5-dihydro-3*H*-pyrrolo[2,3-*c*]quinoline-1-carboxylate d'éthyle | B |
| 57 | 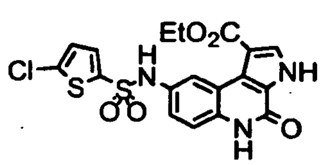 | 8-(5-Chloro-thiophène-2-sulfonylamino)-4-oxo-4,5-dihydro-3*H*-pyrrolo[2,3-*c*]quinoline-1-carboxylate d'éthyle | B |

(suite)

| Composé | Formule | Nom | IC50 |
|---|---|---|---|
| 58 | 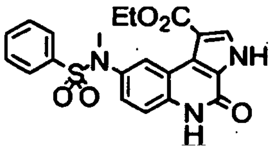 | 8-(Benzènesulfonyl-méthyl-amino)-4-oxo-4,5-dihydro-3*H*-pyrrolo[2,3-*c*]quinoline-1-carboxylate d'éthyle | B |
| 59 | 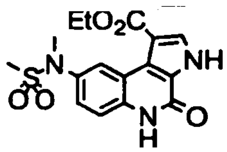 | 8-(Méthanesulfonyl-méthyl-amino)-4-oxo-4,5-dihydro-3*H*-pyrrolo[2,3-*c*]quinoline-1-carboxylate d'éthyle | B |

**TABLEAU IV**

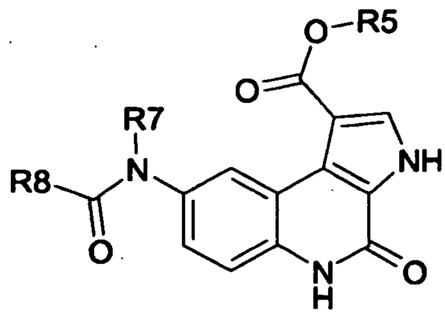

V)

| Composé | Formule | Nom | IC50 |
|---|---|---|---|
| 60 | 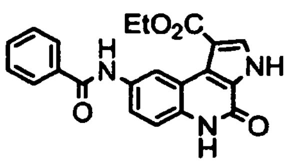 | 8-Benzoylamino-4-oxo-4,5-dihydro-3*H*-pyrrolo [2,3-*c*]quinoline-1-carboxylate d'éthyle | C |
| 61 | 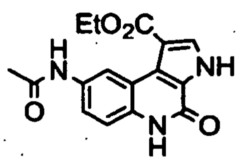 | 8-Acétylamino-4-oxo-4,5-dihydro-3*H*-pyrrolo [2,3-*c*]quinoline-1-carboxylate d'éthyle | C |

## TABLEAU V

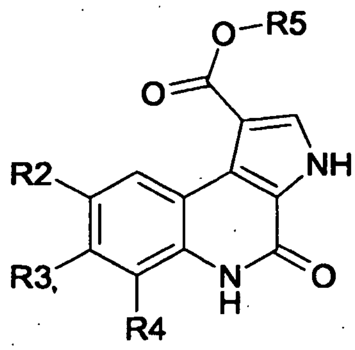

VI)

| Composé | Formule | Nom | IC50 |
|---|---|---|---|
| 62 | 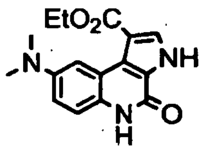 | 8-Diméthylamino-4-oxo-4,5-dihydro-3*H*-pyrrolo [2,3-*c*]quinoline-1-carboxylate d'éthyle | C |
| 63 | 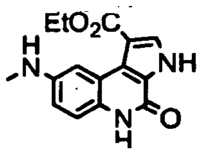 | 8-méthylamino-4-oxo-4,5-dihydro-3*H*-pyrrolo [2,3-*c*]quinoline-1-carboxylate d'éthyle | C |
| 64 | 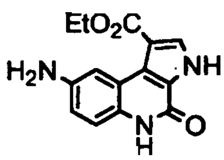 | 8-Amino-4-oxo-4,5-dihydro-3*H*-pyrrolo[2,3-*c*] quinoline-1-carboxylate d'éthyle | C |
| 66 | 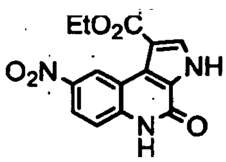 | 8-Nitro-4-oxo-4,5-dihydro-3*H*-pyrrolo[2,3-*c*] quinoline-1-carboxylate d'éthyle | C |
| 67 | 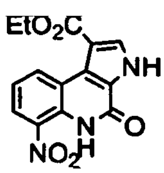 | 6-Nitro-4-oxo-4,5-dihydro-3*H*-pyrrolo[2,3-*c*] quinoline-1-carboxylate d'éthyle | D |
| 65 | 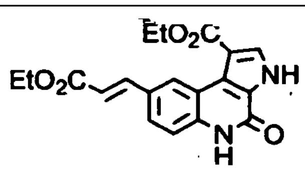 | 8-(2-Ethoxycarbonyl-vinyl)-4-oxo-4,5-dihydro-3*H*-pyrrolo[2,3-*c*]quinolino-1-carboxylate d'éthyle | C |
| 68 | 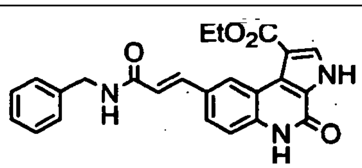 | 8-(2-Benzylcarbamoyl-vinyl)-4-oxo-4,5-dihydro-3*H*-pyrrolo[2,3-*c*]quinoline-1-carboxylate d'éthyle | C |

(suite)

| Composé | Formule | Nom | IC50 |
|---|---|---|---|
| 69 | 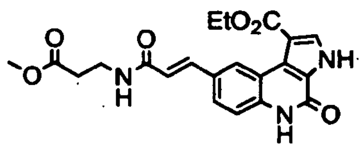 | 8-[2-(2-Méthoxycarbonyl-éthylcarbamoyl)-vinyl]-4-oxo-4,5-dihydro-3*H*-pyrrolo[2,3-*c*]quinoline-1-carboxylate d'éthyle | C |
| 70 | 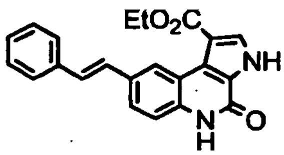 | 4-Oxo-8-styryl-4,5-dihydro-3*H*-pyrrolo[2,3-*c*] quinoline-1-carboxylate d'éthyle | C |
| 199 | 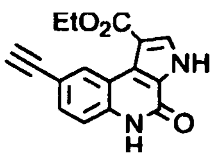 | 8-Ethynyl-4-oxo-4,5-dihydro-3*H*-pyrrolo[2,3-*c*] quinoline-1-carboxylate d'éthyle | C |
| 200 | 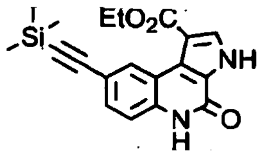 | 4-Oxo-8-triméthylsilanyléthynyl-4,5-dihydro-3*H*-pyrrolo[2,3-*c*]quinoline-1-carboxylate d'éthyle | C |
| 201 | 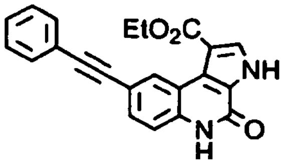 | 4-Oxo-8-phényléthynyl-4,5-dihydro-3*H*-pyrrolo [2,3-*c*]quinoline-1-carboxylate d'éthyle | C |
| 202 | 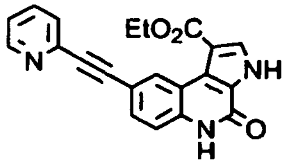 | 4-Oxo-8-pyridin-2-yléthynyl-4,5-dihydro-3*H*-pyrrolo[2,3-*c*]quinoline-1-carboxylate d'éthyle | C |
| 203 | 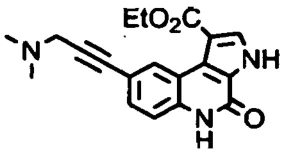 | 8-(3-Diméthylamino-prop-1-ynyl)-4-oxo-4,5-dihydro-3*H*-pyrrolo[2,3-*c*]quinoline-1-carbokylate d'éthyle | B |
| 204 | 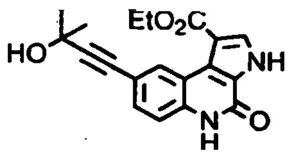 | 8-(3-Hydroxy-3-méthyl-but-1-ynyl)-4-oxo-4,5-dihydro-3*H*-pyrrolo[2,3-*c*]quinoline-1-carboxylate d'éthyle | B |
| 205 | 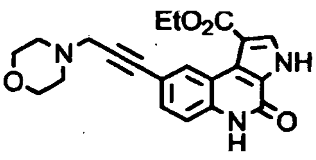 | 8-(3-Morpholin-4-yl-prop-1-ynyl)-4-oxo-4,5-dihydro-3*H*-pyrrolo[2,3-*c*]quinoline-1-carboxylate d'éthyle | B |

(suite)

| Composé | Formule | Nom | IC50 |
|---|---|---|---|
| 206 | 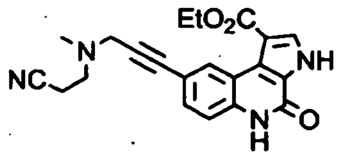 | 8-{3-[(2-Cyano-éthyl)-méthyl-amino]-prop-1-ynyl}-4-oxo-4,5-dihydro-3*H*-pyrrolo[2,3-*c*] quinoline-1-carboxylate d'éthyle | B |
| 207 | 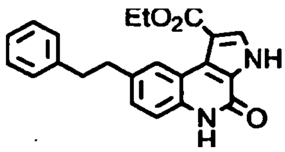 | 4-Oxo-8-phénéthyl-4,5-dihydro-3*H*-pyrrolo[2,3-*c*] quinoline-1-carboxylate d'éthyle | B |
| 208 | 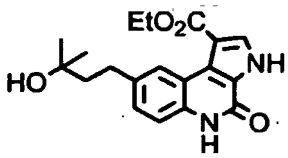 | 8-(3-Hydroxy-3-méthyl-butyl)-4-oxo-4,5-dihydro-3*H*-pyrrolo[2,3-*c*]quinoline-1-carboxylate d'éthyle | B |
| 71 | 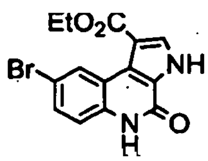 | 8-Bromo-4-oxo-4,5-dihydro-3*H*-pyrrolo[2,3-*c*] quinoline-1-carboxylate d'éthyle | B |
| 209 | 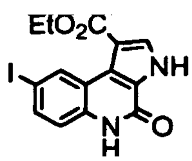 | 8-Iodo-4-oxo-4,5-dihydro-3*H*-pyrrolo[2,3-*c*] quinoline-1-carboxylate d'éthyle | B |
| 210 | 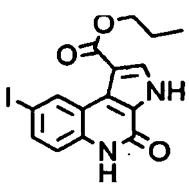 | 8-Iodo-4-oxo-4,5-dihydro-3*H*-pyrrolo[2,3-*c*] quinoline-1-carboxylate de propyle | B |
| 211 | 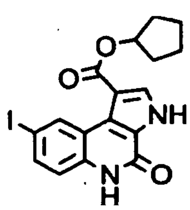 | 8-Iodo-4-oxo-4,5-dihydro-3*H*-pyrrolo[2,3-*c*] quinoline-1-carboxylate de cyclopentyle | C |
| 212 | 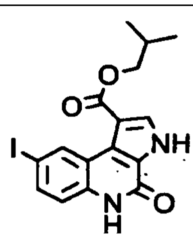 | 8-Iodo-4-oxo-4,5-dihydro-3*H*-pyrrolo[2,3-*c*] quinoline-1-carboxylate d'isobutyle | C |
| 72 | 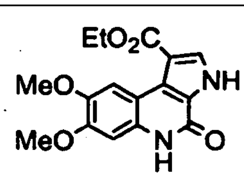 | 7,8-Diméthoxy-4-oxo-4,5-dihydro-3*H*-pyrrolo [2,3-*c*]quinoline-1-carboxylate d'éthyle | C |

(suite)

| Composé | Formule | Nom | IC50 |
|---|---|---|---|
| 213 | 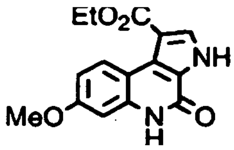 | 7-Méthoxy-4-oxo-4,5-dihydro-3*H*-pyrrolo[2,3-*c*] quinoline-1-carboxylaie d'éthyle | C |
| 214 | 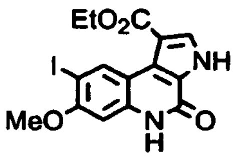 | 8-Iodo-7-méthoxy-4-oxo-4,5-dihydro-3*H*-pyrrolo [2,3-*c*]quinoline-1-carboxylate d'éthyle | C |
| 99 | 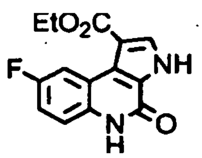 | 8-Fluoro-4-oxo-4,5-dihydro-3*H*-pyrrolo[2,3-*c*] quinoline-1-carboxylate d'éthyle | D |
| 100 | 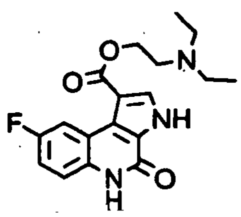 | 8-Fluoro-4-oxo-4,5-dihydro-3*H*-pyrrolo[2,3-*c*] quinoline-1-carboxylate de 2-diéthylaminoéthyle | D |
| 215 | 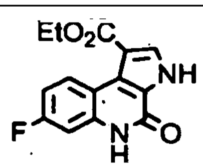 | 7-Fluoro-4-oxo-4,5-dihydro-3*H*-pyrrolo[2,3-*c*] quinoline-1-carboxylate d'éthyle | C |
| 216 | 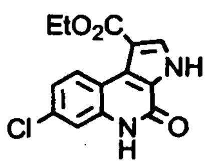 | 7-Chloro-4-oxo-4,5-dihydro-3*H*-pyrrolo[2,3-*c*] quinoline-1-carboxylate d'éthyle | C |
| 101 | 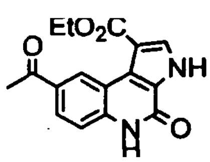 | 8-Acétyl-4-oxo-4,5-dihydro-3*H*-pyrrolo[2,3-*c*] quinoline-1-carboxylale d'éthyle | C |
| 102 | 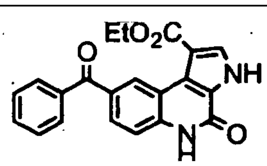 | 8-Benzoyl-4-oxo-4,5-dihydro-3*H*-pyrrolo[2,3-*c*] quinoline-1-carboxylate d'éthyle | C |
| 217 | 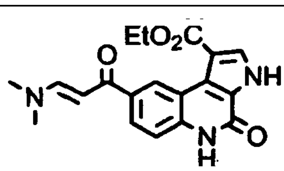 | 8-(3-Diméthylamino-acryloyl)-4-oxo-4,5-dihydro-3*H*-pyrrolo[2,3-*c*]quinoline-1-carboxylale d'éthyle | B |

(suite)

| Composé | Formule | Nom | IC50 |
|---|---|---|---|
| 218 | 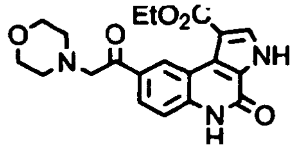 | 8-(2-Morpholin-4-yl-acétyl)-4-oxo-4,5-dihydro-3*H*-pyrrolo[2,3-*c*]quinoline-1-carboxylate d'éthyle | C |
| 219 | 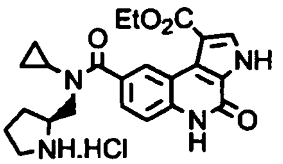 | Chlorhydrate de (S) 8-(cyclopropyl-pyrrolidin-2-ylméthyl-carbamoyl)-4-oxo-4,5-dihydro-3*H*-pyrrolo[2,3-*c*]quinoline-1-carboxylate d'éthyle | C |
| 220 | 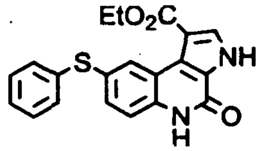 | 4-Oxo-8-phénylsulfanyl-4,5-dihydro-3*H*-pyrrolo [2,3-*c*]quinoline-1-carboxylate d'éthyle | B |
| 221 | 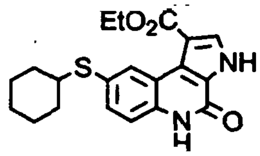 | 8-Cyclohexylsulfanyl-4-oxo-4,5-dihydro-3*H*-pyrrolo[2,3-*c*]quinoline-1-carboxylate d'éthyle | B |
| 222 | 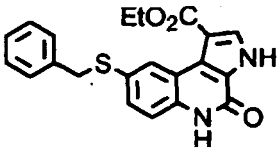 | 8-Benzylsulfanyl-4-oxo-4,5-dihydro-3*H*-pyrrolo [2,3-*c*]quinoline-1-carboxylate d'éthyle | B |
| 223 | 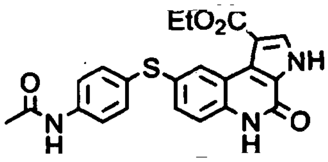 | 8-(4-Acétylamino-phénylsulfanyl)-4-oxo-4,5-dihydro-3*H*-pyrrolo[2,3-*c*]quinoline-1-carboxylate d'éthyle | B |
| 224 | 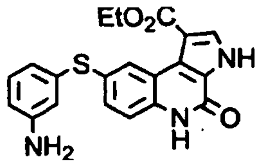 | 8-(3-Amino-phénylsulfanyl)-4-oxo-4,5-dihydro-3*H*-pyrrolo[2,3-*c*]quinoline-1-carboxylate d'éthyle | B |
| 225 | 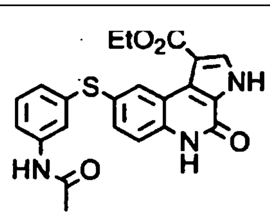 | 8-(3-Acétylamino-phénylsulfanyl)-4-oxo-4,5-dihydro-3*H*-pyrrolo[2,3-*c*]quinoline-1-carboxylate d'éthyle | B |
| 226 | 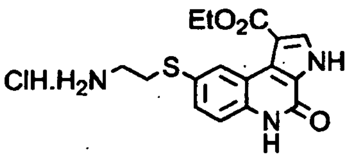 | Chlorhydrate de 8-(2-amino-éthylsulfanyl)-4-oxo-4,5-dihydro-3*H*-pyrrolo[2,3-*c*]quinoline-1-carboxylate d'éthyle | A |

(suite)

| Composé | Formule | Nom | IC50 |
|---|---|---|---|
| 227 | 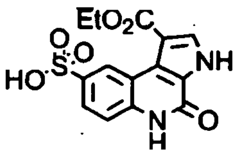 | 4-Oxo-8-sulfo-4,5-dihydro-3*H*-pyrrolo[2,3-*c*] quinoline-1-carboxylate d'éthyle. | C |

**TABLEAU VI**

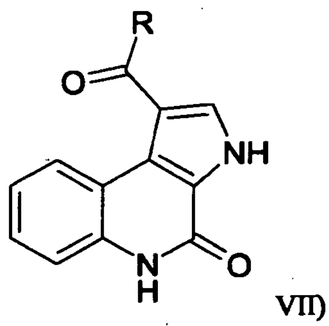

VII)

| Composé | Formule | Nom | IC50 |
|---|---|---|---|
| 1 | 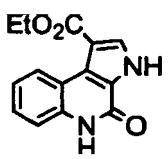 | 4-Oxo-4,5-dihydro-3*H*-pyrrolo[2,3-*c*]quinoline-1-carboxylate d'éthyle | C |
| 73 | 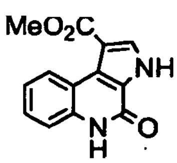 | 4-Oxo-4,5-dihydro-3*H*-pyrrolo[2,3-*c*]quinoline-1-carboxylate de méthyle | C |
| 74 | 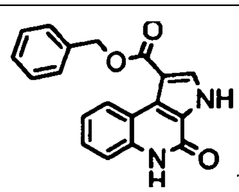 | 4-Oxo-4,5-dihydro-3*H*-pyrrolo[2,3-*c*]quinoline-1-carboxylate de benzyle | C |
| 78 | 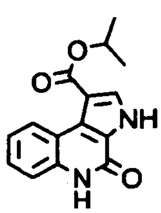 | 4-Oxo-4,5-dihydro-3*H*-pyrrolo[2,3-*c*]quinoline-1-carboxylate d'isopropyle | C |

(suite)

| Composé | Formule | Nom | IC50 |
|---|---|---|---|
| 75 | 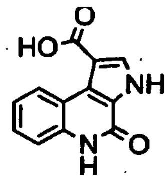 | Acide 4-oxo-4,5-dihydro-3*H*-pyrrolo[2,3-*c*]quinoline-1-carboxylique | D |
| 76 | 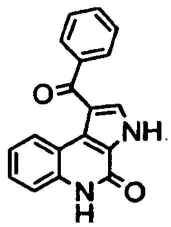 | 1-Benzoyl-3,5-dihydro-pyrrolo[2,3-*c*]quinolin-4-one | C |
| 77 | 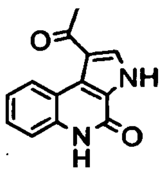 | 1-Acétyl-3,5-dihydro-pyrrolo[2,3-*c*]quinolin-4-oné | D |
| 79 | 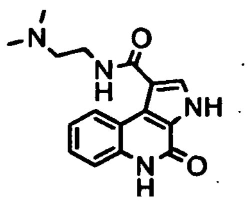 | *N*-(2-Diméthylamino-éthyl)-4-oxo-4,5-dihydro-3*H*-pyrrolo [2,3-*c*]quinoline-1-carboxamide | D |
| 80 | 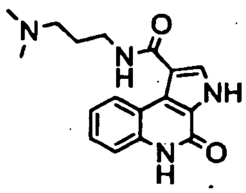 | *N*-(3-Diméthylamino-propyl)-4-oxo-4,5-dihydro-3*H*-pyrrolo [2,3-*c*]quinoline-1-carboxamide | D |
| 81 | 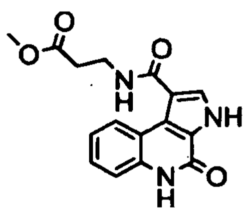 | 3-[(4-Oxo-4,5-dihydro-3*H*-pyrrolo[2,3-*c*]quinoline-1-carbonyl)-amino]-propanoate de méthyle | D |
| 82 | 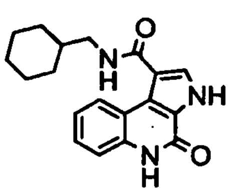 | *N*-(cyclohexylméthyl)-4-oxo-4,5-dihydro-3*H*-pyrrolo[2,3-*c*] quinoline-1-carboxamide | D |
| 83 | 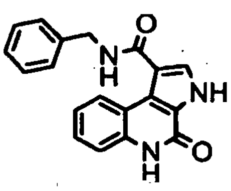 | *N*-(benzyl)-4-oxo-4,5-dihydro-3*H*-pyrrolo[2,3-*c*]quinoline-1-carboxylamide | D |

(suite)

| Composé | Formule | Nom | IC50 |
|---|---|---|---|
| 84 | 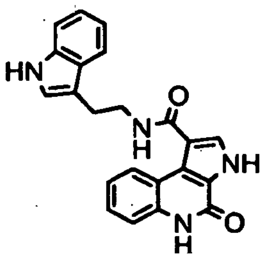 | *N*-[2-(1*H*-indol-3-yl)-éthyl]-4-oxo-4,5-dihydro-3*H*-pyrrolo [2,3-*c*]quinoline-1-carboxamide | D |
| 85 | 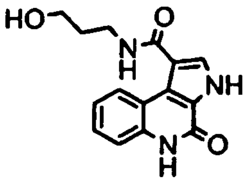 | *N*-(3-Hydroxy-propyl)-4-oxo-4,5-dihydro-3*H*-pyrrolo[2,3-*c*] quinoline-1-carboxamide | D |
| 86 | 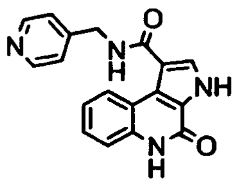 | *N*-(Pyridin-4-ylméthyl)-4-oxo-4,5-dihydro-3*H*-pyrrolo[2,3-*c*] quinoline-1-carboxamide | D |
| 87 | 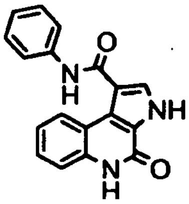 | *N*-(Phényl)-4-oxo-4,5-dihydro-3*H*-pyrrolo[2,3-*c*]quinoline-1-carboxamide | D |
| 88 | 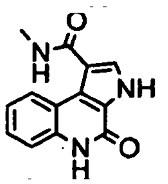 | *N*-(Méthyl)-4-oxo-4,5-dihydro-3*H*-pyrrolo[2,3-*c*]quinoline-1-carboxamide | D |
| 89 | 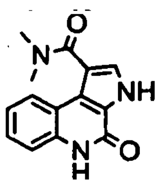 | *N*,*N*-(Diméthyl)-4-oxo-4,5-dihydro-3*H*-pyrrolo[2,3-*c*] quinoline-1-carboxamide | D |
| 90 | 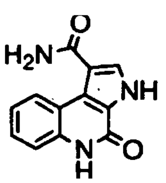 | 4-Oxo-4,5-dihydro-3*H*-pyrrolo[2,3-*c*]quinoline-1-carboxamide | D |

## TABLEAU VII

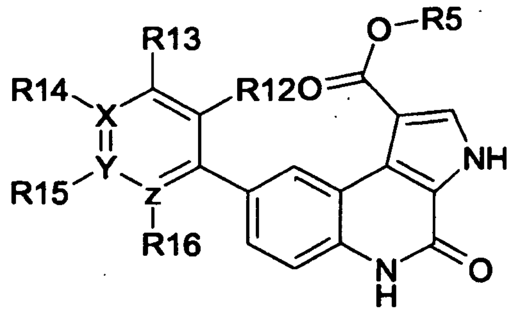

(VIII)

| Composé | Formule | Nom | IC50 |
|---|---|---|---|
| 228 | 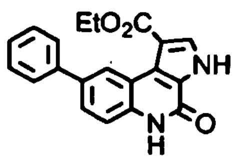 | 4-Oxo-8-phényl-4,5-dihydro-3*H*-pyrrolo [2,3-*c*]quinoline-1-carboxylate d'éthyle | C |
| 229 | 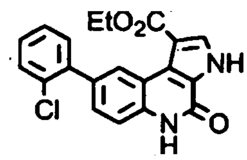 | 8-(2-Chloro-phényl)-4-oxo-4,5-dihydro-3*H*-pyrrolo[2,3-*c*]quinoline-1-carboxylate d'éthyle | B |
| 230 | 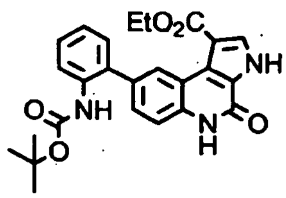 | 8-(2-*tert*-Butoxycarbonylamino-phényl)-4-oxo-4,5-dihydro-3*H*-pyrrolo[2,3-*c*] quinoline-1-carboxylate d'éthyle | D |
| 231 | 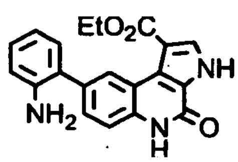 | 8-(2-Amino-phényl)-4-oxo-4,5-dihydro-3*H*-pyrrolo[2,3-*c*]quinoline-1-carboxylate d'éthyle | B |
| 232 | 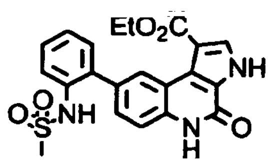 | 8-(2-Méthanesulfonylamino-phényl)-4-oxo-4,5-dihydro-3*H*-pyrrolo[2,3-*c*] quinoline-1-carboxylate d'éthyle | B |
| 233 | 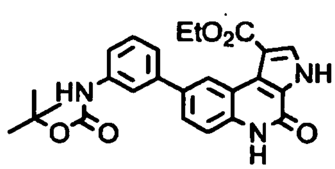 | 8-(3-*tert*-Butoxycarbonylamino-phényl)-4-oxo-4,5-dihydro-3*H*-pyrrolo[2,3-*c*] quinoline-1-carboxylate d'éthyle | C |
| 234 | 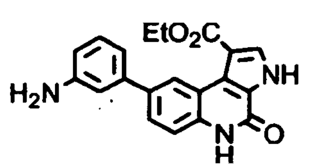 | 8-(3-Amino-phényl)-4-oxo-4,5-dihydro-3*H*-pyrrolo[2,3-*c*]quinoline-1-carboxylate d'éthyle | B |

(suite)

| Composé | Formule | Nom | IC50 |
|---|---|---|---|
| 235 | 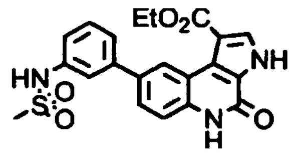 | 8-(3-Méthanesulfonylamino-phényl)-4-oxo-4,5-dihydro-3*H*-pyrrolo[2,3-*c*] quinoline-1-carboxylate d'éthyle | B |
| 236 | 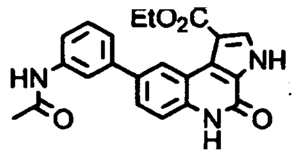 | 8-(3-Acétylamino-phényl)-4-oxo-4,5-dihydro-3*H*-pyrrolo[2,3-*c*]quinoline-1-carboxylate d'éthyle | B |
| 237 | 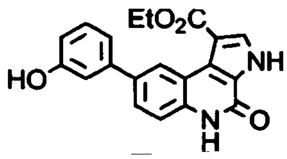 | 8-(3-Hydroxy-phényl)-4-oxo-4,5-dihydro-3*H*-pyrrolo[2,3-*c*]quinoline-1-carboxylate d'éthyle | B |
| 238 | 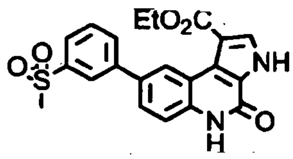 | 8-(3-Méthanesulfonyl-phényl)-4-oxo-4,5-dihydro-1*H*-pyrrolo[2,3-*c*]quinoline-1-carboxylate d'éthyle | B |
| 239 | 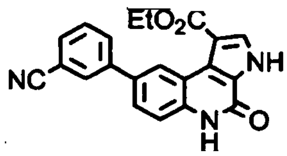 | 8-(3-Cyano-phényl)-4-oxo-4,5-dihydro-3*H*-pyrrolo[2,3-*c*]quinoline-1-carboxylate d'éthyle | B |
| 240 | 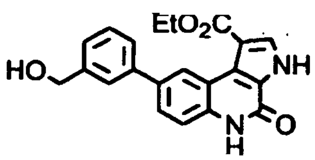 | 8-(3-Hydroxyméthyl-phényl)-4-oxo-4,5-dihydro-3*H*-pyrrolo[2,3-*c*]quinoline-1-carboxylate d'éthyle | B |
| 241 | 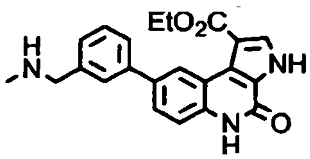 | 8-(3-Méthylaminométhyl-phényl)-4-oxo-4,5-dihydro-3*H*-pyrrolo[2,3-*c*]quinoline-1-carboxylate d'éthyle | B |
| 242 | 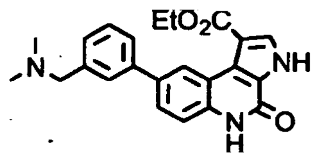 | 8-(3-Diméthylaminométhyl-phényl)-4-oxo-4,5-dihydro-3*H*-pyrrolo[2,3-*c*] quinoline-1-carboxylate d'éthyle | B |
| 243 | 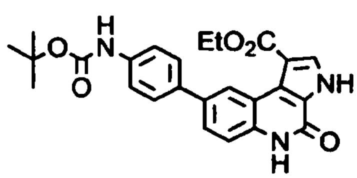 | 8-(4-*tert*-Butoxycarbonylamino-phényl)-4-oxo-4,5-dihydro-3*H*-pyrrolo[2,3-*c*] quinoline-1-carboxylate d'éthyle | C |

(suite)

| Composé | Formule | Nom | IC50 |
|---|---|---|---|
| 244 | 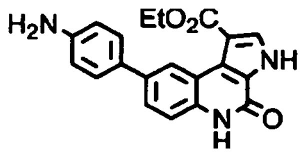 | 8-(4-Amino-phényl)-4-oxo-4,5-dihydro-3*H*-pyrrolo[2,3-*c*]quinoline-1-carboxylate d'éthyle | B |
| 245 | 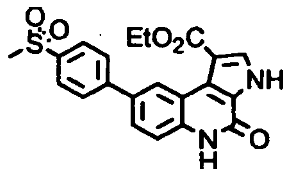 | 8-(4-Méthanesuifonyl-phényl)-4-oxo-4,5-dihydro-3*H*-pyrrolo[2,3-*c*]quinoline-1-carboxylate d'éthyle | C |
| 246 | 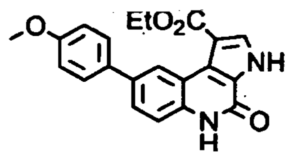 | 8-(4-Méthoxy-phényl)-4-oxo-4,5-dihydro-3*H*-pyrrolo[2,3-*c*]quinoline-1-carboxylate d'éthyle | C |
| 247 | 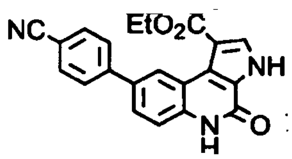 | 8-(4-Cyano-phényl)-4-oxo-4,5-dihydro-3*H*-pyrrolo[2,3-*c*]quinoline-1-carboxylate d'éthyle | D |
| 248 | 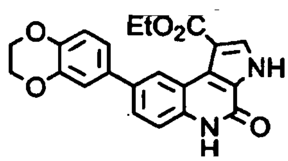 | 8-(2,3-Dihydro-benzo[1,4]dioxin-6-yl)-4-oxo4,5-dihydro-3*H*-pyrrolo[2,3-*c*] quinoline-1-carboxylaie d'éthyle | B |
| 249 | 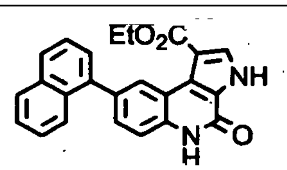 | 8-Naphthalèn-1-yl-4-oxo-4,5-dihydro-3*H*-pyrrolo[2,3-*c*]quinoline-1-carboxylate d'éthyle | B |
| 250 | 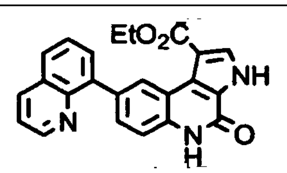 | 4-Oxo-8-quinolin-8-yl-4,5-dihydro-3*H*-pyrrolo[2,3-*c*]quinoline-1-carboxylate d'éthyle | C |
| 251 | 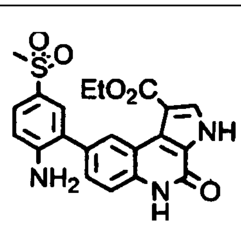 | 8-(2-Amino-5-méthanesulfonyl-phényl)-4-oxo-4,5-dihydro-3*H*-pyrrolo[2,3-*c*] quinoline-1-carboxylate d'éthyle | B |
| 252 | 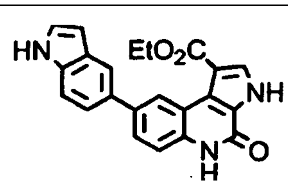 | 8-(1*H*-Indol-5-yl)-4-oxo-4,5-dihydro-3*H*-pyrrolo[2,3-*c*]quinoline-1-carboxylate d'éthyle | B |

(suite)

| Composé | Formule | Nom | IC50 |
|---|---|---|---|
| 253 | 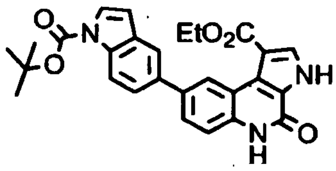 | 8-(1-*tert*-Butoxycarbonyl-1H-indol-5-yl)-4-oxo-4,5-dihydro-3*H*-pyrrolo[2,3-*c*]quinoline-1-carboxylate d'éthyle | C |
| 254 | 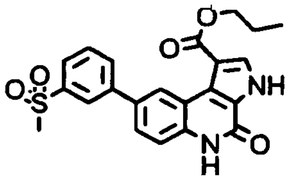 | 8-(3-Méthanesulfonyl-phényl)-4-oxo-4,5-dihydro-3*H*-pyrrolo[2,3-*c*]quinoline-1-carboxylate de propyle | B |
| 255 | 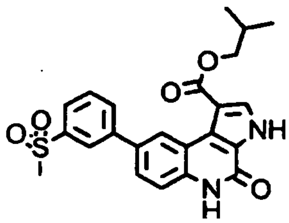 | 8-(3-Méthanesulfonyl-phenyl)-4-oxo-4,5-dihydro-3*H*-pyrrolo[2,3-*c*]quinoline-1-carboxylate d'isobutyle | C |
| 256 | 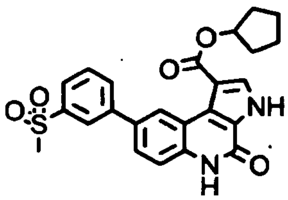 | 8-(3-Méthanesulfonyl-phényl)-4-oxo-4,5-dihydro-3*H*-pyrrolo[2,3-*c*]quinoline-1-carboxylate de cyclopentyle | B |
| 257 | 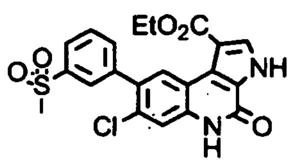 | 7-Chloro-8-(3-méthanesulfonyl-phényl)-4-oxo-4,5-dihydro-3*H*-pyrrolo[2,3-*c*]quinoline-1-carboxylate d'éthyle | B |
| 258 | 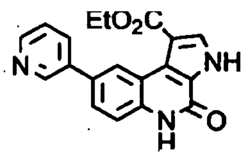 | 4-Oxo-8-pyridin-3-yl-4,5-dihydro-3*H*-pyrrolo[2,3-*c*]quinoline-1-carboxylate d'éthyle | C |
| 259 | 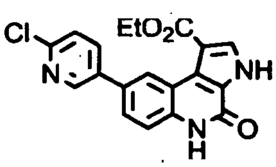 | 8-(6-Chloro-pyridin-3-yl)-4-oxo-4,5-dihydro-3*H*-pyrrolo[2,3-*c*]quinoline-1-carboxylate d'éthyle | C |
| 260 | 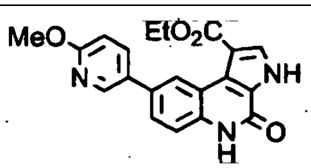 | 8-(6-Méthoxy-pyridin-3-yl)-4-oxo-4,5-dihydro-3*H*-pyrrolo[2,3-*c*]quinoline-1-carboxylate d'éthyle | C |

(suite)

| Composé | Formule | Nom | IC50 |
|---|---|---|---|
| 261 | 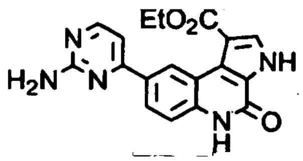 | 8-(2-Amino-pyrimidin-4-yl)-4-oxo-4,5-dihydro-3*H*-pyrrolo[2,3-*c*]quinoline-1-carboxylate d'éthyle | B |
| 262 | 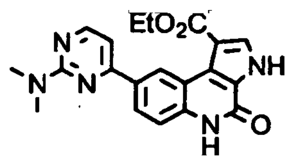 | 8-(2-Diméthylamino-pyrimidin-4-yl)-4-oxo-4,5-dihydro-3*H*-pyrrolo[2,3-*c*] quinoline-1-carboxylate d'éthyle | C |
| 263 | 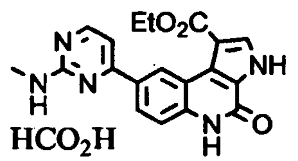 | Formiate de 8-(2-méthylamino-pyrimidin-4-yl)-4-oxo-4,5-dihydro-3*H*-pyrrolo[2,3-*c*]quinoline-1-carboxylate d'éthyle | B |
| 264 | 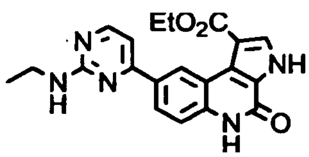 | 8-(2-Ethylamino-pyrimidin-4-yl)-4-oxo-4,5-dihydro-3*H*-pyrrolo[2,3-*c*]quinoline-1-carboxylate d'éthyle | B |
| 265 | 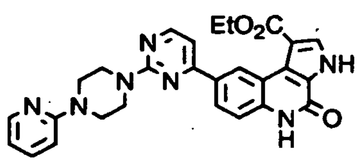 | 4-Oxo-8-[2-(4-pyridin-2-yl-pipérazin-1-yl)-pyrimidin-4-yl]-4,5-dihydro-3*H*-pyrrolo[2,3-*c*]quinoline-1-carboxylate d'éthyle | C |
| 266 | 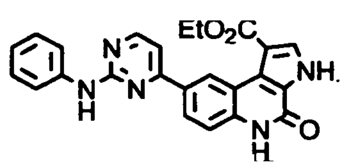 | 4-Oxo-8-(2-phénylamino-pyrimidin-4-yl)-4,5-dihydro-3*H*-pyrrolo[2,3-*c*] quinoline-1-carboxylate d'éthyle | C |
| 267 | 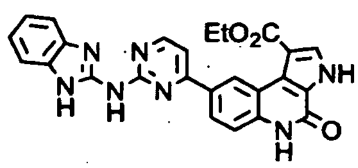 | 8-[2-(1*H*-Benzoimidazol-2-ylamino)-pyrimidin-4-yl]-4-oxo-4,5-dihydro-3*H*-pyrrolo[2,3-*c*]quinoline-1-carboxylate d'éthyle | C |
| 268 | 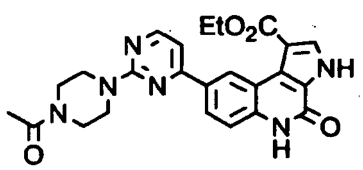 | 8-[2-(4-Acétyl-pipérazin-1-yl)-pyrimidin-4-yl]-4-oxo-4,5-dihydro-, 3*H*-pyrrolo [2,3-*c*]quinoline-1-carboxylate d'éthyle | C |
| 269 | 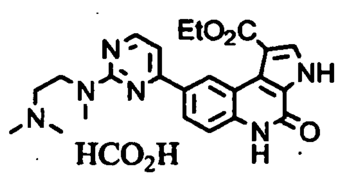 | Formiate de 8-{2-[(2-Diméthylamino-éthyl)-méthyl-amino]-pyrimidin-4-yl}-4-oxo-4,5-dihydro-3*H*-pyrrolo[2,3-*c*] quinoline-1-carboxylate d'éthyle | C |

**TABLEAU VIII**

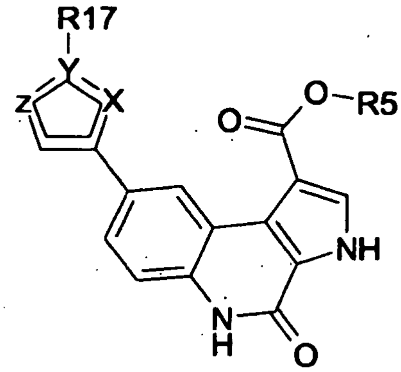

(IX)

| Composé | Formule | Nom | IC50 |
|---|---|---|---|
| 270 | 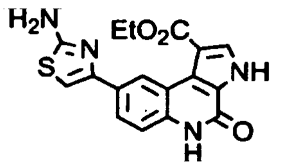 | 8-(2Amino-thiazol-4-yl)-4-oxo-4,5-dihydro-3*H*-pyrrolo[2,3-*c*]quinoline-1-carboxylate d'éthyle | B |
| 271 | 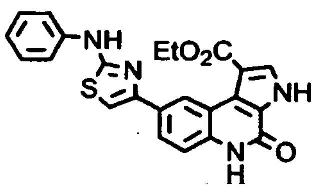 | 8-[2-phénylamino-thiazol-4-yl]-4-oxo-4,5-dihydro-3*H*-pyrrolo[2,3-*c*]quinoline-1-carboxylate d'éthyle | B |
| 272 | 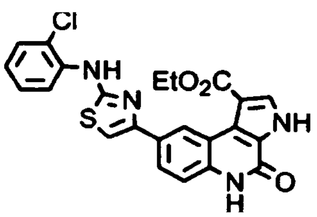 | 8-[2-(2-Chloro-phénylamino)-thiazol-4-yl]-4-oxo-4,5-dihydro-3*H*-pyrrolo[2,3-*c*]quinoline-1-cartoxylate d'éthyle | C |
| 273 | 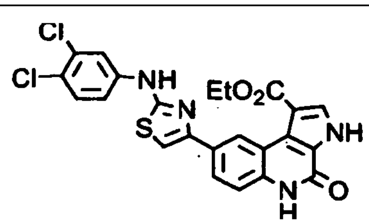 | 8-[2-(3,4-Dichloro-phénylamino)-thiazol-4-yl]-4-oxo-4,5-dihydro-3*H*-pyrrolo[2,3-*c*]quinoline-1-carboxylate d'éthyle | C |
| 274 | 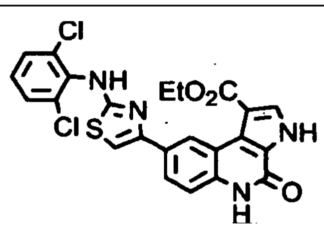 | 8-[2-(2,6-Dichloro-phénylamino)-thiazol-4-yl]-4-oxo-4,5-dihydro-3*H*-pyrrolo[2,3-*c*]quinoline-1-carboxylate d'éthyle | D |

(suite)

| Composé | Formule | Nom | IC50 |
|---|---|---|---|
| 275 | 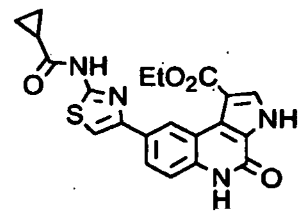 | 8-[2-(Cyclopropanecarbonyl-amino)-thiazol-4-yl]-4-oxo-4,5-dihydro-3*H*-pyrrolo[2,3-*c*]quinoline-1-carboxylate d'éthyle | B |
| 276 | 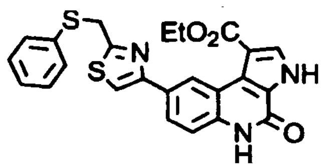 | 4-Oxo-8-(2-phénylsulfanylméthyl-thiazol-4-yl)-4,5-dihydro-3*H*-pyrrolo[2,3-*c*]quinoline-1-carboxylate d'éthyle | B |
| 277 | 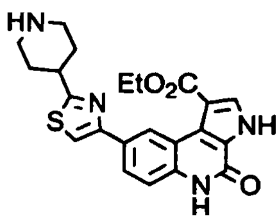 | 4-Oxo-8-(2-pipéridin4-yl-thiazol-4-yl)-4,5-dihydro-3*H*-pyrrolo[2,3-*c*]quinoline-1-carboxylate d'éthyle | B |
| 278 | 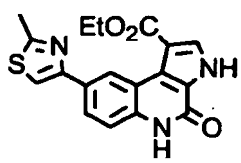 | 8-(2-Méthyl-thiazol-4-yl)-4-oxo-4,5-dihydro-3*H*-pyrrolo[2,3-*c*]quinoline-1-carboxylate d'éthyle | C |
| 279 | 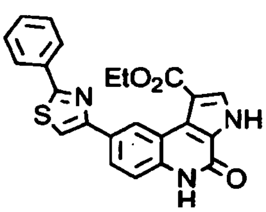 | 4-Oxo-8-(2-phényl-thiazol-4-yl)-4,5-dihydro-3*H*-pyrrolo[2,3-*c*]quinoline-1-carboxylate d'éthyle | C |
| 280 | 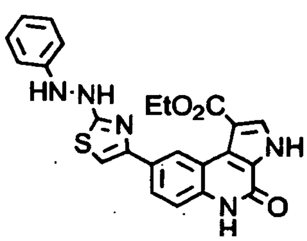 | 4-Oxo-8-[2-(*N'*-phényl-hydrazino)-thiazol-4-yl]-4,5-dihydro-3*H*-pyrrolo[2,3-*c*]quinoline-1-carboxylate d'éthyle | C |
| 281 | 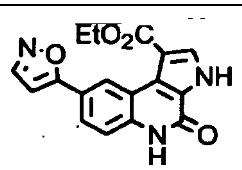 | 8-Isoxazol-5-yl-4-oxo-4,5-dihydro-3*H*-pyrrolo[2,3-*c*]quinoline-1-carboxylate d'éthyle | B |
| 282 | 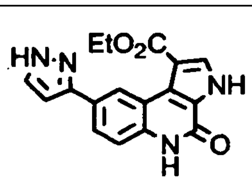 | 4-Oxo-8-(1*H*-pyrazol-3-yl)-4,5-dihydro-3*H*-pyrrolo[2,3-*c*]quinoline-1-carboxylate d'éthyle | B |

(suite)

| Composé | Formule | Nom | IC50 |
|---|---|---|---|
| 283 | 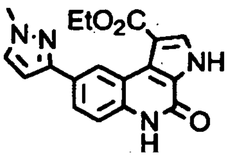 | 8-(1-Méthyl-1*H*-pyrazol-3-yl)-4-oxo-4,5-dihydro-3*H*-pyrrolo[2,3-*c*]quinoline-1-carboxylate d'éthyle | B |

**Exemple 3 : Test de mesure de l'inhibition de l'activité kinase de ALK, PIM-1, TrkA et TrkB**

L'inhibition de l'activité kinase de certains des composés de l'invention a également été estimée par l'inhibition par la mesure de leur IC50 sur d'autres kinases.

**Test de mesure de l'inhibition de la kinase PIM-1 :**

La protéine PIM-1 (Upstate #14-573 lot 25864U) est incubée à une concentration de 0,625μg/ml dans un tampon contenant 8mM de MOPS/NaOH pH7, 0,2mM EDTA, 5mM MgAc (Sigma #229768), 0,01% Triton Tx100, en présence de 70μM de peptide substrat S6 Kinase/Rsk2 (Upstate #12-243), et de 10μM d'ATP.

Le tampon de dilution de PIM-1 est le suivant: 20mM MOPS/NaOH pH7, 1mM EDTA, 5% Glycérol, 0.01% Brij35,0.1% 2-Mercapto, 1mg/ml BSA

Le composé à tester est ajouté préalablement à l'enzyme de manière à atteindre un volume réactionnel de 50μl. La dilution des composés de l'invention est répartie de façon à obtenir une échelle de concentrations finales comprises entre 10nM et 30μM.

Après une incubation de la réaction de quarante minutes à 30°C, le taux d'ATP restant est dosé en utilisant le Kinase-Glo Luminescent Kinase Assay (Promega #V6712) selon les conditions préconisées par le vendeur. La luminescence induite est lue dans un lecteur de plaque (Envision-Perkin Elmer).

**Test de mesure de l'inhibition de la kinase TrkA :**

La protéine TrkA (Upstate 14-571 ; lot #25654U) est incubée à une concentration de 4μg/ml dans un tampon contenant 8mM de MOPS/NaOH pH7, 0,2mM EDTA, 40mM MgAc (Sigma #229768), 1mM DTT (Fluka #43816), NaOV 1mM, 0,01% Triton Tx100, en présence de 1 mg/ml de peptide substrat poly-EY (Upstate #P0275), et de 30μM d'ATP.

Le tampon de dilution de TrkA est le suivant: 20mM MOPS/NaOH pH7, 1mM EDTA, 5% Glycérol, 0.01% Brij35, 0.1% 2-Mercapto, 1mg/ml BSA.

Le composé à tester est ajouté préalablement à l'enzyme de manière à atteindre un volume réactionnel de 50μl. La dilution des composés de l'invention est répartie de façon à obtenir une échelle de concentrations finales comprises entre 10nM et 30μM.

Après une incubation de la réaction de deux heures à 30°C, le taux d'ATP restant est dosé en utilisant le Kinase-Glo Luminescent Kinase Assay (Promega #V6712) selon les conditions préconisées par le vendeur. La luminescence induite est lue dans un lecteur de plaque (Envision-Perkin Elmer).

**Test de mesure de l'inhibition de la kinase TrkB :**

La protéine TrkB (Upstate 14-507 ; lot #25578U) est incubée à une concentration de 4μg/ml dans un tampon contenant 8mM de MOPS/NaOH pH7, 0,2mM EDTA, 40mM MgAc (Sigma #229768), 1mM DTT (Fluka #43816), NaOV 1mM, 25mM 2-glycerolphosphate (Sigma # G9891), en présence de 2,5mg/ml de peptide substrat poly-EY (Upstate #P0275), et de 40μM d'ATP.

Le tampon de dilution de TrkB est le suivant: 20mM MOPS/NaOH pH7, 1mM EDTA, 5% Glycérol, 0.01% Brij35, 0.1% 2-Mercapto, 1mg/ml BSA.

Le composé à tester est ajouté préalablement à l'enzyme de manière à atteindre un volume réactionnel de 50μl. La dilution des composés de l'invention est répartie de façon à obtenir une échelle de concentrations finales comprises entre 10nM et 30μM.

Après une incubation de la réaction de deux heures à 30°C, le taux d'ATP restant est dosé en utilisant le Kinase-Glo Luminescent Kinase Assay (Promega #V6712) selon les conditions préconisées par le vendeur. La luminescence induite est lue dans un lecteur de plaque (Envision-Perkin Elmer).

**Test de mesure de l'inhibition de la kinase ALK :**

Une plaque ViewPlate (Packard #6005182) est incubée avec le substrat PLCγ1-GST (forme recombinante purifiée) à 0,1mg/ml dans du tampon PBS (100µl/puit) durant une heure sous agitation. Ensuite, la plaque est saturée par une solution de bloquage comprenant 5% de BSA (Sigma #B-7906) dans du tampon PBS.

Après avoir ajouté l'inhibiteur à la concentration finale désirée (gamme habituelle comprise entre 30µM et 10nM) la réaction est effectuée en ajoutant la kinase ALK à 180ng/ml dans du tampon de réaction (Tris 13mM pH7,5, MgCl2 6,5mM, DTT 0,65mM, β-glycerophosphate. 39mM, NaOV 0,65mM) et 250µM d'ATP. L'incubation est menée durant 30 minutes à 30°C sous agitation.

Après trois lavages en tampon PBS/Tween-20 0,1% sous agitation, un anticorps anti-phosphotyrosine couplé à la HRP (UBI #16105) dilué au 1/1000 dans du tampon PBS contenant BSA 5mg/ml est incubé durant une heure sous agitation. Puis, après trois nouveaux lavages en PBS/tween, les puits sont incubés deux minutes avec 100µl de mélange SuperSignal ELISA (Pierce #37070).

Le signal est lu en mode luminescent à l'aide d'un luminomètre.

Le tableau IX ci-après regroupe quelques valeurs d'IC50 pour les composés de type II à IX sur les kinases ALK, PIM-1, TrkA et TrkB. Les valeurs d'IC50 sont données par les mêmes catégories que précédemment.

**Tableau IX**

| Composé | ALK | PIM-1 | TrkA | TrkB | | 63 | | D | | |
|---|---|---|---|---|---|---|---|---|---|---|
| 1 | | D | | | | 64 | | D | | |
| 2 | | | C | C | | 66 | | C | | |
| 3 | | D | C | D | | 70 | | C | | |
| 6 | | | D | D | | 71 | | C | | |
| 8 | | | D | D | | 79 | | D | | |
| 12 | B | | C | C | | 91 | B | C | | |
| 32 | D | D | | | | 93 | B | C | | |
| 33 | C | D | | | | 94 | B | | | |
| 34 | | C | | | | 95 | | D | | |
| 37 | | D | C | C | | 97 | B | D | | |
| 43 | | | C | C | | 111 | | | C | C |
| 44 | | | C | C | | 112 | B | C | | |
| 45 | | | C | C | | 113 | C | C | | |
| 49 | | | D | C | | 114 | | C | D | D |
| 56 | | | C | C | | 115 | | C | D | D |
| 58 | | | C | C | | 116 | | C | | |

(suite)

| | | | | | | | | | | |
|---|---|---|---|---|---|---|---|---|---|---|
| 117 | | C | D | D | | 227 | | C | C | C |
| 118 | | D | | | | 234 | | | C | C |
| 120 | | D | C | D | | 237 | | C | C | B |
| 121 | | C | | | | 240 | | C | C | C |
| 123 | C | | | | | 241 | | C | | |
| 127 | | D | C | C | | 242 | | C | D | D |
| 130 | B | | | | | 244 | | B | | |
| 141 | B | | | | | 259 | | C | | |
| 144 | B | | | | | 261 | | D | | |
| 145 | C | | | | | 262 | | C | | |
| 149 | B | | | | | 263 | | C | | |
| 150 | B | | | | | 264 | | C | D | D |
| 153 | B | | | | | 266 | | | C | C |
| 155 | B | | | | | 267 | | | D | D |
| 162 | | | C | C | | 268 | | C | | |
| 177 | B | | | | | 269 | | C | | |
| 209 | | B | | | | 270 | | D | | |
| 219 | C | | | | | 271 | B | D | C | C |
| 221 | | C | | | | 277 | | C | | |
| 222 | | C | | | | 282 | | D | D | D |

**Exemple 4 : Test de mesure de l'inhibition de l'activité d'un panel de kinases par les composés décrits dans l'invention:**

Ces kinases sont produites par Upstate et criblées selon les protocoles respectifs indiqués par le prestataire (http://www.upstate.com/features/kp protocols.asp).
Les exemples 1, 12, 166 et 238 ont été testés à une concentration respective de 20μM, 10μM, 1μM et 1μM en présence de 10μM d'ATP pour les exemples 1 et 12 et à des concentrations d'ATP indiquées entre parenthèses pour les exemples 166 et 238. Les résultats indiqués tableau VII correspondent à un pourcentage d'inhibition de la kinase testée en présence du composé, par rapport à un contrôle effectué sans le composé

Les résultats sont rapportés dans le Tableau X ci-après.

**TABLEAU X**

| Kinase | Composé 1 % inhibition à 20 μMol | Composé 12 % inhibition à 10 μMol | Composé 166 % inhibition à 1 μMol | Composé 238 % inhibition à 1 μMol |
|---|---|---|---|---|
| **Aurora-A (h)** | 16 | **94** | 6 (15) | 24 (15) |
| **c-RAF (h)** | 17 | **79** | 0 (45) | 3 (45) |
| **CDK2/cyclinE (h)** | **97** | 12 | | |
| **cKit (h)** | 20 | 45 | 15 (200) | 4 (200) |
| **cSRC (h)** | **86** | **91** | 13 (200) | 7 (200) |
| **EGFR (h)** | 19 | 13 | 3 (10) | 3 (10) |
| **FGFR1 (h)** | | | **71 (200)** | **51 (200)** |
| **FGFR2 (h)** | **82** | **91** | | |

(suite)

| Kinase | Composé 1 % inhibition à 20 μMol | Composé 12 % inhibition à 10 μMol | Composé 166 % inhibition à 1 μMol | Composé 238 % inhibition à 1 μMol |
|---|---|---|---|---|
| **Flt3 (h)** | **91** | **51** | 0 (200) | 0 (200) |
| **GSK3B (h)** | | 22 | 0 (15) | 0 (15) |
| **Hck (h)** | | **91** | | |
| **IGF-1R (h)** | 42 | 0 | | |
| **IKKβ (h)** | 22 | 0 | 0 (10) | 0 (10) |
| **JAK3 (h)** | **58** | **50** | 0 (10) | 5 (10) |
| **KDR (h)** | **100** | **69** | 9 (90) | 21 (90) |
| **MAPK2 (h)** | 1 | 3 | 0 (155) | 0 (155) |
| **Mct (h)** | **52** | | | |
| **MLCK (h)** | | | **75 (10)** | 21 (10) |
| **PDGFRβ (h)** | 13 | 0 | 23 (120) | 0 (120) |
| **PIM-1 (h)** | **92** | | | |
| **PKBγ (h)** | 5 | 20 | **0 (200)** | 2 (200) |
| **RET (h)** | **99** | | | |
| **ROCK-II (h)** | 8 | 17 | 3 (15) | 5 (15) |
| **Rsk2 (h)** | | | **60 (10)** | 25 (10) |
| **TrkA (h)** | **97** | | | |
| **TrkB (h)** | | **98** | | |

**Exemple 5 : Test de mesure de l'inhibition de la prolifération d'un panel de lignées cellulaires par les composés décrits dans l'invention**

L'inhibition de la prolifération de lignées cellulaires par certains composés de l'invention a été testée. D'une manière générale, les lignées de cellules de thyroïde, et en particulier les cellules issues de cancers de thyroïde médullaire (MTC) présentent une prolifération fortement inhibée par la plupart des composés de l'invention. Dans l'exemple, des lignées dérivées de cancers de l'utérus, du système nerveux central, du colon, de l'estomac, de la tête et cou, du poumon, du sein, du pancréas, du rein ainsi que de lymphome, de myélome, de la plèvre, ou de mélanome ont été utilisées.

Le test WST de mesure de prolifération et de viabilité cellulaire utilisé (Roche Applied Science) est basé sur le clivage de sel de tétrazolium par les dehydrogénases mitochondriales dans les cellules viables.

Après quatre jours d'incubation des composés avec la lignée cellulaire, 14μl de réactif WST-1 est ajouté dans chaque puit et les plaques sont incubées durant quatre heures à 37° en atmosphère humide en 5%CO2. Le taux de formazan formé est alors dosé en spectrophotométrie à 450nm alors qu'une référence est mesurée à 650nm. Un contrôle avec des cellules non traitées est représenté par six puits par plaques et le groupe des cellules traitées est produit en triplicat pour chacune des concentrations mesurées. Les composés sont testés à cinq concentrations comprises entre 10nM et 100μM et deux agents cytotoxiques sont utilisés en tant que contrôles: l'adriamycine et le 5-fluorouracile.

Les résultats obtenus (tableau XI pour l'exemple 238) montrent une inhibition de la prolifération par le composé 238, en particulier dans les cellules de poumon, les cellules provenant du système nerveux central, les cellules de sein et dans une moindre mesure, les cellules d'utérus, de la plèvre, de mélanome, du pancréas, de rein et les lymphocytes. Un autre composé testé, le composé 166 donne des résultats similaires à ceux obtenus avec l'exemple 238 sur ces lignées, avec des taux d'inhibition légèrement inférieurs.

Tableau XI: Activité antiproliférative *in vitro* du composé 238 en lignées cellulaires

| lignée | tissu d'origine | IC50 (μM) | lignée | tissu d'origine | IC50 (μM) |
|---|---|---|---|---|---|
| U251 | CNS | 33 | 536L | Tête/cou | 36 |

(suite)

| lignée | tissu d'origine | IC50 (μM) | lignée | tissu d'origine | IC50 (μM) |
|---|---|---|---|---|---|
| SKNAS | CNS | 12 | A427 | Poumon (NSCLC) | 36 |
| A172 | CNS | 13 | H69 | Poumon (SCLC) | 0,5 |
| MDA-453 | Sein | 7 | RAJI | Lymphome | 34 |
| MDA-468 | Sein | 38 | HT144 | Mélanome | 31 |
| MDA-231 | Sein | 41 | CAPAN2 | Pancréas | 47 |
| SW756 | Col de l'uterus | 35 | 1752L | Pleuromésothélium | 35 |
| SKUT1B | Utérus | 33 | A498 | Rein | 49 |

**Revendications**

**1.** Composé de formule I :

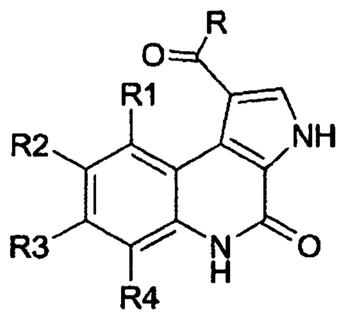

(I)

dans laquelle :

R représente un atome d'hydrogène ou un groupe choisi parmi les radicaux alkyle, alkényle, alkynyle, cycloalkyle, aralkyle, aryle, hétéroaryle -O-R5, ou -NRS-R6, éventuellement substitués par un ou plusieurs substituants,
R1 et R4 représentent indépendamment un atome d'hydrogène, un halogène ou un groupe choisi parmi les radicaux nitro, alkyle, alkényle, alkynyle, alcoxy ou cycloalkyle, éventuellement substitués par un ou plusieurs substituants,
R2 et R3 représentent un atome d'hydrogène, un halogène ou un groupe choisi parmi les radicaux alkyle alkényle, alkynyle, alcoxy, aryle, hétéroaryle, aryloxy, aralkyloxy, éventuellement substitués par un ou plusieurs substituants, et les radicaux hydroxy, cyano, -Y-(NR7)n-R8, -SOm-R8, -NR7-(Y)n-R8, -NO2, COOR9,
n = 0 ou 1
m = 0 ou 1
Y = -CO- ou -SO2-
R2 et R3 n'étant pas simultanément sur le même composé les groupes suivants : aryle, hétéroaryle, aryloxy, aralkyloxy, -Y-(NR7)n-R8, -SOm-R8, -NR7-(Y)n-R8, -NO2, COOR9,
R5 représente un atome d'hydrogène ou un radical alkyle, alkényle d'au moins 3 atomes de carbones non directement branché sur l'insaturation, alkynyle d'au moins 3 atomes de carbone non directement branché sur l'insaturation, cycloalkyle, cycloalkylalkyle, aralkyle, hétéroaralkyle, hétérocycle saturé non azoté alkyle éventuellement substitué par un ou plusieurs substituants,
R6 représente un atome d'hydrogène ou un radical alkyle, alkényle d'au moins 3 atomes de carbones non directement branché sur l'insaturation, alkynyle d'au moins 3 atomes de carbone non directement branché sur l'insaturation, cycloalkyle, cycloalkylalkyle, aryle, hétéroaryle, aralkyle, hétéroaralkyle, hétérocycle saturé non azoté alkyle éventuellement substitué par un ou plusieurs substituants,
R7 représente un atome d'hydrogène ou un radical alkyle, alkényle d'au moins 3 atomes de carbone non directement branché sur l'insaturation, alkynyle d'au moins 3 atomes de carbone non directement branché sur l'insaturation, cycloalkyle, cycloalkylalkyle, éventuellement substitué par un ou plusieurs substituants,
R8 représente un atome d'hydrogène ou un groupe choisi parmi les radicaux alkyle, alkényle, alkynyle, alcoxy,

cycloalkyle, cycloalkylalkyle, aralkyle, aryle, hétéroaryle, hétéroaralkyle, hétérocycle saturé ou hétérocycle saturé alkyle, éventuellement substitués par un ou plusieurs substituants,
R9 représente un atome d'hydrogène ou un groupe choisi parmi les radicaux alkyle, alkényle, alkynyle, cycloalkyle, cycloalkylalkyle, aralkyle, aryle, hétéroaralkyle, hétérocycle saturé ou hétérocycle saturé alkyle, éventuellement substitués par un ou plusieurs substituants,
R7 et R8 pouvant former ensemble un cycle ou un bicycle contenant 1 ou plusieurs hétéroatomes avec l'azote auquel ils sont rattachés ledit hétérocycle étant éventuellement substitué par un ou plusieurs groupes alkyle, alkényle, alkynyle, amino, NR10R11, hydroxy, alcoxy, alcoyle, carbamoyle, sulfamoyle, sulfonyle, sulfényle, sulfanyle, cyano ou un radical oxo,
R10 représente un atome d'hydrogène ou un radical alkyle, alkényle d'au moins 3 atomes de carbone non directement branché sur l'insaturation, alkynyle d'au moins 3 atomes de carbone non directement branché sur l'insaturation, cycloalkyle, cycloalkylalkyle, éventuellement substitué par un ou plusieurs substituants,
R11 représente indépendamment un atome d'hydrogène ou un groupe choisi parmi les radicaux alkyle, alkényle, alkynyle, cycloalkyle, cycloalkylalkyle, aralkyle, aryle, hétéroaryle, hétéroaralkyle, hétérocycle saturé ou hétérocycle saturé alkyle, alcoyle, alcoxycarbonyle, carbamoyle, sulfamoyle, éventuellement substitués par un ou plusieurs substituants,
R10 et R11 pouvant former ensemble un cycle contenant 1 ou plusieurs hétéroatomes avec l'azote auquel ils sont rattachés ledit hétérocycle étant éventuellement substitué

et leurs sels physiologiquement acceptables
à l'exception des composés suivants :

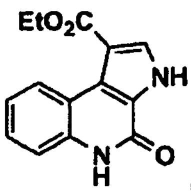 , 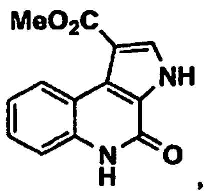 , 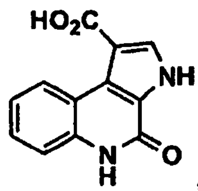 , 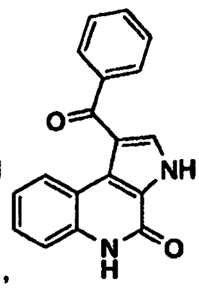

étant entendu que
par alkyle, on entend les radicaux alkyles linéaires ou ramifiés C1, C2, C3, C4, C5 ou C6, cette définition s'appliquant également aux parties alkyles des radicaux alcoxy, alcoxy-carbonyle, carbonylamino, carbonyle, aralkyle ou alkoxycarbonyl-alkyle,
par alkényle, on entend une chaîne hydrocarbonée, monovalente, insaturée et comprenant au moins une double liaison, linéaire ou ramifiée, comportant de 2 à 6 atomes de carbone,
par alkynyle, on entend une chaîne hydrocarbonée, monovalente, insaturée et comprenant au moins une triple liaison, linéaire ou ramifiée, comportant de 2 à 6 atomes de carbone,
par cycloalkyle, on entend les cycloalkyles en C3-C7,
par aryle, on entend un ou plusieurs cycles aromatiques ayant 6 à 10 atomes de carbone, pouvant être accolés ou fusionnés,
par hétéroaryle, on entend un ou plusieurs cycles aromatiques ayant 5 à 10 atomes, pouvant être accolés ou fusionnés, comprenant dans son cycle au moins un hétéroatome choisi parmi l'azote, l'oxygène ou le soufre,
les substituants étant choisis parmi les halogènes, les groupes hydroxyle, amino (amine primaire, secondaire ou tertiaire), nitro, les radicaux alkyle en C1-C4, les radicaux perfluoroalkyle en C1-C4, alcoxy, perfluoroalcoxy, alcoxycarbonyle, cycloalkyle, carbamoyle, sulfamoyle, sulfonyle, sulfényle, sulfanyle, carbonylamino, aryle, hétéroaryle, cyano ou un radical oxo.

**2.** Composé selon la revendication 1, **caractérisé en ce qu'**il répond à la formule générale I' :

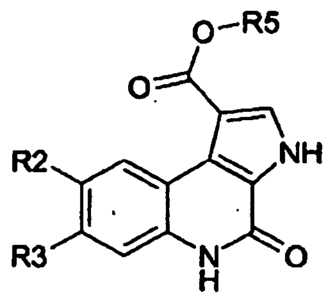

(I')

dans laquelle R2, R3 et R5 sont définis dans la revendication 1.

**3.** Composé selon l'une des revendications 1 ou 2, **caractérisé en ce que :**

R2 est choisi parmi les radicaux -S02-(NR7)n-R8 ou -NR7-(Y)n-R8,
n, R7 et R8 étant définis dans la revendication 1.

**4.** Composé selon l'une des revendications 1 à 3, **caractérisé en ce que :**

- R5 est choisi parmi les groupes méthyle, éthyle, propyle, iso-propyle, butyle, iso-butyle, iso-pentyle, allyle, isoprènyle, propargyle, cyclopentyle, cyclohexyle, cyclopropylméthyle, cyclopentylméthyle, cyclohexylméthyle, tétrahydropyran-4-yl-méthyle ou tétrahydrofuran-3-yl-méthyle, et/ou
- R7 est un hydrogène ou choisi parmi les groupes méthyle, éthyle, propyle, iso-propyle, butyle, iso-butyle, allyle, isoprènyle, propargyle cyclopropyle, cyclopentyle, cyclohexyle, cyclopropylméthyle, cyclopentylméthyle, cyclohexylméthyle, tétrahydropyran-4-yl-méthyle, tétrahydtofuran-3-yl-méthyle, hydroxy-2-éthyle, et/ou
- R8 est choisi parmi les groupes méthyle, hydroxy-2-éthyle, amino-2-éthyle, méthylamino-2-éthyle, diméthylamino-2-éthyle, cyanométhyle, carbamoylméthyle, 2-cyanoéthyle, cyclohexyle, cycloheptyle, cyclopropylméthyle, cyclopentylméthyle, cyclohexylméthyle, pipéridin-4-yle, pipéridin-3-yle, pipéridin-2-yle, pipéridin-4-méthyle, pipéridin-3-méthyle, pipéridin-2-méthyle, pyrrolidin-3-yl-méthyle, pyrrolidin-2-yl-méthyle, pyrrolidin-3-yle, azétidin-3-yl-méthyle, tétrahydro-thiopyran-4-yle, tétrahydropyran-4-yle, imidazol-4-yl-méthyle, phényle, o-tolyle, m-tolyle, p-tolyle, 2-fluoro-phényle, 3-fluoro-phényle, 4-fluoro-phényle, 2-chloro-phényle, 3-chloro-phényle, 4-chloro-phényle, 3-hydroxy-phényle, 2-méthoxy-phényle, 3-méthoxy-phényle, 4-méthoxy-phényle, 4-amino-phényle, 4-nitro-phényle, 4-fluoro-2-méthyl-phényle, 3-fluoro-2-méthyl-phényle, 3,4-difluorophényle, 3,5-difluorophényle, 2,6-dichloro-phényle, pyridyle, thiophène, 5-ehloro-thiophène ou benzyle,
- R7 et R8 pouvant former avec l'azote auquel ils sont rattachés un cycle 2,3-dihydro-indolyle, pipéridinyle, pipérazinyle, pyrrolidinyle ou morpholinyle.

**5.** Composé selon la revendication 4, **caractérisé en ce que** les substituants sont choisis parmi les halogènes, les groupes hydroxyle, amino (amine primaire, secondaire ou tertiaire), nitro, les radicaux alkyle en C1-C4, les radicaux perfluoroalkyle en C1-C4, alcoxy, perfluoroalcoxy, alcoxycarbonyle, carbamoyle, sulfamoyle, sulfonyle, sulfényle, sulfanyle, cyano ou un radical oxo, carbonylamino, aryle, hétéroaryleou cycloalkyle.

**6.** Composé selon l'une des revendications 1 à 5, **caractérisé en ce qu'**il répond à la formule générale (II) :

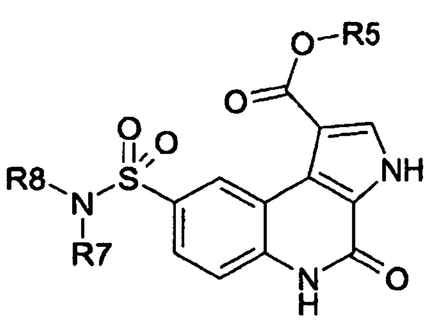

(II),

R5, R7 et R8 étant définis dans les revendications 1 à 5.

**7.** Composé selon la revendication 6, choisi parmi les composés suivants : et leurs sels physiologiquement acceptables :

4-Oxo-8-phénylsulfamoyl-4,5-dihydro-3*H*-pyrrolo[2,3-*c*]quinoline-1-carboxylate d'éthyle
8-(Méthyl-phényl-sulfamoyl)-4-oxo-4,5-dihydry-3*H*-pyrrolo[2,3-*c*]quinoline-1-carboxylate d'éthyle
4-Oxo-8-o-tolylsulfamoyl-4,5-dihydro-3*H*-pyrrolo[2,3-*c*]quinoline-1-carboxylate d'éthyle
4-Oxo-8-*m*-tolylsulfamoyl-4,5-dihydro-3*H*-pyrrolo[2,3-*c*]quinoline-1-carboxylate d'éthyle
4-Oxo-8-*p*-totylsulfamoyl-4,5-dihydro-3*H*-pyrrolo[2,3-*c*]quinoline-1-carboxylate d'éthyle
8-(2-Méthoxy-phénylsulfamoyl)-4-oxo-4,5-dihydro-3*H*-pyrrolo[2,3-*c*]quinoline-1-carboxylate d'éthyle
8-(3-Méthoxy-phénylsulfamoyl)-4-oxo-4,5-dihydro-3*H*-pyrrolo[2,3-*c*]quinoline-1-carboxylate d'éthyle
8-(4-Méthoxy-phénylsulfamoyl)-4-oxo-4,5-dihydro-3*H*-pyrrolo[2,3-*c*]quinoline-1-carboxylate d'éthyle
8-(3-Chloro-phénylsulfamoyl)-4-oxo-4,5-dihydro-3*H*-pyrrolo[2,3-*c*]quinoline-1-carboxylate d'éthyle
8-(4-Chloro-phénylsulfamoyl)-4-oxo-4,5-dihydro-3*H*-pyrrolo[2,3-*c*]quinoline-1-carboxylate d'éthyle
8-(4-Fluoro-phénylsulfamoyl)-4-oxo-4,5-dihydro-3*H*-pyrrolo[2,3-*c*]quinoline-1-carboxylate d'éthyle
8-[(4-Fluoro-phényl)-méthyl-sulfamoyl)-4-oxo-4,5-dihydro-3*H*-pyrrolo[2,3-*c*]quinoline-1-carboxylate d'éthyle
Acide 8-[(4-Fluoro-phényl)-méthyl-sulfamoyl]-4-oxo-4,5-dihydro-3*H*-pyrrolo[2,3-*c*]quinoline-1-carboxylique
8-[(4-Fluoro-phényl)-méthyl-sulfamoyl]-4-oxo-4,5-dihydro-3*H*-pyrrolo[2,3-*c*]quinoline-1-carboxylate de 2-hydroxyéthyle
Chlorhydrate de 8-[(4-fluoro-phényl)-méthyl-sulfamoyl]-4-oxo-4,5-dihydro-3*H*-pyrrolo[2,3-*c*]quinoline-1-carboxylate de 2-aminoéthyle
8-[(4-Fluoro-phényl)-méthyl-sulfamoyl]-4-oxo-4,5-dihydro-3*H*-pyrrolo[2,3-*c*]quinoline-1-carboxylate de 2-*tert*-butoxycarbonylaminoéthyle
Chlorhydrate de 8-[(4-fluoro-phényl)-méthyl-sulfamoyl]-4-oxo-4,5-dihydro-3*H*-pyrrolo[2,3-*c*]quinoline-1-carboxylate de 3-aminopropyle
8-[(4-Fluoro-phényl)-méthyl-sulfamoyl]-4-oxo-4,5-dihydro-3*H*-pyrrolo[2,3-*c*]quinoline-1-carboxylate de 3-*tert*-butoxycarbonylamino-propyle
8-(4-Ethoxycarbonyl-phénylsulfamoyi)-4-oxo-4,5-dihydro-3*H*-pyrrolo[2,3-*c*]quinoline-1-carboxylate d'éthyle
8-(3-Hydroxy-phénylsulfamoyl)-4-oxo-4,5-dihydro-3*H*-pyrrolo[2,3-*c*]quinoline-1-carboxylate d'éthyle
8-(3-Fluoro-2-méthyl-phénylsulfamoyl)-4-oxo-4,5-dihydro-3*H*-pyrrolo[2,3-*c*]quinoline-1-carboxylate d'éthyle
8-(4-Fluoro-2-méthyl-phénylsulfamoyl)-4-oxo-4,5-dihydro-3*H*-pyrrolo[2,3-*c*]quinoline-1-carboxylate d'éthyle
8-(3,4-Difluoro-phénylsulfamoyl)-4-oxo-4,5-dihydro-3*H*-pyrrolo[2,3-*c*]quinoline-1-carboxylate d'éthyle
8-(3,5-Difluoro-phénylsulfamoyl)-4-oxo-4,5-dihydro-3*H*-pyrrolo[2,3-*c*]quinoline-1-carboxylate d'éthyle
8-(4-Amino-phénylsulfamoyl)-4-oxo-4,5-dihydro-3*H*-pyrrolo[2,3-*c*]quinoline-1-carboxylate d'éthyle
8-(2,3-Dihydro-indole-1-sulfonyl)-4-oxo-4,5-dihydro-3*H*-pyrrolo[2,3-*c*]quinoline-1-carboxylate d'éthyle
8-(4-Morpholin-4-yl-phénylsulfamoyl)-4-oxo-4,5-dihydro-3*H*-pyrrolo[2,3-*c*]quinoline-1-carboxylate d'éthyle
4-Oxo-8-(pyridin-3-ylsulfamoyl)- 4,5-dihydro-3*H*-pyrrolo[2,3-*c*]quinoline-1-carboxylate d'éthyle 8-(Benzyl-méthyl-sulfamoyl)-4-oxo-4,5-dihydro-3*H*-pyrrolo[2,3-*c*]quinoline-1-carboxylate d'éthyle
8-(Méthyl-phénétyl-sulfamoyl)-4-oxo-4,5-dihydro-3*H*-pyrrolo[2,3-*c*]quinoline-1-carboxylate d'éthyle
8-Diméthylsulfamoyl-4-oxo-4,5-dihydro-3*H*-pyrrolo[2,3-*c*]quinoline-1-carboxylate d'éthyle
*N*-propyl-8-diméthylsulfamoyl-4-oxo-4,5-dihydro-3*H*-pyrrolo[2,3-*c*]quinoline-1-carboxamide
*N*-Méthyl-*N*-propyl-8-diméthylsulfainoyl-4-oxo-4,5-dihydro-3*H*-pyrrolo[2,3-*c*]quinoline-1-carboxamide
8-Méthylsulfamoyl-4-oxo-4,5-dihydro-3*H*-pyrrolo[2,3-*c*]quinoline-1-carboxylate d'éthyle
8-(Morpholine-4-sulfonyl)-4-oxo-4,5-dihydro-3*H*-pyrrolo[2,3-*c*]quinoline-1-carboxylate d'éthyle
8-(2-Ethoxycarbonyl-éthylsulfamoyl)-4-oxo-4,5-dihydro-3*H*-pyrrolo[2,3-*c*]quinoline-1-carboxylate d'éthyle
8-Cyclohexylsulfamoyl-4-oxo-4,5-dihydro-3*H*-pyrrolo[2,3-*c*]quinoline-1-carboxylate de méthyle
8-Cyclohexylsulfamoyl-4-oxo-4,5-dihydro-3*H*-pyrrolo[2,3-*c*]quinoline-1-carboxylate d'éthyle
8-Cycloheptylsulfamoyl-4-oxo-4,5-dihydro-3*H*-pyrrolo[2,3-*c*]quinoline-1-carboxylate d'éthyle
8-(3-Diméthylamino-propylsulfamoyl)-4-oxo-4,5-dihydro-3*H*-pyrrolo[2,3-*c*]quinoline-1-carboxylate d'éthyle
8-(4-Amino-butylsulfamoyl)-4-oxo-4,5-dihydro-3*H*-pyrrolo[2,3-*c*]quinoline-1-carboxylate d'éthyle
8-[(3-Chloro-phényl)-méthyl-sulfamoyl)-4-oxo-4,5-dihydro-3*H*-pyrrolo[2,3-*c*]quinoline-1-carboxylate d'éthyle
4-Oxo-8-(pipéridin-4-ylsulfamoyl)-4,5-dihydro-3*H*-pyrrolo[2,3-*c*]quinoline-1-carboxylate d'éthyle
4-Oxo-à-(pipéridin-3-ylsulfamoyl)-4,5-dihydro-3*H*-pyrrolo[2,3-*c*]quinoline-1-carboxylate de méthyle
4-Oxo-8-(pipéridin-4-ylsulfamoyl)-4,5-dihydro-3*H*-pyrrolo[2,3-*c*]quinoline-1-carboxylate de méthyle
4-Oxo-8-(pipéridin-4-ylsulfamoyl)-4,5-dihydro-3*H*-pyrrolo[2,3-*c*]quinoline-1-carboxylate de propyle
4-Oxo-9-(pipéridin-4-ylsulfamoyl)-4,5-dihydro-3*H*-pyrrolo[2,3-*c*]quinoline-1-carboxylate de butyle
4-Oxo-8-(pipéridin-4-ylsulfamoyl)-4,5-dihydro-3*H*-pyrrolo[2,3-*c*]quinoline-1-carboxylate de pentyle

4-Oxo-8-(pipéridin-4-ylsulfamoyl)-4,5-dihydro-3*H*-pyrrolo[2,3-*c*]quinoline-1-carboxylate de méthylecyclohexyle
4-Oxo-8-(pipéridin-4-yisulfamoyl)-4,5-dihydro-3*H*-pyrrolo[2,3-*c*]quinoline-1-carboxylate de méthyltétrahydrofuran-3-yle
8-(1-Méthyl-pipéridin-4-ylsulfamoyl)-4-oxo-4,5-dihydro-3*H*-pyrrolo[2,3-*c*]quinoline-1-carboxylate de cyclopentyle
8-(Méthyl-pipéridin-4-yl-sulfamoyl)-4-oxo-4,5-dihydro-3*H*-pyrrolo[2,3-*c*]quinoline-1-carboxylate de propyle
4-Oxo-8-[(pipéridin-4-ylméthyl)-sulfamoyl]-4,5-dihydro-3*H*-pyrrolo[2,3-c]quinoline-1-carboxylate d'éthyle
8-(8-Aza-bicyclo[3.2.1]oct-3-ylsulfamoyl)-4-oxo-4,5-dihydro-3*H*-pyrrolo[2,3-*c*]quinoline-1-carboxylate d'éthyle
8-Cyclopentylsulfamoyl-4-oxo-4,5-dihydro-3*H*-pyrrolo[2,3-*c*]quinoline-1-carboxylate de méthyle
8-(Hexahydro-pyrrolo[3,4-c]pyrrole-2-sulfonyl)-4-oxo-4,5-dihydro-3*H*-pyrrolo[2,3-*c*]quinoline-1-carboxylate d'éthyle
8-(1-Azabicyclo[2.2.2]oct-3-ylsulfamoyl)-4-oxo-4,5-dihydro-3*H*-pyrrolo[2,3-c]quinoline-1-carboxylate d'éthyle
8-(4-Méthyl-pipérazine-1-sulfonyl)-4-oxo-4,5-dihydro-3*H*-pyrrolo[2,3-*c*]quinoline-1-carboxylate d'éthyle
4-Oxo-8-sulfamoyl-4,5-dihydro-3*H*-pyrrolo[2,3-*c*]quinoline-1-carboxylate d'éthyle
4-Oxo-8-[(pyrrolidin-3-méthyl)-sulfamoyl]-4,5-dihydro-3*H*-pyrrolo[2,3-*c*]quinoline-1-carboxylate d'éthyle
4-Oxo-8-(4-Hydroxy-phénylsulfamoyl)-4,5-dihydro-3*H*-pyrrolo[2,3-*c*]quinoline-1-carboxylate d'éthyle
8-Cyclopropylsulfamoyl-4-oxo-4,5-dihydro-3*H*-pyrrolo[2,3-*c*]quinoline-1-carboxylate d'éthyle
8-Cyclopropylsulfamoyl-4-oxo-4,5-dihydro-3*H*-pyrrolo[2,3-*c*]quinoline-1-carboxylate de propyle
8-(Cyclopropyl-méthyl-sulfamoyl)-4-oxo-4,5-dihydro-3*H*-pyrrolo[2,3-*c*]quinoline-1-carboxylate de propyle
8-[(2-Cyano-éthyl)-méthyl-sulfamoyl]-4-oxo-4,5-dihydro-3*H*-pyrrolo[2,3-*c*]quinoline-1-carboxylate de propyle
8-[(2-Cyano-éthyl)-cyclopropyl-sulfamoyl]-4-oxo-4,5-dihydro-3*H*-pyrrolo[2,3-*c*]quinoline-1-carboxylate d'éthyle
8-(Cyanométhyl-méthyl-sulfamoyl)-4-oxo-4,5-dihydro-3*H*-pyrrolo[2,3-*c*]quinoline-1-carboxylate de propyle
8-([1,3]Dioxolan-2-ylméthyl-méthyl-sulfamoyl)-4-oxo-4,5-dihydro-3*H*-pyrrolo[2,3-*c*]quinoline-1-carboxylate de propyle
8-[(1-Benzhydryl-azétidin-3-yl)-méthyl-sulfamoyl]-4-oxo-4,5-dihydro-3*H*-pyrrolo[2,3-*c*]quinoline-1-carboxylate de propyle
8-[Méthyl-(tétrahydro-thiopyran-4-yl)-sulfamoyl]-4-oxo-4,5-dihydro-3*H*-pyrrolo[2,3-*c*]quinoline-1-carboxylate de propyle
8-[(1,1-Dioxo-hexahydro-116-thiopyran-4-yl)-méthyl-sulfamoyl]-4-oxo-4,5-dihydro-3*H*-pyrrolo[2,3-*c*]quinoline-1-carboxylate de propyle
8-[Méthyl-(tétrahydro-pyran-4-yl)-sulfamoyl]-4-oxo-4,5-dihydro-3*H*-pyrrolo[2,3-*c*]quinoline-1-carboxylate de propyle
8-(Méthyl-prop-2-ynyl-sulfamoyl)-4-oxo-4,5-dihydro-3*H*-pyrrolo[2,3-*c*]quinoline-1-carboxylate de propyle
8-[Méthyl-(2-méthylsulfanyl-éthyl)-sulfamoyl]-4-oxo-4,5-dihydro-3*H*-pyrrolo[2,3-*c*]quinoline-1-carboxylate de propyle
8-[(2-Diméthylamino-éthyl)-méthyl-sulfamoyl]-4-oxo-4,5-dihydro-3*H*-pyrrolo[2,3-*c*]quinoline-1-carboxylate de propyle
8-[Méthyl-(2-pyrrolidin-1-yl-éthyl)-sulfamoyl]-4-oxo-4,5-dihydro-3*H*-pyrrolo[2,3-*c*]quinoline-1-carboxylate de propyle
8-[(2-Diéthylamino-éthyl)-méthyl-sulfamoyl]-4-oxo-4,5-dihydro-3*H*-pyrrolo[2,3-c]quinoline-1-carboxylate de propyle
Chlorhydrate de 8-[méthyl-(2-méthylamino-éthyl)-sulfamoyl]-4-oxo-4,5-dihydro-3*H*-pyrrolo[2,3-*c*]quinoline-1-carboxylate de propyle
8-{[2-(*tert*-Butoxycarbonyl-méthyl-amino)-éthyl]-méthyl-sulfamoyl}-4-oxo-4,5-dihydro-3*H*-pyrrolo[2,3-*c*]quinoline-1-carboxylate de propyle
Chlorhydrate de 8-[cyclopropyl-(2-méthyl-2-méthylamino-propyl)-sulfamoyl]-4-oxo-4,5-dihydro-3*H*-pyrrolo[2,3-*c*]quinoline-1-carboxylate de propyle
Chlorhydrate de 8-[cyclopropyl-(2-isopropylamino-éthyl)-sulfamoyl]-4-oxo-4,5-dihydro-3*H*-pyrrolo[2,3-*c*]quinoline-1-carboxylate de propyle
Chlorhydrate de 8-[cyclopropyl-(1*H*-imidazol-4-ylméthyl)-sulfamoyl]-4-oxo-4,5-dihydro-3*H*-pyrrolo[2,3-*c*]quinoline-1-carboxylate de propyle
Chlorhydrate de (S) 8-(cyclopropyl-pyrrolidin-2-ylméthyl-sulfamoyl)-4-oxo-4,5-dihydro-3*H*-pyrrolo[2,3-*c*]quinoline-1-carboxylate de propyle
(S) 8-[Cyclopropyl-(1-méthyl-pyrrolidin-2-ylméthyl)-sulfamoyl]-4-oxo-4,5-dihydro-3*H*-pyrrolo[2,3-*c*]quinoline-1-carboxylate de propyle
(R) 8-[Cyclopropyl-(1-méthyl-pyrrolidin-2-ylméthyl)-sulfamoyl]-4-oxo-4,5-dihydro-3*H*-pyrrolo[2,3-*c*]quinoline-1-carboxylate de propyle
(R) 8-[(1-*tert*-Butoxycarbonyl-pyrrolidin-2-ylméthyl)-cyclopropyl-sulfamoyl]-4-oxo-4,5-dihydro-3*H*-pyrrolo[2,3-

*c*]quinoline-1-carboxylate de propyle
Chlorhydrate de (R) 8-(cyclopropyl-pyrrolidin-2-ylméthyl-sulfamoyl)-4-oxo-4,5-dihydro-3*H*-pyrrolo[2,3-*c*]quinoline-1-carboxylate de propyle
Chlorhydrate de (S) 8-(isopropyl-pyrrolidin-2-ylméthyl-sulfamoyl)-4-oxo-4,5-dihydro-3*H*-pyrrolo[2,3-c]quinoline-1-carboxylate de propyle
Chlorhydrate de (S) 4-Oxo-8-(prop-2-ynyl-pyrrolidin-2-ylméthyl-sulfamoyl)-4,5-dihydro-3*H*-pyrrolo[2,3-*c*]quinoline-1-carboxylate de propyle
(S) 8-[(1-Méthyl-pyrrolidin-2-ylméthyl)-prop-2-ynyl-sulfamoyl]-4-oxo-4,5-dihydro-3*H*-pyrrolo[2,3-*c*]quinoline-1-carboxylate de propyle
(S) 8-[(2-*tert*-Butoxycarbonylamino-propyl)-cyclopropyl-sulfamoyl]-4-oxo-4,5-dihydro-3*H*-pyrrolo[2,3-*c*]quinoline-1-carboxylate de propyle
Chlorhydrate de (S) 8-[(2-amino-propyl)-cyclopropyl-sulfamoyl]-4-oxo-4,5-dihydro-3*H*-pyrrolo[2,3-c]quinoline-1-carboxylate de propyle
(R) 8-[(2-*tert*-Butoxycarbonylamino-propyl)-cyclopropyl-sulfamoyl]-4-oxo-4,5-dihydro-3*H*-pyrrolo[2,3-c]quinoline-1-carboxylate de propyle
Chlorhydrate de (R) 8-[(2-amino-propyl)-cyclopropyl-sulfamoyl]-4-oxo-4,5-dihydro-3*H*-pyrrolo[2,3-c]quinoline-1-carboxylate de propyle
Chlorhydrate de 8-[(2-méthylamino-éthyl)-prop-2-ynyl-sulfamoyl]-4-oxo-4,5-dihydro-3*H*-pyrrolo[2,3-*c*]quinoline-1-carboxylate de propyle
8-{[2-(*tert*-Butoxycarbonyl-méthyl-amino)-éthyl]-prop-2-ynyl-sulfamoyl}-4-oxo-4,5-dihydro-3*H*-pyrrolo[2,3-*c*] quinoline-1-carboxylate de propyle
Chlorhydrate de 8-[(2-isopropylamino-éthyl)-prop-2-ynyl-sulfamoyl]-4-oxo-4,5-dihydro-3*H*-pyrrolo[2,3-*c*]quinoline-1-carboxylate de propyle
8-{[2-(*tert*-Butoxycarbonyl-isopropyl-amino)-éthyl]-prop-2-ynyl-sulfamoyl}-4-oxo-4,5-dihydro-3*H*-pyrrolo[2,3-*c*] quinoline-1-carboxylate de propyle
Chlorhydrate de 8-(éthyl-pyrrolidin-3-yl-sulfamoyl)-4-oxo-4,5-dihydro-3*H*-pyrrolo[2,3-*c*]quinoline-1-carboxylate de propyle
8-[Ethyl-(1-méthyl-pyrrolidin-3-yl)-sulfamoyl]-4-oxo-4,5-dihydro-3*H*-pyrrolo[2,3-*c*]quinoline-1-carboxylate de propyle
4-Oxo-8-(pyrrolidine-1-sulfonyl)-4,5-dihydro-3*H*-pyrrolo[2,3-*c*]quinoline-1-carboxylate d'éthyle
4-Oxo-8-(2-pyrrolidin-1-ylméthyl-pyrrolidine-1-sulfonyl)-4,5-dihydro-3*H*-pyrrolo[2,3-*c*]quinoline-1-carboxylate d'éthyle
(S) 8-(3-*tert*-Butoxycarbonylamino-pyrrolidine-1-sulfonyl)-4-oxo-4,5-dihydro-3*H*-pyrrolo[2,3-*c*]quinoline-1-carboxylate de propyle
Acide (S) 8-(3-*tert*-butoxycarbonylamino-pyrrolidine-1-sulfonyl)-4-oxo-4,5-dihydro-3*H*-pyrrolo[2,3-*c*]quinoline-1-carboxylique
Chlorhydrate de (S) 8-(3-amino-cyclopentanesulfonyl)-4-oxo-4,5-dihydro-3*H*-pyrrolo[2,3-*c*]quinoline-1-carboxylate de propyle
Chlorhydrate de (R) 8-(3-amino-cyclopentanesulfonyl)-4-oxo-4,5-dihydro-3*H*-pyrrolo[2,3-*c*]quinoline-1-carboxylate de propyle
Chlorhydrate de 8-(3-méthylamino-pyrrolidine-1-sulfonyl)-4-oxo-4,5-dihydro-3*H*-pyrrolo[2,3-*c*]quinoline-1-carboxylate de propyle
8-[3-(*tert*-Butoxycarbonyl-méthyl-amino)-pyrrolidine-1-sulfonyl]-4-oxo-4,5-dihydro-3*H*-pyrrolo[2,3-c]quinoline-1-carboxylate de propyle
Acide de 8-[3-(*tert*-butoxycarbonyl-méthyl-amino)-pyrrolidine-1-sulfonyl]-4-oxo-4,5-dihydro-3*H*-pyrrolo[2,3-*c*] quinoline-1-carboxylique
(S) 8-(3-Diméthylamino-cyclopentanesulfonyl)-4-oxo-4,5-dihydro-3*H*-pyrrolo[2,3-*c*]quinoline-1-carboxylate de propyle
(R) 8-(3-Diméthylamino-cyclopentanesulfonyl)-4-oxo-4,5-dihydro-3*H*-pyrrolo[2,3-*c*]quinoline-1-carboxylate de propyle
8-[(2-Hydroxy-éthyl)-isopropyl-sulfamoyl]-4-oxo-4,5-dihydro-3*H*-pyrrolo[2,3-*c*]quinoline-1-carboxylate de propyle
8-[Cyclopropyl-(2-hydroxy-éthyl)-sulfamoyl]-4-oxo-4,5-dihydro-3*H*-pyrrolo[2,3-*c*]quinoline-1-carboxylate de propyle
8-[(2-Hydroxy-éthyl)-prop-2-ynyl-sulfamoyl]-4-oxo-4,5-dihydro-3*H*-pyrrolo[2,3-*c*]quinoline-1-carboxylate de propyle
8-[Bis-(2-hydroxy-éthyl)-sulfamoyl]-4-oxo-4,5-dihydro-3*H*-pyrrolo[2,3-*c*]quinoline-1-carboxylate de propyle
8-(Carbamoylméthyl-méthyl-sulfamoyl)-4-oxo-4,5-dihydro-3*H*-pyrrolo[2,3-*c*]quinoline-1-carboxylate de propyle

Acide 8-(carbamoylméthyl-méthyl-sulfamoyl)-4-oxo-4,5-dihydro-3*H*-pyrrolo[2,3-*c*]quinoline-1-carboxylique
8-(3-Carbamoyl-pipéridine-1-sulfonyl)-4-oxo-4,5-dihydro-3*H*-pyrrolo[2,3-*c*]quinoline-1-carboxylate de propyle
Acide 8-(3-carbamoyl-pipéridine-1-sulfonyl)-4-oxo-4,5-dihydro-3*H*-pyrrolo[2,3-*c*]quinoline-1-carboxylique
8-[(2-Benzyloxy-éthyl)-cyclopropyl-sulfamoyl]-4-oxo-4,5-dihydro-3*H*-pyrrolo[2,3-*c*]quinoline-1-carboxylate de propyle
8-(Carboxyméthyl-méthyl-sulfamoyl)-4-oxo-4,5-dihydro-3*H*-pyrrolo[2,3-*c*]quinoline-1-carboxylate de propyle
8-(*tert*-Butoxycarbonylméthyl-méthyl-sulfamoyl)-4-oxo-4,5-dihydro-3*H*-pyrrolo[2,3-*c*]quinoline-1-carboxylate de propyle
8-[(2-Diméthylamino-éthyl)-méthyl-sulfamoyl]-7-fluoro-4-oxo-4,5-dihydro-3*H*-pyrrolo[2,3-*c*]quinoline-1-carboxylate de propyle
7-Fluoro-8-[(2-hydroxy-éthyl)-isopropyl-sulfamoyl]-4-oxo-4,5-dihydro-3*H*-pyrrolo[2,3-*c*]quinoline-1-carboxylate de propyle
8-Diméthylsulfamoyl-7-fluoro-4-oxo-4,5-dihydro-3*H*-pyrrolo[2,3-*c*]quinoline-1-carboxylate de propyle
8-(Cyclopropyl-méthyl-sulfamoyl)-7-fluoro-4-oxo-4,5-dihydro-3*H*-pyrrolo[2,3-*c*]quinoline-1-carboxylate de propyle
8-(Cyclopropyl-méthyl-sulfamoyl)- 7-méthoxy-4-oxo-4,5-dihydro-3*H*-pyrrolo[2,3-*c*]quinoline-1-carboxylate de propyle
7-Chloro-8-diméthylsulfamoyl-4-oxo-4,5-dihydro-3*H*-pyrrolo[2,3-*c*]quinoline-1-carboxylate de propyle
Chlorhydrate de (S) 8-(3-amino-cyclopentanesulfonyl)-7-chloro-4-oxo-4,5-dihydro-3*H*-pyrrolo[2,3-*c*]quinoline-1-carboxylate de propyle
4-Oxo-8-(pipéridin-1-ylsulfamoyl)-4,5-dihydro-3*H*-pyrrolo[2,3-*c*]quinoline-1-carboxylate de méthyle
8-(Hexahydro-cyclopenta[c]pyrrol-2-ylsulfamoyl)-4-oxo-4,5-dihydro-3*H*-pyrrolo[2,3-*c*]quinoline-1-carboxylate de méthyle
8-(4-Méthyl-pipérazin-1-ylsulfamoyl)-4-oxo-4,5-dihydro-3*H*-pyrrolo[2,3-*c*]quinoline-1-carboxylate de méthyle.

**8.** Composé selon l'une des revendications 1 à 5, **caractérisé en ce qu'**il répond à la formule générale (III) :

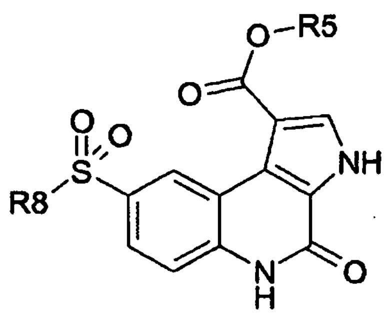

(III),

R8 étant défini dans l'une des revendications 1 à 5.

**9.** Composé selon la revendication 8, choisi parmi les composés suivants : et leurs sels physiologiquement acceptables :

4-Oxo-8-phénylméthanesulfonyl-4,5-dihydro-3*H*-pyrrolo[2,3-*c*]quinoline-1-carboxylate d'éthyle
8-(2,6-Dichloro-phénylméthanesulfonyl)-4-oxo-4,5-dihydro-3*H*-pyrrolo[2,3-*c*]quinoline-1-carboxylate d'éthyle
Acide 8-(2,6-dichloro-phénylméthanesulfonyl)-4-oxo-4,5-dihydro-3*H*-pyrrolo[2,3-*c*]quinoline-1-carboxylate d'éthyle
4-Oxo-8-(pyridin-4-ylméthanesulfonyl)-4,5-dihydro-3*H*-pyrrolo[2,3-*c*]quinoline-1-carboxylate d'éthyle
4-Oxo-8-(pyridin-2-ylméthanesulfonyl)-4,5-dihydro-3*H*-pyrrolo[2,3-*c*]quinoline-1-carboxylate d'éthyle
8-Cyclohexanesulfonyl-4-oxo-4,5-dihydro-3*H*-pyrrolo[2,3-*c*]quinoline-1-carboxylate d'éthyle
4-Oxo-8-phénylméthanesulfinyl-4,5-dihydro-3*H*-pyrrolo[2,3-*c*]quinoline-1-carboxylate d'éthyle.

**10.** Composé selon l'une des revendications 1 à 5, **caractérisé en ce qu'**il répond à la formule générale (IV) :

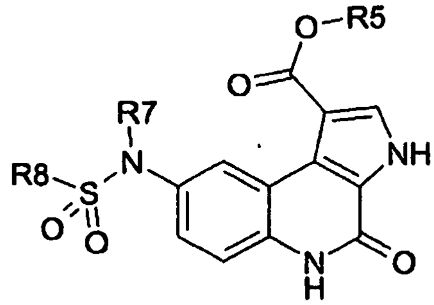

(IV),

R7 et R8 étant définis dans l'une des revendications 1 à 5.

**11.** Composé selon la revendication 10, choisi parmi les composés suivants : et leurs sels physiologiquement acceptables :

8-Benzènesulfonylamino-4-oxo-4,5-dihydro-3*H*-pyrrolo[2,3-*c*]quinoline-1-carboxylate d'éthyle
8-Méthanesulfonylamino-4-oxo-4,5-dihydro-3*H*-pyrrolo[2,3-*c*]quinoline-1-carboxylate d'éthyle
4-Oxo-8-phénylméthanesulfonylamino-4,5-dihydre-3*H*-pyrrolo[2,3-*c*]quinoline-1-carboxylate d'éthyle
8-(4-Chloro-benzènesulfonylamino)-4-oxo-4,5-dihydro-3*H*-pyrrolo[2,3-*c*]quinoline-1-carboxylate d'éthyle
8-(3-Chloro-benzènesulfonylamino)-4-oxo-4,5-dihydro-3*H*-pyrrolo[2,3-*c*]quinoline-1-carboxylate, d'éthyle
8-(2-Chloro-benzènesulfonylamino)-4-oxo-4,5-dihydro-3*H*-pyrrolo[2,3-*c*]quinoline-1-carboxylate d'éthyle
8-(4-Fluoro-benzènesulfonylamino)-4-oxo-4,5-dihydro-3*H*-pyrrolo[2,3-*c*]quinoline-1-carboxylate d'éthyle
8-(3-Fluoro-benzènesulfonylamino)-4-oxo-4,5-dihydro-3*H*-pyrrolo[2,3-*c*]quinoline-1-carboxylate d'éthyle
8-(2-Fluoro-bènzenesulfonylamino)-4-oxo-4,5-dihydro-3*H*-pyrrolo[2,3-*c*]quinoline-1-carboxylate d'éthyle
8-(4-Bromo-benzènesulfonylamino)-4-oxo-4,5-dihydro-3*H*-pyrrolo[2,3-*c*]quinoline-1- carboxylate d'éthyle
4-Oxo-8-(4-toluènesulfonylamino)-4,5-dihydro-3*H*-pyrrolo[2,3-*c*]quinoline-1-carboxylate d'éthyle
4-Oxo-8-(3-toluènesulfonylamino)-4,5-dihydro-3*H*-pyrrolo[2,3-*c*]quinoline-1-carboxylate d'éthyle
8-(4-*t*ert-Butyl-benzènesulfonylamino)-4-oxo-4,5-dihydro-3*H*-pyrrolo[2,3-*c*]quinoline-1-carboxylate d'éthyle
8-(4-Méthoxy-benzènesulfonylamino)-4-oxo-4,5-dihydro-3*H*-pyrrolo[2,3-*c*]quinoline-1-carboxylate d'éthyle
8-(3-Méthoxy-benzènesulfonylamino)-4-oxo-4,5-dihydro-3*H*-pyrrolo[2,3-*c*]quinoline-1-carboxylate d'éthyle
8-(4-Acétylamino-benzènesulfonylamino)-4-oxo-4,5-dihydro-3*H*-pyrrolo[2,3-*c*]quinoline-1-carboxylate d'éthyle
8-(4-Nitro-benzènesulfonylamino)-4-oxo-4,5-dihydro-3*H*-pyrrolo[2,3-*c*]quinoline-1-carboxylate d'éthyle
8-(Naphtalène-2-sulfonylamino)-4-oxo-4,5-dihydro-3*H*-pyrrolo[2,3-*c*]quinoline-1-carboxylate d'éthyle
8-(4-Acétyl-benzènesulfonylamino)-4-oxo-4,5-dihydro-3*H*-pyrrolo[2,3-*c*]quinoline-1-carboxylate d'éthyle
4-Oxo-8-(thiophène-2-sulfonylamino)-4,5-dihydro-3*H*-pyrrolo[2,3-*c*]quinoline-1-carboxylate d'éthyle
8-(5-Chloro-thiophène-2-sulfonylamino)-4-oxo-4,5-dihydro-3*H*-pyrrolo[2,3-*c*]quinoline-1-carboxylate d'éthyle
8-(Benzènesulfonyl-méthyl-amino)-4-oxo-4,5-dihydro-3*H*-pyrrolo[2,3-*c*]quinoline-1-carboxylate d'éthyle
8-(Méthanesulfonyl-méthyl-amino)-4-oxo-4,5-dihydro-3*H*-pyrrolo[2,3-*c*]quinolinecarboxylate d'éthyle.

**12.** Composé selon l'une des revendications 1 à 5, **caractérisé en ce qu'**il répond à la formule générale (V) :

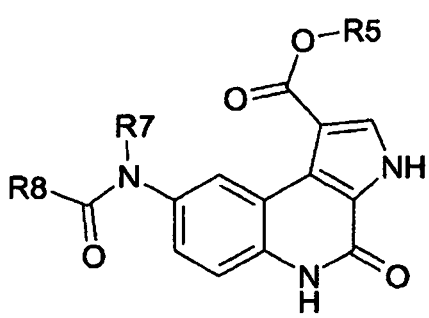

(V),

R7 et R8 étant définis dans l'une des revendications 1 à 5.

**13.** Composé selon la revendication 12, choisi parmi les composés suivants : et leurs sels physiologiquement acceptables :

8-Benzoylamino-4-oxo-4,5-dihydro-3*H*-pyrrolo[2,3-*c*]quinoline-1-carboxylate d'éthyle
8-Acétylamino-4-oxo-4,5-dihydro-3*H*-pyrrolo[2,3-*c*]quinoline-1-carboxylate d'éthyle.

**14.** Composé selon l'une des revendications 1 à 5, **caractérisé en ce qu'**il répond à la formule générale (VI) :

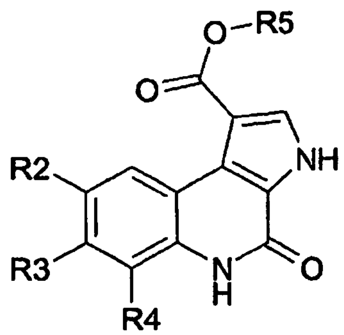

(VI),

R2, R3 et R4 étant définis dans l'une des revendications 1 à 5.

**15.** Composé selon la revendication 14, choisi parmi les composés suivants : et leurs sels physiologiquement acceptables :

8-Diméthylamino-4-oxo-4,5-dihydro-3*H*-pyrrolo[2,3-*c*]quinoline-1-carboxylate d'éthyle
8-Méthylamino-4-oxo-4,5-dihydro-3*H*-pyrrolo[2,3-*c*]quinoline-1-carboxylate d'éthyle
8-Amino-4-oxo-4,5-dihydro-3*H*-pyrrolo[2,3-*c*]quinoline-1-carboxylate d'éthyle
8-Nitro-4-oxo-4,5-dihydro-3*H*-pyrrolo[2,3-*c*]quinoline-1-carboxylate d'éthyle
6-Nitro-4-oxe-4,5-dihydro-3*H*-pyrrolo[2,3-*c*]quinoline-1-carboxylate d'éthyle
8-(2-Ethoxycarbonyl-vinyl)-4-oxo-4,5-dihydro-3*H*-pyrrolo[2,3-*c*]quinoline-1-carboxylate d'éthyle
8-(2-Benzylcarbamoyl-vinyl)-4-oxo-4,5-dihydro-3*H*-pyrrolo[2,3-*c*]quinoline-1-carboxylate d'éthyle
8-[2-(2-Méthoxycarbonyl-éthylcarbamoyl)-vinyi]-4-oxo-4,5-dihydro-3*H*-pyrrolo[2,3-*c*]quinoline-1-carboxylate d'éthyle
4-Oxo-8-styryl-4,5-dihydro-3*H*-pyrrolo[2,3-*c*]quinoline-1-carboxylate d'éthyle
8-Ethynyl-4-oxo-4,5-dihydro-3*H*-pyrrolo[2,3-*c*]quinoline-1-carboxylate d'éthyle
4-Oxo-8-triméthylsilanyléthynyl-4,5-dihydro-3*H*-pyrrolo[2,3-*c*]quinoline-1-carboxylate d'éthyle
4-Oxo-8-phényléthynyl-4,5-dihydro-3*H*-pyrrolo[2,3-*c*]quinoline-1-carboxylate d'éthyle
4-Oxo-8-pyridin-2-yléthynyi-4,5-dihydro-3*H*-pyrrolo[2,3-*c*]quinoline-1-carboxylate d'éthyle
8-(3-Diméthylamino-prop-l-ynyl)-4-oxo-4,5-dihydro-3*H*-pyrrolo[2,3-*c*]quinoline-1-carboxylate d'éthyle
8-(3-Hydroxy-3-méthyl-but-1-ynyl)-4-oxo-4,5-dihydro-3*H*-pyrrolo[2,3-*c*]quinoine-1-carboxylate d'éthyle
8-(3-Morpholin-4-yl-prop-1-ynyl)-4-oxo-4,5-dihydro-3*H*-pyrrolo[2,3-*c*]quinoline-1-carboxylate d'éthyle
8-{3-[(2-Cyano-éthyl)-méthyl-amino]-prop-1-ynyl}-4-oxo-4,5-dihydro-3*H*-pyrrolo[2,3-*c*]quinoline-1-carboxylate d'éthyle
4-Oxo-8-phénéthyl-4,5-dihydro-3*H*-pyrrolo[2,3-*c*]quinoline-1-carboxylate d'éthyle
8-(3-Hydroxy-3-méthyl-butyl)-4-oxo-4,5-dihydro-3*H*-pyrrolo[2,3-*c*]quinoline-1-carboxylate d'éthyle
8-Bromo-4-oxo-4,5-dihydro-3*H*-pyrrolo[2,3-*c*]quinoline-1-carboxylate d'éthyle
8-Iodo-4-oxo-4,5-dihydro-3*H*-pyrrolo[2,3-*c*]quinoline-1-carboxylate d'éthyle
8-Iodo-4-oxo-4,5-dihydro-3*H*-pyrrolo[2,3-*c*]quinoline-1-carboxylate de propyle
8-Iodo-4-oxo-4,5-dihydro-3*H*-pyrrolo[2,3-*c*]quinoline-1-carboxylate de cyclopentyle
8-Iodo-4-oxo-4,5-dihydro-3*H*-pyrrolo[2,3-*c*]quinoline-1-carboxylate d'isobutyle
7,8-Diméthoxy-4-oxo-4,5-dihydro-3*H*-pyrrolo[2,3-*c*]quinoline-1-carboxylate d'éthyle
7-Méthoxy-4-oxo-4,5-dihydro-3*H*-pyrrolo[2,3-*c*]quinoline-1-carboxylate d'éthyle
8-Iodo-7-méthoxy-4-oxo-4,5-dihydro-3*H*-pyrrolo[2,3-*c*]quinoline-1-carboxylate d'éthyle
8-Fluoro-4-oxo-4,5-dihydro-3*H*-pyrrolo[2,3-*c*]quinoline-1-arboxylate d'éthyle
8-Fluoro-4-oxo-4,5-dihydro-3*H*-pyrrolo[2,3-*c*]quinoline-1-carboxylate de 2-diéthylaminoéthyle
7-Fluoro-4-oxo-4,5-dihydro-3*H*-pyrrolo[2,3-*c*]quinoline-1-carboxylate d'éthyle

7-Chloro-4-oxo-4,5-dihydro-3*H*-pyrrolo[2,3-*c*]quinoline-1-carboxylate d'éthyle
8-Acétyl-4-oxo-4,5-dihydro-3*H*-pyrrolo[2,3-*c*]quinoline-1-carboxylate d'éthyle
8-Benzoyl-4-oxo-4,5-dihydro-3*H*-pyrrolo[2,3-*c*]quinoline-1-carboxylate d'éthyle
8-(3-Diméthylamino-acryloyl)-4-oxo-4,5-dihydro-3*H*-pyrrolo[2,3-*c*]quinoline-1-carboxylate d'éthyle
8-(2-Moipholin-4-yl-acétyl)-4-oxo-4,5-dihydro-3*H*-pyrrolo[2,3-*c*]quinoline-1-carboxylate d'éthyle
Chlorhydrate de (S) 8-(cyclopropyl-pyrrolidin-2-ylméthyl-carbamoyl)-4-oxo-4,5-dihydro-3*H*-pyrrolo[2,3-*c*]quinoline-1-carboxylate d'éthyle
4-Oxo-8-phénylsulfanyl-4,5-dihydro-3*H*-pyrrolo[2,3-*c*]quinoline-1-carboxylate d'éthyle
8-Cyclohexylsulfanyl-4-oxo-4,5-dihydro-3*H*-pyrrolo[2,3-*c*]quinoline-1-carboxylate d'éthyle
8-Benzylsulfanyl-4-oxo-4,5-dihydro-3*H*-pyrrolo[2,3-*c*]quinoline-1-carboxylate d'éthyle
8-(4-Acétylamino-phénylsulfanyl)-4-oxo-4,5-dihydro-3*H*-pyrrolo[2,3-*c*]quinoline-1-carboxylate d'éthyle
8-(3-Amino-phénylsulfanyl)-4-oxo-4,5-dihydro-3*H*-pyrrolo[2,3-*c*]quinoline-1-carboxylate d'éthyle
8-(3-Acétylamino-phénylsulfanyl)-4-oxo-4,5-dihydro-3*H*-pyrrolo[2,3-*c*]quinoline-1-carboxylate d'éthyle
Chlorhydrate de 8-(2-amino-éthylsulfanyl)-4-oxo-4,5-dihydro-3*H*-pyrrolo[2,3-*c*]quinoline-1-carboxylate d'éthyle
4-Oxo-8-sulfo-4,5-dihydro-3*H*-pyrrolo[2,3-*c*]quinoline-1-carboxylate d'éthyle.

**16.** Composé selon l'une des revendications 1 à 5, **caractérisé en ce qu'**il répond à la formule générale (VII) :

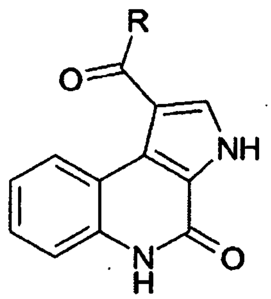

(VII),

R étant définis dans l'une des revendications 1 à 5.

**17.** Composé selon la revendication 16, choisi parmi les composés suivants : et leurs sels physiologiquement acceptables :

4-Oxo-4,5-dihydro-3*H*-pyrrolo[2,3-*c*]quinoline-1-carboxylate de benzyle
4-Oxo-4,5-dihydro-3*H*-pyrrolo[2,3-*c*]quinoline-1-carboxylate d'isopropyle
1-Acétyl-3,5-dihydro-pyrrolo[2,3-*c*]quinolin-4-one
*N*-(2-Diméthylamino-éthyl)-4-oxo-4,5-dihydro-3*H*-pyrrolo[2,3-*c*]quinoline-1-carboxamide
*N*-(3-Diméthylamino-propyl)-4-oxo-4,5-dihydro-3*H*-pyrrolo[2,3-*c*]quinoline-1-carboxamide
3-[(4-Oxo-4,5-dihydro-3*H*-pyrrolo[2,3-*c*]quinoline-1-carbonyl)-amino]-propanoate de méthyle
*N*-(cyclohexylméthyl)-4-oxo-4,5-dihydro-3*H*-pyrrolo[2,3-*c*]quinoline-1-carboxamide
*N*-(benzyl)-4-oxo-4,5-dihydro-3*H*-pyrrolo[2,3-*c*]quinoline-1-carboxylamide
*N*-[2-(1*H*-indol-3-yl)-éthyl]-4-oxo-4,5-dihydro-3*H*-pyrrolo[2,3-*c*]quinoline-1-carboxamide
*N*-(3-Hydroxy-propyl)-4-oxo-4,5-dihydro-3*H*-pyrrolo[2,3-*c*]quinoline-1-carboxamide
*N*-(Pyridin-4-ylméthyl)-4-oxo-4,5-dihydro-3*H*-pyrrolo[2,3-*c*]quinoline-1-carboxamide
*N*-(Phényl)-4-oxo-4,5-dihydro-3*H*-pyrrolo[2,3-*c*]quinoline-1-carboxamide
*N*-(Méthyl)-4-oxo-4,5-dihydro-3*H*-pyrrolo[2,3-*c*]quinoline-1-carboxamide
*N,N*-(Diméthyl)-4-oxo-4,5-dihydro-3*H*-pyrrolo[2,3-*c*]quinoline-1-carboxamide
4-Oxo-4,5-dihydro-3H-pyrrolo[2,3-*c*]quinoline-1-carboxamide.

**18.** Composé selon l'une des revendications 1 à 5, **caractérisé en ce qu'**il répond à la formule générale (VIII) :

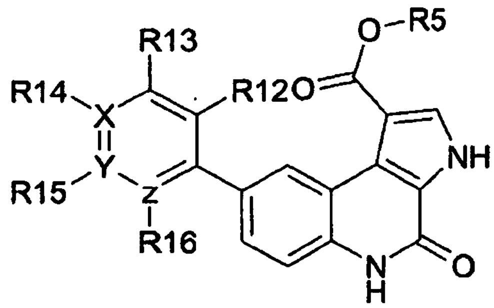

(VIII)

dans laquelle X, Y ou Z représentent indépendamment l'un de l'autre un atome de carbone, un atome d'azote, un atome de soufre ou un atome d'oxygène, étant entendu que lorsque X, Y ou Z sont un atome d'azote, de soufre ou d'oxygène, la présence d'un radical R14, R15 ou R16 dépend du respect de la valence dudit atome d'azote ou de soufre préservant l'aromaticité du cycle,
R12 représente un atome d'hydrogène, un halogène ou un groupe alkyle R13 à R16, représentent, indépendamment l'un de l'autre, un atome d'hydrogène, un atome d'halogène, un groupe hydroxy, un groupe cyano, un radical choisi parmi les groupes alkyle linéaires ou ramifiés, alcoxy, alkylsulfonyle, alkoxycarbonyle, carbamoyle, cycloalkyle, aryle, éventuellement substitués par un ou plusieurs substituants, et le radical -NR7-(Y)n-R8,
R13 et R14, R14 et R15 ou R15 et R16 pouvant former un cycle aliphatique ou aromatique, avec un ou plusieurs hétéroatomes choisi parmi l'oxygène ou l'azote, éventuellement substituées par un ou plusieurs substituants,
R5, R7 et R8 étant définis dans l'une des revendications 1 à 5.

**19.** Composé selon la revendication 18, choisi parmi les composés suivants : et leurs sels physiologiquement acceptables :

4-Oxo-8-phényl-4,5-dihydro-3*H*-pyrmio[2,3-*c*]quinoline-1-carboxylate d'éthyle
8-(2-Chloro-phényl)-4-oxo-4,5-dihydro-3*H*-pyrrolo[2,3-*c*]quinoline-1-carboxylate d'éthyle
8-(2-*tert*-Butoxycarbonylamino-phényl)-4-oxo-4,5-dihydro-3*H*-pyrrolo[2,3-*c*]quinoline-1-carboxylate d'éthyle
8-(2-Amino-phényl)-4-oxo-4,5-dihydro-3*H*-pyrrolo[2,3-*c*]quinoline-1-carboxylate d'éthyle
8-(2-Méthanesulfonylamino-phényl)-4-oxo-4,5-dihydro-3*H*-pyrrolo[2,3-*c*]quinoline-1-carboxylate d'éthyle
8-(3-*tert*-Butoxycarbonylamino-phényl)-4-oxo-4,5-dihydro-3*H*-pyrrolo[2,3-*c*]quinoline-1-carboxylate d'éthyle
8-(3-Amino-phényl)-4-oxo-4,5-dihydro-3*H*-pyrrolo[2,3-*c*]quinoline-1-carboxylate d'éthyle
8-(3-Méthatiesulfonylamine-phényl)-4-oxo-4,5-dihydro-3*H*-pyrrolo[2,3-*c*]quinoline-1-carboxylate d'éthyle
8-(3-Acétylamino-phényl)-4-oxo-4,5-dihydro-3*H*-pyrrolo[2,3-*c*]quinoline-1-carboxylate d'éthyle
8-(3-Hydroxy-phényl)-4-oxo-4,5-dihydro-3*H*-pyrrolo[2,3-*c*]quinoline-1-carboxylate d'éthyle
8-(3-Méthanesulfonyl-phényl)-4-oxo-4,5-dihydro-3*H*-pyrrolo[2,3-*c*]quinoline-1-carboxylate d'éthyle
8-(3-Cyano-phényl)-4-oxo-4,5-dihydro-3*H*-pyrrolo[2,3-*c*]quinoline-1-carboxylate d'éthyle
8-(3-Hydroxyméthyl-phényl)-4-oxo-4,5-dihydro-3*H*-pyrrolo[2,3-*c*]quinoline-1-carboxylate d'éthyle
8-(3-Méthylaminométhyl-phényl)-4-oxo-4,5-dihydro-3*H*-pyrrolo[2,3-*c*]quinoline-1-carboxylate d'éthyle
8-(3-Diméthylaminométhyl-phényl)-4-oxo-4,5-dihydro-3*H*-pyrrolo[2,3-*c*]quinoline-1-carboxylate d'éthyle
8-(4-*tert*-Butoxycarbonylamino-phényl)-4-oxo-4,5-dihydro-3*H*-pyrrolo[2,3-*c*]quinoline-1-carboxylate d'éthyle
8-(4-Amino-phényl)-4-oxo-4,5-dihydro-3*H*-pyrrolo[2,3-*c*]quinoline-1-carboxylate d'éthyle
8-(4-Méthanesulfonyl-phényl)-4-oxo-4,5-dihydro-3*H*-pyrrolo[2,3-*c*]quinoline-1-carboxylate d'éthyle
8-(4-Méthoxy-phényl)-4-oxo-4,5-dihydro-3*H*-pyrrolo[2,3-*c*]quinoline-1-carboxylate d'éthyle
8-(4-Cyano-phényl)-4-oxo-4,5-dihydro-3*H*-pyrrolo[2,3-*c*]quinoline-1-carboxylate d'éthyle
8-(2,3-Dihydro-benzo[1,4]dioxin-6-yl)-4-oxo-4,5-dihydro-3*H*-pyrrolo[2,3-*c*]quinoline-1-carboxylate d'éthyle
8-Naphthalèn-1-yl-4-oxo-4,5-dihydro-3*H*-pyrrolo[2,3-*c*]quinoline-1-carboxylate d'éthyle
4-Oxo-8-quinolin-8-yl-4,5-dihydro-3*H*-pyrrolo[2,3-*c*]quinoline-1-carboxylate d'éthyle
8-(2-Amino-5-méthanesulfonyl-phényl)-4-oxo-4,5-dihydro-3*H*-pyrrolo[2,3-c]quinoline-1-carboxylate d'éthyle
8-(1*H*-Indol-5-yl)-4-oxo-4,5-dihydro-3*H*-pyrrolo[2,3-*c*]quinoline-1-carboxylate d'éthyle
8-(1-*tert*-Butoxycarbonyl-1*H*-indol-5-yl)-4-oxo-4,5-dihydro-3*H*-pyrrolo[2,3-*c*]quinoline-1-carboxylate d'éthyle
8-(3-Méthanesulfonyl-phényl)-4-oxo-4,5-dihydro-3*H*-pyrrolo[2,3-*c*]quinoline-1-carboxylate de propyle
8-(3-Méthanesulfonyl-phenyl)-4-oxo-4,5-dihydro-3*H*-pyrrolo[2,3-*c*]quinoline-1-carboxylate d'isobutyle
8-(3-Méthanesulfonyl-phényl)-4-oxo-4,5-dihydro-3*H*-pyrrolo[2,3-*c*]quinoline-1-carboxylate de cyclopentyle
7-Chloro-8-(3-méthanesulfonyl-phényl)-4-oxo-4,5-dihydro-3*H*-pyrrolo[2,3-*c*]quinoline-1-carboxylate d'éthyle
4-Oxo-8-pyridin-3-yl-4,5-dihydro-3*H*-pyrrolo[2,3-*c*]quinoline-1-carboxylate d'éthyle

8-(6-Chloro-pyridin-3-yl)-4-oxo-4,5-dihydro-3*H*-pyrrolo[2,3-*c*]quinoline-1-carboxylate d'éthyle
8-(6-Méthoxy-pyridin-3-yl)-4-oxo-4,5-dihydro-3*H*-pyrrolo[2,3-*c*]quinoline-1-carboxylate d'éthyle
8-(2-Amino-pyrimidin-4-yl)-4-oxo-4,5-dihydro-3*H*-pyrrolo[2,3-*c*]quinoline-1-carboxylate d'éthyle
8-(2-Diméthylamino-pyrimidin-4-yl)-4-oxo-4,5-dihydro-3*H*-pyrrolo[2,3-*c*]quinoline-1-carboxylate d'éthyle
Formiate de 8-(2-méthylamino-pyrimidin-4-yl)-4-oxo-4,5-dihydro-3*H*-pyrrolo[2,3-*c*]quinoline-1-carboxylate d'éthyle
8-(2-Ethylamino-pyrimidin-4-yl)-4-oxo-4,5-dihydro-3*H*-pyrrolo[2,3-*c*]quinoline-1-carboxylate d'éthyle
4-Oxo-8-[2-(4-pyridin-2-yl-pipérazin-1-yl)-pyrimidin-4-yl]-4,5-dihydro-3*H*-pyrrolo[2,3-*c*]quinoline-1-carboxylate d'éthyle
4-Oxo-8-(2-phénylamino-pyrimidin-4-yl)-4,5-dihydro-3*H*-pyrrolo[2,3-*c*]quinoline-1-carboxylate d'éthyle
8-[2-(1*H*-Benzoimidazol-2-ylamino)-pyrimidin-4-yl]-4-oxo-4,5-dihydro-3*H*-pyrrolo[2,3-*c*]quinoline-1-carboxylate d'éthyle
8-[2-(4- Acétyl- pipérazin- 1- yl)-pyrimidin- 4- yl]- 4- oxo- 4,5- dihydro- 3*H*-pyrrolo [2,3-*c*] quinoline- 1- carboxylate d'éthyle
Formiate de 8-{2-[(2-Diméthylamino-éthyl)-méthyl-amino]-pyrimidin-4-yl}-4-oxo-4,5-dihydro-3*H*-pyrrolo[2,3-*c*] quinoline-1-carboxylate d'éthyle.

**20.** Composé selon l'une des revendications 1 à 5, **caractérisé en ce qu'**il répond à la formule générale (IX) :

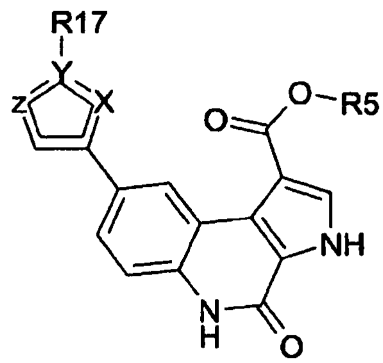

(IX)

dans laquelle X, Y ou Z représentent indépendamment l'un de l'autre un atome de carbone, un atome d'azote, un atome de soufre ou un atome d'oxygène, étant entendu que lorsque X, Y ou Z sont un atome d'azote, de soufre ou d'oxygène, la présence d'un radical R17 dépend du respect de la valence dudit atome d'azote ou de soufre préservant l'aromaticité du cycle,
R17 représente un atome d'hydrogène, un radical alkyle, cycloalkyle ou aryle, éventuellement substitués par un ou plusieurs substituants, ou un aryle, radical -NR7-(Y)n-R8 ou - NR7-NH-R8,
R5, R7 et R8 étant définis dans l'une des revendications 1 à 5.

**21.** Composé selon la revendication 20, choisi parmi les composés suivants : et leurs sels physiologiquement acceptables :

8-(2Amino-thiazol-4-yl)-4-oxo-4,5-dihydro-3*H*-pyrrolo[2,3-*c*]quinoline-1-carboxylate d'éthyle
8-[2-phénylamino-thiazol-4-yl]-4-oxo-4,5-dihydro-3*H*-pyrrlo[2,3-*c*]quinoline-1-carboxylate d'éthyle
8-[2-(2-Chloro-phénylamino)-thiazol-4-yl]-4-oxo-4,5-dihydro-3*H*-pyrrolo[2,3-*c*]quinoline-1-carboxylate d'éthyle
8-[2-(3,4- Dichloro- phénylamino)-thiazol- 4- yl]- 4- oxo- 4,5- dihydro- 3*H*-pyrrolo [2,3-*c*] quinoline- 1- carboxylate d'éthyle
8-[2-(2,6- Dichloro- phénylamino)-thiazol- 4- yl]- 4- oxo- 4,5- dihydro- 3*H*-pyrrolo [2,3-*c*] quinoline- 1- carboxylate d'éthyle
8-[2-(Cyclopropanecarbonyl-amino)-thiazol-4-yl]-4-oxo-4,5-dihydro-3*H*-pyrrolo[2,3-*c*]quinoline-1-carboxylate d'éthyle
4-Oxo-8-(2-phénylsulfanylméthyl-thiazol-4-yl)-4,5-dihydro-3*H*-pyrrolo[2,3-*c*]quinoline-1-carboxylate d'éthyle
4-Oxo-8-(2-pipéridin-4-yl-thiazol-4-yl)-4,5-dihydro-3*H*-pyrrolo[2,3-*c*]quinoline-1-carboxylate d'éthyle
8-(2-Méthyl-thiazol-4-yl)-4-oxo-4,5-dihydro-3*H*-pyrrolo[2,3-*c*]quinoline-1-carboxylate d'éthyle
4-Oxo-8-(2-phényl-thiazol-4-yl)-4,5-dihydro-3*H*-pyrrolo[2,3-*c*]quinoline-1-carboxylate d'éthyle
4-Oxo-8-[2-(*N*'-phényl-hydrazino)-thiazol-4-yl]-4,5-dihydro-3*H*-pyrrolo[2,3-*c*]quinoline-1-carboxylate d'éthyle

8-Isoxazol-5-yl-4-oxo-4,5-dihydro-3*H*-pyrrolo[2,3-*c*]quinoline-1-carboxylate d'éthyle
4-Oxo-8-(1*H*-pyrazol-3-yl)-4,5-dihydro-3*H*-pyrrolo[2,3-*c*]quinoline-1-carboxylate d'éthyle
8-(1-Méthyl-1*H*-pyrazol-3-yl)-4-oxo-4,5-dihydro-3Hpyrrolo[2,3-*c*]quinoline-1-carboxylate d'éthyle.

**22.** Composition pharmaceutique comprenant un véhicule pharmaceutique approprié et un composé choisi parmi les composés selon l'une des revendications 1 à 21 et les composés suivants et leurs sels physiologiquement acceptables :

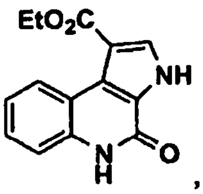 , 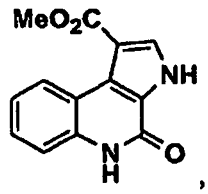 , 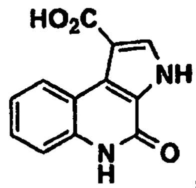 , 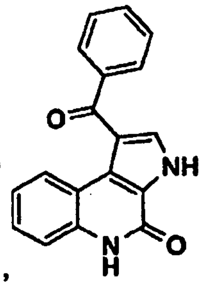 .

**23.** Un composé choisi parmi les composés selon l'une des revendications 1 à 21 et les composés suivants et leurs sels physiologiquement acceptables : pour l'utilisation en thérapie:

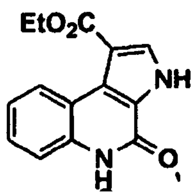 , 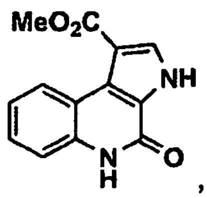 , 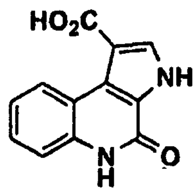 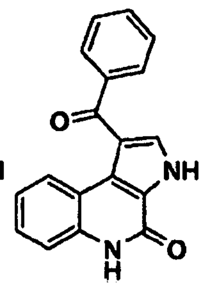 .

**24.** Composés selon la revendication 23 pour l'utilisation dans le traitement des cancers, des maladies inflammatoires ou des affections du système nerveux central.

**25.** Utilisation selon l'une des revendications 23 ou 24, en association avec au moins un autre agent actif.

**26.** Utilisation d'un composé choisi parmi les composés selon l'une des revendications 1 à 21 et les composés suivants et leurs sels physiologiquement acceptables :

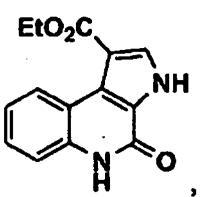 , 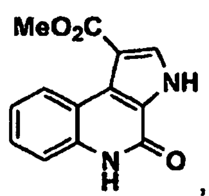 , 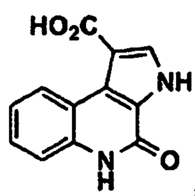 , 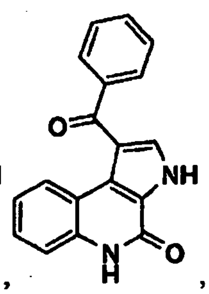 ,

pour la préparation d'un médicament destiné au traitement des cancers, des maladies inflammatoires ou des affections du système nerveux central.

**27.** Utilisation selon la revendication 26, en association avec au moins un autre agent actif.

**28.** Utilisation non thérapeutique comme inhibiteur de kinase d'un composé choisi parmi les composés selon l'une des

revendications 1 à 21 et les composés suivants et leurs sels physiologiquement acceptables :

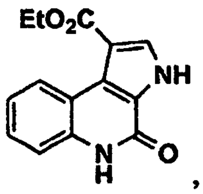, 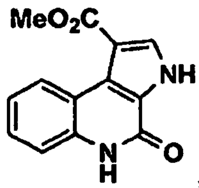, 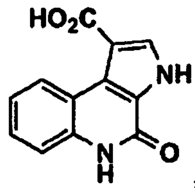, 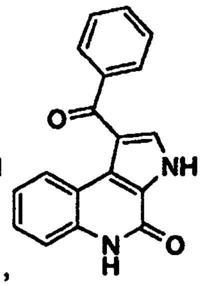 ;

**29.** Produit comprenant un composé choisi parmi les composés selon l'une des revendications 1 à 21 et les composés suivants et leurs sels physiologiquement acceptables :

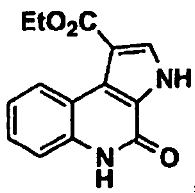, 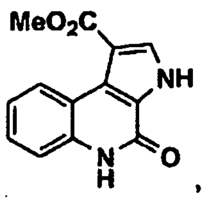, 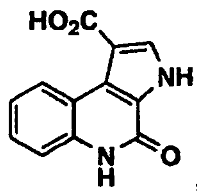, 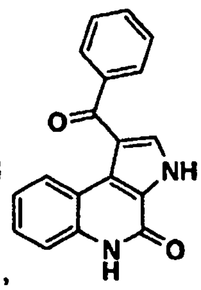

et un autre agent actif comme produit de combinaison pour une utilisation simultanée, séparée ou étalée dans le temps en thérapie.

**30.** Produit selon la revendication 29, pour son utilisation dans le traitement des cancers, des maladies inflammatoires ou des affections du système nerveux central.

## Claims

**1.** Compound of formula I:

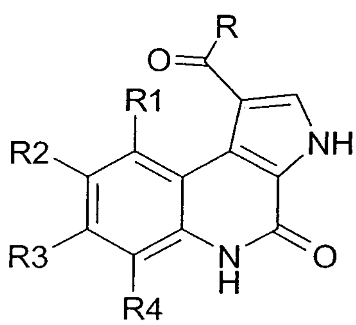

(I)

in which:

R represents a hydrogen atom or a group selected from the radicals alkyl, alkenyl, alkynyl, cycloalkyl, aralkyl, aryl, heteroaryl, -O-R5, or -NR5-R6, optionally substituted with one or more substitucnts,
R1 and R4 represent, independently, a hydrogen atom, a halogen or a group selected from the radicals nitro, alkyl, alkenyl, alkynyl, alkoxy or cycloalkyl, optionally substituted with one or more substituents,

R2 and R3 represent a hydrogen atom, a halogen or a group selected from the radicals alkyl, alkenyl, alkynyl, alkoxy, aryl, heteroaryl, aryloxy, aralkyloxy, optionally substituted with one or more substituents, and the radicals hydroxy, cyano, -Y-(NR7)n-R8, -SOm-R8, -NR7-(Y)n-R8, -N02, COOR9,
n = 0 or 1
m = 0 or 1
Y = -CO- or -SO2-
R2 and R3 not being the following groups simultaneously on the same compound: aryl, heteroaryl, aryloxy, aralkyloxy, -Y-(NR7)n-R8, -SOm-R8, -NR7-(Y)n-R8, -N02, COOR9,
R5 represents a hydrogen atom or an alkyl or alkenyl radical of at least 3 carbon atoms not directly branched on the unsaturation, alkynyl of at least 3 carbon atoms not directly branched on the unsaturation, cycloalkyl, cycloalkylalkyl, aralkyl, heteroaralkyl, saturated non-nitrogenous alkyl heterocycle optionally substituted with one or more substituents,
R6 represents a hydrogen atom or an alkyl or alkenyl radical of at least 3 carbon atoms not directly branched on the unsaturation, alkynyl of at least 3 carbon atoms not directly branched on the unsaturation, cycloalkyl, cycloalkylalkyl, aryl, heteroaryl, aralkyl, heteroaralkyl, saturated non-nitrogenous alkyl heterocycle optionally substituted with one or more substituents,
R7 represents a hydrogen atom or an alkyl or alkenyl radical of at least 3 carbon atoms not directly branched on the unsaturation, alkynyl of at least 3 carbon atoms not directly branched on the unsaturation, cycloalkyl, cycloalkylalkyl, optionally substituted with one or more substituents,
R8 represents a hydrogen atom or a group selected from the radicals alkyl, alkenyl, alkynyl, alkoxy, cycloalkyl, cycloalkylalkyl, aralkyl, aryl, heteroaryl, heteroaralkyl, saturated heterocycle or saturated alkyl heterocycle, optionally substituted with one or more substituents,
R9 represents a hydrogen atom or a group selected from the radicals alkyl, alkenyl, alkynyl, cycloalkyl, cycloalkylalkyl, aralkyl, aryl, heteroaralkyl, saturated heterocycle or saturated alkyl heterocycle, optionally substituted with one or more substituents,
R7 and R8 being able to form, together, a ring or a double ring containing 1 or more heteroatoms with the nitrogen to which they are attached, said heterocycle being optionally substituted with one or more alkyl, alkenyl, alkynyl, amino, NR10R11, hydroxy, alkoxy, carbamoyl, sulfamoyl, sulfonyl, sulfenyl, sulfanyl, or cyano groups or an oxo radical,
R10 represents a hydrogen atom or an alkyl or alkenyl radical of at least 3 carbon atoms not directly branched on the unsaturation, alkynyl of at least 3 carbon atoms not directly branched on the unsaturation, cycloalkyl, cycloalkylalkyl, optionally substituted with one or more substituents,
R11 represents independently a hydrogen atom or a group selected from the radicals alkyl, alkenyl, alkynyl, cycloalkyl, cycloalkylalkyl, aralkyl, aryl, heteroaryl, heteroaralkyl, saturated heterocycle or saturated alkyl heterocycle, alkoxycarbonyl, carbamoyl, sulfamoyl, optionally substituted with one or more substituents,
R10 and R11 being able to form, together, a ring containing I or more heteroatoms with the nitrogen to which they are attached, said heterocycle being optionally substituted

and physiologically acceptable salts thereof
with the exception of the following compounds:

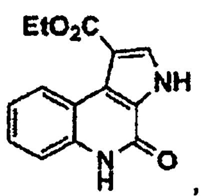 , 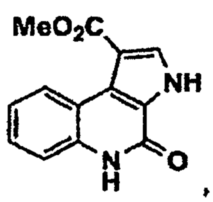 , 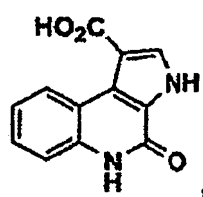 , 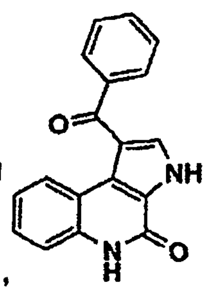

it being understood that
the term "alkyl" is intended to mean linear or branched C1, C2, C3, C4, C5 or C6 alkyl radicals, this definition also applying to the alkyl parts of the alkoxy, alkoxycarbonyl, carbonylamino, carbonyl, aralkyl or alkoxycarbonylalkyl radicals,
the term "alkenyl" is intended to mean a linear or branched, unsaturated monovalent hydrocarbon-based chain comprising at least one double bond and containing from 2 to 6 carbon atoms,

the term "alkynyl" is intended to mean a linear or branched, unsaturated monovalent hydrocarbon-based chain comprising at least one triple bond and containing from 2 to 6 carbon atoms,
the term "cycloalkyl" is intended to mean C3-C7 cycloalkyls,
the term "aryl" is intended to mean one or more aromatic rings containing 6 to 10 carbon atoms, which may be placed side by side or fused,
the term "heteroaryl" is intended to mean one or more aromatic rings containing 5 to 10 atoms, which may be placed side by side or fused, comprising, in its ring, at least one heteroatom chosen from a nitrogen, oxygen or sulfur,
the substituents being selected from the halogens, the groups hydroxyl, amino (primary, secondary or tertiary amine), nitro, the C1-C4 alkyl radicals, the C1-C4 perfluoroalkyl, alkoxy, perfluoroalkoxy, alkoxycarbonyl, cycloalkyl, carbamoyl, sulfamoyl, sulfonyl, sulfenyl, sulfanyl, carbonylamino, aryl, heteroaryl or cyano radicals or an oxo radical.

2. Compound according to Claim 1, **characterized in that** it corresponds to general formula I':

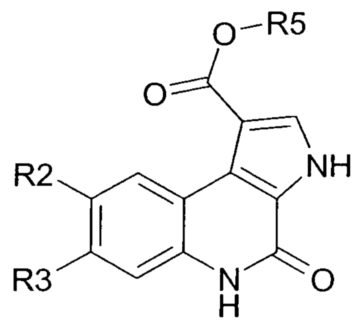

(I')

in which R2, R3 and R5 are defined in Claim 1.

3. Compound according to either of Claims 1 and 2, **characterized in that:**

R2 is selected from the radicals -S02-(NR7)n-R8 or -NR7-(Y)n-R8,
n, R7 and R8 are as defined in Claim 1.

4. Compound according to one of Claims 1 to 3, **characterized in that:**

- R5 is selected from the groups methyl, ethyl, propyl, iso-propyl, butyl, iso-butyl, iso-pentyl, allyl, isoprenyl, propargyl, cyclopentyl, cyclohexyl, cyclopropylmethyl, cyclopentylmethyl, cyclohexylmethyl, tetrahydropyran-4-yl-methyl or tetrahydrofuran-3-yl-methyl, and/or
- R7 is a hydrogen or is selected from the groups methyl, ethyl, propyl, iso-propyl, butyl, iso-butyl, allyl, isoprenyl, propargyl, cyclopropyl, cyclopentyl, cyclohexyl, cyclopropylmethyl, cyclopentylmethyl, cyclohexylmethyl, tetrahydropyran-4-yl-methyl, tetrahydrofuran-3-yl-methyl, 2-hydroxyethyl, and/or
- R8 is selected from the groups methyl, hydroxy-2-ethyl, amino-2-ethyl, methylamino-2-ethyl, dimethylamino-2-ethyl, cyanomethyl, carbamoylmethyl, 2-cyanoethyl, cyclohexyl, cycloheptyl, cyclopropylmethyl, cyclopentylmethyl, cyclohexylmethyl, piperidin-4-yl, piperidin-3-yl, piperidin-2-yl, piperidine-4-methyl, piperidine-3-methyl, piperidine-2-methyl, pyrrolidin-3-yl-methyl, pyrrolidin-2-yl-methyl, pyrrolidin-3-yl, azetidin-3-yl-methyl, tetrahydro-thiopyran-4-yl, tetrahydropyran-4-yl, imidazol-4-yl-methyl, phenyl, o-tolyl, m-tolyl, p-tolyl, 2-fluorophenyl, 3-fluorophenyl, 4-fluorophenyl, 2-chlorophenyl, 3-chlorophenyl, 4-chlorophenyl, 3-hydroxy-phenyl, 2-methoxyphenyl, 3-methoxy-phenyl, 4-methoxy-phenyl, 4-aminophenyl, 4-nitrophenyl, 4-fluoro-2-methyl-phenyl, 3-fluoro-2-methyl-phenyl, 3,4-difluorophenyl, 3,5-difluorophenyl, 2,6-dichlorophenyl, pyridyl, thiophene, 5-chlorothiophene or benzyl,
- R7 and R8 being able to form, with the nitrogen to which they are attached, a 2,3-dihydro-indolyl, piperidinyl, piperazinyl, pyrrolidinyl or morpholinyl ring.

5. Compound according to Claim 4, **characterized in that** the substituents are selected from the halogens, the groups hydroxyl, amino (primary, secondary or tertiary amine), nitro, the C1-C4 alkyl radicals, the C1-C4 perfluoroalkyl, alkoxy, perfluoroalkoxy, alkoxycarbonyl, carbamoyl, sulfamoyl, sulfonyl, sulfenyl, sulfanyl, or cyano radicals or an oxo, carbonylamino, aryl, heteroaryl or cycloalkyl radical.

6. Compound according to one of Claims 1 to 5, **characterized in that** it corresponds to general formula (II):

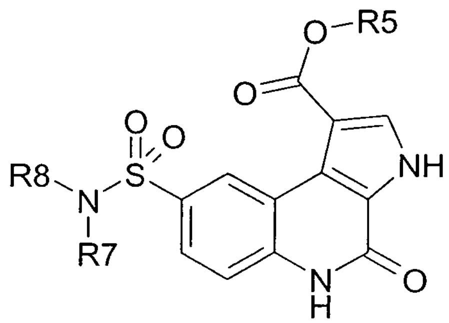

(II),

R5, R7 and R8 being defined in Claims 1 to 5.

7. Compound according to Claim 6, selected from the following compounds, and physiologically acceptable salts thereof:

Ethyl 4-oxo-8-phenylsulfamoyl-4,5-dihydro-3*H*-pyrrolo[2,3-*c*]quinoline-1-carboxylate
Ethyl 8-(methyl-phenyl-sulfamoyl)-4-oxo-4,5-dihydro-3*H*-pyrrolo[2,3-*c*]quinoline-1-carboxylate
Ethyl 4-oxo-8-*o*-tolylsulfamoyl-4,5-dihydro-3*H*-pyrrolo[2,3-*c*]quinoline-1-carboxylate
Ethyl 4-oxo-8-*m*-tolylsulfamoyl-4,5-dihydro-3*H*-pyrrolo[2,3-*c*]quinoline-1-carboxylate
Ethyl 4-oxo-8-*p*-tolylsulfamoyl-4,5-dihydro-3*H*-pyrrolo[2,3-*c*]quinoline-1-carboxylate
Ethyl 8-(2-methoxy-phenylsulfamoyl)-4-oxo-4,5-dihydro-3*H*-pyrrolo[2,3-*c*]quinoline-1-carboxylate
Ethyl 8-(3-methoxy-phenylsulfamoyl)-4-oxo-4,5-dihydro-3*H*-pyrrolo[2,3-*c*]quinoline-1-carboxylate
Ethyl 8-(4-methoxy-phenylsulfamoyl)-4-oxo-4,5-dihydro-3*H*-pyrrolo[2,3-*c*]quinoline-1-carboxylate
Ethyl 8-(3-chlorophenylsulfamoyl)-4-oxo-4,5-dihydro-3*H*-pyrrolo[2,3-*c*]quinoline-1-carboxylate
Ethyl 8-(4-chlorophenylsulfamoyl)-4-oxo-4,5-dihydro-3*H*-pyrrolo[2,3-*c*]quinoline-1-carboxylate
Ethyl 8-(4-fluorophenylsulfamoyl)-4-oxo-4,5-dihydro-3*H*-pyrrolo[2,3-*c*]quinoline-1-carboxylate
Ethyl 8-[(4-fluorophenyl)-methyl-sulfamoyl)-4-oxo-4,5-dihydro-3*H*-pyrrolo[2,3-*c*]quinoline-1-carboxylate
8-[(4-Fluorophenyl)-methyl-sulfamoyl]-4-oxo-4,5-dihydro-3*H*-pyrrolo[2,3-*c*]quinoline-1-carboxylic acid
2-Hydroxyethyl 8-[(4-fluorophenyl)-methyl-sulfamoyl]-4-oxo-4,5-dihydro-3*H*-pyrrolo[2,3-*c*]quinoline-1-carboxylate
2-Aminoethyl 8-[(4-fluorophenyl)-methyl-sulfamoyl]-4-oxo-4,5-dihydro-3*H*-pyrrolo[2,3-*c*]quinoline-1-carboxylate hydrochloride
2-*tert*-Butoxycarbonylaminoethyl 8-[(4-fluorophenyl)-methyl-sulfamoyl]-4-oxo-4,5-dihydro-3*H*-pyrrolo[2,3-*c*] quinoline-1-carboxylate
3-Aminopropyl 8-[(4-fluorophenyl)-methyl-sulfamoyl]-4-oxo-4,5-dihydro-3*H*-pyrrolo[2,3-*c*]quinoline-1-carboxylate hydrochloride
3-*tert*-Butoxycarbonylamino-propyl 8-[(4-fluorophenyl)-methyl-sulfamoyl]-4-oxo-4,5-dihydro-3*H*-pyrrolo[2,3-*c*] quinoline-1-carboxylate
Ethyl 8-(4-ethoxycarbonyl-phenylsulfamoyl)-4-oxo-4,5-dihydro-3*H*-pyrrolo[2,3-*c*]quinoline-1-carboxylate
Ethyl 8-(3-hydroxy-phenylsulfamoyl)-4-oxo-4,5-dihydro-3*H*-pyrrolo[2,3-*c*]quinoline-1-carboxylate
Ethyl 8-(3-fluoro-2-methyl-phenylsulfamoyl)-4-oxo-4,5-dihydro-3*H*-pyrrolo[2,3-*c*]quinoline-1-carboxylate
Ethyl 8-(4-fluoro-2-methyl-phenylsulfamoyl)-4-oxo-4,5-dihydro-3*H*-pyrrolo[2,3-*c*]quinoline-1-carboxylate
Ethyl 8-(3,4-difluorophenylsulfamoyl)-4-oxo-4,5-dihydro-3*H*-pyrrolo[2,3-*c*]quinoline-1-carboxylate
Ethyl 8-(3,5-difluorophenylsulfamoyl)-4-oxo-4,5-dihydro-3*H*-pyrrolo[2,3-*c*]quinoline-1-carboxylate
Ethyl 8-(4-aminophenylsulfamoyl)-4-oxo-4,5-dihydro-3*H*-pyrrolo[2,3-*c*]quinoline-1-carboxylate
Ethyl 8-(2,3-dihydro-indole-1-sulfonyl)-4-oxo-4,5-dihydro-3*H*-pyrrolo[2,3-*c*]quinoline-1-carboxylate
Ethyl 8-(4-morpholin-4-yl-phenylsulfamoyl)-4-oxo-4,5-dihydro-3*H*-pyrrolo[2,3-*c*]quinoline-1-carboxylate
Ethyl 4-oxo-8-(pyridin-3-ylsulfamoyl)-4,5-dihydro-3*H*-pyrrolo[2,3-*c*]quinoline-1-carboxylate
Ethyl 8-(benzyl-methyl-sulfamoyl)-4-oxo-4,5-dihydro-3*H*-pyrrolo[2,3-*c*]quinoline-1-carboxylate
Ethyl 8-(methyl-phenethyl-sulfamoyl)-4-oxo-4,5-dihydro-3*H*-pyrrolo[2,3-*c*]quinoline-1-carboxylate
Ethyl 8-dimethylsulfamoyl-4-oxo-4,5-dihydro-3*H*-pyrrolo[2,3-*c*]quinoline-1-carboxylate
*N*-Propyl-8-dimethylsulfamoyl-4-oxo-4,5-dihydro-3*H*-pyrrolo[2,3-*c*]quinoline-1-carboxamide

*N*-Methyl-*N*-propyl-8-dimethylsulfamoyl-4-oxo-4,5-dihydro-3*H*-pyrrolo[2,3-*c*]quinoline-1-carboxamide
Ethyl 8-methylsulfamoyl-4-oxo-4,5-dihydro-3*H*-pyrrolo[2,3-*c*]quinoline-1-carboxylate
Ethyl 8-(morpholine-4-sulfonyl)-4-oxo-4,5-dihydro-3*H*-pyrrolo[2,3-*c*]quinoline-1-carboxylate
Ethyl 8-(2-ethoxycarbonyl-ethylsulfamoyl)-4-oxo-4,5-dihydro-3*H*-pyrrolo[2,3-*c*]quinoline-1-carboxylate
Methyl 8-cyclohexylsulfamoyl-4-oxo-4,5-dihydro-3*H*-pyrrolo[2,3-*c*]quinoline-1-carboxylate
Ethyl 8-cyclohexylsulfamoyl-4-oxo-4,5-dihydro-3*H*-pyrrolo[2,3-*c*]quinoline-1-carboxylate
Ethyl 8-cycloheptylsulfamoyl-4-oxo-4,5-dihydro-3*H*-pyrrolo[2,3-*c*]quinoline-1-carboxylate
Ethyl 8-(3-dimethylamino-propylsulfamoyl)-4-oxo-4,5-dihydro-3*H*-pyrrolo[2,3-*c*]quinoline-1-carboxylate
Ethyl 8-(4-amino-butylsulfamoyl)-4-oxo-4,5-dihydro-3*H*-pyrrolo[2,3-*c*]quinoline-1-carboxylate
Ethyl 8-[(3-chlorophenyl)-methyl-sulfamoyl)-4-oxo-4,5-dihydro-3*H*-pyrrolo[2,3-*c*]quinoline-1-carboxylate
Ethyl 4-oxo-8-(piperidin-4-ylsulfamoyl)-4,5-dihydro-3*H*-pyrrolo[2,3-*c*]quinoline-1-carboxylate
Methyl 4-oxo-8-(piperidin-3-ylsulfamoyl)-4,5-dihydro-3*H*-pyrrolo[2,3-*c*]quinoline-1-carboxylate
Methyl 4-oxo-8-(piperidin-4-ylsulfamoyl)-4,5-dihydro-3*H*-pyrrolo[2,3-*c*]quinoline-1-carboxylate
Propyl 4-oxo-8-(piperidin-4-ylsulfamoyl)-4,5-dihydro-3*H*-pyrrolo[2,3-*c*]quinoline-1-carboxylate
Butyl 4-oxo-8-(piperidin-4-ylsulfamoyl)-4,5-dihydro-3*H*-pyrrolo[2,3-*c*]quinoline-1-carboxylate
Pentyl 4-oxo-8-(piperidin-4-ylsulfamoyl)-4,5-dihydro-3*H*-pyrrolo[2,3-*c*]quinoline-1-carboxylate
Methylcyclohexyl 4-oxo-8-(piperidin-4-ylsulfamoyl)-4,5-dihydro-3*H*-pyrrolo[2,3-*c*]quinoline-1-carboxylate
Methyltetrahydrofuran-3-yl 4-oxo-8-(piperidin-4-ylsulfamoyl)-4,5-dihydro-3*H*-pyrrolo[2,3-*c*]quinoline-1-carboxylate
Cyclopentyl 8-(1-methyl-piperidin-4-ylsulfamoyl)-4-oxo-4,5-dihydro-3*H*-pyrrolo[2,3-*c*]quinoline-1-carboxylate
Propyl 8-(methyl-piperidin-4-yl-sulfamoyl)-4-oxo-4,5-dihydro-3*H*-pyrrolo[2,3-*c*]quinoline-1-carboxylate
Ethyl 4-oxo-8-[(piperidin-4-ylmethyl)-sulfamoyl]-4,5-dihydro-3*H*-pyrrolo[2,3-*c*]quinoline-1-carboxylate
Ethyl 8-(8-aza-bicyclo[3.2.1]oct-3-ylsulfamoyl)-4-oxo-4,5-dihydro-3*H*-pyrrolo[2,3-*c*]quinoline-1-carboxylate
Methyl 8-cyclopentylsulfamoyl-4-oxo-4,5-dihydro-3*H*-pyrrolo[2,3-*c*]quinoline-1-carboxylate
Ethyl 8-(hexahydro-pyrrolo[3,4-*c*]pyrrole-2-sulfonyl)-4-oxo-4,5-dihydro-3*H*-pyrrolo[2,3-*c*]quinoline-1-carboxylate
Ethyl 8-(1-azabicyclo[2.2.2]oct-3-ylsulfamoyl)-4-oxo-4,5-dihydro-3*H*-pyrrolo[2,3-*c*]quinoline-1-carboxylate
Ethyl 8-(4-methyl-piperazine-1-sulfonyl)-4-oxo-4,5-dihydro-3*H*-pyrrolo[2,3-*c*]quinoline-1-carboxylate
Ethyl 4-oxo-8-sulfamoyl-4,5-dihydro-3*H*-pyrrolo[2,3-*c*]quinoline-1-carboxylate
Ethyl 4-oxo-8-[(pyrrolidin-3-methyl)-sulfamoyl]-4,5-dihydro-3*H*-pyrrolo[2,3-*c*]quinoline-1-carboxylate
Ethyl 4-oxo-8-(4-Hydroxy-phenylsulfamoyl)-4,5-dihydro-3*H*-pyrrolo[2,3-*c*]quinoline-1-carboxylate
Ethyl 8-cyclopropylsulfamoyl-4-oxo-4,5-dihydro-3*H*-pyrrolo[2,3-*c*]quinoline-1-carboxylate
Propyl 8-cyclopropylsulfamoyl-4-oxo-4,5-dihydro-3*H*-pyrrolo[2,3-*c*]quinoline-1-carboxylate
Propyl 8-(cyclopropyl-methyl-sulfamoyl)-4-oxo-4,5-dihydro-3*H*-pyrrolo[2,3-*c*]quinoline-1-carboxylate
Propyl 8-[(2-cyano-ethyl)-methyl-sulfamoyl]-4-oxo-4,5-dihydro-3*H*-pyrrolo[2,3-*c*]quinoline-1-carboxylate
Ethyl 8-[(2-cyano-ethyl)-cyclopropyl-sulfamoyl]-4-oxo-4,5-dihydro-3*H*-pyrrolo[2,3-*c*]quinoline-1-carboxylate
Propyl 8-(cyanomethyl-methyl-sulfamoyl)-4-oxo-4,5-dihydro-3*H*-pyrrolo[2,3-*c*]quinoline-1-carboxylate
Propyl 8-([1,3]dioxolan-2-ylmethyl-methyl-sulfamoyl)-4-oxo-4,5-dihydro-3*H*-pyrrolo[2,3-*c*]quinoline-1-carboxylate
Propyl 8-[(1-benzhydryl-azetidin-3-yl)-methyl-sulfamoyl]-4-oxo-4,5-dihydro-3*H*-pyrrolo[2,3-*c*]quinoline-1-carboxylate
Propyl 8-[methyl-(tetrahydro-thiopyran-4-yl)-sulfamoyl]-4-oxo-4,5-dihydro-3*H*-pyrrolo[2,3-*c*]quinoline-1-carboxylate
Propyl 8-[(1,1-dioxo-hexahydro-116-thiopyran-4-yl)-methyl-sulfamoyl]-4-oxo-4,5-dihydro-3*H*-pyrrolo[2,3-*c*]quinoline-1-carboxylate
Propyl 8-[methyl-(tetrahydro-pyran-4-yl)-sulfamoyl]-4-oxo-4,5-dihydro-3*H*-pyrrolo[2,3-*c*]quinoline-1-carboxylate
Propyl 8-(methyl-prop-2-ynyl-sulfamoyl)-4-oxo-4,5-dihydro-3*H*-pyrrolo[2,3-*c*]quinoline-1-carboxylate
Propyl 8-[methyl-(2-methylsulfanyl-ethyl)-sulfamoyl]-4-oxo-4,5-dihydro-3*H*-pyrrolo[2,3-*c*]quinoline-1-carboxylate
Propyl 8-[(2-dimethylamino-ethyl)-methyl-sulfamoyl]-4-oxo-4,5-dihydro-3*H*-pyrrolo[2,3-*c*]quinoline-1-carboxylate
Propyl 8-[methyl-(2-pyrrolidin-1-yl-ethyl)-sulfamoyl]-4-oxo-4,5-dihydro-3*H*-pyrrolo[2,3-*c*]quinoline-1-carboxylate
Propyl 8-[(2-diethylamino-ethyl)-methyl-sulfamoyl]-4-oxo-4,5-dihydro-3*H*-pyrrolo[2,3-*c*]quinoline-1-carboxylate
Propyl 8-[methyl-(2-methylamino-ethyl)-sulfamoyl]-4-oxo-4,5-dihydro-3*H*-pyrrolo[2,3-*c*]quinoline-1-carboxylate hydrochloride

Propyl 8-{[2-(*tert*-butoxycarbonyl-methyl-amino)-ethyl]-methyl-sulfamoyl}-4-oxo-4,5-dihydro-3*H*-pyrrolo[2,3-*c*]quinoline-1-carboxylate

Propyl 8-[cyclopropyl-(2-methyl-2-methylamino-propyl)-sulfamoyl]-4-oxo-4,5-dihydro-3*H*-pyrrolo[2,3-*c*]quinoline-1-carboxylate hydrochloride

Propyl 8-[cyclopropyl-(2-isopropylamino-ethyl)-sulfamoyl]-4-oxo-4,5-dihydro-3*H*-pyrrolo[2,3-*c*]quinoline-1-carboxylate hydrochloride

Propyl 8-[cyclopropyl-(1*H*-imidazol-4-ylmethyl)-sulfamoyl]-4-oxo-4,5-dihydro-3*H*-pyrrolo[2,3-*c*]quinoline-1-carboxylate hydrochloride

Propyl (S)-8-(cyclopropyl-pyrrolidin-2-ylmethyl-sulfamoyl)-4-oxo-4,5-dihydro-3*H*-pyrrolo[2,3-*c*]quinoline-1-carboxylate hydrochloride

Propyl (S)-8-[cyclopropyl-(1-methyl-pyrrolidin-2-ylmethyl)-sulfamoyl]-4-oxo-4,5-dihydro-3*H*-pyrrolo[2,3-*c*]quinoline-1-carboxylate

Propyl (R)-8-[cyclopropyl-(1-methyl-pyrrolidin-2-ylmethyl)-sulfamoyl]-4-oxo-4,5-dihydro-3*H*-pyrrolo[2,3-*c*]quinoline-1-carboxylate

Propyl (R)-8-[(1-*tert*-butoxycarbonyl-pyrrolidin-2-ylmethyl)-cyclopropyl-sulfamoyl]-4-oxo-4,5-dihydro-3*H*-pyrrolo[2,3-*c*]quinoline-1-carboxylate

Propyl (R)-8-(cyclopropyl-pyrrolidin-2-ylmethyl-sulfamoyl)-4-oxo-4,5-dihydro-3*H*-pyrrolo[2,3-*c*]quinoline-1-carboxylate hydrochloride

Propyl (S)-8-(isopropyl-pyrrolidin-2-ylmethyl-sulfamoyl)-4-oxo-4,5-dihydro-3*H*-pyrrolo[2,3-*c*]quinoline-1-carboxylate hydrochloride

Propyl (S)-4-oxo-8-(prop-2-ynyl-pyrrolidin-2-ylmethyl-sulfamoyl)-4,5-dihydro-3*H*-pyrrolo[2,3-*c*]quinoline-1-carboxylate hydrochloride

Propyl (S)-8-[(1-methyl-pyrrolidin-2-ylmethyl)-prop-2-ynyl-sulfamoyl]-4-oxo-4,5-dihydro-3*H*-pyrrolo[2,3-*c*]quinoline-1-carboxylate

Propyl (S)-8-[(2-*tert*-butoxycarbonylamino-propyl)-cyclopropyl-sulfamoyl]-4-oxo-4,5-dihydro-3*H*-pyrrolo[2,3-*c*]quinoline-1-carboxylate

Propyl (S)-8-[(2-amino-propyl)-cyclopropyl-sulfamoyl]-4-oxo-4,5-dihydro-3*H*-pyrrolo[2,3-*c*]quinoline-1-carboxylate hydrochloride

Propyl (R)-8-[(2-*tert*-butoxycarbonylamino-propyl)-cyclopropyl-sulfamoyl]-4-oxo-4,5-dihydro-3*H*-pyrrolo[2,3-*c*]quinoline-1-carboxylate

Propyl (R)-8-[(2-amino-propyl)-cyclopropyl-sulfamoyl]-4-oxo-4,5-dihydro-3*H*-pyrrolo[2,3-*c*]quinoline-1-carboxylate hydrochloride

Propyl 8-[(2-methylamino-ethyl)-prop-2-ynyl-sulfamoyl]-4-oxo-4,5-dihydro-3*H*-pyrrolo[2,3-*c*]quinoline-1-carboxylate hydrochloride

Propyl 8-{[2-(*tert*-butoxycarbonyl-methyl-amino)-ethyl]-prop-2-ynyl-sulfamoyl}-4-oxo-4,5-dihydro-3*H*-pyrrolo[2,3-*c*]quinoline-1-carboxylate

Propyl 8-[(2-isopropylamino-ethyl)-prop-2-ynyl-sulfamoyl]-4-oxo-4,5-dihydro-3*H*-pyrrolo[2,3-*c*]quinoline-1-carboxylate hydrochloride

Propyl 8-{[2-(*tert*-butoxycarbonyl-isopropyl-amino)-ethyl]-prop-2-ynyl-sulfamoyl}-4-oxo-4,5-dihydro-3*H*-pyrrolo[2,3-*c*]quinoline-1-carboxylate

Propyl 8-(ethyl-pyrrolidin-3-yl-sulfamoyl)-4-oxo-4,5-dihydro-3*H*-pyrrolo[2,3-*c*]quinoline-1-carboxylate hydrochloride

Propyl 8-[ethyl-(1-methyl-pyrrolidin-3-yl)-sulfamoyl]-4-oxo-4,5-dihydro-3*H*-pyrrolo[2,3-*c*]quinoline-1-carboxylate

Ethyl 4-oxo-8-(pyrrolidine-1-sulfonyl)-4,5-dihydro-3*H*-pyrrolo[2,3-*c*]quinoline-1-carboxylate

Ethyl 4-oxo-8-(2-pyrrolidin-1-ylmethyl-pyrrolidine-1-sulfonyl)-4,5-dihydro-3*H*-pyrrolo[2,3-*c*]quinoline-1-carboxylate

Propyl (S)-8-(3-*tert*-butoxycarbonylamino-pyrrolidine-1-sulfonyl)-4-oxo-4,5-dihydro-3*H*-pyrrolo[2,3-*c*]quinoline-1-carboxylate

(S)-8-(3-*tert*-Butoxycarbonylamino-pyrrolidine-1-sulfonyl)-4-oxo-4,5-dihydro-3*H*-pyrrolo[2,3-*c*]quinoline-1-carboxylic acid

Propyl (S)-8-(3-amino-cyclopentanesulfonyl)-4-oxo-4,5-dihydro-3*H*-pyrrolo[2,3-*c*]quinoline-1-carboxylate hydrochloride

Propyl (R)-8-(3-amino-cyclopentanesulfonyl)-4-oxo-4,5-dihydro-3*H*-pyrrolo[2,3-*c*]quinoline-1-carboxylate hydrochloride

Propyl 8-(3-methylamino-pyrrolidine-1-sulfonyl)-4-oxo-4,5-dihydro-3*H*-pyrrolo[2,3-*c*]quinoline-1-carboxylate hydrochloride

Propyl 8-[3-(*tert*-butoxycarbonyl-methyl-amino)-pyrrolidine-1-sulfonyl]-4-oxo-4,5-dihydro-3*H*-pyrrolo[2,3-*c*]quinoline-1-carboxylate
8-[3-(*tert*-Butoxycarbonyl-methyl-amino)-pyrrolidine-1-sulfonyl]-4-oxo-4,5-dihydro-3*H*-pyrrolo[2,3-*c*]quinoline-1-carboxylic acid
Propyl (S)-8-(3-dimethylamino-cyclopentanesulfonyl)-4-oxo-4,5-dihydro-3*H*-pyrrolo[2,3-*c*]quinoline-1-carboxylate
Propyl (R)-8-(3-dimethylamino-cyclopentanesulfonyl)-4-oxo-4,5-dihydro-3*H*-pyrrolo[2,3-*c*]quinoline-1-carboxylate
Propyl 8-[(2-hydroxy-ethyl)-isopropyl-sulfamoyl]-4-oxo-4,5-dihydro-3*H*-pyrrolo[2,3-*c*]quinoline-1-carboxylate
Propyl 8-[cyclopropyl-(2-hydroxy-ethyl)-sulfamoyl]-4-oxo-4,5-dihydro-3*H*-pyrrolo[2,3-*c*]quinoline-1-carboxylate
Propyl 8-[(2-hydroxy-ethyl)-prop-2-ynyl-sulfamoyl]-4-oxo-4,5-dihydro-3*H*-pyrrolo[2,3-*c*]quinoline-1-carboxylate
Propyl 8-[bis-(2-hydroxy-ethyl)-sulfamoyl]-4-oxo-4,5-dihydro-3*H*-pyrrolo[2,3-*c*]quinoline-1-carboxylate
Propyl 8-(carbamoylmethyl-methyl-sulfamoyl)-4-oxo-4,5-dihydro-3*H*-pyrrolo[2,3-*c*]quinoline-1-carboxylate
8-(Carbamoylmethyl-methyl-sulfamoyl)-4-oxo-4,5-dihydro-3*H*-pyrrolo[2,3-*c*]quinoline-1-carboxylic acid
Propyl 8-(3-carbamoyl-piperidine-1-sulfonyl)-4-oxo-4,5-dihydro-3*H*-pyrrolo[2,3-*c*]quinoline-1-carboxylate
8-(3-Carbamoyl-piperidine-1-sulfonyl)-4-oxo-4,5-dihydro-3*H*-pyrrolo[2,3-*c*]quinoline-1-carboxylic acid
Propyl 8-[(2-benzyloxy-ethyl)-cyclopropyl-sulfamoyl]-4-oxo-4,5-dihydro-3*H*-pyrrolo[2,3-*c*]quinoline-1-carboxylate
Propyl 8-(carboxymethyl-methyl-sulfamoyl)-4-oxo-4,5-dihydro-3*H*-pyrrolo[2,3-*c*]quinoline-1-carboxylate
Propyl 8-(*tert*-butoxycarbonylmethyl-methyl-sulfamoyl)-4-oxo-4,5-dihydro-3*H*-pyrrolo[2,3-*c*]quinoline-1-carboxylate
Propyl 8-[(2-dimethylamino-ethyl)-methyl-sulfamoyl]-7-fluoro-4-oxo-4,5-dihydro-3*H*-pyrrolo[2,3-*c*]quinoline-1-carboxylate
Propyl 7-fluoro-8-[(2-hydroxy-ethyl)-isopropyl-sulfamoyl]-4-oxo-4,5-dihydro-3*H*-pyrrolo[2,3-*c*]quinoline-1-carboxylate
Propyl 8-dimethylsulfamoyl-7-fluoro-4-oxo-4,5-dihydro-3*H*-pyrrolo[2,3-*c*]quinoline-1-carboxylate
Propyl 8-(cyclopropyl-methyl-sulfamoyl)-7-fluoro-4-oxo-4,5-dihydro-3*H*-pyrrolo[2,3-*c*]quinoline-1-carboxylate
Propyl 8-(cyclopropyl-methyl-sulfamoyl)-7-methoxy-4-oxo-4,5-dihydro-3*H*-pyrrolo[2,3-*c*]quinoline-1-carboxylate
Propyl 7-chloro-8-dimethylsulfamoyl-4-oxo-4,5-dihydro-3*H*-pyrrolo[2,3-*c*]quinoline-1-carboxylate
Propyl (S)-8-(3-amino-cyclopentanesulfonyl)-7-chloro-4-oxo-4,5-dihydro-3*H*-pyrrolo[2,3-*c*]quinoline-1-carboxylate hydrochloride
Methyl 4-oxo-8-(piperidin-1-ylsulfamoyl)-4,5-dihydro-3*H*-pyrrolo[2,3-*c*]quinoline-1-carboxylate
Methyl 8-(hexahydro-cyclopenta[*c*]pyrrol-2-ylsulfamoyl)-4-oxo-4,5-dihydro-3*H*-pyrrolo[2,3-*c*]quinoline-1-carboxylate
Methyl 8-(4-methyl-piperazin-1-ylsulfamoyl)-4-oxo-4,5-dihydro-3*H*-pyrrolo[2,3-*c*]quinoline-1-carboxylate.

8. Compound according to one of Claims 1 to 5, **characterized in that** it corresponds to general formula (III):

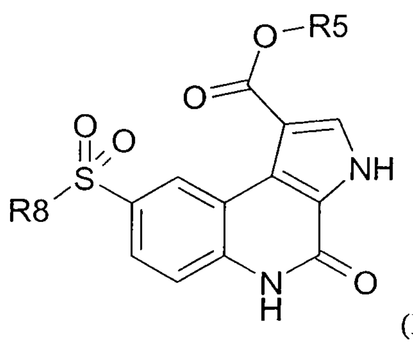

(III),

R8 being defined in one of Claims 1 to 5.

9. Compound according to Claim 8, selected from the following compounds, and physiologically acceptable salts thereof:

Ethyl 4-oxo-8-phenylmethanesulfonyl-4,5-dihydro-3*H*-pyrrolo[2,3-*c*]quinoline-1-carboxylate
Ethyl 8-(2,6-dichlorophenylmethanesulfonyl)-4-oxo-4,5-dihydro-3*H*-pyrrolo[2,3-*c*]quinoline-1-carboxylate

8-(2,6-Dichlorophenylmethanesulfonyl)-4-oxo-4,5-dihydro-3*H*-pyrrolo[2,3-*c*]quinoline-1-carboxylic acid
Ethyl 4-oxo-8-(pyridin-4-ylmethanesulfonyl)-4,5-dihydro-3*H*-pyrrolo[2,3-*c*]quinoline-1-carboxylate
Ethyl 4-oxo-8-(pyridin-2-ylmethanesulfonyl)-4,5-dihydro-3*H*-pyrrolo[2,3-*c*]quinoline-1-carboxylate
Ethyl 8-cyclohexanesulfonyl-4-oxo-4,5-dihydro-3*H*-pyrrolo[2,3-*c*]quinoline-1-carboxylate
Ethyl 4-oxo-8-phenylmethanesulfinyl-4,5-dihydro-3*H*-pyrrolo[2,3-*c*]quinoline-1-carboxylate.

**10.** Compound according to one of Claims 1 to 5, **characterized in that** it corresponds to general formula (IV):

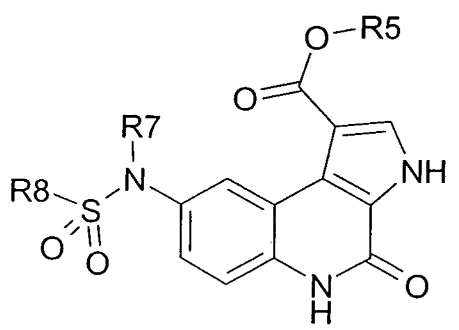

(IV),

R7 and R8 being defined in one of Claims 1 to 5.

**11.** Compound according to Claim 10, selected from the following compounds, and physiologically acceptable salts thereof:

Ethyl 8-benzenesulfonylamino-4-oxo-4,5-dihydro-3*H*-pyrrolo[2,3-*c*]quinoline-1-carboxylate
Ethyl 8-methanesulfonylamino-4-oxo-4,5-dihydro-3*H*-pyrrolo[2,3-*c*]quinoline-1-carboxylate
Ethyl 4-oxo-8-phenylmethanesulfonylamino-4,5-dihydro-3*H*-pyrrolo[2,3-*c*]quinoline-1-carboxylate
Ethyl 8-(4-chloro-benzenesulfonylamino)-4-oxo-4,5-dihydro-3*H*-pyrrolo[2,3-*c*]quinoline-1-carboxylate
Ethyl 8-(3-chloro-benzenesulfonylamino)-4-oxo-4,5-dihydro-3*H*-pyrrolo[2,3-*c*]quinoline-1-carboxylate
Ethyl 8-(2-chloro-benzenesulfonylamino)-4-oxo-4,5-dihydro-3*H*-pyrrolo[2,3-*c*]quinoline-1-carboxylate
Ethyl 8-(4-fluoro-benzenesulfonylamino)-4-oxo-4,5-dihydro-3*H*-pyrrolo[2,3-*c*]quinoline-1-carboxylate
Ethyl 8-(3-fluoro-benzenesulfonylamino)-4-oxo-4,5-dihydro-3*H*-pyrrolo[2,3-*c*]quinoline-1-carboxylate
Ethyl 8-(2-fluoro-benzenesulfonylamino)-4-oxo-4,5-dihydro-3*H*-pyrrolo[2,3-*c*]quinoline-1-carboxylate
Ethyl 8-(4-bromo-benzenesulfonylamino)-4-oxo-4,5-dihydro-3*H*-pyrrolo[2,3-*c*]quinoline-1-carboxylate
Ethyl 4-oxo-8-(4-toluenesulfonylamino)-4,5-dihydro-3*H*-pyrrolo[2,3-*c*]quinoline-1-carboxylate
Ethyl 4-oxo-8-(3-toluenesulfonylamino)-4,5-dihydro-3*H*-pyrrolo[2,3-*c*]quinoline-1-carboxylate
Ethyl 8-(4-*t*ert-butyl-benzenesulfonylamino)-4-oxo-4,5-dihydro-3*H*-pyrrolo[2,3-*c*]quinoline-1-carboxylate
Ethyl 8-(4-methoxy-benzenesulfonylamino)-4-oxo-4,5-dihydro-3*H*-pyrrolo[2,3-*c*]quinoline-1-carboxylate
Ethyl 8-(3-methoxy-benzenesulfonylamino)-4-oxo-4,5-dihydro-3*H*-pyrrolo[2,3-*c*]quinoline-1-carboxylate
Ethyl 8-(4-acetylamino-benzenesulfonylamino)-4-oxo-4,5-dihydro-3*H*-pyrrolo[2,3-*c*]quinoline-1-carboxylate
Ethyl 8-(4-nitro-benzenesulfonylamino)-4-oxo-4,5-dihydro-3*H*-pyrrolo[2,3-*c*]quinoline-1-carboxylate
Ethyl 8-(naphthalene-2-sulfonylamino)-4-oxo-4,5-dihydro-3*H*-pyrrolo[2,3-*c*]quinoline-1-carboxylate
Ethyl 8-(4-acetyl-benzenesulfonylamino)-4-oxo-4,5-dihydro-3*H*-pyrrolo[2,3-*c*]quinoline-1-carboxylate
Ethyl 4-oxo-8-(thiophene-2-sulfonylamino)-4,5-dihydro-3*H*-pyrrolo[2,3-*c*]quinoline-1-carboxylate
Ethyl 8-(5-chloro-thiophene-2-sulfonylamino)-4-oxo-4,5-dihydro-3*H*-pyrrolo[2,3-*c*]quinoline-1-carboxylate
Ethyl 8-(benzenesulfonyl-methyl-amino)-4-oxo-4,5-dihydro-3*H*-pyrrolo[2,3-*c*]quinoline-1-carboxylate
Ethyl 8-(methanesulfonyl-methyl-amino)-4-oxo-4,5-dihydro-3*H*-pyrrolo[2,3-*c*]quinoline-carboxylate.

**12.** Compound according to one of Claims 1 to 5, **characterized in that** it corresponds to general formula (V):

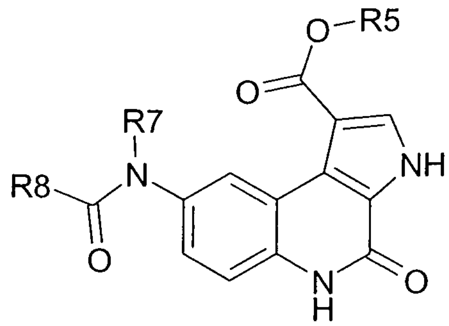

(V),

R7 and R8 being defined in one of Claims 1 to 5.

**13.** Compound according to Claim 12, selected from the following compounds, and physiologically acceptable salts thereof:

8-Benzoylamino-4-oxo-4,5-dihydro-3*H*-pyrrolo[2,3-*c*]quinoline-1-ethyl carboxylate
8-Acetylamino-4-oxo-4,5-dihydro-3*H*-pyrrolo[2,3-*c*]quinoline-1-ethyl carboxylate.

**14.** Compound according to one of Claims 1 to 5, **characterized in that** it corresponds to general formula (VI):

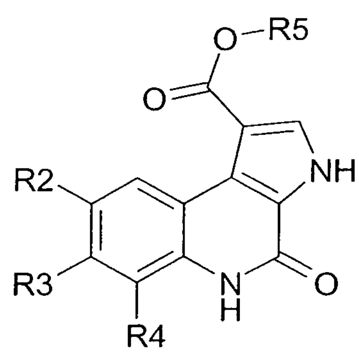

(VI),

R2, R3 and R4 being defined in one of Claims 1 to 5.

**15.** Compound according to Claim 14, selected from the following compounds, and physiologically acceptable salts thereof:

Ethyl 8-dimethylamino-4-oxo-4,5-dihydro-3*H*-pyrrolo[2,3-*c*]quinoline-1-carboxylate
Ethyl 8-methylamino-4-oxo-4,5-dihydro-3*H*-pyrrolo[2,3-*c*]quinoline-1-carboxylate
Ethyl 8-amino-4-oxo-4,5-dihydro-3*H*-pyrrolo[2,3-*c*]quinoline-1-carboxylate
Ethyl 8-nitro-4-oxo-4,5-dihydro-3*H*-pyrrolo[2,3-*c*]quinoline-1-carboxylate
Ethyl 6-nitro-4-oxo-4,5-dihydro-3*H*-pyrrolo[2,3-*c*]quinoline-1-carboxylate
Ethyl 8-(2-ethoxycarbonyl-vinyl)-4-oxo-4,5-dihydro-3*H*-pyrrolo[2,3-*c*]quinoline-1-carboxylate
Ethyl 8-(2-benzylcarbamoyl-vinyl)-4-oxo-4,5-dihydro-3*H*-pyrrolo[2,3-*c*]quinoline-1-carboxylate
Ethyl 8-[2-(2-methoxycarbonyl-ethylcarbamoyl)-vinyl]-4-oxo-4,5-dihydro-3*H*-pyrrolo[2,3-*c*]quinoline-1-carboxylate
Ethyl 4-oxo-8-styryl-4,5-dihydro-3*H*-pyrrolo[2,3-*c*]quinoline-1-carboxylate
Ethyl 8-ethynyl-4-oxo-4,5-dihydro-3*H*-pyrrolo[2,3-*c*]quinoline-1-carboxylate
Ethyl 4-oxo-8-trimethylsilanylethynyl-4,5-dihydro-3*H*-pyrrolo[2,3-*c*]quinoline-1-carboxylate
Ethyl 4-oxo-8-phenylethynyl-4,5-dihydro-3*H*-pyrrolo[2,3-*c*]quinoline-1-carboxylate
Ethyl 4-oxo-8-pyridin-2-ylethynyl-4,5-dihydro-3*H*-pyrrolo[2,3-*c*]quinoline-1-carboxylate
Ethyl 8-(3-dimethylamino-prop-1-ynyl)-4-oxo-4,5-dihydro-3*H*-pyrrolo[2,3-*c*]quinoline-1-carboxylate

Ethyl 8-(3-hydroxy-3-methyl-but-1-ynyl)-4-oxo-4,5-dihydro-3*H*-pyrrolo[2,3-*c*]quinoline-1-carboxylate
Ethyl 8-(3-morpholin-4-yl-prop-1-ynyl)-4-oxo-4,5-dihydro-3*H*-pyrrolo[2,3-*c*]quinoline-1-carboxylate
Ethyl 8-{3-[(2-cyano-ethyl)-methyl-amino]-prop-1-ynyl}-4-oxo-4,5-dihydro-3*H*-pyrrolo[2,3-*c*]quinoline-1-carboxylate
Ethyl 4-oxo-8-phenethyl-4,5-dihydro-3*H*-pyrrolo[2,3-*c*]quinoline-1-carboxylate
Ethyl 8-(3-hydroxy-3-methyl-butyl)-4-oxo-4,5-dihydro-3*H*-pyrrolo[2,3-*c*]quinoline-1-carboxylate
Ethyl 8-bromo-4-oxo-4,5-dihydro-3*H*-pyrrolo[2,3-*c*]quinoline-1-carboxylate
Ethyl 8-iodo-4-oxo-4,5-dihydro-3*H*-pyrrolo[2,3-*c*]quinoline-1-carboxylate
Propyl 8-iodo-4-oxo-4,5-dihydro-3*H*-pyrrolo[2,3-*c*]quinoline-1-carboxylate
Cyclopentyl 8-iodo-4-oxo-4,5-dihydro-3*H*-pyrrolo[2,3-*c*]quinoline-1-carboxylate
Isobutyl 8-iodo-4-oxo-4,5-dihydro-3*H*-pyrrolo[2,3-*c*]quinoline-1-carboxylate
Ethyl 7,8-dimethoxy-4-oxo-4,5-dihydro-3*H*-pyrrolo[2,3-*c*]quinoline-1-carboxylate
Ethyl 7-methoxy-4-oxo-4,5-dihydro-3*H*-pyrrolo[2,3-*c*]quinoline-1-carboxylate
Ethyl 8-iodo-7-methoxy-4-oxo-4,5-dihydro-3*H*-pyrrolo[2,3-*c*]quinoline-1-carboxylate
Ethyl 8-fluoro-4-oxo-4,5-dihydro-3*H*-pyrrolo[2,3-*c*]quinoline-1-carboxylate
2-Diethylaminoethyl 8-fluoro-4-oxo-4,5-dihydro-3*H*-pyrrolo[2,3-*c*]quinoline-1-carboxylate
Ethyl 7-fluoro-4-oxo-4,5-dihydro-3*H*-pyrrolo[2,3-*c*]quinoline-1-carboxylate
Ethyl 7-chloro-4-oxo-4,5-dihydro-3*H*-pyrrolo[2,3-*c*]quinoline-1-carboxylate
Ethyl 8-acetyl-4-oxo-4,5-dihydro-3*H*-pyrrolo[2,3-*c*]quinoline-1-carboxylate
Ethyl 8-benzoyl-4-oxo-4,5-dihydro-3*H*-pyrrolo[2,3-*c*]quinoline-1-carboxylate
Ethyl 8-(3-dimethylamino-acryloyl)-4-oxo-4,5-dihydro-3*H*-pyrrolo[2,3-*c*]quinoline-1-carboxylate
Ethyl 8-(2-morpholin-4-yl-acetyl)-4-oxo-4,5-dihydro-3*H*-pyrrolo[2,3-*c*]quinoline-1-carboxylate
Ethyl (S)-8-(cyclopropyl-pyrrolidin-2-ylmethyl-carbamoyl)-4-oxo-4,5-dihydro-3*H*-pyrrolo[2,3-*c*]quinoline-1-carboxylate hydrochloride
Ethyl 4-oxo-8-phenylsulfanyl-4,5-dihydro-3*H*-pyrrolo[2,3-*c*]quinoline-1-carboxylate
Ethyl 8-cyclohexylsulfanyl-4-oxo-4,5-dihydro-3*H*-pyrrolo[2,3-*c*]quinoline-1-carboxylate
Ethyl 8-benzylsulfanyl-4-oxo-4,5-dihydro-3*H*-pyrrolo[2,3-*c*]quinoline-1-carboxylate
Ethyl 8-(4-acetylaminophenylsulfanyl)-4-oxo-4,5-dihydro-3*H*-pyrrolo[2,3-*c*]quinoline-1-carboxylate
Ethyl 8-(3-aminophenylsulfanyl)-4-oxo-4,5-dihydro-3*H*-pyrrolo[2,3-*c*]quinoline-1-carboxylate
Ethyl 8-(3-acetylaminophenylsulfanyl)-4-oxo-4,5-dihydro-3*H*-pyrrolo[2,3-*c*]quinoline-1-carboxylate
Ethyl 8-(2-amino-ethylsulfanyl)-4-oxo-4,5-dihydro-3*H*-pyrrolo[2,3-*c*]quinoline-1-carboxylate hydrochloride
Ethyl 4-oxo-8-sulfo-4,5-dihydro-3*H*-pyrrolo[2,3-*c*]quinoline-1-carboxylate.

**16.** Compound according to one of Claims 1 to 5, **characterized in that** it corresponds to general formula (VII):

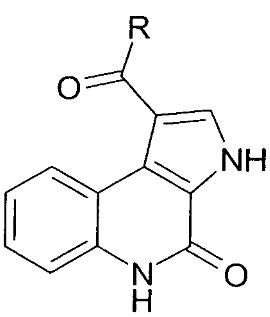

(VII),

R being defined in one of Claims 1 to 5.

**17.** Compound according to Claim 16, selected from the following compounds, and physiologically acceptable salts thereof:

4-Oxo-4,5-dihydro-3*H*-pyrrolo[2,3-*c*]quinoline-1-benzyl carboxylate
4-Oxo-4,5-dihydro-3*H*-pyrrolo[2,3-*c*]quinoline-1-isopropyl carboxylate
1-Acetyl-3,5-dihydro-pyrrolo[2,3-*c*]quinolin-4-one
*N*-(2-Dimethylamino-ethyl)-4-oxo-4,5-dihydro-3*H*-pyrrolo[2,3-*c*]quinoline-1-carboxamide
*N*-(3-Dimethylamino-propyl)-4-oxo-4,5-dihydro-3*H*-pyrrolo[2,3-*c*]quinoline-1-carboxamide

Methyl 3-[(4-oxo-4,5-dihydro-3*H*-pyrrolo[2,3-*c*]quinoline-1-carbonyl)-amino]propanoate
*N*-(Cyclohexylmethyl)-4-oxo-4,5-dihydro-3*H*-pyrrolo[2,3-*c*]quinoline-1-carboxamide
*N*-(Benzyl)-4-oxo-4,5-dihydro-3*H*-pyrrolo[2,3-*c*]quinoline-1-carboxylamide
*N*-[2-(1*H*-indol-3-yl)-ethyl]-4-oxo-4,5-dihydro-3*H*-pyrrolo[2,3-*c*]quinoline-1-carboxamide
*N*-(3-Hydroxy-propyl)-4-oxo-4,5-dihydro-3*H*-pyrrolo[2,3-*c*]quinoline-1-carboxamide
*N*-(Pyridin-4-ylmethyl)-4-oxo-4,5-dihydro-3*H*-pyrrolo[2,3-*c*]quinoline-1-carboxamide
*N*-(Phenyl)-4-oxo-4,5-dihydro-3*H*-pyrrolo[2,3-*c*]quinoline-1-carboxamide
*N*-(Methyl)-4-oxo-4,5-dihydro-3*H*-pyrrolo[2,3-*c*]quinoline-1-carboxamide
*N*,*N*-(Dimethyl)-4-oxo-4,5-dihydro-3*H*-pyrrolo[2,3-*c*]quinoline-1-carboxamide
4-Oxo-4,5-dihydro-3*H*-pyrrolo[2,3-*c*]quinoline-1-carboxamide.

**18.** Compound according to one of Claims 1 to 5, **characterized in that** it corresponds to general formula (VIII):

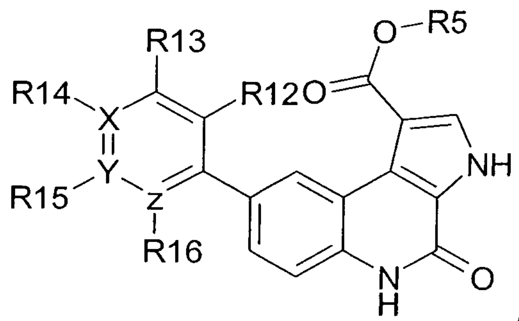

(VIII)

in which X, Y or Z represent, independently of one another, a carbon atom, a nitrogen atom, a sulfur atom or an oxygen atom, it being understood that when X, Y or Z are a nitrogen, sulfur or oxygen atom, the presence of a radical R14, R15 or R16 depends on respect of the valence of said nitrogen atom or sulfur atom, preserving the aromaticity of the ring,
R12 represents a hydrogen atom, a halogen or an alkyl group, R13 to R16 represent, independently of one another, a hydrogen atom, a halogen atom, a hydroxy group, a cyano group, a radical selected from the linear or branched alkyl groups, alkoxy, alkylsulfonyl, alkoxycarbonyl, carbamoyl, cycloalkyl, aryl groups, optionally substituted with one or more substituents, and the radical -NR7-(Y)n-R8,
R13 and R14, R14 and R15 or R15 and R16 being able to form an aliphatic or aromatic ring, with one or more heteroatoms selected from oxygen or nitrogen, optionally substituted with one or more substituents,
R5, R7 and R8 being defined in one of Claims 1 to 5.

**19.** Compound according to Claim 18, selected from the following compounds, and physiologically acceptable salts thereof:

Ethyl 4-oxo-8-phenyl-4,5-dihydro-3*H*-pyrrolo[2,3-*c*]quinoline-1-carboxylate
Ethyl 8-(2-chlorophenyl)-4-oxo-4,5-dihydro-3*H*-pyrrolo[2,3-*c*]quinoline-1-carboxylate
Ethyl 8-(2-*tert*-butoxycarbonylaminophenyl)-4-oxo-4,5-dihydro-3*H*-pyrrolo[2,3-*c*]quinoline-1-carboxylate
Ethyl 8-(2-aminophenyl)-4-oxo-4,5-dihydro-3*H*-pyrrolo[2,3-*c*]quinoline-1-carboxylate
Ethyl 8-(2-methanesulfonylaminophenyl)-4-oxo-4,5-dihydro-3*H*-pyrrolo[2,3-*c*]quinoline-1-carboxylate
Ethyl 8-(3-*tert*-butoxycarbonylaminophenyl)-4-oxo-4,5-dihydro-3*H*-pyrrolo[2,3-*c*]quinoline-1-carboxylate
Ethyl 8-(3-aminophenyl)-4-oxo-4,5-dihydro-3*H*-pyrrolo[2,3-*c*]quinoline-1-carboxylate
Ethyl 8-(3-methanesulfonylaminophenyl)-4-oxo-4,5-dihydro-3*H*-pyrrolo[2,3-*c*]quinoline-1-carboxylate
Ethyl 8-(3-acetylaminophenyl)-4-oxo-4,5-dihydro-3*H*-pyrrolo[2,3-*c*]quinoline-1-carboxylate
Ethyl 8-(3-hydroxy-phenyl)-4-oxo-4,5-dihydro-3*H*-pyrrolo[2,3-*c*]quinoline-1-carboxylate
Ethyl 8-(3-methanesulfonyl-phenyl)-4-oxo-4,5-dihydro-3*H*-pyrrolo[2,3-*c*]quinoline-1-carboxylate
Ethyl 8-(3-cyano-phenyl)-4-oxo-4,5-dihydro-3*H*-pyrrolo[2,3-*c*]quinoline-1-carboxylate
Ethyl 8-(3-hydroxymethyl-phenyl)-4-oxo-4,5-dihydro-3*H*-pyrrolo[2,3-*c*]quinoline-1-carboxylate
Ethyl 8-(3-methylaminomethyl-phenyl)-4-oxo-4,5-dihydro-3*H*-pyrrolo[2,3-*c*]quinoline-1-carboxylate
Ethyl 8-(3-dimethylaminomethyl-phenyl)-4-oxo-4,5-dihydro-3*H*-pyrrolo[2,3-*c*]quinoline-1-carboxylate
Ethyl 8-(4-*tert*-butoxycarbonylaminophenyl)-4-oxo-4,5-dihydro-3*H*-pyrrolo[2,3-*c*]quinoline-1-carboxylate

Ethyl 8-(4-aminophenyl)-4-oxo-4,5-dihydro-3*H*-pyrrolo[2,3-*c*]quinoline-1-carboxylate
Ethyl 8-(4-methanesulfonyl-phenyl)-4-oxo-4,5-dihydro-3*H*-pyrrolo[2,3-*c*]quinoline-1-carboxylate
Ethyl 8-(4-methoxy-phenyl)-4-oxo-4,5-dihydro-3*H*-pyrrolo[2,3-*c*]quinoline-1-carboxylate
Ethyl 8-(4-cyano-phenyl)-4-oxo-4,5-dihydro-3*H*-pyrrolo[2,3-*c*]quinoline-1-carboxylate
Ethyl 8-(2,3-dihydro-benzo[1,4]dioxin-6-yl)-4-oxo-4,5-dihydro-3*H*-pyrrolo[2,3-*c*]quinoline-1-carboxylate
Ethyl 8-naphthalen-1-yl-4-oxo-4,5-dihydro-3*H*-pyrrolo[2,3-*c*]quinoline-1-carboxylate
Ethyl 4-oxo-8-quinolin-8-yl-4,5-dihydro-3*H*-pyrrolo[2,3-*c*]quinoline-1-carboxylate
Ethyl 8-(2-amino-5-methanesulfonyl-phenyl)-4-oxo-4,5-dihydro-3*H*-pyrrolo[2,3-*c*]quinoline-1-carboxylate
Ethyl 8-(1*H*-indol-5-yl)-4-oxo-4,5-dihydro-3*H*-pyrrolo[2,3-*c*]quinoline-1-carboxylate
Ethyl 8-(1-*tert*-butoxycarbonyl-1*H*-indol-5-yl)-4-oxo-4,5-dihydro-3*H*-pyrrolo[2,3-*c*]quinoline-1-carboxylate
Propyl 8-(3-methanesulfonyl-phenyl)-4-oxo-4,5-dihydro-3*H*-pyrrolo[2,3-*c*]quinoline-1-carboxylate
Isobutyl 8-(3-methanesulfonyl-phenyl)-4-oxo-4,5-dihydro-3*H*-pyrrolo[2,3-*c*]quinoline-1-carboxylate
Cyclopentyl 8-(3-methanesulfonyl-phenyl)-4-oxo-4,5-dihydro-3*H*-pyrrolo[2,3-*c*]quinoline-1-carboxylate
Ethyl 7-chloro-8-(3-methanesulfonyl-phenyl)-4-oxo-4,5-dihydro-3*H*-pyrrolo[2,3-*c*]quinoline-1-carboxylate
Ethyl 4-oxo-8-pyridin-3-yl-4,5-dihydro-3*H*-pyrrolo[2,3-*c*]quinoline-1-carboxylate
Ethyl 8-(6-chloro-pyridin-3-yl)-4-oxo-4,5-dihydro-3*H*-pyrrolo[2,3-*c*]quinoline-1-carboxylate
Ethyl 8-(6-methoxy-pyridin-3-yl)-4-oxo-4,5-dihydro-3*H*-pyrrolo[2,3-*c*]quinoline-1-carboxylate
Ethyl 8-(2-amino-pyrimidin-4-yl)-4-oxo-4,5-dihydro-3*H*-pyrrolo[2,3-*c*]quinoline-1-carboxylate
Ethyl 8-(2-dimethylamino-pyrimidin-4-yl)-4-oxo-4,5-dihydro-3*H*-pyrrolo[2,3-*c*]quinoline-1-carboxylate
Ethyl 8-(2-methylamino-pyrimidin-4-yl)-4-oxo-4,5-dihydro-3*H*-pyrrolo[2,3-*c*]quinoline-1-carboxylate formate
Ethyl 8-(2-ethylamino-pyrimidin-4-yl)-4-oxo-4,5-dihydro-3*H*-pyrrolo[2,3-*c*]quinoline-1-carboxylate
Ethyl 4-oxo-8-[2-(4-pyridin-2-yl-piperazin-1-yl)-pyrimidin-4-yl]-4,5-dihydro-3*H*-pyrrolo[2,3-*c*]quinoline-1-carboxylate
Ethyl 4-oxo-8-(2-phenylamino-pyrimidin-4-yl)-4,5-dihydro-3*H*-pyrrolo[2,3-*c*]quinoline-1-carboxylate
Ethyl 8-[2-(1*H*-benzoimidazol-2-ylamino)-pyrimidin-4-yl]-4-oxo-4,5-dihydro-3*H*-pyrrolo[2,3-*c*]quinoline-1-carboxylate
Ethyl 8-[2-(4-acetyl-piperazin-1-yl)-pyrimidin-4-yl]-4-oxo-4,5-dihydro-3*H*-pyrrolo[2,3-*c*]quinoline-1-carboxylate
Ethyl 8-{2-[(2-dimethylamino-ethyl)-methyl-amino]-pyrimidin-4-yl}-4-oxo-4,5-dihydro-3*H*-pyrrolo[2,3-*c*]quinoline-1-carboxylate formate.

**20.** Compound according to one of Claims 1 to 5, **characterized in that** it corresponds to general formula (IX):

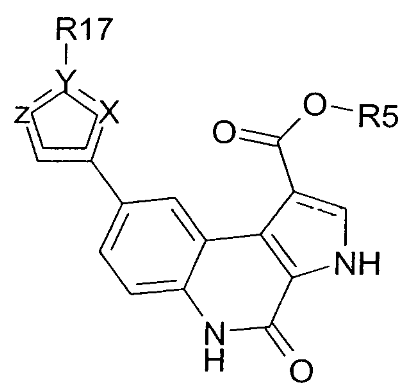

(IX)

in which X, Y or Z represent, independently of one another, a carbon atom, a nitrogen atom, a sulfur atom or an oxygen atom, it being understood that when X, Y or Z are a nitrogen, sulfur or oxygen atom, the presence of a radical R17 depends on respect of the valence of said nitrogen atom or sulfur atom, preserving the aromaticity of the ring,
R17 represents a hydrogen atom, an alkyl, cycloalkyl or aryl radical, optionally substituted with one or more substituents, or an aryl, -NR7-(Y)n-R8 or -NR7-NH-R8 radical,
R5, R7 and R8 being defined in one of Claims 1 to 5.

**21.** Compound according to Claim 20, selected from the following compounds, and physiologically acceptable salts thereof:

Ethyl 8-(2-amino-thiazol-4-yl)-4-oxo-4,5-dihydro-3*H*-pyrrolo[2,3-*c*]quinoline-1-carboxylate
Ethyl 8-[2-phenylamino-thiazol-4-yl]-4-oxo-4,5-dihydro-3*H*-pyrrolo[2,3-*c*]quinoline-1-carboxylate
Ethyl 8-[2-(2-chlorophenylamino)-thiazol-4-yl]-4-oxo-4,5-dihydro-3*H*-pyrrolo[2,3-*c*]quinoline-1-carboxylate
Ethyl 8-[2-(3,4-dichlorophenylamino)-thiazol-4-yl]-4-oxo-4,5-dihydro-3*H*-pyrrolo[2,3-*c*]quinoline-1-carboxylate
Ethyl 8-[2-(2,6-dichlorophenylamino)-thiazol-4-yl]-4-oxo-4,5-dihydro-3*H*-pyrrolo[2,3-*c*]quinoline-1-carboxylate
Ethyl 8-[2-(cyclopropanecarbonyl-amino)-thiazol-4-yl]-4-oxo-4,5-dihydro-3*H*-pyrrolo[2,3-*c*]quinoline-1-carboxylate
Ethyl 4-oxo-8-(2-phenylsulfanylmethyl-thiazol-4-yl)-4,5-dihydro-3*H*-pyrrolo[2,3-*c*]quinoline-1-carboxylate
Ethyl 4-oxo-8-(2-piperidin-4-yl-thiazol-4-yl)-4,5-dihydro-3*H*-pyrrolo[2,3-*c*]quinoline-1-carboxylate
Ethyl 8-(2-methyl-thiazol-4-yl)-4-oxo-4,5-dihydro-3*H*-pyrrolo[2,3-*c*]quinoline-1-carboxylate
Ethyl 4-oxo-8-(2-phenyl-thiazol-4-yl)-4,5-dihydro-3*H*-pyrrolo[2,3-*c*]quinoline-1-carboxylate
Ethyl 4-oxo-8-[2-(*N'*-phenyl-hydrazino)-thiazol-4-yl]-4,5-dihydro-3*H*-pyrrolo[2,3-*c*]quinoline-1-carboxylate
Ethyl 8-isoxazol-5-yl-4-oxo-4,5-dihydro-3*H*-pyrrolo[2,3-*c*]quinoline-1-carboxylate
Ethyl 4-oxo-8-(1*H*-pyrazol-3-yl)-4,5-dihydro-3*H*-pyrrolo[2,3-*c*]quinoline-1-carboxylate
Ethyl 8-(l-methyl-1*H*-pyrazol-3-yl)-4-oxo-4,5-dihydro-3*H*-pyrrolo[2,3-*c*]quinoline-1-carboxylate.

**22.** Pharmaceutical composition containing a suitable pharmaceutical vehicle and a compound selected from the compounds according to one of Claims 1 to 21 and the following compounds and the physiologically acceptable salts thereof:

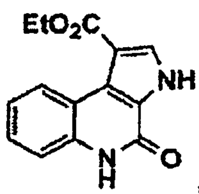 , 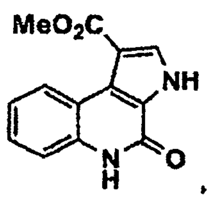 , 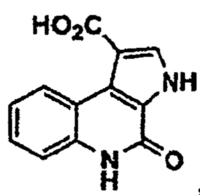 , 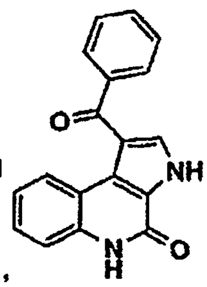 .

**23.** Compound selected from the compounds according to one of Claims 1 to 21 and the following compounds and the physiologically acceptable salts thereof, for use in therapy:

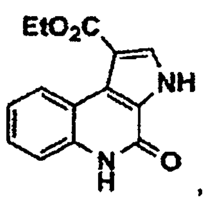 , 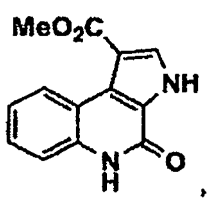 , 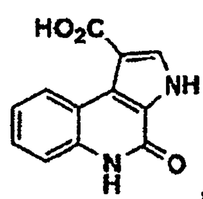 , 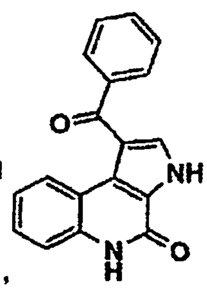 .

**24.** Compounds according to Claim 23 for use in the treatment of cancers, inflammatory diseases or disorders of the central nervous system.

**25.** Use according to either of Claims 23 and 24, in combination with at least one other active agent.

**26.** Use of a compound selected from the compounds according to one of Claims 1 to 21, and the following compounds and the physiologically acceptable salts thereof:

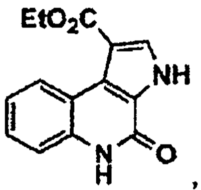, 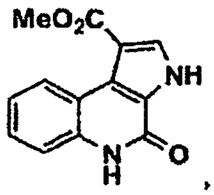, 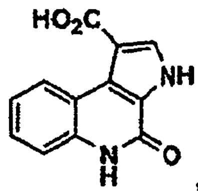, 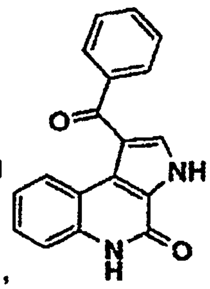

for the preparation of a medicinal product intended for the treatment of cancers, inflammatory diseases or disorders of the central nervous system.

**27.** Use according to Claim 26, in combination with at least one other active agent.

**28.** Nontherapeutic use, as a kinase inhibitor, of a compound selected from the compounds according to one of Claims 1 to 21 and the following compounds and the physiologically acceptable salts thereof:

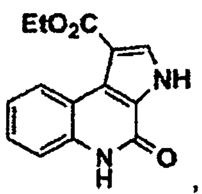, 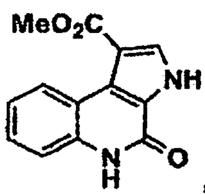, 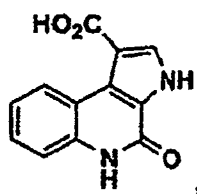, 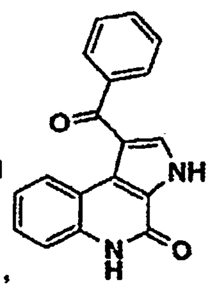

**29.** Product containing a compound selected from the compounds according to one of Claims 1 to 21 and the following compounds and the physiologically acceptable salts thereof:

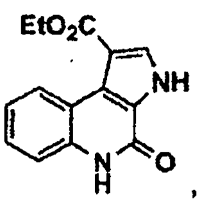, 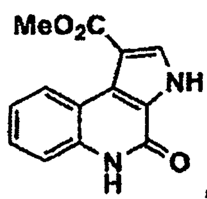, 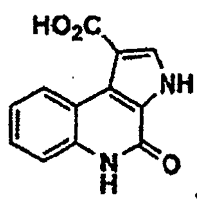, 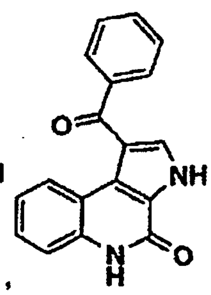

and another active agent as a combination product for therapeutic use, simultaneously, separately or spread over time.

**30.** Product according to Claim 29, for use in the treatment of cancers, inflammatory diseases or disorders of the central nervous system.

**Patentansprüche**

**1.** Verbindung der Formel I:

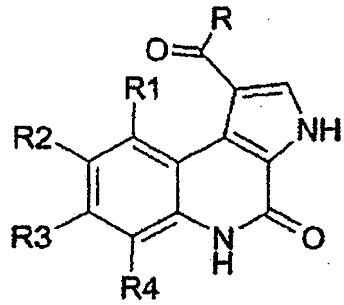

(I)

worin:

R für ein Wasserstoffatom oder eine unter Alkyl-, Alkenyl-, Alkinyl-, Cycloalkyl-, Aralkyl-, Aryl-, Heteroaryl-, -O-R5- oder -NR5-R6-Resten, die gegebenenfalls durch einen oder mehrere Substituenten substituiert sind, ausgewählte Gruppe steht,

R1 und R4 unabhängig für ein Wasserstoffatom, ein Halogen oder eine unter Nitro-, Alkyl-, Alkenyl-, Alkinyl-, Alkoxy- oder Cycloalkylresten, die gegebenenfalls durch einen oder mehrere Substituenten substituiert sind, ausgewählte Gruppe stehen,

R2 und R3 für ein Wasserstoffatom, ein Halogen oder eine unter Alkyl-, Alkenyl-, Alkinyl-, Alkoxy-, Aryl-, Heteroaryl-, Aryloxy- oder Aralkyloxyresten, die gegebenenfalls durch einen oder mehrere Substituenten substituiert sind, oder Hydroxy-, Cyano-, -Y-(NR7)n-R8-, -SOm-R8-, -NR7-(Y)n-R8-, -NO2- oder COOR9-Resten ausgewählte Gruppe stehen,

n = 0 oder 1

m = 0 oder 1

Y = -CO- oder -SO2-

wobei R2 und R3 nicht gleichzeitig in der gleichen Verbindung für die folgenden Gruppen stehen: Aryl, Heteroaryl, Aryloxy, Aralkyloxy, -Y-(NR7)n-R8, -SOm-R8, -NR7-(Y)n-R8, -N02, COOR9,

R5 für ein Wasserstoffatom oder einen Alkylrest, einen Alkenylrest mit mindestens 3 Kohlenstoffatomen, der nicht direkt an der Ungesättigtheit verzweigt ist, einen Alkinylrest mit mindestens 3 Kohlenstoffatomen, der nicht direkt an der Ungesättigtheit verzweigt ist, einen Cycloalkylrest, einen Cycloalkylalkylrest, einen Aralkylrest, einen Heteroaralkylrest oder einen stickstofffreien gesättigten Alkylheterocyclus, der gegebenenfalls durch einen oder mehrere Substituenten substituiert ist, steht,

R6 für ein Wasserstoffatom oder einen Alkylrest, einen Alkenylrest mit mindestens 3 Kohlenstoffatomen, der nicht direkt an der Ungesättigtheit verzweigt ist, einen Alkinylrest mit mindestens 3 Kohlenstoffatomen, der nicht direkt an der Ungesättigtheit verzweigt ist, einen Cycloalkylrest, einen Cycloalkylalkylrest, einen Arylrest, einen Heteroarylrest, einen Aralkylrest, einen Heteroaralkylrest oder einen stickstofffreien gesättigten Alkylheterocyclus, der gegebenenfalls durch einen oder mehrere Substituenten substituiert ist, steht,

R7 für ein Wasserstoffatom oder einen Alkylrest, einen Alkenylrest mit mindestens 3 Kohlenstoffatomen, der nicht direkt an der Ungesättigtheit verzweigt ist, einen Alkinylrest mit mindestens 3 Kohlenstoffatomen, der nicht direkt an der Ungesättigtheit verzweigt ist, einen Cycloalkylrest oder einen Cycloalkylalkylrest, der gegebenenfalls durch einen oder mehrere Substituenten substituiert ist, steht,

R8 für ein Wasserstoffatom oder eine unter Alkyl-, Alkenyl-, Alkinyl-, Alkoxy-, Cycloalkyl-, Cycloalkylalkyl-, Aralkyl-, Aryl-, Heteroaryl- oder Heteroaralkylresten, einem gesättigten Heterocyclus oder einem gesättigten Alkylheterocyclus, die gegebenenfalls durch einen oder mehrere Substituenten substituiert sind, ausgewählte Gruppe steht,

R9 für ein Wasserstoffatom oder eine unter Alkyl-, Alkenyl-, Alkinyl-, Cycloalkyl-, Cycloalkylalkyl-, Aralkyl-, Aryl- oder Heteroaralkylresten, einem gesättigten Heterocyclus oder einem gesättigten Alkylheterocyclus, die gegebenenfalls durch einen oder mehrere Substituenten substituiert sind, ausgewählte Gruppe steht,

wobei R7 und R8 gemeinsam mit dem Stickstoffatom, an das sie gebunden sind, einen Ring oder Doppelring mit 1 oder mehreren Heteroatomen bilden können, wobei der Heterocyclus gegebenenfalls durch eine oder mehrere Alkyl-, Alkenyl-, Alkinyl-, Amino-, NR10R11-, Hydroxy-, Alkoxy-, Carbamoyl-, Sulfamoyl-, Sulfonyl-, Sulfenyl-, Sulfanyl- oder Cyanogruppen oder einen Oxorest substituiert ist,

R10 für ein Wasserstoffatom oder einen Alkylrest, einen Alkenylrest mit mindestens 3 Kohlenstoffatomen, der nicht direkt an der Ungesättigtheit verzweigt ist, einen Alkinylrest mit mindestens 3 Kohlenstoffatomen, der nicht

direkt an der Ungesättigtheit verzweigt ist, einen Cycloalkylrest oder einen Cycloalkylalkylrest, der gegebenenfalls durch einen oder mehrere Substituenten substituiert ist, steht,
R11 unabhängig für ein Wasserstoffatom oder eine unter Alkyl-, Alkenyl-, Alkinyl-, Cycloalkyl-, Cycloalkylalkyl-, Aralkyl-, Aryl-, Heteroaryl- oder Heteroaralkylresten, einem gesättigten Heterocyclus oder einem gesättigten Alkylheterocyclus, Alkoxycarbonyl-, Carbamoyl- oder Sulfamoylresten, die gegebenenfalls durch einen oder mehrere Substituenten substituiert sind, ausgewählte Gruppe steht,
wobei R10 und R11 gemeinsam mit dem Stickstoffatom, an das sie gebunden sind, einen Cyclus mit 1 oder mehreren Heteroatomen bilden können, wobei der Heterocyclus gegebenenfalls substituiert ist,
und ihre physiologisch annehmbaren Salze,
mit Ausnahme der folgenden Verbindungen:

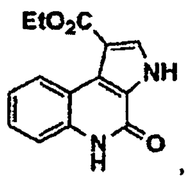 , 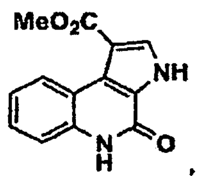 , 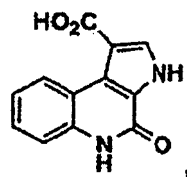 , 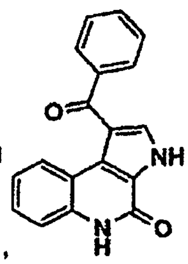

wobei
unter Alkyl lineare oder verzweigte C1-, C2-, C3-, C4-, C5- oder C6-Alkylreste zu verstehen sind,
wobei diese Definition auch für Alkylteile von Alkoxy-, Alkoxycarbonyl-, Carbonylamino-, Carbonyl-, Aralkyl- oder Alkoxycarbonylalkylresten gilt,
unter Alkenyl eine lineare oder verzweigte, ungesättigte, einwertige Kohlenwasserstoffkette mit mindestens einer Doppelbindung und 2 bis 6 Kohlenstoffatomen zu verstehen ist,
unter Alkinyl eine lineare oder verzweigte, ungesättigte, einwertige Kohlenwasserstoffkette mit mindestens einer Dreifachbindung und 2 bis 6 Kohlenstoffatomen zu verstehen ist,
unter Cycloalkyl C3-C7-Cycloalkylgruppen zu verstehen sind,
unter Aryl ein oder mehrere aromatische Ringe mit 5 bis 10 Kohlenstoffatomen, die verbunden oder anelliert sein können, zu verstehen sind,
unter Heteroaryl ein oder mehrere aromatische Ringe mit 5 bis 10 Atomen, die verbunden oder anelliert sein können und im Ring mindestens ein unter Stickstoff, Sauerstoff und Schwefel ausgewähltes Heteroatom enthalten, zu verstehen sind,
wobei die Substituenten unter Halogenen, Hydroxyl-, Amino- (primäre, sekundäre oder tertiäre Amin-), Nitrogruppen, C1-C4-Alkylresten, C1-C4-Perfluoralkyl-, Alkoxy-, Perfluoralkoxy-, Alkoxycarbonyl-, Cycloalkyl-, Carbamoyl-, Sulfamoyl-, Sulfonyl-, Sulfenyl-, Sulfanyl-, Carbonylamino-, Aryl-, Heteroaryl- oder Cyanoresten oder einem Oxorest ausgewählt sind.

2. Verbindung nach Anspruch 1, **dadurch gekennzeichnet, daß** sie der allgemeinen Formel I' entspricht:

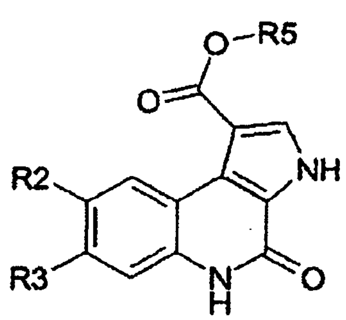

(I')

worin R2, R3 und R5 die in Anspruch 1 angegebene Bedeutung besitzen.

**3.** Verbindung nach einem der Ansprüche 1 oder 2,
**dadurch gekennzeichnet, daß**
R2 unter -S02-(NR7)n-R8- oder -NR7-(Y)n-R8-Resten ausgewählt ist.

**4.** Verbindung nach einem der Ansprüche 1 bis 3,
**dadurch gekennzeichnet, daß**

- R5 unter Methyl-, Ethyl-, Propyl-, Isopropyl-, Butyl-, Isobutyl-, Isopentyl-, Allyl-, Isoprenyl-, Propargyl-, Cyclopentyl-, Cyclohexyl-, Cyclopropylmethyl-, Cyclopentylmethyl-, Cyclohexylmethyl-, Tetrahydropyran-4-ylmethyl- oder Tetrahydrofuran-3-ylmethylgruppen ausgewählt ist und/oder
- R7 für Wasserstoff steht oder unter Methyl-, Ethyl-, Propyl-, Isopropyl-, Butyl-, Isobutyl-, Allyl-, Isoprenyl-, Propargyl-, Cyclopropyl-, Cyclopentyl-, Cyclohexyl-, Cyclopropylmethyl-, Cyclopentylmethyl-, Cyclohexylmethyl-, Tetrahydropyran-4-ylmethyl-, Tetrahydrofuran-3-ylmethyl- oder 2-Hydroxyethylgruppen ausgewählt ist und/oder
- R8 unter Methyl-, 2-Hydroxyethyl-, 2-Aminoethyl-, 2-Methylaminoethyl-, 2-Dimethylaminoethyl-, Cyanomethyl-, Carbamoylmethyl-, 2-Cyanoethyl-, Cyclohexyl-, Cycloheptyl-, Cyclopropylmethyl-, Cyclopentylmethyl-, Cyclohexylmethyl-, Piperidin-4-yl-, Piperidin-3-yl-, Piperidin-2-yl-, Piperidin-4-methyl-, Piperidin-3-methyl-, Piperidin-2-methyl-, Pyrrolidin-3-ylmethyl-, Pyrrolidin-2-ylmethyl-, Pyrrolidin-3-yl-, Azetidin-3-ylmethyl-, Tetrahydrothiopyran-4-yl-, Tetrahydropyran-4-yl-, Imidazol-4-ylmethyl-, Phenyl-, o-Tolyl-, m-Tolyl-, p-Tolyl-, 2-Fluorphenyl-, 3-Fluorphenyl-, 4-Fluorphenyl-, 2-Chlorphenyl-, 3-Chlorphenyl-, 4-Chlorphenyl-, 3-Hydroxyphenyl-, 2-Methoxyphenyl-, 3-Methoxyphenyl-, 4-Methoxyphenyl-, 4-Aminophenyl-, 4-Nitrophenyl-, 4-Fluor-2-methylphenyl-, 3-Fluor-2-methylphenyl-, 3,4-Difluorphenyl-, 3,5-Difluorphenyl-, 2,6-Dichlorphenyl-, Pyridyl-, Thiophen-, 5-Chlorthiophen- oder Benzylgruppen ausgewählt ist,
- wobei R7 und R8 mit dem Stickstoffatom, an das sie gebunden sind, einen 2,3-Dihydroindolyl-, Piperidinyl-, Piperazinyl-, Pyrrolidinyl- oder Morpholinylring bilden können.

**5.** Verbindung nach Anspruch 4, **dadurch gekennzeichnet, daß** die Substituenten unter Halogenen, Hydroxyl-, Amino- (primäre, sekundäre oder tertiäre Amin-), Nitrogruppen, C1-C4-Alkylresten, C1-C4-Perfluoralkyl-, Alkoxy-, Perfluoralkoxy-, Alkoxycarbonyl-, Carbamoyl-, Sulfamoyl-, Sulfonyl-, Sulfenyl-, Sulfanyl- oder Cyanoresten oder einem Oxo-, Carbonylamino-, Aryl-, Heteroaryl- oder Cycloalkylrest ausgewählt sind.

**6.** Verbindung nach einem der Ansprüche 1 bis 5, **dadurch gekennzeichnet, daß** sie der allgemeinen Formel (II) entspricht:

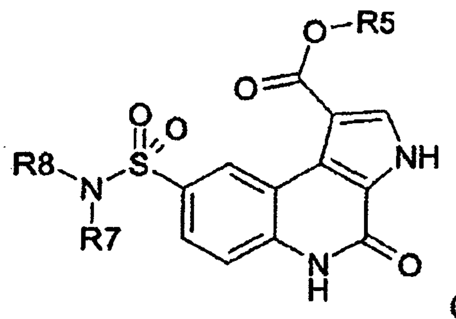

(II),

worin R5, R7 und R8 die in den Ansprüchen 1 bis 5 angegebene Bedeutung besitzen.

**7.** Verbindung nach Anspruch 6, ausgewählt unter den folgenden Verbindungen und ihren physiologisch annehmbaren Salzen:

4-Oxo-8-phenylsulfamoyl-4,5-dihydro-3*H*-pyrrolo-[2,3-*c*]chinolin-1-carbonsäureethylester
8-(Methylphenylsulfamoyl)-4-oxo-4,5-dihydro-3*H*-pyrrolo[2,3-*c*]chinolin-1-carbonsäureethylester
4-Oxo-8-o-tolylsulfamoyl-4,5-dihydro-3*H*-pyrrolo-[2,3-*c*]chinolin-1-carbonsäureethylester
4-Oxo-8-m-tolylsulfamoyl-4,5-dihydro-3*H*-pyrrolo-[2,3-*c*]chinolin-1-carbonsäureethylester
4-Oxo-8-*p*-tolylsulfamoyl-4,5-dihydro-3*H*-pyrrolo-[2,3-*c*]chinolin-1-carbonsäureethylester
8-(2-Methoxyphenylsulfamoyl)-4-oxo-4,5-dihydro-3*H*-pyrrolo[2,3-*c*]chinolin-1-carbonsäureethylester

8-(3-Methoxyphenylsulfamoyl)-4-oxo-4,5-dihydro-3*H*-pyrrolo[2,3-*c*]chinolin-1-carbonsäureethylester
8-(4-Methoxyphenylsulfamoyl)-4-oxo-4,5-dihydro-3*H*-pyrrolo[2,3-*c*]chinolin-1-carbonsäureethylester
8-(3-Chlorphenylsulfamoyl)-4-oxo-4,5-dihydro-3*H*-pyrrolo[2,3-*c*]chinolin-1-carbonsäureethylester
8-(4-Chlorphenylsulfamoyl)-4-oxo-4,5-dihydro-3*H*-pyrrolo[2,3-*c*]chinolin-1-carbonsäureethylester
8-(4-Fluorphenylsulfamoyl)-4-oxo-4,5-dihydro-3*H*-pyrrolo[2,3-*c*]chinolin-1-carbonsäureethylester
8-[(4-Fluorphenyl)methylsulfamoyl)-4-oxo-4,5-dihydro-3*H*-pyrrolo[2,3-*c*]chinolin-1-carbonsäureethylester
8-[(4-Fluorphenyl)methylsulfamoyl]-4-oxo-4,5-dihydro-3*H*-pyrrolo[2,3-*c*]chinolin-1-carbonsäure
8-[(4-Fluorphenyl)methylsulfamoyl]-4-oxo-4,5-dihydro-3*H*-pyrrolo[2,3-*c*]chinolin-1-carbonsäure-2-hydroxyethylester
8-[(4-Fluorphenyl)methylsulfamoyl]-4-oxo-4,5-dihydro-3*H*-pyrrolo[2,3-*c*]chinolin-1-carbonsäure-2-aminoethylester-hydrochlorid
8-[(4-Fluorphenyl)methylsulfamoyl]-4-oxo-4,5-dihydro-3*H*-pyrrolo[2,3-*c*]chinolin-1-carbonsäure-2-tert.-butoxycarbonylaminoethylester
8-[(4-Fluorphenyl)methylsulfamoyl]-4-oxo-4,5-dihydro-3*H*-pyrrolo[2,3-*c*]chinolin-1-carbonsäure-3-aminopropylester-hydrochlorid
8-[(4-Fluorphenyl)methylsulfamoyl]-4-oxo-4,5-dihydro-3*H*-pyrrolo[2,3-*c*]chinolin-1-carbonsäure-3-*tert*.-butoxycarbonylaminopropylester
8-(4-Ethoxycarbonyl-phenylsulfamoyl)-4-oxo-4,5-dihydro-3*H*-pyrrolo[2,3-*c*]chinolin-1-carbonsäureethylester
8-(3-Hydroxyphenylsulfamoyl)-4-oxo-4,5-dihydro-3*H*-pyrrolo[2,3-*c*]chinolin-1-carbonsäureethylester
8-(3-Fluor-2-methyl-phenylsulfamoyl)-4-oxo-4,5-dihydro-3*H*-pyrrolo[2,3-*c*]chinolin-1-carbonsäureethylester
8-(4-Fluor-2-methylphenylsulfamoyl)-4-oxo-4,5-dihydro-3*H*-pyrrolo[2,3-*c*]chinolin-1-carbonsäureethylester
8-(3,4-Difluorphenylsulfamoyl)-4-oxo-4,5-dihydro-3*H*-pyrrolo[2,3-*c*]chinolin-1-carbonsäureethylester
8-(3,5-Difluorphenylsulfamoyl)-4-oxo-4,5-dihydro-3*H*-pyrrolo[2,3-*c*]chinolin-1-carbonsäureethylester
8-(4-Aminophenylsulfamoyl)-4-oxo-4,5-dihydro-3*H*-pyrrolo[2,3-*c*]chinolin-1-carbonsäureethylester
8-(2,3-Dihydro-indol-1-sulfonyl)-4-oxo-4,5-dihydro-3*H*-pyrrolo[2,3-*c*]chinolin-1-carbonsäureethylester
8-(4-Morpholin-4-yl-phenylsulfamoyl)-4-oxo-4,5-dihydro-3*H*-pyrrolo[2,3-*c*]chinolin-1-carbonsäureethylester
4-Oxo-8-(pyridin-3-ylsulfamoyl)-4,5-dihydro-3*H*-pyrrolo[2,3-*c*]chinolin-1-carbonsäureethylester
8-(Benzylmethylsulfamoyl)-4-oxo-4,5-dihydro-3*H*-pyrrolo[2,3-*c*]chinolin-1-carbonsäureethylester
8-(Methylphenethylsulfamoyl)-4-oxo-4,5-dihydro-3*H*-pyrrolo[2,3-*c*]chinolin-1-carbonsäureethylester
8-Dimethylsulfamoyl-4-oxo-4,5-dihydro-3*H*-pyrrolo-[2,3-*c*]chinolin-1-carbonsäureethylester
*N*-Propyl-8-dimethylsulfamoyl-4-oxo-4,5-dihydro-3*H*-pyrrolo[2,3-*c*]chinolin-1-carboxamid
*N*-Methyl-*N*-propyl-8-dimethylsulfamoyl-4-oxo-4,5-dihydro-3*H*-pyrrolo[2,3-*c*]chinolin-1-carboxamid
8-Methylsulfamoyl-4-oxo-4,5-dihydro-3*H*-pyrrolo-[2,3-*c*]chinolin-1-carbonsäureethylester
8-(Morpholin-4-sulfonyl)-4-oxo-4,5-dihydro-3*H*-pyrrolo[2,3-*c*]chinolin-1-carbonsäureethylester
8-(2-Ethoxycarbonylethylsulfamoyl)-4-oxo-4,5-dihydro-3*H*-pyrrolo[2,3-*c*]chinolin-1-carbonsäureethylester
8-Cyclohexylsulfamoyl-4-oxo-4,5-dihydro-3*H*-pyrrolo[2,3-*c*]chinolin-1-carbonsäuremethylester
8-Cyclohexylsulfamoyl-4-oxo-4,5-dihydro-3*H*-pyrrolo[2,3-*c*]chinolin-1-carbonsäureethylester
8-Cycloheptylsulfamoyl-4-oxo-4,5-dihydro-3*H*-pyrrolo[2,3-*c*]chinolin-1-carbonsäureethylester
8-(3-Dimethylaminopropylsulfamoyl)-4-oxo-4,5-dihydro-3*H*-pyrrolo[2,3-*c*]chinolin-1-carbonsäureethylester
8-(4-Amino-butylsulfamoyl)-4-oxo-4,5-dihydro-3*H*-pyrrolo[2,3-*c*]chinolin-1-carbonsäureethylester
8-[(3-Chlorphenyl)-methyl-sulfamoyl)-4-oxo-4,5-dihydro-3*H*-pyrrolo[2,3-*c*]chinolin-1-carbonsäureethylester
4-Oxo-8-(piperidin-4-ylsulfamoyl)-4,5-dihydro-3*H*-pyrrolo[2,3-*c*]chinolin-1-carbonsäureethylester
4-Oxo-8-(piperidin-3-ylsulfamoyl)-4,5-dihydro-3*H*-pyrrolo[2,3-*c*]chinolin-1-carbonsäuremethylester
4-Oxo-8-(piperidin-4-ylsulfamoyl)-4,5-dihydro-3*H*-pyrrolo[2,3-*c*]chinolin-1-carbonsäuremethylester
4-Oxo-8-(piperidin-4-ylsulfamoyl)-4,5-dihydro-3*H*-pyrrolo[2,3-*c*]chinolin-1-carbonsäurepropylester
4-Oxo-8-(piperidin-4-ylsulfamoyl)-4,5-dihydro-3*H*-pyrrolo[2,3-*c*]chinolin-1-carbonsäurebutylester
4-Oxo-8-(piperidin-4-ylsulfamoyl)-4,5-dihydro-3*H*-pyrrolo[2,3-*c*]chinolin-1-carbonsäurepentylester
4-Oxo-8-(piperidin-4-ylsulfamoyl)-4,5-dihydro-3*H*-pyrrolo[2,3-*c*]chinolin-1-carbonsäuremethylcyclohexylester
4-Oxo-8-(piperidin-4-ylsulfamoyl)-4,5-dihydro-3*H*-pyrrolo[2,3-*c*]chinolin-1-carbonsäuremethyltetrahydrofuran-3-ylester
8-(1-Methylpiperidin-4-ylsulfamoyl)-4-oxo-4,5-dihydro-3*H*-pyrrolo[2,3-*c*]chinolin-1-carbonsäurecyclopentylester
8-(Methyl-piperidin-4-yl-sulfamoyl)-4-oxo-4,5-dihydro-3*H*-pyrrolo[2,3-*c*]chinolin-1-carbonsäurepropylester
4-Oxo-8-[(piperidin-4-ylmethyl)-sulfamoyl]-4,5-dihydro-3*H*-pyrrolo[2,3-*c*]chinolin-1-carbonsäureethylester
8-(8-Azabicyclo[3.2.1]oct-3-ylsulfamoyl)-4-oxo-4,5-dihydro-3*H*-pyrrolo[2,3-*c*]chinolin-1-carbonsäureethylester
8-Cyclopentylsulfamoyl-4-oxo-4,5-dihydro-3*H*-pyrrolo[2,3-*c*]chinolin-1-carbonsäuremethylester
8-(Hexahydropyrrolo[3,4-*c*]pyrrol-2-sulfonyl)-4-oxo-4,5-dihydro-3*H*-pyrrolo[2,3-*c*]chinolin-1-carbonsäureethylester

8-(1-Azabicyclo[2.2.2]oct-3-ylsulfamoyl)-4-oxo-4,5-dihydro-3*H*-pyrrolo[2,3-*c*]chinolin-1-carbonsäureethylester
8-(4-Methylpiperazin-1-sulfonyl)-4-oxo-4,5-dihydro-3*H*-pyrrolo[2,3-*c*]chinolin-1-carbonsäureethylester
4-Oxo-8-sulfamoyl-4,5-dihydro-3*H*-pyrrolo[2,3-*c*]-chinolin-1-carbonsäureethylester
4-Oxo-8-[(pyrrolidin-3-methyl)sulfamoyl]-4,5-dihydro-3*H*-pyrrolo[2,3-*c*]chinolin-1-carbonsäureethylester
4-Oxo-8-(4-hydroxy-phenylsulfamoyl)-4,5-dihydro-3*H*-pyrrolo[2,3-*c*]chinolin-1-carbonsäureethylester
8-Cyclopropylsulfamoyl-4-oxo-4,5-dihydro-3*H*-pyrrolo[2,3-*c*]chinolin-1-carbonsäureethylester
8-Cyclopropylsulfamoyl-4-oxo-4,5-dihydro-3*H*-pyrrolo[2,3-*c*]chinolin-1-carbonsäurepropylester
8-(Cyclopropylmethylsulfamoyl)-4-oxo-4,5-dihydro-3*H*-pyrrolo[2,3-*c*]chinolin-1-carbonsäurepropylester
8-[(2-Cyanoethyl)methylsulfamoyl]-4-oxo-4,5-dihydro-3*H*-pyrrolo[2,3-*c*]chinolin-1-carbonsäurepropylester
8-[(2-Cyanoethyl)cyclopropylsulfamoyl]-4-oxo-4,5-dihydro-3*H*-pyrrolo[2,3-*c*]chinolin-1-carbonsäureethylester
8-(Cyanomethylmethylsulfamoyl)-4-oxo-4,5-dihydro-3*H*-pyrrolo[2,3-*c*]chinolin-1-carbonsäurepropylester
8-([1,3]Dioxolan-2-ylmethylmethylsulfamoyl)-4-oxo-4,5-dihydro-3*H*-pyrrolo[2,3-*c*]chinolin-1-carbonsäurepropylester
8-[(1-Benzhydrylazetidin-3-yl)methylsulfamoyl]-4-oxo-4,5-dihydro-3*H*-pyrrolo[2,3-*c*]chinolin-1-carbonsäurepropylester
8-[Methyl-(tetrahydrothiopyran-4-yl)-sulfamoyl]-4-oxo-4,5-dihydro-3*H*-pyrrolo[2,3-*c*]chinolin-1-carbonsäurepropylester
8-[(1,1-Dioxohexahydro-1H-thiopyran-4-yl)-methyl-sulfamoyl]-4-oxo-4,5-dihydro-3*H*-pyrrolo[2,3-*c*]-chinolin-1-carbonsäurepropylester
8-[Methyl(tetrahydropyran-4-yl)sulfamoyl]-4-oxo-4,5-dihydro-3*H*-pyrrolo[2,3-*c*]chinolin-1-carbonsäurepropylester
8-(Methylprop-2-inylsulfamoyl)-4-oxo-4,5-dihydro-3*H*-pyrrolo[2,3-*c*]chinolin-1-carbonsäurepropylester
8-[Methyl-(2-methylsulfanylethyl)-sulfamoyl]-4-oxo-4,5-dihydro-3*H*-pyrrolo[2,3-*c*]chinolin-1-carbonsäurepropylester
8-[(2-Dimethylaminoethyl)methylsulfamoyl]-4-oxo-4,5-dihydro-3*H*-pyrrolo[2,3-*c*]chinolin-1-carbonsäurepropylester
8-[Methyl(2-pyrrolidin-1-ylethyl)sulfamoyl]-4-oxo-4,5-dihydro-3*H*-pyrrolo[2,3-*c*]chinolin-1-carbonsäurepropylester
8-[(2-Diethylaminoethyl)methylsulfamoyl]-4-oxo-4,5-dihydro-3*H*-pyrrolo[2,3-*c*]chinolin-1-carbonsäurepropylester
8-[Methyl-(2-methylaminoethyl)sulfamoyl]-4-oxo-4,5-dihydro-3*H*-pyrrolo[2,3-*c*]chinolin-1-carbonsäurepropylester-hydrochlorid
8-{[2-(*tert*.-Butoxycarbonylmethylamino)ethyl]-methylsulfamoyl}-4-oxo-4,5-dihydro-3*H*-pyrrolo[2,3-*c*]chinolin-1-carbonsäurepropylester
8-[Cyclopropyl(2-methyl-2-methylaminopropyl)-sulfamoyl]-4-oxo-4,5-dihydro-3*H*-pyrrolo[2,3-*c*]-chinolin-1-carbonsäurepropylester-hydrochlorid
8-[Cyclopropyl(2-isopropylaminoethyl)sulfamoyl]-4-oxo-4,5-dihydro-3*H*-pyrrolo[2,3-*c*]chinolin-1-carbonsäurepropylester-hydrochlorid
8-[Cyclopropyl(1*H*-imidazol-4-ylmethyl)-sulfamoyl]-4-oxo-4,5-dihydro-3*H*-pyrrolo[2,3-*c*]chinolin-1-carbonsäurepropylester-hydrochlorid
(S)-8-(Cyclopropylpyrrolidin-2-ylmethylsulfamoyl)-4-oxo-4,5-dihydro-3*H*-pyrrolo[2,3-*c*]chinolin-1-carbonsäurepropylester-hydrochlorid
(S)-8-[Cyclopropyl-(1-methylpyrrolidin-2-ylmethyl)sulfamoyl]-4-oxo-4,5-dihydro-3*H*-pyrrolo-[2,3-*c*]chinolin-1-carbonsäurepropylester
(R)-8-[Cyclopropyl(1-methylpyrrolidin-2-ylmethyl)-sulfamoyl]-4-oxo-4,5-dihydro-3*H*-pyrrolo[2,3-*c*]-chinolin-1-carbonsäurepropylester
(R)-8-[(1-*tert*.-Butoxycarbonylpyrrolidin-2-ylmethyl)cyclopropylsulfamoyl]-4-oxo-4,5-dihydro-3*H*-pyrrolo[2,3-*c*]chinolin-1-carbonsäurepropylester
(R)-8-(Cyclopropylpyrrolidin-2-ylmethylsulfamoyl)-4-oxo-4,5-dihydro-3*H*-pyrrolo[2,3-*c*]chinolin-1-carbonsäurepropylester-hydrochlorid
(S)-8-(Isopropylpyrrolidin-2-ylmethylsulfamoyl)-4-oxo-4,5-dihydro-3*H*-pyrrolo[2,3-*c*]chinolin-1-carbonsäurepropylester-hydrochlorid
(S)-4-Oxo-8-(prop-2-inylpyrrolidin-2-ylmethylsulfamoyl)-4,5-dihydro-3*H*-pyrrolo[2,3-*c*]chinolin-1-carbonsäurepropylester-hydrochlorid
(S)-8-[(1-Methylpyrrolidin-2-ylmethyl)prop-2-inylsulfamoyl]-4-oxo-4,5-dihydro-3*H*-pyrrolo[2,3-*c*]-chinolin-1-carbonsäurepropylester
(S)-8-[(2-*tert*.-Butoxycarbonylaminopropyl)cyclopropylsulfamoyl]-4-oxo-4,5-dihydro-3*H*-pyrrolo[2,3-*c*]chinolin-1-carbonsäurepropylester

(S)-8-[(2-Aminopropyl)cyclopropylsulfamoyl]-4-oxo-4,5-dihydro-3*H*-pyrrolo[2,3-*c*]chinolin-1-carbonsäurepropylester-hydrochlorid
(R)-8-[(2-*tert*.-Butoxycarbonylaminopropyl)cyclopropylsulfamoyl]-4-oxo-4,5-dihydro-3*H*-pyrrolo[2,3-*c*]chinolin-1-carbonsäurepropylester
(R)-8-[(2-Aminopropyl)cyclopropylsulfamoyl]-4-oxo-4,5-dihydro-3*H*-pyrrolo[2,3-*c*]chinolin-1-carbonsäurepropylester-hydrochlorid
8-[(2-Methylaminoethyl)prop-2-inylsulfamoyl]-4-oxo-4,5-dihydro-3*H*-pyrrolo[2,3-*c*]chinolin-1-carbonsäurepropylester-hydrochlorid
8-{[2-(*tert*.-Butoxycarbonylmethylamino)ethyl]prop-2-inylsulfamoyl}-4-oxo-4,5-dihydro-3*H*-pyrrolo[2,3-*c*]chinolin-1-carbonsäurepropylester
8-[(2-Isopropylaminoethyl)prop-2-inylsulfamoyl]-4-oxo-4,5-dihydro-3*H*-pyrrolo[2,3-*c*]chinolin-1-carbonsäurepropylester-hydrochlorid
8-{[2-(*tert*.-Butoxycarbonylisopropylamino)-ethyl]-prop-2-inylsulfamoyl}-4-oxo-4,5-dihydro-3*H*-pyrrolo[2,3-*c*]chinolin-1-carbonsäurepropylester
8-(Ethylpyrrolidin-3-ylsulfamoyl)-4-oxo-4,5-dihydro-3*H*-pyrrolo[2,3-*c*]chinolin-1-carbonsäurepropylester-hydrochlorid
8-[Ethyl-(1-methylpyrrolidin-3-yl)sulfamoyl]-4-oxo-4,5-dihydro-3*H*-pyrrolo[2,3-*c*]chinolin-1-carbonsäurepropylester
4-Oxo-8-(pyrrolidin-1-sulfonyl)-4,5-dihydro-3*H*-pyrrolo[2,3-*c*]chinolin-1-carbonsäureethylester
4-Oxo-8-(2-pyrrolidin-1-ylmethylpyrrolidin-1-sulfonyl)-4,5-dihydro-3*H*-pyrrolo[2,3-*c*]chinolin-1-carbonsäureethylester
(S)-8-(3-*tert*.-Butoxycarbonylaminopyrrolidin-1-sulfonyl)-4-oxo-4,5-dihydro-3H-pyrrolo[2,3-c]-chinolin-1-carbonsäurepropylester
(S)-8-(3-*tert*.-Butoxycarbonylaminopyrrolidin-1-sulfonyl)-4-oxo-4,5-dihydro-3*H*-pyrrolo[2,3-*c*]-chinolin-1-carbonsäure
(S)-8-(3-Aminocyclopentansulfonyl)-4-oxo-4,5-dihydro-3*H*-pyrrolo[2,3-*c*]chinolin-1-carbonsäurepropylester-hydrochlorid
(R)-8-(3-Aminocyclopentansulfonyl)-4-oxo-4,5-dihydro-3*H*-pyrrolo[2,3-*c*]chinolin-1-carbonsäurepropylester-hydrochlorid
8-(3-Methylaminopyrrolidin-1-sulfonyl)-4-oxo-4,5-dihydro-3*H*-pyrrolo[2,3-*c*]chinolin-1-carbonsäurepropylester-hydrochlorid
8-[3-(*tert*.-Butoxycarbonylmethylamino)pyrrolidin-1-sulfonyl]-4-oxo-4,5-dihydro-3*H*-pyrrolo[2,3-*c*]-chinolin-1-carbonsäurepropylester
8-[3-(*tert*.-Butoxycarbonylmethylamino)pyrrolidin-1-sulfonyl]-4-oxo-4,5-dihydro-3*H*-pyrrolo[2,3-*c*]-chinolin-1-carbonsäure
(S)-8-(3-Dimethylaminocyclopentansulfonyl)-4-oxo-4,5-dihydro-3*H*-pyrrolo[2,3-*c*]chinolin-1-carbonsäurepropylester
(R)-8-(3-Dimethylaminocyclopentansulfonyl)-4-oxo-4,5-dihydro-3*H*-pyrrolo[2,3-*c*]chinolin-1-carbonsäurepropylester
8-[(2-Hydroxyethyl)isopropylsulfamoyl]-4-oxo-4,5-dihydro-3*H*-pyrrolo[2,3-*c*]chinolin-1-carbonsäurepropylester
8-[Cyclopropyl(2-hydroxyethyl)sulfamoyl]-4-oxo-4,5-dihydro-3*H*-pyrrolo[2,3-*c*]chinolin-1-carbonsäurepropylester
8-[(2-Hydroxyethyl)prop-2-inylsulfamoyl]-4-oxo-4,5-dihydro-3*H*-pyrrolo[2,3-*c*]chinolin-1-carbonsäurepropylester
8-[Bis(2-hydroxyethyl)sulfamoyl]-4-oxo-4,5-dihydro-3*H*-pyrrolo[2,3-*c*]chinolin-1-carbonsäurepropylester
8-(Carbamoylmethylmethylsulfamoyl)-4-oxo-4,5-dihydro-3*H*-pyrrolo[2,3-*c*]chinolin-1-carbonsäurepropylester
8-(Carbamoylmethylmethylsulfamoyl)-4-oxo-4,5-dihydro-3*H*-pyrrolo[2,3-*c*]chinolin-1-carbonsäure
8-(3-Carbamoylpiperidin-1-sulfonyl)-4-oxo-4,5-dihydro-3*H*-pyrrolo[2,3-*c*]chinolin-1-carbonsäurepropylester
8-(3-Carbamoylpiperidin-1-sulfonyl)-4-oxo-4,5-dihydro-3*H*-pyrrolo[2,3-*c*]chinolin-1-carbonsäure
8-[(2-Benzyloxyethyl)cyclopropylsulfamoyl]-4-oxo-4,5-dihydro-3*H*-pyrrolo[2,3-*c*]chinolin-1-carbonsäurepropylester
8-(Carboxymethylmethylsulfamoyl)-4-oxo-4,5-dihydro-3*H*-pyrrolo[2,3-*c*]chinolin-1-carbonsäurepropylester
8-(*tert*.-Butoxycarbonylmethylmethylsulfamoyl)-4-oxo-4,5-dihydro-3*H*-pyrrolo[2,3-*c*]chinolin-1-carbonsäurepropylester
8-[(2-Dimethylaminoethyl)methylsulfamoyl]-7-fluor-4-oxo-4,5-dihydro-3*H*-pyrrolo[2,3-*c*]chinolin-1-carbonsäurepropylester
7-Fluor-8-[(2-hydroxyethyl)isopropylsulfamoyl]-4-oxo-4,5-dihydro-3*H*-pyrrolo[2,3-*c*]chinolin-1-carbonsäurepropylester

8-Dimethylsulfamoyl-7-fluor-4-oxo-4,5-dihydro-3*H*-pyrrolo[2,3-*c*]chinolin-1-carbonsäurepropylester
8-(Cyclopropylmethylsulfamoyl)-7-fluor-4-oxo-4,5-dihydro-3*H*-pyrrolo[2,3-*c*]chinolin-1-carbonsäurepropylester
8-(Cyclopropylmethylsulfamoyl)-7-methoxy-4-oxo-4,5-dihydro-3*H*-pyrrolo[2,3-*c*]chinolin-1-carbonsäurepropylester
7-Chlor-8-dimethylsulfamoyl-4-oxo-4,5-dihydro-3*H*-pyrrolo[2,3-*c*]chinolin-1-carbonsäurepropylester
(S)-8-(3-Aminocyclopentansulfonyl)-7-chlor-4-oxo-4,5-dihydro-3*H*-pyrrolo[2,3-*c*]chinolin-1-carbonsäurepropylester-hydrochlorid
4-Oxo-8-(piperidin-1-ylsulfamoyl)-4,5-dihydro-3*H*-pyrrolo[2,3-*c*]chinolin-1-carbonsäuremethylester
8-(Hexahydrocyclopenta[c]pyrrol-2-ylsulfamoyl)-4-oxo-4,5-dihydro-3*H*-pyrrolo[2,3-*c*]chinolin-1-carbonsäuremethylester
8-(4-Methylpiperazin-1-ylsulfamoyl)-4-oxo-4,5-dihydro-3*H*-pyrrolo[2,3-*c*]chinolin-1-carbonsäuremethylester.

**8.** Verbindung nach einem der Ansprüche 1 bis 5, **dadurch gekennzeichnet, daß** sie der allgemeinen Formel (III) entspricht:

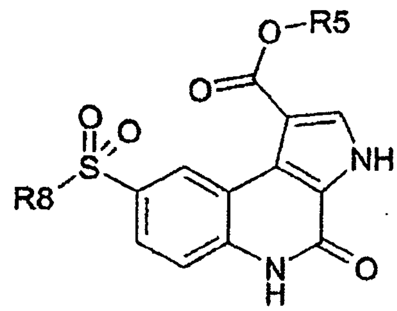

(III),

worin R8 die in einem der Ansprüche 1 bis 5 angegebene Bedeutung besitzt.

**9.** Verbindung nach Anspruch 8, ausgewählt unter den folgenden Verbindungen und ihren physiologisch annehmbaren Salzen:

4-Oxo-8-phenylmethansulfonyl-4,5-dihydro-3*H*-pyrrolo[2,3-*c*]chinolin-1-carbonsäureethylester
8-(2,6-Dichlorphenylmethansulfonyl)-4-oxo-4,5-dihydro-3*H*-pyrrolo[2,3-*c*]chinolin-1-carbonsäureethylester
8-(2,6-Dichlorphenylmethansulfonyl)-4-oxo-4,5-dihydro-3*H*-pyrrolo[2,3-*c*]chinolin-1-carbonsäure
4-Oxo-8-(pyridin-4-ylmethansulfonyl)-4,5-dihydro-3*H*-pyrrolo[2,3-*c*]chinolin-1-carbonsäureethylester
4-Oxo-8-(pyridin-2-ylmethansulfonyl)-4,5-dihydro-3*H*-pyrrolo[2,3-*c*]chinolin-1-carbonsäureethylester
8-Cyclohexansulfonyl-4-oxo-4,5-dihydro-3*H*-pyrrolo-[2,3-*c*]chinolin-1-carbonsäureethylester
4-Oxo-8-phenylmethansulfinyl-4,5-dihydro-3*H*-pyrrolo[2,3-*c*]chinolin-1-carbonsäureethylester.

**10.** Verbindung nach einem der Ansprüche 1 bis 5, **dadurch gekennzeichnet, daß** sie der allgemeinen Formel (IV) entspricht:

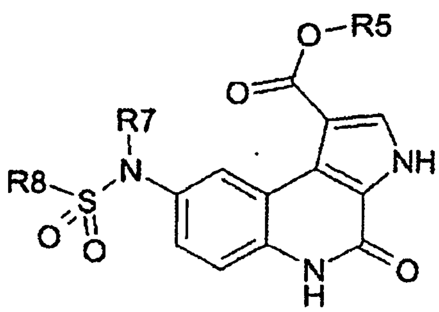

(IV),

worin R7 und R8 die in einem der Ansprüche 1 bis 5 angegebene Bedeutung besitzen.

**11.** Verbindung nach Anspruch 10, ausgewählt unter den folgenden Verbindungen und ihren physiologisch annehmbaren Salzen:

8-Benzolsulfonylamino-4-oxo-4,5-dihydro-3*H*-pyrrolo[2,3-*c*]chinolin-1-carbonsäureethylester
8-Methansulfonylamino-4-oxo-4,5-dihydro-3*H*-pyrrolo[2,3-*c*]chinolin-1-carbonsäureethylester
4-Oxo-8-phenylmethansulfonylamino-4,5-dihydro-3*H*-pyrrolo[2,3-*c*]chinolin-1-carbonsäureethylester
8-(4-Chlorbenzolsulfonylamino)-4-oxo-4,5-dihydro-3*H*-pyrrolo[2,3-*c*]chinolin-1-carbonsäureethylester
8-(3-Chlorbenzolsulfonylamino)-4-oxo-4,5-dihydro-3*H*-pyrrolo[2,3-*c*]chinolin-1-carbonsäureethylester
8-(2-Chlorbenzolsulfonylamino)-4-oxo-4,5-dihydro-3*H*-pyrrolo[2,3-*c*]chinolin-1-carbonsäureethylester
8-(4-Fluorbenzolsulfonylamino)-4-oxo-4,5-dihydro-3*H*-pyrrolo[2,3-*c*]chinolin-1-carbonsäureethylester
8-(3-Fluorbenzolsulfonylamino)-4-oxo-4,5-dihydro-3*H*-pyrrolo[2,3-*c*]chinolin-1-carbonsäureethylester
8-(2-Fluorbenzolsulfonylamino)-4-oxo-4,5-dihydro-3*H*-pyrrolo[2,3-*c*]chinolin-1-carbonsäureethylester
8-(4-Brombenzolsulfonylamino)-4-oxo-4,5-dihydro-3*H*-pyrrolo[2,3-*c*]chinolin-1-carbonsäureethylester
4-Oxo-8-(4-toluolsulfonylamino)-4,5-dihydro-3*H*-pyrrolo[2,3-*c*]chinolin-1-carbonsäureethylester
4-Oxo-8-(3-toluolsulfonylamino)-4,5-dihydro-3*H*-pyrrolo[2,3-*c*]chinolin-1-carbonsäureethylester
8-(4-*t*ert.-Butylbenzolsulfonylamino)-4-oxo-4,5-dihydro-3*H*-pyrrolo[2,3-*c*]chinolin-1-carbonsäureethylester
8-(4-Methoxybenzolsulfonylamino)-4-oxo-4,5-dihydro-3*H*-pyrrolo[2,3-*c*]chinolin-1-carbonsäureethylester
8-(3-Methoxybenzolsulfonylamino)-4-oxo-4,5-dihydro-3*H*-pyrrolo[2,3-*c*]chinolin-1-carbonsäureethylester
8-(4-Acetylaminobenzolsulfonylamino)-4-oxo-4,5-dihydro-3*H*-pyrrolo[2,3-*c*]chinolin-1-carbonsäureethylester
8-(4-Nitrobenzolsulfonylamino)-4-oxo-4,5-dihydro-3*H*-pyrrolo[2,3-*c*]chinolin-1-carbonsäureethylester
8-(Naphthalin-2-sulfonylamino)-4-oxo-4,5-dihydro-3*H*-pyrrolo[2,3-*c*]chinolin-1-carbonsäureethylester
8-(4-Acetylbenzolsulfonylamino)-4-oxo-4,5-dihydro-3*H*-pyrrolo[2,3-*c*]chinolin-1-carbonsäureethylester
4-Oxo-8-(thiophen-2-sulfonylamino)-4,5-dihydro-3*H*-pyrrolo[2,3-*c*]chinolin-1-carbonsäureethylester
8-(5-Chlorthiophen-2-sulfonylamino)-4-oxo-4,5-dihydro-3*H*-pyrrolo[2,3-*c*]chinolin-1-carbonsäureethylester
8-(Benzolsulfonylmethylamino)-4-oxo-4,5-dihydro-3*H*-pyrrolo[2,3-*c*]chinolin-1-carbonsäureethylester
8-(Methansulfonylmethylamino)-4-oxo-4,5-dihydro-3*H*-pyrrolo[2,3-c]chinolincarbonsäureethylester.

**12.** Verbindung nach einem der Ansprüche 1 bis 5, **dadurch gekennzeichnet, daß** sie der allgemeinen Formel (V) entspricht:

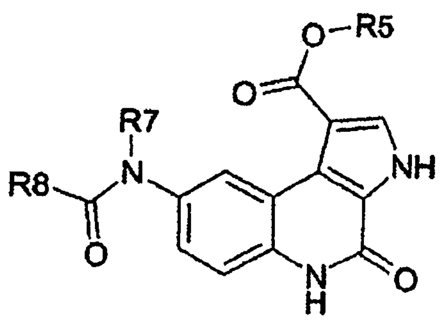
(V),

worin R7 und R8 die in einem der Ansprüche 1 bis 5 angegebene Bedeutung besitzen.

**13.** Verbindung nach Anspruch 12, ausgewählt unter den folgenden Verbindungen und ihren physiologisch annehmbaren Salzen:

8-Benzoylamino-4-oxo-4,5-dihydro-3*H*-pyrrolo[2,3-*c*]chinolin-1-carbonsäureethylester
8-Acetylamino-4-oxo-4,5-dihydro-3*H*-pyrrolo[2,3-*c*]-chinolin-1-carbonsäureethylester.

**14.** Verbindung nach einem der Ansprüche 1 bis 5, **dadurch gekennzeichnet, daß** sie der allgemeinen Formel (VI) entspricht:

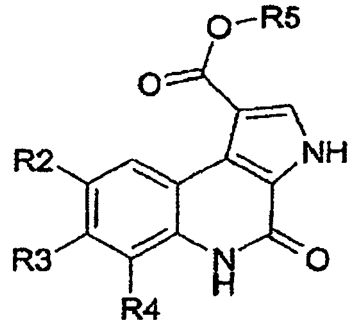

(VI),

worin R2, R3 und R4 die in einem der Ansprüche 1 bis 5 angegebene Bedeutung besitzen.

**15.** Verbindung nach Anspruch 14, ausgewählt unter den folgenden Verbindungen und ihren physiologisch annehmbaren Salzen:

8-Dimethylamino-4-oxo-4,5-dihydro-3*H*-pyrrolo[2,3-*c*]chinolin-1-carbonsäureethylester
8-Methylamino-4-oxo-4,5-dihydro-3*H*-pyrrolo[2,3-*c*]-chinolin-1-carbonsäureethylester
8-Amino-4-oxo-4,5-dihydro-3*H*-pyrrolo[2,3-*c*]-chinolin-1-carbonsäureethylester
8-Nitro-4-oxo-4,5-dihydro-3*H*-pyrrolo[2,3-*c*]-chinolin-1-carbonsäureethylester
6-Nitro-4-oxo-4,5-dihydro-3*H*-pyrrolo[2,3-*c*]-chinolin-1-carbonsäureethylester
8-(2-Ethoxycarbonylvinyl)-4-oxo-4,5-dihydro-3*H*-pyrrolo[2,3-*c*]chinolin-1-carbonsäureethylester
8-(2-Benzylcarbamoylvinyl)-4-oxo-4,5-dihydro-3*H*-pyrrolo[2,3-*c*]chinolin-1-carbonsäureethylester
8-[2-(2-Methoxycarbonylethylcarbamoyl)-vinyl]-4-oxo-4,5-dihydro-3*H*-pyrrolo[2,3-*c*]chinolin-1-carbonsäureethylester
4-Oxo-8-styryl-4,5-dihydro-3*H*-pyrrolo[2,3-*c*]-chinolin-1-carbonsäureethylester
8-Ethinyl-4-oxo-4,5-dihydro-3*H*-pyrrolo[2,3-*c*]-chinolin-1-carbonsäureethylester
4-Oxo-8-trimethylsilanylethinyl-4,5-dihydro-3*H*-pyrrolo[2,3-*c*]chinolin-1-carbonsäureethylester
4-Oxo-8-phenylethinyl-4,5-dihydro-3*H*-pyrrolo[2,3-*c*]chinolin-1-carbonsäureethylester
4-Oxo-8-pyridin-2-ylethinyl-4,5-dihydro-3*H*-pyrrolo[2,3-*c*]chinolin-1-carbonsäureethylester
8-(3-Dimethylaminoprop-1-inyl)-4-oxo-4,5-dihydro-3*H*-pyrrolo[2,3-*c*]chinolin-1-carbonsäureethylester
8-(3-Hydroxy-3-methylbut-l-inyl)-4-oxo-4,5-dihydro-3*H*-pyrrolo[2,3-*c*]chinolin-1-carbonsäureethylester
8-(3-Morpholin-4-ylprop-1-inyl)-4-oxo-4,5-dihydro-3*H*-pyrrolo[2,3-*c*]chinolin-1-carbonsäureethylester
8-{3-[(2-Cyanoethyl)methylamino]prop-1-inyl}-4-oxo-4,5-dihydro-3*H*-pyrrolo[2,3-*c*]chinolin-1-carbonsäureethylester
4-Oxo-8-phenethyl-4,5-dihydro-3*H*-pyrrolo[2,3-*c*]-chinolin-1-carbonsäureethylester
8-(3-Hydroxy-3-methylbutyl)-4-oxo-4,5-dihydro-3*H*-pyrrolo[2,3-*c*]chinolin-1-carbonsäureethylester
8-Brom-4-oxo-4,5-dihydro-3*H*-pyrrolo[2,3-*c*]-chinolin-1-carbonsäureethylester
8-Iod-4-oxo-4,5-dihydro-3*H*-pyrrolo[2,3-*c*]chinolin-1-carbonsäureethylester
8-Iod-4-oxo-4,5-dihydro-3*H*-pyrrolo[2,3-*c*]chinolin-1-carbonsäurepropylester
8-Iod-4-oxo-4,5-dihydro-3*H*-pyrrolo[2,3-*c*]chinolin-1-carbonsäurecyclopentylester
8-Iod-4-oxo-4,5-dihydro-3*H*-pyrrolo[2,3-*c*]chinolin-1-carbonsäureisobutylester
7,8-Dimethoxy-4-oxo-4,5-dihydro-3*H*-pyrrolo[2,3-*c*]-chinolin-1-carbonsäureethylester
7-Methoxy-4-oxo-4,5-dihydro-3*H*-pyrrolo[2,3-*c*]-chinolin-1-carbonsäureethylester
8-Iod-7-methoxy-4-oxo-4,5-dihydro-3*H*-pyrrolo[2,3-*c*]chinolin-1-carbonsäureethylester
8-Fluor-4-oxo-4,5-dihydro-3*H*-pyrrolo[2,3-*c*]-chinolin-1-carbonsäureethylester
8-Fluor-4-oxo-4,5-dihydro-3*H*-pyrrolo[2,3-*c*]-chinolin-1-carbonsäure-2-diethylaminoethylester
7-Fluor-4-oxo-4,5-dihydro-3*H*-pyrrolo[2,3-*c*]-chinolin-1-carbonsäureethylester
7-Chlor-4-oxo-4,5-dihydro-3*H*-pyrrolo[2,3-*c*]-chinolin-1-carbonsäureethylester
8-Acetyl-4-oxo-4,5-dihydro-3*H*-pyrrolo[2,3-*c*]-chinolin-1-carbonsäureethylester
8-Benzoyl-4-oxo-4,5-dihydro-3*H*-pyrrolo[2,3-*c*]-chinolin-1-carbonsäureethylester
8-(3-Dimethylaminoacryloyl)-4-oxo-4,5-dihydro-3*H*-pyrrolo[2,3-*c*]chinolin-1-carbonsäureethylester
8-(2-Morpholin-4-ylacetyl)-4-oxo-4,5-dihydro-3*H*-pyrrolo[2,3-*c*]chinolin-1-carbonsäureethylester
(S)-8-(Cyclopropylpyrrolidin-2-ylmethylcarbamoyl)-4-oxo-4,5-dihydro-3*H*-pyrrolo[2,3-*c*]chinolin-1-carbonsäureethylester-hydrochlorid

4-Oxo-8-phenylsulfanyl-4,5-dihydro-3*H*-pyrrolo[2,3-*c*]chinolin-1-carbonsäureethylester
8-Cyclohexylsulfanyl-4-oxo-4,5-dihydro-3*H*-pyrrolo-[2,3-*c*]chinolin-1-carbonsäureethylester
8-Benzylsulfanyl-4-oxo-4,5-dihydro-3*H*-pyrrolo[2,3-c]chinolin-l-carbonsäureethylester
8-(4-Acetylaminophenylsulfanyl)-4-oxo-4,5-dihydro-3*H*-pyrrolo[2,3-*c*]chinolin-1-carbonsäureethylester
8-(3-Aminophenylsulfanyl)-4-oxo-4,5-dihydro-3*H*-pyrrolo[2,3-*c*]chinolin-1-carbonsäureethylester
8-(3-Acetylaminophenylsulfanyl)-4-oxo-4,5-dihydro-3*H*-pyrrolo[2,3-*c*]chinolin-1-carbonsäureethylester
8-(2-Aminoethylsulfanyl)-4-oxo-4,5-dihydro-3*H*-pyrrolo[2,3-*c*]chinolin-1-carbonsäureethylester-hydrochlorid
4-Oxo-8-sulfo-4,5-dihydro-3*H*-pyrrolo[2,3-*c*]-chinolin-1-carbonsäureethylester.

**16.** Verbindung nach einem der Ansprüche 1 bis 5, **dadurch gekennzeichnet, daß** sie der allgemeinen Formel (VII) entspricht:

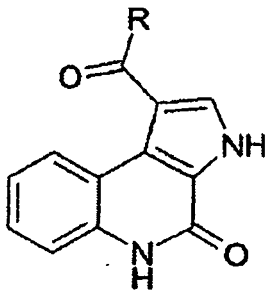

(VII),

worin R die in einem der Ansprüche 1 bis 5 angegebene Bedeutung besitzt.

**17.** Verbindung nach Anspruch 16, ausgewählt unter den folgenden Verbindungen und ihren physiologisch annehmbaren Salzen:

4-Oxo-4,5-dihydro-3*H*-pyrrolo[2,3-*c*]chinolin-1-carbonsäurebenzylester
4-Oxo-4,5-dihydro-3*H*-pyrrolo[2,3-*c*]chinolin-1-carbonsäureisopropylester
1-Acetyl-3,5-dihydropyrrolo[2,3-*c*]chinolin-4-on
*N*-(2-Dimethylaminoethyl)-4-oxo-4,5-dihydro-3*H*-pyrrolo[2,3-*c*]chinolin-1-carboxamid
*N*-(3-Dimethylaminopropyl)-4-oxo-4,5-dihydro-3*H*-pyrrolo[2,3-*c*]chinolin-1-carboxamid
3-[(4-Oxo-4,5-dihydro-3*H*-pyrrolo[2,3-*c*]chinolin-1-carbonyl)amino]propansäuremethylester
*N*-(Cyclohexylmethyl)-4-oxo-4,5-dihydro-3*H*-pyrrolo-[2,3-*c*]chinolin-1-carboxamid
*N*-(Benzyl)-4-oxo-4,5-dihydro-3*H*-pyrrolo[2,3-*c*]-chinolin-1-carboxylamid
*N*-[2-(1*H*-Indol-3-yl)ethyl]-4-oxo-4,5-dihydro-3*H*-pyrrolo[2,3-*c*]chinolin-1-carboxamid
*N*-(3-Hydroxypropyl)-4-oxo-4,5-dihydro-3*H*-pyrrolo-[2,3-*c*]chinolin-1-carboxamid
*N*-(Pyridin-4-ylmethyl)-4-oxo-4,5-dihydro-3*H*-pyrrolo[2,3-*c*]chinolin-1-carboxamid
*N*-(Phenyl)-4-oxo-4,5-dihydro-3*H*-pyrrolo[2,3-*c*]-chinolin-1-carboxamid
*N*-(Methyl)-4-oxo-4,5-dihydro-3*H*-pyrrolo[2,3-*c*]-chinolin-1-carboxamid
*N,N*-(Dimethyl)-4-oxo-4,5-dihydro-3*H*-pyrrolo[2,3-*c*]chinolin-1-carboxamid
4-Oxo-4,5-dihydro-3*H*-pyrrolo[2,3-*c*]chinolin-1-carboxamid.

**18.** Verbindung nach einem der Ansprüche 1 bis 5, **dadurch gekennzeichnet, daß** sie der allgemeinen Formel (VIII) entspricht:

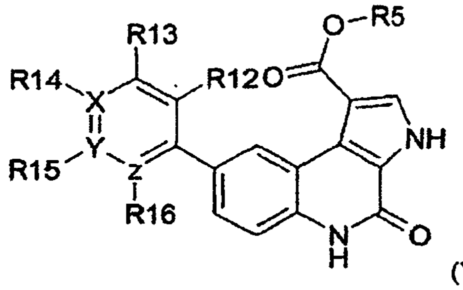

(VIII)

worin X, Y oder Z unabhängig voneinander für ein Kohlenstoffatom, ein Stickstoffatom, ein Schwefelatom oder ein Sauerstoffatom stehen, mit der Maßgabe, daß dann, wenn X, Y oder Z für ein Stickstoff-, Schwefel- oder Sauerstoffatom stehen, das Vorliegen eines Rests R14, R15 oder R16 von der Einhaltung der Wertigkeit des Stickstoff- oder Schwefelatoms, die die Aromatizität des Rings bewahrt, abhängt,
R12 für ein Wasserstoffatom, ein Halogen oder eine Alkylgruppe stehen,
R13 bis R16 unabhängig voneinander für ein Wasserstoffatom, ein Halogenatom, eine Hydroxygruppe, eine Cyanogruppe oder einen unter linearen oder verzweigten Alkyl-, Alkoxy-, Alkylsulfonyl-, Alkoxycarbonyl-, Carbamoyl-, Cycloalkyl- oder Arylgruppen, die gegebenenfalls durch einen oder mehrere Substituenten substituiert sind, und dem Rest -NR7-(Y)n-R8 ausgewählten Rest stehen,
wobei R13 und R14, R14 und R15 oder R15 und R16 einen aliphatischen oder aromatischen Ring mit einem oder mehreren unter Sauerstoff oder Stickstoff ausgewählten Heteroatomen, die gegebenenfalls durch einen oder mehrere Substituenten substituiert sind, bilden können,
wobei R5, R7 und R8 die in einem der Ansprüche 1 bis 5 angegebene Bedeutung besitzen.

**19.** Verbindung nach Anspruch 18, ausgewählt unter den folgenden Verbindungen und ihren physiologisch annehmbaren Salzen:

4-Oxo-8-phenyl-4,5-dihydro-3*H*-pyrrolo[2,3-*c*]-chinolin-1-carbonsäureethylester
8-(2-Chlorphenyl)-4-oxo-4,5-dihydro-3*H*-pyrrolo-[2,3-*c*]chinolin-1-carbonsäureethylester
8-(2-*tert*.-Butoxycarbonylaminophenyl)-4-oxo-4,5-dihydro-3*H*-pyrrolo[2,3-*c*]chinolin-1-carbonsäureethylester
8-(2-Aminophenyl)-4-oxo-4,5-dihydro-3*H*-pyrrolo-[2,3-*c*]chinolin-1-carbonsäureethylester
8-(2-Methansulfonylaminophenyl)-4-oxo-4,5-dihydro-3*H*-pyrrolo[2,3-*c*]chinolin-1-carbonsäureethylester
8-(3-*tert*.-Butoxycarbonylaminophenyl)-4-oxo-4,5-dihydro-3*H*-pyrrolo[2,3-*c*]chinolin-1-carbonsäureethylester
8-(3-Aminophenyl)-4-oxo-4,5-dihydro-3*H*-pyrrolo-[2,3-*c*]chinolin-1-carbonsäureethylester
8-(3-Methansulfonylaminophenyl)-4-oxo-4,5-dihydro-3*H*-pyrrolo[2,3-*c*]chinolin-1-carbonsäureethylester
8-(3-Acetylaminophenyl)-4-oxo-4,5-dihydro-3*H*-pyrrolo[2,3-*c*]chinolin-1-carbonsäureethylester
8-(3-Hydroxyphenyl)-4-oxo-4,5-dihydro-3*H*-pyrrolo-[2,3-*c*]chinolin-1-carbonsäureethylester
8-(3-Methansulfonylphenyl)-4-oxo-4,5-dihydro-3*H*-pyrrolo[2,3-*c*]chinolin-1-carbonsäureethylester
8-(3-Cyanophenyl)-4-oxo-4,5-dihydro-3*H*-pyrrolo-[2,3-*c*]chinolin-1-carbonsäureethylester
8-(3-Hydroxymethylphenyl)-4-oxo-4,5-dihydro-3*H*-pyrrolo[2,3-*c*]chinolin-1-carbonsäureethylester
8-(3-Methylaminomethylphenyl)-4-oxo-4,5-dihydro-3*H*-pyrrolo[2,3-*c*]chinolin-1-carbonsäureethylester
8-(3-Dimethylaminomethyl-phenyl)-4-oxo-4,5-dihydro-3*H*-pyrrolo[2,3-*c*]chinolin-1-carbonsäureethylester
8-(4-*tert*.-Butoxycarbonylaminophenyl)-4-oxo-4,5-dihydro-3*H*-pyrrolo[2,3-*c*]chinolin-1-carbonsäureethylester
8-(4-Aminophenyl)-4-oxo-4,5-dihydro-3*H*-pyrrolo-[2,3-*c*]chinolin-1-carbonsäureethylester
8-(4-Methansulfonyl-phenyl)-4-oxo-4,5-dihydro-3*H*-pyrrolo[2,3-*c*]chinolin-1-carbonsäureethylester
8-(4-Methoxyphenyl)-4-oxo-4,5-dihydro-3*H*-pyrrolo-[2,3-*c*]chinolin-1-carbonsäureethylester
8-(4-Cyanophenyl)-4-oxo-4,5-dihydro-3*H*-pyrrolo-[2,3-*c*]chinolin-1-carbonsäureethylester
8-(2,3-Dihydrobenzo[1,4]dioxin-6-yl)-4-oxo-4,5-dihydro-3*H*-pyrrolo[2,3-*c*]chinolin-1-carbonsäureethylester
8-Naphthalin-1-yl-4-oxo-4,5-dihydro-3*H*-pyrrolo-[2,3-*c*]chinolin-1-carbonsäureethylester
4-Oxo-8-chinolin-8-yl-4,5-dihydro-3*H*-pyrrolo[2,3-*c*]chinolin-1-carbonsäureethylester
8-(2-Amino-5-methansulfonylphenyl)-4-oxo-4,5-dihydro-3*H*-pyrrolo[2,3-*c*]chinolin-1-carbonsäureethylester
8-(1*H*-Indol-5-yl)-4-oxo-4,5-dihydro-3*H*-pyrrolo-[2,3-*c*]chinolin-1-carbonsäureethylester
8-(1-*tert*.-Butoxycarbonyl-1*H*-indol-5-yl)-4-oxo-4,5-dihydro-3*H*-pyrrolo[2,3-*c*]chinolin-1-carbonsäureethylester
8-(3-Methansulfonylphenyl)-4-oxo-4,5-dihydro-3*H*-pyrrolo[2,3-*c*]chinolin-1-carbonsäurepropylester
8-(3-Methansulfonylphenyl)-4-oxo-4,5-dihydro-3*H*-pyrrolo[2,3-*c*]chinolin-1-carbonsäureisobutylester
8-(3-Methansulfonylphenyl)-4-oxo-4,5-dihydro-3*H*-pyrrolo[2,3-*c*]chinolin-1-carbonsäurecyclopentylester

7-Chlor-8-(3-methansulfonylphenyl)-4-oxo-4,5-dihydro-3*H*-pyrrolo[2,3-*c*]chinolin-1-carbonsäureethylester
4-Oxo-8-pyridin-3-yl-4,5-dihydro-3*H*-pyrrolo[2,3-*c*]chinolin-1-carbonsäureethylester
8-(6-Chlorpyridin-3-yl)-4-oxo-4,5-dihydro-3*H*-pyrrolo[2,3-*c*]chinolin-1-carbonsäureethylester
8-(6-Methoxypyridin-3-yl)-4-oxo-4,5-dihydro-3*H*-pyrrolo[2,3-*c*]chinolin-1-carbonsäureethylester
8-(2-Aminopyrimidin-4-yl)-4-oxo-4,5-dihydro-3*H*-pyrrolo[2,3-*c*]chinolin-1-carbonsäureethylester
8-(2-Dimethylaminopyrimidin-4-yl)-4-oxo-4,5-dihydro-3*H*-pyrrolo[2,3-*c*]chinolin-1-carbonsäureethylester
8-(2-Methylaminopyrimidin-4-yl)-4-oxo-4,5-dihydro-3*H*-pyrrolo[2,3-*c*]chinolin-1-carbonsäureethylesterformiat
8-(2-Ethylaminopyrimidin-4-yl)-4-oxo-4,5-dihydro-3*H*-pyrrolo[2,3-*c*]chinolin-1-carbonsäureethylester
4-Oxo-8-[2-(4-pyridin-2-ylpiperazin-1-yl)-pyrimidin-4-yl]-4,5-dihydro-3*H*-pyrrolo[2,3-*c*]-chinolin-1-carbonsäureethylester
4-Oxo-8-(2-phenylaminopyrimidin-4-yl)-4,5-dihydro-3*H*-pyrrolo[2,3-*c*]chinolin-1-carbonsäureethylester
8-[2-(1*H*-Benzoimidazol-2-ylamino)pyrimidin-4-yl]-4-oxo-4,5-dihydro-3*H*-pyrrolo[2,3-*c*]chinolin-1-carbonsäureethylester
8-[2-(4-Acetylpiperazin-1-yl)-pyrimidin-4-yl]-4-oxo-4,5-dihydro-3*H*-pyrrolo[2,3-*c*]chinolin-1-carbonsäureethylester
8-{2-[(2-Dimethylaminoethyl)methylamino]-pyrimidin-4-yl}-4-oxo-4,5-dihydro-3*H*-pyrrolo[2,3-*c*]chinolin-1-carbonsäureethylester-formiat.

**20.** Verbindung nach einem der Ansprüche 1 bis 5, **dadurch gekennzeichnet, daß** sie der allgemeinen Formel (IX) entspricht:

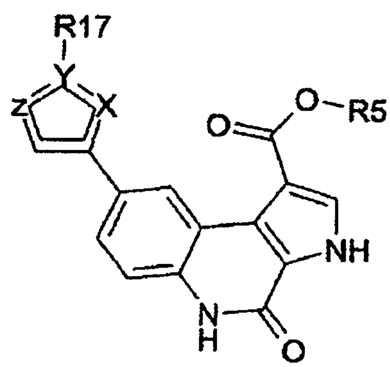

(IX)

worin X, Y oder Z unabhängig voneinander für ein Kohlenstoffatom, ein Stickstoffatom, ein Schwefelatom oder ein Sauerstoffatom stehen, mit der Maßgabe, daß dann, wenn X, Y oder Z für ein Stickstoff-, Schwefel- oder Sauerstoffatom stehen, das Vorliegen eines Rests R17 von der Einhaltung der Wertigkeit des Stickstoff- oder Schwefelatoms, die die Aromatizität des Rings bewahrt, abhängt,
R17 für ein Wasserstoffatom oder eine Alkyl-, Cycloalkyl- oder Arylgruppe, die gegebenenfalls durch einen oder mehrere Substituenten substituiert sind, oder einen Aryl-, -NR7-(Y)n-R8- oder -NR7-NH-R8-Rest steht,
wobei R5, R7 und R8 die in einem der Ansprüche 1 bis 5 angegebene Bedeutung besitzen.

**21.** Verbindung nach Anspruch 20, ausgewählt unter den folgenden Verbindungen und ihren physiologisch annehmbaren Salzen:

8-(2Aminothiazol-4-yl)-4-oxo-4,5-dihydro-3*H*-pyrrolo[2,3-*c*]chinolin-1-carbonsäureethylester
8-[2-Phenylaminothiazol-4-yl]-4-oxo-4,5-dihydro-3*H*-pyrrolo[2,3-*c*]chinolin-1-carbonsäureethylester
8-[2-(2-Chlorphenylamino)thiazol-4-yl]-4-oxo-4,5-dihydro-3*H*-pyrrolo[2,3-*c*]chinolin-1-carbonsäureethylester
8-[2-(3,4-Dichlorphenylamino)thiazol-4-yl]-4-oxo-4,5-dihydro-3*H*-pyrrolo[2,3-*c*]chinolin-1-carbonsäureethylester
8-[2-(2,6-Dichlorphenylamino)thiazol-4-yl]-4-oxo-4,5-dihydro-3*H*-pyrrolo[2,3-*c*]chinolin-1-carbonsäureethylester
8-[2-(Cyclopropancarbonylamino) thiazol- 4- yl]- 4- oxo- 4,5- dihydro- 3*H*-pyrrolo [2,3-*c*] chinolin- 1- carbonsäureethylester
4-Oxo-8-(2-phenylsulfanylmethylthiazol-4-yl)-4,5-dihydro-3*H*-pyrrolo[2,3-*c*]chinolin-1-carbonsäureethylester
4-Oxo-8-(2-piperidin-4-ylthiazol-4-yl)-4,5-dihydro-3*H*-pyrrolo[2,3-*c*]chinolin-1-carbonsäureethylester

8-(2-Methylthiazol-4-yl)-4-oxo-4,5-dihydro-3*H*-pyrrolo[2,3-*c*]chinolin-1-carbonsäureethylester
4-Oxo-8-(2-phenylthiazol-4-yl)-4,5-dihydro-3*H*-pyrrolo[2,3-*c*]chinolin-1-carbonsäureethylester
4-Oxo-8-[2-(*N'*-phenylhydrazino)thiazol-4-yl]-4,5-dihydro-3*H*-pyrrolo[2,3-*c*]chinolin-1-carbonsäureethylester
8-Isoxazol-5-yl-4-oxo-4,5-dihydro-3H-pyrrolo[2,3-*c*]chinolin-1-carbonsäureethylester
4-Oxo-8-(1*H*-pyrazol-3-yl)-4,5-dihydro-3*H*-pyrrolo-[2,3-*c*]chinolin-1-carbonsäureethylester
8-(1-Methyl-1*H*-pyrazol-3-yl)-4-oxo-4,5-dihydro-3*H*-pyrrolo[2,3-*c*]chinolin-1-carbonsäureethylester.

**22.** Pharmazeutische Zusammensetzung, enthaltend einen geeigneten pharmazeutischen Träger und eine Verbindung, die unter den Verbindungen nach einem der Ansprüche 1 bis 21 und den folgenden Verbindungen und ihren physiologisch annehmbaren Salzen ausgewählt ist:

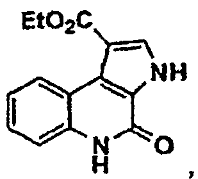 , 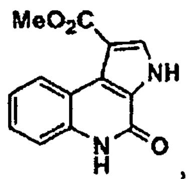 , 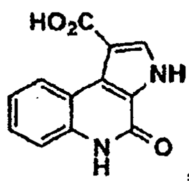 , 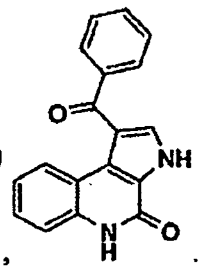 .

**23.** Verbindung, die unter den Verbindungen nach einem der Ansprüche 1 bis 21 und den folgenden Verbindungen und ihren physiologisch annehmbaren Salzen ausgewählt ist:

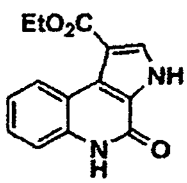 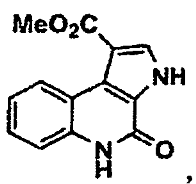 , 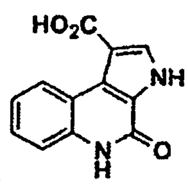 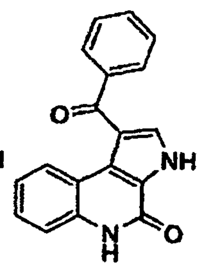

zur Verwendung bei der Therapie.

**24.** Verbindungen nach Anspruch 23 zur Verwendung bei der Behandlung von Krebserkrankungen, entzündlichen Erkrankungen oder Störungen des Zentralnervensystems.

**25.** Verwendung nach einem der Ansprüche 23 oder 24 in Kombination mit mindestens einem anderen Wirkstoff.

**26.** Verwendung einer Verbindung, die unter den Verbindungen nach einem der Ansprüche 1 bis 21 und den folgenden Verbindungen und ihren physiologisch annehmbaren Salzen ausgewählt ist:

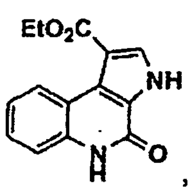 , 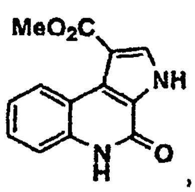 , 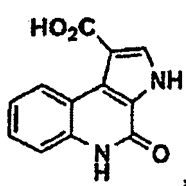 , 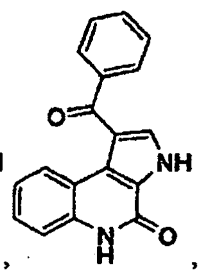 ,

zur Herstellung eines Arzneimittels zur Behandlung von Krebserkrankungen, entzündlichen Erkrankungen oder Störungen des Zentralnervensystems.

**27.** Verwendung nach Anspruch 26 in Kombination mit mindestens einem anderen Wirkstoff.

**28.** Nichttherapeutische Verwendung einer Verbindung, die unter den Verbindungen nach einem der Ansprüche 1 bis 21 und den folgenden Verbindungen und ihren physiologisch annehmbaren Salzen ausgewählt ist:

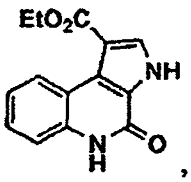,
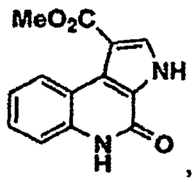,
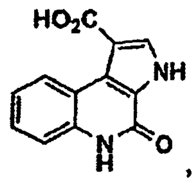,
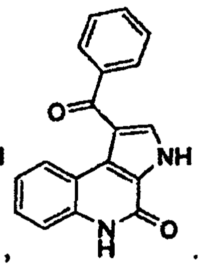.

als Kinaseinhibitor.

**29.** Produkt, enthaltend eine Verbindung, die unter den Verbindungen nach einem der Ansprüche 1 bis 21 und den folgenden Verbindungen und ihren physiologisch annehmbaren Salzen ausgewählt ist:

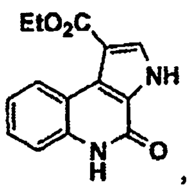,
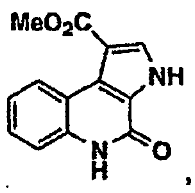,
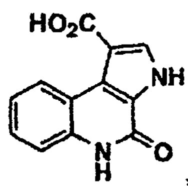,
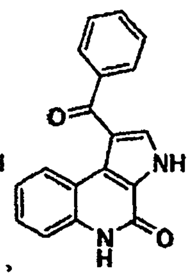

und einen anderen Wirkstoff, als Kombinationsprodukt zur gleichzeitigen, getrennten oder zeitlich gestaffelten Verwendung bei der Therapie.

**30.** Produkt nach Anspruch 29 zur Verwendung bei der Behandlung von Krebserkrankungen, entzündlichen Erkrankungen oder Störungen des Zentralnervensystems.

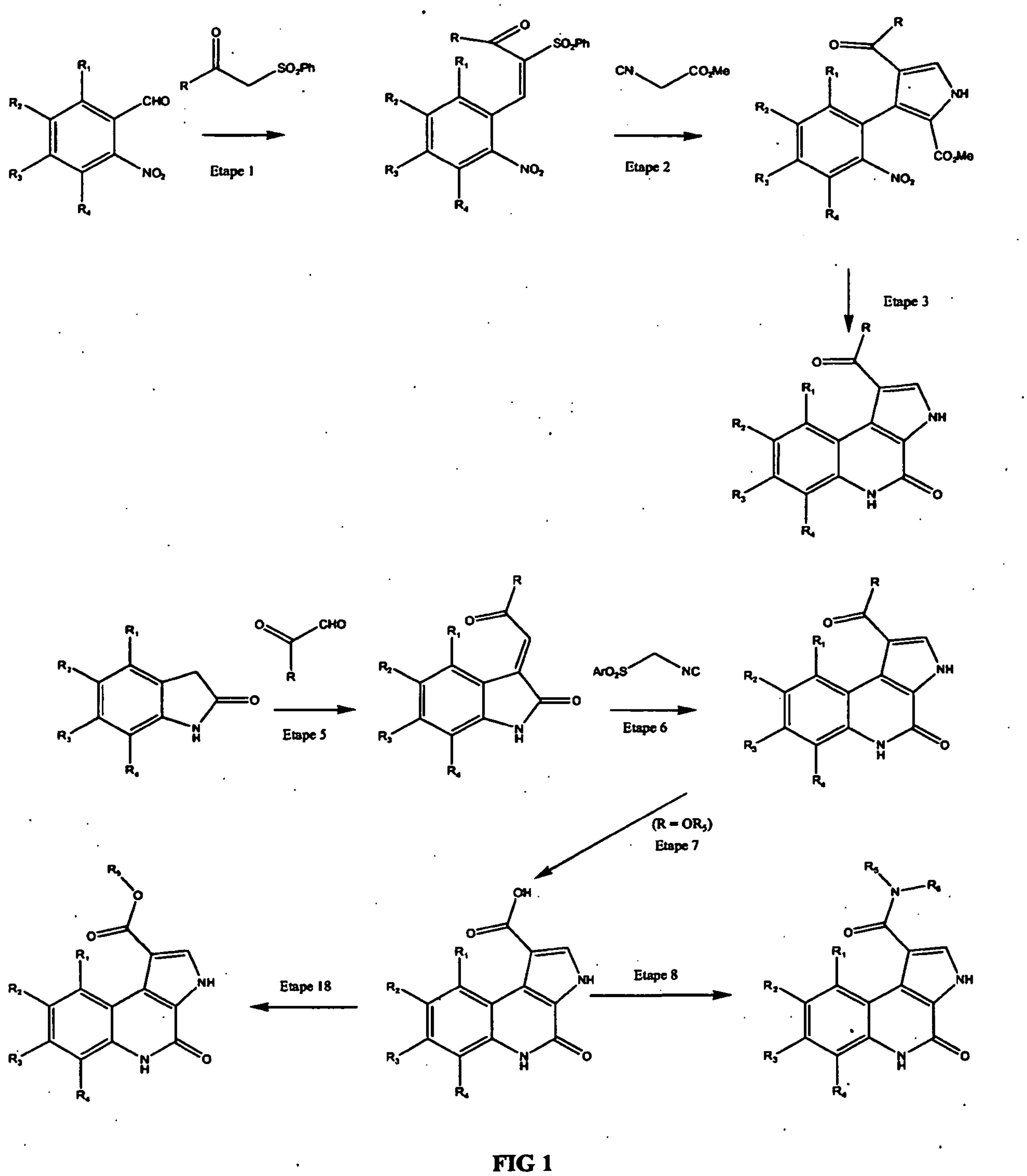
Etape 1
Etape 2
Etape 3
Etape 5
Etape 6
(R = OR5)
Etape 7
Etape 18
Etape 8

**FIG 1**

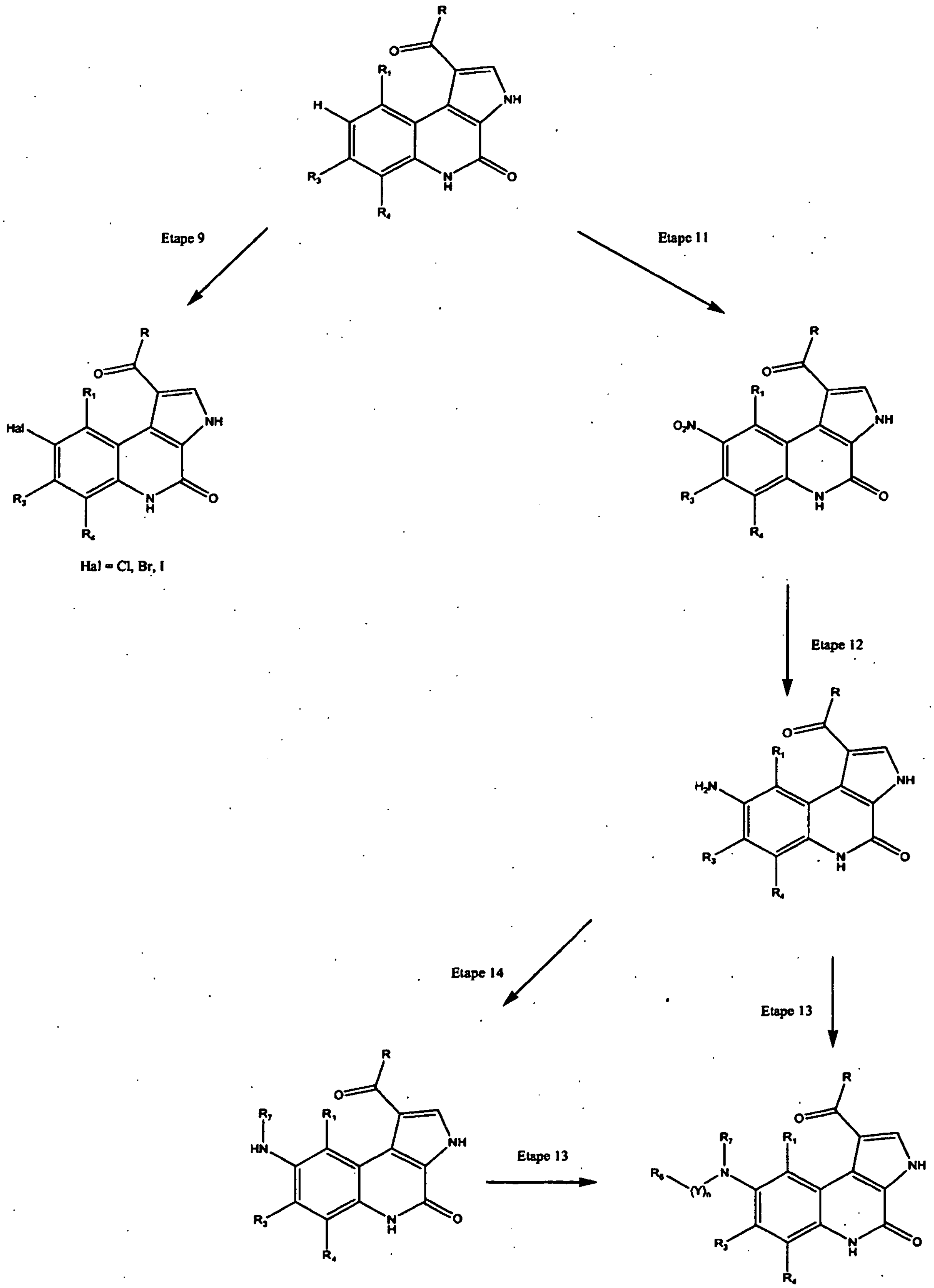


FIG 2

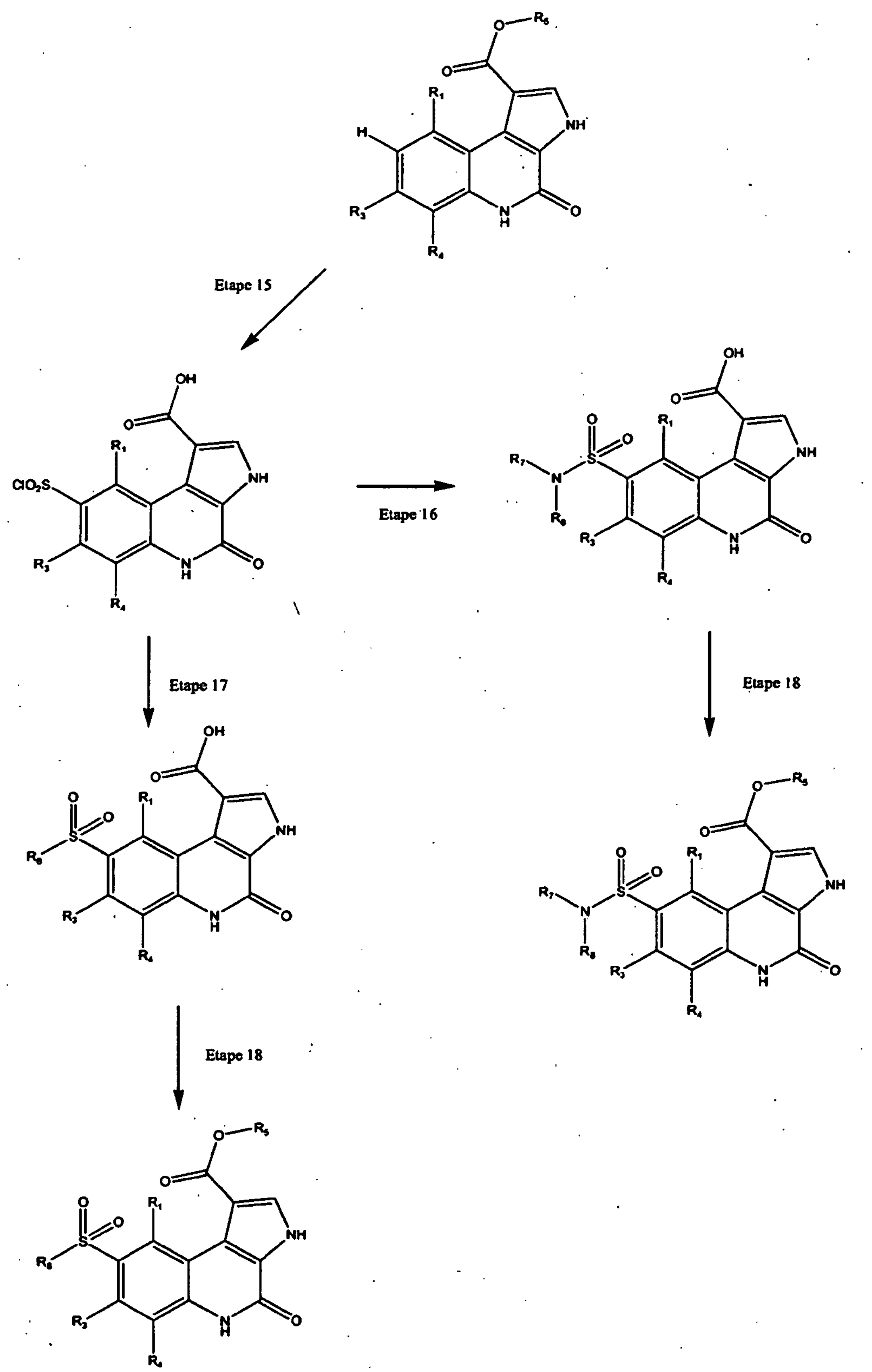


FIG 3

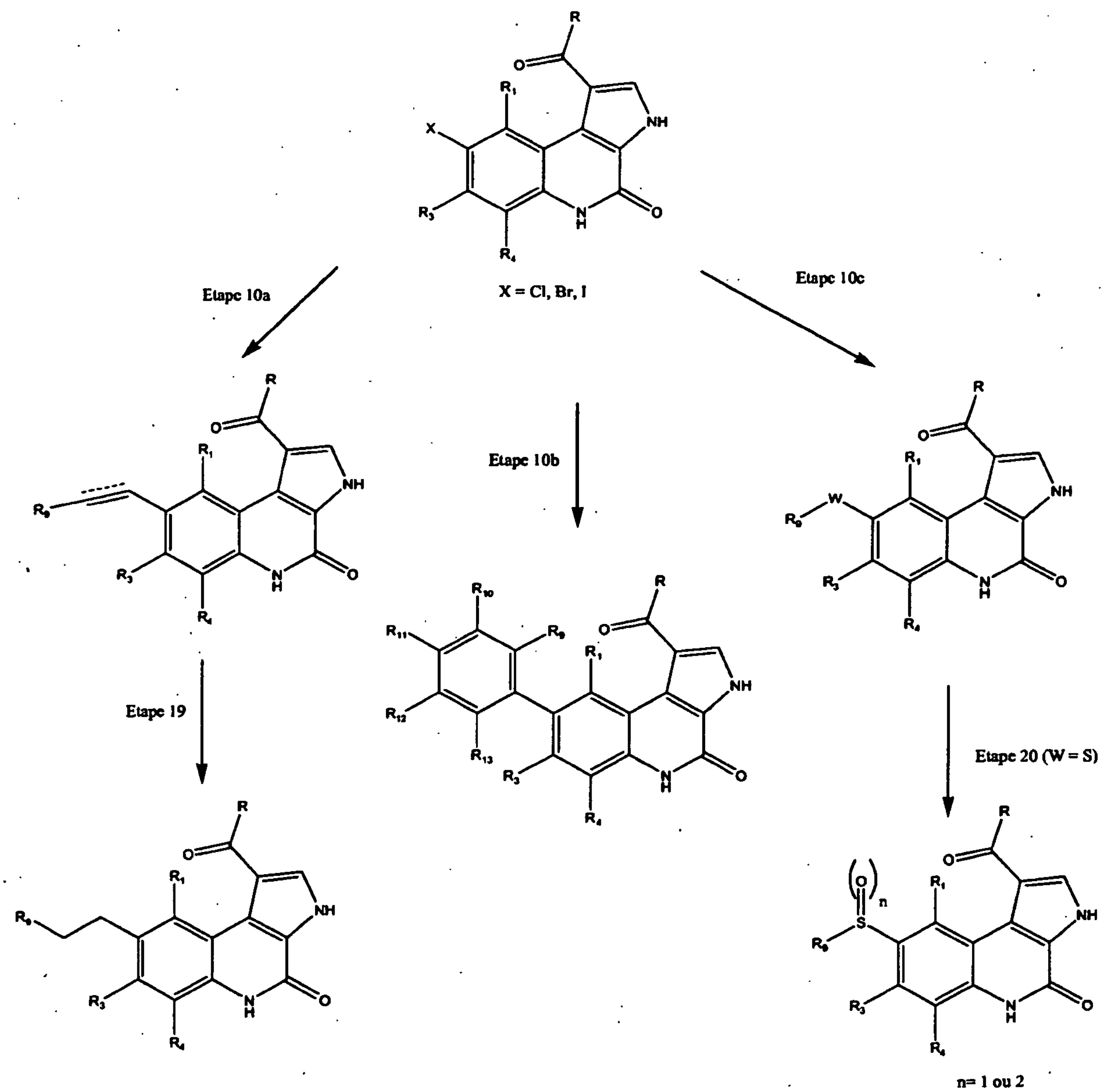
X = Cl, Br, I
Etape 10a
Etape 10b
Etape 10c
Etape 19
Etape 20 (W = S)
n= 1 ou 2

**FIG 4**

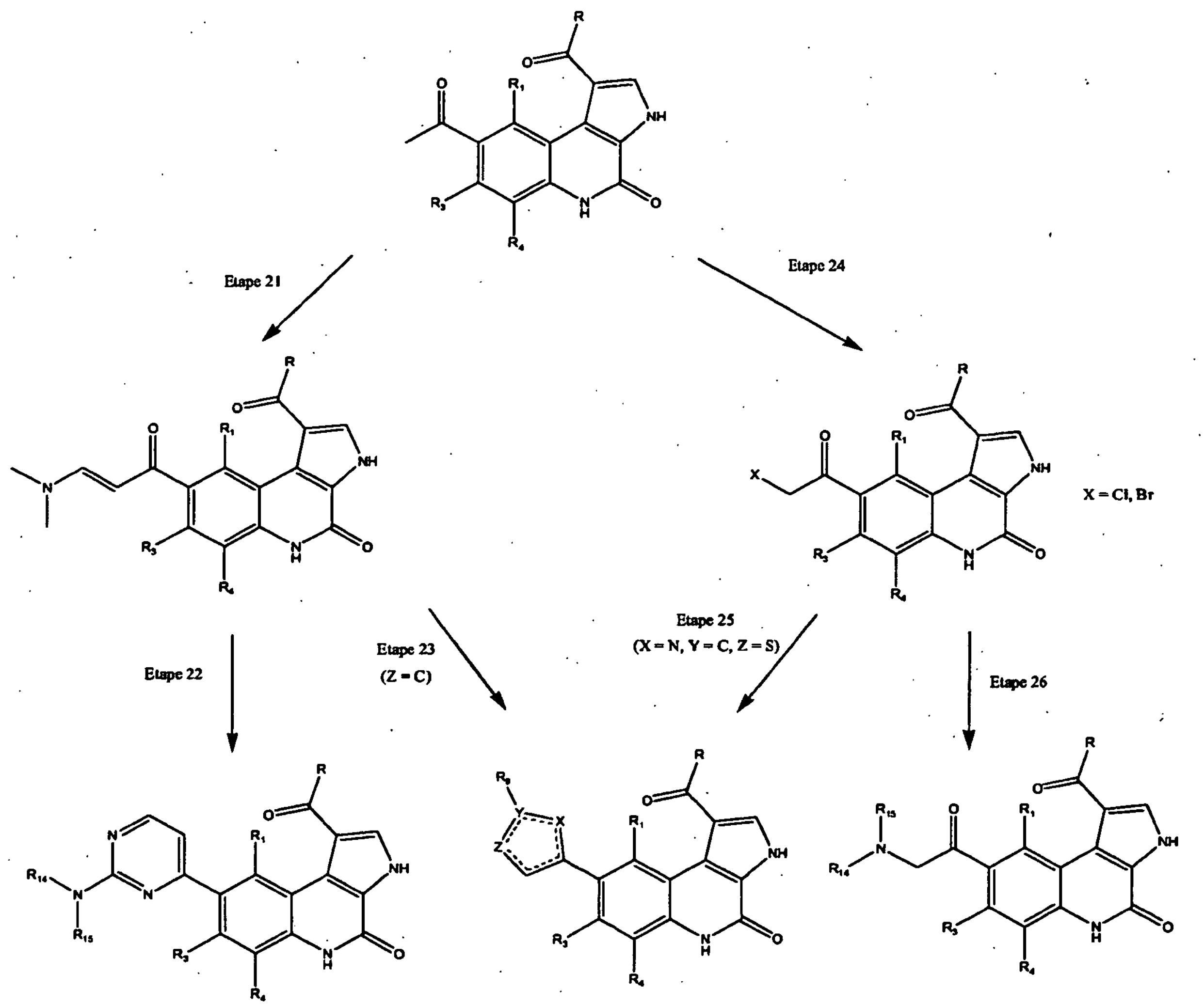
Etape 21
Etape 24
X = Cl, Br
Etape 22
Etape 23
(Z = C)
Etape 25
(X = N, Y = C, Z = S)
Etape 26

FIG 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20030069451 A
- WO 9910325 A
- WO 2004009083 A
- US 20030069421 A
- WO 2004009083 A1
- US 006107321 A, A. Madin
- US 20030069421 A1, Peng Cho Tang


### Littérature non-brevet citée dans la description


- **Salvatore et al.** *J. Clin. Endocr. Metab.,* 2000, vol. 85, 3898-3907
- **Pützer ; Drosten.** *Trends in Mol. Med.,* 2004, vol. 10 (7), 351-357
- **Drosten et al.** *Surgery,* 2002, vol. 132, 991-7
- **Cohen et al.** *Surgery,* 2002, vol. 132, 960-7
- **Carlomagno et al.** *Cancer Research,* 2002, vol. 62, 1077-1082
- **Ikeda et al.** *Oncogene,* 1990, vol. 5, 1291-1296
- **Santoro et al.** *Oncogene,* 1990, vol. 5, 1595-1598
- **Avantaggiato et al.** *Cell Growth Differ.,* 1994, vol. 5, 305-311
- **Attie-Bitach et al.** *Am. J. Med. Genet.,* 1998, vol. 80, 481-486
- **Takahashi et al.** *Oncogene,* 1991, vol. 6, 297-301
- **Jhiang SM.** *Oncogene,* 2000, vol. 19 (49), 5590-5597
- **Russell et al.** *J. Immunol.,* 2004, vol. 172 (7), 4059-4067
- **Harada et al.** *Diabetes Care,* 2002, vol. 25 (6), 1060-1065
- **Jan Bergman ; Stanley Rehn.** *Tetrahedron,* 2002, vol. 58, 9179-9185
- **SoonY Ko et al.** *J Org. Chem.,* 1994, vol. 59, 2570-6
- **J.Bergman.** *Tetrahedron,* 2002, vol. 58, 9179-9185
- **J. Bergman.** *Tetrahedron,* 2002, vol. 58, 9179-9185
- **Okada Tetsuo et al.** *Chem. Pharm. Bull.,* 1993, vol. 41 (1), 126-31
- **Kleemann, Heinz-Werner et al.** *J. Med. Chem.,* 1992, vol. 35 (3), 559-67
- **Kato, shiro et al.** *J. Chem. Soc. Perkin Trans. 1,* 1997, vol. 21, 3219-26
- **Itaya, taisuke et al.** *Tetrahedron Lett.,* 1986, vol. 52 (27), 6349-52
- **Morrow, D. F. et al.** *J. Med Chem.,* 1973, vol. 16 (6), 736-9
- **Carretero, Juan Carlos ; Adrio Javier.** *Synthesis,* 2001, vol. 12, 1888-96
- **George J. Quallich ; P.M. Morissey.** *Synthesis,* 1993, 51-53
- **Li Sun ; Ngoc Tran ; Congxin Liang ; Flora Tang ; Audie Rice ; Randall Schreck ; Kara Waltz ; Laura K. Shawver ; Gerald McMahon ; Cho Tang.** *Journal of Medicinal Chemistry,* 1999, vol. 42 (25), 5120
- **Chan wing yan.** *J. Heterocycl. Chem.,* 2003, vol. 10 (2), 289-96